# EUROPEAN PATENT APPLICATION

(11) **EP 4 450 490 A1**
(43) Date of publication of application: **23.10.2024**
(21) Application number: 22907924.9
(22) Date of filing: 14.12.2022
(51) Int. Cl.: C07D 307/68, A61K 31/55, C07D 295/125, C07D 211/58, C07D 405/06, C07D 405/04, C07D 495/14, C07C 219/06, C07C 237/20

(54) **NOVEL COMPOUND FOR DEGRADING TARGET PROTEIN OR POLYPEPTIDE BY USING POLYUBIQUITINATION**

(30) Priority: 14.12.2021 KR 20210178569; 02.06.2022 KR 20220067712
(71) Applicant: Prazer Therapeutics Inc., Seoul 02455 (KR)
(72) Inventor: INN, Kyung Soo, Goyang-si, Gyeonggi-do 10491 (KR); KIM, Nam Jung, Seoul 06354 (KR); LEE, Jong Kil, Seoul 02567 (KR); KIM, Ga Yeong, Seoul 05837 (KR); KIM, Kyeo Jin, Seoul 06354 (KR); KIM, Dong Hwan, Seoul 02796 (KR); KIM, So Young, Yongin-si, Gyeonggi-do 16817 (KR); KIM, Soo Lim, Seoul 05333 (KR); KIM, Hee Jin, Seoul 07982 (KR); DO, Ji Min, Seoul 05354 (KR); MIN, Chan Ki, Seoul 05804 (KR); SEO, Min Jung, Incheon 22779 (KR); SONG, Chae Won, Seoul 05245 (KR); LEE, Na Rae, Seoul 01827 (KR); LEE, Hye Won, Seoul 08748 (KR); SON, Seung Hwan, Seoul 02443 (KR); LEE, Dan Bi, Seoul 05531 (KR); LEE, Se In, Seoul 05259 (KR)
(74) Representative: Maiwald GmbH
(86) International application number: PCT/KR2022/020310
(87) International publication number: WO 2023/113457

(57) **Abstract**

The present invention relates to a compound capable of degrading a target protein by using ubiquitination, and provides a compound having a targeted protein binding moiety (TBM) structure, which binds to a target protein or polypeptide, and a ubiquitin binding moiety (UBM) structure to which ubiquitin binds. Since polyubiquitination can be induced by means of the UBM structure present in the compound, the compounds of the present invention can be a degrader (protein degrader) capable of degrading a target protein or polypeptide.

## Description

### TECHNICAL FIELD

The present invention relates to a novel compound capable of degrading a target protein or polypeptide by using ubiquitin, and more specifically, to a compound having a ubiquitin binding moiety (UBM) structure which is able to be covalently linked to a targeted protein binding moiety (TBM) structure containing a ligand binding to a target protein, and directly bound to ubiquitin at the same time.

### BACKGROUND ART

Ubiquitin is a molecular identification marker that covalently binds to the terminal of a protein that needs degradation, and in this process, E1 enzyme (ubiquitin-activating enzyme), E2 enzyme (ubiquitin carrier protein), and E3 enzyme (ubiquitin-protein ligase, E3 ligase) present in the body are involved. The binding of ubiquitin is called ubiquitination of a protein, and when polyubiquitination in which several ubiquitins are bound takes place in vivo, a protein is degraded through an intracellular protein degradation mechanism including proteasome or lysosome. The protein debris thus degraded may be recycled in cells. The process in which a protein is recognized by endosome or autophagosome in a cell, bound to lysosome, and degraded through endo/auto lysosome is called endo/lysomal degradation. In addition, the process in which a protein is targeted and degraded by proteosomes is called a ubiquitin-proteasome system (UPS). In the degradation of a target protein or polypeptide, it is very useful to use an ubiquitination pathway, particularly when a kinase, which is a phosphorylation enzyme, or a specific receptor is a target.

Meanwhile, in order to use the ubiquitin-proteasome system, a bifunctional compound in which a "moiety binding to a target protein or polypeptide" and a "moiety binding to an E3 enzyme" are linked by a "linker" has been used, and a technique for inducing degradation of a target protein by ubiquitination of a lysine residue of the target protein is called a proteolysis-targeting chimera (PROTAC). This has advantages that it is possible to degrade disease-related proteins, such as transcription factors, scaffold proteins, and tau proteins, which are difficult to be regulated by existing small molecules, antibodies and pharmaceuticals, has high selectivity for the target and is able to be reused allowing for low-dose administration. In addition, neutralizing the excessive action of ligands by blocking ligands or using antagonists in diseases caused by ligand-receptor binding is a therapeutic method having a high possibility of inducing resistance in patients and having very low selectivity, and thus an approach to degrade a receptor protein itself that binds to ligands is more effective.

However, PROTAC compound has significantly different efficacy in degrading target proteins depending on the type of E3 enzyme they bind to. Among 600 or more E3 enzymes, about 5 types are currently used in PROTAC technology, and those mainly used are CRBN, VHL, IAP, and the like. In addition, the PROTAC compound in which both the target protein binding moiety and the E3 enzyme binding moiety are composed of peptides has a disadvantage that cell permeability is low, and in order to improve this, it has been reported that small molecules other than the peptide are used as the E3 enzyme binding ligand, or in some embodiments of WO 2020/251971, a moiety that mimics lysine is ubiquitinated and thus can be degraded through the ubiquitin-proteasome pathway (UPP), but there is no commercially available product, so that there is a need to research and develop a technology for the same.

### DISCLOSURE OF THE INVENTION

### TECHNICAL PROBLEM

An aspect of the present invention provides a compound having a ubiquitin binding moiety (UBM) structure which is able to directly bind to ubiquitin.

Another aspect of the present invention provides a compound in which the compound having the UBM structure and a compound having a targeted protein/polypeptide binding moiety (TBM) structure are linked to each other.

### TECHNICAL SOLUTION

According to an aspect of the present invention, there is provided a compound represented by Formula I to Formula IV below, or a pharmaceutically acceptable salt, isomer, solvate, hydrate, or prodrug thereof:

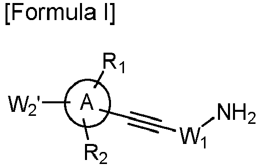

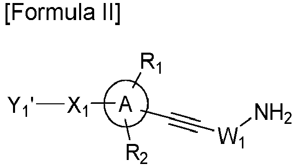

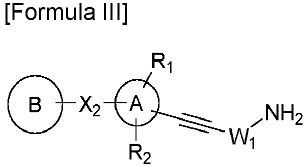

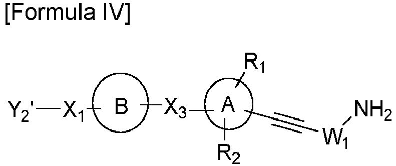

In addition, according to another aspect of the present invention, there is provided a compound or a pharmaceutically acceptable salt, isomer, solvate, hydrate, or prodrug thereof, the compound having a chemical structure of TBM-UBM, wherein the TBM is a moiety binding to a target protein or polypeptide, and the UBM is a chemical structure represented by Formula V to Formula VIII below:

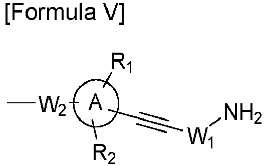

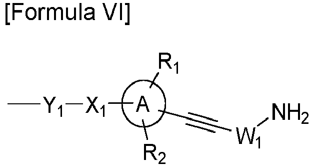

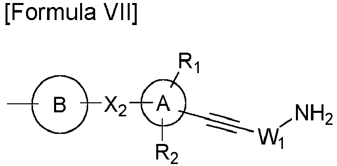

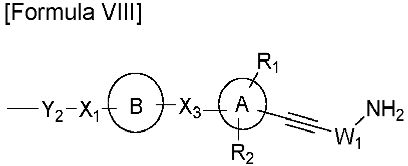

### ADVANTAGEOUS EFFECTS

The compounds according to the present invention may be include the UBM structure capable of inducing polyubiquitination of a target protein or polypeptide, and thus may be degraders capable of selectively degrading the target protein or polypeptide by proteasome or lysosome. That is, the target protein or polypeptide in which ubiquitination is induced by the UBM may use endo/autolysosomal degradation as well as target protein degradation using the UPS like the existing PROTAC, and thus the TBM-UBM compound of the present invention has an advantage that it may selectively target and degrade a protein complex and a protein aggregate.

However, the effects of the present invention are not limited to the above-mentioned effects and other effects not mentioned will be clearly understood from the following description by a person skilled in the art.

### BRIEF DESCRIPTION OF THE DRAWINGS

FIG. 1 is a schematic view illustrating the principle of operation of a protein degrader which is formed by binding TBM-UBM compounds of the present invention with a target protein or polypeptide.
FIG. 2 shows Pulldown assay results performed to confirm that UBM compounds are receptors directly binding to ubiquitin.
FIG. 3 shows mass spectrometry values through intact protein analysis performed to confirm that a BRD4 degrader directly binds to ubiquitin.

### MODE FOR CARRYING OUT THE INVENTION

First, terms, as used herein, are defined.

The following terms herein have the following meanings unless otherwise indicated. Any term that is not defined has a meaning that is understood in the art.

Throughout the specification, a part "including" an element means that it may further include other elements rather than exclude other elements unless otherwise indicated.

In a structural formula of the specification, a symbol "-" that binds an atom and/or a group may mean a single bond, and a symbol "=" may mean a double bond. The symbols may be omitted, or, when necessary, may be indicated such as in case where the binding atom or binding position need to be specified.

As used herein, the term "halogen" refers to fluorine (F), chlorine (CI), bromine (Br), and iodine (I).

As used herein, the term "alkyl" refers to a cyclic or acyclic aliphatic hydrocarbon group that does not include a double bond or a triple bond, and unless otherwise indicated, the acyclic alkyl may have 1 to 20 carbon atoms, 1 to 19 carbon atoms, 1 to 18 carbon atoms, 1 to 17 carbon atoms, 1 to 16 carbon atoms, 1 to 15 carbon atoms, 1 to 14 carbon atoms, 1 to 13 carbon atoms, 1 to 12 carbon atoms, 1 to 11 carbon atoms, 1 to 10 carbon atoms, for example, 1 to 6 carbon atoms, and in particular, 1 to 4 carbon atoms, and may be linear or branched. Specific examples of the alkyl group may include a methyl group, an ethyl group, a propyl group, an n-propyl group, an isopropyl group, a butyl group, an n-butyl group, an isobutyl group, a *tert*-butyl group, a sec-butyl group, a 1-methylbutyl group, a 1-ethylbutyl group, a pentyl group, an n-pentyl group, an isopentyl group, a neopentyl group, a *tert*-pentyl group, a hexyl group, an n-hexyl group, a 1-methylpentyl group, a 2-methylpentyl group, a 4-methyl-2-pentyl group, a 3,3-dimethyl butyl group, a 2-ethylbutyl group, a heptyl group, an n-heptyl group, a 1-methylhexyl group, an octyl group, an n-octyl group, a *tert*-octyl group, a 1-methylheptyl group, a 2-ethylhexyl group, a 2-propylpentyl group, an n-nonyl group, a 2,2-dimethylheptyl group, a 1-ethylpropyl group, a 1,1-dimethylpropyl group, an isohexyl group, a 4-methylhexyl group, a 5-methylhexyl group, a benzyl group, *etc.,* but are not limited thereto. In addition, the cyclic alkyl is specifically referred to as "cycloalkyl" herein, and the "cycloalkyl" is a cyclic aliphatic hydrocarbon group that does not include a double bond or a triple bond, and unless otherwise indicated, the cycloalkyl may have a 3 to 30 carbon atoms, 3 to 28 carbon atoms, 3 to 26 carbon atoms, 3 to 24 carbon atoms, 3 to 22 carbon atoms, 3 to 20 carbon atoms, 3 to 18 carbon atoms, 3 to 16 carbon atoms, 3 to 14 carbon atoms, 3 to 12 carbon atoms, 3 to 10 carbon atoms, for example, 3 to 8 carbon atoms, and in particular, 3 to 6 carbon atoms, and may be monocyclic or polycyclic. The polycyclic group refers to a group in which a cycloalkyl group is directly linked to or fused with another cyclic group, wherein the other cyclic group may be a cycloalkyl group, but may be another type of cyclic group, for example, a heterocycloalkyl group, an aryl group, a heteroaryl group, or the like. Specific examples of the cycloalkyl group may include a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-*tert*-butylcyclohexyl group, a cycloheptyl group, a cyclooctyl group, a cyclononyl group, a cyclodecyl group, a cycloundecyl group, a cyclododecyl group, a bicyclo[2.2.1]heptyl group, a bicyclo[2.2.2]octyl group, a bicyclo[3.2.2]nonyl group, a bicyclo[4.4.0]decyl group, a bicyclo[4.1.0]heptyl group, and the like, but are not limited thereto.

As used herein, the term "alkenyl" refers to a cyclic or acyclic aliphatic hydrocarbon group including at least one double bond, and unless otherwise indicated, the acyclic alkenyl may have 2 to 20 carbon atoms, 2 to 19 carbon atoms, 2 to 18 carbon atoms, 2 to 17 carbon atoms, 2 to 16 carbon atoms, 2 to 15 carbon atoms, 2 to 14 carbon atoms, 2 to 13 carbon atoms, 2 to 12 carbon atoms, 2 to 11 carbon atoms, 2 to 10 carbon atoms, for example, 2 to 6 carbon atoms, and in particular, 2 to 4 carbon atoms, and may be linear or branched. Specific examples of the alkenyl group may include a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group, and the like, but are not limited thereto. In addition, the cyclic alkenyl is specifically referred to as "cycloalkenyl" herein, and the "cycloalkenyl" is a cyclic aliphatic hydrocarbon group including at least one double bond, and unless otherwise indicated, may have 3 to 30 carbon atoms, 3 to 28 carbon atoms, 3 to 26 carbon atoms, 3 to 24 carbon atoms, 3 to 22 carbon atoms, 3 to 20 carbon atoms, 3 to 18 carbon atoms, 3 to 16 carbon atoms, 3 to 14 carbon atoms, 3 to 12 carbon atoms, 3 to 10 carbon atoms, for example, 3 to 8 carbon atoms, and in particular, 3 to 6 carbon atoms, and may be monocyclic or polycyclic. The polycyclic group refers to a group in which a cycloalkenyl group is directly linked to or fused with another cyclic group, wherein the other cyclic group may be a cycloalkyl group, but may be another type of cyclic group, for example, a heterocycloalkyl group, an aryl group, a heteroaryl group, or the like. Specific examples of the cycloalkenyl group include a cyclopentenyl group, a cyclohexenyl group, a cyclopenta-1,3-dienyl group, a cycloheptenyl group, a cyclooctenyl group, a cycloocta-1 ,4-dienyl group, and the like, but are not limited thereto.

As used herein, the term "alkynyl" refers to an aliphatic hydrocarbon group including at least one triple bond, and unless otherwise indicated, may have 2 to 20 carbon atoms, 2 to 19 carbon atoms, 2 to 18 carbon atoms, 2 to 17 carbon atoms, 2 to 16 carbon atoms, 2 to 15 carbon atoms, 2 to 14 carbon atoms, 2 to 13 carbon atoms, 2 to 12 carbon atoms, 2 to 11 carbon atoms, 2 to 10 carbon atoms, for example, 2 to 6 carbon atoms, and in particular, 2 to 4 carbon atoms, and may be linear or branched. Specific examples of the alkynyl group include an ethynyl group, a propynyl group, a butynyl group, a pentynyl group, a hexynyl group, and the like, but are not limited thereto.

As used herein, the term "aryl" refers to an aromatic hydrocarbon group, and unless otherwise indicated, may have 6 to 30 carbon atoms, 6 to 28 carbon atoms, 6 to 26 carbon atoms, 6 to 24 carbon atoms, 6 to 22 carbon atoms, 6 to 20 carbon atoms, 6 to 18 carbon atoms, 6 to 16 carbon atoms, 6 to 14 carbon atoms, for example, 6 to 12 carbon atoms, and may be monocyclic or polycyclic. The polycyclic group refers to a group in which an aryl group is directly linked or fused with another cyclic group, wherein the other cyclic group may be an aryl group, but may be other types of cyclic groups such as a cycloalkyl group, a heterocycloalkyl group, and a heteroaryl group. In addition, the aryl group includes a spiro group. Specific examples of the aryl group may include a phenyl group, a biphenyl group, a triphenyl group, a naphthyl group, an anthryl group, a chrysenyl group, a phenanthrenyl group, a perylenyl group, a fluoranthenyl group, a triphenylenyl group, a phenalenyl group, a pyrenyl group, a tetracenyl group, a pentacenyl group, a fluorenyl group, an indenyl group, an acenaphthylenyl group, a benzofluorenyl group, a spirobifluorenyl group, a 2,3-dihydro-1H-indenyl group, a fused cyclic group thereof, and the like, but are not limited thereto.

As used herein, the terms "heterocycloalkyl," "heterocycloalkenyl," and "heteroaryl" mean that at least one atom constituting the above-described ring of cycloalkyl, cycloalkenyl, and aryl is substituted with a heteroatom such as O, S, Se, N, Si, or the like, respectively.

The term "pharmaceutically acceptable" as used herein means that it may be approved or is preferably approved by a regulatory agency of a Federal or a state government or listed in the U.S. Pharmacopoeia or other generally recognized pharmacopeia for use in animals, more specifically in humans, since the significant toxic effect can be avoided when used with a common medicinal dosage.

The term "pharmaceutically acceptable salt" as used herein refers to a salt of a compound of the present invention that is pharmaceutically acceptable and has a preferable biological or pharmacological activity of a parent compound. Examples of such salts include, but are not limited to, acid addition salts formed of inorganic acids (*e.g.,* hydrochloric acid, hydrobromic acid, sulfuric acid, phosphoric acid, nitric acid, and the like), and salts formed of organic acids such as acetic acid, oxalic acid, tartaric acid, succinic acid, malic acid, fumaric acid, maleic acid, ascorbic acid, trifluoroacetic acid, benzoic acid, tannic acid, pamoic acid, alginic acid, polyglutamic acid, naphthalene sulfonic acid, naphthalene disulfonic acid, and poly-galacturonic acid. The compounds may also be administered as pharmaceutically acceptable quaternary salts known by those skilled in the art, and in particular, include chloride, bromide, iodide, -O-alkyl, toluenesulfonate, methylsulfonate, sulfonate, phosphate, or carboxylate (*e.g.,* benzoate, succinate, acetate, glycolate, maleate, malate, malate, fumarate, citrate, tartrate, ascorbate, cinnamoate, mandeloate, and diphenylacetate). The compound of the formula of the present invention may include not only pharmaceutically acceptable salts, but also all salts, hydrates, and solvates that can be prepared by typical methods.

The term "hydrate" as used herein refers to a compound of the present invention or a salt thereof that includes a stoichiometric or non-stoichiometric amount of water bound by non-covalent intermolecular forces.

The term "solvate" as used herein refers to a compound of the present invention or a salt thereof that includes a stoichiometric or non-stoichiometric amount of solvent bound by non-covalent intermolecular forces. Preferred solvents are volatile, non-toxic, and/or acceptable for administration to humans.

The term "prodrug" as used herein refers to a substance that is converted into a parent drug *in vivo.* This refers to a compound of the present invention that can hydrolyze, oxidize, or otherwise react under biological conditions (*in vitro* or *in vivo*) in order to provide an active compound, in particular the compound of the present invention. Examples of the prodrug include compounds that are biohydrolyzed to produce compounds of the present invention, including biohydrolyzable moieties such as biohydrolyzable amides, biohydrolyzable esters, biohydrolyzable carbamates, biohydrolyzable carbonates, biohydrolyzable ureides, and biohydrolyzable phosphate analogs, but are not limited to these specific embodiments. Such a prodrug includes compounds easily prepared on the basis of various known documents.

The term "isomer" as used herein refers to a compound of the present invention or a salt thereof that has the same chemical formula or molecular formula but is structurally or sterically different. Such isomers include all the structural isomers such as tautomers, R or S isomers having an asymmetric carbon center, stereoisomers such as geometric isomers (trans or cis), and enantiomers. In addition, all these isomers and mixtures thereof also fall within the scope of the present invention.

As used herein, the phrase "pharmaceutically acceptable carrier" refers to a diluent, an adjuvant, an additive or a carrier administered with a compound of the present invention.

As used herein, "prevention" refers to a reduction in the risk of acquiring a disease or disorder (*i.e.,* causing one or more clinical symptoms of the disease not to develop in an individual that is exposed to or predisposed to the disease but does not yet experience or display the symptoms of the disease).

As used herein, "treatment" refers to relieving a disease or disorder (i.e., arresting or reducing the progression of the disease or one or more clinical symptoms of the disease), improving one or more physical parameters which may not be discernible by the individual, or modulating the disease or disorder physically (*e.g.,* stabilizing discernible symptoms), mentally (*e.g.,* stabilizing physical parameters), or both.

Hereinafter, the present invention will be described in detail.

### 1. Ubiquitin Binding Moiety (UBM) Compound

According to an aspect of the present invention, there is provided a compound represented by Formulae I to IV below, or a pharmaceutically acceptable salt, isomer, solvate, hydrate, or prodrug thereof:

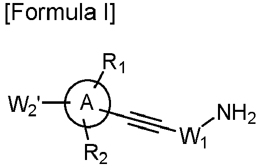

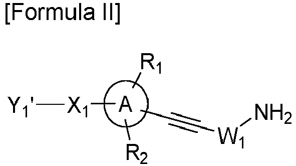

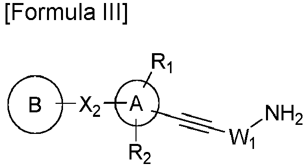

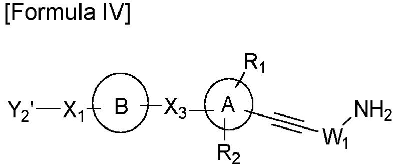

In Formula I to Formula IV above, substituents W₁, A, R₁, R₂, X₁, X₂, X₃, B, W₂', Y₁', and Y₂' may be each independently defined as follows.

W₁ above may be any one selected from the group consisting of -alkylene-, - alkylene-O-, -alkylene-OSO₂-, and -alkylene-N(R')SO₂-.

W₁ above may be unsubstituted or substituted with at least one selected from the group consisting of deuterium, alkyl, C(O)OR', C(O)N(R')(R"), alkylene-C(O)OR', alkylene-C(O)N(R')(R"), alkylene-OR', alkylene-SR', alkylene-N(R')(R"), alkylene-OP(O)(OR')(OR"), alkylene-P(O)(OR')(OR"), and alkylene-OP(O)(R')(R"). For example, W₁ above may be unsubstituted or substituted with at least one selected from the group consisting of C(O)OR', C(O)N(R')(R"), alkylene-OR', alkylene-SR', and alkylene-OP(O)(OR')(OR"). Specifically, W₁ above may be unsubstituted or substituted with at least one selected from the group consisting of alkylene-OR' and alkylene-OP(O)(OR')(OR").

A above may be any one selected from the group consisting of aryl, heteroaryl, cycloalkyl, heterocycloalkyl, and heterocycloalkenyl. For example, A above may be any one selected from the group consisting of aryl, 5- to 10-membered heteroaryl containing at least one selected from the group consisting of N, O, and S, 3- to 10-membered cycloalkyl, 3- to 10-membered heterocycloalkyl containing at least one selected from the group consisting of N, O, and S, and 3- to 10-membered heterocycloalkenyl containing at least one selected from the group consisting of N, O, and S. Specifically, A above may be any one selected from the group consisting of benzene, furan, benzofuran, pyridine, thiophene, imidazole, and oxazole.

R₁ and R₂ above may be each independently any one selected from the group consisting of hydrogen, alkyl, alkenyl, alkynyl, halogen, oxo, CN, NO₂, OR', SR', N(R')(R"), O-(alkylene)-OR', -N(R')-(alkylene)-OR', C(O)R', C(NH)R', C(S)R', C(O)OR', C(O)N(R')(R"), P(O)(R')(R"), P(O)(OR')(OR"), C(NH)N(R')(R"), C(S)OR', C(S)N(R')(R"), C(O)O-(alkylene)-OC(O)R', C(O)O-(alkylene)-methacryloyloxy, (alkylene)-OR', (alkylene)-SR', (alkylene)-N(R')(R"), (alkynylene)-N(R')(R"), (alkylene)-P(O)(R')(R")-, (alkylene)-P(O)(OR')(OR"), (alkylene)-OP(O)(R')(R"), (alkylene)-OP(O)(OR')(OR"), (alkylene)-OP(O)(benzyloxy)₂, (alkylene)-OP(O)(phenoxy)₂, (alkylene)-OP(O)(phenoxy)(NH-CH(R')C(O)OR"), (alkylene)-C(O)OR', (alkenylene)-C(O)OR', (alkynylene)-C(O)OR', (alkylene)-C(O)N(R')(R"), (alkenylene)-C(NH)N(R')(R"), (alkynylene)-C(S)N(R')(R"), (alkylene)-C(O)R', (alkylene)-C(NH)R', (alkylene)-C(S)R', aryl, heteroaryl, heterocycloalkyl, and heterocycloalkenyl. For example, R₁ and R₂ may be each independently any one selected from the group consisting of hydrogen, alkyl, alkenyl, halogen, OR', N(R')(R"), O-(alkylene)-OR', C(O)R', C(O)OR', C(O)N(R')(R"), C(NH)N(R')(R"), C(O)O-(alkylene)-OC(O)R', C(O)O-(alkylene)-methacryloyloxy, (alkylene)-OR', (alkylene)-SR', (alkylene)-N(R')(R"), (alkynylene)-N(R')(R"), (alkylene)-OP(O)(OR')(OR"), (alkylene)-OP(O)benzyloxy)₂, (alkylene)-OP(O)(phenoxy)₂, (alkylene)-OP(O)(phenoxy)(NH-CH(R')C(O)OR"), (alkylene)-C(O)OR', aryl, heteroaryl, heterocycloalkyl, and heterocycloalkenyl Specifically, R₁ and R₂ above may be each independently any one selected from the group consisting of hydrogen, alkyl, OR', C(O)R', C(O)OR', C(O)N(R')(R"), (alkylene)-OR', (alkylene)-SR', (alkylene)-OP(O)(OR')(OR"), heteroaryl, and heterocycloalkyl.

In addition, optionally R₁ and R₂ above may be each independently substituted with at least one halogen, and when R₁ and R₂ above are each independently aryl, heteroaryl, heterocycloalkyl, or heterocycloalkenyl, optionally R₁ and R₂ may be each independently substituted with alkyl or N(R')(R") unsubstituted or substituted with at least one halogen at one or more positions.

X₁ above may be a direct linkage, or may be any one selected from among X1a group consisting of -N(R')-, -O-, -S-, -C(O)-, -SO-, SO₂-, =N-O-, and -O-N=, X1b group consisting of -OC(O)-, -C(O)O-, -N(R')C(O)-, -C(O)N(R')-, -N(R')SO-, -SON(R')-, -N(R')SO₂-, and -SO₂N(R')-, and X1c group consisting of -alkylene-, -alkenylene-, and -alkynylene-, or that two or more thereof are linked to each other. For example, X₁ above may be a direct linkage, any one selected from among the X1a group and the X1b group, or that any one selected from among the X1a group and the X1b group and any one selected from the X1c group are linked in the form ofX1a-X1c orX1b-X1c. Specifically, X₁ above may be a direct linkage, or the X1a group constituting X₁ above may be composed of -N(R')- and -O-, the X1b group may be composed of -C(O)O-, -N(R')C(O)-, and -C(O)N(R')-, or the X1c group may be composed of -alkylene- and -alkynylene-.

X₂ above may be a direct linkage, or may be any one selected from among X2a group consisting of -N(R')-, -O-, -S-, -C(O)-, -SO-, SO₂-, =N-O-, and -O-N=, X2b group consisting of -OC(O)-, -N(R')C(O)-, -N(R')C(O)O-, -N(R')SO₂-O-, and -N(R')C(S)O-, X2c group consisting of-C(O)O-, -C(O)N(R')-, -O-SO₂-N(R')-, -O-C(S)N(R')-, and -O-C(O)N(R')-, and X2d group consisting of -alkylene-, -alkenylene-, and -alkynylene-, or that two or more thereof are linked to each other. For example, X₂ above may be a direct linkage, or any one selected from the X2a group, the X2b group, and the X2c group, or that two or more thereof are linked to each other, or that any one or two or more selected from the X2b group, the X2c group, and the X2d group are linked in the form of X2b-X2d-X2b, X2b-X2d-X2c, X2c-X2d-X2b, or X2c-X2d-X2c. Specifically, X₂ above may be a direct linkage, or the X2a group constituting X₂ above may be composed of -N(R')-, -O-, -C(O)-, and =N-O-, the X2b group may be composed of -N(R')C(O)-, the X2c group may be composed of -C(O)N(R')-, or the X2d group may be composed of -alkylene-, -alkenylene-, and -alkynylene-.

X₃ above may be a direct linkage, or may be any one selected from the group consisting of -alkylene-, -O-, -S-, -C(O)-, -N(R')-, -SO-, -SO₂-, and a combination thereof. For example, X₃ above may be a direct linkage, or any one selected from the group consisting of -C(O)-, -N(R')-, and a combination thereof.

B above may be any one selected from the group consisting of aryl, heteroaryl, cycloalkyl, heterocycloalkyl, and heterocycloalkenyl, which are unsubstituted or substituted with at least one selected from the group consisting of hydroxy, alkoxy, and oxo, or that two or more selected from the group consisting of aryl, heteroaryl, cycloalkyl, heterocycloalkyl, and heterocycloalkenyl, which are unsubstituted or substituted with at least one selected from the group consisting of hydroxy, alkoxy, and oxo, are directly linked, or linked via - alkylene-, -O-, -S-, -C(O)-, -N(R')-, -SO-, -SO₂-, or a combination thereof. For example, B above may be any one selected from the group consisting of aryl, 5- to 10-membered heteroaryl, 3- to 10-membered heterocycloalkyl, and 3- to 10-membered heterocycloalkenyl, which are unsubstituted or substituted with at least one selected from the group consisting of hydroxy, alkoxy, and oxo, or that two or more selected from the group consisting of aryl, 5- to 10-membered heteroaryl, 3- to 10-membered heterocycloalkyl, and 3- to 10-membered heterocycloalkenyl, which are unsubstituted or substituted with at least one selected from the group consisting of hydroxy and oxo, are directly linked, or linked via -alkylene-, -C(O)-, -N(R')-, or a combination thereof. Specifically, B above may be any one selected from the group consisting of 5- to 10-membered heteroaryl, 3- to 10-membered heterocycloalkyl, and 3- to 10-membered heterocycloalkenyl, which are unsubstituted or substituted with at least one selected from the group consisting of hydroxy and oxo, or two or more selected from the group consisting of 5- to 10-membered heteroaryl, 3- to 10-membered heterocycloalkyl, and 3- to 10-membered heterocycloalkenyl, which are unsubstituted, are directly linked, or linked via -alkylene-, -C(O)-, -N(R')-, or a combination thereof.

W₂' above may be any one selected from the group consisting of hydrogen, alkyl, alkenyl, and alkynyl. For example, W₂' above may be any one selected from the group consisting of hydrogen, alkenyl, and alkynyl. Specifically, W₂' above may be any one selected from the group consisting of hydrogen and alkynyl.

Furthermore, W₂' above may be unsubstituted or substituted with at least one selected from the group consisting of deuterium, halogen, alkyl, -alkylene-C(O)OR', alkylene-C(O)N(R')(R"), alkylene-OR', alkylene-SR', alkylene-N(R')(R"), alkylene-OP(O)(OR')(OR"), alkylene-P(O)(OR')(OR"), alkylene-OP(O)(R')(R"), -N(R')(R"), -OR', - SR', -C(O)R', -SOR', -SO₂R', =N-OR', -C(O)OR', -C(O)N(R')(R"), -OC(O)OR', - OC(O)N(R')(R"), -N(R')C(O)O(R"), -N(R')SO(R'), -SON(R')(R"), -N(R')SO₂(R"), and - SO₂N(R')(R"). For example, W₂' above may be unsubstituted or substituted with at least one selected from the group consisting of halogen, alkylene-OR', alkylene-OP(O)(OR')(OR"), -N(R')(R"), -OR', and -C(O)OR'.

Y₁' above may be any one selected from the group consisting of alkyl, alkenyl, alkynyl, (CH₃CH₂O)-(CH₂CH₂O)ₙ₋₁CH₂-, and (CH₃CH₂O)-(CH₂CH₂O)ₙ₋₁CH₂CH₂-, wherein n above may be 1-4. For example, Y₁' above may be any one selected from the group consisting of alkyl, alkenyl, alkynyl, (CH₃CH₂O)-(CH₂CH₂O)ₙ₋₁CH₂-, and (CH₃CH₂O)-(CH₂CH₂O)ₙ₋₁CH₂CH₂-, wherein n above may be 1 to 2. Specifically, Y₁' above may be any one selected from the group consisting of alkyl, alkenyl, and alkynyl.

In addition, Y₁' above may be unsubstituted or substituted with at least one selected from the group consisting of halogen, -N(R')(R"), -OR', -SR', -C(O)R', -SOR', SO₂R', =N-OR', -C(O)OR', -C(O)N(R')(R"), -OC(O)OR', -OC(O)N(R')(R"), -N(R')C(O)O(R"), -N(R')SO(R'), -SON(R')(R"), -N(R')SO₂(R"), and -SO₂N(R')(R"). Specifically, Y₁' above may be unsubstituted or substituted with at least one selected from the group consisting of halogen, -N(R')(R"), -OR', and -C(O)OR'.

Y₂' above may be any one selected from the group consisting of alkyl, alkenyl, alkynyl, heterocycloalkyl, and heterocycloalkenyl. For example, Y₂' above may be any one selected from the group consisting of alkyl, alkenyl, alkynyl, and heterocycloalkyl. Specifically, Y₂' above may be any one selected from the group consisting of alkyl and heterocycloalkyl.

In addition, Y₂' above may be unsubstituted or substituted with at least one selected from the group consisting of halogen, -N(R')(R"), -OR', -SR', -C(O)R', -SOR', SO₂R', =N-OR', -C(O)OR', -C(O)N(R')(R"), -OC(O)OR', -OC(O)N(R')(R"), -N(R')C(O)O(R"), -N(R')SO(R'), -SON(R')(R"), -N(R')SO₂(R"), and -SO₂N(R')(R"). For example, Y₂' above may be unsubstituted or substituted with at least one selected from the group consisting of halogen, -N(R')(R"), -OR', and -C(O)OR'.

Meanwhile, in the functional groups constituting the substituents, R'and R" may be each independently hydrogen or alkyl.

In particular, specific examples of the compound represented by Formula I above may be presented in Table 1 below, but are not limited thereto.

**[Table 1]**

| No. | Compound |
|---|---|
| 1-1 | 4-(3-aminoprop-1-yn-1-yl)aniline hydrochloride |
| | 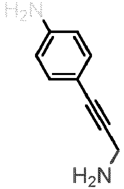 |
| 1-3 | 3-(3-aminoprop-1-yn-1-yl)aniline hydrochloride |
| | 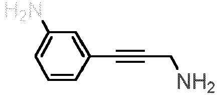 |
| 3-1 | 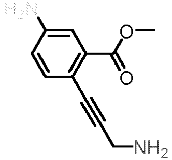 methyl 5-amino-2-(3-aminoprop-1-yn-1-yl)benzoate |
| 3-2 | 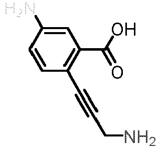 5-amino-2-(3-aminoprop-1-yn-1-yl)benzoic acid |
| 3-3 | 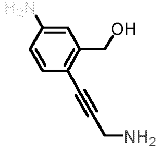 (5-amino-2-(3-aminoprop-1-yn-1-yl)phenyl)methanol |
| 3-6 | 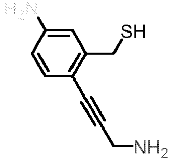 (5-amino-2-(3-aminoprop-1-yn-1-yl)phenyl)methanethiol |
| 3-7 | 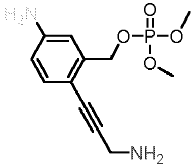 5-amino-2-(3-aminoprop-1-yn-1-yl)benzyl dimethyl phosphate |
| 3-8 | 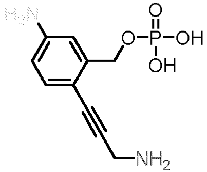 5-amino-2-(3-aminoprop-1-yn-1-yl)benzyl dihydrogen phosphate |
| 3-9 | 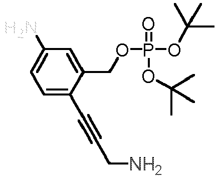 5-amino-2-(3-aminoprop-1-yn-1-yl)benzyl di-tert-butyl phosphate |
| 3-10 | 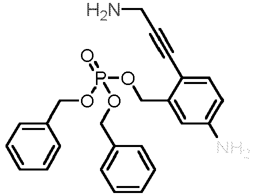 5-amino-2-(3-aminoprop-1-yn-1-yl)benzyl dibenzyl phosphate |
| 3-11 | 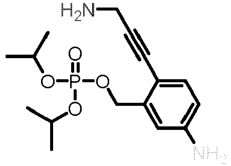 5-amino-2-(3-aminoprop-1-yn-1-yl)benzyl diisopropyl phosphate |
| 3-12 | 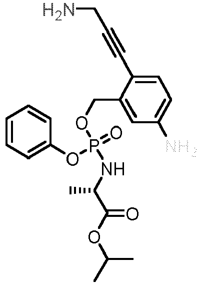 isopropyl (((5-amino-2-(3-aminoprop-1-yn-1-yl)benzyl)oxy)(phenoxy)phosphoryl)-L-alaninate |
| 3-25 | 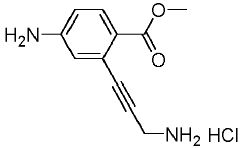 methyl 4-amino-2-(3-aminoprop-1-yn-1-yl)benzoate hydrochloride |
| 3-26 | 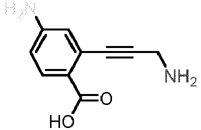 4-amino-2-(3-aminoprop-1-yn-1-yl)benzoic acid |
| 3-27 | 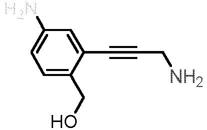 (4-amino-2-(3-aminoprop-1-yn-1-yl)phenyl)methanol |
| 3-28 | 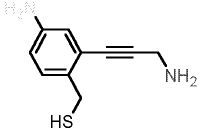 (4-amino-2-(3-aminoprop-1-yn-1-yl)phenyl)methanethiol |
| 3-30 | 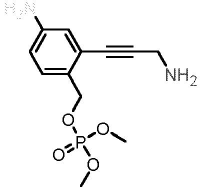 4-amino-2-(3-aminoprop-1-yn-1-yl)benzyl dimethyl phosphate |
| 3-31 | 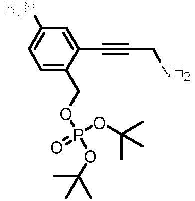 4-amino-2-(3-aminoprop-1-yn-1-yl)benzyl di-tert-butyl phosphate |
| 3-32 | 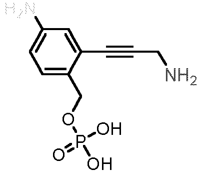 4-amino-2-(3-aminoprop-1-yn-1-yl)benzyl dihydrogen phosphate |
| 3-33 | 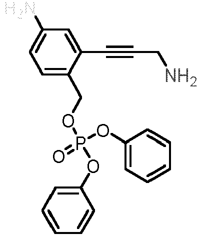 4-amino-2-(3-aminoprop-1-yn-1-yl)benzyl diphenyl phosphate |
| 3-34 | 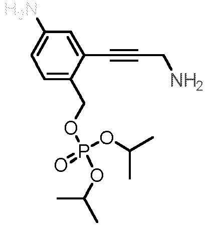 4-amino-2-(3-aminoprop-1-yn-1-yl)benzyl diisopropyl phosphate |
| 3-35 | 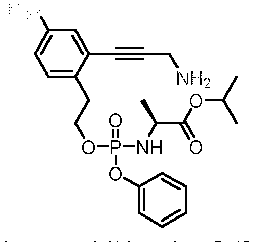 isopropyl ((4-amino-2-(3-aminoprop-1-yn-1-yl)phenethoxy)(phenoxy)phosphoryl)-L-alaninate |
| 3-49 | 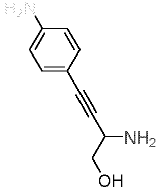 2-amino-4-(4-aminophenyl)but-3-yn-1-ol hydrochloride |
| 3-51 | 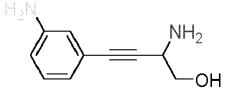 2-amino-4-(3-aminophenyl)but-3-yn-1-ol |
| 3-53 | 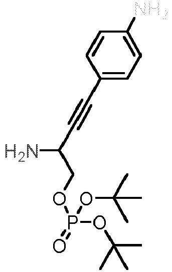 2-amino-4-(4-aminophenyl)but-3-yn-1-yl di-tert-butyl phosphate |
| 3-55 | 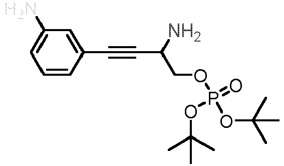 2-amino-4-(3-aminophenyl)but-3-yn-1-yl di-tert-butyl phosphate |
| 3-57 | 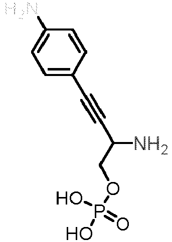 2-amino-4-(4-aminophenyl)but-3-yn-1-yl dihydrogen phosphate |
| 3-60 | 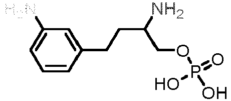 2-amino-4-(3-aminophenyl)butyl dihydrogen phosphate |
| 4-1 | 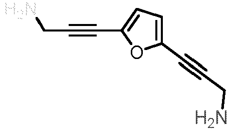 3,3'-(furan-2,5-diyl)bis(prop-2-yn-1-amine) |
| 4-10 | 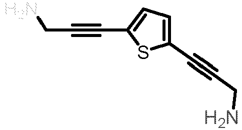 3,3'-(thiophene-2,5-diyl)bis(prop-2-yn-1-amine) |
| 4-13 | 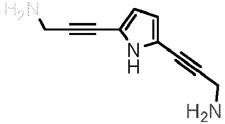 3,3'-(1H-pyrrole-2,5-diyl)bis(prop-2-yn-1-amine) |
| 4-16 | 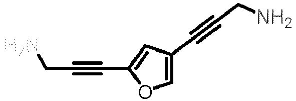 3,3'-(furan-2,4-diyl)bis(prop-2-yn-1-amine) |
| 4-19 | 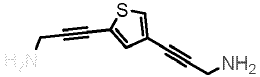 3,3'-(thiophene-2,4-diyl)bis(prop-2-yn-1-amine) |
| 4-25 | 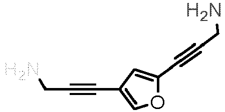 3,3'-(furan-2,4-diyl)bis(prop-2-yn-1-amine) |
| 4-28 | 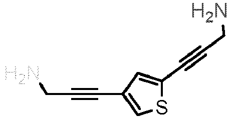 3,3'-(thiophene-2,4-diyl)bis(prop-2-yn-1-amine) |
| 4-31 | 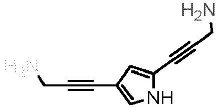 3,3'-(1H-pyrrole-2,4-diyl)bis(prop-2-yn-1-amine) |
| 4-34 | 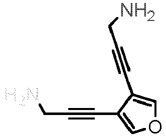 3,3'-(furan-3,4-diyl)bis(prop-2-yn-1-amine) |
| 4-40 | 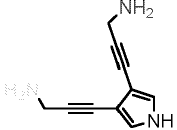 3,3'-(1H-pyrrole-3,4-diyl)bis(prop-2-yn-1-amine) |
| 4-43 | 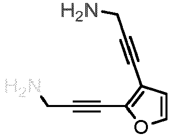 3,3'-(furan-2,3-diyl)bis(prop-2-yn-1-amine) |
| 4-52 | 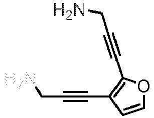 3,3'-(furan-2,3-diyl)bis(prop-2-yn-1-amine) |
| 4-61 | 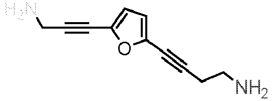 4-(5-(3-aminoprop-1-yn-1-yl)furan-2-yl)but-3-yn-1-amine |
| 4-62 | 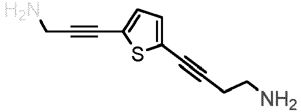 4-(5-(3-aminoprop-1-yn-1-yl)thiophen-2-yl)but-3-yn-1-amine |
| 4-63 | 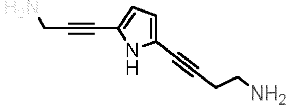 4-(5-(3-aminoprop-1-yn-1-yl)-1H-pyrrol-2-yl)but-3-yn-1-amine |
| 4-64 | 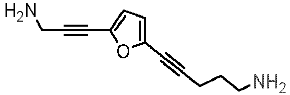 5-(5-(3-aminoprop-1-yn-1-yl)furan-2-yl)pent-4-yn-1-amine |
| 4-65 | 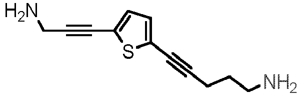 5-(5-(3-aminoprop-1-yn-1-yl)thiophen-2-yl)pent-4-yn-1-amine |
| 4-66 | 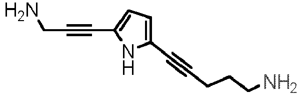 5-(5-(3-aminoprop-1-yn-1-yl)-1H-pyrrol-2-yl)pent-4-yn-1-amine |
| 4-67 | 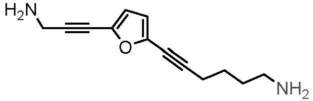 6-(5-(3-am inoprop-1-yn-1-yl)furan-2-yl)hex-5-yn-1-amine |
| 4-68 | 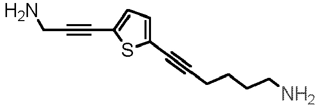 6-(5-(3-aminoprop-1-yn-1-yl)thiophen-2-yl)hex-5-yn-1-amine |
| 4-69 | 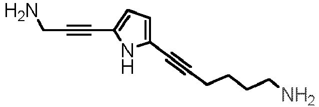 6-(5-(3-aminoprop-1-yn-1-yl)-1H-pyrrol-2-yl)hex-5-yn-1-amine |
| 4-70 | 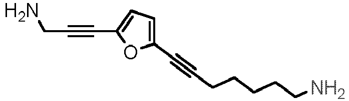 7-(5-(3-aminoprop-1-yn-1-yl)furan-2-yl)hept-6-yn-1-amine |
| 4-71 | 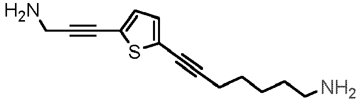 7-(5-(3-aminoprop-1-yn-1-yl)thiophen-2-yl)hept-6-yn-1-amine |
| 4-72 | 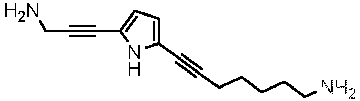 7-(5-(3-aminoprop-1-yn-1-yl)-1H-pyrrol-2-yl)hept-6-yn-1-amine |
| 4-73 | 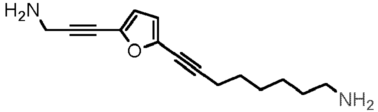 8-(5-(3-aminoprop-1-yn-1-yl)furan-2-yl)oct-7-yn-1-amine |
| 4-74 | 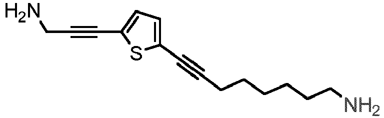 8-(5-(3-aminoprop-1-yn-1-yl)thiophen-2-yl)oct-7-yn-1-amine |
| 4-75 | 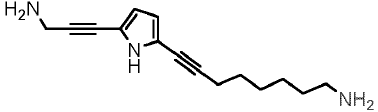 8-(5-(3-aminoprop-1-yn-1-yl)-1H-pyrrol-2-yl)oct-7-yn-1-amine |
| 4-128 | 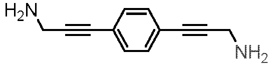 3,3'-(1,4-phenylene)bis(prop-2-yn-1-amine) |
| 4-129 | 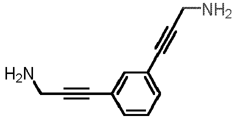 3,3'-(1,3-phenylene)bis(prop-2-yn-1-amine) |
| 4-130 | 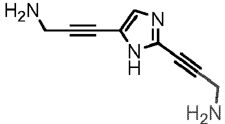 3,3'-(1H-imidazole-2,5-diyl)bis(prop-2-yn-1-amine) |
| 4-131 | 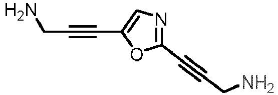 3,3'-(oxazole-2,5-diyl)bis(prop-2-yn-1-amine) |
| 4-147 | 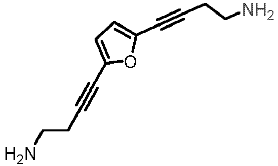 4,4'-(furan-2,5-diyl)bis(but-3-yn-1-amine) |
| 4-177 | 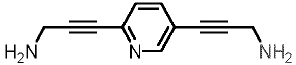 3,3'-(pyridine-2,5-diyl)bis(prop-2-yn-1-amine) |
| 4-178 | 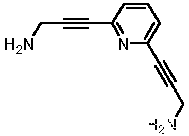 3,3'-(pyridine-2,6-diyl)bis(prop-2-yn-1-amine) |
| 4-179 | 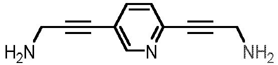 3,3'-(pyridine-2,5-diyl)bis(prop-2-yn-1-amine) |
| 4-180 | 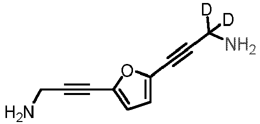 3-(5-(3-aminoprop-1-yn-1-yl)furan-2-yl)prop-2-yn-1, 1-d2-1-amine |
| 4-181 | 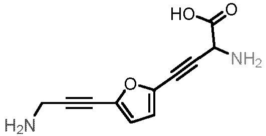 2-amino-4-(5-(3-aminoprop-1-yn-1-yl)furan-2-yl)but-3-ynoic acid |
| 4-182 | 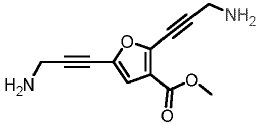 methyl 2,5-bis(3-aminoprop-1-yn-1-yl)furan-3-carboxylate |
| 4-183 | 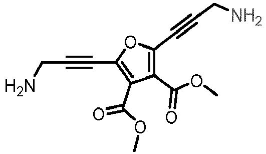 dimethyl 2,5-bis(3-aminoprop-1-yn-1-yl)furan-3,4-dicarboxylate |
| 4-184 | 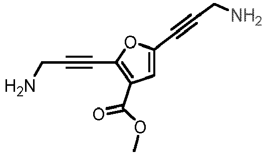 methyl 2,5-bis(3-aminoprop-1-yn-1-yl)furan-3-carboxylate |
| 4-185 | 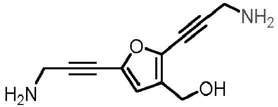 (2,5-bis(3-aminoprop-1-yn-1-yl)furan-3-yl)methanol |
| 4-186 | 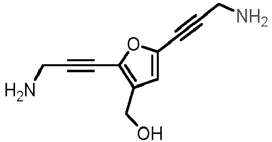 (2,5-bis(3-aminoprop-1-yn-1-yl)furan-3-yl)methanol |
| 4-187 | 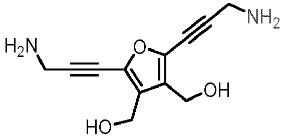 (2,5-bis(3-aminoprop-1-yn-1-yl)furan-3,4-diyl)dimethanol |
| 4-188 | 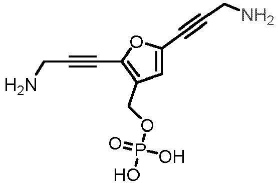 (2,5-bis(3-aminoprop-1-yn-1-yl)furan-3-yl)methyl dihydrogen phosphate |
| 4-189 | 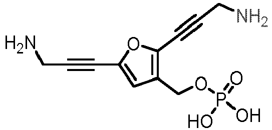 (2,5-bis(3-aminoprop-1-yn-1-yl)furan-3-yl)methyl dihydrogen phosphate |
| 4-200 | 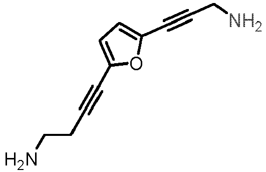 4-(5-(3-aminoprop-1-yn-1-yl)furan-2-yl)but-3-yn-1-amine |
| 4-201 | 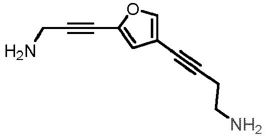 4-(5-(3-aminoprop-1-yn-1-yl)furan-3-yl)but-3-yn-1-amine |
| 4-202 | 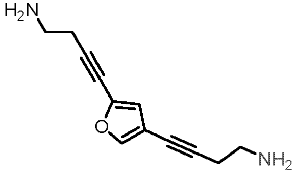 4,4'-(furan-2,4-diyl)bis(but-3-yn-1-amine) |
| 4-218 | 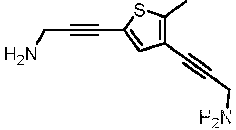 3,3'-(5-methylthiophene-2,4-diyl)bis(prop-2-yn-1-amine) |
| 4-219 | 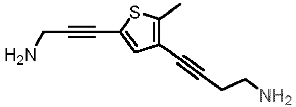 4-(5-(3-aminoprop-1-yn-1-yl)-2-methylthiophen-3-yl)but-3-yn-1-amine |
| 4-223 | 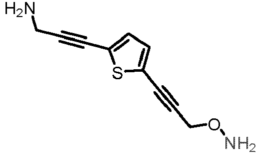 3-(5-(3-(aminooxy)prop-1-yn-1-yl)thiophen-2-yl)prop-2-yn-1-amine |
| 5-52 | 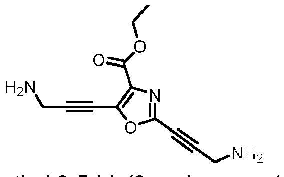 ethyl 2,b-bis(3-aminoprop-1-yn-1-yl)oxazole-4-carboxylate |
| 5-116 | 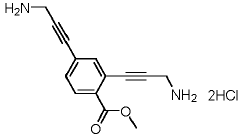 methyl 2,4-bis(3-aminoprop-1-yn-1-yl)benzoate dihydrochloride |

In particular, specific examples of the compound represented by Formula II above may be presented in Table 2 below, but are not limited thereto.

**[Table 2]**

| No. | Compound |
|---|---|
| 4-6 | 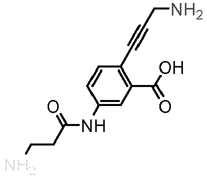 2-(3-aminoprop-1-yn-1-yl)-5-(3-aminopropanamido)benzoic acid |
| 4-109 | 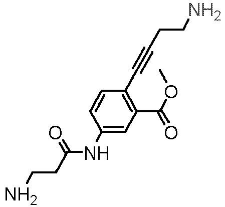 methyl 2-(4-aminobut-1-yn-1-yl)-5-(3-aminopropanamido)benzoate |
| 4-110 | 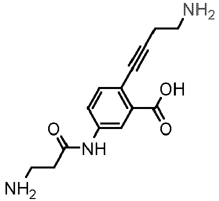 2-(4-aminobut-1-yn-1-yl)-5-(3-aminopropanamido)benzoic acid |
| 4-111 | 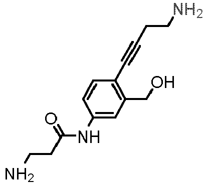 3-amino-N-(4-(4-aminobut-1-yn-1-yl)-3-(hydroxymethyl)phenyl)propanamide |
| 4-112 | 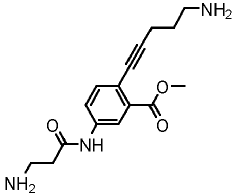 methyl 2-(5-aminopent-1-yn-1-yl)-5-(3-aminopropanamido)benzoate |
| 4-113 | 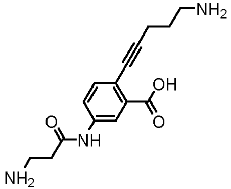 2-(5-aminopent-1-yn-1-yl)-5-(3-aminopropanamido)benzoic acid |
| 4-114 | 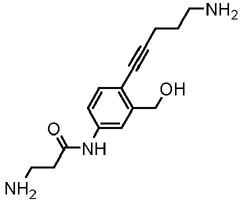 3-amino-N-(4-(5-aminopent-1-yn-1-yl)-3-(hydroxymethyl)phenyl)propanamide |
| 4-115 | 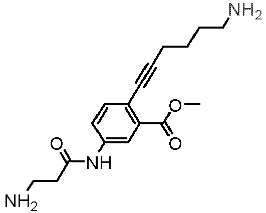 methyl 2-(6-aminohex-1-yn-1-yl)-5-(3-aminopropanamido)benzoate |
| 4-116 | 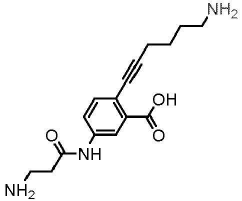 2-(6-aminohex-1-yn-1-yl)-5-(3-aminopropanamido)benzoic acid |
| 4-117 | 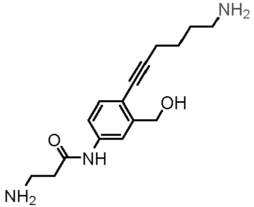 3-amino-N-(4-(6-aminohex-1-yn-1-yl)-3-(hydroxymethyl)phenyl)propanamide |
| 4-118 | 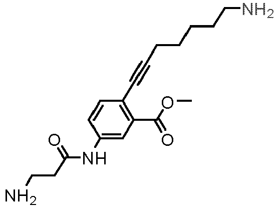 methyl 2-(7-aminohept-1-yn-1-yl)-5-(3-aminopropanamido)benzoate |
| 4-119 | 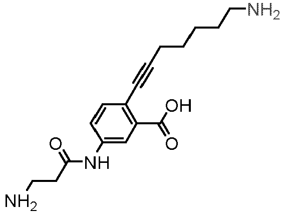 2-(7-aminohept-1-yn-1-yl)-5-(3-aminopropanamido)benzoic acid |
| 4-120 | 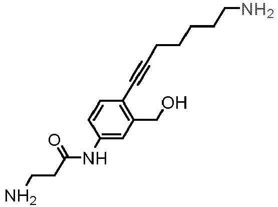 3-amino-N-(4-(7-aminohept-1-yn-1-yl)-3-(hydroxymethyl)phenyl)propanamide |
| 4-121 | 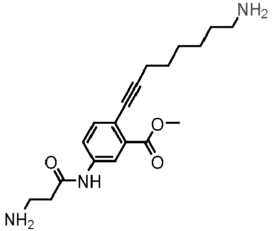 methyl 2-(8-aminooct-1-yn-1-yl)-5-(3-aminopropanamido)benzoate |
| 4-122 | 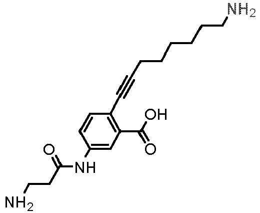 2-(8-aminooct-1-yn-1-yl)-5-(3-aminopropanamido)benzoic acid |
| 4-123 | 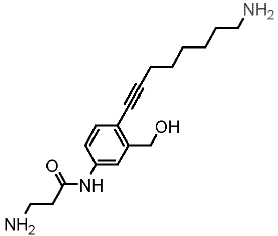 3-amino-N-(4-(8-aminooct-1-yn-1-yl)-3-(hydroxymethyl)phenyl)propanamide |
| 4-124 | 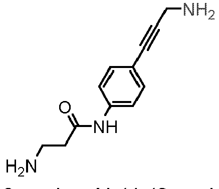 3-amino-N-(4-(3-aminoprop-1-yn-1-yl)phenyl)propanamide |
| 4-125 | 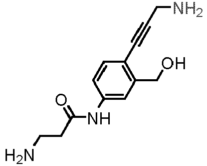 3-amino-N-(4-(3-aminoprop-1-yn-1-yl)-3-(hydroxymethyl)phenyl)propanamide |
| 4-126 | 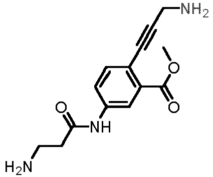 methyl 2-(3-aminoprop-1-yn-1-yl)-5-(3-aminopropanamido)benzoate |
| 4-127 | 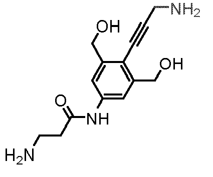 3-amino-N-(4-(3-aminoprop-1-yn-1-yl)-3,5-bis(hydroxymethyl)phenyl)propanamide |
| 4-148 | 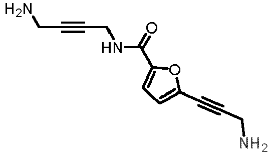 N-(4-aminobut-2-yn-1-yl)-5-(3-aminoprop-1-yn-1-yl)furan-2-carboxamide |
| 4-149 | 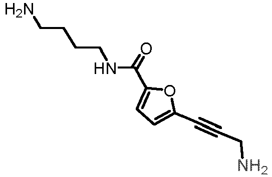 N-(4-aminobutyl)-5-(3-aminoprop-1-yn-1-yl)furan-2-carboxamide |
| 4-150 | 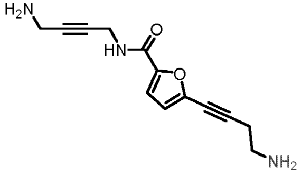 5-(4-aminobut-1-yn-1-yl)-N-(4-aminobut-2-yn-1-yl)furan-2-carboxamide |
| 4-161 | 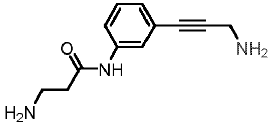 3-amino-N-(3-(3-aminoprop-1-yn-1-yl)phenyl)propanamide |
| 4-162 | 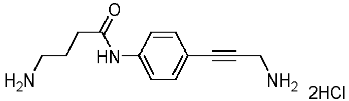 4-amino-N-(4-(3-aminoprop-1-yn-1-yl)phenyl)butanamide dihydrochloride |
| 4-163 | 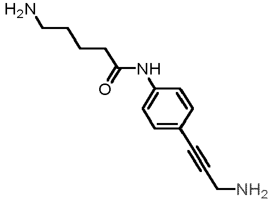 5-amino-N-(4-(3-aminoprop-1-yn-1-yl)phenyl)pentanamide |
| 4-164 | 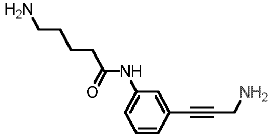 5-amino-N-(3-(3-aminoprop-1-yn-1-yl)phenyl)pentanamide |
| 4-165 | 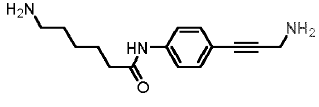 6-amino-N-(4-(3-aminoprop-1-yn-1-yl)phenyl)hexanamide |
| 4-166 | 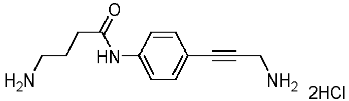 5-(3-aminoprop-1-yn-1-yl)-N-(3-aminopropyl)furan-2-carboxamide dihydrochloride |
| 4-167 | 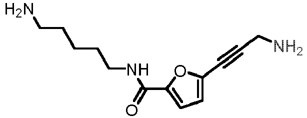 N-(5-aminopentyl)-5-(3-aminoprop-1-yn-1-yl)furan-2-carboxamide |
| 4-168 | 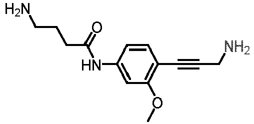 4-amino-N-(4-(3-aminoprop-1-yn-1-yl)-3-methoxyphenyl)butanamide |
| 4-169 | 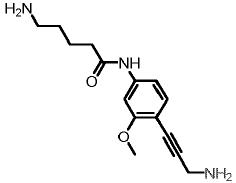 5-amino-N-(4-(3-aminoprop-1-yn-1-yl)-3-methoxyphenyl)pentanamide |
| 4-170 | 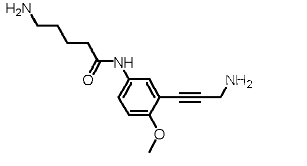 5-ammo-N-(3-(3-ammoprop-1-yn-1-yl)-4-methoxyphenyl)pentanamide |
| 4-171 | 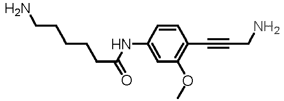 6-amino-N-(4-(3-aminoprop-1-yn-1-yl)-3-methoxyphenyl)hexanamide |
| 4-192 | 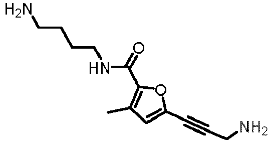 N-(4-aminobutyl)-5-(3-aminoprop-1-yn-1-yl)-3-methylfuran-2-carboxamide |
| 4-199 | 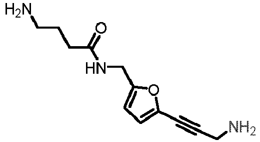 4-amino-N-((5-(3-aminoprop-1-yn-1-yl)furan-2-yl)methyl)butanamide |
| 4-203 | 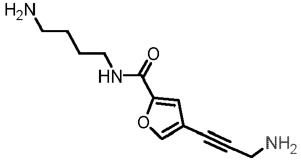 N-(4-aminobutyl)-4-(3-aminoprop-1-yn-1-yl)furan-2-carboxamide |
| 4-204 | 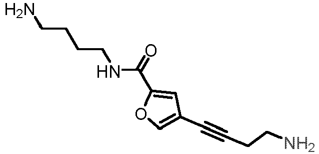 4-(4-aminobut-1-yn-1-yl)-N-(4-aminobutyl)furan-2-carboxamide |
| 4-205 | 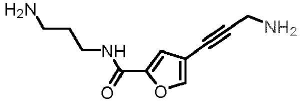 4-(3-aminoprop-1-yn-1-yl)-N-(3-aminopropyl)furan-2-carboxamide |
| 4-206 | 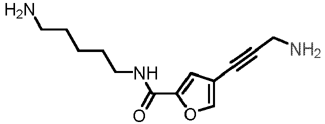 N-(5-aminopentyl)-4-(3-aminoprop-1-yn-1-yl)furan-2-carboxamide |
| 4-209 | 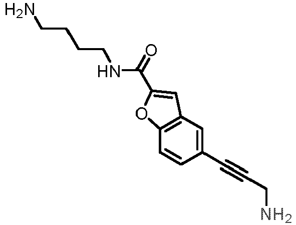 N-(4-aminobutyl)-5-(3-aminoprop-1-yn-1-yl)benzofuran-2-carboxamide |
| 4-210 | 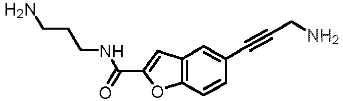 5-(3-aminoprop-1-yn-1-yl)-N-(3-aminopropyl)benzofuran-2-carboxamide |
| 4-214 | 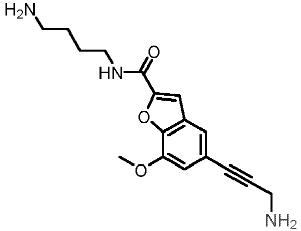 N-(4-aminobutyl)-5-(3-aminoprop-1-yn-1-yl)-7-methoxybenzofuran-2-carboxamide |
| 4-215 | 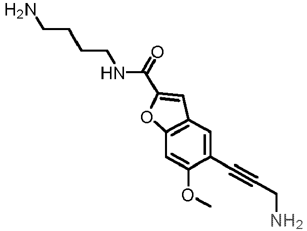 N-(4-aminobutyl)-5-(3-aminoprop-1-yn-1-yl)-6-methoxybenzofuran-2-carboxamide |
| 4-216 | 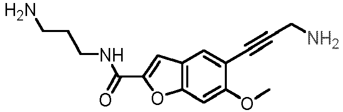 5-(3-aminoprop-1-yn-1-yl)-N-(3-aminopropyl)-6-methoxybenzofuran-2-carboxamide |
| 4-217 | 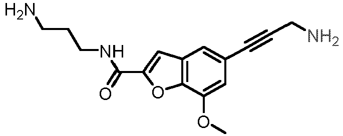 5-(3-aminoprop-1-yn-1-yl)-N-(3-aminopropyl)-7-methoxybenzofuran-2-carboxamide |
| 4-224 | 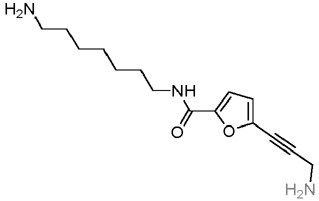 N-(7-aminoheptyl)-5-(3-aminoprop-1-yn-1-yl)furan-2-carboxamide |
| 4-225 | 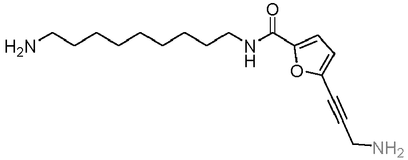 N-(9-aminononyl)-5-(3-aminoprop-1-yn-1-yl)furan-2-carboxamide |
| 4-228 | 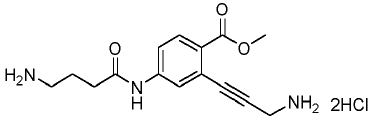 methyl 4-(4-aminobutanamido)-2-(3-aminoprop-1-yn-1-yl)benzoate dihydrochloride |
| 4-229 | 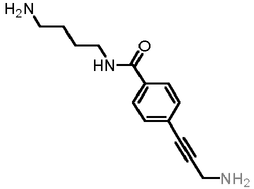 N-(4-aminobutyl)-4-(3-aminoprop-1-yn-1-yl)benzamide |
| 4-231 | 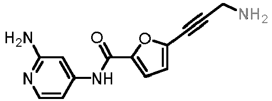 5-(3-aminoprop-1-yn-1-yl)-N-(2-aminopyridin-4-yl)furan-2-carboxamide |
| 4-232 | 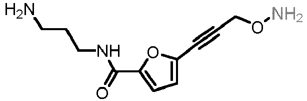 5-(3-(aminooxy)prop-1-yn-1-yl)-N-(3-aminopropyl)furan-2-carboxamide |
| 4-233 | 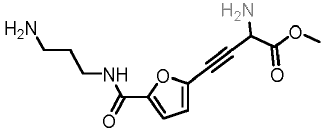 methyl 2-amino-4-(5-((3-aminopropyl)carbamoyl)furan-2-yl)but-3-ynoate |
| 4-234 | 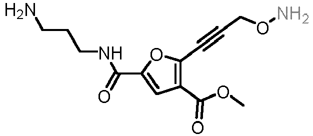 methyl 2-(3-(aminooxy)prop-1-yn-1-yl)-5-((3-aminopropyl)carbamoyl)furan-3-carboxylate |
| 4-235 | 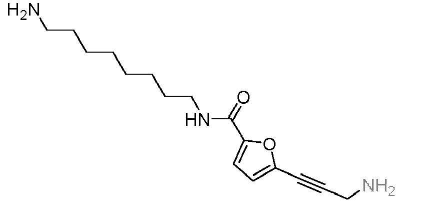 N-(8-aminooctyl)-5-(3-aminoprop-1-yn-1-yl)tu ran-2-carboxam ide |
| 5-1 | 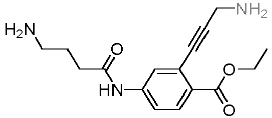 ethyl 4-(4-aminobutanamido)-2-(3-aminoprop-1-yn-1-yl)benzoate |
| 5-2 | 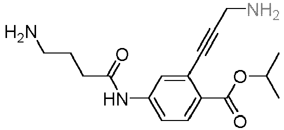 isopropyl 4-(4-aminobutanamido)-2-(3-aminoprop-1-yn-1-yl)benzoate |
| 5-3 | 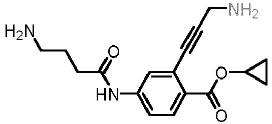 cyclopropyl 4-(4-aminobutanamido)-2-(3-aminoprop-1-yn-1-yl)benzoate |
| 5-4 | 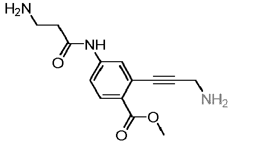 methyl 2-(3-aminoprop-1-yn-1-yl)-4-(3-aminopropanamido)benzoate |
| 5-5 | 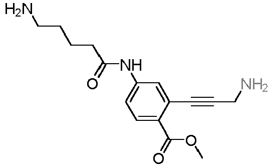 methyl 4-(5-aminopentanamido)-2-(3-aminoprop-1-yn-1-yl)benzoate |
| 5-6 | 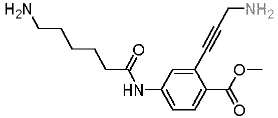 methyl 4-(6-aminohexanamido)-2-(3-aminoprop-1-yn-1-yl)benzoate |
| 5-8 | 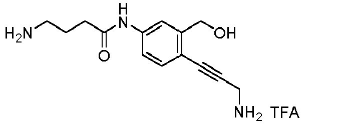 4-amino-N-(4-(3-aminoprop-1-yn-1-yl)-3-(hydroxymethyl)phenyl)butanamide trifluoroacetic acid |
| 5-9 | 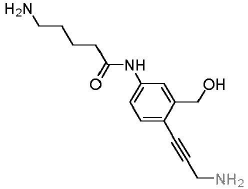 5-amino-N-(4-(3-aminoprop-1-yn-1-yl)-3-(hydroxymethyl)phenyl)pentanamide |
| 5-10 | 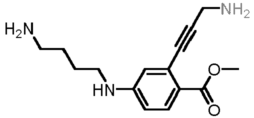 methyl 4-((4-aminobutyl)amino)-2-(3-aminoprop-1-yn-1-yl)benzoate |
| 5-11 | 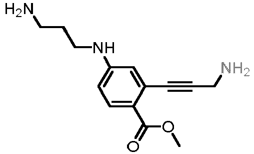 methyl 2-(3-aminoprop-1-yn-1-yl)-4-((3-aminopropyl)amino)benzoate |
| 5-12 | 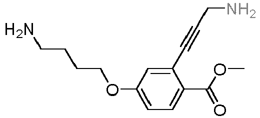 methyl 4-(4-aminobutoxy)-2-(3-aminoprop-1-yn-1-yl)benzoate |
| 5-13 | 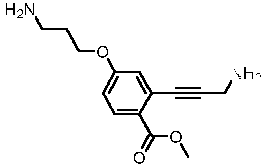 methyl 2-(3-aminoprop-1-yn-1-yl)-4-(3-aminopropoxy)benzoate |
| 5-14 | 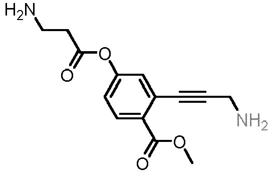 methyl 2-(3-aminoprop-1-yn-1-yl)-4-((3-aminopropanoyl)oxy)benzoate |
| 5-15 | 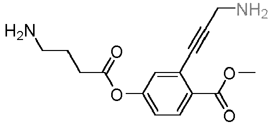 methyl 4-((4-aminobutanoyl)oxy)-2-(3-aminoprop-1-yn-1-yl)benzoate |
| 5-16 | 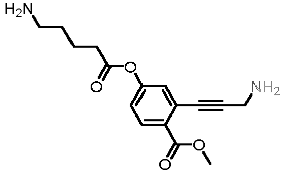 methyl 4-((5-aminopentanoyl)oxy)-2-(3-aminoprop-1-yn-1-yl)benzoate |
| 5-17 | 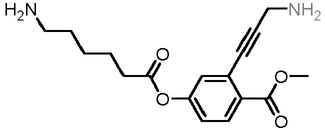 methyl 4-((6-aminohexanoyl)oxy)-2-(3-aminoprop-1-yn-1-yl)benzoate |
| 5-18 | 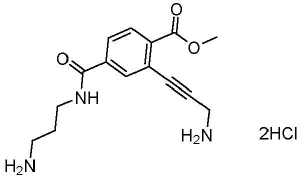 methyl 2-(3-aminoprop-1-yn-1-yl)-4-((3-aminopropyl)carbamoyl)benzoate dihydrochloride |
| 5-19 | 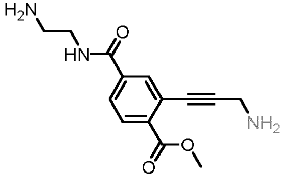 methyl 4-((2-aminoethyl)carbamoyl)-2-(3-aminoprop-1-yn-1-yl)benzoate |
| 5-20 | 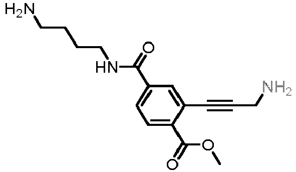 methyl 4-((4-aminobutyl)carbamoyl)-2-(3-aminoprop-1-yn-1-yl)benzoate |
| 5-21 | methyl 4-((5-aminopentyl)carbamoyl)-2-(3-aminoprop-1-yn-1-yl)benzoate |
| 5-30 | methyl 4-((4-aminobut-2-yn-1-yl)carbamoyl)-2-(3-aminoprop-1-yn-1-yl)benzoate |
| 5-31 | methyl (Z)-4-((4-aminobut-2-en-1-yl)carbamoyl)-2-(3-aminoprop-1-yn-1-yl)benzoate |
| 5-34 | methyl 2-(3-aminoprop-1-yn-1-yl)-4-(((1r,4r)-4-hydroxycyclohexyl)amino)benzoate |
| 5-35 | 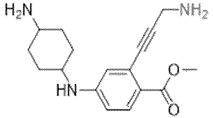 methyl 4-((4-aminocyclohexyl)amino)-2-(3-aminoprop-1-yn-1-yl)benzoate |
| 5-49 | methyl 4-(4-aminobicyclo[2.2.2]octane-1-carboxamido)-2-(3-aminoprop-1-yn-1-yl)benzoate |
| 5-50 | 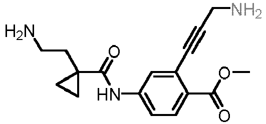 methyl 4-(1-(2-aminoethyl)cyclopropane-1-carboxamido)-2-(3-aminoprop-1-yn-1-yl)benzoate |
| 5-53 | 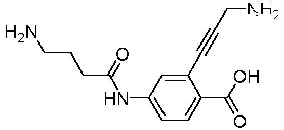 4-(4-aminobutanamido)-2-(3-aminoprop-1-yn-1-yl)benzoic acid |
| 5-54 | 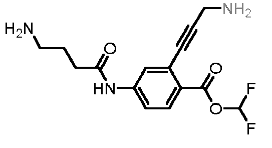 ditluoromethyl 4-(4-aminobutanamido)-2-(3-aminoprop-1-yn-1-yl)benzoate |
| 5-55 | 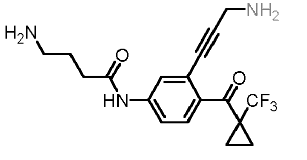 4-amino-N-(3-(3-aminoprop-1-yn-1-yl)-4-(1-(trifluoromethyl)cyclopropane-1-carbonyl)phenyl)butanamide |
| 5-56 | 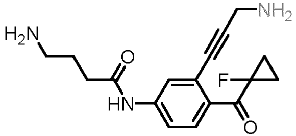 4-amino-N-(3-(3-aminoprop-1-yn-1-yl)-4-(1-fluorocyclopropane-1-carbonyl)phenyl)butanamide |
| 5-57 | 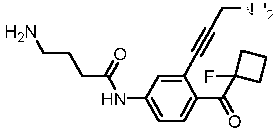 4-amino-N-(3-(3-aminoprop-1-yn-1-yl)-4-(1-fluorocyclobutane-1-carbonyl)phenyl)butanamide |
| 5-58 | 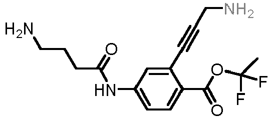 1,1-difluoroethyl 4-(4-aminobutanamido)-2-(3-aminoprop-1-yn-1-yl)benzoate |
| 5-59 | 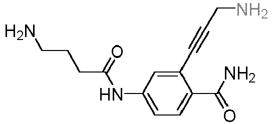 4-(4-aminobutanamido)-2-(3-aminoprop-1-yn-1-yl)benzamide |
| 5-60 | 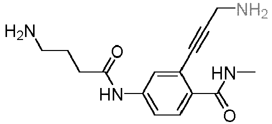 4-(4-aminobutanamido)-2-(3-aminoprop-1-yn-1-yl)-N-methylbenzamide |
| 5-61 | 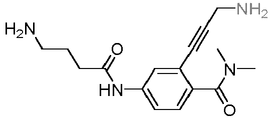 4-(4-aminobutanamido)-2-(3-aminoprop-1-yn-1-yl)-N,N-dimethylbenzamide |
| 5-62 | 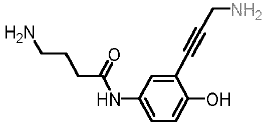 4-amino-N-(3-(3-aminoprop-1-yn-1-yl)-4-hydroxyphenyl)butanamide |
| 5-63 | 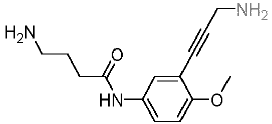 4-amino-N-(3-(3-aminoprop-1-yn-1-yl)-4-methoxyphenyl)butanamide |
| 5-64 | 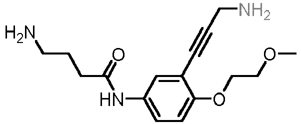 4-amino-N-(3-(3-aminoprop-1-yn-1-yl)-4-(2-methoxyethoxy)phenyl)butanamide |
| 5-65 | 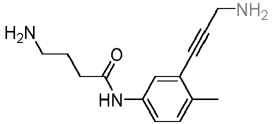 4-amino-N-(3-(3-aminoprop-1-yn-1-yl)-4-methylphenyl)butanamide |
| 5-66 | 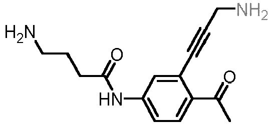 N-(4-acetyl-3-(3-aminoprop-1-yn-1-yl)phenyl)-4-aminobutanamide |
| 5-67 | 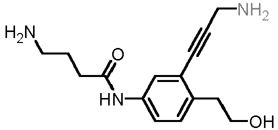 4-amino-N-(3-(3-aminoprop-1-yn-1-yl)-4-(2-hydroxyethyl)phenyl)butanamide |
| 5-68 | 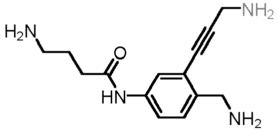 4-amino-N-(4-(aminomethyl)-3-(3-aminoprop-1-yn-1-yl)phenyl)butanamide |
| 5-69 | 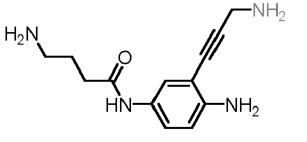 4-amino-N-(4-amino-3-(3-aminoprop-1-yn-1-yl)phenyl)butanamide |
| 5-70 | 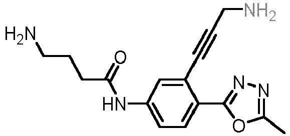 4-amino-N-(3-(3-aminoprop-1-yn-1-yl)-4-(5-methyl-1,3,4-oxadiazol-2-yl)phenyl)butanamide |
| 5-71 | 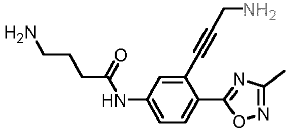 4-amino-N-(3-(3-aminoprop-1-yn-1-yl)-4-(3-methyl-1,2,4-oxadiazol-5-yl)phenyl)butanamide |
| 5-72 | 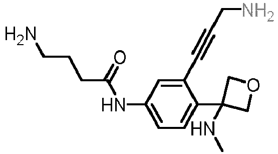 4-amino-N-(3-(3-aminoprop-1-yn-1-yl)-4-(3-(methylamino)oxetan-3-yl)phenyl)butanamide |
| 5-73 | 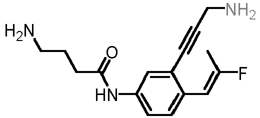 (E)-4-amino-N-(3-(3-aminoprop-1-yn-1-yl)-4-(2-fluoroprop-1-en-1-yl)phenyl)butanamide |
| 5-74 | 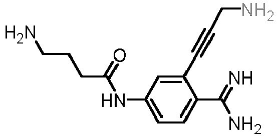 4-amino-N-(3-(3-aminoprop-1-yn-1-yl)-4-carbamimidoylphenyl)butanamide |
| 5-75 | 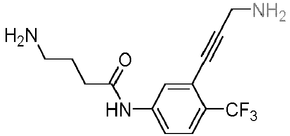 4-amino-N-(3-(3-aminoprop-1-yn-1-yl)-4-(trifluoromethyl)phenyl)butanamide |
| 5-76 | 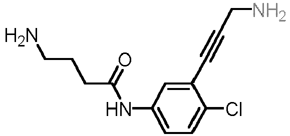 4-amino-N-(3-(3-aminoprop-1-yn-1-yl)-4-chlorophenyl)butanamide |
| 5-77 | 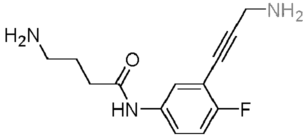 4-amino-N-(3-(3-aminoprop-1-yn-1-yl)-4-fluorophenyl)butanamide |
| 5-78 | 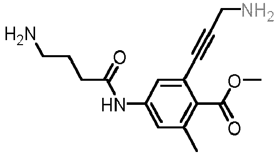 methyl 4-(4-aminobutanamido)-2-(3-aminoprop-1-yn-1-yl)-6-methylbenzoate |
| 5-79 | 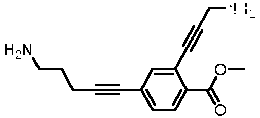 methyl 4-(5-aminopent-1-yn-1-yl)-2-(3-aminoprop-1-yn-1-yl)benzoate |
| 5-80 | 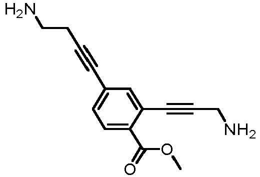 methyl 4-(4-aminobut-1-yn-1-yl)-2-(3-aminoprop-1-yn-1-yl)benzoate |
| 5-81 | 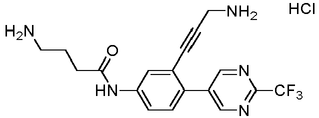 4-amino-N-(3-(3-aminoprop-1-yn-1-yl)-4-(2-(trifluoromethyl)pyrimidin-5-yl)phenyl)butanamide dihydrochloride |
| 5-82 | 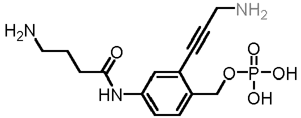 4-(4-aminobutanamido)-2-(3-aminoprop-1-yn-1-yl)benzyl dihydrogen phosphate |
| 5-83 | 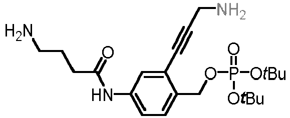 4-(4-aminobutanamido)-2-(3-aminoprop-1-yn-1-yl)benzyl di-tert-butyl phosphate |
| 5-87 | 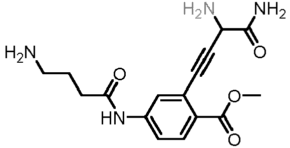 methyl 4-(4-aminobutanamido)-2-(3,4-diamino-4-oxobut-1-yn-1-yl)benzoate |
| 5-89 | 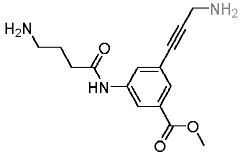 methyl 3-(4-aminobutanamido)-5-(3-aminoprop-1-yn-1-yl)benzoate |
| 5-90 | 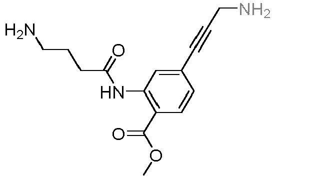 methyl 2-(4-aminobutanamido)-4-(3-aminoprop-1-yn-1-yl)benzoate |
| 5-92 | 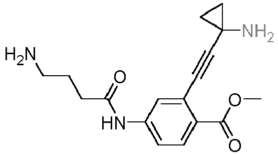 methyl 4-(4-aminobutanamido)-2-((1-aminocyclopropyl)ethynyl)benzoate |
| 5-93 | 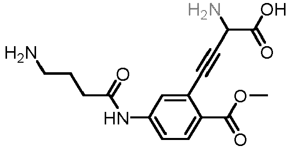 2-amino-4-(5-(4-aminobutanamido)-2-(methoxycarbonyl)phenyl)but-3-ynoic acid |
| 5-94 | 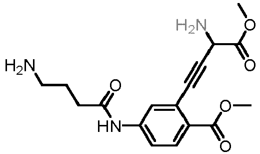 methyl 2-(3-amino-4-methoxy-4-oxobut-1-yn-1-yl)-4-(4-aminobutanamido)benzoate |
| 5-95 | 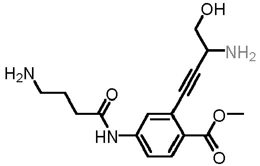 methyl 2-(3-amino-4-hydroxybut-1-yn-1-yl)-4-(4-aminobutanamido)benzoate |
| 5-96 | 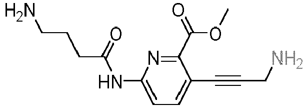 methyl 6-(4-aminobutanamido)-3-(3-aminoprop-1-yn-1-yl)picolinate |
| 5-97 | 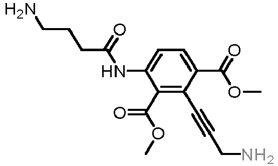 dimethyl 4-(4-aminobutanamido)-2-(3-aminoprop-1-yn-1-yl)isophthalate |
| 5-98 | 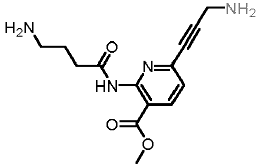 methyl 2-(4-aminobutanamido)-6-(3-aminoprop-1-yn-1-yl)nicotinate |
| 5-99 | 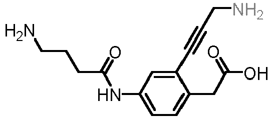 2-(4-(4-aminobutanamido)-2-(3-aminoprop-1-yn-1-yl)phenyl)acetic acid |
| 5-100 | 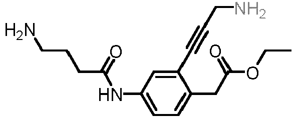 ethyl 2-(4-(4-aminobutanamido)-2-(3-aminoprop-1-yn-1-yl)phenyl)acetate |
| 5-101 | 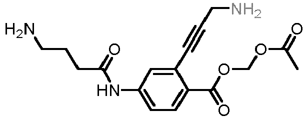 acetoxymethyl 4-(4-am inobutanam ido)-2-(3-am inoprop-1-yn-1-yl)benzoate |
| 5-102 | 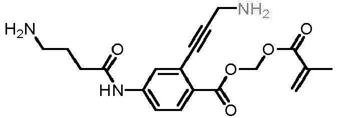 (methacryloyloxy)methyl 4-(4-aminobutanamido)-2-(3-aminoprop-1-yn-1-yl)benzoate |
| 5-103 | 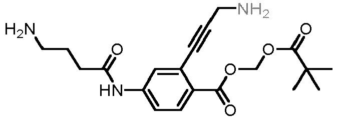 (pivaloyloxy)methyl 4-(4-aminobutanamido)-2-(3-aminoprop-1-yn-1-yl)benzoate |
| 5-104 | 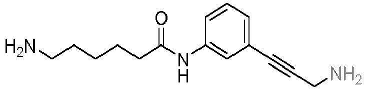 6-amino-N-(3-(3-aminoprop-1-yn-1-yl)phenyl)hexanamide |
| 5-106 | 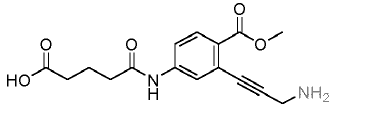 5-((3-(3-aminoprop-1-yn-1-yl)-4-(methoxycarbonyl)phenyl)amino)-5-oxopentanoic acid |
| 5-107 | 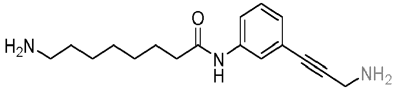 8-amino-N-(3-(3-aminoprop-1-yn-1-yl)phenyl)octanamide |
| 5-108 | 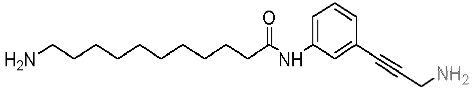 11-amino-N-(3-(3-aminoprop-1-yn-1-yl)phenyl)undecanamide |
| 5-109 | 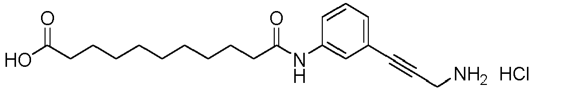 11-((3-(3-aminoprop-1-yn-1-yl)phenyl)amino)-11-oxoundecanoic acid hydrochloride |
| 5-110 | 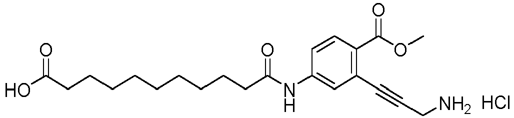 11-((3-(3-aminoprop-1-yn-1-yl)-4-(methoxycarbonyl)phenyl)amino)-11-oxoundecanoic acid hydrochloride |
| 5-113 | 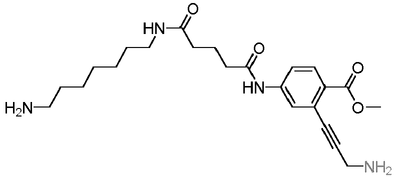 methyl 4-(5-((7-aminoheptyl)amino)-5-oxopentanamido)-2-(3-aminoprop-1-yn-1-yl)benzoate |
| 5-115 | 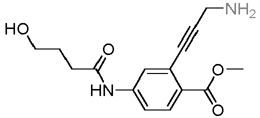 methyl 2-(3-aminoprop-1-yn-1-yl)-4-(4-hydroxybutanamido)benzoate |
| 5-121 | 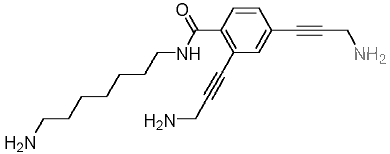 N-(7-aminoheptyl)-2,4-bis(3-aminoprop-1-yn-1-yl)benzamide |
| 5-122 | 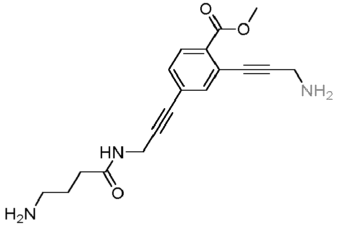 methyl 4-(3-(4-aminobutanamido)prop-1-yn-1-yl)-2-(3-aminoprop-1-yn-1-yl)benzoate |
| 5-123 | 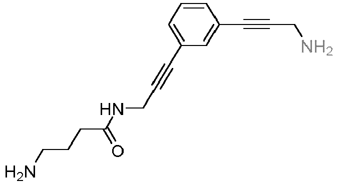 4-amino-N-(3-(3-(3-aminoprop-1-yn-1-yl)phenyl)prop-2-yn-1-yl)butanamide |
| 5-126 | 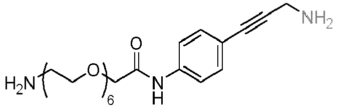 20-amino-N-(4-(3-aminoprop-1-yn-1-yl)phenyl)-3,6,9,12,15,18-hexaoxaicosanamide |
| 5-127 | 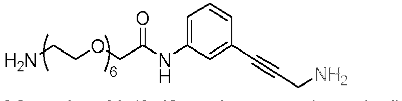 20-amino-N-(3-(3-aminoprop-1-yn-1-yl)phenyl)-3,6,9,12,15,18-hexaoxaicosanamide |
| 5-135 | 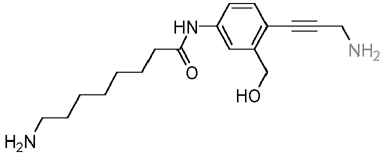 8-amino-N-(4-(3-aminoprop-1-yn-1-yl)-3-(hydroxymethyl)phenyl)octanamide |
| 5-148 | 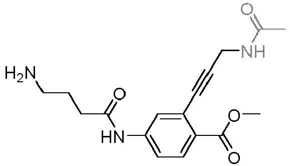 methyl 2-(3-acetamidoprop-1-yn-1-yl)-4-(4-aminobutanamido)benzoate |
| 5-149 | 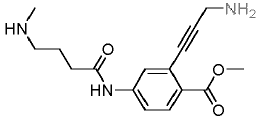 methyl 2-(3-aminoprop-1-yn-1-yl)-4-(4-(methylamino)butanamido)benzoate |
| 5-150 | 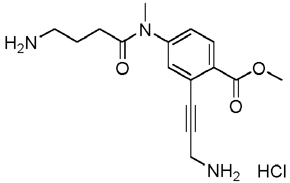 methyl 4-(4-amino-N-methylbutanamido)-2-(3-aminoprop-1-yn-1-yl)benzoate hydrochloride |
| 5-169 | 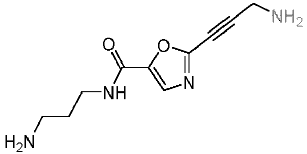 2-(3-aminoprop-1-yn-1-yl)-N-(3-aminopropyl)oxazole-5-carboxamide |
| 5-170 | 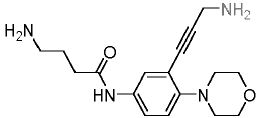 4-amino-N-(3-(3-aminoprop-1-yn-1-yl)-4-morpholinophenyl)butanamide |
| 5-171 | 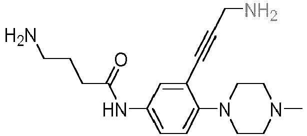 4-amino-N-(3-(3-aminoprop-1-yn-1-yl)-4-(4-methylpiperazin-1-yl)phenyl)butanamide |

In particular, specific examples of the compound represented by Formula III above may be presented in Table 3 below, but are not limited thereto.

**[Table 3]**

| No. | Compound |
|---|---|
| 4-2 | 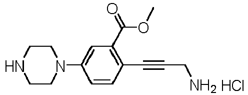 methyl 2-(3-aminoprop-1-yn-1-yl)-5-(piperazin-1-yl)benzoate hydrochloride |
| 4-3 | 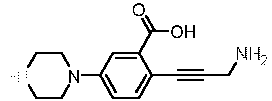 2-(3-aminoprop-1-yn-1-yl)-5-(piperazin-1-yl)benzoic acid |
| 4-4 | 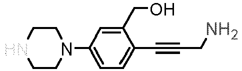 (2-(3-aminoprop-1-yn-1-yl)-5-(piperazin-1-yl)phenyl)methanol |
| 4-94 | 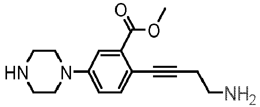 methyl 2-(4-aminobut-1-yn-1-yl)-5-(piperazin-1-yl)benzoate |
| 4-95 | 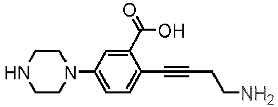 2-(4-aminobut-1-yn-1-yl)-5-(piperazin-1-yl)benzoic acid |
| 4-96 | 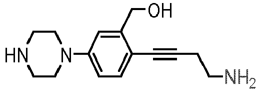 (2-(4-aminobut-1-yn-1-yl)-5-(piperazin-1-yl)phenyl)methanol |
| 4-97 | 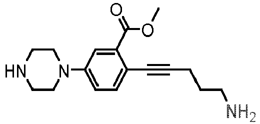 methyl 2-(5-aminopent-1-yn-1-yl)-5-(piperazin-1-yl)benzoate |
| 4-98 | 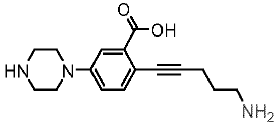 2-(5-aminopent-1-yn-1-yl)-5-(piperazin-1-yl)benzoic acid |
| 4-99 | 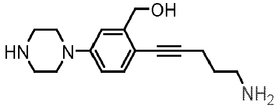 (2-(5-aminopent-1-yn-1-yl)-5-(piperazin-1-yl)phenyl)methanol |
| 4-100 | 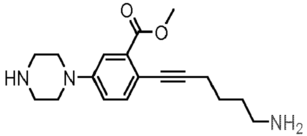 methyl 2-(6-aminohex-1-yn-1-yl)-5-(piperazin-1-yl)benzoate |
| 4-101 | 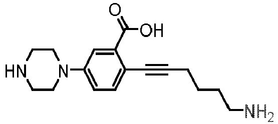 2-(6-aminohex-1-yn-1-yl)-5-(piperazin-1-yl)benzoic acid |
| 4-102 | 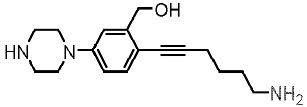 (2-(6-aminohex-1-yn-1-yl)-5-(piperazin-1-yl)phenyl)methanol |
| 4-103 | 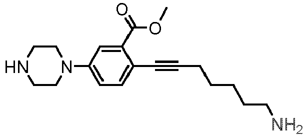 methyl 2-(7-aminohept-1-yn-1-yl)-5-(piperazin-1-yl)benzoate |
| 4-104 | 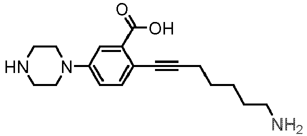 2-(7-aminohept-1-yn-1-yl)-5-(piperazin-1-yl)benzoic acid |
| 4-105 | 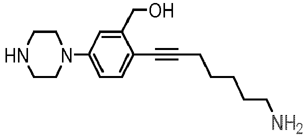 (2-(7-aminohept-1-yn-1-yl)-5-(piperazin-1-yl)phenyl)methanol |
| 4-106 | 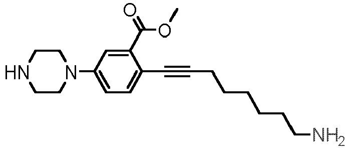 methyl 2-(8-aminooct-1-yn-1-yl)-5-(piperazin-1-yl)benzoate |
| 4-107 | 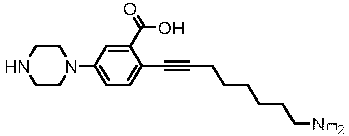 2-(8-aminooct-1-yn-1-yl)-5-(piperazin-1-yl)benzoic acid |
| 4-108 | 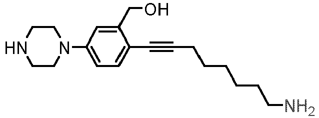 (2-(8-aminooct-1-yn-1-yl)-5-(piperazin-1-yl)phenyl)methanol |
| 4-134 | 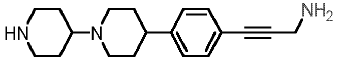 3-(4-([1,4'-bipiperidin]-4-yl)phenyl)prop-2-yn-1-amine |
| 4-135 | 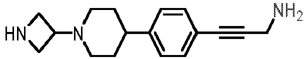 3-(4-(1-(azetidin-3-yl)piperidin-4-yl)phenyl)prop-2-yn-1-amine |
| 4-136 | 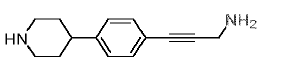 3-t4-piperidin-4-yl)phenyl)prop-2-yn-1-amine |
| 4-146 | 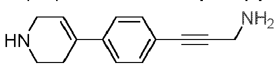 3-(4-(1,2,3,6-tetrahydropyridin-4-yl)phenyl)prop-2-yn-1-amine |
| 4-226 | 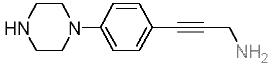 3-(4-(piperazin-1-yl)phenyl)prop-2-yn-1-amine |
| 4-227 | 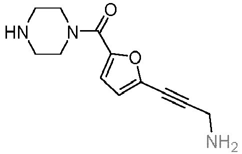 (5-(3-aminoprop-1-yn-1-yl)furan-2-yl)(piperazin-1-yl)methanone |
| 5-7 | 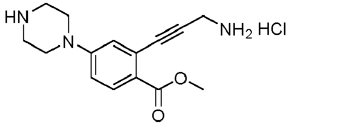 methyl 2-(3-aminoprop-1-yn-1-yl)-4-(piperazin-1-yl)benzoate hydrochloride |
| 5-22 | 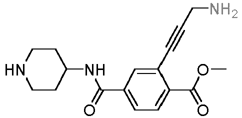 methyl 2-(3-aminoprop-1-yn-1-yl)-4-(piperidin-4-ylcarbamoyl)benzoate |
| 5-23 | 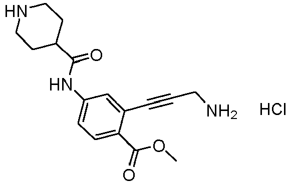 methyl 2-(3-aminoprop-1-yn-1-yl)-4-(piperidine-4-carboxamido)benzoate hydrochloride |
| 5-24 | 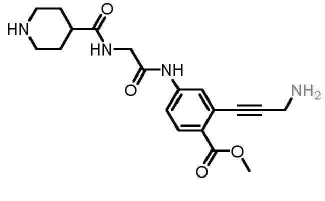 methyl 2-(3-aminoprop-1-yn-1-yl)-4-(2-(piperidine-4-carboxamido)acetamido)benzoate |
| 5-25 | 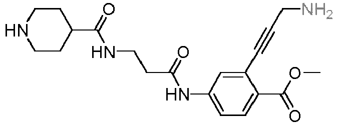 methyl 2-(3-aminoprop-1-yn-1-yl)-4-(3-(piperidine-4-carboxamido)propanamido)benzoate |
| 5-26 | 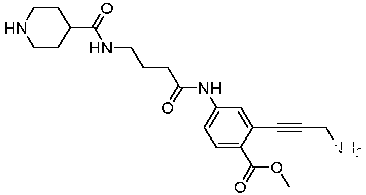 methyl 2-(3-aminoprop-1-yn-1-yl)-4-(4-(piperidine-4-carboxamido)butanamido)benzoate |
| 5-27 | 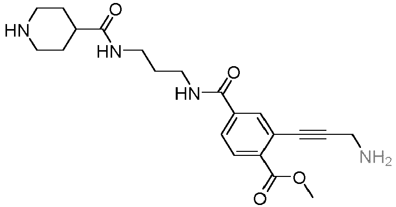 methyl 2-(3-aminoprop-1-yn-1-yl)-4-((3-(piperidine-4-carboxamido)propyl)carbamoyl)benzoate |
| 5-28 | 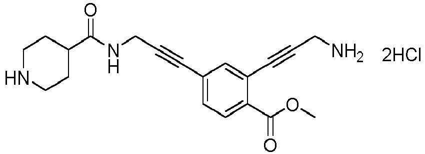 methyl 2-(3-aminoprop-1-yn-1-yl)-4-(3-(piperidine-4-carboxamido)prop-1-yn-1-yl)benzoate dihydrochloride |
| 5-29 | 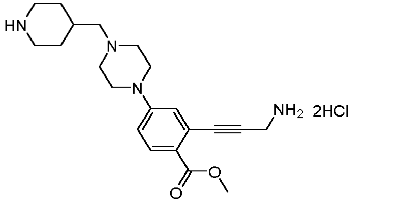 methyl 2-(6-aminoprop-1-yn-1-yl)-4-(4-(piperidin-4-ylmethyl)piperazin-1-yl)benzoate dihydrochloride |
| 5-36 | 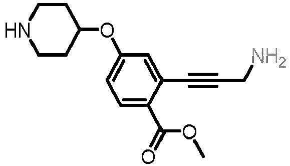 methyl 2-(3-aminoprop-1-yn-1-yl)-4-(piperidin-4-yloxy)benzoate |
| 5-37 | 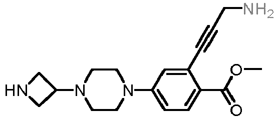 methyl 2-(3-aminoprop-1-yn-1-yl)-4-(4-(azetidin-3-yl)piperazin-1-yl)benzoate |
| 5-38 | 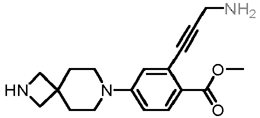 methyl 2-(3-aminoprop-1-yn-1-yl)-4-(2,7-diazaspiro[3.5]nonan-7-yl)benzoate |
| 5-39 | 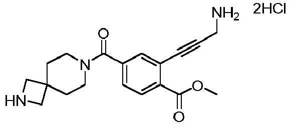 methyl 2-(3-aminoprop-1-yn-1-yl)-4-(2,7-diazaspiro[3.5]nonane-7-carbonyl)benzoate dihydrochloride |
| 5-40 | 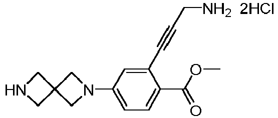 methyl 2-(3-aminoprop-1-yn-1-yl)-4-(2,6-diazaspiro[3.3]heptan-2-yl)benzoate dihydrochloride |
| 5-41 | 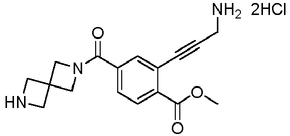 methyl 2-(3-aminoprop-1-yn-1-yl)-4-(2,6-diazaspiro[3.3]heptane-2-carbonyl)benzoate dihydrochloride |
| 5-42 | 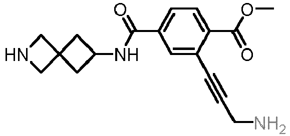 methyl 4-((2-azaspiro[3.3]heptan-6-yl)carbamoyl)-2-(3-aminoprop-1-yn-1-yl)benzoate |
| 5-43 | 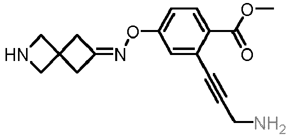 methyl 4-(((2-azaspiro[3.3]heptan-6-ylidene)amino)oxy)-2-(3-aminoprop-1-yn-1-yl)benzoate |
| 5-44 | 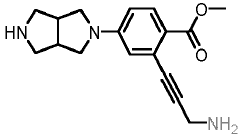 methyl 2-(3-aminoprop-1-yn-1-yl)-4-(hexahydropyrrolo[3,4-c]pyrrol-2(1H)-yl)benzoate |
| 5-45 | 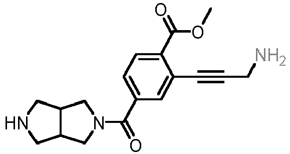 methyl 2-(3-aminoprop-1-yn-1-yl)-4-(octahydropyrrolo[3,4-c]pyrrole-2-carbonyl)benzoate |
| 5-46 | 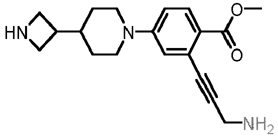 methyl 2-(3-aminoprop-1-yn-1-yl)-4-(4-(azetidin-3-yl)piperidin-1-yl)benzoate |
| 5-47 | 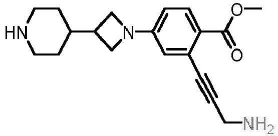 methyl 2-(3-aminoprop-1-yn-1-yl)-4-(3-(piperidin-4-yl)azetidin-1-yl)benzoate |
| 5-48 | 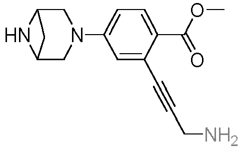 methyl 2-(3-aminoprop-1-yn-1-yl)-4-(3,6-diazabicyclo[3.1.1]heptan-3-yl)benzoate |
| 5-85 | 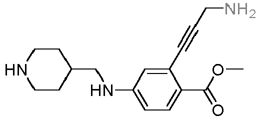 methyl 2-(3-aminoprop-1-yn-1-yl)-4-((piperidin-4-ylmethyl)amino)benzoate |
| 5-86 | 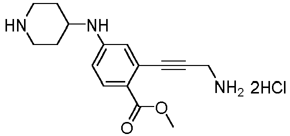 methyl 2-(3-aminoprop-1-yn-1-yl)-4-(piperidin-4-ylamino)benzoate dihydrochloride |
| 5-88 | 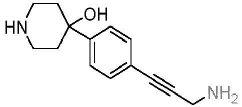 4-(4-(3-aminoprop-1-yn-1-yl)phenyl)piperidin-4-ol |
| 5-112 | 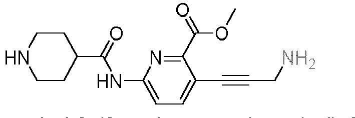 methyl 3-(3-aminoprop-1-yn-1-yl)-6-(piperidine-4-carboxamido)picolinate |
| 5-118 | 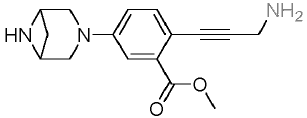 methyl 2-(3-aminoprop-1-yn-1-yl)-5-(3,6-diazabicyclo[3.1.1]heptan-3-yl)benzoate |
| 5-119 | 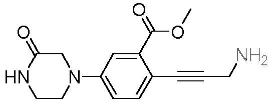 methyl 2-(3-aminoprop-1-yn-1-yl)-5-(3-oxopiperazin-1-yl)benzoate |
| 5-120 | 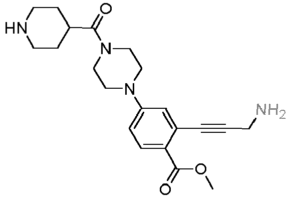 methyl 2-(3-aminoprop-1-yn-1-yl)-4-(4-(piperidine-4-carbonyl)piperazin-1-yl)benzoate |
| 5-124 | 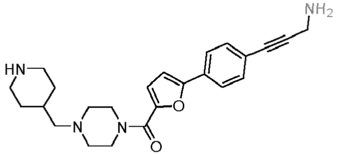 (5-(4-(3-aminoprop-1-yn-1-yl)phenyl)furan-2-yl)(4-(piperidin-4-ylmethyl)piperazin-1-yl)methanone |
| 5-125 | 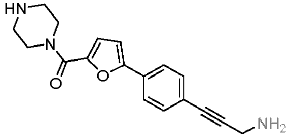 (5-(4-(3-aminoprop-1-yn-1-yl)phenyl)furan-2-yl)(piperazin-1-yl)methanone |
| 5-129 | 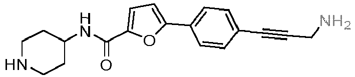 5-(4-(3-aminoprop-1-yn-1-yl)phenyl)-N-(piperidin-4-yl)furan-2-carboxamide |
| 5-131 | 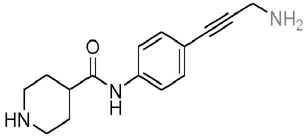 N-(4-(3-aminoprop-1-yn-1-yl)phenyl)piperidine-4-carboxamide |
| 5-134 | 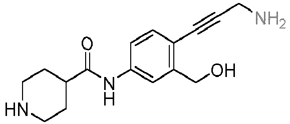 N-(4-(3-aminoprop-1-yn-1-yl)-3-(hydroxymethyl)phenyl)piperidine-4-carboxamide |
| 5-139 | 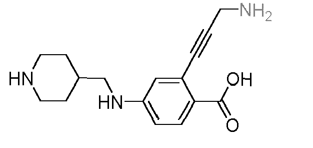 2-(3-aminoprop-1-yn-1-yl)-4-((piperidin-4-ylmethyl)amino)benzoic acid |
| 5-140 | 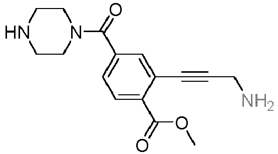 methyl 2-(3-aminoprop-1-yn-1-yl)-4-(piperazine-1-carbonyl)benzoate |
| 5-152 | 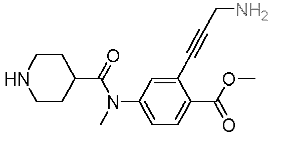 methyl 2-(3-aminoprop-1-yn-1-yl)-4-(N-methylpiperidine-4-carboxamido)benzoate |
| 5-153 | 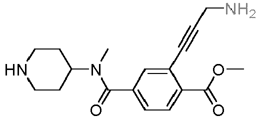 methyl 2-(3-aminoprop-1-yn-1-yl)-4-(methyl(piperidin-4-yl)carbamoyl)benzoate |
| 5-154 | 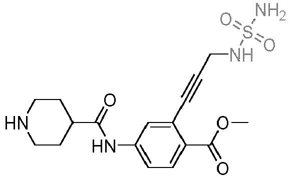 methyl 4-(piperidine-4-carboxamido)-2-(3-(sulfamoylamino)prop-1-yn-1-yl)benzoate |
| 5-155 | 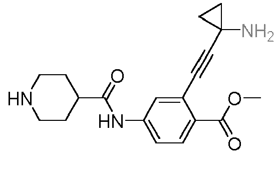 methyl 2-((1-aminocyclopropyl)ethynyl)-4-(piperidine-4-carboxamido)benzoate |
| 5-156 | 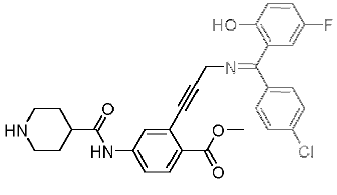 methyl 2-(3-(((4-chlorophenyl)(5-fluoro-2-hydroxyphenyl)methylene)amino)prop-1-yn-1-yl)-4-(piperidine-4-carboxamido)benzoate |
| 5-157 | 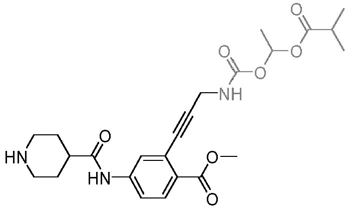 methyl 2-(3-(((1-(isobutyryloxy)ethoxy)carbonyl)amino)prop-1-yn-1-yl)-4-(piperidine-4-carboxamido)benzoate |
| 5-158 | 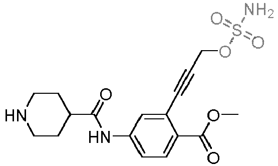 methyl 4-(piperidine-4-carboxamido)-2-(3-(sulfamoyloxy)prop-1-yn-1-yl)benzoate |
| 5-159 | 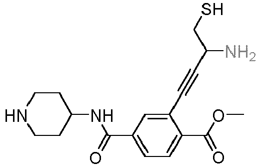 methyl 2-(3-amino-4-mercaptobut-1-yn-1-yl)-4-(piperidin-4-ylcarbamoyl)benzoate |
| 5-160 | 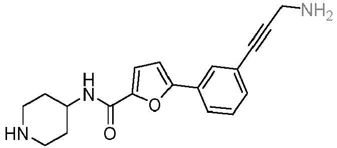 5-(3-(3-aminoprop-1-yn-1-yl)phenyl)-N-(piperidin-4-yl)furan-2-carboxamide |
| 5-162 | 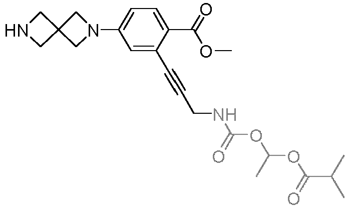 methyl 2-(3-(((1-(isobutyryloxy)ethoxy)carbonyl)amino)prop-1-yn-1-yl)-4-(2,6-diazaspiro[3.3]heptan-2-yl)benzoate |
| 5-167 | 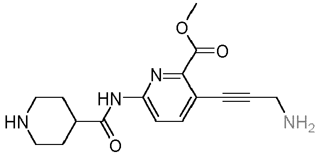 methyl 3-(3-aminoprop-1-yn-1-yl)-6-(piperidine-4-carboxamido)picolinate |
| 5-168 | 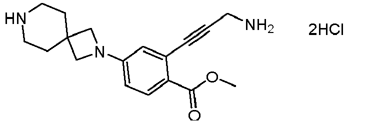 methyl 2-(3-aminoprop-1-yn-1-yl)-4-(2,7-diazaspiro[3.5]nonan-2-yl)benzoate dihydrochloride |

In particular, specific examples of the compound represented by Formula IV above may be presented in Table 4 below, but are not limited thereto.

**[Table 4]**

| No. | Compound |
|---|---|
| 4-207 | 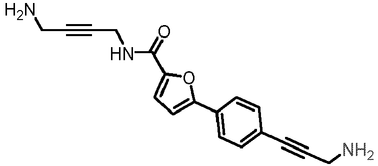 N-(4-aminobut-2-yn-1-yl)-5-(4-(3-aminoprop-1-yn-1-yl)phenyl)furan-2-carboxamide |
| 4-208 | 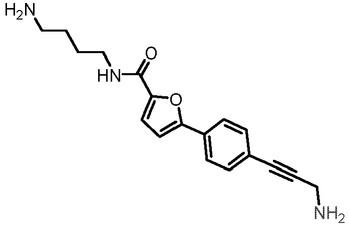 N-(4-aminobutyl)-5-(4-(3-aminoprop-1-yn-1-yl)phenyl)furan-2-carboxamide |
| 4-211 | 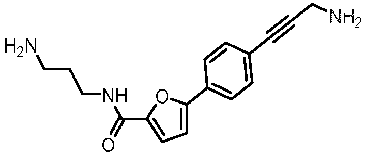 5-(4-(3-aminoprop-1-yn-1-yl)phenyl)-N-(3-aminopropyl)furan-2-carboxamide |
| 5-32 | 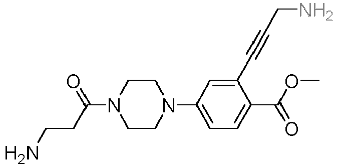 methyl 2-(3-aminoprop-1-yn-1-yl)-4-(4-(3-aminopropanoyl)piperazin-1-yl)benzoate |
| 5-33 | 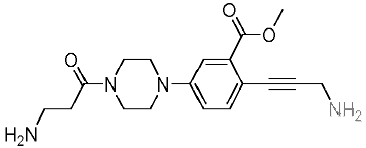 methyl 2-(3-aminoprop-1-yn-1-yl)-5-(4-(3-aminopropanoyl)piperazin-1-yl)benzoate |
| 5-51 | 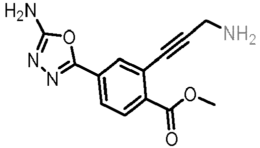 methyl 4-(5-amino-1,3,4-oxadiazol-2-yl)-2-(3-aminoprop-1-yn-1-yl)benzoate |
| 5-111 | 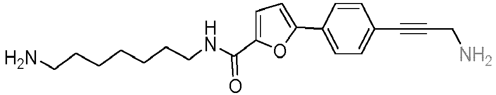 N-(7-aminoheptyl)-5-(4-(3-aminoprop-1-yn-1-yl)phenyl)furan-2-carboxamide |
| 5-114 | 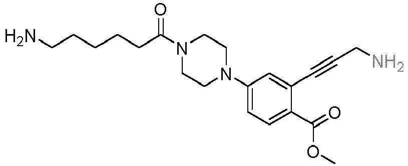 methyl 4-(4-(6-aminohexanoyl)piperazin-1-yl)-2-(3-aminoprop-1-yn-1-yl)benzoate |
| 5-117 | 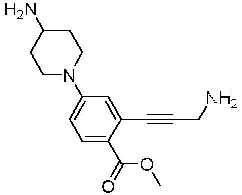 methyl 4-(4-aminopiperidin-1-yl)-2-(3-aminoprop-1-yn-1-yl)benzoate |
| 5-128 | 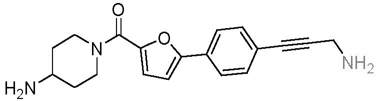 (4-aminopiperidin-1-yl)(5-(4-(3-aminoprop-1-yn-1-yl)phenyl)furan-2-yl)methanone |
| 5-130 | 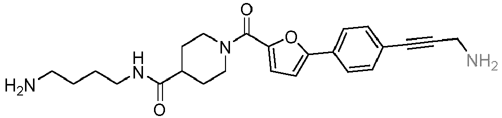 N-(4-aminobutyl)-1-(5-(4-(3-aminoprop-1-yn-1-yl)phenyl)furan-2-carbonyl)piperidine-4-carboxamide |
| 5-132 | 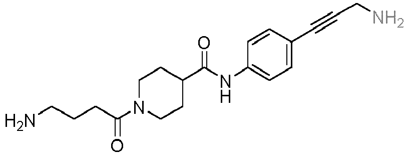 1-(4-aminobutanoyl)-N-(4-(3-aminoprop-1-yn-1-yl)phenyl)piperidine-4-carboxamide |
| 5-133 | 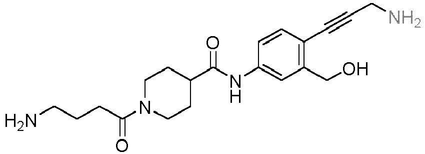 1-(4-aminobutanoyl)-N-(4-(3-aminoprop-1-yn-1-yl)-3-(hydroxymethyl)phenyl)piperidine-4-carboxamide |
| 5-136 | 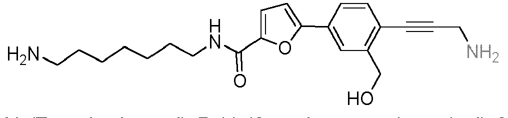 N-(7-aminoheptyl)-5-(4-(3-aminoprop-1-yn-1-yl)-3-(hydroxymethyl)phenyl)furan-2-carboxamide |
| 5-137 | 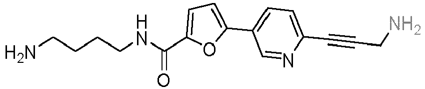 N-(4-aminobutyl)-5-(6-(3-aminoprop-1-yn-1-yl)pyridin-3-yl)furan-2-carboxamide |
| 5-138 | 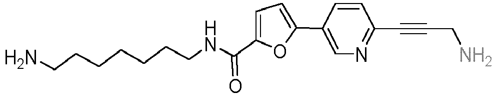 N-(7-aminoheptyl)-5-(6-(3-aminoprop-1-yn-1-yl)pyridin-3-yl)furan-2-carboxamide |
| 5-141 | 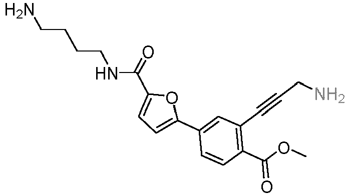 methyl 4-(5-((4-aminobutyl)carbamoyl)furan-2-yl)-2-(3-aminoprop-1-yn-1-yl)benzoate |
| 5-142 | 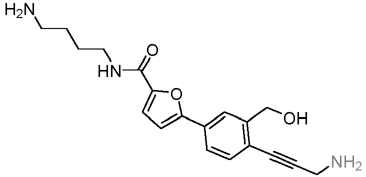 N-(4-aminobutyl)-5-(4-(3-aminoprop-1-yn-1-yl)-3-(hydroxymethyl)phenyl)furan-2-carboxamide |
| 5-143 | 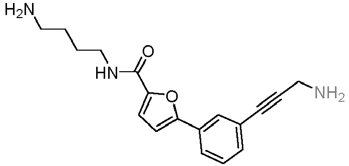 N-(4-aminobutyl)-5-(3-(3-aminoprop-1-yn-1-yl)phenyl)furan-2-carboxamide |
| 5-144 | 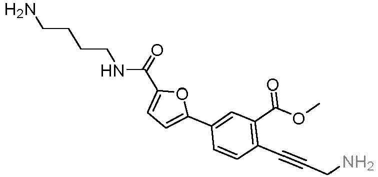 methyl 5-(5-((4-aminobutyl)carbamoyl)furan-2-yl)-2-(3-aminoprop-1-yn-1-yl)benzoate |
| 5-145 | 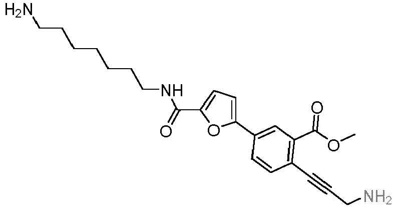 methyl 5-(5-((7-aminoheptyl)carbamoyl)furan-2-yl)-2-(3-aminoprop-1-yn-1-yl)benzoate |
| 5-164 | 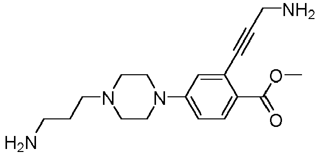 methyl 2-(3-aminoprop-1-yn-1-yl)-4-(4-(3-aminopropyl)piperazin-1-yl)benzoate |
| 5-165 | 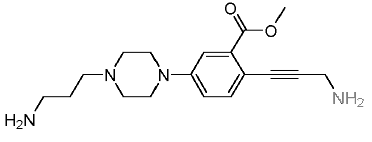 methyl 2-(3-aminoprop-1-yn-1-yl)-5-(4-(3-aminopropyl)piperazin-1-yl)benzoate |

In particular, in the compounds represented by Formulae I to IV, an amine group (-NH₂) in a propargyl group including W₁ is a binding site for ubiquitin. Therefore, the compounds represented by Formulae I to IV can be used as degraders of the target protein or polypeptide by themselves or by linking to a ligand that specifically binds to another target protein or polypeptide. As described above, the amine group (-NH₂) in the propargyl group including W₁ in the compounds represented by Formulae I to IV is a site to which ubiquitin is bound, and thus when the compounds represented by Formulae I to IV are linked to the ligand for another target protein or polypeptide, the compounds are preferably linked to a site other than the amine group (-NH₂) in the propargyl group including W₁ above.

### 2. Target Protein/Polypeptide Degrader

According to another aspect of the present invention, there is provided a compound having a chemical structure of TBM-UBM, or a pharmaceutically acceptable salt, isomer, solvate, hydrate, or prodrug thereof.

The UBM may be a chemical structure represented by Formulae V to VIII below:

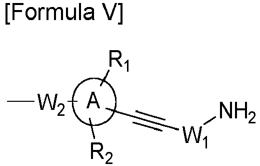

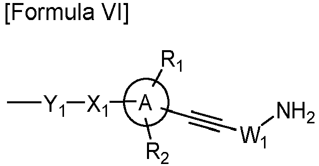

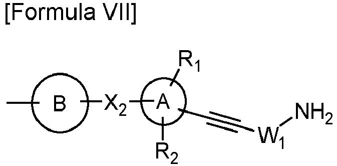

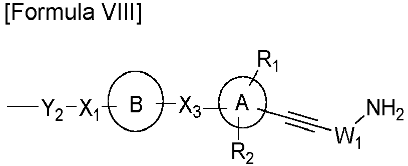

In Formulae V to VIII above, substituents W₁, A, R₁, R₂, X₁, X₂, X₃, and B are each independently the same as described above with respect to the compounds represented by Formulae I to IV.

Meanwhile, in Formulae V to VIII above, substituents W₂, Y₁, and Y₂ may be each independently defined as follows.

W₂ above may be a direct linkage, or any one selected from the group consisting of -alkylene-, -alkenylene-, and -alkynylene-. For example, W₂ above may be a direct linkage, or any one selected from the group consisting of -alkenylene- and -alkynylene-. Specifically, W₂ above may be a direct linkage, or -alkynylene-.

In addition, W₂ above may be unsubstituted or substituted with at least one selected from the group consisting of deuterium, alkyl, C(O)OR', C(O)N(R')(R"), alkylene-C(O)OR', alkylene-C(O)N(R')(R"), alkylene-OR', alkylene-SR', alkylene-N(R')(R"), alkylene-OP(O)(OR')(OR"), alkylene-P(O)(OR')(OR"), and alkylene-OP(O)(R')(R"). For example, W₂ above may be unsubstituted or substituted with at least one selected from the group consisting of alkylene-OR', alkylene-SR', and alkylene-OP(O)(OR')(OR"). Specifically, W₂ above may be unsubstituted or substituted with at least one selected from the group consisting of alkylene-OR' and alkylene-OP(O)(OR')(OR").

Y₁ above may be a direct linkage, or any one selected from the group consisting of alkylene, alkenylene, alkynylene, -(CH₂CH₂O)ₙCH₂-, and -(CH₂CH₂O)ₙCH₂CH₂-, wherein n above may be 1-4. For example, Y₁ above may be a direct linkage, or any one selected from the group consisting of alkylene, alkenylene, alkynylene, -(CH₂CH₂O)ₙCH₂-, and -(CH₂CH₂O)ₙCH₂CH₂-, wherein n above may be 1 to 2. Specifically, Y₁ above may be any one selected from the group consisting of a direct linkage, alkylene, alkenylene, and alkynylene.

Y₂ above may be any one selected from the group consisting of a direct linkage, alkylene, alkenylene, alkynylene, heterocycloalkylene, and heterocycloalkenylene. For example, Y₂ above may be any one selected from the group consisting of a direct linkage, alkylene, alkenylene, alkynylene, and heterocycloalkylene. Specifically, Y₂ above may be any one selected from the group consisting of a direct linkage, alkylene and heterocycloalkylene.

In addition, in the functional groups constituting the substituents, R'and R" may be each independently hydrogen or alkyl.

The TBM may be a moiety that binds to a target protein or polypeptide.

The target protein or polypeptide refers to a protein or polypeptide to be bound to and degraded by the compound according to the present invention, that is, a targeted protein or polypeptide. The target protein or polypeptide includes any protein or polypeptide, as well as fragments, analogs and/or homologs thereof. The target protein or polypeptide may be a protein or polypeptide having any biological function or activity such as structural, regulatory, hormonal, enzymatic, genetic, immunological, contractile, storage, transport, signal transduction and the like. For example, the target protein or polypeptide may include structural proteins, receptors, enzymes, cell surface proteins, proteins pertinent to the integrated function of a cell, including proteins involved in catalytic activity, aromatase activity, motor activity, helicase activity, metabolic processes (anabolism and catabolism), antioxidant activity, proteolysis, biosynthesis, proteins with kinase activity, oxidoreductase activity, transferase activity, hydrolase activity, lyase activity, isomerase activity, ligase activity, enzyme regulator activity, signal transducer activity, structural molecule activity, binding activity (protein, lipid carbohydrate), receptor activity, cell motility, membrane fusion, cell communication, regulation of biological processes, development, cell differentiation, response to stimulus, behavioral proteins, cell adhesion proteins, proteins involved in cell death, proteins involved in transport (including protein transporter activity, nuclear transport, ion transporter activity, channel transporter activity, carrier activity, permease activity, secretion activity, electron transporter activity), pathogenesis, chaperone regulator activity, nucleic acid binding activity, transcription regulator activity, extracellular organization and biogenesis activity, translation regulator activity. As another example, the target protein or polypeptide may include proteins or polypeptides derived from eukaryotes or prokaryotes including microbials, other antimicrobials, plant, and even virus for the determination of targets for antibiotics, as well as among numerous targets for drug therapy for humans, microorganisms, viruses, bacteria, fungi and parasites, and other animals including livestock

More specifically, the target protein or polypeptide may be the targets of a number of drugs used as therapeutic agents for humans. For example, the target protein or polypeptide includes proteins associated with an apoptosis mechanism including B7.1, B7, TINFRIm, TNFR2, NADPH oxidase, and Bcl2Bax, a MAP/ERK signal pathway-related protein including Ras and Raf, proteins associated with the NF-κB signal pathway, proteins associated with the mTOR signal pathway, proteins associated with the wnt signal pathway, proteins associated with the AKT signal pathway, proteins associated with the JAK-STAT signal pathway, NOX complexes and proteins associated with the signal pathway thereof, vascular endothelial growth factor and receptors and proteins associated with the signal pathway thereof, TLR receptors and proteins associated with the signal pathway thereof, G protein-coupled receptors and proteins associated with the signal pathway thereof, TCR-MHC complex-related receptors and proteins associated with the signal pathway thereof, cell cycle checkpoint proteins and proteins associated with the signal pathway thereof, proteins including a factor that modulates gene transcription and translation, proteins associated with gene replication and regulatory factors thereof, proteins including histone protein regulatory factors for gene expression, factors that regulate epigenetics, and the related proteins, proteins that constitute and modulate cytoskeleton, a protein that modulates intracellular transport and proteins that modulate the protein, a C5a receptor, dopamine receptors, 5HT receptors, chemokine receptors, histamine receptors, CD40/CD40L, CD23, CD124, CD4, CD5, VCAM, VLA-4 integrin, selectin, ICOS, ICAM1, IL-2 receptors, IL-1 receptors, TNF-alpha receptors, and other related cytokine receptors and chemokine receptors, oxytocin receptors, glycine receptors, noradrenaline reuptake receptors, endothelin receptors, estrogen receptors, androgen receptors, adenosine receptors, a TrkA receptor for NGF, a vitronectin receptor, an integrin receptor, neurokinins and receptors, epithelial growth factor receptors, purinergic receptors (P2Y1, P2Y2, P2Y4, P2Y6, and P2X1-7), EGF receptors, HMG-CoA reductase, PDE V phosphodiesterase type, PDE IV phosphodiesterase type 4, PDE I, PDEII, PDEIII, nitric oxide (NO) synthase, cyclo-oxygenase 1, cyclo-oxygenase 2, 5-lipoxygenase, tryptase, serine protease, thymidylate synthase, purine nucleoside phosphorylase, GAPDH trypanosomal, glycogen phosphorylase, carbonic anhydrase, analogs thereof, P-glycoproteins and MRP, tyrosine kinase, p56, Ick, Cat+ channels, inosine monophosphate dehydrogenase, interleukin-1 converting enzyme, caspases-1, glycinamide ribonucleotide formyl transferase, poly(ADP-ribose) polymerase, acetylcholinesterase, cyclin dependent kinases, microsomal transfer protein inhibitors, bile acid transport inhibitors, 5 alpha reductase inhibitors, angiotensin 11, adenosine receptors, adenosine kinase and AMP deaminase, farnesyltransferases, geranylgeranyl transferase, beta-amyloid, tyrosine kinase Her-21/neu, telomerase inhibition, cytosolic phospholipase A2 and tyrosine kinase, acetyl-CoA carboxylase, adenylosuccinate synthetase, protoporphyrinogen oxidase, and enolpyruvylshikimate-phosphate synthase, ecdysone 20-monooxygenase, ion channel of the GABA gated chloride channel, voltage-sensitive sodium channel protein, sodium channel, calcium release channel proteins, chloride channel proteins and polypeptides derived from these proteins, bacterial or viral proteins and polypeptides derived therefrom, such as HCV NS3 protease, HCV NS3 RNA helicase, rhinovirus 3C protease, herpes simplex virus-1 (HSV-I) protease, cytomegalovirus (CMV) protease, multidrug resistance (MDR) proteins, HIV 1 protease, HIV 1 integrase, influenza, neuraminidase, and hepatitis B reverse transcriptase, and proteins and polypeptides such as vasoactive intestinal peptides, prions, neuropeptide Y, and receptors. In particular, the target protein or polypeptide may be a bromodomain-containing protein, Aurora kinase A, or p38 mitogen-activated protein kinase (MAPK).

As described above, since the TBM is a moiety that binds to the target protein or polypeptide, various compounds known to bind to the target protein or polypeptide may be used as the TBM without limitation. For example, the TBM may include, but is not limited to, a compound targeting a bromodomain-containing protein, a compound targeting Aurora kinase A, a compound targeting p38 MAPK, and the like. In particular, the compound targeting the bromodomain-containing protein may be a BRD4 inhibitor, and the BRD4 inhibitor may have a chemical structure represented by Formula 1 below. In addition, the compound targeting the Aurora kinase A may be an Aurora A inhibitor, and the Aurora A inhibitor may have a chemical structure represented by Formula 2 below. In addition, the compound targeting p38 MAPK may be a p38 inhibitor, and the p38 inhibitor may have a chemical structure represented by Formula 3 below.

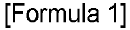

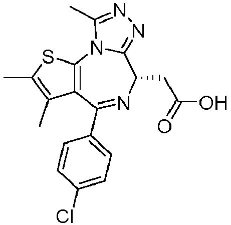

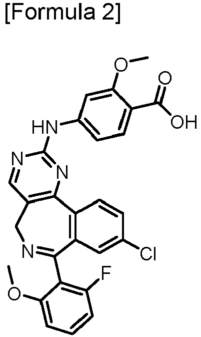

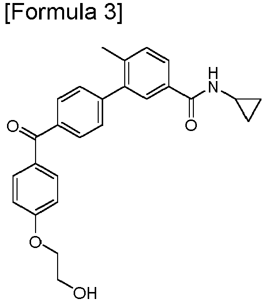

Meanwhile, the TBM and the UBM are covalently linked to each other, and the covalent bond site may be a direct linkage, or the TBM and the UBM may each be modified to form any one selected from the group consisting of -N(R')-, -O-, -C(R')(R")-O-, -S-, -C(O)-, -SO-, SO₂-, =N-O-, -O-N=, -OC(O)-, -C(O)O-, -N(R')C(O)-, -C(O)N(R')-, -OC(O)O-, - OC(O)N(R')-, -N(R')C(O)O-, -N(R')SO-, -SON(R')-, -N(R')SO₂-, and -SO₂N(R')-. For example, the covalent bond site at which the TBM and the UBM are linked to each other may be a direct linkage, or the TBM and the UBM may each be modified to form any one selected from the group consisting of -N(R')-, -O-, -C(R')(R")-O-, -OC(O)-, -OC(O)N(R')-, and -C(O)N(R')-. Specifically, the covalent bond site at which the TBM and the UBM are linked to each other may be a direct linkage, or the TBM and the UBM may each be modified to form any one selected from the group consisting of -O-, -C(R')(R")-O-, -OC(O)- - OC(O)N(R')-, and -C(O)N(R')-. Thus, for example, when the TBM is a compound having a chemical structure represented by Formula 1 above, the TBM may be bound to the UBM to be a compound having a chemical structure represented by Formula 4 below, when the TBM is a compound having a chemical structure represented by Formula 2 above, the TBM may be bound to the UBM to be a compound having a chemical structure represented by Formula 5 below, when the TBM is a compound having a chemical structure represented by Formula 3 above, the TBM may be bound to the UBM to be a compound having a chemical structure represented by Formula 6 below, and in Formula 4 to Formula 6 below, each independently, Z may be a direct linkage, or may be any one selected from the group consisting of -N(R')-, -O-, -C(R')(R")-O-, -S-, -C(O)-, -SO-, SO₂-, =N-O-, -O-N=, -OC(O)-, - C(O)O-, -N(R')C(O)-, -C(O)N(R')-, -OC(O)O-, -OC(O)N(R')-, -N(R')C(O)O-, -N(R')SO-, - SON(R')-, -N(R')SO₂-, and -SO₂N(R')-, and R' and R" above are each independently hydrogen or alkyl.

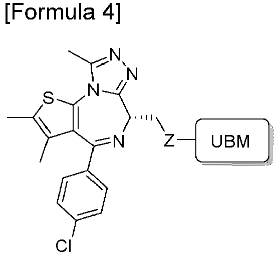

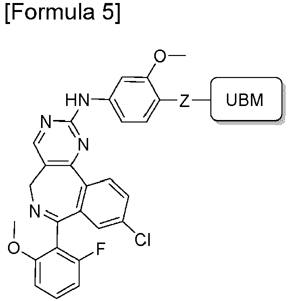

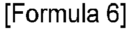

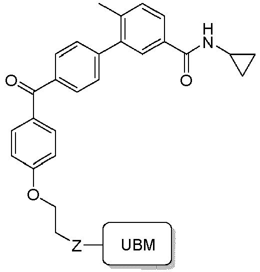

More specifically, in the compound represented by Formula V above, specific examples of the case when the compound of Formula 1 above is bound as the TBM may be presented in Table 5 below, specific examples of the case where the compound of Formula 2 above is bound as the TBM may be presented in Table 6 below, and specific examples of the case where the compound of Formula 3 above is bound as the TBM may be presented in Table 7 below, but are not limited thereto.

**[Table 5]**

| No. | Compound |
|---|---|
| 1-1' | 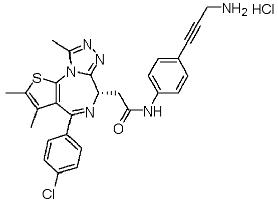 (S)-N-(4-(3-aminoprop-1-yn-1-yl)phenyl)-2-(4-(4-chlorophenyl)-2,3,9-trimethyl-6H-thieno[3,2-f][1,2,4]triazolo[4,3-a][1,4]diazepin-6-yl)acetamide hydrochloride |
| 1-3' | 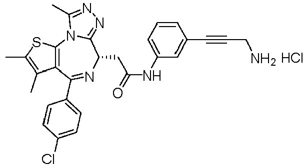 (S)-N-(3-(3-aminoprop-1-yn-1-yl)phenyl)-2-(4-(4-chlorophenyl)-2,3,9-trimethyl-6H-thieno[3,2-f][1,2,4]triazolo[4,3-a][1,4]diazepin-6-yl)acetamide hydrochloride |
| 3-1' | methyl (S)-2-(3-aminoprop-1-yn-1-yl)-5-(2-(4-(4-chlorophenyl)-2,3,9-trimethyl-6H-thieno[3,2-f][1,2,4]triazolo[4,3-a][1,4]diazepin-6-yl)acetamido)benzoate hydrochloride |
| 3-2' | (S)-2-(3-aminoprop-1-yn-1-yl)-5-(2-(4-(4-chlorophenyl)-2,3,9-trimethyl-6H-thieno[3,2-f][1,2,4]triazolo[4,3-a][1,4]diazepin-6-yl)acetamido)benzoic acid hydrochloride |
| 3-3' | (S)-N-(4-(3-aminoprop-1-yn-1-yl)-3-(hydroxymethyl)phenyl)-2-(4-(4-chlorophenyl)-2,3,9-trimethyl-6H-thieno[3,2-f][1,2,4]triazolo[4,3-a][1,4]diazepin-6-yl)acetamide hydrochloride |
| 3-6' | 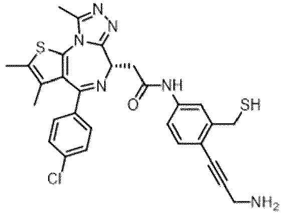 (S)-N-(4-(3-aminoprop-1-yn-1-yl)-3-(mercaptomethyl)phenyl)-2-(4-(4-chlorophenyl)-2,3,9-trimethyl-6H-thieno[3,2-f][1,2,4]triazolo[4,3-a][1,4]diazepin-6-yl)acetamide |
| 3-7' | 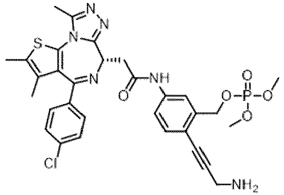 (S)-2-(3-aminoprop-1-yn-1-yl)-5-(2-(4-(4-chlorophenyl)-2,3,9-trimethyl-6H-thieno[3,2-f][1,2,4]triazolo[4,3-a][1,4]diazepin-6-yl)acetamido)benzyl dimethyl phosphate |
| 3-8' | (S)-2-(3-aminoprop-1-yn-1-yl)-5-(2-(4-(4-chlorophenyl)-2,3,9-trimethyl-6H-thieno[3,2-f][1,2,4]triazolo[4,3-a][1,4]diazepin-6-yl)acetamido)benzyl dihydrogen phosphate trifluoroacetic acid |
| 3-9' | (S)-2-(3-aminoprop-1-yn-1-yl)-5-(2-(4-(4-chlorophenyl)-2,3,9-trimethyl-6H-thieno[3,2-f][1,2,4]triazolo[4,3-a][1,4]diazepin-6-yl)acetamido)benzyl di-tert-butyl phosphate |
| 3-10' | (S)-2-(3-aminoprop-1-yn-1-yl)-5-(2-(4-(4-chlorophenyl)-2,3,9-trimethyl-6H-thieno[3,2-f][1,2,4]triazolo[4,3-a][1,4]diazepin-6-yl)acetamido)benzyl dibenzyl phosphate |
| 3-11' | (S)-2-(3-aminoprop-1-yn-1-yl)-5-(2-(4-(4-chlorophenyl)-2,3,9-trimethyl-6H-thieno[3,2-f][1,2,4]triazolo[4,3-a][1,4]diazepin-6-yl)acetamido)benzyl diisopropyl phosphate |
| 3-12' | 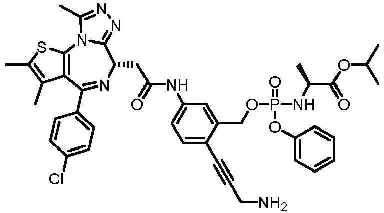 isopropyl (((2-(3-aminoprop-1-yn-1-yl)-5-(2-((S)-4-(4-chlorophenyl)-2,3,9-trimethyl-6H-thieno[3,2-f][1,2,4]triazolo[4,3-a][1,4]diazepin-6-yl)acetamido)benzyl)oxy)(phenoxy)phosphoryl)-L-alaninate |
| 3-25' | 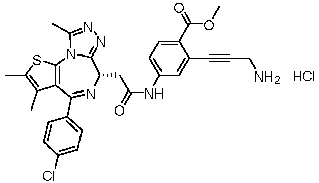 methyl (S)-2-(3-aminoprop-1-yn-1-yl)-4-(2-(4-(4-chlorophenyl)-2,3,9-trimethyl-6H-thieno[3,2-f][1,2,4]triazolo[4,3-a][1,4]diazepin-6-yl)acetamido)benzoate hydrochloride |
| 3-26' | 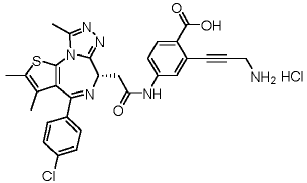 (S)-2-(3-aminoprop-1-yn-1-yl)-4-(2-(4-(4-chlorophenyl)-2,3,9-trimethyl-6H-thieno[3,2-f][1,2,4]triazolo[4,3-a][1,4]diazepin-6-yl)acetamido)benzoic acid hydrochloride |
| 3-27' | 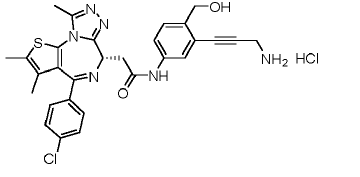 (S)-N-(3-(3-aminoprop-1-yn-1-yl)-4-(hydroxymethyl)phenyl)-2-(4-(4-chlorophenyl)-2,3,9-trimethyl-6H-thieno[3,2-f][1,2,4]triazolo[4,3-a][1,4]diazepin-6-yl)acetamide hydrochloride |
| 3-28' | 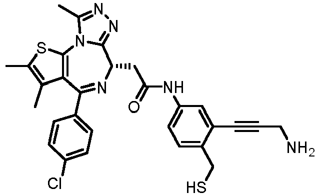 (S)-N-(3-(3-aminoprop-1-yn-1-yl)-4-(mercaptomethyl)phenyl)-2-(4-(4-chlorophenyl)-2,3,9-trimethyl-6H-thieno[3,2-f][1,2,4]triazolo[4,3-a][1,4]diazepin-6-yl)acetamide |
| 3-30' | 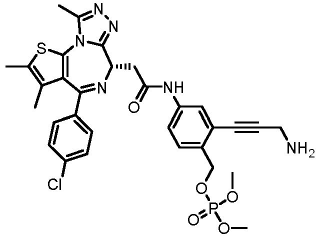 (S)-2-(3-aminoprop-1-yn-1-yl)-4-(2-(4-(4-chlorophenyl)-2,3,9-trimethyl-6H-thieno[3,2-f][1,2,4]triazolo[4,3-a][1,4]diazepin-6-yl)acetamido)benzyl dimethyl phosphate |
| 3-31' | 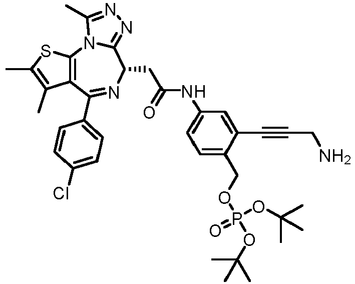 (S)-2-(3-aminoprop-1-yn-1-yl)-4-(2-(4-(4-chlorophenyl)-2,3,9-trimethyl-6H-thieno[3,2-f][1,2,4]triazolo[4,3-a][1,4]diazepin-6-yl)acetamido)benzyl di-tert-butyl phosphate |
| 3-32' | 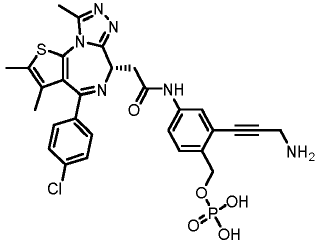 (S)-2-(3-aminoprop-1-yn-1-yl)-4-(2-(4-(4-chlorophenyl)-2,3,9-trimethyl-6H-thieno[3,2-f][1,2,4]triazolo[4,3-a][1,4]diazepin-6-yl)acetamido)benzyl dihydrogen phosphate |
| 3-33' | 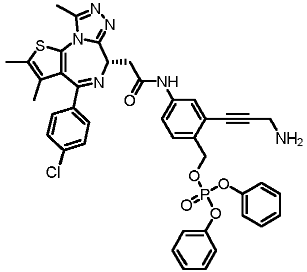 (S)-2-(3-aminoprop-1-yn-1-yl)-4-(2-(4-(4-chlorophenyl)-2,3,9-trimethyl-6H-thieno[3,2-f][1,2,4]triazolo[4,3-a][1,4]diazepin-6-yl)acetamido)benzyl diphenyl phosphate |
| 3-34' | 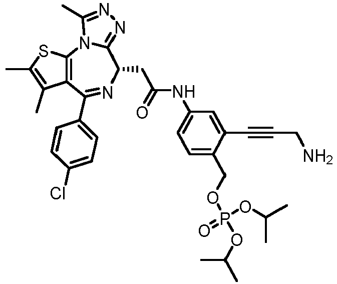 (S)-2-(3-aminoprop-1-yn-1-yl)-4-(2-(4-(4-chlorophenyl)-2,3,9-trimethyl-6H-thieno[3,2-f][1,2,4]triazolo[4,3-a][1,4]diazepin-6-yl)acetamido)benzyl diisopropyl phosphate |
| 3-35' | 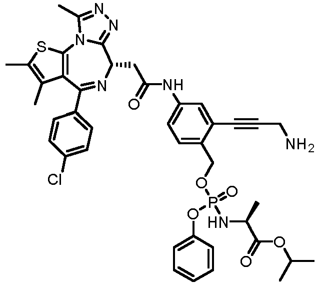 isopropyl (((2-(3-aminoprop-1-yn-1-yl)-4-(2-((S)-4-(4-chlorophenyl)-2,3,9-trimethyl-6H-thieno[3,2-f][1,2,4]triazolo[4,3-a][1,4]diazepin-6-yl)acetamido)benzyl)oxy)(phenoxy)phosphoryl)-L-alaninate |
| 3-49' | 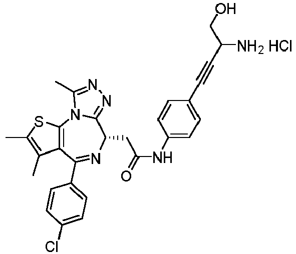 N-(4-(3-amino-4-hydroxybut-1-yn-1-yl)phenyl)-2-((S)-4-(4-chlorophenyl)-2,3,9-trimethyl-6H-thieno[3,2-f][1,2,4]triazolo[4,3-a][1,4]diazepin-6-yl)acetamide hydrochloride |
| 3-51' | 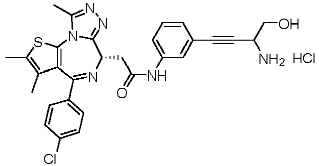 N-(3-(3-amino-4-hydroxybut-1-yn-1-yl)phenyl)-2-((S)-4-(4-chlorophenyl)-2,3,9-trimethyl-6H-thieno[3,2-f][1,2,4]triazolo[4,3-a][1,4]diazepin-6-yl)acetamide hydrochloride |
| 3-53' | 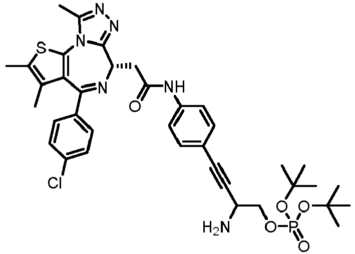 2-amino-4-(4-(2-((S)-4-(4-chlorophenyl)-2,3,9-trimethyl-6H-thieno[3,2-fl[1,2,4]triazolo[4,3-a][1,4]diazepin-6-yl)acetamido)phenyl)but-3-yn-1-yl di-tert-butyl phosphate |
| 3-55' | 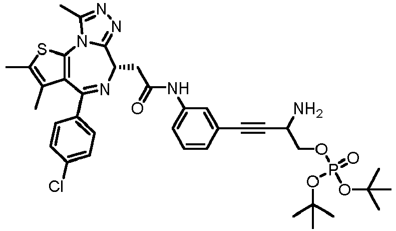 2-amino-4-(3-(2-((S)-4-(4-chlorophenyl)-2,3,9-trimethyl-6H-thieno[3,2-fl[1,2,4]triazolo[4,3-a][1,4]diazepin-6-yl)acetamido)phenyl)but-3-yn-1-yl di-tert-butyl phosphate |
| 3-57' | 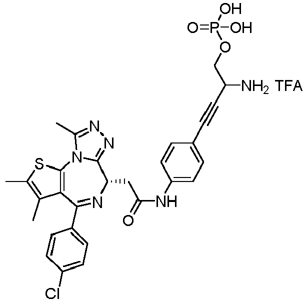 2-amino-4-(4-(2-((S)-4-(4-chlorophenyl)-2,3,9-trimethyl-6H-thieno[3,2-f][1,2,4]triazolo[4,3-a][1,4]diazepin-6-yl)acetamido)phenyl)but-3-yn-1-yl dihydrogen phosphate trifluoroacetic acid |
| 3-60' | 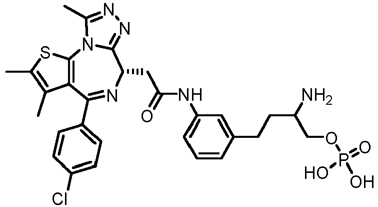 2-amino-4-(3-(2-((S)-4-(4-chlorophenyl)-2,3,9-trimethyl-6H-thieno[3,2-fl[1,2,4]triazolo[4,3-a][1,4]diazepin-6-yl)acetamido)phenyl)butyl dihydrogen phosphate |
| 4-1' | 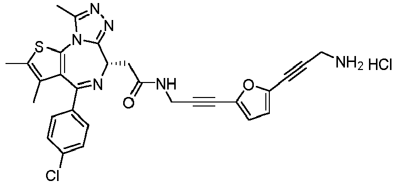 (S)-N-(3-(5-(3-aminoprop-1-yn-1-yl)furan-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide hydrochloride |
| 4-10' | 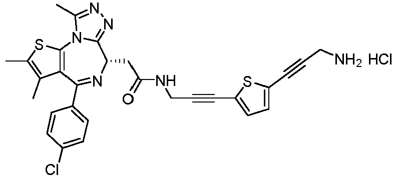 (S)-N-(3-(5-(3-aminoprop-1-yn-1-yl)thiophen-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide hydrochloride |
| 4-13' | 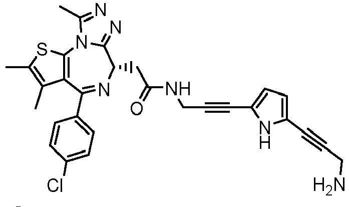 (S)-N-(3-(5-(3-aminoprop-1-yn-1-yl)-1H-pyrrol-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-16' | 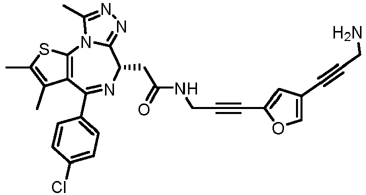 (S)-N-(3-(4-(3-aminoprop-1-yn-1-yl)furan-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-19' | 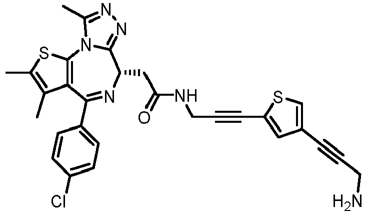 (S)-N-(3-(4-(3-aminoprop-1-yn-1-yl)thiophen-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-25' | 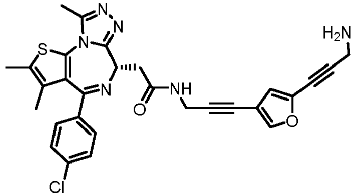 (S)-N-(3-(5-(3-aminoprop-1-yn-1-yl)furan-3-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-28' | 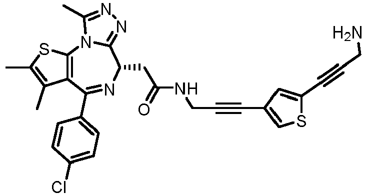 (S)-N-(3-(5-(3-aminoprop-1-yn-1-yl)thiophen-3-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-31' | 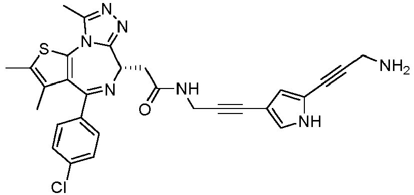 (S)-N-(3-(5-(3-aminoprop-1-yn-1-yl)-1H-pyrrol-3-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-34' | 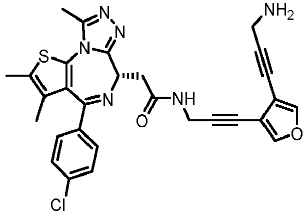 (S)-N-(3-(4-(3-aminoprop-1-yn-1-yl)furan-3-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-40' | 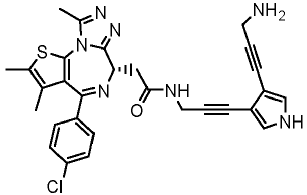 (S)-N-(3-(4-(3-aminoprop-1-yn-1-yl)-1H-pyrrol-3-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-43' | 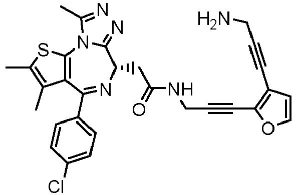 (S)-N-(3-(3-(3-aminoprop-1-yn-1-yl)furan-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-52' | 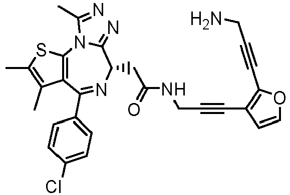 (S)-N-(3-(2-(3-aminoprop-1-yn-1-yl)furan-3-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-61' | 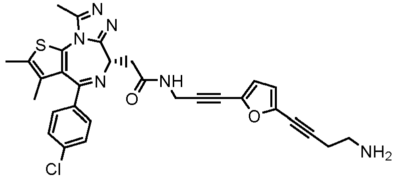 (S)-N-(3-(5-(4-aminobut-1-yn-1-yl)furan-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-62' | 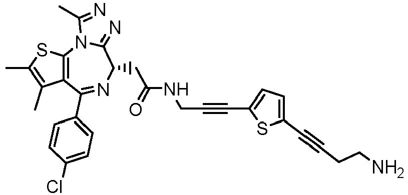 (S)-N-(3-(5-(4-aminobut-1-yn-1-yl)thiophen-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-63' | 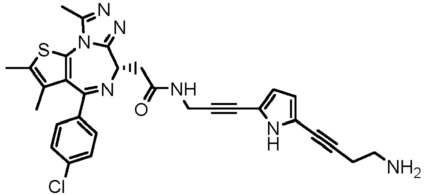 (S)-N-(3-(5-(4-aminobut-1-yn-1-yl)-1H-pyrrol-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-64' | 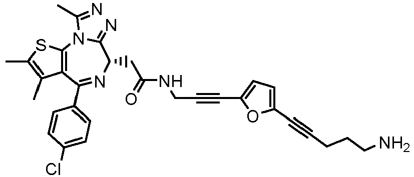 (S)-N-(3-(5-(5-aminopent-1-yn-1-yl)furan-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-65' | 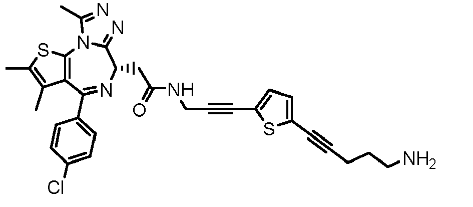 (S)-N-(3-(5-(5-aminopent-1-yn-1-yl)thiophen-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-66' | 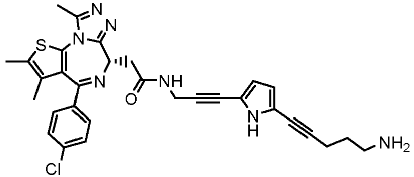 (S)-N-(3-(5-(5-aminopent-1-yn-1-yl)-1H-pyrrol-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-67' | 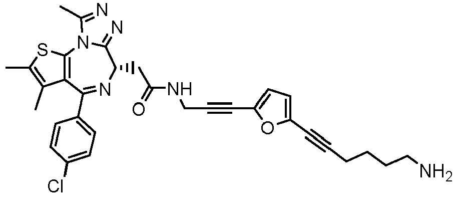 (S)-N-(3-(5-(6-aminohex-1-yn-1-yl)turan-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-68' | 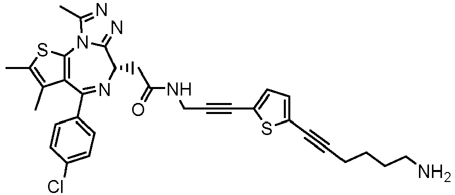 (S)-N-(3-(5-(6-aminohex-1-yn-1-yl)thiophen-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-69' | 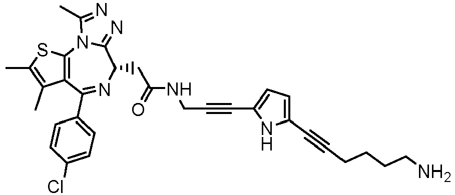 (S)-N-(3-(5-(6-aminohex-1-yn-1-yl)-1H-pyrrol-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-70' | 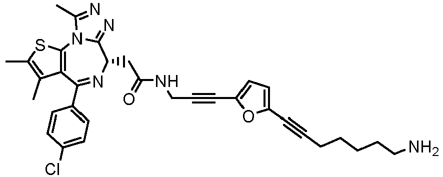 (S)-N-(3-(5-(7-aminohept-1-yn-1-yl)furan-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-71' | 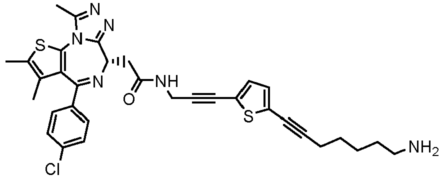 (S)-N-(3-(5-(7-aminohept-1-yn-1-yl)thiophen-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-72' | 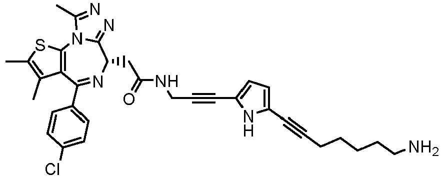 (S)-N-(3-(5-(7-aminohept-1-yn-1-yl)-1H-pyrrol-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-73' | 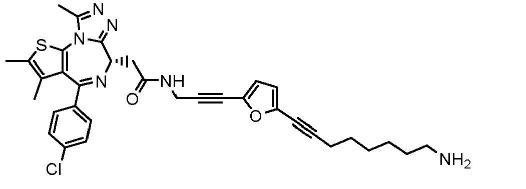 (S)-N-(3-(5-(8-aminooct-1-yn-1-yl)furan-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-74' | 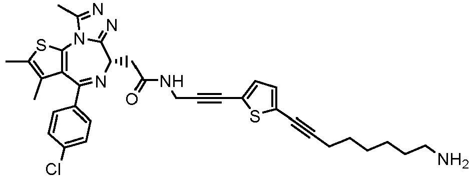 (S)-N-(3-(5-(8-aminooct-1-yn-1-yl)thiophen-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-75' | 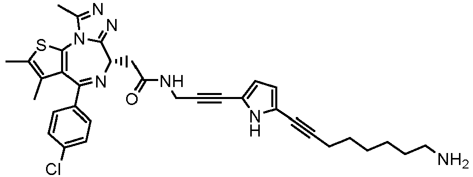 (S)-N-(3-(5-(8-aminooct-1-yn-1-yl)-1H-pyrrol-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-128' | 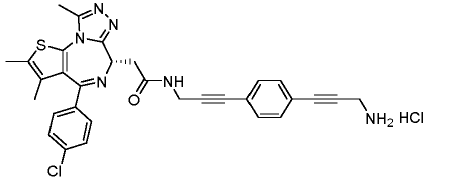 ((S)-N-(3-(4-(3-aminoprop-1-yn-1-yl)phenyl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide hydrochloride |
| 4-129' | 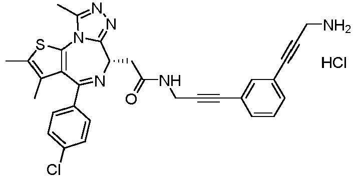 (S)-N-(3-(3-(3-aminoprop-1-yn-1-yl)phenyl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide hydrochloride |
| 4-130' | 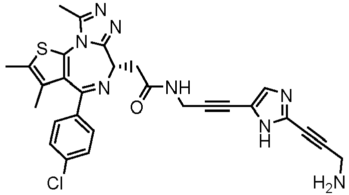 (S)-N-(3-(2-(3-aminoprop-1-yn-1-yl)-1H-imidazol-5-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-131' | 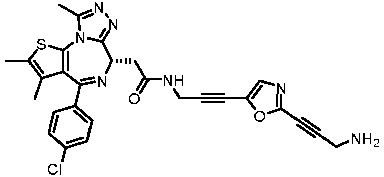 (S)-N-(3-(2-(3-aminoprop-1-yn-1-yl)oxazol-5-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-147' | 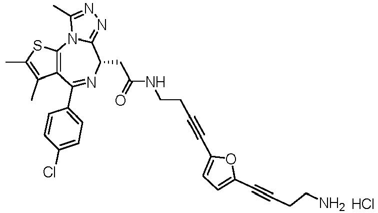 (S)-N-(4-(5-(4-aminobut-1-yn-1-yl)furan-2-yl)but-3-yn-1-yl)-2-(4-(4-chlorophenyl)-2,3,9-trimethyl-6H-thieno[3,2-f][1,2,4]triazolo[4,3-a][1,4]diazepin-6-yl)acetamide hydrochloride |
| 4-177' | 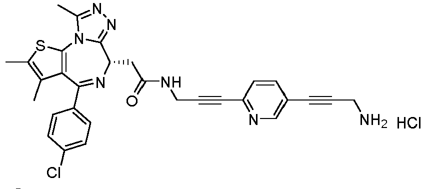 (S)-N-(3-(5-(3-aminoprop-1-yn-1-yl)pyridin-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide hydrochloride |
| 4-178' | 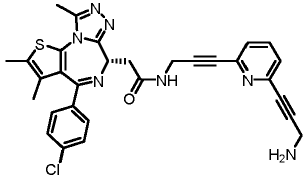 (S)-N-(3-(6-(3-aminoprop-1-yn-1-yl)pyridin-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-179' | 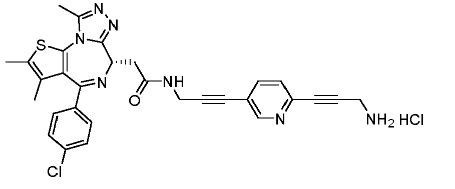 (S)-N-(3-(6-(3-aminoprop-1-yn-1-yl)pyridin-3-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide hydrochloride |
| 4-180' | 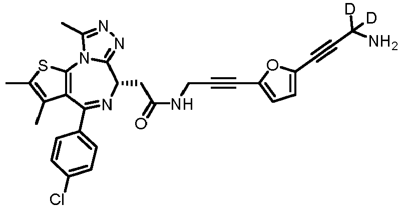 (S)-N-(3-(5-(3-aminoprop-1-yn-1-yl-3,3-d2)furan-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-181' | 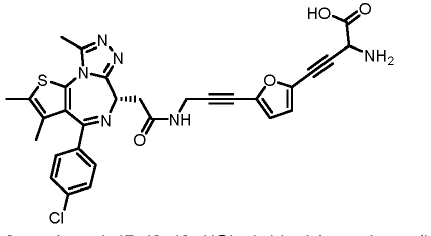 2-amino-4-(5-(3-(2-((S)-4-(4-chlorophenyl)-2,3,9-trimethyl-6H-thieno[3,2-f][1,2,4]triazolo[4,3-a][1,4]diazepin-6-yl)acetamido)prop-1-yn-1-yl)furan-2-yl)but-3-ynoic acid |
| 4-182' | 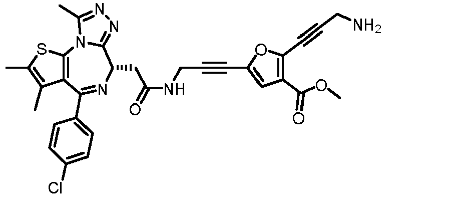 methyl (S)-2-(3-aminoprop-1-yn-1-yl)-5-(3-(2-(4-(4-chlorophenyl)-2,3,9-trimethyl-6H-thieno[3,2-f][1,2,4]triazolo[4,3-a][1,4]diazepin-6-yl)acetamido)prop-1-yn-1-yl)furan-3-carboxylate |
| 4-183' | 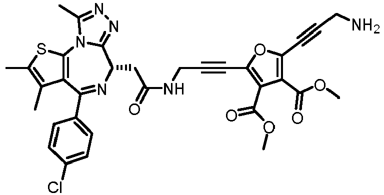 dimethyl (S)-2-(3-aminoprop-1-yn-1-yl)-5-(3-(2-(4-(4-chlorophenyl)-2,3,9-trimethyl-6H-thieno[3,2-f][1,2,4]triazolo[4,3-a][1,4]diazepin-6-yl)acetamido)prop-1-yn-1-yl)furan-3,4-dicarboxylate |
| 4-184' | 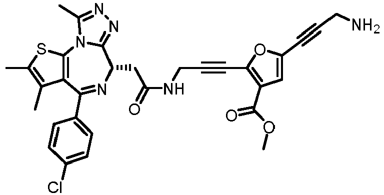 methyl (S)-5-(3-aminoprop-1-yn-1-yl)-2-(3-(2-(4-(4-chlorophenyl)-2,3,9-trimethyl-6H-thieno[3,2-f][1,2,4]triazolo[4,3-a][1,4]diazepin-6-yl)acetamido)prop-1-yn-1-yl)furan-3-carboxylate |
| 4-185' | 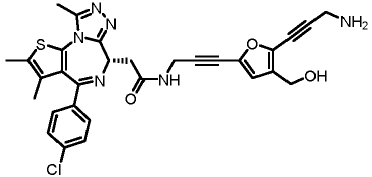 (S)-N-(3-(5-(3-aminoprop-1-yn-1-yl)-4-(hydroxymethyl)furan-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-186' | 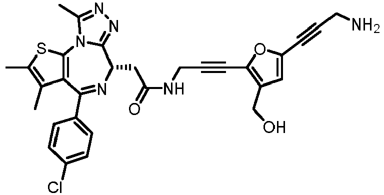 (S)-N-(3-(5-(3-aminoprop-1-yn-1-yl)-3-(hydroxymethyl)furan-2-yl)prop-2-yn-1-yl)-2-(4-(4-chlorophenyl)-2,3,9-trimethyl-6H-thieno[3,2-fl[1,2,4]triazolo[4,3-a][1,4]diazepin-6-yl)acetamide |
| 4-187' | 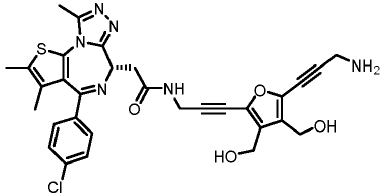 (S)-N-(3-(5-(3-aminoprop-1-yn-1-yl)-3,4-bis(hydroxymethyl)furan-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-188' | 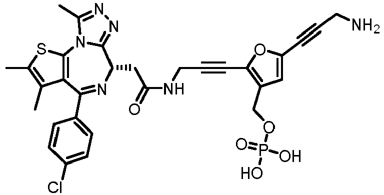 (S)-(5-(3-aminoprop-1-yn-1-yl)-2-(3-(2-(4-(4-chlorophenyl)-2,3,9-trimethyl-6H-thieno[3,2-f][1,2,4]triazolo[4,3-a][1,4]diazepin-6-yl)acetamido)prop-1-yn-1-yl)furan-3-yl)methyl dihydrogen phosphate |
| 4-189' | 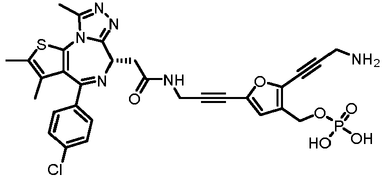 (S)-(2-(3-aminoprop-1-yn-1-yl)-5-(3-(2-(4-(4-chlorophenyl)-2,3,9-trimethyl-6H-thieno[3,2-f][1,2,4]triazolo[4,3-a][1,4]diazepin-6-yl)acetamido)prop-1-yn-1-yl)furan-3-yl)methyl dihydrogen phosphate |
| 4-200' | 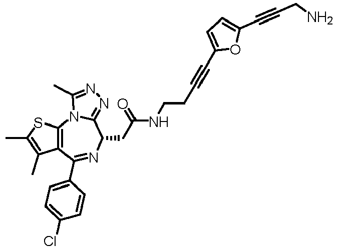 (S)-N-(4-(5-(3-aminoprop-1-yn-1-yl)furan-2-yl)but-3-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-201' | (S)-N-(3-(4-(4-aminobut-1-yn-1-yl)furan-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-202' | (S)-N-(4-(4-(4-aminobut-1-yn-1-yl)furan-2-yl)but-3-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-218' | 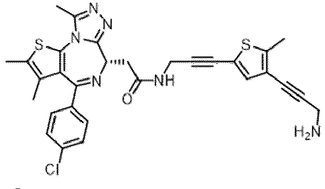 (S)-N-(3-(4-(3-aminoprop-1-yn-1-yl)-5-methylthiophen-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-219' | (S)-N-(3-(4-(4-aminobut-1-yn-1-yl)-5-methylthiophen-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-223' | 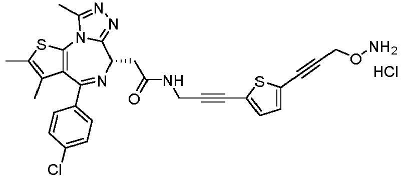 (S)-N-(3-(5-(3-(aminooxy)prop-1-yn-1-yl)thiophen-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide hydrochloride |
| 5-52' | 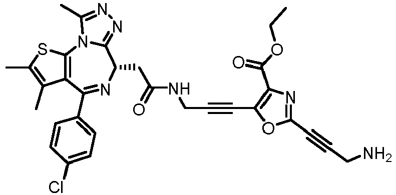 ethyl (S)-2-(3-aminoprop-1-yn-1-yl)-5-(3-(2-(4-(4-chlorophenyl)-2,3,9-trimethyl-6H-thieno[3,2-f][1,2,4]triazolo[4,3-a][1,4]diazepin-6-yl)acetamido)prop-1-yn-1-yl)oxazole-4-carboxylate |
| 5-116' | 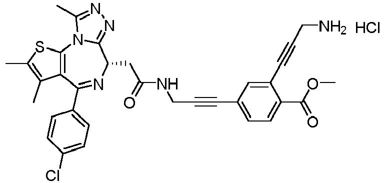 methyl (S)-2-(3-aminoprop-1-yn-1-yl)-4-(3-(2-(4-(4-chlorophenyl)-2,3,9-trimethyl-6H-thieno[3,2-f][1,2,4]triazolo[4,3-a][1,4]diazepin-6-yl)acetamido)prop-1-yn-1-yl)benzoate hydrochloride |

**[Table 6]**

| No. | Compound |
|---|---|
| 1-1" | 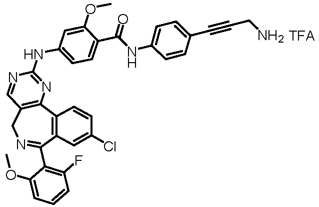 N-(4-(3-aminoprop-1-yn-1-yl)phenyl)-4-((9-chloro-7-(2-fluoro-6-methoxyphenyl)-5H-benzo[c]pyrimido[4,5-e]azepin-2-yl)amino)-2-methoxybenzamide trifluoroacetic acid |
| 1-3" | 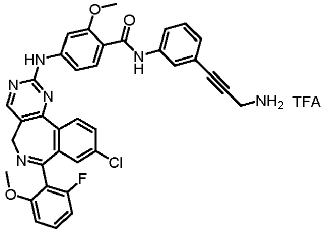 N-(3-(3-aminoprop-1-yn-1-yl)phenyl)-4-((9-chloro-7-(2-fluoro-6-methoxyphenyl)-5H-benzo[c]pyrimido[4,5-e]azepin-2-yl)amino)-2-methoxybenzamide trifluoroacetic acid |
| 3-25" | 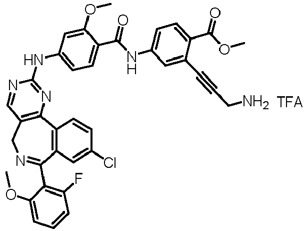 methyl 2-(3-aminoprop-1-yn-1-yl)-4-(4-((9-chloro-7-(2-fluoro-6-methoxyphenyl)-5H-benzo[c]pyrimido[4,5-e]azepin-2-yl)amino)-2-methoxybenzamido)benzoate trifluoroacetic acid |
| 3-49" | 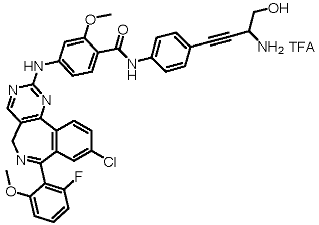 N-(4-(3-amino-4-hydroxybut-1-yn-1-yl)phenyl)-4-((9-chloro-7-(2-fluoro-6-methoxyphenyl)-5H-benzo[c]pyrimido[4,5-e]azepin-2-yl)amino)-2-methoxybenzamide trifluoroacetic acid |
| 4-1" | 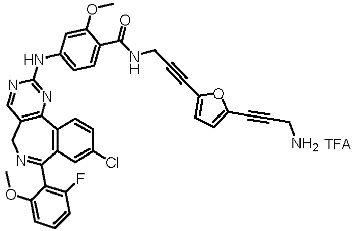 N-(3-(5-(3-aminoprop-1-yn-1-yl)furan-2-yl)prop-2-yn-1-yl)-4-((9-chloro-7-(2-fluoro-6-methoxyphenyl)-5H-benzo[c]pyrimido[4,5-e]azepin-2-yl)amino)-2-methoxybenzamide trifluoroacetic acid |
| 5-116" | 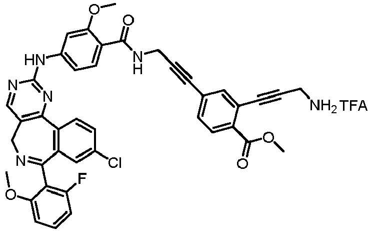 methyl 2-(3-aminoprop-1-yn-1-yl)-4-(3-(4-((9-chloro-7-(2-fluoro-6-methoxyphenyl)-5H-benzo[c]pyrimido[4,5-e]azepin-2-yl)amino)-2-methoxybenzamido)prop-1-yn-1-yl)benzoate trifluoroacetic acid |

**[Table 7]**

| No. | Compound |
|---|---|
| 1-1‴ | 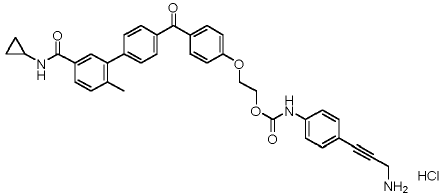 2-(4-(5'-(cyclopropylcarbamoyl)-2'-methyl-[1,1'-biphenyl]-4-carbonyl)phenoxy)ethyl (4-(3-aminoprop-1-yn-1-yl)phenyl)carbamate hydrochloride |
| 3-49‴ | 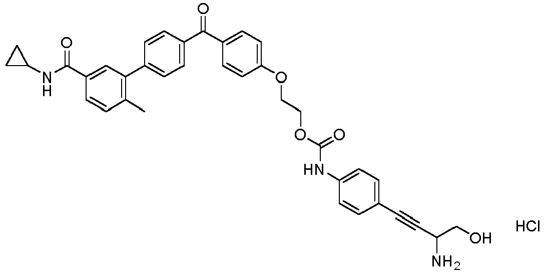 2-(4-(5'-(cyclopropylcarbamoyl)-2'-methyl-[1,1'-biphenyl]-4-carbonyl)phenoxy)ethyl (4-(3-amino-4-hydroxybut-1-yn-1-yl)phenyl)carbamate hydrochloride |
| 5-116‴ | 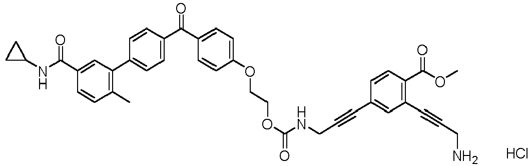 methyl 2-(3-aminoprop-1-yn-1-yl)-4-(3-(((2-(4-(5'-(cyclopropylcarbamoyl)-2'-methyl-[1,1'-biphenyl]-4-carbonyl)phenoxy)ethoxy)carbonyl)amino)prop-1-yn-1-yl)benzoate hydrochloride |

More specifically, in the compound represented by Formula VI above, specific examples of the case where the compound of Formula 1 above is bound to the TBM may be presented in Table 8 below, specific examples of the case where the compound of Formula 2 above is bound to the TBM may be presented in Table 9 below, and specific examples of the case where the compound of Formula 3 above is bound to the TBM may be presented in Table 10 below, but are not limited thereto.

**[Table 8]**

| No. | Compound |
|---|---|
| 4-6' | 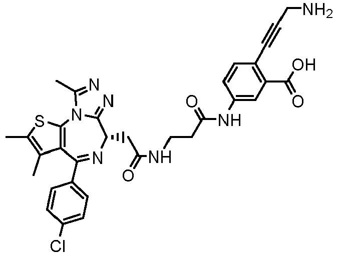 (S)-2-(3-aminoprop-1-yn-1-yl)-5-(3-(2-(4-(4-chlorophenyl)-2,3,9-trimethyl-6H-thieno[3,2-f][1,2,4]triazolo[4,3-a][1,4]diazepin-6-yl)acetamido)propanamido)benzoic acid |
| 4-109' | 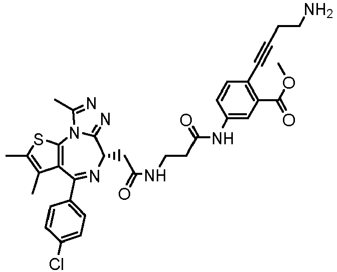 methyl (S)-2-(4-aminobut-1-yn-1-yl)-5-(3-(2-(4-(4-chlorophenyl)-2,3,9-trimethyl-6H-thieno[3,2-f][1,2,4]triazolo[4,3-a][1,4]diazepin-6-yl)acetamido)propanamido)benzoate |
| 4-110' | 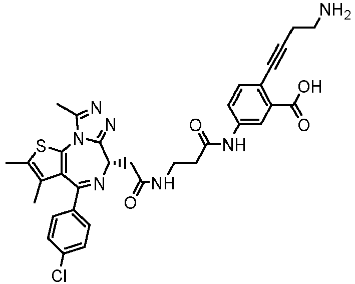 (S)-2-(4-aminobut-1-yn-1-yl)-5-(3-(2-(4-(4-chlorophenyl)-2,3,9-trimethyl-6H-thieno[3,2-f][1,2,4]triazolo[4,3-a][1,4]diazepin-6-yl)acetamido)propanamido)benzoic acid |
| 4-111' | 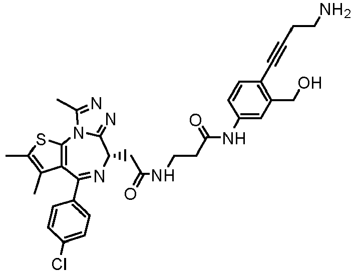 (S)-N-(4-(4-aminobut-1-yn-1-yl)-3-(hydroxymethyl)phenyl)-3-(2-(4-(4-chlorophenyl)-2,3,9-trimethyl-6H-thieno[3,2-f][1,2,4]triazolo[4,3-a][1,4]diazepin-6-yl)acetamido)propanamide |
| 4-112' | 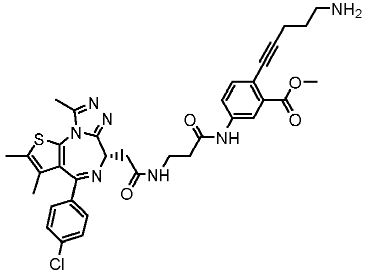 methyl (S)-2-(5-aminopent-1-yn-1-yl)-5-(3-(2-(4-(4-chlorophenyl)-2,3,9-trimethyl-6H-thieno[3,2-f][1,2,4]triazolo[4,3-a][1,4]diazepin-6-yl)acetamido)propanamido)benzoate |
| 4-113' | 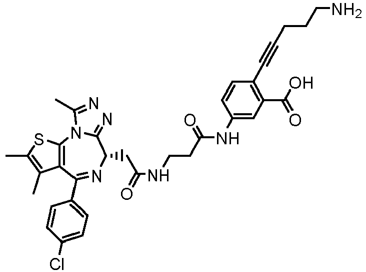 (S)-2-(5-aminopent-1-yn-1-yl)-5-(3-(2-(4-(4-chlorophenyl)-2,3,9-trimethyl-6H-thieno[3,2-f][1,2,4]triazolo[4,3-a][1,4]diazepin-6-yl)acetamido)propanamido)benzoic acid |
| 4-114' | 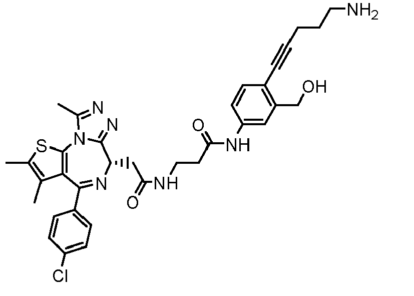 (S)-N-(4-(5-aminopent-1-yn-1-yl)-3-(hydroxymethyl)phenyl)-3-(2-(4-(4-chlorophenyl)-2,3,9-trimethyl-6H-thieno[3,2-f][1,2,4]triazolo[4,3-a][1,4]diazepin-6-yl)acetamido)propanamide |
| 4-115' | 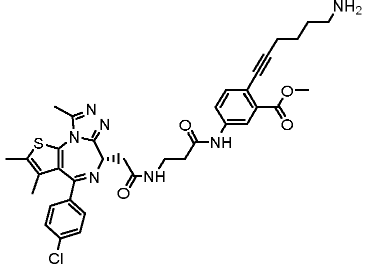 methyl (S)-2-(6-aminohex-1-yn-1-yl)-5-(3-(2-(4-(4-chlorophenyl)-2,3,9-trimethyl-6H-thieno[3,2-f][1,2,4]triazolo[4,3-a][1,4]diazepin-6-yl)acetamido)propanamido)benzoate |
| 4-116' | 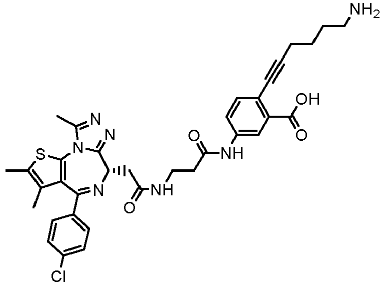 (S)-2-(6-aminohex-1-yn-1-yl)-5-(3-(2-(4-(4-chlorophenyl)-2,3,9-trimethyl-6H-thieno[3,2-f][1,2,4]triazolo[4,3-a][1,4]diazepin-6-yl)acetamido)propanamido)benzoic acid |
| 4-117' | 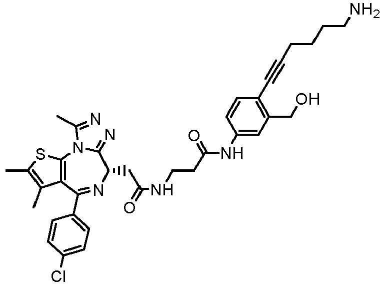 (S)-N-(4-(6-aminohex-1-yn-1-yl)-3-(hydroxymethyl)phenyl)-3-(2-(4-(4-chlorophenyl)-2,3,9-trimethyl-6H-thieno[3,2-f][1,2,4]triazolo[4,3-a][1,4]diazepin-6-yl)acetamido)propanamide |
| 4-118' | 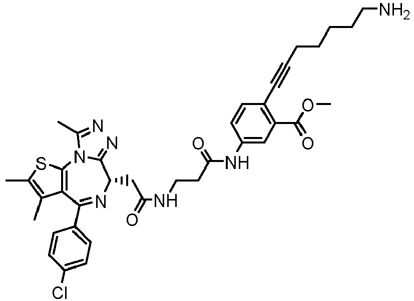 methyl (S)-2-(7-aminohept-1-yn-1-yl)-5-(3-(2-(4-(4-chlorophenyl)-2,3,9-trimethyl-6H-thieno[3,2-f][1,2,4]triazolo[4,3-a][1,4]diazepin-6-yl)acetamido)propanamido)benzoate |
| 4-119' | 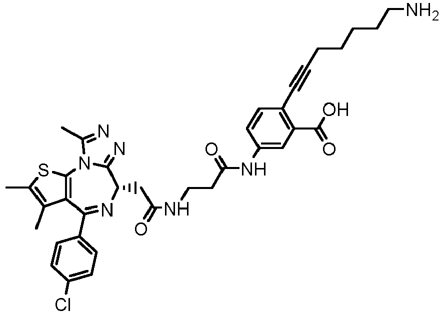 (S)-2-(7-aminohept-1-yn-1-yl)-5-(3-(2-(4-(4-chlorophenyl)-2,3,9-trimethyl-6H-thieno[3,2-f][1,2,4]triazolo[4,3-a][1,4]diazepin-6-yl)acetamido)propanamido)benzoic acid |
| 4-120' | 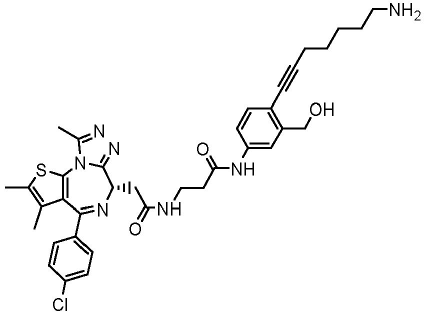 (S)-N-(4-(7-aminohept-1-yn-1-yl)-3-(hydroxymethyl)phenyl)-3-(2-(4-(4-chlorophenyl)-2,3,9-trimethyl-6H-thieno[3,2-f][1,2,4]triazolo[4,3-a][1,4]diazepin-6-yl)acetamido)propanamide |
| 4-121' | 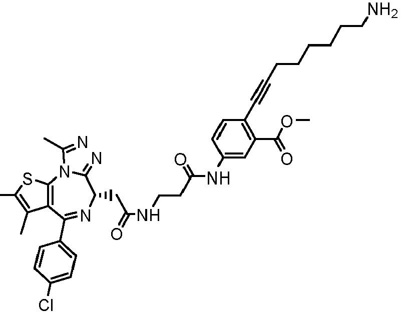 methyl (S)-2-(8-aminooct-1-yn-1-yl)-5-(3-(2-(4-(4-chlorophenyl)-2,3,9-trimethyl-6H-thieno[3,2-f][1,2,4]triazolo[4,3-a][1,4]diazepin-6-yl)acetamido)propanamido)benzoate |
| 4-122' | 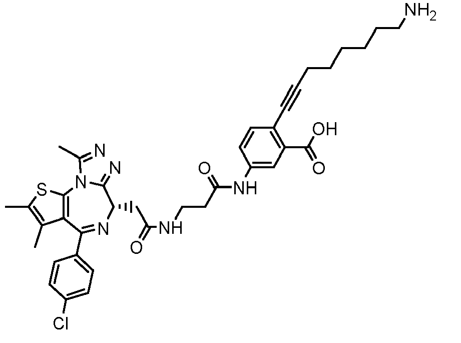 (S)-2-(8-aminooct-1-yn-1-yl)-5-(3-(2-(4-(4-chlorophenyl)-2,3,9-trimethyl-6H-thieno[3,2-f][1,2,4]triazolo[4,3-a][1,4]diazepin-6-yl)acetamido)propanamido)benzoic acid |
| 4-123' | 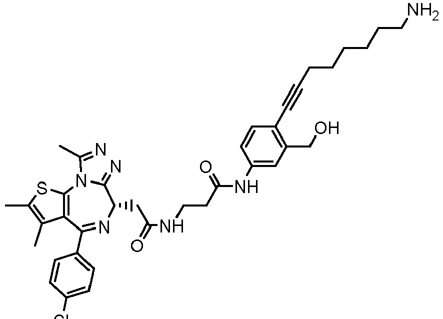 (S)-N-(4-(8-aminooct-1-yn-1-yl)-3-(hydroxymethyl)phenyl)-3-(2-(4-(4-chlorophenyl)-2,3,9-trimethyl-6H-thieno[3,2-f][1,2,4]triazolo[4,3-a][1,4]diazepin-6-yl)acetamido)propanamide |
| 4-124' | 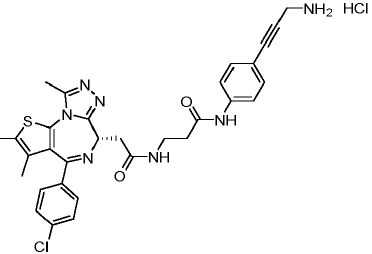 (S)-N-(4-(3-aminoprop-1-yn-1-yl)phenyl)-3-(2-(4-(4-chlorophenyl)-2,3,9-trimethyl-6H-thieno[3,2-f][1,2,4]triazolo[4,3-a][1,4]diazepin-6-yl)acetamido)propanamide hydrochloride |
| 4-125' | 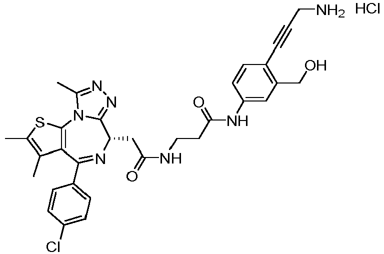 (S)-N-(4-(3-aminoprop-1-yn-1-yl)-3-(hydroxymethyl)phenyl)-3-(2-(4-(4-chlorophenyl)-2,3,9-trimethyl-6H-thieno[3,2-f][1,2,4]triazolo[4,3-a][1,4]diazepin-6-yl)acetamido)propanamide hydrochloride |
| 4-126' | 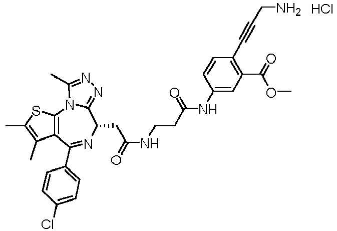 methyl (S)-2-(3-aminoprop-1-yn-1-yl)-5-(3-(2-(4-(4-chlorophenyl)-2,3,9-trimethyl-6H-thieno[3,2-f][1,2,4]triazolo[4,3-a][1,4]diazepin-6-yl)acetamido)propanamido)benzoate hydrochloride |
| 4-127' | 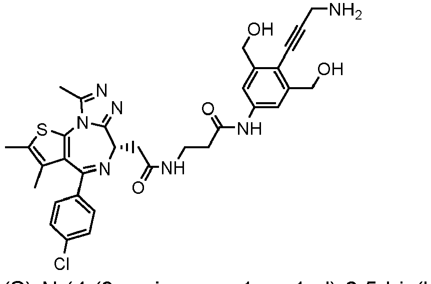 (S)-N-(4-(3-aminoprop-1-yn-1-yl)-3,5-bis(hydroxymethyl)phenyl)-3-(2-(4-(4-chlorophenyl)-2,3,9-trimethyl-6H-thieno[3,2-f][1,2,4]triazolo[4,3-a][1,4]diazepin-6-yl)acetamido)propanamide |
| 4-148' | 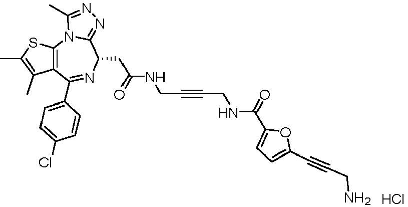 (S)-5-(3-aminoprop-1-yn-1-yl)-N-(4-(2-(4-(4-chlorophenyl)-2,3,9-trimethyl-6H-thieno[3,2-f][1,2,4]triazolo[4,3-a][1,4]diazepin-6-yl)acetamido)but-2-yn-1-yl)furan-2-carboxamide hydrochloride |
| 4-149' | 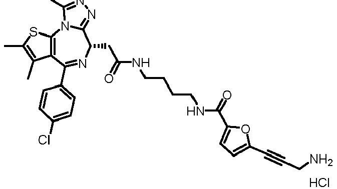 ((S)-5-(3-aminoprop-1-yn-1-yl)-N-(4-(2-(4-(4-chlorophenyl)-2,3,9-trimethyl-6H-thieno[3,2-f][1,2,4]triazolo[4,3-a][1,4]diazepin-6-yl)acetamido)butyl)furan-2-carboxamide hydrochloride |
| 4-150' | 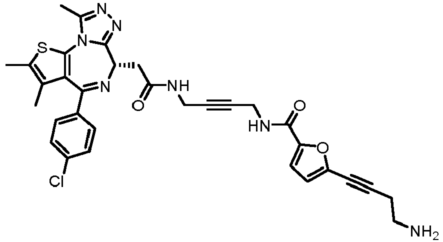 (S)-5-(4-aminobut-1-yn-1-yl)-N-(4-(2-(4-(4-chlorophenyl)-2,3,9-trimethyl-6H-thieno[3,2-f][1,2,4]triazolo[4,3-a][1,4]diazepin-6-yl)acetamido)but-2-yn-1-yl)furan-2-carboxamide |
| 4-161' | 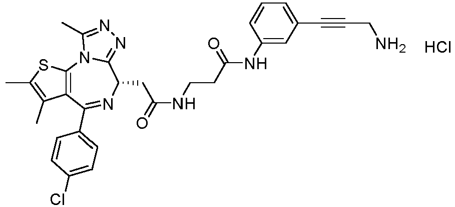 (S)-N-(3-(3-aminoprop-1-yn-1-yl)phenyl)-3-(2-(4-(4-chlorophenyl)-2,3,9-trimethyl-6H-thieno[3,2-f][1,2,4]triazolo[4,3-a][1,4]diazepin-6-yl)acetamido)propanamide hydrochloride |
| 4-162' | 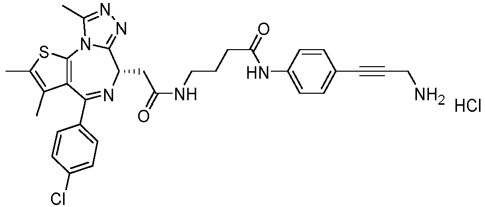 (S)-N-(4-(3-aminoprop-1-yn-1-yl)phenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide hydrochloride |
| 4-163' | 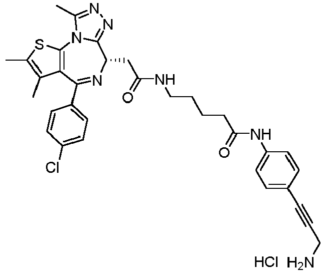 (S)-N-(4-(3-aminoprop-1-yn-1-yl)phenyl)-5-(2-(4-(4-chlorophenyl)-2,3,9-trimethyl-6H-thieno[3,2-f][1,2,4]triazolo[4,3-a][1,4]diazepin-6-yl)acetamido)pentanamide hydrochloride |
| 4-164' | 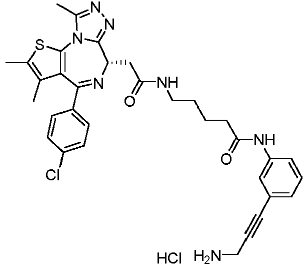 (S)-N-(3-(3-aminoprop-1-yn-1-yl)phenyl)-5-(2-(4-(4-chlorophenyl)-2,3,9-trimethyl-6H-thieno[3,2-f][1,2,4]triazolo[4,3-a][1,4]diazepin-6-yl)acetamido)pentanamide hydrochloride |
| 4-165' | 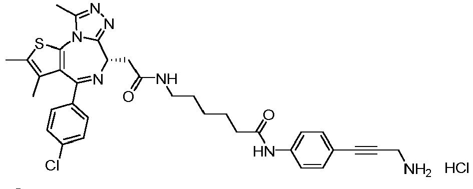 (S)-N-(4-(3-aminoprop-1-yn-1-yl)phenyl)-6-(2-(4-(4-chlorophenyl)-2,3,9-trimethyl-6H-thieno[3,2-f][1,2,4]triazolo[4,3-a][1,4]diazepin-6-yl)acetamido)hexanamide hydrochloride |
| 4-166' | 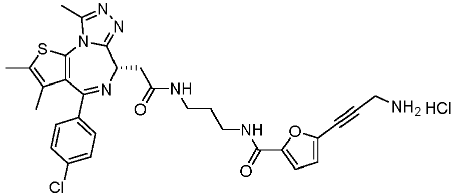 ((S)-5-(3-aminoprop-1-yn-1-yl)-N-(3-(2-(4-(4-chlorophenyl)-2,3,9-trimethyl-6H-thieno[3,2-f][1,2,4]triazolo[4,3-a][1,4]diazepin-6-yl)acetamido)propyl)furan-2-carboxamide hyd rochloride |
| 4-167' | 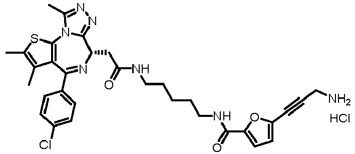 (S)-5-(3-aminoprop-1-yn-1-yl)-N-(5-(2-(4-(4-chlorophenyl)-2,3,9-trimethyl-6H-thieno[3,2-f][1,2,4]triazolo[4,3-a][1,4]diazepin-6-yl)acetamido)pentyl)furan-2-carboxamide hydrochloride |
| 4-168' | 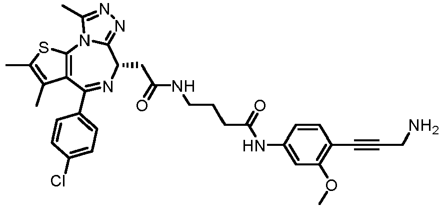 (S)-N-(4-(3-aminoprop-1-yn-1-yl)-3-methoxyphenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide |
| 4-169' | 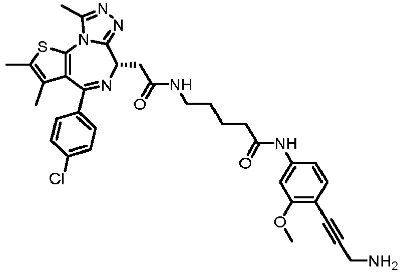 (S)-N-(4-(3-aminoprop-1-yn-1-yl)-3-methoxyphenyl)-5-(2-(4-(4-chlorophenyl)-2,3,9-trimethyl-6H-thieno[3,2-f][1,2,4]triazolo[4,3-a][1,4]diazepin-6-yl)acetamido)pentanamide |
| 4-170' | 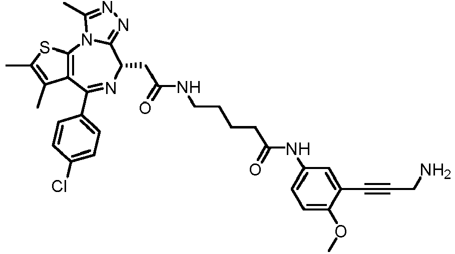 (S)-N-(3-(3-aminoprop-1-yn-1-yl)-4-methoxyphenyl)-5-(2-(4-(4-chlorophenyl)-2,3,9-trimethyl-6H-thieno[3,2-f][1,2,4]triazolo[4,3-a][1,4]diazepin-6-yl)acetamido)pentanamide |
| 4-171' | 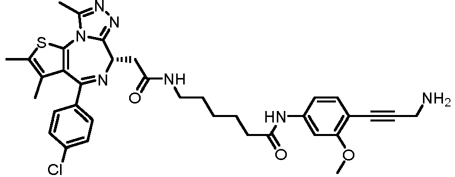 (S)-N-(4-(3-aminoprop-1-yn-1-yl)-3-methoxyphenyl)-6-(2-(4-(4-chlorophenyl)-2,3,9-trimethyl-6H-thieno[3,2-f][1,2,4]triazolo[4,3-a][1,4]diazepin-6-yl)acetamido)hexanamide |
| 4-192' | 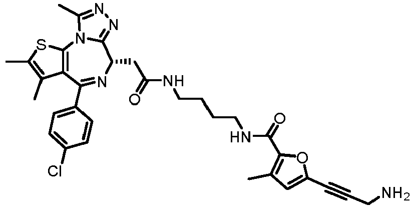 (S)-5-(3-aminoprop-1-yn-1-yl)-N-(4-(2-(4-(4-chlorophenyl)-2,3,9-trimethyl-6H-thieno[3,2-f][1,2,4]triazolo[4,3-a][1,4]diazepin-6-yl)acetamido)butyl)-3-methylfuran-2-carboxamide |
| 4-199' | 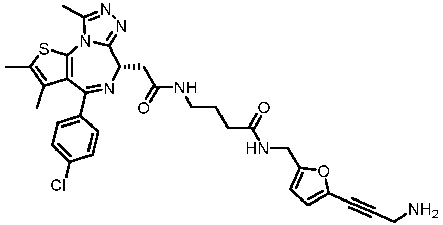 (S)-N-((5-(3-aminoprop-1-yn-1-yl)furan-2-yl)methyl)-4-(2-(4-(4-chlorophenyl)-2,3,9-trimethyl-6H-thieno[3,2-f][1,2,4]triazolo[4,3-a][1,4]diazepin-6-yl)acetamido)butanamide |
| 4-203' | 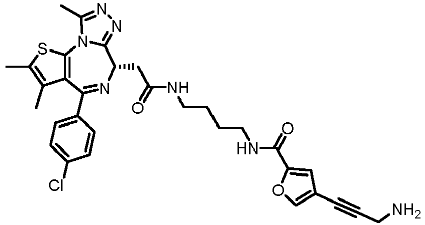 (S)-4-(3-aminoprop-1-yn-1-yl)-N-(4-(2-(4-(4-chlorophenyl)-2,3,9-trimethyl-6H-thieno[3,2-f][1,2,4]triazolo[4,3-a][1,4]diazepin-6-yl)acetamido)butyl)furan-2-carboxamide |
| 4-204' | 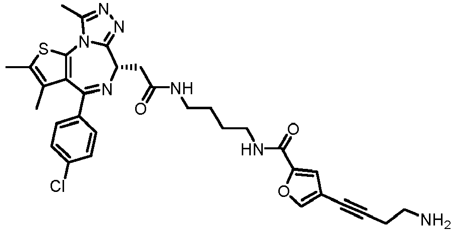 (S)-4-(4-aminobut-1-yn-1-yl)-N-(4-(2-(4-(4-chlorophenyl)-2,3,9-trimethyl-6H-thieno[3,2-f][1,2,4]triazolo[4,3-a][1,4]diazepin-6-yl)acetamido)butyl)furan-2-carboxamide |
| 4-205' | 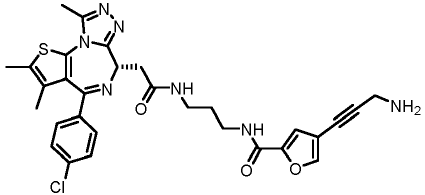 (S)-4-(3-aminoprop-1-yn-1-yl)-N-(3-(2-(4-(4-chlorophenyl)-2,3,9-trimethyl-6H-thieno[3,2-f][1,2,4]triazolo[4,3-a][1,4]diazepin-6-yl)acetamido)propyl)furan-2-carboxamide |
| 4-206' | 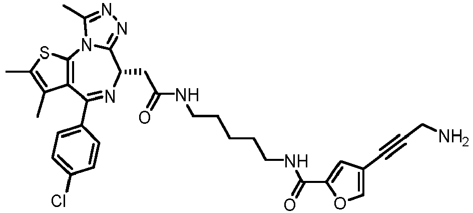 (S)-4-(3-aminoprop-1-yn-1-yl)-N-(5-(2-(4-(4-chlorophenyl)-2,3,9-trimethyl-6H-thieno[3,2-f][1,2,4]triazolo[4,3-a][1,4]diazepin-6-yl)acetamido)pentyl)furan-2-carboxamide |
| 4-209' | 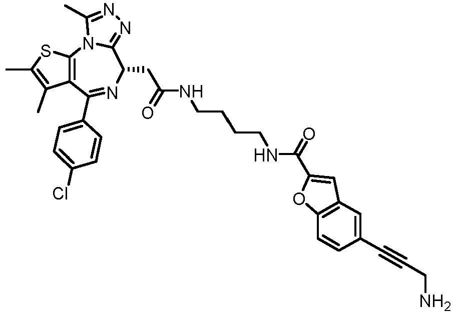 (S)-5-(3-aminoprop-1-yn-1-yl)-N-(4-(2-(4-(4-chlorophenyl)-2,3,9-trimethyl-6H-thieno[3,2-f][1,2,4]triazolo[4,3-a][1,4]diazepin-6-yl)acetamido)butyl)benzofuran-2-carboxamide |
| 4-210' | 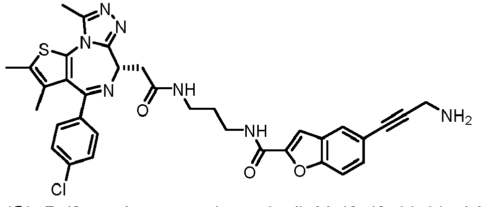 (S)-5-(3-aminoprop-1-yn-1-yl)-N-(3-(2-(4-(4-chlorophenyl)-2,3,9-trimethyl-6H-thieno[3,2-f][1,2,4]triazolo[4,3-a][1,4]diazepin-6-yl)acetamido)propyl)benzofuran-2-carboxamide |
| 4-214' | 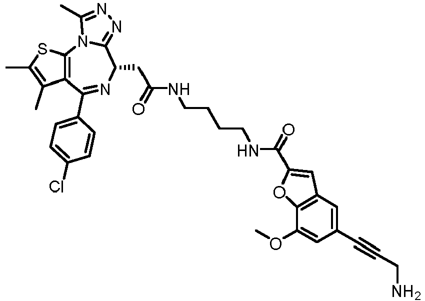 (S)-5-(3-aminoprop-1-yn-1-yl)-N-(4-(2-(4-(4-chlorophenyl)-2,3,9-trimethyl-6H-thieno[3,2-f][1,2,4]triazolo[4,3-a][1,4]diazepin-6-yl)acetamido)butyl)-7-methoxybenzofuran-2-carboxamide |
| 4-215' | 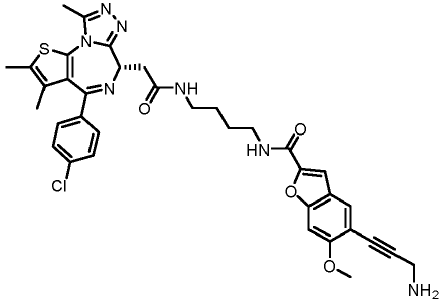 (S)-5-(3-aminoprop-1-yn-1-yl)-N-(4-(2-(4-(4-chlorophenyl)-2,3,9-trimethyl-6H-thieno[3,2-f][1,2,4]triazolo[4,3-a][1,4]diazepin-6-yl)acetamido)butyl)-6-methoxybenzofuran-2-carboxamide |
| 4-216' | 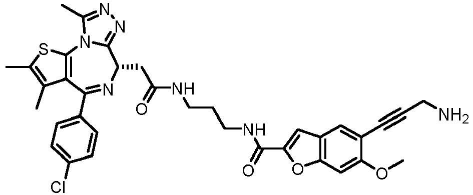 (S)-5-(3-aminoprop-1-yn-1-yl)-N-(3-(2-(4-(4-chlorophenyl)-2,3,9-trimethyl-6H-thieno[3,2-f][1,2,4]triazolo[4,3-a][1,4]diazepin-6-yl)acetamido)propyl)-6-methoxybenzofuran-2-carboxamide |
| 4-217' | 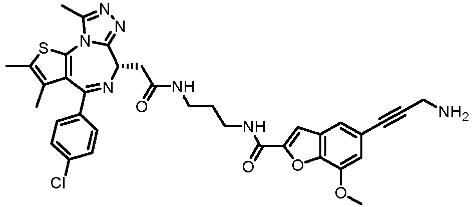 (S)-5-(3-aminoprop-1-yn-1-yl)-N-(3-(2-(4-(4-chlorophenyl)-2,3,9-trimethyl-6H-thieno[3,2-f][1,2,4]triazolo[4,3-a][1,4]diazepin-6-yl)acetamido)propyl)-7-methoxybenzofuran-2-carboxamide |
| 4-224' | 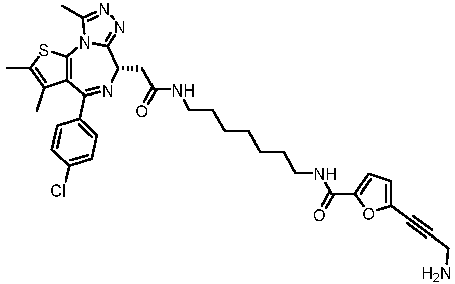 (S)-5-(3-aminoprop-1-yn-1-yl)-N-(7-(2-(4-(4-chlorophenyl)-2,3,9-trimethyl-6H-thieno[3,2-f][1,2,4]triazolo[4,3-a][1,4]diazepin-6-yl)acetamido)heptyl)furan-2-carboxamide |
| 4-225' | 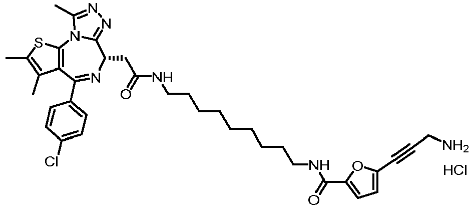 (S)-5-(3-aminoprop-1-yn-1-yl)-N-(9-(2-(4-(4-chlorophenyl)-2,3,9-trimethyl-6H-thieno[3,2-f][1,2,4]triazolo[4,3-a][1,4]diazepin-6-yl)acetamido)nonyl)furan-2-carboxamide hydrochloride |
| 4-228' | 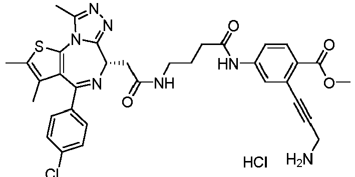 methyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzoate hydrochloride |
| 4-229' | 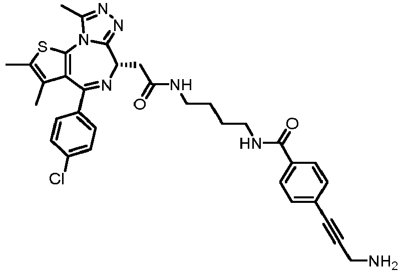 (S)-4-(3-aminoprop-1-yn-1-yl)-N-(4-(2-(4-(4-chlorophenyl)-2,3,9-trimethyl-6H-thieno[3,2-f][1,2,4]triazolo[4,3-a][1,4]diazepin-6-yl)acetamido)butyl)benzamide |
| 4-231' | 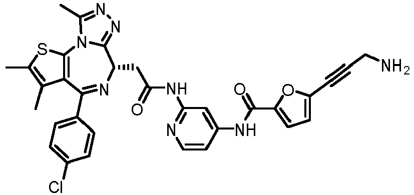 (S)-5-(3-aminoprop-1-yn-1-yl)-N-(2-(2-(4-(4-chlorophenyl)-2,3,9-trimethyl-6H-thieno[3,2-f][1,2,4]triazolo[4,3-a][1,4]diazepin-6-yl)acetamido)pyridin-4-yl)furan-2-carboxamide |
| 4-232' | 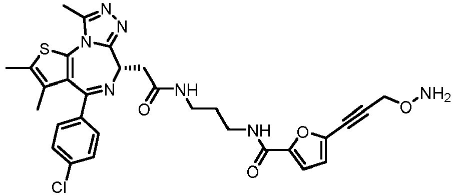 (S)-5-(3-(aminooxy)prop-1-yn-1-yl)-N-(3-(2-(4-(4-chlorophenyl)-2,3,9-trimethyl-6H-thieno[3,2-f][1,2,4]triazolo[4,3-a][1,4]diazepin-6-yl)acetamido)propyl)furan-2-carboxamide |
| 4-233' | 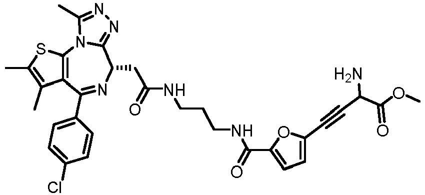 methyl 2-amino-4-(5-((3-(2-((S)-4-(4-chlorophenyl)-2,3,9-trimethyl-6H-thieno[3,2-f][1,2,4]triazolo[4,3-a][1,4]diazepin-6-yl)acetamido)propyl)carbamoyl)furan-2-yl)but-3-ynoate |
| 4-234' | 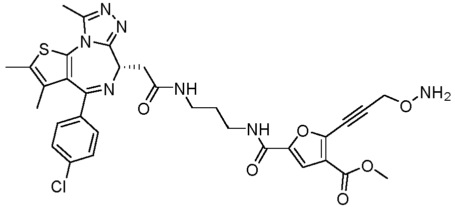 methyl (S)-2-(3-(aminooxy)prop-1-yn-1-yl)-5-((3-(2-(4-(4-chlorophenyl)-2,3,9-trimethyl-6H-thieno[3,2-f][1,2,4]triazolo[4,3-a][1,4]diazepin-6-yl)acetamido)propyl)carbamoyl)furan-3-carboxylate |
| 4-235' | 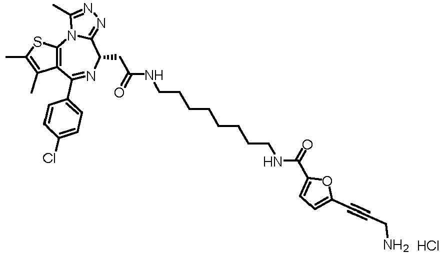 ((S)-5-(3-aminoprop-1-yn-1-yl)-N-(8-(2-(4-(4-chlorophenyl)-2,3,9-trimethyl-6H-thieno[3,2-f][1,2,4]triazolo[4,3-a][1,4]diazepin-6-yl)acetamido)octyl)furan-2-carboxamide hydrochloride |
| 5-1' | 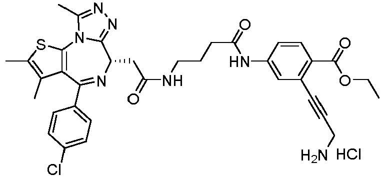 ethyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzoate hydrochloride |
| 5-2' | 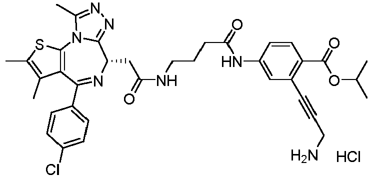 isopropyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzoate hydrochloride |
| 5-3' | 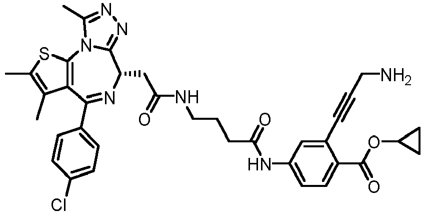 cyclopropyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzoate |
| 5-4' | 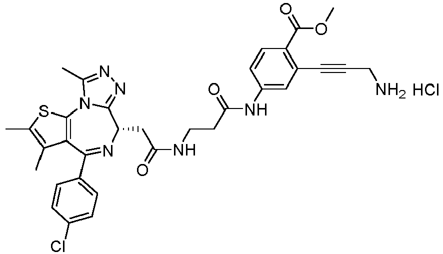 methyl (S)-2-(3-aminoprop-1-yn-1-yl)-4-(3-(2-(4-(4-chlorophenyl)-2,3,9-trimethyl-6H-thieno[3,2-f][1,2,4]triazolo[4,3-a][1,4]diazepin-6-yl)acetamido)propanamido)benzoate hydrochloride |
| 5-5' | 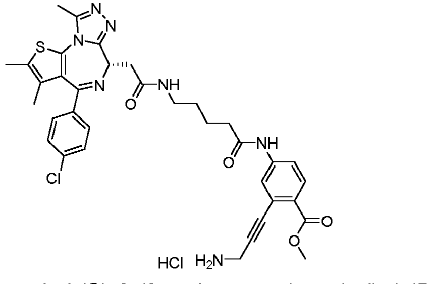 methyl (S)-2-(3-aminoprop-1-yn-1-yl)-4-(5-(2-(4-(4-chlorophenyl)-2,3,9-trimethyl-6H-thieno[3,2-f][1,2,4]triazolo[4,3-a][1,4]diazepin-6-yl)acetamido)pentanamido)benzoate hydrochloride |
| 5-6' | 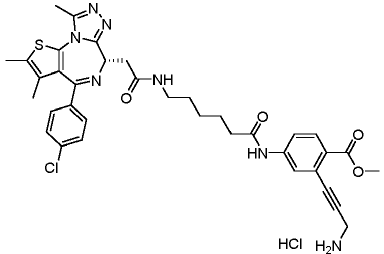 methyl (S)-2-(3-aminoprop-1-yn-1-yl)-4-(6-(2-(4-(4-chlorophenyl)-2,3,9-trimethyl-6H-thieno[3,2-f][1,2,4]triazolo[4,3-a][1,4]diazepin-6-yl)acetamido)hexanamido)benzoate hydrochloride |
| 5-8' | 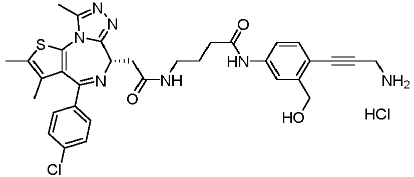 (S)-N-(4-(3-aminoprop-1-yn-1-yl)-3-(hydroxymethyl)phenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide hydrochloride |
| 5-9' | 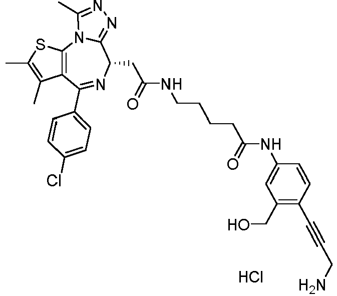 (S)-N-(4-(3-aminoprop-1-yn-1-yl)-3-(hydroxymethyl)phenyl)-5-(2-(4-(4-chlorophenyl)-2,3,9-trimethyl-6H-thieno[3,2-f][1,2,4]triazolo[4,3-a][1,4]diazepin-6-yl)acetamido)pentanamide hydrochloride |
| 5-10' | 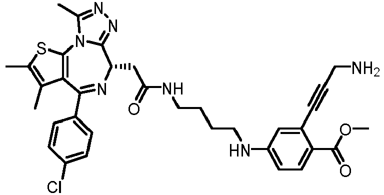 methyl (S)-2-(3-aminoprop-1-yn-1-yl)-4-((4-(2-(4-(4-chlorophenyl)-2,3,9-trimethyl-6H-thieno[3,2-f][1,2,4]triazolo[4,3-a][1,4]diazepin-6-yl)acetamido)butyl)amino)benzoate |
| 5-11' | 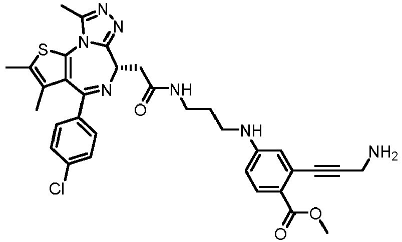 methyl (S)-2-(3-aminoprop-1-yn-1-yl)-4-((3-(2-(4-(4-chlorophenyl)-2,3,9-trimethyl-6H-thieno[3,2-f][1,2,4]triazolo[4,3-a][1,4]diazepin-6-yl)acetamido)propyl)amino)benzoate |
| 5-12' | 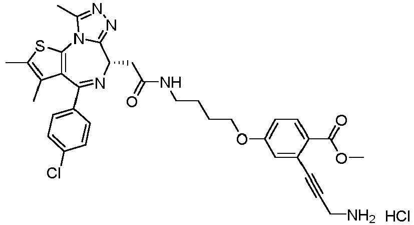 methyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butoxy)benzoate hydrochloride |
| 5-13' | 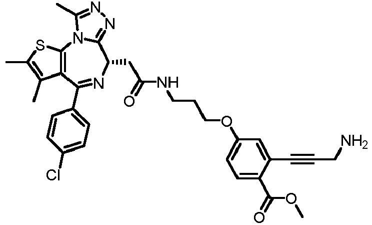 methyl (S)-2-(3-aminoprop-1-yn-1-yl)-4-(3-(2-(4-(4-chlorophenyl)-2,3,9-trimethyl-6H-thieno[3,2-f][1,2,4]triazolo[4,3-a][1,4]diazepin-6-yl)acetamido)propoxy)benzoate |
| 5-14' | 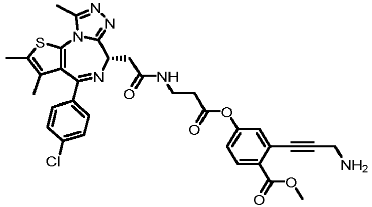 methyl (S)-2-(3-aminoprop-1-yn-1-yl)-4-((3-(2-(4-(4-chlorophenyl)-2,3,9-trimethyl-6H-thieno[3,2-f][1,2,4]triazolo[4,3-a][1,4]diazepin-6-yl)acetamido)propanoyl)oxy)benzoate |
| 5-15' | 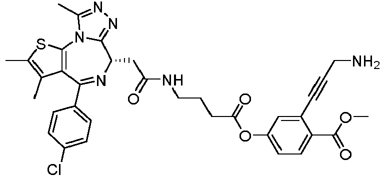 methyl (S)-2-(3-aminoprop-1-yn-1-yl)-4-((4-(2-(4-(4-chlorophenyl)-2,3,9-trimethyl-6H-thieno[3,2-f][1,2,4]triazolo[4,3-a][1,4]diazepin-6-yl)acetamido)butanoyl)oxy)benzoate |
| 5-16' | 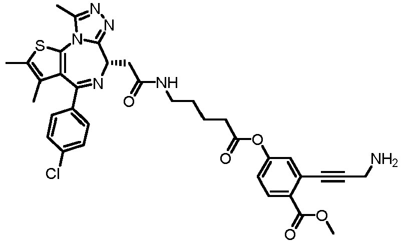 methyl (S)-2-(3-aminoprop-1-yn-1-yl)-4-((5-(2-(4-(4-chlorophenyl)-2,3,9-trimethyl-6H-thieno[3,2-f][1,2,4]triazolo[4,3-a][1,4]diazepin-6-yl)acetamido)pentanoyl)oxy)benzoate |
| 5-17' | 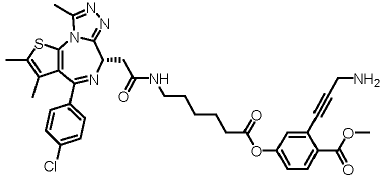 methyl (S)-2-(3-aminoprop-1-yn-1-yl)-4-((6-(2-(4-(4-chlorophenyl)-2,3,9-trimethyl-6H-thieno[3,2-f][1,2,4]triazolo[4,3-a][1,4]diazepin-6-yl)acetamido)hexanoyl)oxy)benzoate |
| 5-18' | 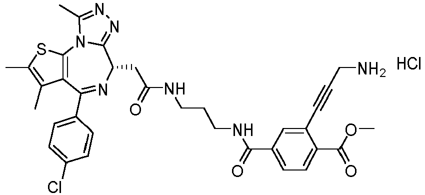 methyl (S)-2-(3-aminoprop-1-yn-1-yl)-4-((3-(2-(4-(4-chlorophenyl)-2,3,9-trimethyl-6H-thieno[3,2-f][1,2,4]triazolo[4,3-a][1,4]diazepin-6-yl)acetamido)propyl)carbamoyl)benzoate hydrochloride |
| 5-19' | 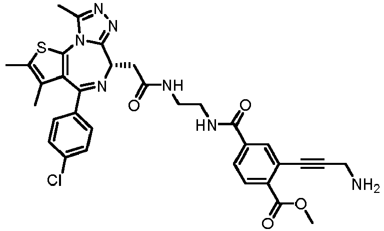 methyl (S)-2-(3-aminoprop-1-yn-1-yl)-4-((2-(2-(4-(4-chlorophenyl)-2,3,9-trimethyl-6H-thieno[3,2-f][1,2,4]triazolo[4,3-a][1,4]diazepin-6-yl)acetamido)ethyl)carbamoyl)benzoate |
| 5-20' | 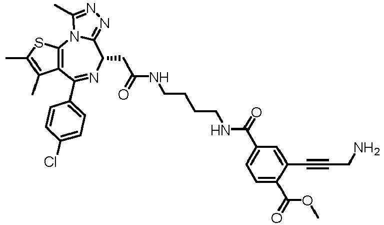 methyl (S)-2-(3-aminoprop-1-yn-1-yl)-4-((4-(2-(4-(4-chlorophenyl)-2,3,9-trimethyl-6H-thieno[3,2-f][1,2,4]triazolo[4,3-a][1,4]diazepin-6-yl)acetamido)butyl)carbamoyl)benzoate |
| 5-21' | 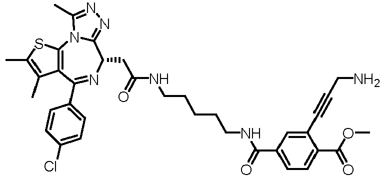 methyl (S)-2-(3-aminoprop-1-yn-1-yl)-4-((5-(2-(4-(4-chlorophenyl)-2,3,9-trimethyl-6H-thieno[3,2-f][1,2,4]triazolo[4,3-a][1,4]diazepin-6-yl)acetamido)pentyl)carbamoyl)benzoate |
| 5-30' | 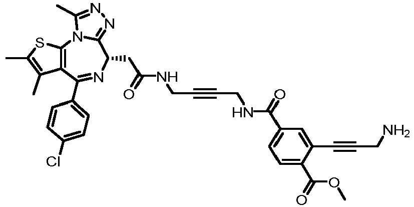 methyl (S)-2-(3-aminoprop-1-yn-1-yl)-4-((4-(2-(4-(4-chlorophenyl)-2,3,9-trimethyl-6H-thieno[3,2-f][1,2,4]triazolo[4,3-a][1,4]diazepin-6-yl)acetamido)but-2-yn-1-yl)carbamoyl)benzoate |
| 5-31' | 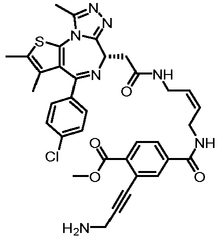 methyl (S,Z)-2-(3-aminoprop-1-yn-1-yl)-4-((4-(2-(4-(4-chlorophenyl)-2,3,9-trimethyl-6H-thieno[3,2-f][1,2,4]triazolo[4,3-a][1,4]diazepin-6-yl)acetamido)but-2-en-1-yl)carbamoyl)benzoate |
| 5-34' | 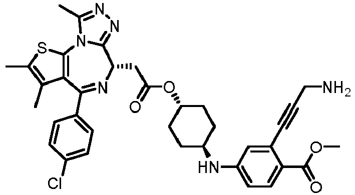 methyl 2-(3-aminoprop-1-yn-1-yl)-4-(((1S,4r)-4-(2-((S)-4-(4-chlorophenyl)-2,3,9-trimethyl-6H-thieno[3,2-f][1,2,4]triazolo[4,3-a][1,4]diazepin-6-yl)acetoxy)cyclohexyl)amino)benzoate |
| 5-35' | 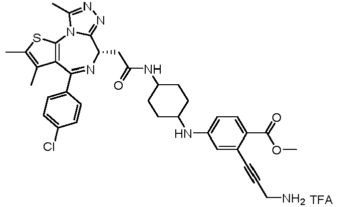 methyl (S)-2-(3-aminoprop-1-yn-1-yl)-4-((4-(2-(4-(4-chlorophenyl)-2,3,9-trimethyl-6H-thieno[3,2-f][1,2,4]triazolo[4,3-a][1,4]diazepin-6-yl)acetamido)cyclohexyl)amino)benzoate trifluoroacetic acid |
| 5-49' | 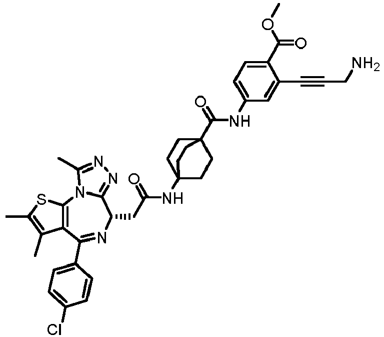 methyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)bicyclo[2.2.2]octane-1-carboxamido)benzoate |
| 5-50' | 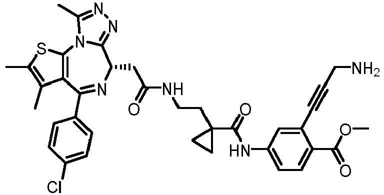 methyl (S)-2-(3-aminoprop-1-yn-1-yl)-4-(1-(2-(2-(4-(4-chlorophenyl)-2,3,9-trimethyl-6H-thieno[3,2-f][1,2,4]triazolo[4,3-a][1,4]diazepin-6-yl)acetamido)ethyl)cyclopropane-1-carboxamido)benzoate |
| 5-53' | 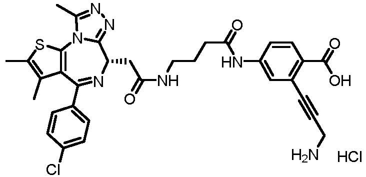 (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzoic acid hydrochloride |
| 5-54' | 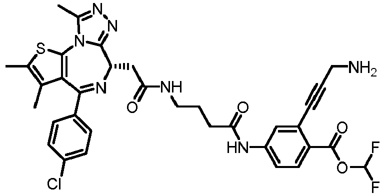 difluoromethyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzoate |
| 5-55' | 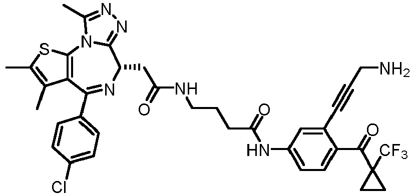 (S)-N-(3-(3-aminoprop-1-yn-1-yl)-4-(1-(trifluoromethyl)cyclopropane-1-carbonyl)phenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide |
| 5-56' | 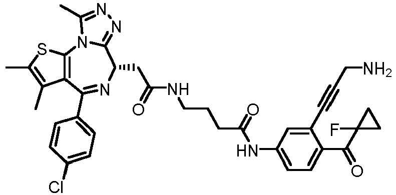 (S)-N-(3-(3-aminoprop-1-yn-1-yl)-4-(1-fluorocyclopropane-1-carbonyl)phenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide |
| 5-57' | 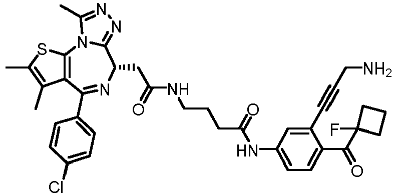 (S)-N-(3-(3-aminoprop-1-yn-1-yl)-4-(1-fluorocyclobutane-1-carbonyl)phenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide |
| 5-58' | 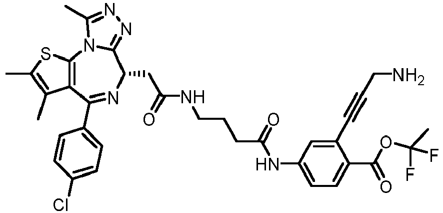 1,1-difluoroethyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzoate |
| 5-59' | 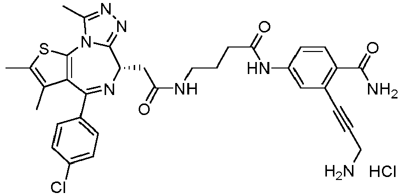 (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzamide hydrochloride |
| 5-60' | 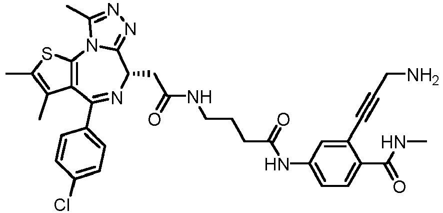 (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)-N-methylbenzamide |
| 5-61' | 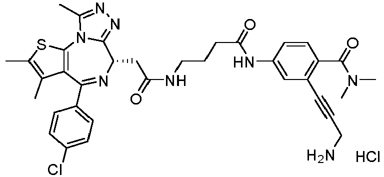 (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)-N,N-dimethylbenzamide hydrochloride |
| 5-62' | 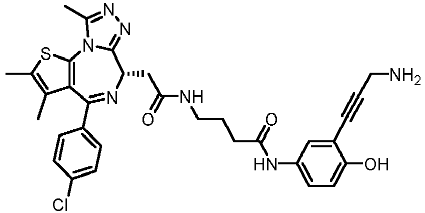 (S)-N-(3-(3-aminoprop-1-yn-1-yl)-4-hydroxyphenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide |
| 5-63' | 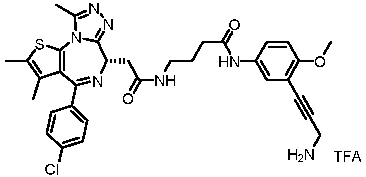 (S)-N-(3-(3-aminoprop-1-yn-1-yl)-4-methoxyphenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide trifluoroacetic acid |
| 5-64' | 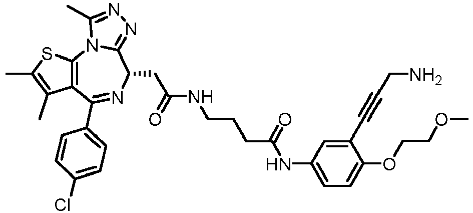 (S)-N-(3-(3-aminoprop-1-yn-1-yl)-4-(2-methoxyethoxy)phenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide |
| 5-65' | 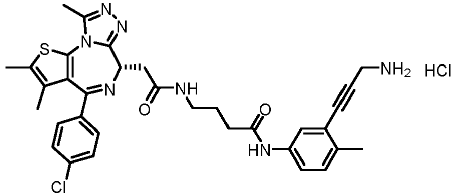 (S)-N-(3-(3-aminoprop-1-yn-1-yl)-4-methylphenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide hydrochloride |
| 5-66' | 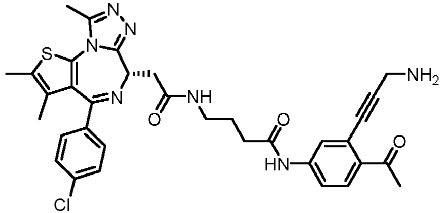 (S)-N-(4-acetyl-3-(3-aminoprop-1-yn-1-yl)phenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide |
| 5-67' | 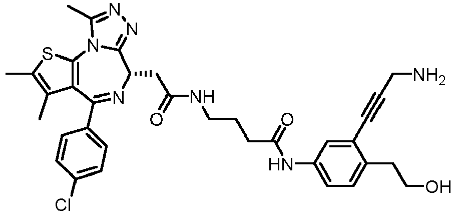 (S)-N-(3-(3-aminoprop-1-yn-1-yl)-4-(2-hydroxyethyl)phenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide |
| 5-68' | 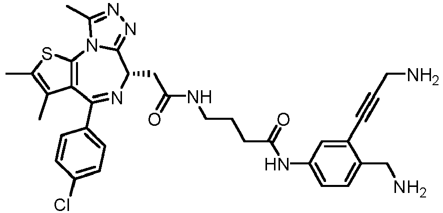 (S)-N-(4-(aminomethyl)-3-(3-aminoprop-1-yn-1-yl)phenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide |
| 5-69' | 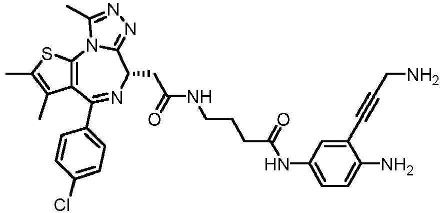 (S)-N-(4-amino-3-(3-aminoprop-1-yn-1-yl)phenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide |
| 5-70' | 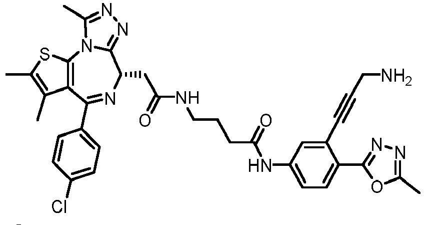 (S)-N-(3-(3-aminoprop-1-yn-1-yl)-4-(5-methyl-1,3,4-oxadiazol-2-yl)phenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide |
| 5-71' | 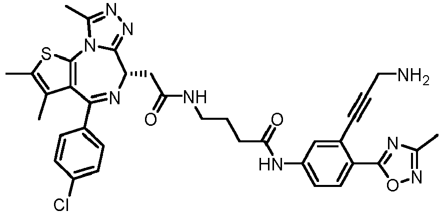 (S)-N-(3-(3-aminoprop-1-yn-1-yl)-4-(3-methyl-1,2,4-oxadiazol-5-yl)phenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide |
| 5-72' | 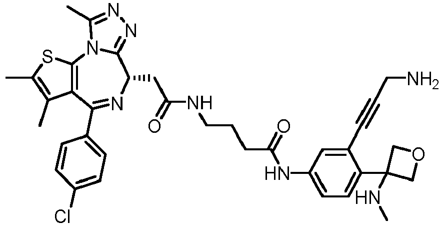 (S)-N-(3-(3-aminoprop-1-yn-1-yl)-4-(3-(methylamino)oxetan-3-yl)phenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide |
| 5-73' | 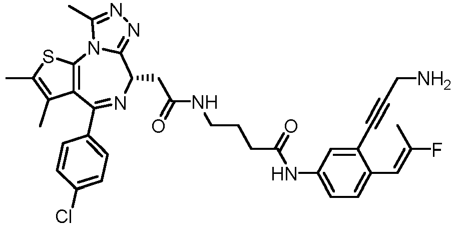 (S, E)-N-(3-(3-aminoprop-1-yn-1-yl)-4-(2-fluoroprop-1-en-1-yl)phenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide |
| 5-74' | 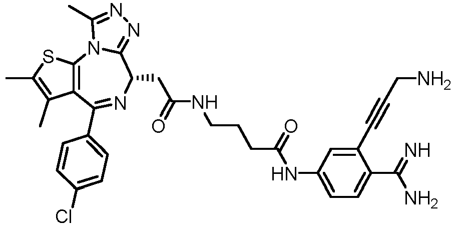 (S)-N-(3-(3-aminoprop-1-yn-1-yl)-4-carbamimidoylphenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide |
| 5-75' | 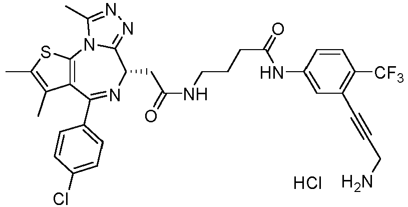 (S)-N-(3-(3-aminoprop-1-yn-1-yl)-4-(trifluoromethyl)phenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide hydrochloride |
| 5-76' | 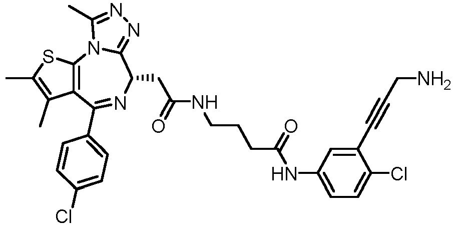 (S)-N-(3-(3-aminoprop-1-yn-1-yl)-4-chlorophenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide |
| 5-77' | 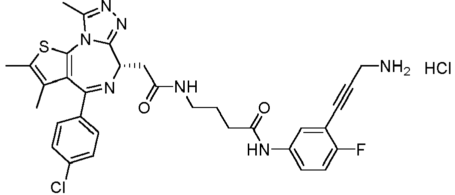 (S)-N-(3-(3-aminoprop-1-yn-1-yl)-4-fluorophenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide hydrochloride |
| 5-78' | 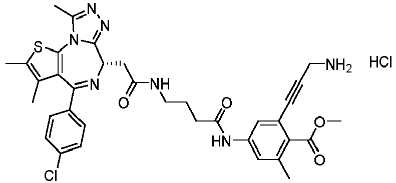 methyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)-6-methylbenzoate hydrochloride |
| 5-79' | 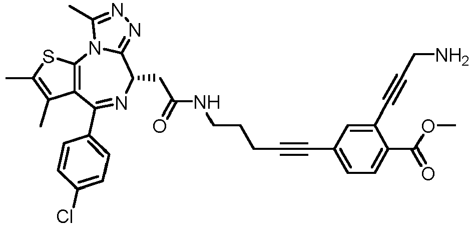 methyl (S)-2-(3-aminoprop-1-yn-1-yl)-4-(5-(2-(4-(4-chlorophenyl)-2,3,9-trimethyl-6H-thieno[3,2-f][1,2,4]triazolo[4,3-a][1,4]diazepin-6-yl)acetamido)pent-1-yn-1-yl)benzoate |
| 5-80' | 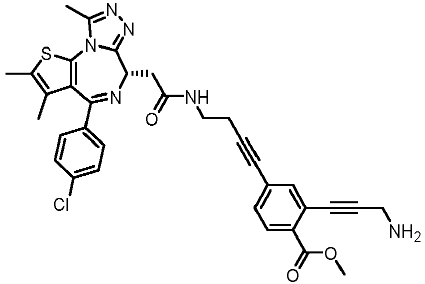 methyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)but-1-yn-1-yl)benzoate |
| 5-81' | 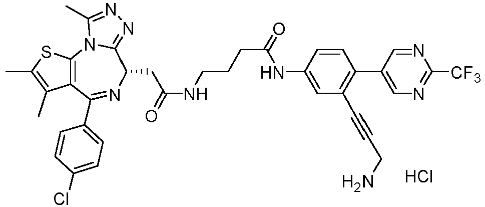 (S)-N-(3-(3-aminoprop-1-yn-1-yl)-4-(2-(trifluoromethyl)pyrimidin-5-yl)phenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide hydrochloride |
| 5-82' | 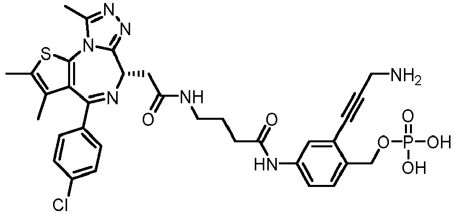 (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzyl dihydrogen phosphate |
| 5-83' | 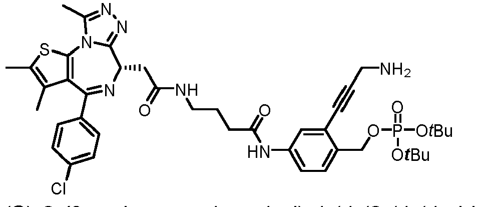 (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzyl di-tert-butyl phosphate |
| 5-87' | 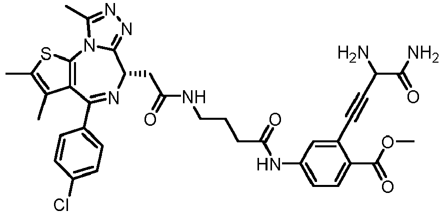 methyl 4-(4-(2-((S)-4-(4-chlorophenyl)-2,3,9-trimethyl-6H-thieno[3,2-f][1,2,4]triazolo[4,3-a][1,4]diazepin-6-yl)acetamido)butanamido)-2-(3,4-diamino-4-oxobut-1-yn-1-yl)benzoate |
| 5-89' | 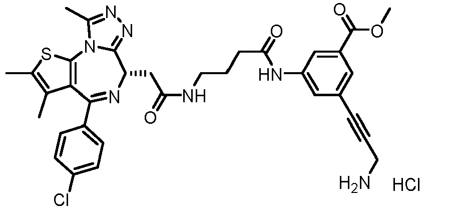 methyl (b)-3-(3-aminoprop-1-yn-1-yl)-5-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzoate hydrochloride |
| 5-90' | 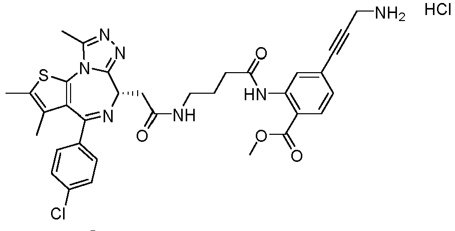 methyl (S)-4-(3-aminoprop-1-yn-1-yl)-2-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzoate hydrochloride |
| 5-92' | 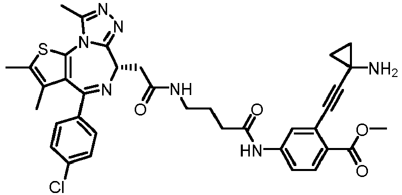 methyl (S)-2-((1-aminocyclopropyl)ethynyl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzoate |
| 5-93' | 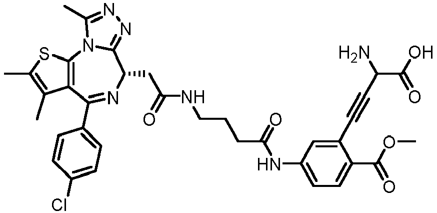 2-amino-4-(5-(4-(2-((S)-4-(4-chlorophenyl)-2,3,9-trimethyl-6H-thieno[3,2-f][1,2,4]triazolo[4,3-a][1,4]diazepin-6-yl)acetamido)butanamido)-2-(methoxycarbonyl)phenyl)but-3-ynoic acid |
| 5-94' | 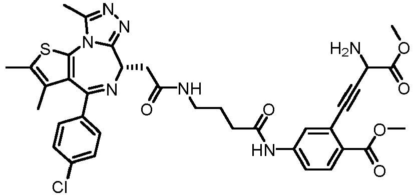 methyl 2-(3-am i no-4-methoxy-4-oxobut-1-yn-1-yl)-4-(4-(2-((S)-4-(4-chlorophenyl)-2,3,9-trimethyl-6H-thieno[3,2-f][1,2,4]triazolo[4,3-a][1,4]diazepin-6-yl)acetamido)butanamido)benzoate |
| 5-95' | 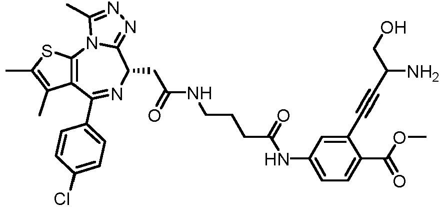 methyl 2-(3-amino-4-hydroxybut-1-yn-1-yl)-4-(4-(2-((S)-4-(4-chlorophenyl)-2,3,9-trimethyl-6H-thieno[3,2-f][1,2,4]triazolo[4,3-a][1,4]diazepin-6-yl)acetamido)butanamido)benzoate |
| 5-96' | 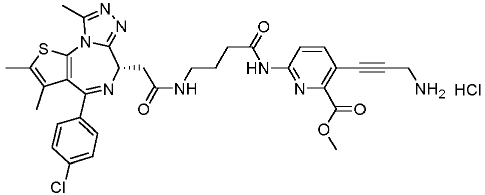 methyl (S)-3-(3-aminoprop-1-yn-1-yl)-6-(4-(2-(4-(4-chlorophenyl)-2,3,9-trimethyl-6H-thieno[3,2-f][1,2,4]triazolo[4,3-a][1,4]diazepin-6-yl)acetamido)butanamido)picolinate hydrochloride |
| 5-97' | 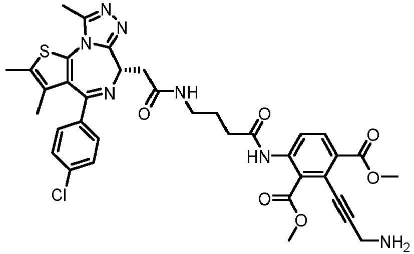 dimethyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)isophthalate |
| 5-98' | 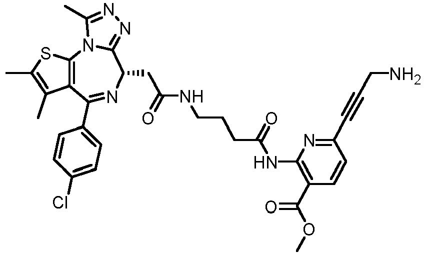 methyl (S)-6-(3-aminoprop-1-yn-1-yl)-2-(4-(2-(4-(4-chlorophenyl)-2,3,9-trimethyl-6H-thieno[3,2-f][1,2,4]triazolo[4,3-a][1,4]diazepin-6-yl)acetamido)butanamido)nicotinate |
| 5-99' | 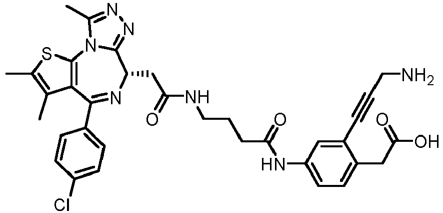 (S)-2-(2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)phenyl)acetic acid |
| 5-100' | 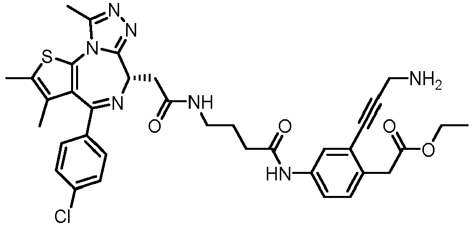 ethyl (S)-2-(2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)phenyl)acetate |
| 5-101' | 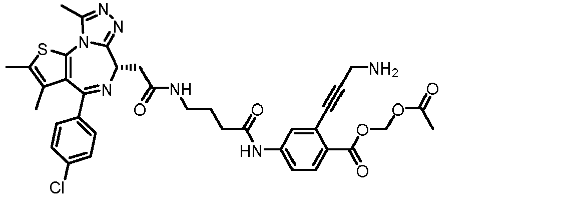 acetoxymethyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzoate |
| 5-102' | 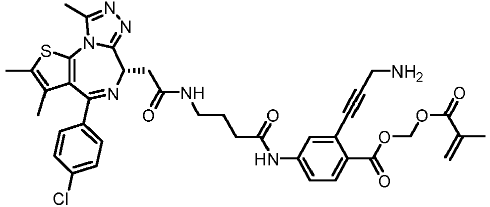 (methacryloyloxy)methyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzoate |
| 5-103' | 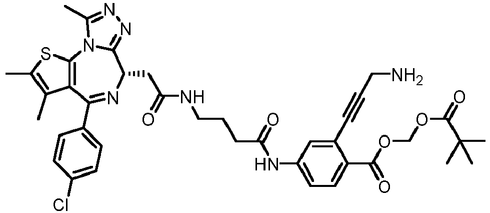 (pivaloyloxy)methyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzoate |
| 5-104' | 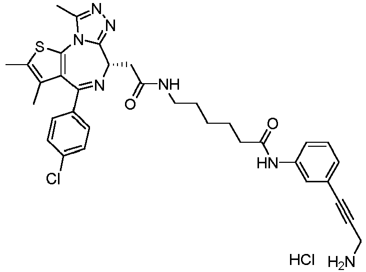 (S)-N-(3-(3-aminoprop-1-yn-1-yl)phenyl)-6-(2-(4-(4-chlorophenyl)-2,3,9-trimethyl-6H-thieno[3,2-f][1,2,4]triazolo[4,3-a][1,4]diazepin-6-yl)acetamido)hexanamide hydrochloride |
| 5-106' | 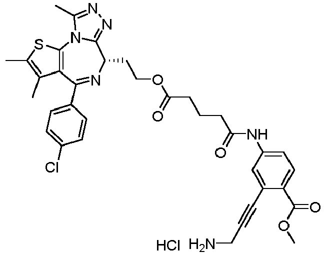 methyl (S)-2-(3-aminoprop-1-yn-1-yl)-4-(5-(2-(4-(4-chlorophenyl)-2,3,9-trimethyl-6H-thieno[3,2-f][1,2,4]triazolo[4,3-a][1,4]diazepin-6-yl)ethoxy)-5-oxopentanamido)benzoate hydrochloride |
| 5-107' | 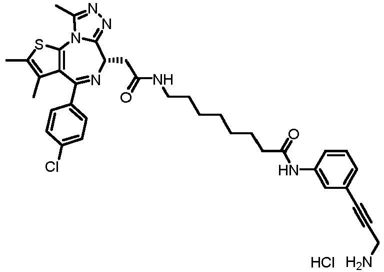 (S)-N-(3-(3-aminoprop-1-yn-1-yl)phenyl)-8-(2-(4-(4-chlorophenyl)-2,3,9-trimethyl-6H-thieno[3,2-f][1,2,4]triazolo[4,3-a][1,4]diazepin-6-yl)acetamido)octanamide hydrochloride |
| 5-108' | 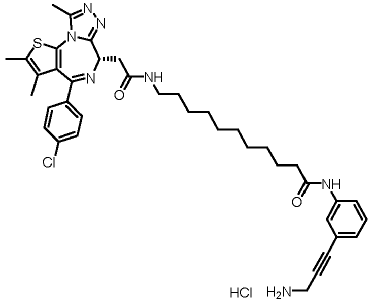 (S)-N-(3-(3-aminoprop-1-yn-1-yl)phenyl)-11-(2-(4-(4-chlorophenyl)-2,3,9-trimethyl-6H-thieno[3,2-f][1,2,4]triazolo[4,3-a][1,4]diazepin-6-yl)acetamido)undecanamide hydrochloride |
| 5-109' | 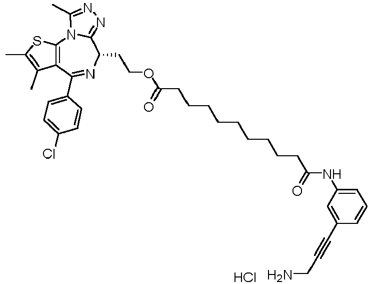 (S)-2-(4-(4-chlorophenyl)-2,3,9-trimethyl-6H-thieno[3,2-f][1,2,4]triazolo[4,3-a][1,4]diazepin-6-yl)ethyl 11-((3-(3-aminoprop-1-yn-1-yl)phenyl)amino)-11-oxoundecanoate hydrochloride |
| 5-110' | 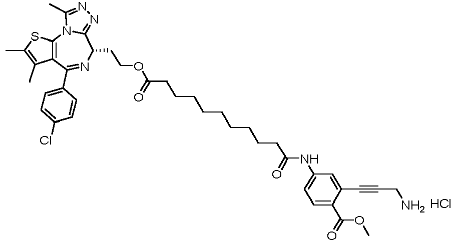 methyl (S)-2-(3-aminoprop-1-yn-1-yl)-4-(11-(2-(4-(4-chlorophenyl)-2,3,9-trimethyl-6H-thieno[3,2-f][1,2,4]triazolo[4,3-a][1,4]diazepin-6-yl)ethoxy)-11-oxoundecanamido)benzoate hydrochloride |
| 5-113' | 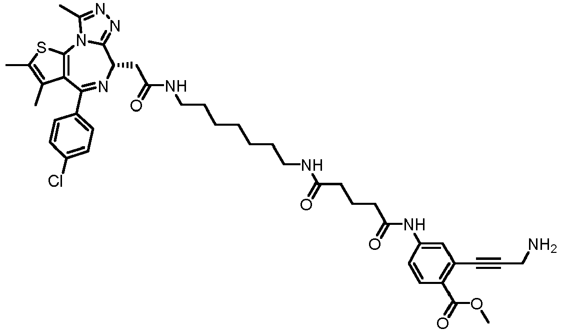 methyl (S)-2-(3-aminoprop-1-yn-1-yl)-4-(5-((7-(2-(4-(4-chlorophenyl)-2,3,9-trimethyl-6H-thieno[3,2-f][1,2,4]triazolo[4,3-a][1,4]diazepin-6-yl)acetamido)heptyl)amino)-5-oxopentanamido)benzoate |
| 5-115' | 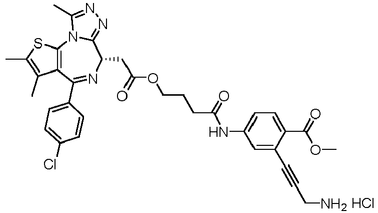 methyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetoxy)butanamido)benzoate hydrochloride |
| 5-121' | 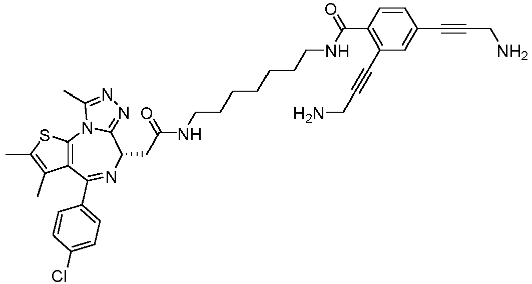 (S)-2,4-bis(3-aminoprop-1-yn-1-yl)-N-(7-(2-(4-(4-chlorophenyl)-2,3,9-trimethyl-6H-thieno[3,2-f][1,2,4]triazolo[4,3-a][1,4]diazepin-6-yl)acetamido)heptyl)benzamide |
| 5-122' | 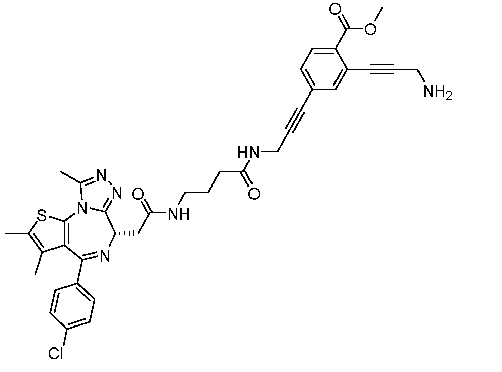 methyl (b)-2-(3-aminoprop-1-yn-1-yl)-4-(3-(4-(2-(4-(4-chlorophenyl)-2,3,9-trimethyl-6H-thieno[3,2-f][1,2,4]triazolo[4,3-a][1,4]diazepin-6-yl)acetamido)butanamido)prop-1-yn-1-yl)benzoate |
| 5-123' | 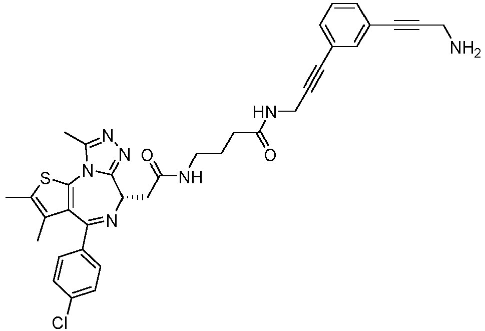 (S)-N-(3-(3-(3-aminoprop-1-yn-1-yl)phenyl)prop-2-yn-1-yl)-4-(2-(4-(4-chlorophenyl)-2,3,9-trimethyl-6H-thieno[3,2-f][1,2,4]triazolo[4,3-a][1,4]diazepin-6-yl)acetamido)butanamide |
| 5-126' | 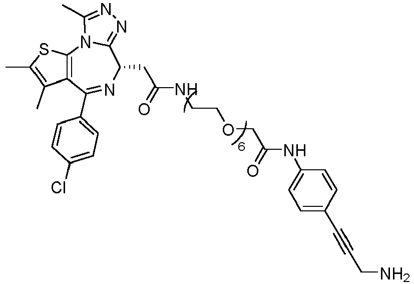 (S)-N-(4-(3-aminoprop-1-yn-1-yl)phenyl)-20-(2-(4-(4-chlorophenyl)-2,3,9-trimethyl-6H-thieno[3,2-f][1,2,4]triazolo[4,3-a][1,4]diazepin-6-yl)acetamido)-3,6,9,12,15,18-hexaoxaicosanamide |
| 5-127' | 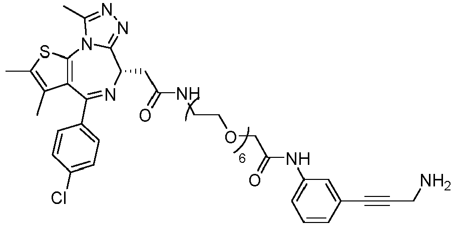 (S)-N-(3-(3-aminoprop-1-yn-1-yl)phenyl)-20-(2-(4-(4-chlorophenyl)-2,3,9-trimethyl-6H-thieno[3,2-f][1,2,4]triazolo[4,3-a][1,4]diazepin-6-yl)acetamido)-3,6,9,12,15,18-hexaoxaicosanamide |
| 5-135' | 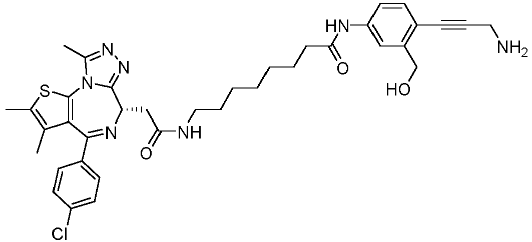 (S)-N-(4-(3-aminoprop-1-yn-1-yl)-3-(hydroxymethyl)phenyl)-8-(2-(4-(4-chlorophenyl)-2,3,9-trimethyl-6H-thieno[3,2-f][1,2,4]triazolo[4,3-a][1,4]diazepin-6-yl)acetamido)octanamide |
| 5-148' | 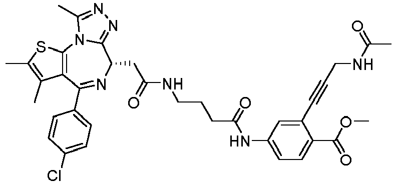 methyl (S)-2-(3-acetamidoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzoate |
| 5-149' | 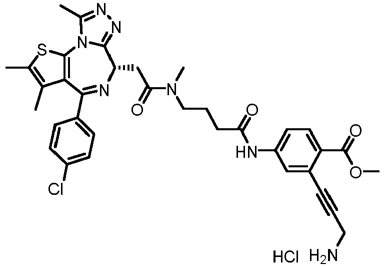 methyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)-N-methylacetamido)butanamido)benzoate hydrochloride |
| 5-150' | 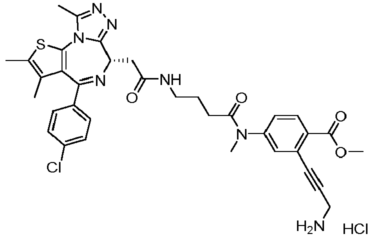 methyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)-N-methylbutanamido)benzoate hydrochloride |
| 5-169' | 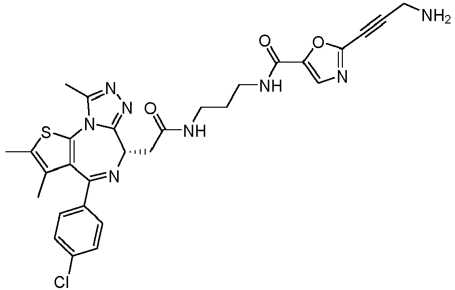 (S)-2-(3-aminoprop-1-yn-1-yl)-N-(3-(2-(4-(4-chlorophenyl)-2,3,9-trimethyl-6H-thieno[3,2-f][1,2,4]triazolo[4,3-a][1,4]diazepin-6-yl)acetamido)propyl)oxazole-5-carboxamide |
| 5-170' | 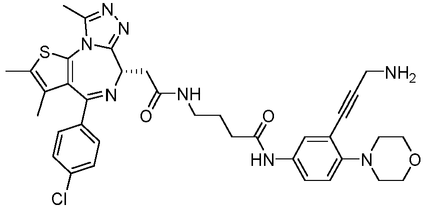 (S)-N-(3-(3-aminoprop-1-yn-1-yl)-4-morpholinophenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide |
| 5-171' | 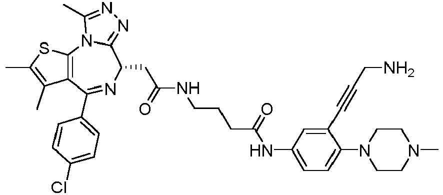 (S)-N-(3-(3-aminoprop-1-yn-1-yl)-4-(4-methylpiperazin-1-yl)phenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide |

**[Table 9]**

| No. | Compound |
|---|---|
| 4-165" | 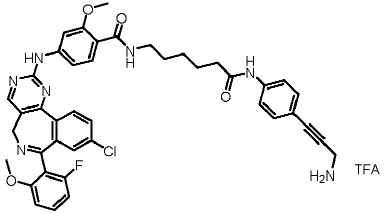 N-(6-((4-(3-aminoprop-1-yn-1-yl)phenyl)amino)-6-oxohexyl)-4-((9-chloro-7-(2-fluoro-6-methoxyphenyl)-5H-benzo[c]pyrimido[4,5-e]azepin-2-yl)amino)-2-methoxybenzamide trifluoroacetic acid |
| 4-166" | 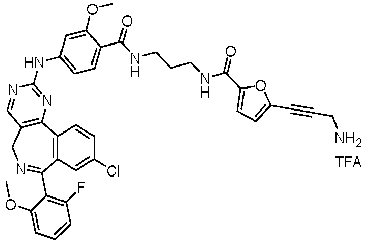 5-(3-aminoprop-1-yn-1-yl)-N-(3-(4-((9-chloro-7-(2-fluoro-6-methoxyphenyl)-5H-benzo[c]pyrimido[4,5-e]azepin-2-yl)amino)-2-methoxybenzamido)propyl)furan-2-carboxamide trifluoroacetic acid |
| 4-228" | 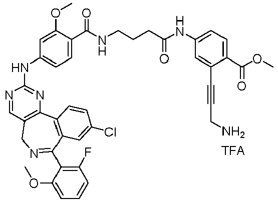 methyl 2-(3-aminoprop-1-yn-1-yl)-4-(4-(4-((9-chloro-7-(2-fluoro-6-methoxyphenyl)-5H-benzo[c]pyrimido[4,5-e]azepin-2-yl)amino)-2-methoxybenzamido)butanamido)benzoate trifluoroacetic acid |
| 5-8" | 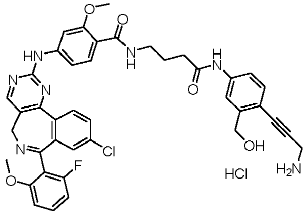 N-(4-((4-(3-aminoprop-1-yn-1-yl)-3-(hydroxymethyl)phenyl)amino)-4-oxobutyl)-4-((9-chloro-7-(2-fluoro-6-methoxyphenyl)-5H-benzo[c]pyrimido[4,5-e]azepin-2-yl)amino)-2-methoxybenzamide trifluoroacetic acid |
| 5-18" | 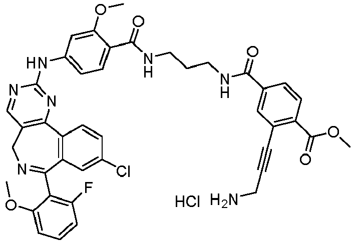 methyl 2-(3-aminoprop-1-yn-1-yl)-4-((3-(4-((9-chloro-7-(2-fluoro-6-methoxyphenyl)-5H-benzo[c]pyrimido[4,5-e]azepin-2-yl)amino)-2-methoxybenzamido)propyl)carbamoyl)benzoate hydrochloride |
| 5-35" | 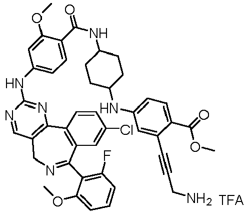 methyl 2-(3-aminoprop-1-yn-1-yl)-4-((4-(4-((9-chloro-7-(2-fluoro-6-methoxyphenyl)-5H-benzo[c]pyrimido[4,5-e]azepin-2-yl)amino)-2-methoxybenzamido)cyclohexyl)amino)benzoate trifluoroacetic acid |
| 5-81" | 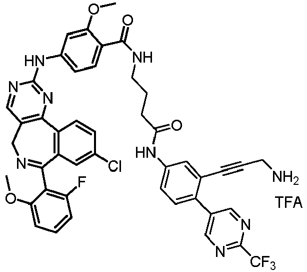 N-(4-((3-(3-aminoprop-1-yn-1-yl)-4-(2-(trifluoromethyl)pyrimidin-5-yl)phenyl)amino)-4-oxobutyl)-4-((9-chloro-7-(2-fluoro-6-methoxyphenyl)-5H-benzo[c]pyrimido[4,5-e]azepin-2-yl)amino)-2-methoxybenzamide trifluoroacetic acid |
| 5-104" | 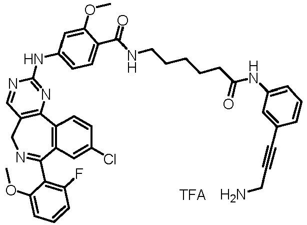 N-(6-((3-(3-aminoprop-1-yn-1-yl)phenyl)amino)-6-oxohexyl)-4-((9-chloro-7-(2-fluoro-6-methoxyphenyl)-5H-benzo[c]pyrimido[4,5-e]azepin-2-yl)amino)-2-methoxybenzamide trifluoroacetic acid |
| 5-150" | 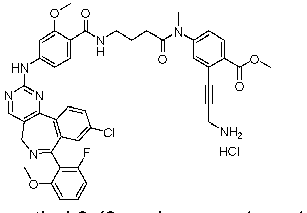 methyl 2-(3-ammoprop-1-yn-1-yl)-4-(4-(4-((9-chloro-7-(2-fluoro-6-methoxyphenyl)-5H-benzo[c]pyrimido[4,5-e]azepin-2-yl)amino)-2-methoxybenzamido)-N-methylbutanamido)benzoate trifluoroacetic acid |

**[Table 10]**

| No. | Compound |
|---|---|
| 4-166''' | 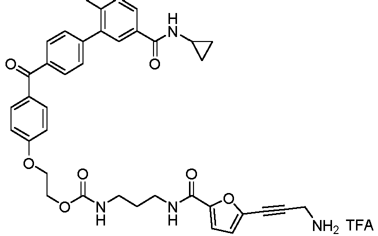 2-(4-(5'-(cyclopropylcarbamoyl)-2'-methyl-[1,1'-biphenyl]-4-carbonyl)phenoxy)ethyl (3-(5-(3-aminoprop-1-yn-1-yl)furan-2-carboxamido)propyl)carbamate trifluoroacetic acid |
| 5-106''' | 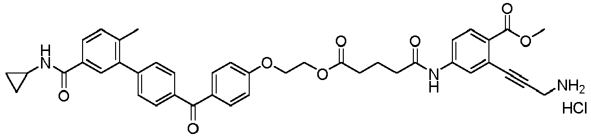 methyl 2-(3-aminoprop-1-yn-1-yl)-4-(5-(2-(4-(5'-(cyclopropylcarbamoyl)-2'-methyl-[1,1'-biphenyl]-4-carbonyl)phenoxy)ethoxy)-5-oxopentanamido)benzoate hydrochloride |

More specifically, in the compound represented by Formula VII above, specific examples of the case where the compound of Formula 1 above is bound to the TBM may be presented in Table 11 below, specific examples of the case where the compound of Formula 2 above is bound to the TBM may be presented in Table 12 below, and specific examples of the case where the compound of Formula 3 above is bound to the TBM may be presented in Table 13 below, but are not limited thereto.

**[Table 11]**

| No. | Compound |
|---|---|
| 4-2' | 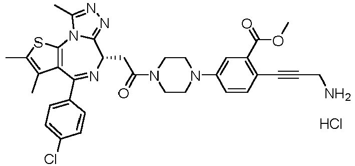 methyl (S)-2-(3-aminoprop-1-yn-1-yl)-5-(4-(2-(4-(4-chlorophenyl)-2,3,9-trimethyl-6H-thieno[3,2-f][1,2,4]triazolo[4,3-a][1,4]diazepin-6-yl)acetyl)piperazin-1-yl)benzoate hydrochloride |
| 4-3' | 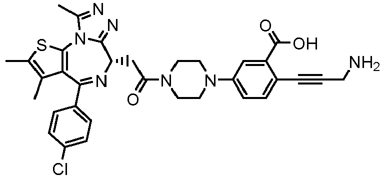 (S)-2-(3-aminoprop-1-yn-1-yl)-5-(4-(2-(4-(4-chlorophenyl)-2,3,9-trimethyl-6H-thieno[3,2-f][1,2,4]triazolo[4,3-a][1,4]diazepin-6-yl)acetyl)piperazin-1-yl)benzoic acid |
| 4-4' | 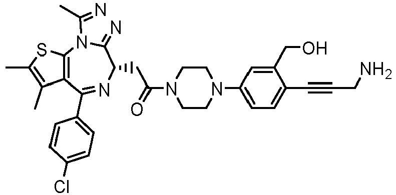 (S)-1-(4-(4-(3-aminoprop-1-yn-1-yl)-3-(hydroxymethyl)phenyl)piperazin-1-yl)-2-(4-(4-chlorophenyl)-2,3,9-trimethyl-6H-thieno[3,2-f][1,2,4]triazolo[4,3-a][1,4]diazepin-6-yl)ethan-1-one |
| 4-94' | 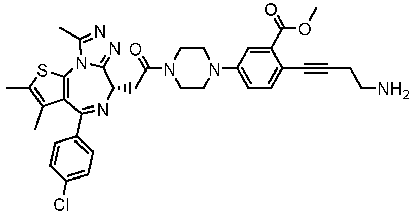 methyl (S)-2-(4-aminobut-1-yn-1-yl)-5-(4-(2-(4-(4-chlorophenyl)-2,3,9-trimethyl-6H-thieno[3,2-f][1,2,4]triazolo[4,3-a][1,4]diazepin-6-yl)acetyl)piperazin-1-yl)benzoate |
| 4-95' | 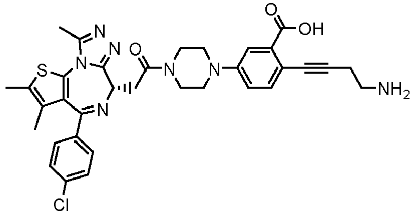 (S)-2-(4-aminobut-1-yn-1-yl)-5-(4-(2-(4-(4-chlorophenyl)-2,3,9-trimethyl-6H-thieno[3,2-f][1,2,4]triazolo[4,3-a][1,4]diazepin-6-yl)acetyl)piperazin-1-yl)benzoic acid |
| 4-96' | 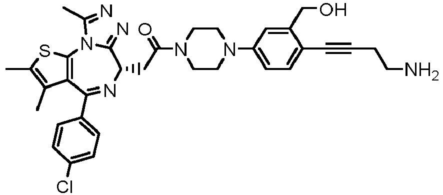 (S)-1-(4-(4-(4-aminobut-1-yn-1-yl)-3-(hydroxymethyl)phenyl)piperazin-1-yl)-2-(4-(4-chlorophenyl)-2,3,9-trimethyl-6H-thieno[3,2-f][1,2,4]triazolo[4,3-a][1,4]diazepin-6-yl)ethan-1-one |
| 4-97' | 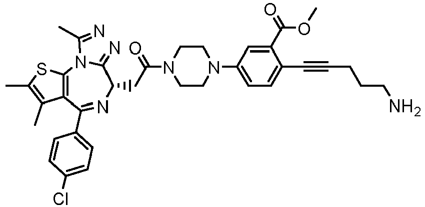 methyl (S)-2-(5-aminopent-1-yn-1-yl)-5-(4-(2-(4-(4-chlorophenyl)-2,3,9-trimethyl-6H-thieno[3,2-f][1,2,4]triazolo[4,3-a][1,4]diazepin-6-yl)acetyl)piperazin-1-yl)benzoate |
| 4-98' | 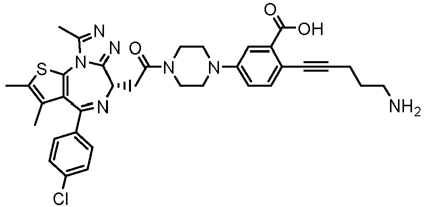 (S)-2-(5-aminopent-1-yn-1-yl)-5-(4-(2-(4-(4-chlorophenyl)-2,3,9-trimethyl-6H-thieno[3,2-f][1,2,4]triazolo[4,3-a][1,4]diazepin-6-yl)acetyl)piperazin-1-yl)benzoic acid |
| 4-99' | 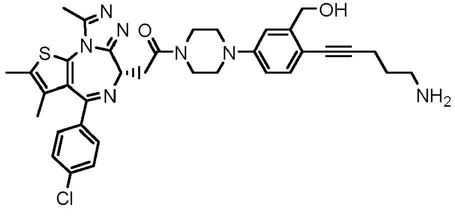 (S)-1-(4-(4-(5-aminopent-1-yn-1-yl)-3-(hydroxymetnyl)phenyl)piperazin-1-yl)-2-(4-(4-chlorophenyl)-2,3,9-trimethyl-6H-thieno[3,2-f][1,2,4]triazolo[4,3-a][1,4]diazepin-6-yl)ethan-1-one |
| 4-100' | 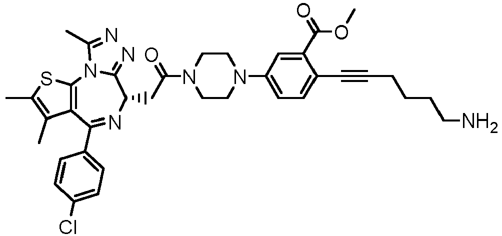 methyl (S)-2-(6-aminohex-1-yn-1-yl)-5-(4-(2-(4-(4-chlorophenyl)-2,3,9-trimethyl-6H-thieno[3,2-f][1,2,4]triazolo[4,3-a][1,4]diazepin-6-yl)acetyl)piperazin-1-yl)benzoate |
| 4-101' | 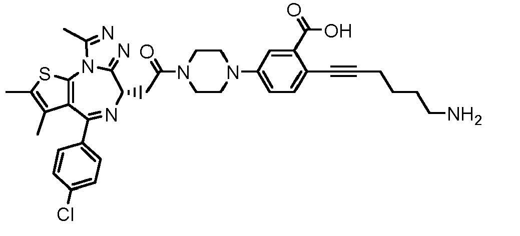 (S)-2-(6-aminonex-1-yn-1-yl)-5-(4-(2-(4-(4-cnioropnenyl)-2,3,9-trimethyl-6H-thieno[3,2-f][1,2,4]triazolo[4,3-a][1,4]diazepin-6-yl)acetyl)piperazin-1-yl)benzoic acid |
| 4-102' | 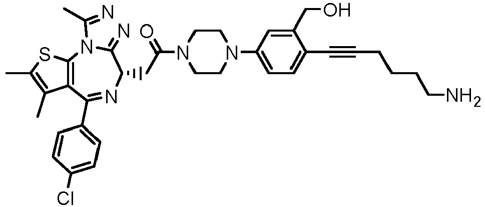 (S)-1-(4-(4-(6-aminohex-1-yn-1-yl)-3-(hydroxymethyl)phenyl)piperazin-1-yl)-2-(4-(4-chlorophenyl)-2,3,9-trimethyl-6H-thieno[3,2-f][1,2,4]triazolo[4,3-a][1,4]diazepin-6-yl)ethan-1-one |
| 4-103' | 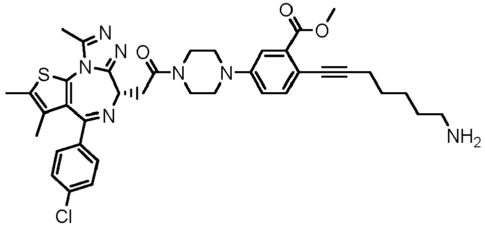 methyl (S)-2-(7-aminohept-1-yn-1-yl)-5-(4-(2-(4-(4-chlorophenyl)-2,3,9-trimethyl-6H-thieno[3,2-f][1,2,4]triazolo[4,3-a][1,4]diazepin-6-yl)acetyl)piperazin-1-yl)benzoate |
| 4-104' | 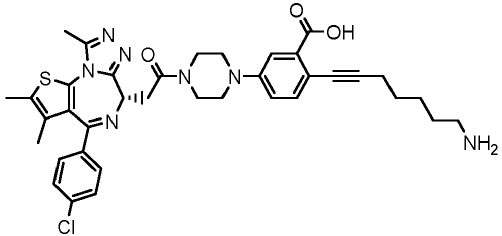 (S)-2-(7-aminohept-1-yn-1-yl)-5-(4-(2-(4-(4-chlorophenyl)-2,3,9-trimethyl-6H-thieno[3,2-f][1,2,4]triazolo[4,3-a][1,4]diazepin-6-yl)acetyl)piperazin-1-yl)benzoic acid |
| 4-105' | 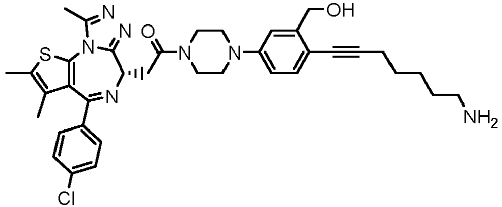 (S)-1-(4-(4-(7-aminohept-1-yn-1-yl)-3-(hydroxymethyl)phenyl)piperazin-1-yl)-2-(4-(4-chlorophenyl)-2,3,9-trimethyl-6H-thieno[3,2-f][1,2,4]triazolo[4,3-a][1,4]diazepin-6-yl)ethan-1-one |
| 4-106' | 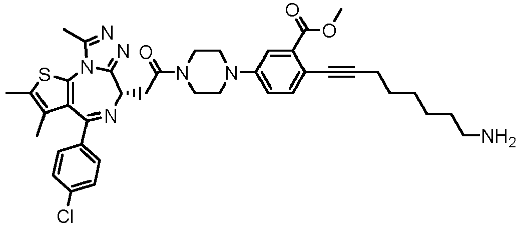 methyl (S)-2-(8-aminooct-1-yn-1-yl)-5-(4-(2-(4-(4-chlorophenyl)-2,3,9-trimethyl-6H-thieno[3,2-f][1,2,4]triazolo[4,3-a][1,4]diazepin-6-yl)acetyl)piperazin-1-yl)benzoate |
| 4-107' | 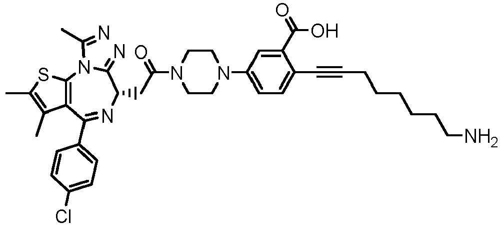 (S)-2-(8-aminooct-1-yn-1-yl)-5-(4-(2-(4-(4-chlorophenyl)-2,3,9-trimethyl-6H-thieno[3,2-f][1,2,4]triazolo[4,3-a][1,4]diazepin-6-yl)acetyl)piperazin-1-yl)benzoic acid |
| 4-108' | 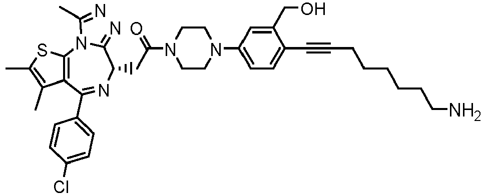 (S)-1-(4-(4-(8-aminooct-1-yn-1-yl)-3-(hydroxymethyl)phenyl)piperazin-1-yl)-2-(4-(4-chlorophenyl)-2,3,9-trimethyl-6H-thieno[3,2-f][1,2,4]triazolo[4,3-a][1,4]diazepin-6-yl)ethan-1-one |
| 4-134' | 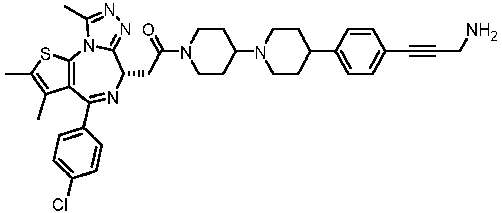 (S)-1-(4-(4-(3-aminoprop-1-yn-1-yl)phenyl)-[1,4'-bipiperidin]-1'-yl)-2-(4-(4-chlorophenyl)-2,3,9-trimethyl-6H-thieno[3,2-f][1,2,4]triazolo[4,3-a][1,4]diazepin-6-yl)ethan-1-one |
| 4-135' | 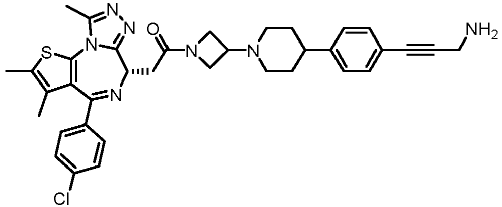 (S)-1-(3-(4-(4-(3-aminoprop-1-yn-1-yl)phenyl)piperidin-1-yl)azetidin-1-yl)-2-(4-(4-chlorophenyl)-2,3,9-trimethyl-6H-thieno[3,2-f][1,2,4]triazolo[4,3-a][1,4]diazepin-6-yl)ethan-1-one |
| 4-136' | 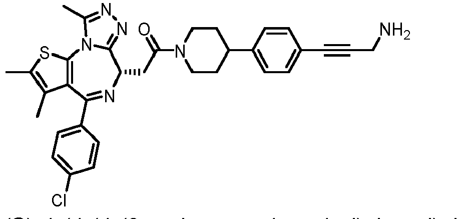 (S)-1-(4-(4-(3-aminoprop-1-yn-1-yl)phenyl)piperidin-1-yl)-2-(4-(4-chlorophenyl)-2,3,9-trimethyl-6H-thieno[3,2-f][1,2,4]triazolo[4,3-a][1,4]diazepin-6-yl)ethan-1-one |
| 4-146' | 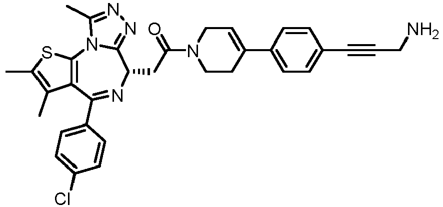 (S)-1-(4-(4-(3-aminoprop-1-yn-1-yl)phenyl)-3,6-dihydropyridin-1(2H)-yl)-2-(4-(4-chlorophenyl)-2,3,9-trimethyl-6H-thieno[3,2-f][1,2,4]triazolo[4,3-a][1,4]diazepin-6-yl)ethan-1-one |
| 4-226' | 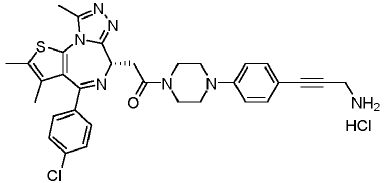 (S)-1-(4-(4-(3-aminoprop-1-yn-1-yl)phenyl)piperazin-1-yl)-2-(4-(4-chlorophenyl)-2,3,9-trimethyl-6H-thieno[3,2-f][1,2,4]triazolo[4,3-a][1,4]diazepin-6-yl)ethan-1-one hydrochloride |
| 4-227' | 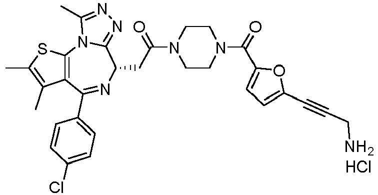 (S)-1-(4-(5-(3-aminoprop-1-yn-1-yl)furan-2-carbonyl)piperazin-1-yl)-2-(4-(4-chlorophenyl)-2,3,9-trimethyl-6H-thieno[3,2-f][1,2,4]triazolo[4,3-a][1,4]diazepin-6-yl)ethan-1-one hydrochloride |
| 5-7' | 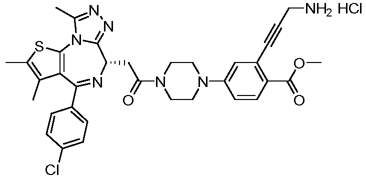 methyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetyl)piperazin-1-yl)benzoate hydrochloride |
| 5-22' | 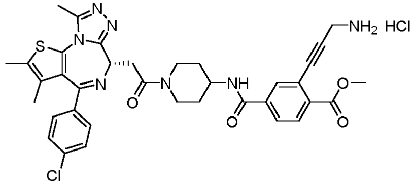 methyl (S)-2-(3-aminoprop-1-yn-1-yl)-4-((1-(2-(4-(4-chlorophenyl)-2,3,9-trimethyl-6H-thieno[3,2-f][1,2,4]triazolo[4,3-a][1,4]diazepin-6-yl)acetyl)piperidin-4-yl)carbamoyl)benzoate hydrochloride |
| 5-23' | 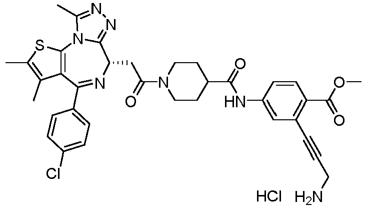 methyl (S)-2-(3-aminoprop-1-yn-1-yl)-4-(1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamido)benzoate hydrochloride |
| 5-24' | 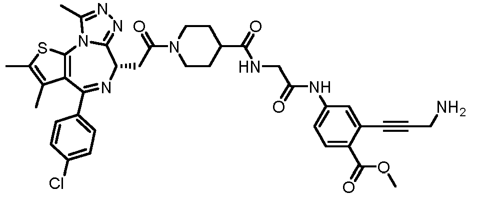 methyl (S)-2-(3-aminoprop-1-yn-1-yl)-4-(2-(1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamido)acetamido)benzoate |
| 5-25' | 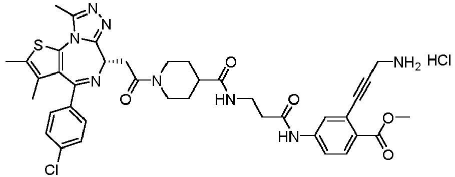 methyl (S)-2-(3-aminoprop-1-yn-1-yl)-4-(3-(1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamido)propanamido)benzoate hydrochloride |
| 5-26' | 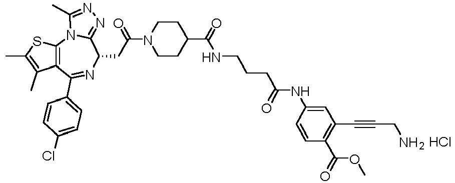 methyl (S)-2-(3-aminoprop-1-yn-1-yl)-4-(4-(1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamido)butanamido)benzoate hydrochloride |
| 5-27' | 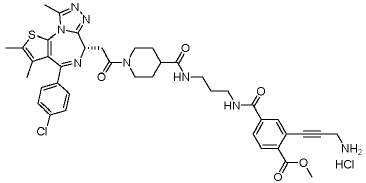 methyl (S)-2-(3-aminoprop-1-yn-1-yl)-4-((3-(1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamido)propyl)carbamoyl)benzoate hydrochloride |
| 5-28' | 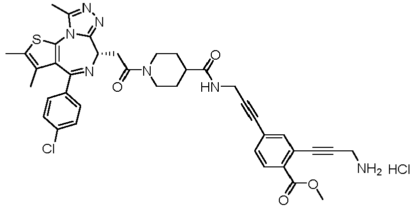 methyl (S)-2-(3-aminoprop-1-yn-1-yl)-4-(3-(1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamido)prop-1-yn-1-yl)benzoate hydrochloride |
| 5-29' | 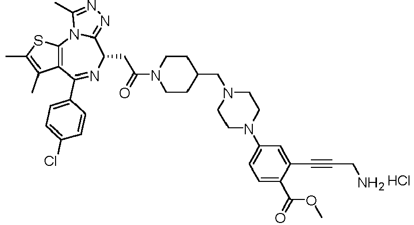 methyl (S)-2-(3-aminoprop-1-yn-1-yl)-4-(4-((1-(2-(4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperazin-1-yl)benzoate hydrochloride |
| 5-36' | 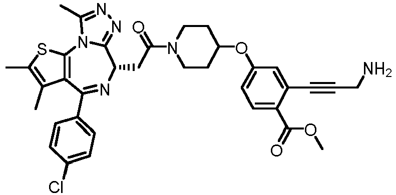 methyl (S)-2-(3-aminoprop-1-yn-1-yl)-4-((1-(2-(4-(4-chlorophenyl)-2,3,9-trimethyl-6H-thieno[3,2-f][1,2,4]triazolo[4,3-a][1,4]diazepin-6-yl)acetyl)piperidin-4-yl)oxy)benzoate |
| 5-37' | 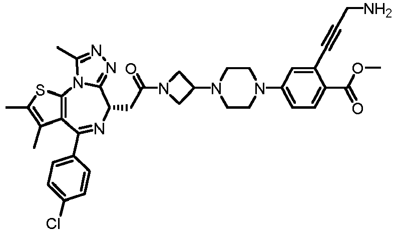 methyl (S)-2-(3-aminoprop-1-yn-1-yl)-4-(4-(1-(2-(4-(4-chlorophenyl)-2,3,9-trimethyl-6H-thieno[3,2-f][1,2,4]triazolo[4,3-a][1,4]diazepin-6-yl)acetyl)azetidin-3-yl)piperazin-1-yl)benzoate |
| 5-38' | 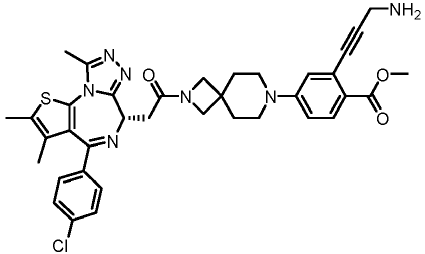 methyl (S)-2-(3-aminoprop-1-yn-1-yl)-4-(2-(2-(4-(4-chlorophenyl)-2,3,9-trimethyl-6H-thieno[3,2-f][1,2,4]triazolo[4,3-a][1,4]diazepin-6-yl)acetyl)-2,7-diazaspiro[3.5]nonan-7-yl)benzoate |
| 5-39' | 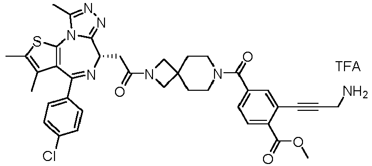 methyl (S)-2-(3-aminoprop-1-yn-1-yl)-4-(2-(2-(4-(4-chlorophenyl)-2,3,9-trimethyl-6H-thieno[3,2-f][1,2,4]triazolo[4,3-a][1,4]diazepin-6-yl)acetyl)-2,7-diazaspiro[3.5]nonane-7-carbonyl)benzoate trifluoroacetic acid |
| 5-40' | 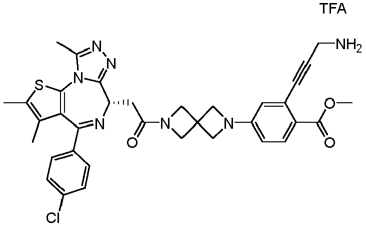 methyl (S)-2-(3-aminoprop-1-yn-1-yl)-4-(6-(2-(4-(4-chlorophenyl)-2,3,9-trimethyl-6H-thieno[3,2-f][1,2,4]triazolo[4,3-a][1,4]diazepin-6-yl)acetyl)-2,6-diazaspiro[3.3]heptan-2-yl)benzoate trifluoroacetic acid |
| 5-41' | 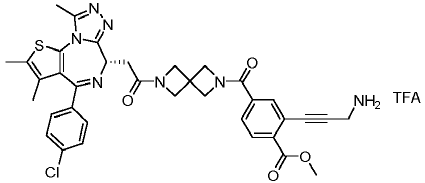 methyl (S)-2-(3-aminoprop-1-yn-1-yl)-4-(6-(2-(4-(4-chlorophenyl)-2,3,9-trimethyl-6H-thieno[3,2-f][1,2,4]triazolo[4,3-a][1,4]diazepin-6-yl)acetyl)-2,6-diazaspiro[3.3]heptane-2-carbonyl)benzoate trifluoroacetic acid |
| 5-42' | 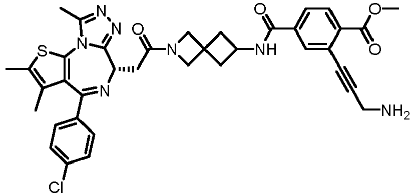 methyl (S)-2-(3-aminoprop-1-yn-1-yl)-4-((2-(2-(4-(4-chlorophenyl)-2,3,9-trimethyl-6H-thieno[3,2-f][1,2,4]triazolo[4,3-a][1,4]diazepin-6-yl)acetyl)-2-azaspiro[3.3]heptan-6-yl)carbamoyl)benzoate |
| 5-43' | 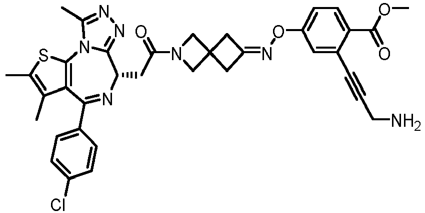 methyl (S)-2-(3-aminoprop-1-yn-1-yl)-4-(((2-(2-(4-(4-chlorophenyl)-2,3,9-trimethyl-6H-thieno[3,2-f][1,2,4]triazolo[4,3-a][1,4]diazepin-6-yl)acetyl)-2-azaspiro[3.3]heptan-6-ylidene)amino)oxy)benzoate |
| 5-44' | 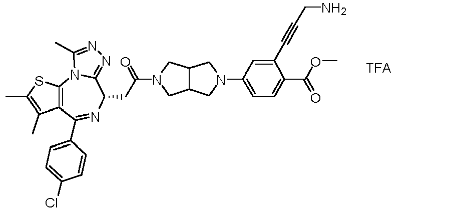 methyl 2-(3-aminoprop-1-yn-1-yl)-4-(5-(2-((5)-4-(4-chlorophenyl)-2, 3, 9-trimethyl-6H-thieno[3,2-f][1,2,4]triazolo[4,3-a][1,4]diazepin-6-yl)acetyl)hexahydropyrrolo[3,4-c]pyrrol-2(1H)-yl)benzoate trifluoroacetic acid |
| 5-45' | 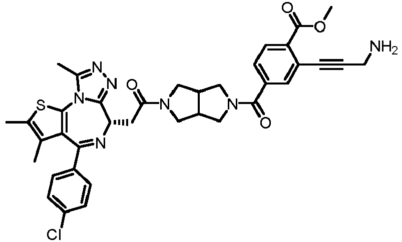 methyl 2-(3-aminoprop-1-yn-1-yl)-4-(5-(2-((S)-4-(4-chlorophenyl)-2,3,9-trimethyl-6H-thieno[3,2-f][1,2,4]triazolo[4,3-a][1,4]diazepin-6-yl)acetyl)octahydropyrrolo[3,4-c]pyrrole-2-carbonyl)benzoate |
| 5-46' | 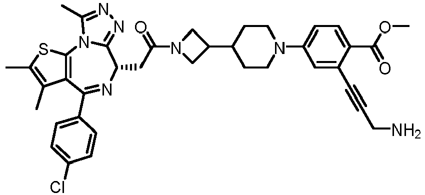 methyl (S)-2-(3-aminoprop-1-yn-1-yl)-4-(4-(1-(2-(4-(4-chlorophenyl)-2,3,9-trimethyl-6H-thieno[3,2-f][1,2,4]triazolo[4,3-a][1,4]diazepin-6-yl)acetyl)azetidin-3-yl)piperidin-1-yl)benzoate |
| 5-47' | 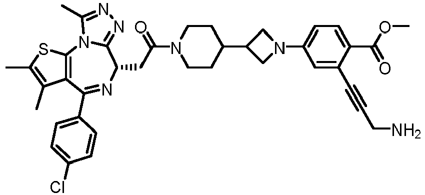 methyl (S)-2-(3-aminoprop-1-yn-1-yl)-4-(3-(1-(2-(4-(4-chlorophenyl)-2,3,9-trimethyl-6H-thieno[3,2-f][1,2,4]triazolo[4,3-a][1,4]diazepin-6-yl)acetyl)piperidin-4-yl)azetidin-1-yl)benzoate |
| 5-48' | 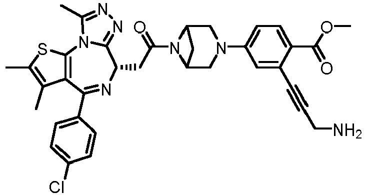 methyl 2-(3-aminoprop-1-yn-1-yl)-4-(6-(2-((S)-4-(4-chlorophenyl)-2,3,9-trimethyl-6H-thieno[3,2-f][1,2,4]triazolo[4,3-a][1,4]diazepin-6-yl)acetyl)-3,6-diazabicyclo[3.1.1]heptan-3-yl)benzoate |
| 5-85' | 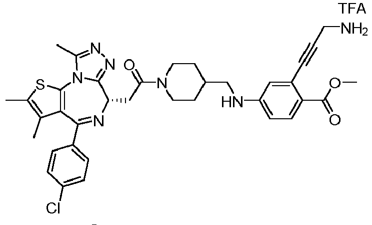 methyl (S)-2-(3-aminoprop-1-yn-1-yl)-4-(((1-(2-(4-(4-chlorophenyl)-2,3,9-trimethyl-6H-thieno[3,2-f][1,2,4]triazolo[4,3-a][1,4]diazepin-6-yl)acetyl)piperidin-4-yl)methyl)amino)benzoate trifluoroacetic acid |
| 5-86' | 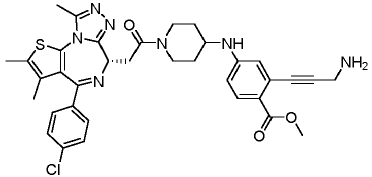 methyl (S)-2-(3-aminoprop-1-yn-1-yl)-4-((1-(2-(4-(4-chlorophenyl)-2,3,9-trimethyl-6H-thieno[3,2-f][1,2,4]triazolo[4,3-a][1,4]diazepin-6-yl)acetyl)piperidin-4-yl)amino)benzoate |
| 5-88' | 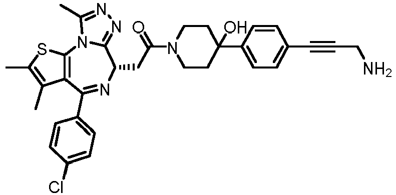 (S)-1-(4-(4-(3-aminoprop-1-yn-1-yl)phenyl)-4-hydroxypiperidin-1-yl)-2-(4-(4-chlorophenyl)-2,3,9-trimethyl-6H-thieno[3,2-f][1,2,4]triazolo[4,3-a][1,4]diazepin-6-yl)ethan-1-one |
| 5-112' | 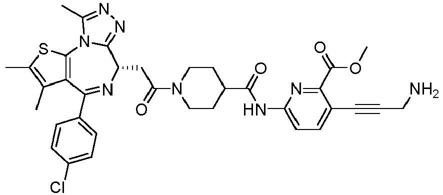 methyl (S)-3-(3-aminoprop-1-yn-1-yl)-6-(1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamido)picolinate |
| 5-118' | 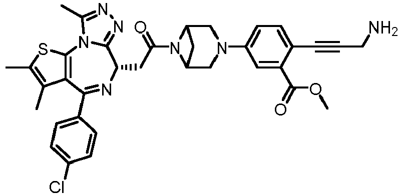 methyl 2-(3-aminoprop-1-yn-1-yl)-5-(6-(2-((S)-4-(4-chlorophenyl)-2,3,9-trimethyl-6H-thieno[3,2-f][1,2,4]triazolo[4,3-a][1,4]diazepin-6-yl)acetyl)-3,6-diazabicyclo[3.1.1]heptan-3-yl)benzoate |
| 5-119' | 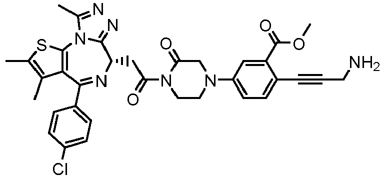 methyl (S)-2-(3-aminoprop-1-yn-1-yl)-5-(4-(2-(4-(4-chlorophenyl)-2,3,9-trimethyl-6H-thieno[3,2-f][1,2,4]triazolo[4,3-a][1,4]diazepin-6-yl)acetyl)-3-oxopiperazin-1-yl)benzoate |
| 5-120' | 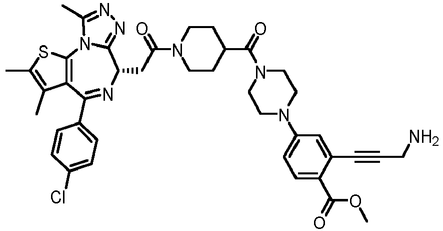 methyl (S)-2-(3-aminoprop-1-yn-1-yl)-4-(4-(1-(2-(4-(4-chlorophenyl)-2,3,9-trimethyl-6H-thieno[3,2-f][1,2,4]triazolo[4,3-a][1,4]diazepin-6-yl)acetyl)piperidine-4-carbonyl)piperazin-1-yl)benzoate |
| 5-124' | 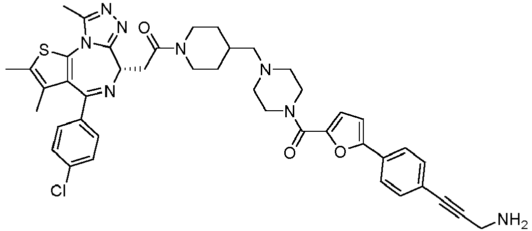 (S)-1-(4-((4-(5-(4-(3-aminoprop-1-yn-1-yl)phenyl)furan-2-carbonyl)piperazin-1-yl)methyl)piperidin-1-yl)-2-(4-(4-chlorophenyl)-2,3,9-trimethyl-6H-thieno[3,2-f][1,2,4]triazolo[4,3-a][1,4]diazepin-6-yl)ethan-1-one |
| 5-125' | 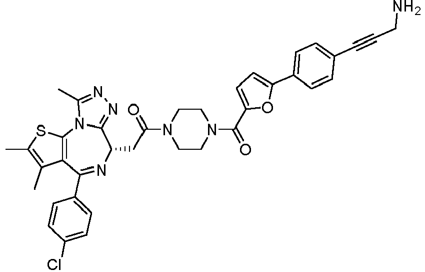 (S)-1-(4-(5-(4-(3-aminoprop-1-yn-1-yl)phenyl)furan-2-carbonyl)piperazin-1-yl)-2-(4-(4-chlorophenyl)-2,3,9-trimethyl-6H-thieno[3,2-f][1,2,4]triazolo[4,3-a][1,4]diazepin-6-yl)ethan-1-one |
| 5-129' | 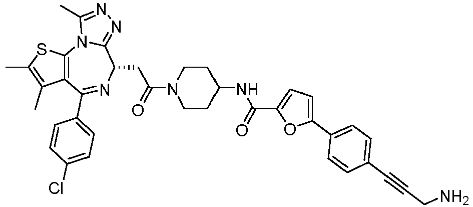 (S)-5-(4-(3-aminoprop-1-yn-1-yl)phenyl)-N-(1-(2-(4-(4-chlorophenyl)-2,3,9-trimethyl-6H-thieno[3,2-f][1,2,4]triazolo[4,3-a][1,4]diazepin-6-yl)acetyl)piperidin-4-yl)furan-2-carboxamide |
| 5-131' | 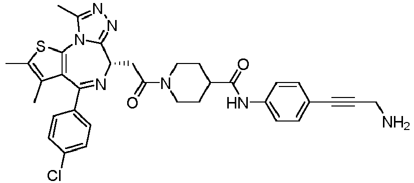 (S)-N-(4-(3-aminoprop-1-yn-1-yl)phenyl)-1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamide |
| 5-134' | 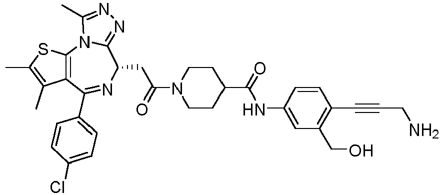 (S)-N-(4-(3-aminoprop-1-yn-1-yl)-3-(hydroxymethyl)phenyl)-1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamide |
| 5-139' | 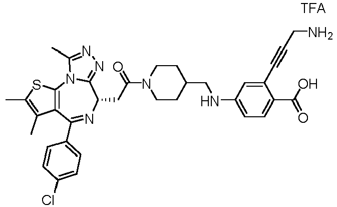 (S)-2-(3-aminoprop-1-yn-1-yl)-4-(((1-(2-(4-(4-chlorophenyl)-2,3,9-trimethyl-6H-thieno[3,2-f][1,2,4]triazolo[4,3-a][1,4]diazepin-6-yl)acetyl)piperidin-4-yl)methyl)amino)benzoic acid trifluoroacetic acid |
| 5-140' | 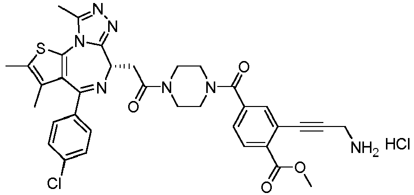 methyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetyl)piperazine-1-carbonyl)benzoate hydrochloride |
| 5-152' | 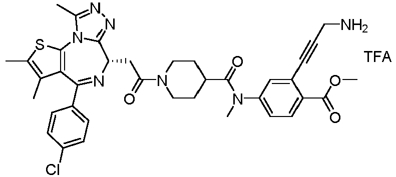 methyl (S)-2-(3-aminoprop-1-yn-1-yl)-4-(1-(2-(4-(4-chlorophenyl)-2,3,9-trimethyl-6H-thieno[3,2-f][1,2,4]triazolo[4,3-a][1,4]diazepin-6-yl)acetyl)-N-methylpiperidine-4-carboxamido)benzoate trifluoroacetic acid |
| 5-153' | 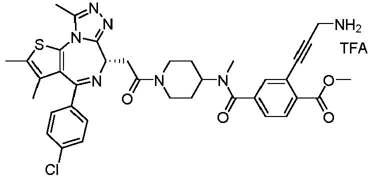 methyl (S)-2-(3-aminoprop-1-yn-1-yl)-4-((1-(2-(4-(4-chlorophenyl)-2,3,9-trimethyl-6H-thieno[3,2-f][1,2,4]triazolo[4,3-a][1,4]diazepin-6-yl)acetyl)piperidin-4-yl)(methyl)carbamoyl)benzoate trifluoroacetic acid |
| 5-154' | 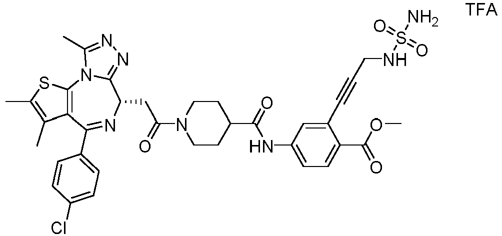 methyl (S)-4-(1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamido)-2-(3-(sulfamoylamino)prop-1-yn-1-yl)benzoate trifluoroacetic acid |
| 5-155' | 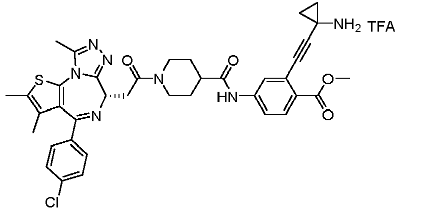 methyl (S)-2-((1-aminocyclopropyl)ethynyl)-4-(1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamido)benzoate |
| 5-156' | 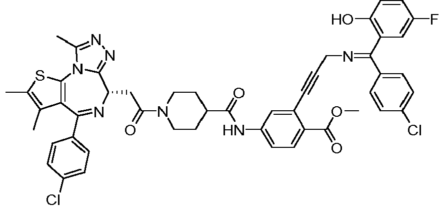 methyl (S)-2-(3-(((4-chlorophenyl)(5-fluoro-2-hydroxyphenyl)methylene)amino)prop-1-yn-1-yl)-4-(1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamido)benzoate |
| 5-157' | 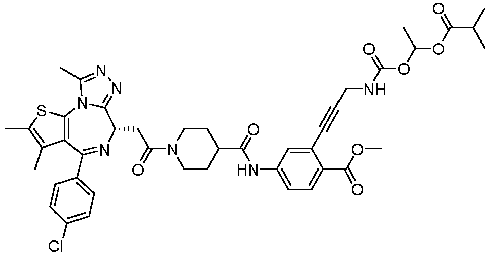 methyl 4-(1-(2-((S)-4-(4-chlorophenyl)-2,3,9-trimethyl-6H-thieno[3,2-f][1,2,4]triazolo[4,3-a][1,4]diazepin-6-yl)acetyl)piperidine-4-carboxamido)-2-(3-(((1-(isobutyryloxy)ethoxy)carbonyl)amino)prop-1-yn-1-yl)benzoate |
| 5-158' | 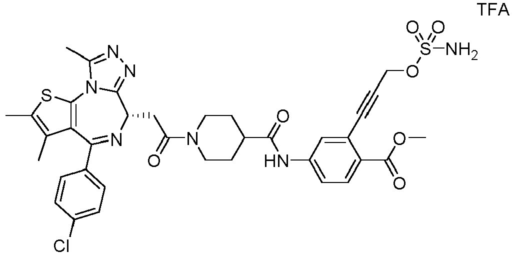 methyl (S)-4-(1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamido)-2-(3-(sulfamoyloxy)prop-1-yn-1-yl)benzoate trifluoroacetic acid |
| 5-159' | 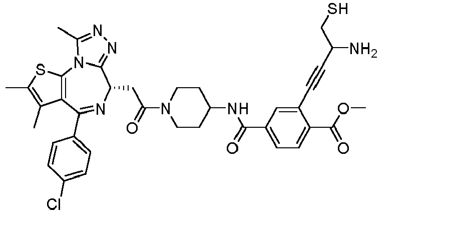 methyl 2-(3-amino-4-mercaptobut-1-yn-1-yl)-4-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)carbamoyl)benzoate |
| 5-160' | 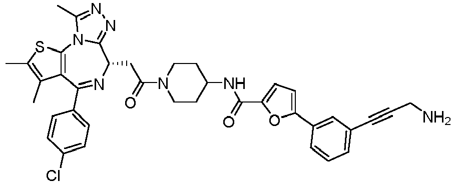 (S)-5-(3-(3-aminoprop-1-yn-1-yl)phenyl)-N-(1-(2-(4-(4-chlorophenyl)-2,3,9-trimethyl-6H-thieno[3,2-f][1,2,4]triazolo[4,3-a][1,4]diazepin-6-yl)acetyl)piperidin-4-yl)furan-2-carboxamide |
| 5-162' | 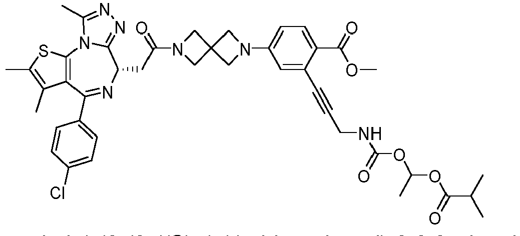 methyl 4-(6-(2-((S)-4-(4-chlorophenyl)-2,3,9-trimethyl-6H-thieno[3,2-f][1,2,4]triazolo[4,3-a][1,4]diazepin-6-yl)acetyl)-2,6-diazaspiro[3.3]heptan-2-yl)-2-(3-(((1-(isobutyryloxy)ethoxy)carbonyl)amino)prop-1-yn-1-yl)benzoate |
| 5-167' | 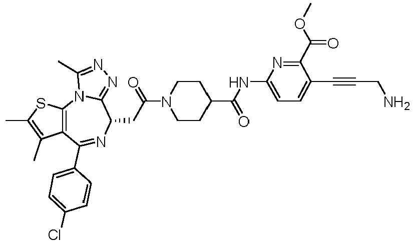 methyl (S)-3-(3-aminoprop-1-yn-1-yl)-6-(1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamido)picolinate |
| 5-168' | 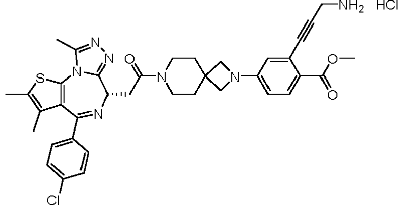 methyl (S)-2-(3-aminoprop-1-yn-1-yl)-4-(7-(2-(4-(4-chlorophenyl)-2,3,9-trimethyl-6H-thieno[3,2-f][1,2,4]triazolo[4,3-a][1,4]diazepin-6-yl)acetyl)-2,7-diazaspiro[3.5]nonan-2-yl)benzoate hydrochloride |

**[Table 12]**

| No. | Compound |
|---|---|
| 5-23" | 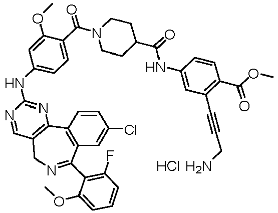 methyl 2-(3-aminoprop-1-yn-1-yl)-4-(1-(4-((9-chloro-7-(2-fluoro-6-methoxyphenyl)-5H-benzo[c]pyrimido[4,5-e]azepin-2-yl)amino)-2-methoxybenzoyl)piperidine-4-carboxamido)benzoate hydrochloride |
| 5-28" | 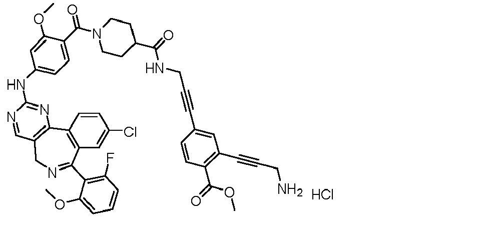 methyl 2-(3-aminoprop-1-yn-1-yl-4-(3-(1-(4-((9-chloro-7-(2-fluoro-6-methoxyphenyl)-5H-benzo[c]pyrimido[4,5-e]azepin-2-yl)amino)-2-methoxybenzoyl)piperidine-4-carboxamido)prop-1-yn-1-yl)benzoate trifluoroacetic acid |
| 5-29" | 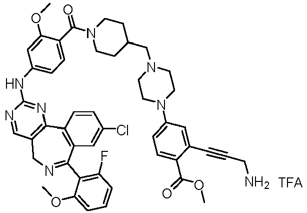 methyl 2-(3-aminoprop-1-yn-1-yl)-4-(4-((1-(4-((9-chloro-7-(2-fluoro-6-methoxyphenyl)-5H-benzo[c]pyrimido[4,5-e]azepin-2-yl)amino)-2-methoxybenzoyl)piperidin-4-yl)methyl)piperazin-1-yl)benzoate trifluoroacetic acid |
| 5-40" | 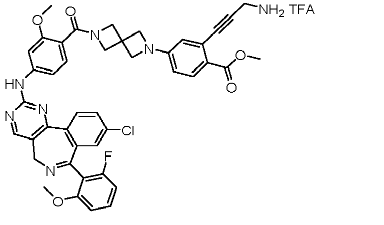 metnyl 2-(6-aminoprop-1-yn-1-yl)-4-(b-(4-((9-chloro-7-(2-fluoro-6-methoxyphenyl)-5H-benzo[c]pyrimido[4,5-e]azepin-2-yl)amino)-2-methoxybenzoyl)-2,6-diazaspiro[3.3]heptan-2-yl)benzoate trifluoroacetic acid |

**[Table 13]**

| No. | Compound |
|---|---|
| 5-39''' | 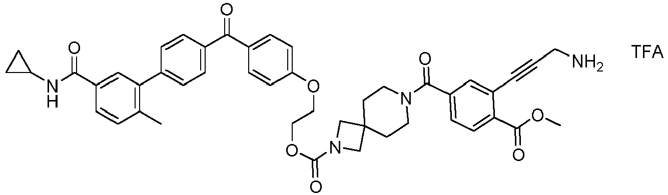 2-(4-(5'-(cyclopropylcarbamoyl)-2'-methyl-[1,1'-biphenyl]-4-carbonyl)phenoxy)ethyl 7-(3-(3-aminoprop-1-yn-1-yl)-4-(methoxycarbonyl)benzoyl)-2,7-diazaspiro[3.5]nonane-2-carboxylate trifluoroacetic acid |
| 5-140''' | 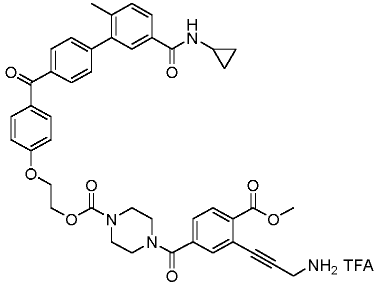 2-(4-(5'-(cyclopropylcarbamoyl)-2'-methyl-[1,1'-biphenyl]-4-carbonyl)phenoxy)ethyl 4-(3-(3-aminoprop-1-yn-1-yl)-4-(methoxycarbonyl)benzoyl)piperazine-1-carboxylate trifluoroacetic acid |

More specifically, in the compound represented by Formula VIII above, specific examples of the case where the compound of Formula 1 above is bound to the TBM may be presented in Table 14 below, and specific examples of the case where the compound of Formula 2 above is bound to the TBM may be presented in Table 15 below, but are not limited thereto.

**[Table 14]**

| No. | Compound |
|---|---|
| 4-207' | 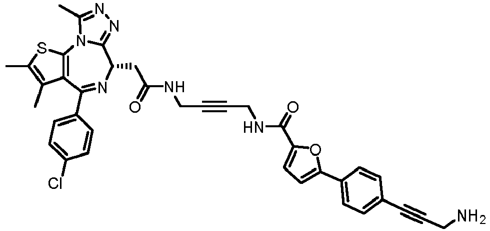 (S)-5-(4-(3-aminoprop-1-yn-1-yl)phenyl)-N-(4-(2-(4-(4-chlorophenyl)-2,3,9-trimethyl-6H-thieno[3,2-f][1,2,4]triazolo[4,3-a][1,4]diazepin-6-yl)acetamido)but-2-yn-1-yl)furan-2-carboxamide |
| 4-208' | 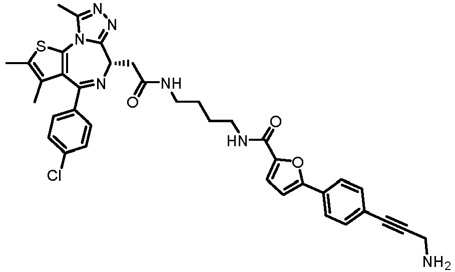 (S)-5-(4-(3-aminoprop-1-yn-1-yl)phenyl)-N-(4-(2-(4-(4-chlorophenyl)-2,3,9-trimethyl-6H-thieno[3,2-f][1,2,4]triazolo[4,3-a][1,4]diazepin-6-yl)acetamido)butyl)furan-2-carboxamide |
| 4-211' | 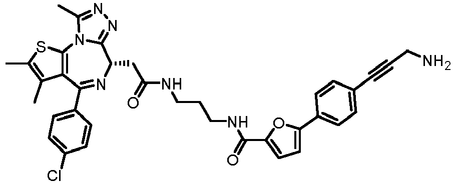 (S)-5-(4-(3-aminoprop-1-yn-1-yl)phenyl)-N-(3-(2-(4-(4-chlorophenyl)-2,3,9-trimethyl-6H-thieno[3,2-f][1,2,4]triazolo[4,3-a][1,4]diazepin-6-yl)acetamido)propyl)furan-2-carboxamide |
| 5-32' | 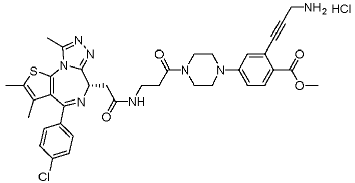 methyl (S)-2-(3-aminoprop-1-yn-1-yl)-4-(4-(3-(2-(4-(4-chlorophenyl)-2,3,9-trimethyl-6H-thieno[3,2-f][1,2,4]triazolo[4,3-a][1,4]diazepin-6-yl)acetamido)propanoyl)piperazin-1-yl)benzoate hydrochloride |
| 5-33' | 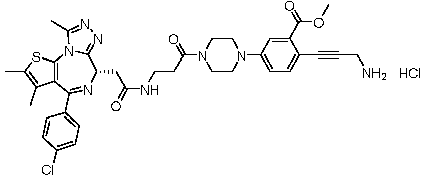 methyl (S)-2-(3-aminoprop-1-yn-1-yl)-5-(4-(3-(2-(4-(4-chlorophenyl)-2,3,9-trimethyl-6H-thieno[3,2-f][1,2,4]triazolo[4,3-a][1,4]diazepin-6-yl)acetamido)propanoyl)piperazin-1-yl)benzoate hydrochloride |
| 5-51' | 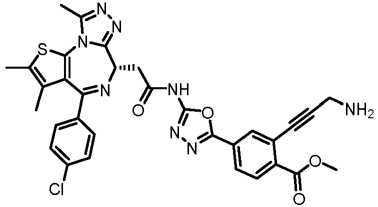 methyl (S)-2-(3-aminoprop-1-yn-1-yl)-4-(5-(2-(4-(4-chlorophenyl)-2,3,9-trimethyl-6H-thieno[3,2-f][1,2,4]triazolo[4,3-a][1,4]diazepin-6-yl)acetamido)-1,3,4-oxadiazol-2-yl)benzoate |
| 5-111' | 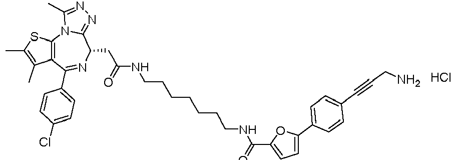 (S)-5-(4-(3-aminoprop-1-yn-1-yl)phenyl)-N-(7-(2-(4-(4-chlorophenyl)-2,3,9-trimethyl-6H-thieno[3,2-f][1,2,4]triazolo[4,3-a][1,4]diazepin-6-yl)acetamido)heptyl)furan-2-carboxamide hydrochloride |
| 5-114' | 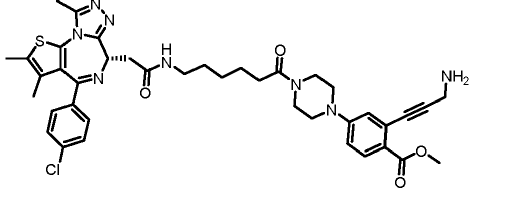 methyl (S)-2-(3-aminoprop-1-yn-1-yl)-4-(4-(6-(2-(4-(4-chlorophenyl)-2,3,9-trimethyl-6H-thieno[3,2-f][1,2,4]triazolo[4,3-a][1,4]diazepin-6-yl)acetamido)hexanoyl)piperazin-1-yl)benzoate |
| 5-117' | 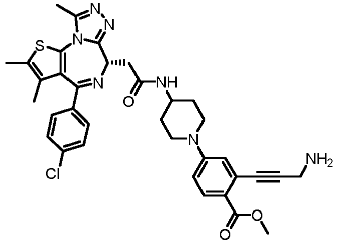 methyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)piperidin-1-yl)benzoate |
| 5-128' | 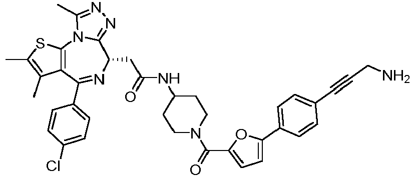 (S)-N-(1-(5-(4-(3-aminoprop-1-yn-1-yl)phenyl)furan-2-carbonyl)piperidin-4-yl)-2-(4-(4-chlorophenyl)-2,3,9-trimethyl-6H-thieno[3,2-f][1,2,4]triazolo[4,3-a][1,4]diazepin-6-yl)acetamide |
| 5-130' | 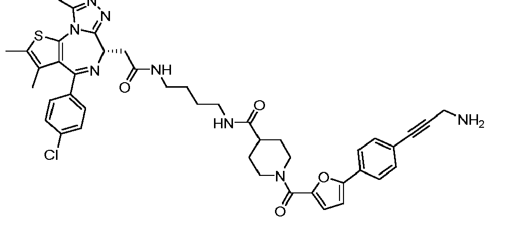 (S)-1-(5-(4-(3-aminoprop-1-yn-1-yl)pnenyl)turan-2-carbonyl)-N-(4-(2-(4-(4-chlorophenyl)-2,3,9-trimethyl-6H-thieno[3,2-f][1,2,4]triazolo[4,3-a][1,4]diazepin-6-yl)acetamido)butyl)piperidine-4-carboxamide |
| 5-132' | 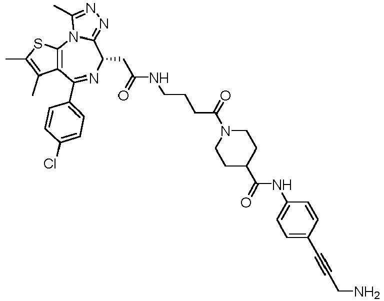 (S)-N-(4-(3-aminoprop-1-yn-1-yl)phenyl)-1-(4-(2-(4-(4-chlorophenyl)-2,3,9-trimethyl-6H-thieno[3,2-f][1,2,4]triazolo[4,3-a][1,4]diazepin-6-yl)acetamido)butanoyl)piperidine-4-carboxamide |
| 5-133' | 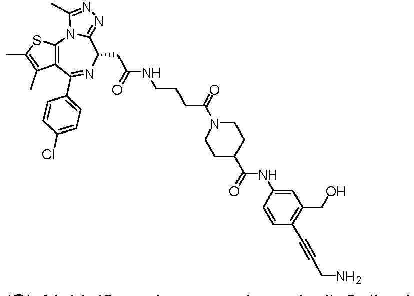 (S)-N-(4-(3-aminoprop-1-yn-1-yl)-3-(hydroxymethyl)phenyl)-1-(4-(2-(4-(4-chlorophenyl)-2,3,9-trimethyl-6H-thieno[3,2-f][1,2,4]triazolo[4,3-a][1,4]diazepin-6-yl)acetamido)butanoyl)piperidine-4-carboxamide |
| 5-136' | 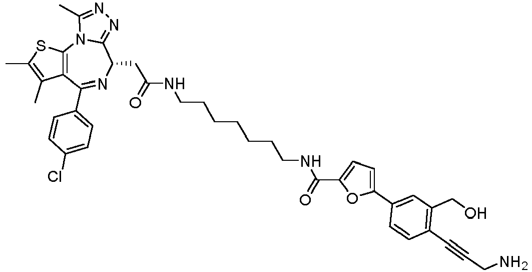 (S)-5-(4-(3-aminoprop-1-yn-1-yl)-3-(hydroxymethyl)phenyl)-N-(7-(2-(4-(4-chlorophenyl)-2,3,9-trimethyl-6H-thieno[3,2-f][1,2,4]triazolo[4,3-a][1,4]diazepin-6-yl)acetamido)heptyl)furan-2-carboxamide |
| 5-137' | 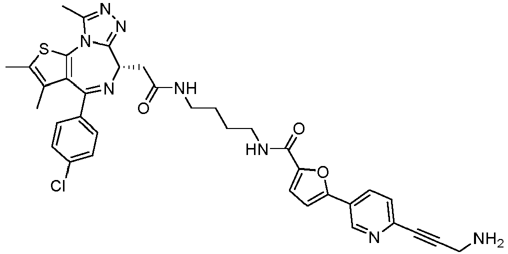 (S)-5-(6-(3-aminoprop-1-yn-1-yl)pyridin-3-yl)-N-(4-(2-(4-(4-chlorophenyl)-2,3,9-trimethyl-6H-thieno[3,2-f][1,2,4]triazolo[4,3-a][1,4]diazepin-6-yl)acetamido)butyl)furan-2-carboxamide |
| 5-138' | 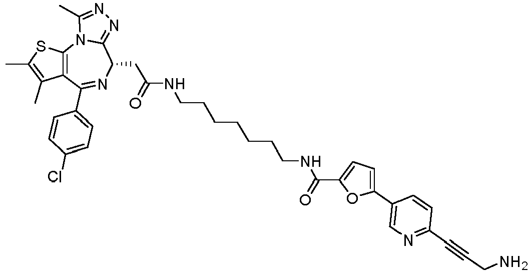 (S)-5-(6-(3-aminoprop-1-yn-1-yl)pyridin-3-yl)-N-(7-(2-(4-(4-chlorophenyl)-2,3,9-trimethyl-6H-thieno[3,2-f][1,2,4]triazolo[4,3-a][1,4]diazepin-6-yl)acetamido)heptyl)furan-2-carboxamide |
| 5-141' | 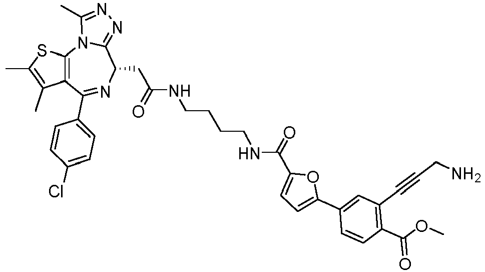 methyl (S)-2-(3-aminoprop-1-yn-1-yl)-4-(5-((4-(2-(4-(4-chlorophenyl)-2,3,9-trimethyl-6H-thieno[3,2-f][1,2,4]triazolo[4,3-a][1,4]diazepin-6-yl)acetamido)butyl)carbamoyl)furan-2-yl)benzoate |
| 5-142' | 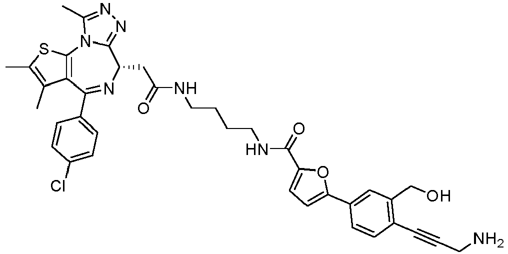 (S)-5-(4-(3-aminoprop-1-yn-1-yl)-3-(hydroxymethyl)phenyl)-N-(4-(2-(4-(4-chlorophenyl)-2,3,9-trimethyl-6H-thieno[3,2-f][1,2,4]triazolo[4,3-a][1,4]diazepin-6-yl)acetamido)butyl)furan-2-carboxamide |
| 5-143' | 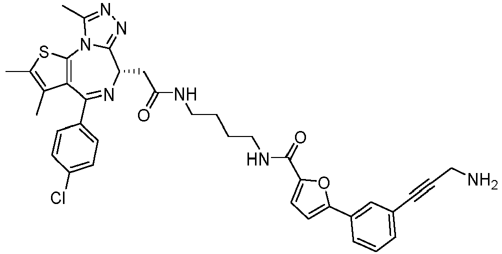 (S)-5-(3-(3-aminoprop-1-yn-1-yl)phenyl)-N-(4-(2-(4-(4-chlorophenyl)-2,3,9-trimethyl-6H-thieno[3,2-f][1,2,4]triazolo[4,3-a][1,4]diazepin-6-yl)acetamido)butyl)furan-2-carboxamide |
| 5-144' | 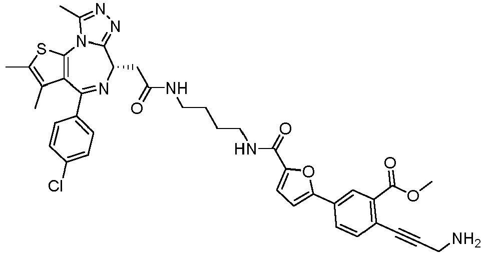 methyl (S)-2-(3-aminoprop-1-yn-1-yl)-5-(5-((4-(2-(4-(4-chlorophenyl)-2,3,9-trimethyl-6H-thieno[3,2-f][1,2,4]triazolo[4,3-a][1,4]diazepin-6-yl)acetamido)butyl)carbamoyl)furan-2-yl)benzoate |
| 5-145' | 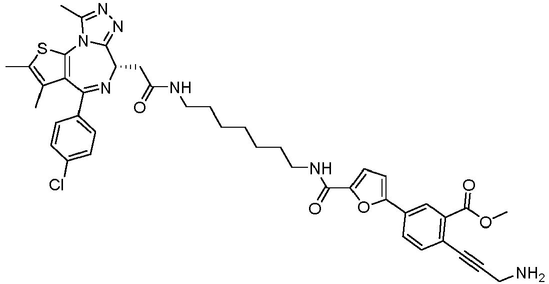 methyl (S)-2-(3-aminoprop-1-yn-1-yl)-5-(5-((7-(2-(4-(4-chlorophenyl)-2,3,9-trimethyl-6H-thieno[3,2-f][1,2,4]triazolo[4,3-a][1,4]diazepin-6-yl)acetamido)heptyl)carbamoyl)furan-2-yl)benzoate |
| 5-164' | methyl (S)-2-(3-aminoprop-1-yn-1-yl)-4-(4-(3-(2-(4-(4-chlorophenyl)-2,3,9-trimethyl-6H-thieno[3,2-f][1,2,4]triazolo[4,3-a][1,4]diazepin-6-yl)acetamido)propyl)piperazin-1-yl)benzoate hydrochloride |
| 5-165' | 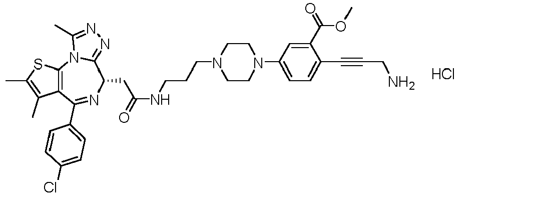 methyl (S)-2-(3-aminoprop-1-yn-1-yl)-5-(4-(3-(2-(4-(4-chlorophenyl)-2,3,9-trimethyl-6H-thieno[3,2-f][1,2,4]triazolo[4,3-a][1,4]diazepin-6-yl)acetamido)propyl)piperazin-1-yl)benzoate hydrochloride |

**[Table 15]**

| No. | Compound |
|---|---|
| 5-32" | 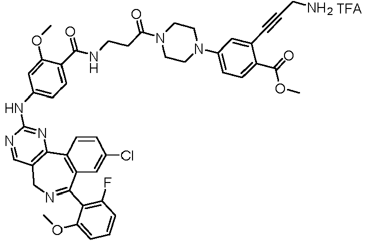 methyl 2-(3-aminoprop-1-yn-1-yl)-4-(4-(3-(4-((9-chloro-7-(2-fluoro-6-methoxyphenyl)-5H-benzo[c]pyrimido[4,5-e]azepin-2-yl)amino)-2-methoxybenzamido)propanoyl)piperazin-1-yl)benzoate trifluoroacetic acid |

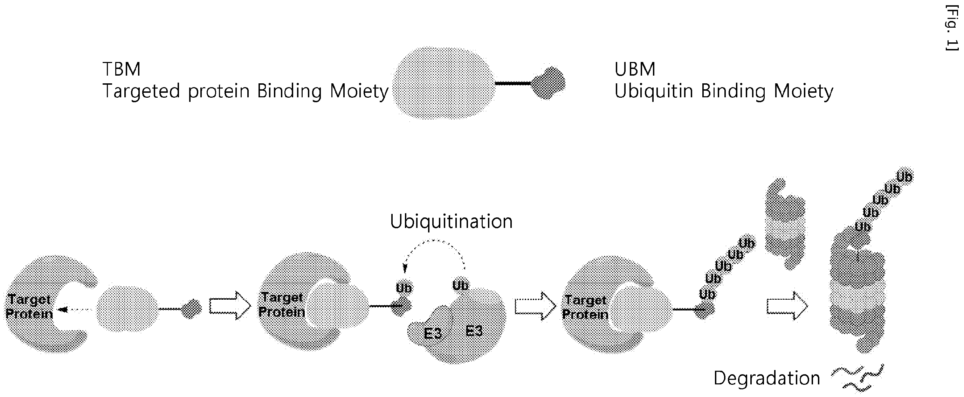

As described above, the compound of the present invention having the chemical structure of TBM-UBM may degrade the target protein or polypeptide. Specifically, as illustrated in FIG. 1, when the TBM of the compound of the present invention binds to the target protein or polypeptide and then ubiquitin binds to the UBM of the compound of the present invention (when (poly)ubiquitinated), the target protein or polypeptide may be degraded by proteasome or lysosome.

### 3. Pharmaceutical Composition

According to another aspect of the present invention, there is provided a pharmaceutical composition containing, as an active ingredient, a compound represented by Formulae I to VIII above, or a pharmaceutically acceptable salt, isomer, solvate, hydrate, or prodrug thereof.

As described above, since the compound represented by Formulae I to IV or the compound represented by Formulae V to VIII has an activity as a degrader of the target protein or polypeptide, the pharmaceutical composition of the present invention may be used to prevent or treat a disease caused by the target protein or polypeptide.

In particular, the pharmaceutical composition of the present invention may contain, as an active ingredient, the compound represented by Formulae V to VIII, or a pharmaceutically acceptable salt, isomer, solvate, hydrate, or prodrug thereof, wherein the specific use of the pharmaceutical composition may vary with the type of target protein or polypeptide to which TBM binds.

For example, when the target protein or polypeptide to which the TBM binds is a bromodomain-containing protein such as BRD2, BRD3, BRD4, or BRDT, or when the TBM is a compound targeting a bromodomain-containing protein such as a BRD4 inhibitor, the pharmaceutical composition may be used to prevent or treat bromodomain-containing protein-related diseases.

The bromodomain-containing protein-related diseases may be, for example, diseases or conditions in which the bromodomain-containing protein, such as BRD2, BRD3, BRD4, or BRDT, is overexpressed or overactivated to develop, progress, or worsen, and specifically, may be acute or chronic autoimmune and inflammatory diseases.

In another embodiment of the present invention, the bromodomain-containing protein-related diseases or conditions include diseases caused by congenital or acquired hyperinflammatory response and persistent inflammation, for example, acquired hemophilia A, alopecia areata, ankylosing spondylitis, Anti-NMDA receptor encephalitis, catastrophic antiphospholipid syndrome (CAPS), Addison's disease, autoimmune autonomic ganglionopathy (AAG), autoimmune gastrointestinal dysmotility (AGID), autoimmune encephalitis, acute disseminated encephalomyelitis (ADEM), autoimmune gastritis, autoimmune hemolytic anemia (AIHA), autoimmune hepatitis (AIH), autoimmune hyperlipidemia, lymphocytic hypophysitis, autoimmune inner ear disease (AIED), autoimmune lymphoproliferative syndrome (ALPS), autoimmune myelofibrosis, autoimmune myocarditis, autoimmune oophoritis, autoimmune pancreatitis (AIP), 1, 2, 3-type autoimmune (autoimmune disease in an existing glossary) polyglandular syndromes (APS type 1, APS type 2, APS type 3, APECED), autoimmune progesterone dermatitis, autoimmune retinopathy (AIR), autoimmune sensorineural hearing loss (SNHL), Balo disease, Behcet's disease, birdshot chorioretinopathy (BSCR), bullous pemphigoid, Castleman's disease, celiac disease, Chagas disease, chronic inflammatory demyelinating polyneuropathy (CIDP), chronic urticaria (CU), eosinophilic granulomatosis with polyangiitis (EGPA), Cogan's syndrome, cold agglutinin disease, CREST syndrome, Crohn's disease (CD), Cronkhite canada syndrome (CSS), Cryptogenic organizing pneumonia (COP), dermatomyositis, neuromyelitis optica (NMO), type 1 diabetes, Dressler syndrome, atopic dermatitis, endometriosis, eosinophilic esophagitis, eosinophilic fasciitis, erythema nodosum, primary mixed cryoglobulinemia, Evans syndrome, fibrinous pneumonia, giant cell arteritis, glomerulonephritis, Goodpasture syndrome, granulomatosis with polyangiitis (GPA), Grave's disease, Guillain-Barre syndrome (GBS), chronic lymphocytic thyroiditis, Henoch-Schonlein purpura, hidradenitis suppurativa, hypogammaglobulinemia, immunoglobulin A nephropathy, immune-mediated necrotizing myopathy (IMNM), immune thrombocytopenia (ITP), inclusion body myositis, igG4-associated multiple sclerosis (ISD), interstitial cystitis, juvenile idiopathic arthritis, juvenile polymyositis, juvenile dermatomyositis, Kawasaki disease, Lambert-Eaton myasthenic syndrome (LEMS), leukocytoclastic vasculitis, lichen planus, lichen sclerosus, conjunctivitis, linear IgA bullous dermatosis (LAD), linear IgA bullous dermatosis (LABD), lupus nephritis, lymphocytic colitis, Meniere disease, microscopic polyangiitis (MPA), mixed connective tissue disease (MCTD), Mooren's ulcer, acute pityriasis lichenoides, multifocal motor neuropathy, multiple sclerosis (MS), myalgic encephalomyelitis (ME), myasthenia gravis (MG), ocular cicatricial pemphigoid, opsoclonus-myoclonus syndrome (OMS), palindromic rheumatism, paraneoplastic cerebellar degeneration, paraneoplastic pemphigus, progressive hemifacial atrophy (HFA), paroxysmal nocturnal hemoglobinuria, periphlebitis, Parsonage-Turner syndrome, pemphigoid gestationis, pemphigus foliaceus, pemphigus vulgaris, pernicious anemia, POEMS syndrome, polyarteritis nodosa (PAN), polymyalgia rheumatica, polymyositis, postural orthostatic tachycardia syndrome (POT), primary biliary cirrhosis (PBC), psoriasis, palmoplantar pustulosis, psoriatic arthritis, idiopathic pulmonary fibrosis (IPF), pure red cell aplasia (PRCA), gangrenous dermatitis, Rayno's disease, reactive arthritis, complex regional pain syndrome, relapsing polychondritis, restless leg syndrome (RLS), rheumatic fever, rheumatoid arthritis, sarcoidosis, autoimmune polyendocrine syndrome type II, sclerotitis, dermatosclerosis, serpiginous choroiditis, Sjogren's syndrome, stiff-person syndrome (SPS), small fiber neuropathy, systemic lupus erythematosus (SLE), Susac's syndrome, Sydenham's chorea, sympathetic nerve fiber, Takayasu arteritis, testitis, Tolosa-Hunt syndrome, transverse myelitis (TM), tublointerstitial nephritis and uveitis syndrome (TINU), ulcerative colitis (UC), undifferentiated connective tissue disease, uveitis, vasculitis, leukoplakia, Vogt-Koyanagi-Harada diseases or conditions, but are not limited thereto, and arteriosclerosis caused by the diseases is included, and inflammatory diseases developed in a subject may be encompassed.

In another embodiment of the present invention, the bromodomain-containing protein-related disease or condition may be a cell proliferative disorder. The cell proliferative disorder includes, but is not limited to, cancerous hyperproliferative disorders (e.g., brain, lung, squamous cell, bladder, gastric, pancreatic, breast, head, neck, renal, liver, kidney, ovarian, prostate, colorectal, colon, epidermoid, esophageal, testicular, gynecological or thyroid cancer, acute myeloid leukemia, multiple myeloma, mesothelioma, Non-small cell lung carcinoma (NSCLC), small cell lung cancer (SCLC), neuroblastoma, and acute lymphoblastic leukemia (ALL)); non-cancerous hyperproliferative disorders (e.g., benign hyperplasia of the skin (e.g., psoriasis), restenosis, and benign prostatic hypertrophy (BPH)); and diseases related to vasculogenesis or angiogenesis (e.g., tumor angiogenesis, hemangioma, glioma, melanoma, Kaposi's sarcoma and ovarian, breast, lung, pancreatic, prostate, colon and epidermoid cancer). The cell proliferative disorder further includes primary and metastatic cancers. In particular, the compounds are useful in the treatment of cancers in a subject, including pseudomyxoma, intrahepatic cholangiocarcinoma, hepatoblastoma, liver cancer, colon cancer, testicular cancer, myelodysplastic syndrome, glioblastoma, oral cancer, lip cancer, oropharynx-tonsillar cancer (HPV-associated), oropharynx-hypopharyngeal cancer (non-HPV-associated), mycotic cell carcinoma, basal cell carcinoma, epithelial ovarian cancer, ovarian germ cell tumor, male breast cancer, brain tumor, pituitary adenoma, choledochocholecystic tumors, gallbladder cancer, biliary tract cancer, colorectal cancer, chronic myeloid leukemia, chronic lymphocytic leukemia, retinoblastoma, choroidal melanoma, diffuse large B cell lymphoma, ampullar of vater cancer, bladder cancer, peritoneal cancer, parathyroid cancer, adrenal cancer, nasal and paranasal cavity cancer, non-hodgkin's lymphoma, tongue cancer, astrocytoma, juvenile brain tumor, juvenile lymphoma, juvenile leukemia, small intestine cancer, meningioma, esophageal cancer, glioma, renal pelvis cancer, renal cancer, heart cancer, duodenal cancer, malignant soft tissue cancer, malignant bone cancer, malignant lymphoma, malignant mesothelioma, malignant melanoma, eye cancer, vulvar cancer, ureteral cancer, urethral cancer, cancer of unknown primary site, gastric lymphoma, gastric cancer, gastric carcinoid, gastrointestinal stromal tumor, Wilms' tumor, breast cancer, sarcoma, penile cancer, gestational choriocarcinoma, cervical cancer, endometrial cancer, uterine sarcoma, prostate cancer, metastatic bone tumor, metastatic brain tumor, mediastinal cancer, rectal cancer, neuroendocrine tumor, rectal carcinoid, vaginal cancer, spinal tumor, vestibular schwannoma, pancreatic cancer, salivary gland cancer, Paget's disease, squamous cell cancer, adenocarcinoma of lung, lung cancer, squamous cell lung cancer, skin cancer, anal cancer, rhabdomyosarcoma, laryngeal cancer, and pleura cancer, but are not limited thereto, and including a disease caused by metastasis of these diseases, including, but not limited to, a variant including a mutation caused by relapse.

For example, when the target protein or polypeptide to which the TBM binds is Aurora kinase A, or the TBM is a compound targeting Aurora kinase A, such as an Aurora A inhibitor, the pharmaceutical composition may be used to prevent or treat cell proliferative disorders. The cell proliferative disorders are the same as described above.

For example, when the target protein or polypeptide to which the TBM binds is the p38 MAPK, or the TBM is a compound targeting the p38 MAPK, such as a p38 inhibitor, the pharmaceutical composition may be used to prevent or treat inflammation and inflammation-related diseases.

The inflammation or inflammation-related diseases include diseases caused by congenital or acquired hyperinflammatory response and persistent inflammation, for example, acquired hemophilia A, alopecia areata, ankylosing spondylitis, Anti-NMDA receptor encephalitis, antiphospholipid antibody syndrome (CAPS), Addison's disease, autoimmune autonomic neuropathy (AAG), autoimmune gastrointestinal dysmotility (AGID), autoimmune encephalitis, acute disseminated encephalomyelitis (ADEM), autoimmune gastritis, autoimmune hemolytic anemia (AIHA), autoimmune hepatitis (AIH), autoimmune hyperlipidemia, lymphocytic hypophysitis, autoimmune inner ear disease (AIED), autoimmune lymphoproliferative syndrome (ALPS), autoimmune myelofibrosis, autoimmune myocarditis, autoimmune oophoritis, autoimmune pancreatitis (AIP), 1, 2, 3-type autoimmune (autoimmune disease in an existing glossary) polyglandular syndromes (APS type 1, APS type 2, APS type 3, APECED), autoimmune progesterone dermatitis, autoimmune retinopathy (AIR), autoimmune sensorineural hearing loss (SNHL), Balo disease, Behcet's disease, birdshot chorioretinopathy (BSCR), bullous pemphigoid, Castleman's disease, celiac disease, Chagas disease, chronic inflammatory demyelinating polyneuropathy (CIDP), chronic urticaria (CU), eosinophilic granulomatosis with polyangiitis (EGPA), Cogan's syndrome, cold agglutinin disease, CREST syndrome, Crohn's disease (CD), Cronkhite canada syndrome (CSS), Cryptogenic organizing pneumonia (COP), dermatomyositis, neuromyelitis optica (NMO), type 1 diabetes, Dressler syndrome, atopic dermatitis, endometriosis, eosinophilic esophagitis, eosinophilic fasciitis, erythema nodosum, primary mixed cryoglobulinemia, Evans syndrome, fibrinous pneumonia, giant cell arteritis, glomerulonephritis, Goodpasture syndrome, granulomatosis with polyangiitis (GPA), Grave's disease, Guillain-Barre syndrome (GBS), chronic lymphocytic thyroiditis, Henoch-Schonlein purpura, hidradenitis suppurativa, hypogammaglobulinemia, immunoglobulin A nephropathy, immune-mediated necrotizing myopathy (IMNM), immune thrombocytopenia (ITP), inclusion body myositis, igG4-associated multiple sclerosis (ISD), interstitial cystitis, juvenile idiopathic arthritis, juvenile polymyositis, juvenile dermatomyositis, Kawasaki disease, Lambert-Eaton myasthenic syndrome (LEMS), leukocytoclastic vasculitis, lichen planus, lichen sclerosus, conjunctivitis, linear IgA bullous dermatosis (LAD), linear IgA bullous dermatosis (LABD), lupus nephritis, lymphocytic colitis, Meniere disease, microscopic polyangiitis (MPA), mixed connective tissue disease (MCTD), Mooren's ulcer, acute pityriasis lichenoides, multifocal motor neuropathy, multiple sclerosis (MS), myalgic encephalomyelitis (ME), myasthenia gravis (MG), ocular cicatricial pemphigoid, opsoclonus-myoclonus syndrome (OMS), palindromic rheumatism, paraneoplastic cerebellar degeneration, paraneoplastic pemphigus, progressive hemifacial atrophy (HFA), paroxysmal nocturnal hemoglobinuria, periphlebitis, Parsonage-Turner syndrome, pemphigoid gestationis, pemphigus foliaceus, pemphigus vulgaris, pernicious anemia, POEMS syndrome, polyarteritis nodosa (PAN), polymyalgia rheumatica, polymyositis, postural orthostatic tachycardia syndrome (POT), primary biliary cirrhosis (PBC), psoriasis, palmoplantar pustulosis, psoriatic arthritis, idiopathic pulmonary fibrosis (IPF), pure red cell aplasia (PRCA), gangrenous dermatitis, Rayno's disease, reactive arthritis, complex regional pain syndrome (RSD), relapsing polychondritis, restless leg syndrome (RLS), rheumatic fever, rheumatoid arthritis, sarcoidosis, autoimmune polyendocrine syndrome type II, sclerotitis, dermatosclerosis, serpiginous choroiditis, Sjogren's syndrome, stiff-person syndrome (SPS), small fiber neuropathy, systemic lupus erythematosus (SLE), Susac's syndrome, Sydenham's chorea, sympathetic nerve fiber, Takayasu arteritis, testitis, Tolosa-Hunt syndrome, transverse myelitis (TM), tublointerstitial nephritis and uveitis syndrome (TINU), ulcerative colitis (UC), undifferentiated connective tissue disease, uveitis, vasculitis, leukoplakia, Vogt-Koyanagi-Harada diseases or conditions, but are not limited thereto, and arteriosclerosis caused by the diseases is included. The mechanism involved in the inflammatory response of P38 can be not only applied to diseases caused by the above-mentioned congenital or acquired immunity, but also applied to acute inflammation caused by infection, including Ebola virus disease, marburg fever, Lassa fever, Crimean-Congo hemorrhagic fever, South American hemorrhagic fever, Rift valley fever, emerging infectious disease syndrome including COIVD19, severe acute respiratory syndrome (SARS), Middle East respiratory syndrome (MERS), animal influenza human infection, new influenza, chicken pox, measles, typhoid, paratyphoid, enterohemorrhagic escherichia coli infections, hepatitis A, pertussis, mumps, rubella, meningococcal infections, Haemophilus influenzae type b, pneumococcosis, scarlet fever, vancomycin resistant staphylococcus aureus (VRSA) infections, Carbapenemresistant Enterobacteriaceae (CRE) infections, hepatitis E, tetanus, Japanese encephalitis, legionellosis, vibrio vulnificus sepsis, exanthematous typhus, typhus fever, tsutsugamushi, leptospirosis, brucellosis, rabies, Nephropathy, Hemorragic fever related renal syndrome, yellow fever, dengue fever, Q fever, West Nile fever, Lyme disease, Tick-borne encephalitis, chikungunya fever, severe fever with thrombocytopenia syndrome (SFTS), vancomycinresistant enterococci (VRE) infections, Methicillin-resistant Staphylococcus aureus (MRSA) infections, Multidrug-resistant Pseudomonas aeruginosa (MRPA) infections, Multidrug-resistant Acinetobacterbaumannii (MRAB) infections, intestinal infections, acute respiratory infections, enterovirus infections, human papillomavirus infections, but is not limited thereto, and can be comprehensively applied to infectious, congenital, or acquired inflammatory diseases developed in a subject.

The pharmaceutical composition of the present invention may further include a pharmaceutically acceptable carrier or additive.

The active ingredient of the present invention may be administered alone or in combination with any convenient carrier, and the like, and the dosage form may be a singledose unit or multiple-dose unit. The pharmaceutical composition may be a solid formulation or a liquid formulation. The solid formulation includes, but is not limited to, a powder, a granule, a tablet, a capsule, a suppository, and the like. The solid formulation may include, but is not limited to, a carrier, a flavor, a binder, a preservative, a disintegrant, a lubricant, a filler, and the like. The liquid formulation includes water, a solution such as a propylene glycol solution, a suspension, an emulsion, and the like, but is not limited thereto, and may be prepared by adding suitable colorants, flavors, stabilizers, thickeners, and the like. For example, a powder may be prepared by simply mixing the active ingredient of the present invention with a suitable pharmaceutically acceptable carrier such as lactose, starch, or microcrystalline cellulose. A granule may be prepared by mixing the active ingredient of the present invention, a suitable pharmaceutically acceptable carrier, and a suitable pharmaceutically acceptable binder such as polyvinylpyrrolidone or hydroxypropyl cellulose, and then utilizing wet granulation using a solvent such as water, ethanol, or isopropanol, or dry granulation using a compressive force. Also, a tablet may be prepared by mixing the granule with a suitable pharmaceutically acceptable lubricant such as magnesium stearate, and then tableting the mixture using a tableting machine.

The pharmaceutical composition of the present invention may be administered in the form of oral formulation, injectable formulation (for example, intramuscular, intraperitoneal, intravenous, infusion, subcutaneous, implant), inhalable, intranasal, vaginal, rectal, sublingual, transdermal, topical, *etc.* depending on the disorders to be treated and the individual's conditions, but is not limited thereto. The composition of the present invention may be formulated in a suitable dosage unit formulation including a pharmaceutically acceptable and non-toxic carrier, additive and vehicle, which are generally used in the art, depending on the routes to be administered.

The pharmaceutical composition of the present invention may be administered at a daily dose of about 0.0001 mg/kg to about 10 g/kg, for example, about 0.001 mg/kg to about 1 g/kg. However, the dosage may vary with the degree of purification of the mixture, the condition of a patient (age, sex, body weight, *etc.*), the severity of the condition being treated, and the like. For convenience, a total daily dose of the pharmaceutical composition may be administered in multiple doses a day as needed.

Hereinafter, the present invention will be described in detail with reference to examples.

However, the following examples specifically illustrate the present invention, and the contents of the present invention are not limited by the following examples.

### [Preparation Examples]

First, the compounds represented by Formulae I to IV above of the present invention were designed and synthesized as follows. Some specimen used in the synthesis process are abbreviated as follows: ACN: acetonitrile, DCM: dichloromethane, HCl: hydrochloride, EtOAc: ethyl acetate, MeOH: methanol, EtOH: ethanol, DCE: dichloroethane, DMSO: dimethyl sulfoxide, DMF: N, N-dimethylformamide, THF; tetrahydrofuran, TBSCI; *tert*-butyldimethylsilyl chloride, TBAI; tetrabutylammonium iodide, DEA; diethylamine, TEA; triethylamine, DIPEA: N, N-diisopropylethylamine, NIS: N-iodosuccinimide, PTSA: p-toluenesulfonic acid, TFA: trifluoroacetic acid, BINAP: (2,2'-bis(diphenylphosphino)-1,1'-binaphthyl), NBS: N-bromosuccinimide, EDC: 1-ethyl-3-(3-dimethylaminopropyl)carbodiimide, HATU: 1-[bis(dimethylamino)methylene]-1H-1,2,3-triazolo[4,5-b]pyridinium 3-oxid hexafluorophosphate, HOBt: 1-hydroxybenzotriazole hydrate, DCC: N,N'-dicyclohexylcarbodiimide, DMAP: 4-dimethylaminopyridine and mCPBA: meta-chloroperoxybenzoic acid.

### [Preparation Example I]

The compounds corresponding to Formula I of the present invention were synthesized as follows:

### [Preparation Example 1-1] 4-(3-aminoprop-1-yn-1-yl)aniline hydrochloride

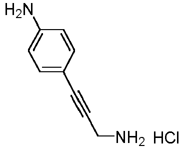

### Step 1: tert-butyl (3-(4-aminophenyl)prop-2-yn-1-yl)carbamate (Intermediate 1-1a)

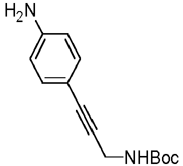

After 4-lodoaniline (1.0 mmol, 1.0 eq), n-boc propargylamine (1.5 eq), Pd(PPh₃)₄ (0.01 eq), Cul (0.03 eq), DEA (1.5 eq), and Et₂O (3.0 mL) were mixed, a gas in a reactor was replaced with an Ar gas, and then the resulting mixture was stirred at room temperature overnight. The reactant was extracted with EtOAc and H₂O to obtain an organic layer. The obtained organic layer was dried over Na₂SO₄. The mixture was filtered under reduced pressure, distilled, and then purified through flash column to synthesize Intermediate 1-1a.

### Step 2: 4-(3-aminoprop-1-yn-1-yl)aniline hydrochloride

Intermediate 1-1a (0.5 mmol, 1.0 eq) synthesized in Step 1 was dissolved in 3.0 mL of DCM, and then a HCl solution (4.0 M in dioxane, 2.0 eq) was added dropwise thereto. After one hour, the resulting solid was filtered and then dried to synthesize Example 1-1 without a separate purification process.

¹H NMR (500 MHz, DMSO-d₆) δ 8.41 (s, 3H), 7.24 (d, *J* = 8.4 Hz, 2H), 6.83 (d, *J* = 8.1 Hz, 2H), 3.89 (q, *J* = 5.6 Hz, 2H)

### [Preparation Example 1-3] 3-(3-aminoprop-1-yn-1-yl)aniline hydrochloride

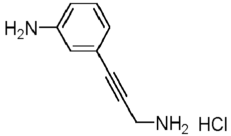

Example 1-3 was synthesized in the same manner as Preparation Example 1-1 from 3-iodoaniline via Intermediate 1-3a (tert-butyl (3-(3-aminophenyl)prop-2-yn-1-yl)carbamate).

¹H NMR (500 MHz, DMSO-d₆) δ 8.57 (s, 3H), 7.42 (d, *J* = 7.4 Hz, 1H), 7.29 (dd, *J* = 24.4, 14.7 Hz, 3H), 3.99 (t, *J* = 5.5 Hz, 2H)

### [Preparation Example 3-1] methyl-5-amino-2-(3-aminoprop-1-yn-1-yl)benzoate hydrochloride

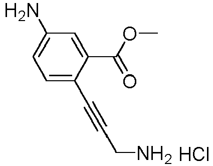

Example 3-1 was synthesized in the same manner as Preparation Example 1-1 from methyl-5-amino-2-iodobenzoate via Intermediate 3-1a (methyl-5-amino-2-(3-((tert butoxycarbonyl)amino)prop-1-yn-1-yl)benzoate).

¹H NMR (500 MHz, DMSO-d₆) δ 8.42 (s, 3H), 7.26 (d, *J* = 8.4 Hz, 1H), 7.13 (d, *J* = 2.4 Hz, 1H), 6.82 (dd, *J* = 8.4, 2.4 Hz, 1H), 3.90 (q, *J* = 5.7 Hz, 2H), 3.79 (s, 3H)

### [Preparation Example 3-25] methyl 4-amino-2-(3-aminoprop-1-yn-1-yl)benzoate hydrochloride

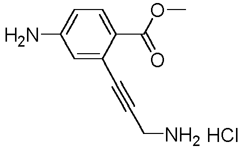

Example 3-25 was synthesized in the same manner as Preparation Example 1-1 from methyl 4-amino-2-iodobenzoate via Intermediate 3-25a (methyl 4-amino-2-(3-((tert-butoxycarbonyl)amino)prop-1-yn-1-yl)benzoate).

¹H NMR (500 MHz, DMSO-d₆) δ 8.47 (s, 3H), 7.67 (d, *J* = 8.7 Hz, 1H), 6.72 (dd, *J* = 4.9, 2.3 Hz, 1H), 6.62 (ddd, *J* = 8.7, 4.3, 2.2 Hz, 1H), 3.99 (dd, *J* = 5.8, 3.0 Hz, 2H), 3.74 (s, 3H)

### [Preparation Example 3-49] 2-amino-4-(4-aminophenyl)but-3-yn-1-ol hydrochloride

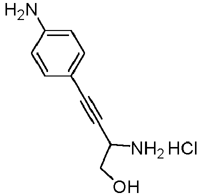

### Step 1: tert-butyl 4-ethynyl-2,2-dimethyloxazolidine-3-carboxylate (Intermediate 3-49a)

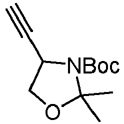

*tert*-Butyl 4-formyl-2,2-dimethyloxazolidine-3-carboxylate (8.7 mmol, 1.0 eq), and dimethyl-1-diazo-2-oxopropyl phosphonate (1.5 eq) were dissolved in MeOH, and then K₂CO₃ (2.0 eq) was added thereto at 0 °C, and the resulting mixture was stirred at 0 °C for 1 hour, and then stirred at room temperature overnight. The reactant was extracted with n-hexane and H₂O to obtain an organic layer. The obtained organic layer was dried over Na₂SO₄. The mixture was filtered under reduced pressure, distilled, and then purified through flash column to synthesize a compound.

### Step 2: tert-butyl 4-((4-aminophenyl)ethynyl)-2,2-dimethyloxazolidine-3-carboxylate (Intermediate 3-49b)

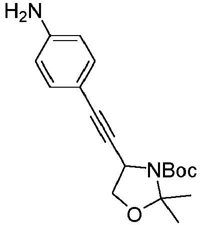

Intermediate 3-49a (4.4 mmol, 1.0 eq) synthesized in Step 1, 4-iodoaniline (1.0 eq), PdCl₂(PPh₃)₂ (0.1 eq), and Cul (0.1 eq) were dissolved in 40.0 mL of DEA, and then the resulting mixture was stirred at room temperature overnight. The reactant was distilled under reduced pressure, and then purified through flash column to synthesize a compound.

### Step 3: 2-amino-4-(4-aminophenyl)but-3-yn-1-ol hydrochloride

Example 3-49 was synthesized in the same manner as in step 2 of Preparation Example 1-1 from Intermediate 3-49b synthesized in Step 2.

¹H NMR (500 MHz, DMSO-d₆) δ 7.98 (s, 3H), 7.38 (d, *J* = 8.4 Hz, 2H), 6.63 - 6.61 (m, 2H), 3.60 - 3.57 (m, 2H), 3.51 (d, *J* = 4.9 Hz, 1H)

### [Preparation Example 5-116] methyl 2,4-bis(3-aminoprop-1-yn-1-yl)benzoate dihydrochloride

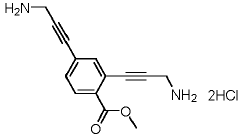

### Step 1: methyl 4-bromo-2-(3-((tert-butoxycarbonyl)amino)prop-1-yn-1-yl)benzoate (Intermediate 5-116a)

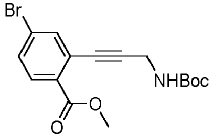

Intermediate 5-116a was synthesized in the same manner as in step 1 of Preparation Example 1-1 from methyl 4-bromo-2-iodobenzoate.

### Step 2: methyl 2-(3-((tert-butoxycarbonyl)amino)prop-1-yn-1-yl)-4-(3-(1,3-dioxoisoindolin-2-yl)prop-1-yn-1-yl)benzoate (Intermediate 5-116b)

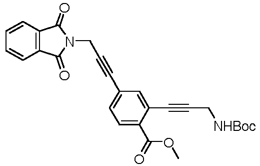

After Intermediate 5-116a (0.3 mmol, 1.0 eq), n-propargylphthalimide (1.5 eq), Pd(PPh₃)₄ (0.1 eq), Cul (0.3 eq), TBAI (3.0 eq), DMF (3.0 mL), and TEA (1.0 mL) were mixed, a gas in a reactor was replaced with an Ar gas, and then the resulting mixture was heated in M.W at 110 °C for 20 minutes. The reactant was extracted with EtOAc and H₂O to obtain an organic layer. The obtained organic layer was dried over Na₂SO₄. The mixture was filtered under reduced pressure, distilled, and then purified through flash column to synthesize a compound.

### Step 3: methyl 4-(3-aminoprop-1-yn-1-yl)-2-(3-((tert-butoxycarbonyl)amino)prop-1-yn-1-yl)benzoate (Intermediate 5-116c)

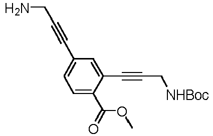

Intermediate 5-116b (0.17 mmol, 1.0 eq), hydrazine monohydrate (3.0 eq), MeOH (0.8 mL), and H₂O (0.2 mL) were mixed and then the resulting mixture was stirred at 50 °C for 2 hours. The reactant was extracted with EtOAc and H₂O to obtain an organic layer. The obtained organic layer was dried over Na₂SO₄, and then distilled under reduced pressure to synthesize a compound without a separate purification process.

### Step 4: methyl-2,4-bis(3-aminoprop-1-yn-1-yl)benzoate dihydrochloride

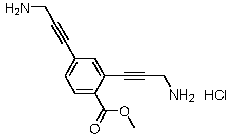

Example 5-116 was synthesized in the same manner as in step 2 of Preparation Example 1-1 from Intermediate 5-116c.

¹H NMR (500 MHz, DMSO-d₆) δ 8.61 (d, *J* = 14.6 Hz, 6H), 7.93 - 7.88 (m, 1H), 7.61 (d, *J* = 1.7 Hz, 1H), 7.57 (dd, *J* = 8.2, 1.7 Hz, 1H), 3.98 (dd, *J* = 5.9, 3.0 Hz, 4H), 3.84 (s, 3H).

### [Preparation Example II]

The compounds corresponding to Formula II of the present invention were synthesized as follows:

### [Preparation Example 4-162] 4-amino-N-(4-(3-aminoprop-1-yn-1-yl)phenyl)butanamide dihydrochloride

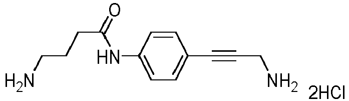

Example 4-162 was synthesized in the same manner as in Preparation Example 1-1' from 4-((*tert*-butoxycarbonyl)amino)butanoic acid and Intermediate 1-1a.

¹H NMR (500 MHz, DMSO-d₆) δ 10.46 (s, 1H), 8.45 (s, 3H), 7.99 (s, 3H), 7.68 - 7.62 (m, 2H), 7.38 - 7.32 (m, 2H), 3.92 (q, *J* = 5.6 Hz, 2H), 2.78 (h, *J* = 6.2 Hz, 2H), 2.43 (d, *J* = 7.2 Hz, 2H), 1.83 (p, *J* = 7.3 Hz, 2H).

### [Preparation Example 4-166] 5-(3-aminoprop-1-yn-1-yl)-N-(3-aminopropyl)furan-2-carboxamide dihydrochloride

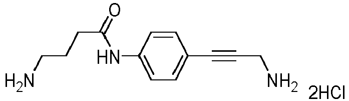

Example 4-166 was synthesized in the same manner as in Preparation Example 1-1' from tert-butyl (3-aminopropyl)carbamate and Intermediate 4-227b.

¹H NMR (500 MHz, DMSO-d₆) δ 8.79 (t, *J* = 5.9 Hz, 1H), 8.67 (s, 3H), 8.00 (s, 3H), 7.21 (d, *J* = 3.6 Hz, 1H), 6.96 (d, *J* = 3.6 Hz, 1H), 4.02 (q, *J* = 5.6 Hz, 2H), 3.24 (q, *J* = 6.5 Hz, 2H), 2.75 (dq, *J* = 11.9, 6.2 Hz, 2H), 1.78-1.71 (m, 2H).

Additionally, Compound 4-166, to which biotin was bonded, was prepared as follows in order to be used in a pulldown assay using biotin.

### 5-(3-aminoprop-1-yn-1-yl)-N-(3-(5-((3aS,4S,6aR)-2-oxohexahydro-1H-thieno[3,4-d]imidazol-4-yl)pentanamido)propyl)furan-2-carboxamide hydrochloride

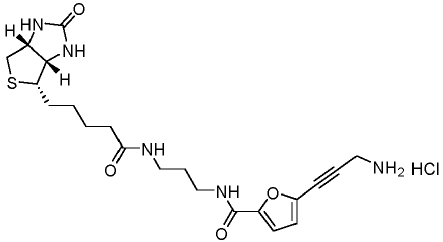

### Step 1: tert-butyl (3-(5-((3aS,4S,6aR)-2-oxohexahydro-1H-thieno[3,4-d]imidazol-4-yl)pentanamido)propyl)carbamate

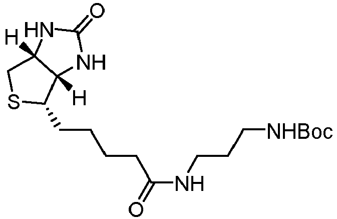

Intermediate, *tert*-Butyl (3-(5-((3aS,4S,6aR)-2-oxohexahydro-1H-thieno[3,4-d]imidazol-4-yl)pentanamido)propyl)carbamate was synthesized in the same manner as in step 1 of Preparation Example 1-1' from biotin and tert-butyl (3-aminopropyl)carbamate.

### Step 2: tert-butyl (3-(5-((3-(5-((3aS,4S,6aR)-2-oxohexahydro-1H-thieno[3,4-d]imidazol-4-yl)pentanamido)propyl)carbamoyl)furan-2-yl)prop-2-yn-1-yl)carbamate

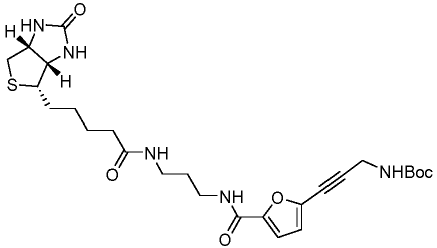

Intermediate, *tert*-Butyl (3-(5-((3-(3-(3-((3aS,4S,6aR)-2-oxohexahydro-1H-thieno[3,4-d]imidazol-4-yl)phenanamigo)carbonyl)furan-2-yl)prop-2-yn-1-yl)carbamate was synthesized in the same manner as in step 1 of Preparation Example 1-1'.

Step 3: 5-(3-aminoprop-1-yn-1-yl)-N-(3-(5-((3aS,4S,6aR)-2-oxohexahydro-1H-thieno[3,4-d]imidazol-4-yl)pentanamido)propyl)furan-2-carboxamide hydrochloride

Compound 4-166, to which biotin was bonded, was synthesized in the same manner as in step 2 of Preparation Example 1-1 from Intermediate of step 2 above.

¹H NMR (500 MHz, DMSO-d₆) δ 8.61 - 8.48 (m, 4H), 7.86 (t, *J* = 5.7 Hz, 1H), 7.19 (d, *J* = 3.6 Hz, 1H), 7.00 (d, *J* = 3.6 Hz, 1H), 6.44 (s, 1H), 4.30 (dd, *J* = 7.7, 4.6 Hz, 1H), 4.14 - 4.10 (m, 1H), 4.08 (q, *J* = 5.6 Hz, 2H), 3.20 (q, *J* = 6.2 Hz, 2H), 3.12 - 3.03 (m, 3H), 2.81 (dd, *J* = 12.4, 5.1 Hz, 1H), 2.57 (d, *J* = 12.4 Hz, 1H), 2.06 (t, *J* = 7.4 Hz, 2H), 1.63 - 1.56 (m, 3H), 1.54 - 1.43 (m, 3H), 1.33 - 1.25 (m, 2H), MS m/z [M+H]⁺ 448.43.

### [Preparation Example 4-228] methyl 4-(4-aminobutanamido)-2-(3-aminoprop-1-yn-1-yl)benzoate dihydrochloride

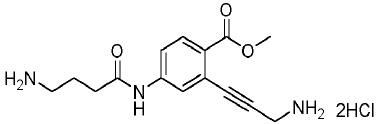

Example 4-228 was synthesized in the same manner as in Preparation Example 1-1' from 4-((*tert*-butoxycarbonyl)amino)butanoic acid and Intermediate 3-25a via Intermediate 4-228a (methyl 4-(4-aminobutanamido)-2-(3-((*tert-*butoxycarbonyl)amino)prop-1-yn-1-yl)benzoate).

¹H NMR (500 MHz, DMSO-*d*₆) δ 10.92 (s, 1H), 8.60 (s, 3H), 8.13 (s, 3H), 8.08 (d, *J* = 2.2 Hz, 1H), 7.88 (d, *J* = 8.7 Hz, 1H), 7.71 (dd, *J* = 8.7, 2.2 Hz, 1H), 4.07 - 3.99 (m, 2H), 3.83 (s, 3H), 2.96 (dt, *J* = 9.2, 4.6 Hz, 2H), 2.82 (q, *J* = 6.7 Hz, 2H), 2.53 (t, *J* = 7.3 Hz, 2H).

Additionally, Compound 4-228, to which biotin was bonded, was prepared as follows in order to be used in a pulldown assay using biotin.

### methyl 2-(3-aminoprop-1-yn-1-v))-4-(4-(5-((3aS,4S,6aR)-2-oxohexahydro-1H-thieno[3,4-d]imidazol-4-yl)pentanamido)butanamido)benzoate hydrochloride

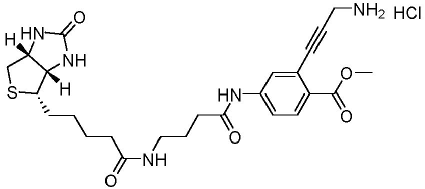

### Step 1: methyl 4-(4-((((9H-fluoren-9-yl)methoxy)carbonyl)amino)butanamido)-2-(3-((tert-butoxycarbonyl)amino)prop-1-yn-1-yl)benzoate

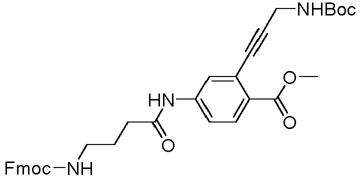

Intermediate 3-25a (0.2 mmol, 1.0 eq), 4-((((9H-fluoren-9-yl)methoxy)carbonyl)amino)butanoic acid (1.0 eq), and HATU (1.1 eq) were dissolved in dry DMF (0.7 mL) and then a gas in a reactor was replaced with an Ar gas. TEA (2.0 eq) was added dropwise thereto, and then the resulting mixture was stirred at room temperature for 6 hours. The reactant was purified through flash column to synthesize methyl 4-(4-((((9H-fluoren-9-yl)methoxy)carbonyl)amino)butanamido)-2-(3-((*tert*-butoxycarbonyl)amino)prop-1-yn-1-yl)benzoate, Intermediate.

### Step 2: methyl-2-(3-((tert-butoxycarbonyl)amino)prop-1-yn-1-yl)-4-(4-(5-((3aS,4S,6aR)-2-oxohexahydro-1H-thieno[3,4-d]imidazol-4-yl)pentanamido)butanamido)benzoate

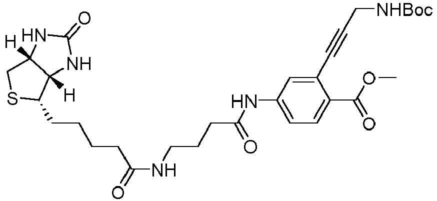

Intermediate (0.06 mmol, 1.0 eq) prepared in step 1 was dissolved in 0.3 mLof piperidine/DMF (1:4), and then the resulting mixture was stirred at room temperature for 1 hour. The reactant was concentrated under reduced pressure, and then biotin (1.0 eq) was added thereto, and methyl-2-(3-((*tert*-butoxycarbonyl)amino)prop-1-yn-1-yl)-4-(4-(5-((3aS,4S,6aR)-2-oxohexahydro-1H-thieno[3,4-d]imidazol-4-yl)pentanamido)butanamido)benzoate, Intermediate, was synthesized in the same manner as in step 1 above.

### Step 3: methyl-2-(3-aminoprop-1-yn-1-yl)-4-(4-(5-((3aS,4S,6aR)-2-oxohexahydro-1H-thieno[3,4-d]imidazol-4-yl)pentanamido)butanamido)benzoate hydrochloride

Compound 4-228, to which biotin was bonded, was synthesized in the same manner as in step 2 of Preparation Example 1-1 from Intermediate prepared in step 2.

¹H NMR (500 MHz, DMSO-d₆) δ 10.43 (s, 1H), 8.36 (s, 3H), 8.13 (d, *J* = 2.2 Hz, 1H), 7.89 (d, *J* = 8.7 Hz, 1H), 7.85 (t, *J* = 5.5 Hz, 1H), 7.55 (dd, *J* = 8.7, 2.2 Hz, 1H), 6.43 (s, 1H), 6.38 (s, 1H), 4.29 (dd, *J* = 7.6, 4.7 Hz, 1H), 4.14 - 4.08 (m, 1H), 4.06 (q, *J* = 5.6 Hz, 2H), 3.83 (s, 3H), 3.10 - 3.05 (m, 3H), 2.80 (dd, *J* = 12.4, 5.1 Hz, 1H), 2.56 (d, *J* = 12.6 Hz, 1H), 2.38 - 2.32 (m, 3H), 2.05 (t, *J* = 7.4 Hz, 2H), 1.73 - 1.67 (m, 2H), 1.62 - 1.56 (m, 1H), 1.53 - 1.46 (m, 2H), 1.31 - 1.26 (m, 2H), MS m/z [M+H]⁺ 516.47.

### [Preparation Example 5-8] 4-amino-N-(4-(3-aminoprop-1-yn-1-yl)-3-(hydroxymethyl)phenyl)butanamide trifluoroacetic acid

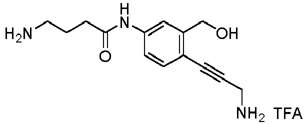

Example 5-8 was synthesized by sequentially applying the same manner as in step 5 of Preparation Example 3-3' and step 2 of Preparation Example 1-1 from 4-((*tert-*butoxycarbonyl)amino)butanoic acid and Intermediate 3-27c to synthesize a compound and then further purifying the compound with prep HPLC.

¹H NMR (500 MHz, DMSO-*d*₆) δ 7.62 (d, *J* = 2.2 Hz, 1H), 7.44 (dd, *J* = 8.4, 2.2 Hz, 1H), 7.35 (d, *J* = 8.3 Hz, 1H), 4.58 (s, 2H), 3.91 (s, 2H), 2.84 (t, *J* = 7.7 Hz, 2H), 2.40 (d, *J* = 7.3 Hz, 2H), 1.86 - 1.82 (m, 2H); MS m/z [M+Na]⁺ 284.30.

### [Preparation Example 5-18] methyl 2-(3-aminoprop-1-yn-1-yl)-4-((3-aminopropyl)carbamoyl)benzoate dihydrochloride

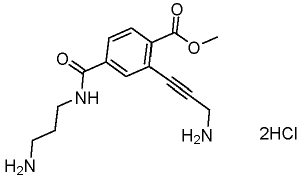

### Step 1: methyl-2-bromo-4-((3-((tert-butoxycarbonyl)amino)propyl)carbamoyl)benzoate (5-18a)

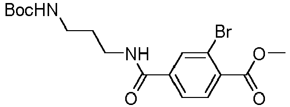

Intermediate 5-18a was synthesized in the same manner as in step 5 of Preparation Example 4-1' from *tert*-butyl (3-aminopropyl)carbamate 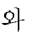 3-bromo-4-(methoxycarbonyl)benzoic acid.

### Step 2: methyl-2-(3-aminoprop-1-yn-1-yl)-4-((3-aminopropyl)carbamoyl)benzoate dihydrochloride

Example 5-18 was synthesized in the same manner as in Preparation Example 1-1 from Intermediate 5-18a.

¹H NMR (500 MHz, DMSO-*d*₆) δ 9.01 (s, 1H), 8.52 (s, 3H), 8.09 - 8.08 (m, 1H), 8.01 - 7.99 (m, 2H), 7.95 (s, 3H), 4.08 - 4.03 (m, 2H), 3.90 (s, 3H), 3.35 - 3.29 (m, 2H), 2.86 - 2.80 (m, 2H), 1.82 (p, J = 6.9 Hz, 2H).

### [Preparation Example 5-81] 4-amino-N-(3-(3-aminoprop-1-yn-1-yl)-4-(2-(trifluoromethyl)pyrimidin-5-yl)phenyl)butanamide dihydrochloride

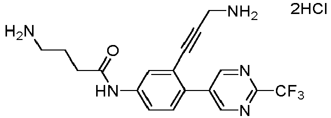

Example 5-81 was synthesized in the same manner as in Preparation Example 1-1' from 4-((*tert*-butoxycarbonyl)amino)butanoic acid and Intermediate 5-81c.

¹H NMR (500 MHz, DMSO-d₆) δ 10.69 (s, 1H), 9.30 (s, 2H), 8.38 (s, 3H), 8.16 (d, *J* = 2.2 Hz, 1H), 7.97 (s, 3H), 7.77 (dd, *J* = 8.6, 2.2 Hz, 1H), 7.66 (d, *J* = 8.5 Hz, 1H), 3.94 (s, 2H), 2.85 (s, 2H), 2.53 (s, 2H), 1.90 - 1.87 (m, 2H).

### [Preparation Example 5-109] 11-((3-(3-aminoprop-1-yn-1-yl)phenyl)amino)-11-oxoundecanoic acid hydrochloride

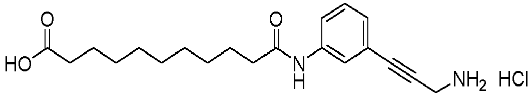

Example 5-109 was synthesized in the same manner as in step 2 of Preparation Example 5-77' from undecanedioic acid and Intermediate 1-3a.

¹H NMR (500 MHz, DMSO-*d*₆) δ 10.15 (s, 1H), 8.45 (s, 3H), 7.96 - 7.91 (m, 1H), 7.53 - 7.49 (m, 1H), 7.33 (t, *J* = 7.9 Hz, 1H), 7.11 (dt, *J* = 7.7, 1.2 Hz, 1H), 3.99 (s, 2H), 2.34 - 2.24 (m, 4H), 1.61 - 1.44 (m, 4H), 1.30 - 1.19 (m, 10H).

### [Preparation Example 5-110] 11-((3-(3-aminoprop-1-yn-1-yl)-4-(methoxycarbonyl)phenyl)amino)-11-oxoundecanoic acid hydrochloride

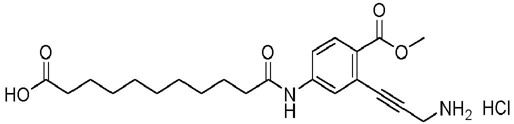

Example 5-110 was synthesized in the same manner as in step 2 of Preparation Example 5-77' from undecanedioic acid and Intermediate 3-25a.

¹H NMR (500 MHz, DMSO-d₆) δ 10.43 (s, 1H), 8.85 (s, 1H), 8.41 (s, 3H), 8.13 (d, *J* = 2.2 Hz, 1H), 7.89 (d, *J* = 8.7 Hz, 1H), 7.56 (dd, *J* = 8.7, 2.2 Hz, 1H), 4.04 (s, 2H), 3.83 (s, 3H), 2.34 (t, *J=* 7.4 Hz, 2H), 2.25 (d, *J* = 7.4 Hz, 2H), 1.57 (t, *J* = 7.0 Hz, 2H), 1.54 - 1.42 (m, 12H).

### [Preparation Example 5-150] methyl 4-(4-amino-N-methylbutanamido)-2-(3-aminoprop-1-yn-1-yl)benzoate hydrochloride

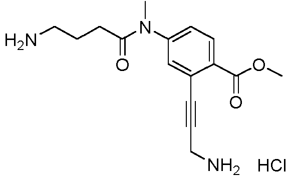

Example 5-150 was synthesized in the same manner as in step 2 of Preparation Example 5-77' from 4-aminobutanoic acid and Intermediate 5-150a.

¹H NMR (500 MHz, DMSO-d₆) δ 8.55 (s, 3H), 7.95 -7.93 (m, 1H), 7.90 (s, 3H), 7.63 - 7.59 (m, 1H), 7.54 - 7.51 (m, 1H), 4.06 - 4.00 (m, 2H), 3.88 (s, 3H), 3.23 (s, 3H), 2.95 - 2.84 (m, 2H), 2.81 - 2.72 (m, 2H), 1.81 - 1.73 (m, 2H).

### [Preparation Example III]

The compounds corresponding to Formula III of the present invention were synthesized as follows:

### [Preparation Example 4-2] methyl 2-(3-aminoprop-1-yn-1-yl)-5-(piperazin-1-yl)benzoate hydrochloride

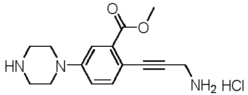

Example 4-2 was synthesized in the same manner as in Preparation Example 5-7 from methyl-2-bromo-5-fluorobenzoate.

¹H NMR (500 MHz, DMSO-*d*₆) δ 9.40 (s, 2H), 8.46 (s, 3H), 7.42 (d, *J* = 8.7 Hz, 1H), 7.34 (d, *J* = 2.7 Hz, 1H), 7.18 (dd, *J* = 8.8, 2.8 Hz, 1H), 3.93 (q, *J* = 5.7 Hz, 2H), 3.82 (s, 3H), 3.47 (s, 4H), 3.15 (s, 4H)

### [Preparation Example 5-7] methyl 2-(3-aminoprop-1-yn-1-yl)-4-(piperazin-1-yl)benzoate hydrochloride

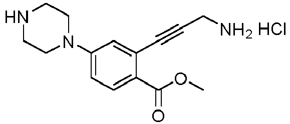

### Step 1: methyl 2-(3-((tert-butoxycarbonyl)amino)prop-1-yn-1-yl)-4-fluorobenzoate (Intermediate 5-7a)

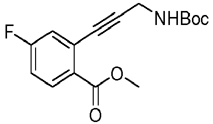

Intermediate 5-7a was synthesized in the same manner as in step 2 of Preparation Example 4-179' from methyl-2-bromo-4-fluorobenzoate.

### Step 2: methyl-2-(3-((tert-butoxycarbonyl)amino)prop-1-yn-1-yl)-4-(piperazin-1-yl)benzoate (Intermediate 5-7b)

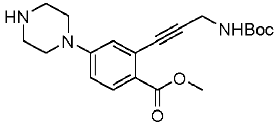

Intermediate 5-7a (0.6 mmol, 1.0 eq), piperazine (3.0 eq), K₂CO₃ (1.5 eq), and DMSO (2.0 mL) were mixed and then a gas in a reactor was replaced with an Ar gas. After external light was blocked, the resulting mixture was stirred at 95 °C for 18 hours. The reactant was extracted with EtOAc and H₂O to obtain an organic layer. The obtained organic layer was dried over Na₂SO₄. The mixture was filtered under reduced pressure, distilled, and then purified through flash column to synthesize Intermediate 5-7b.

### Step 3: methyl 2-(3-aminoprop-1-yn-1-yl)-4-(piperazin-1-yl)benzoate hydrochloride

Example 5-7 was synthesized in the same manner as in step 2 of Preparation Example 1-1 from Intermediate 5-7b.

¹H NMR (500 MHz, DMSO-d₆) δ 9.35 (s, 2H), 8.50 (s, 3H), 7.79 (d, *J* = 8.9 Hz, 1H), 7.10 (d, *J* = 2.7 Hz, 1H), 7.06 (dd, *J* = 8.9, 2.7 Hz, 1H), 3.97 (q, *J* = 5.6 Hz, 2H), 3.77 (s, 3H), 3.53 (t, *J =* 5.3 Hz, 4H), 3.14 (d, *J* = 13.1 Hz, 4H).

### [Preparation Example 5-23] methyl-2-(3-aminoprop-1-yn-1-yl)-4-(piperidine-4-carboxamido)benzoate hydrochloride

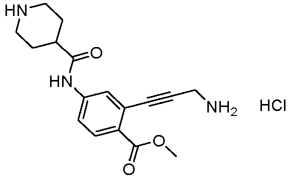

Example 5-23 was synthesized in the same manner as in Preparation Example 1-1' from 1-(*tert*-butoxycarbonyl)piperidine-4-carboxylic acid and Intermediate 3-25a.

¹H NMR (500 MHz, DMSO-d₆) δ 10.85 (s, 1H), 9.04 (d, *J* = 11.2 Hz, 1H), 8.78 (d, *J* = 11.0 Hz, 1H), 8.47 (s, 3H), 8.06 (d, *J* = 2.2 Hz, 1H), 7.86 (d, *J* = 8.7 Hz, 1H), 7.64 (dd, *J* = 8.7, 2.2 Hz, 1H), 4.00 (q, *J* = 5.6 Hz, 2H), 3.80 (s, 3H), 3.31 - 3.23 (m, 2H), 2.86 (q, *J =* 11.7 Hz, 2H), 2.72 (tt, *J* = 11.1, 3.8 Hz, 1H), 1.94 (d, *J* = 13.4 Hz, 2H), 1.83 - 1.74 (m, 2H).

### [Preparation Example 5-28] methyl-2-(3-aminoprop-1-yn-1-yl)-4-(3-(piperidine-4-carboxamido)prop-1-yn-1-yl)benzoate dihydrochloride

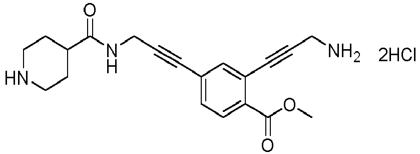

Example 5-28 was synthesized in the same manner as in Preparation Example 1-1' from 1-(*tert*-butoxycarbonyl)piperidine-4-carboxylic acid and Intermediate 5-116c.

¹H NMR (500 MHz, DMSO-d₆) δ 8.56 (dd, *J* = 12.3, 6.8 Hz, 4H), 7.88-7.84 (m, 1H), 7.57 (d, *J* = 1.8 Hz, 1H), 7.51 (dd, *J* = 8.2, 1.7 Hz, 1H), 4.11 (d, *J* = 5.4 Hz, 2H), 3.98 (q, *J* = 5.6 Hz, 2H), 3.83 (s, 3H), 3.21 (d, *J* = 12.5 Hz, 2H), 2.89 - 2.77 (m, 2H), 2.42 (dt, *J* = 11.0, 3.9 Hz, 1H), 1.85 - 1.80 (m, 2H), 1.75 - 1.68 (m, 2H).

### [Preparation Example 5-29] methyl 2-(3-aminoprop-1-yn-1-yl)-4-(4-(piperidin-4-ylmethyl)piperazin-1-yl)benzoate dihydrochloride

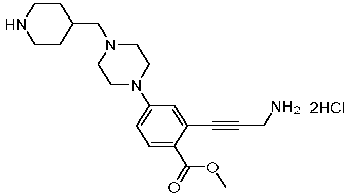

### Step 1: (9H-fluoren-9-yl)methyl 4-(bromomethyl)piperidine-1-carboxylate (Intermediate 5-29a)

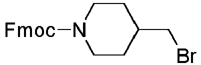

4-(bromomethyl)piperidine hydrochloride (1.0 eq), (9H-fluoren-9-yl)methyl carbonochloridate (1.2 eq), pyridine (1.5 eq), and dry DCM were mixed, and then the resulting mixture was stirred at room temperature for 2 hours. The reactant was extracted with EtOAc and H₂O to obtain an organic layer. The obtained organic layer was dried over Na₂SO₄. The mixture was filtered under reduced pressure, distilled, and then purified through flash column to synthesize Intermediate 5-29a.

### Step 2: (9H-fluoren-9-yl)methyl 4-(piperazin-1-ylmethyl)piperidine-1-carboxylate (Intermediate 5-29b)

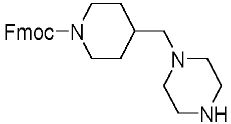

Intermediate 5-29a (1.0 eq), piperazine (9.0 eq), and dry ACN were mixed, and then the mixture was stirred at 80 °C overnight. The reactant was extracted with EtOAc and H₂O to obtain an organic layer. The obtained organic layer was dried over Na₂SO₄. The mixture was filtered under reduced pressure, distilled, and then purified through flash column to synthesize Intermediate 5-29b.

### Step 3: (9H-fluoren-9-yl)methyl 4-((4-(3-(3-((tert-butoxycarbonyl)amino)prop-1-yn-1-yl)-4-(methoxycarbonyl)phenyl)piperazin-1-yl)methyl)piperidine-1-carboxylate (Intermediate 5-29c)

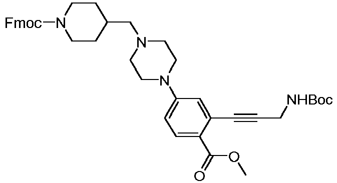

Intermediate 5-29b (1.0 eq), Intermediate 5-116a (1.0 eq), RuPhos Pd G₃ (0.1 eq), CS₂CO₃ (3.0 eq), and dioxane (1.5 mL) were mixed, and then the mixture was stirred at 100 °C overnight. The reactant was extracted with EtOAc and H₂O to obtain an organic layer. The obtained organic layer was dried over Na₂SO₄. The mixture was filtered under reduced pressure, distilled, and then purified through flash column to synthesize Intermediate 5-29c.

### Step 4: methyl-2-(3-((tert-butoxycarbonyl)amino)prop-1-yn-1-yl)-4-(4-(piperidin-4-ylmethyl)piperazin-1-yl)benzoate (Intermediate 5-29d)

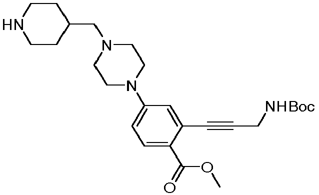

Intermediate 5-29c (1.0 eq), DEA (1.0 mL), and THF (1.0 mL) were mixed, and then the mixture was stirred at room temperature for 1 hour. The reactant was distilled under reduced pressure, and then purified through flash column to synthesize Intermediate 5-29d.

### Step 5: methyl-2-(3-aminoprop-1-yn-1-yl)-4-(4-(piperidin-4-ylmethyl)piperazin-1-yl)benzoate hydrochloride

Example 5-29 was synthesized in the same manner as in step 2 of Preparation Example 1-1 from Intermediate 5-29d.

¹H NMR (500 MHz, DMSO-d₆) δ 9.12 - 8.84 (m, 2H), 8.60 (s, 3H), 7.83 (d, *J* = 8.9 Hz, 1H), 7.20 (d, *J* = 2.7 Hz, 1H), 7.13 (dd, *J* = 9.0, 2.7 Hz, 1H), 3.99 (s, 2H), 3.81 (s, 3H), 3.54 - 3.45 (m, 6H), 3.26 (d, *J* = 12.6 Hz, 2H), 3.08 (s, 4H), 2.85 (q, *J* = 11.8 Hz, 2H), 2.18 (s, 1H), 2.07 - 2.00 (m, 2H), 1.45 (d, *J* = 12.3 Hz, 2H).

### [Preparation Example 5-39] methyl 2-(3-aminoprop-1-yn-1-yl)-4-(2,7-diazaspiro[3.5]nonane-7-carbonyl)benzoate dihydrochloride

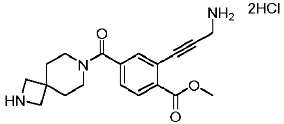

Example 5-39 was synthesized in the same manner as in Preparation Example 5-41 using *tert*-butyl 2,7-diazaspiro[3.5]nonane-2-carboxylate.

¹H NMR (500 MHz, DMSO-d₆) δ 8.49 (s, 3H), 7.92 (d, *J* = 8.0 Hz, 1H), 7.86 (d, *J* = 8.0 Hz, 1H), 7.28 (d, *J* = 1.7 Hz, 1H), 4.00 (s, 2H), 3.85 (s, 3H), 3.68 (s, 8H), 3.53 (s, 4H).

### [Preparation Example 5-40] methyl 2-(3-aminoprop-1-yn-1-yl)-4-(2,6-diazaspiro[3.3]heptan-2-yl)benzoate dihydrochloride

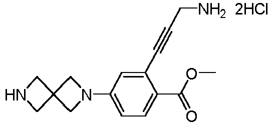

### Step 1: tert-butyl 6-(3-(3-(1,3-dioxoisoindolin-2-yl)prop-1-yn-1-yl)-4-(methoxycarbonyl)phenyl)-2,6-diazaspiro[3.3]heptane-2-carboxylate (Intermediate 5-40a)

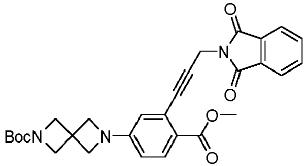

Intermediate 5-35a (0.5 mmol, 1.0 eq), *tert*-butyl 2,6-diazaspiro[3.3]heptane-2-carboxylate (1.0 eq), SPhos Pd G3 (0.1 eq), CS₂CO₃ (3.0 eq), and dioxane (1.5 mL) were mixed and then the resulting mixture was stirred at 100 °C overnight. The reactant was extracted with EtOAc and H₂O to obtain an organic layer. The obtained organic layer was dried over Na₂SO₄. The mixture was filtered under reduced pressure, distilled, and then purified through flash column to synthesize Intermediate 5-40a.

### Step 2: methyl-2-(3-(1,3-dioxoisoindolin-2-yl)prop-1-yn-1-yl)-4-(2,6-diazaspiro[3.3]heptan-2-yl)benzoate trifluoroacetic acid (Intermediate 5-40b)

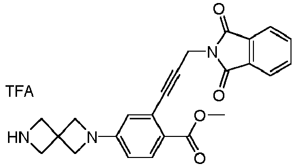

Example 5-40b was synthesized in the same manner as in step 4 of Preparation Example 4-1' from Intermediate 5-40a.

### Step 3: methyl-2-(3-aminoprop-1-yn-1-yl)-4-(2,6-diazaspiro[3.3]heptan-2-yl)benzoate dihydrochloride

Example 5-40 was synthesized by sequentially applying the same manner as in step 3 of Preparation Example 5-116 and step 2 of Preparation Example 1-1 from Intermediate 5-40b.

¹H NMR (500 MHz, DMSO- d₆) δ 9.65 (s, 2H), 8.71 (s, 3H), 7.31 (d, *J* = 8.7 Hz, 1H), 6.72 (d, *J* = 2.3 Hz, 1H), 6.67 (d, *J* = 9.6 Hz, 1H), 3.98 (s, 2H), 3.96 (s, 3H), 3.65 - 3.62 (m, 4H), 3.56 (s, 4H).

### [Preparation Example 5-41] methyl 2-(3-aminoprop-1-yn-1-yl)-4-(2,6-diazaspiro[3.3]heptane-2-carbonyl)benzoate dihydrochloride

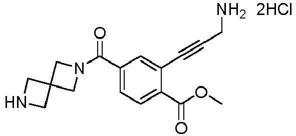

### Step 1: tert-butyl 6-(3-iodo-4-(methoxycarbonyl)benzoyl)-2,6-diazaspiro[3.3]heptane-2-carboxylate (Intermediate 5-41a)

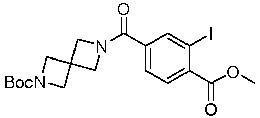

Intermediate 5-41a was synthesized in the same manner as in step 1 of Preparation Example 4-1' from 3-iodo-4-(methoxycarbonyl)benzoic acid and *tert*-butyl 2,6-diazaspiro[3.3]heptane-2-carboxylate.

### Step 2: tert-butyl 6-(3-(3-(1,3-dioxoisoindolin-2-yl)prop-1-yn-1-yl)-4-(methoxycarbonyl)benzoyl)-2,6-diazaspiro[3.3]heptane-2-carboxylate (Intermediate 5-41b)

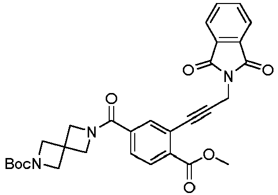

Intermediate 5-41b was synthesized in the same manner as in step 2 of Preparation Example 5-85' from Intermediate 5-41a.

### Step 3: methyl-2-(3-(1,3-dioxoisoindolin-2-yl)prop-1-yn-1-yl)-4-(2,6-diazaspiro[3.3]heptane-2-carbonyl)benzoate trifluoroacetic acid (Intermediate 5-41c)

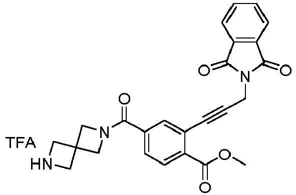

Intermediate 5-41c was synthesized in the same manner as in step 4 of Preparation Example 4-1' from Intermediate 5-41b.

### Step 4: methyl 2-(3-aminoprop-1-yn-1-yl)-4-(2,6-diazaspiro[3.3]heptane-2-carbonyl)benzoate

Example 5-41 was synthesized by sequentially applying the same manner as in step 3 of Preparation Example 5-116 and step 2 of Preparation Example 1-1 from Intermediate 5-41c.

¹H NMR (500 MHz, DMSO- *d*₆) δ 9.27 (s, 3H), 7.53 (s, 1H), 7.46 - 7.41 (m, 2H), 7.39 - 7.35 (m, 2H), 4.06 - 3.99 (m, 2H), 3.94 (s, 3H), 3.91 (s, 8H).

### [Preparation Example 5-86] methyl-2-(3-aminoprop-1-yn-1-yl)-4-(piperidin-4-ylamino)benzoate dihydrochloride

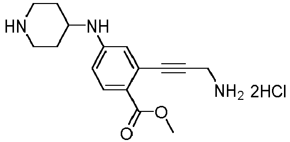

### Step 1: methyl 4-amino-2-(3-(1,3-dioxoisoindolin-2-yl)prop-1-yn-1-yl)benzoate (Intermediate 5-86a)

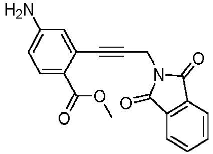

Intermediate 5-86a was synthesized in the same manner as in step 2 of Preparation Example 5-116' from methyl 4-amino-2-iodobenzoate.

### Step 2: tert-butyl 4-((3-(3-(1,3-dioxoisoindolin-2-yl)prop-1-yn-1-yl)-4-(methoxycarbonyl)phenyl)amino)piperidine-1-carboxylate (Intermediate 5-86b)

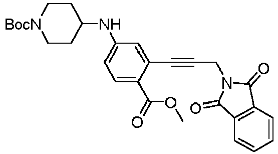

Intermediate 5-86b was synthesized in the same manner as in step 1 of Preparation Example 5-85c using *tert*-butyl 4-oxopiperidine-1-carboxylate from Intermediate 5-86a.

### Step 3: methyl-2-(3-(1,3-dioxoisoindolin-2-yl)prop-1-yn-1-yl)-4-(piperidin-4-ylamino)benzoate trifluoroacetic acid (Intermediate 5-86c)

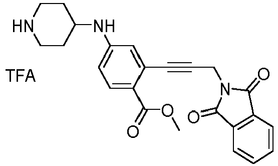

Intermediate 5-86c was synthesized in the same manner as in step 4 of Preparation Example 4-1' from Intermediate 5-86b.

### Step 4: methyl-2-(3-aminoprop-1-yn-1-yl)-4-(piperidin-4-ylamino)benzoate hydrochloride

Example 5-86 was synthesized by sequentially applying the same manner as in step 3 of Preparation Example 5-116 and step 2 of Preparation Example 1-1 from Intermediate 5-86c.

¹H NMR (500 MHz, DMSO- d₆) δ 8.60 (s, 3H), 8.53 (s, 2H), 7.86 (d, *J* = 13.7 Hz, 1H), 6.79 (d, *J* = 50.9 Hz, 1H), 6.67 (dd, *J* = 24.5, 2.4 Hz, 1H), 4.02 (d, *J* = 7.1 Hz, 2H), 3.75 (s, 3H), 3.62 (dt, *J* = 10.9, 4.7 Hz, 2H), 3.31 - 3.26 (m, 2H), 3.21 (dt, *J* = 10.4, 6.5 Hz, 2H), 3.05 - 2.93 (m, 2H), 2.91 - 2.84 (m, 1H).

### [Preparation Example 5-168] methyl-2-(3-aminoprop-1-yn-1-yl)-4-(2,7-diazaspiro[3.5]nonan-2-yl)benzoate dihydrochloride

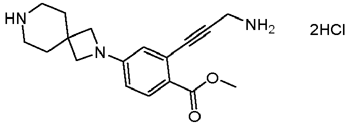

### Step 1: tert-butyl 2-(3-(3-(1,3-dioxoisoindolin-2-yl)prop-1-yn-1-yl)-4-(methoxycarbonyl)phenyl)-2,7-diazaspiro[3.5]nonane-7-carboxylate (Intermediate 5-168a)

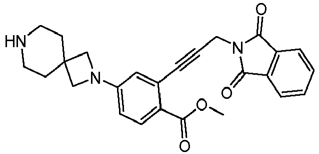

Intermediate 5-35a (0.5 mmol, 1.0 eq), *tert*-butyl 2,7-diazaspiro[3.5]nonane-7-carboxylate (1.0 eq), RuPhos Pd G3 (0.1 eq), CS₂CO₃ (3.0 eq), and toluene (1.5 mL) were mixed and then the resulting mixture was stirred at 80 °C overnight. The reactant was extracted with EtOAc and H₂O to obtain an organic layer. The obtained organic layer was dried over Na₂SO₄. The mixture was filtered under reduced pressure, distilled, and then purified through flash column to synthesize Intermediate 5-168a.

### Step 2: methyl-2-(3-aminoprop-1-yn-1-yl)-4-(2,7-diazaspiro[3.5]nonan-2-yl)benzoate dihydrochloride

Example 5-168 was synthesized by sequentially applying the same manner as in step 2 of Preparation Example 1-1 and step 3 of Preparation Example 5-116 from Intermediate 5-168a via Intermediate 5-168b (methyl-2-(3-(1,3-dioxoisoindolin-2-yl)prop-1-yn-1-yl)-4-(2,7-diazaspiro[3.5]nonan-2-yl)benzoate).

¹H NMR (500 MHz, DMSO-d₆) δ 9.03 (d, *J* = 61.5 Hz, 3H), 8.54 (s, 3H), 7.67 (d, *J* = 8.9 Hz, 1H), 6.88 (d, *J* = 2.5 Hz, 1H), 6.76 (dd, *J* = 8.9, 2.5 Hz, 1H), 3.97 - 3.94 (m, 2H), 3.81 (s, 2H), 3.73 (s, 1H), 3.72 (s, 3H), 3.69 (s, 1H), 3.07 - 2.99 (m, 4H), 1.81 - 1.67 (m, 4H).

### [Preparation Example IV]

The compounds corresponding to Formula IV of the present invention were synthesized as follows:

### [Preparation Example 5-32] methyl 2-(3-aminoprop-1-yn-1-yl)-4-(4-(3-aminopropanoyl)piperazin-1-yl)benzoate hydrochloride

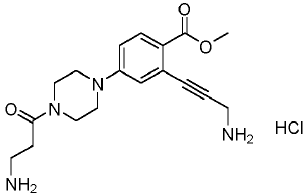

Example 5-32 was synthesized in the same manner as in Preparation Example 1-1' from β-alanine and Intermediate 5-7b.

¹H NMR (500 MHz, DMSO- *d*₆) δ 8.80 (s, 3H), 8.59 (s, 3H), 7.93 (d, *J* = 7.5 Hz, 1H), 7.12 - 7.02 (m, 2H), 3.99 (t, *J* = 5.7 Hz, 2H), 3.79 (s, 3H), 3.62 (t, *J* = 5.3 Hz, 6H), 3.43 - 3.35 (m, 4H), 3.00 (q, *J* = 6.0 Hz, 2H).

### [Preparation Example I']

A TBM-UBM compound in which a compound (JQ1) showing the activity of a bromodomain-containing protein 4 (BRD4) inhibitor is linked to compounds corresponding to Formula I of the present invention was synthesized as follows.

### [Preparation Example 1-1'] (S)-N-(4-(3-aminoprop-1-yn-1-yl)phenyl)-2-(4-(4-chlorophenyl)-2,3,9-trimethyl-6H-thieno[3,2-f][1,2,4]triazolo[4,3-a][1,4]diazepin-6-yl)acetamide hydrochloride

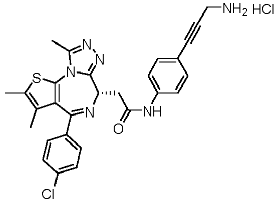

### Step 1: tert-butyl (S)-(3-(4-(2-(4-(4-chlorophenyl)-2,3,9-trimethyl-6H-thieno[3,2-f][1,2,4]triazolo[4,3-a][1,4]diazepin-6-yl)acetamido)phenyl)prop-2-yn-1-yl)carbamate (Intermediate 1-1'a)

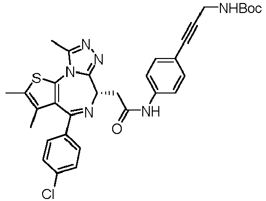

Commercially available JQ1 carboxylic acid ((S)-2-(4-(4-chlorophenyl)-2,3,9-trimethyl-6H-thieno[3,2-f][1,2,4]triazolo[4,3-a][1,4]diazepin-6-yl)acetic acid) (1.0 mmol, 1.2 eq), Intermediate 1-1a (1.0 eq), HOBt (1.5 eq), DIPEA (2.0 eq), EDC (1.5 eq), and dry DCM (3 mL) were mixed, and then the resulting mixture was stirred at room temperature for 16 hours. The reactant was extracted with EtOAc and H₂O to obtain an organic layer. The obtained organic layer was dried over Na₂SO₄. The mixture was filtered under reduced pressure, distilled, and then purified through flash column to synthesize Intermediate 1-1'a.

### Step 2: (S)-N-(4-(3-aminoprop-1-yn-1-yl)phenyl)-2-(4-(4-chlorophenyl)-2,3,9-trimethyl-6H-thieno[3,2-f][1,2,4]triazolo[4,3-a][1,4]diazepin-6-yl)acetamide hydrochloride

Example 1-1' was synthesized in the same manner as in step 2 of Preparation Example 1-1 from Intermediate 1-1'a.

¹H NMR (500 MHz, Methanol-*d*₄) δ 8.41 - 8.34 (m, 2H), 8.24 - 8.12 (m, 6H), 5.51 - 5.48 (m, 1H), 4.45 - 4.35 (m, 4H), 3.49 (s, 3H), 3.23 (d, *J* = 0.9 Hz, 3H), 2.48 (d, *J* = 0.8 Hz, 3H); MS m/z [M+H]⁺ 531.35.

### [Preparation Example 1-3'] (S)-N-(3-(3-aminoprop-1-yn-1-yl)phenyl)-2-(4-(4-chlorophenyl)-2,3,9-trimethyl-6H-thieno[3,2-f][1,2,4]triazolo[4,3-a][1,4]diazepin-6-yl)acetamide hydrochloride

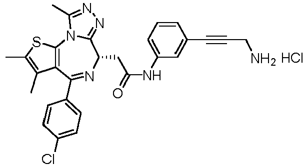

Example 1-3' was synthesized in the same manner as in Preparation Example 1-1' from Intermediate 1-3a via Intermediate 1-3'a (tert-butyl (S)-(3-(3-(2-(4-(4-chlorophenyl)-2,3,9-trimethyl-6H-thieno[3,2-f][1,2,4]triazolo[4,3-a][1,4]diazepin-6-yl)acetamido)phenyl)prop-2-yn-1-yl)carbamate).

¹H NMR (500 MHz, DMSO-d₆) δ 10.53 (s, 1H), 8.39 (s, 3H), 7.89 (t, *J* = 1.9 Hz, 1H), 7.55 (ddd, *J* = 8.3, 2.2, 1.1 Hz, 1H), 7.47 - 7.44 (m, 2H), 7.41 - 7.36 (m, 2H), 7.34 (t, *J* = 7.9 Hz, 1H), 7.11 (dt, *J* = 7.9, 1.2 Hz, 1H), 4.57 (dd, *J* = 7.6, 6.7 Hz, 1H), 3.95 (td, *J* = 6.2, 5.7, 3.6 Hz, 2H), 3.49 (dd, *J* = 7.3, 3.1 Hz, 2H), 2.58 (s, 3H), 2.39 (d, *J* = 0.9 Hz, 3H), 1.59 (d, *J* = 0.8 Hz, 3H); MS m/z [M+H]⁺ 531.35.

### [Preparation Example 3-1'] methyl (S)-2-(3-aminoprop-1-yn-1-yl)-5-(2-(4-(4-chlorophenyl)-2,3,9-trimethyl-6H-thieno[3,2-f][1,2,4]triazolo[4,3-a][1,4]diazepin-6-yl)acetamido)benzoate hydrochloride

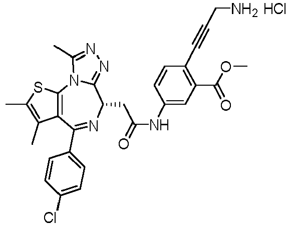

Example 3-1' was synthesized in the same manner as in Preparation Example 1-1' from Intermediate 3-1a via Intermediate 3-1'a (methyl (S)-2-(3-((*tert-*butoxycarbonyl)amino)prop-1-yn-1-yl)-5-(2-(4-(4-chlorophenyl)-2,3,9-trimethyl-6H-thieno[3,2-f][1,2,4]triazolo[4,3-a][1,4]diazepin-6-yl)acetamido)benzoate).

¹H NMR (500 MHz, DMSO-*d*₆) δ 10.79 (s, 1H), 8.44 (s, 3H), 8.26 (d, *J* = 2.2 Hz, 1H), 7.86 (dd, *J* = 8.6, 2.3 Hz, 1H), 7.55 (d, *J* = 8.4 Hz, 1H), 7.50 - 7.47(m, 2H), 7.45 - 7.41 (m, 2H), 4.64 (d, *J* = 7.8 Hz, 1H), 3.99 (d, *J* = 5.8 Hz, 2H), 3.56 (s, 3H), 2.63 (s, 3H), 2.43 (s Hz, 3H), 1.63 (s, 3H); MS m/z [M+H]⁺ 587.35.

### [Preparation Example 3-2'] (S)-2-(3-aminoprop-1-yn-1-yl)-5-(2-(4-(4-chlorophenyl)-2,3,9-trimethyl-6H-thieno[3,2-f][1,2,4]triazolo[4,3-a][1,4]diazepin-6-yl)acetamido)benzoic acid hydrochloride

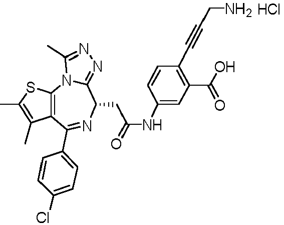

### Step 1: (S)-2-(3-((tert-butoxycarbonyl)amino)prop-1-yn-1-yl)-5-(2-(4-(4-chlorophenyl)-2,3,9-trimethyl-6H-thieno[3,2-f][1,2,4]triazolo[4,3-a][1,4]diazepin-6-yl)acetamido)benzoic acid (Intermediate 3-2'a)

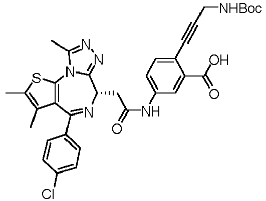

Intermediate 3-1'a (0.1 mmol, 1.0 eq), LiOH·H₂O (3.0 eq), THF (0.1 mL), H₂O (0.1 mL), and MeOH (0.1 mL) were mixed and then the resulting mixture was stirred at room temperature for 5 hours. After the reactant was extracted with H₂O, and then the pH of a water layer was adjusted to 5 or less by adding 2N HCl aqueous solution dropwise, the resulting mixture was extracted with a mixed solvent of chloroform/IPA to obtain an organic layer. The obtained organic layer was dried over Na2SO4, and the mixture was filtered under reduced pressure, and then distilled to synthesize Intermediate 3-2'a without a separate purification process.

### Step 2: (S)-2-(3-aminoprop-1-yn-1-yl)-5-(2-(4-(4-chlorophenyl)-2,3,9-trimethyl-6H-thieno[3,2-f][1,2,4]triazolo[4,3-a][1,4]diazepin-6-yl)acetamido)benzoic acid hydrochloride

Example 3-2' was synthesized in the same manner as in step 2 of Preparation Example 1-1 from Intermediate 3-2'a.

¹H NMR (500 MHz, DMSO-*d*₆) δ 10.77 (s, 1H), 8.35 (s, 3H), 8.28 (d, *J* = 2.1 Hz, 1H), 7.87 (dd, *J* = 8.6, 2.3 Hz, 1H), 7.57 (d, *J* = 8.5 Hz, 1H), 7.51 - 7.46 (m, 2H), 7.45 - 7.41 (m, 2H), 4.62 (d, *J* = 7.6 Hz, 1H), 4.03 (d, *J* = 5.3 Hz, 2H), 2.63 (s, 3H), 2.43 (s, 3H), 1.64 (s, 3H); MS m/z [M+H]⁺ 573.35.

### [Preparation Example 3-3'] (S)-N-(4-(3-aminoprop-1-yn-1-yl)-3-(hydroxymethyl)phenyl)-2-(4-(4-chlorophenyl)-2,3,9-trimethyl-6H-thieno[3,2-f][1,2,4]triazolo[4,3-a][1,4]diazepin-6-yl)acetamide hydrochloride

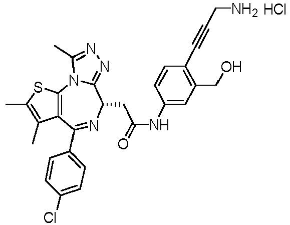

### Step 1: (5-amino-2-iodophenyl)methanol (Intermediate 3-3a)

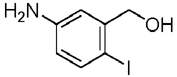

After methyl 5-amino-2-iodobenzoate (0.4 mmol, 1.0 eq) was dissolved in dry THF, lithium aluminium hydride solution (2.0 M in THF, 2.0 eq) was slowly added dropwise thereto, and then the resulting mixture was heated at 50 °C for 2 hours. A 2 N HCl aqueous solution was added to the reactant to adjust the pH thereof to 6-7, and then a compound was extracted with EtOAc to obtain an organic layer. The obtained organic layer was dried over Na₂SO₄, and then filtered under reduced pressure. The mixture was distilled under reduced pressure, and then purified through flash column to synthesize Intermediate 3-3a.

### Step 2: 3-(((tert-butyldimethylsilyl)oxy)methyl)-4-iodoaniline (Intermediate 3-3b)

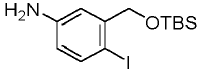

After Intermediate 3-3a (1.1 mmol, 1.0 eq) synthesized in step 1 and imidazole (2.2 eq) were dissolved in dry DCM, TBSCI (1.1 eq) was added, and then a gas in a reactor was replaced with Ar, and then the resulting mixture was stirred at room temperature for 30 minutes. The mixture was distilled under reduced pressure, and then purified through flash column to synthesize Intermediate 3-3b.

### Step 3: tert-butyl (3-(4-amino-2-(((tert-butyldimethylsilyl)oxy)methyl)phenyl)prop-2-yn-1-yl)carbamate (Intermediate 3-3c)

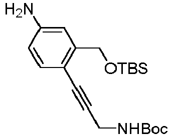

Intermediate 3-3c was synthesized in the same manner as in step 1 of Preparation Example 1-1 from Intermediate 3-3b synthesized in step 2.

### Step 4: tert-butyl (S)-(3-(2-(((tert-butyldimethylsilyl)oxy)methyl)-4-(2-(4-(4-chlorophenyl)-2,3,9-trimethyl-6H-thieno[3,2-f][1,2,4]triazolo[4,3-a][1,4]diazepin-6-yl)acetamido)phenyl)prop-2-yn-1-yl)carbamate (Intermediate 3-3'a)

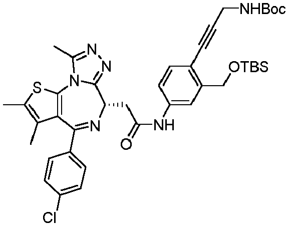

Intermediate 3-3'a was synthesized in the same manner as in step 1 of Preparation Example 1-1' from Intermediate 3-3c.

### Step 5: tert-butyl (S)-(3-(4-(2-(4-(4-chlorophenyl)-2,3,9-trimethyl-6H-thieno[3,2-f][1,2,4]triazolo[4,3-a][1,4]diazepin-6-yl)acetamido)-2-(hydroxymethyl)phenyl)prop-2-yn-1-yl)carbamate (Intermediate 3-3'b)

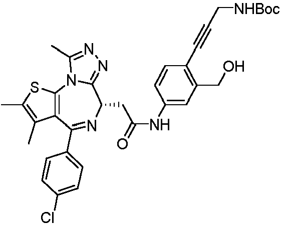

Intermediate 3-3'a (0.2 mmol, 1.0 eq) was dissolved in 1.5 mL of THF, and then TBAF (1.0 M in THF, 1.5 eq) was added dropwise thereto. After 3 hours, the solvent was distilled under reduced pressure, and then purified through flash column to synthesize Intermediate 3-3'b.

### Step 6: (S)-N-(4-(3-aminoprop-1-yn-1-yl)-3-(hydroxymethyl)phenyl)-2-(4-(4-chlorophenyl)-2,3,9-trimethyl-6H-thieno[3,2-f][1,2,4]triazolo[4,3-a][1,4]diazepin-6-yl)acetamide hydrochloride

Example 3-3' was synthesized in the same manner as in step 2 of Preparation Example 1-1 by using Intermediate 3-3'b.

¹H NMR (400 MHz, DMSO-*d*₆) δ 10.54 (s, 1H), 8.37 (s, 3H), 7.83 (s, 1H), 7.63 (d, *J* = 7.8 Hz, 1H), 7.48 (s, 2H), 7.44 (s, 2H), 7.36 (s, 1H), 4.64 (s, 3H), 4.00 (s, 2H), 3.54 (s, 2H), 2.61 (s, 3H), 2.43 (s, 3H), 1.64 (s, 3H); MS m/z [M+H]⁺ 559.35.

### [Preparation Example 3-8'] (S)-2-(3-aminoprop-1-yn-1-yl)-5-(2-(4-(4-chlorophenyl)-2,3,9-trimethyl-6H-thieno[3,2-f][1,2,4]triazolo[4,3-a][1,4]diazepin-6-yl)acetamido)benzyl dihydrogen phosphate trifluoroacetic acid

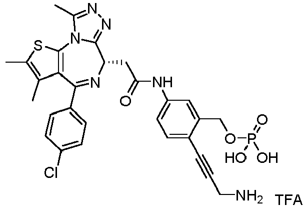

### Step 1: tert-butyl (S)-(3-(4-(2-(4-(4-chlorophenyl)-2,3,9-trimethyl-6H-thieno[3,2-f][1,2,4]triazolo[4,3-a][1,4]diazepin-6-yl)acetamido)-2-(((di-tert-butoxyphosphoryl)oxy)methyl)phenyl)prop-2-yn-1-yl)carbamate (Intermediate 3-8'a)

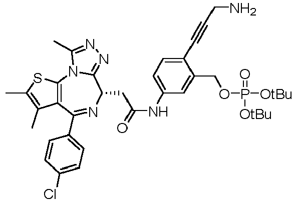

Intermediate 3-3'b (0.1 mmol, 1.0 eq) and tetrazole (10.0 eq) were dissolved in 1.7 mL of DMF, and then a gas in a reactor was replaced with an Ar gas. Di-*tert*-butyl N,N-diisopropylphosphoramidite (5.0 eq) was added thereto, and then the resulting mixture was stirred at room temperature for 3 hours. H₂O₂ (excess) was added dropwise thereto at - 20 °C, and then after 10 minutes, the reaction was terminated with NaHSO₃. The reactant was extracted with EtOAc and H₂O to obtain an organic layer. The obtained organic layer was dried over Na₂SO₄. The mixture was filtered under reduced pressure, distilled, and then purified through flash column to synthesize Intermediate 3-8'a.

### Step 2: (S)-2-(3-aminoprop-1-yn-1-yl)-5-(2-(4-(4-chlorophenyl)-2,3,9-trimethyl-6H-thieno[3,2-f][1,2,4]triazolo[4,3-a][1,4]diazepin-6-yl)acetamido)benzyl dihydrogen phosphate trifluoroacetic acid

Example 3-8' was synthesized in the same manner as in step 4 of Preparation Example 4-1' from Intermediate 3-8'a.

¹H NMR (500 MHz, DMSO-*d*₆) δ 10.56 (s, 1H), 8.56 (s, 3H), 7.72 (d, *J* = 2.2 Hz, 1H), 7.65 (dd, *J* = 8.4, 2.2 Hz, 1H), 7.47 - 7.42 (m, 2H), 7.40 - 7.34 (m, 3H), 4.90 (d, *J* = 5.5 Hz, 2H), 4.56 (t, *J* = 7.2 Hz, 1H), 3.93 (s, 2H), 3.53 - 3.45 (m, 2H), 2.56 (s, 3H), 2.38 (d, *J* = 0.9 Hz, 3H), 1.59 (d, *J* = 0.9 Hz, 3H). ³¹P NMR (202 MHz, DMSO-d₆) δ -0.47; MS m/z [M+H]⁺ 639.26.

### [Preparation Example 3-25'] methyl (S)-2-(3-aminoprop-1-yn-1-yl)-4-(2-(4-(4-chlorophenyl)-2,3,9-trimethyl-6H-thieno[3,2-f][1,2,4]triazolo[4,3-a][1,4]diazepin-6-yl)acetamido)benzoate hydrochloride

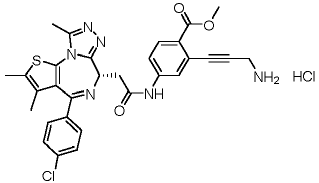

Example 3-25' was synthesized in the same manner as in Preparation Example 1-1' from Intermediate 3-25a via Intermediate 3-25'a (methyl (S)-2-(3-((*tert-*butoxycarbonyl)amino)prop-1-yn-1-yl)-4-(2-(4-(4-chlorophenyl)-2,3,9-trimethyl-6H-thieno[3,2-f][1,2,4]triazolo[4,3-a][1,4]diazepin-6-yl)acetamido)benzoate).

¹H NMR (400 MHz, DMSO-*d*₆) δ 10.89 (s, 1H), 8.46 (s, 3H), 8.10 (d, *J* = 2.2 Hz, 1H), 7.93 (d, *J* = 8.7 Hz, 1H), 7.67 (dd, *J* = 8.7, 2.2 Hz, 1H), 7.49 (d, *J* = 8.4 Hz, 2H), 7.42 (d, *J* = 8.5 Hz, 2H), 4.62 (t, *J* = 7.2 Hz, 1H), 4.03 (t, *J* = 5.6 Hz, 2H), 3.85 (s, 3H), 3.58 (d, *J* = 7.2 Hz, 2H), 2.63 (s, 3H), 2.43 (s, 3H), 1.64 (s, 3H); MS m/z [M+H]⁺ 587.25.

### [Preparation Example 3-26'] (S)-2-(3-aminoprop-1-yn-1-yl)-4-(2-(4-(4-chlorophenyl)-2,3,9-trimethyl-6H-thieno[3,2-f][1,2,4]triazolo[4,3-a][1,4]diazepin-6-yl)acetamido)benzoic acid hydrochloride

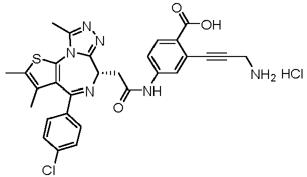

Example 3-26' was synthesized in the same manner as in Preparation Example 3-2' from Intermediate 3-25'a.

¹H NMR (500 MHz, DMSO-*d*₆) δ 10.80 (s, 1H), 8.38 (s, 2H), 8.06 (d, *J* = 2.2 Hz, 1H), 7.88 (d, *J* = 8.7 Hz, 1H), 7.59 (dd, *J* = 8.7, 2.2 Hz, 1H), 7.46 (d, *J* = 8.8 Hz, 2H), 7.39 (d, *J* = 8.5 Hz, 2H), 4.66 - 4.47 (m, 1H), 4.05 - 3.89 (m, 2H), 3.62 - 3.45 (m, 2H), 2.59 (d, *J* = 1.1 Hz, 3H), 2.39 (d, *J* = 1.0 Hz, 3H), 1.65 - 1.52 (m, 3H); MS m/z [M+H]⁺ 573.35.

### [Preparation Example 3-27'] (S)-N-(3-(3-aminoprop-1-yn-1-yl)-4-(hydroxymethyl)phenyl)-2-(4-(4-chlorophenyl)-2,3,9-trimethyl-6H-thieno[3,2-f][1,2,4]triazolo[4,3-a][1,4]diazepin-6-yl)acetamide hydrochloride

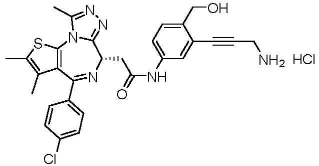

### Step 1: tert-butyl (3-(5-amino-2-(((tert-butyldimethylsilyl)oxy)methyl)phenyl)prop-2-yn-1-yl)carbamate (Intermediate 3-27c)

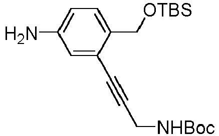

Intermediate 3-27c was synthesized in the same manner as in step 1 to 3 of Preparation Example 3-3' from methyl 4-amino-2-iodobenzoate.

### Step 2: (S)-N-(3-(3-aminoprop-1-yn-1-yl)-4-(hydroxymethyl)phenyl)-2-(4-(4-chlorophenyl)-2,3,9-trimethyl-6H-thieno[3,2-f][1,2,4]triazolo[4,3-a][1,4]diazepin-6-yl)acetamide hydrochloride

Example 3-27' was synthesized in the same manner as in Preparation Example 3-3' from Intermediate 3-27c via Intermediate 3-27'a (*tert*-butyl (S)-(3-(2-(((*tert-*butyldimethylsilyl)oxy)methyl)-5-(2-(4-(4-chlorophenyl)-2,3,9-trimethyl-6H-thieno[3,2-f][1,2,4]triazolo[4,3-a][1,4]diazepin-6-yl)acetamido)phenyl)prop-2-yn-1-yl)carbamate).

¹H NMR (400 MHz, DMSO-d₆) δ 10.54 (s, 1H), 8.53 (s, 3H), 7.87 (s, 1H), 7.69 - 7.55 (m, 1H), 7.53 - 7.47 (m, 3H), 7.43 (d, *J* = 8.3 Hz, 2H), 4.88 (s, 1H), 4.62 (d, *J* = 14.8 Hz, 2H), 4.09 - 3.96 (m, 2H), 3.54 (td, *J* = 7.3, 6.8, 3.5 Hz, 2H), 2.65 (s, 3H), 2.43 (s, 3H), 1.64 (s, 3H); MS m/z [M+H]⁺ 559.25.

### [Preparation Example 3-49'] N-(4-(3-amino-4-hydroxybut-1-yn-1-yl)phenyl)-2-((S)-4-(4-chlorophenyl)-2,3,9-trimethyl-6H-thieno[3,2-f][1,2,4]triazolo[4,3-a][1,4]diazepin-6-yl)acetamide hydrochloride

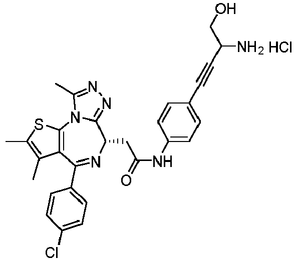

Example 3-49' was synthesized in the same manner as in Preparation Example 1-1' from Intermediate 3-49b.

MS m/z [M+H]⁺ 559.35.

### [Preparation Example 3-51'] N-(3-(3-amino-4-hydroxybut-1-yn-1-yl)phenyl)-2-((S)-4-(4-chlorophenyl)-2,3,9-trimethyl-6H-thieno[3,2-f][1,2,4]triazolo[4,3-a][1,4]diazepin-6-yl)acetamide hydrochloride

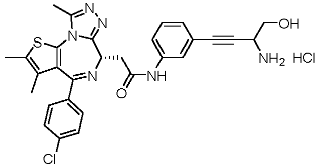

### Step 1: tert-butyl 4-((3-aminophenyl)ethynyl)-2,2-dimethyloxazolidine-3-carboxylate (Intermediate 3-51a)

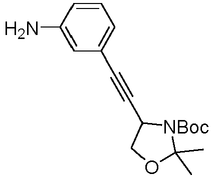

Intermediate 3-51a was synthesized in the same manner as in step 2 of Preparation Example 3-49 from 3-iodoaniline as a starting material, and Intermediate 3-49a.

### Step 2: N-(3-(3-amino-4-hydroxybut-1-yn-1-yl)phenyl)-2-((S)-4-(4-chlorophenyl)-2,3,9-trimethyl-6H-thieno[3,2-f][1,2,4]triazolo[4,3-a][1,4]diazepin-6-yl)acetamide hydrochloride

Example 3-51' was synthesized in the same manner as in Preparation Example 1-1' from Intermediate 3-51a.

¹H NMR (500 MHz, DMSO-*d*₆) δ 10.57 (s, 1H), 8.43 (bs, 3H), 7.93 (t, *J* = 1.8 Hz, 1H), 7.59 (ddd, *J* = 8.3, 2.2, 1.1 Hz, 1H), 7.53 - 7.46 (m, 2H), 7.45 - 7.34 (m, 3H), 7.15 (dt, *J* = 7.8, 1.3 Hz, 1H), 4.61 (dd, *J* = 7.6, 6.8 Hz, 1H), 4.03 - 3.96 (m, 2H), 3.55 - 3.50 (m, 2H), 2.62 (s, 3H), 2.43 (s, 3H), 1.63 (s, 3H); MS m/z [M+H]⁺ 559.35.

### [Preparation Example 3-57'] 2-amino-4-(4-(2-((S)-4-(4-chlorophenyl)-2,3,9-trimethyl-6H-thieno[3,2-f][1,2,4]triazolo[4,3-a][1,4]diazepin-6-yl)acetamido)phenyl)but-3-yn-1-yl dihydrogen phosphate trifluoroacetic acid

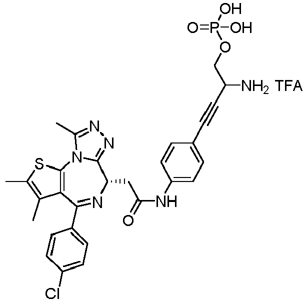

### Step 1: tert-butyl (4-(4-(2-((S)-4-(4-chlorophenyl)-2,3,9-trimethyl-6H-thieno[3,2-f][1,2,4]triazolo[4,3-a][1,4]diazepin-6-yl)acetamido)phenyl)-1-hydroxybut-3-yn-2-yl)carbamate (Intermediate 3-57'a)

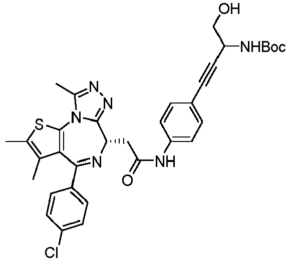

Example 3-49' (0.1 mmol, 1.0 eq) was dissolved in 3 mL of MeOH, and then TEA (excess) and Boc₂O (excess) were slowly added thereto at 0 °C, and the resulting mixture was stirred for 6 hours. The reactant was extracted with DCM and H₂O to obtain an organic layer. The obtained organic layer was dried over Na₂SO₄. The mixture was filtered under reduced pressure, distilled, and then purified through flash column to synthesize Intermediate 3-57'a.

### Step 2: 2-amino-4-(4-(2-((S)-4-(4-chlorophenyl)-2,3,9-trimethyl-6H-thieno[3,2-f][1,2,4]triazolo[4,3-a][1,4]diazepin-6-yl)acetamido)phenyl)but-3-yn-1-yl dihydrogen phosphate trifluoroacetic acid

Example 3-57' was synthesized in the same manner as in Preparation Example 3-8' from Intermediate 3-57'a.

¹H NMR (500 MHz, DMSO-*d*₆) δ 10.53 (s, 1H), 7.70-7.63 (m, 2H), 7.49-7.42 (m, 2H), 7.42 - 7.36 (m, 4H), 4.54 (dd, *J* = 4.1, 2.4 Hz, 1H), 4.11 - 3.97 (m, 2H), 3.49 (d, *J* = 7.2 Hz, 2H), 2.56 (s, 3H), 2.38 (d, *J* = 1.0 Hz, 3H), 1.59 (d, *J* = 1.0 Hz, 3H). ³¹P NMR (202 MHz, DMSO-*d*₆) δ -0.60; MS m/z [M+H]⁺ 639.36.

### [Preparation Example 4-1'] (S)-N-(3-(5-(3-aminoprop-1-yn-1-yl)furan-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide hydrochloride

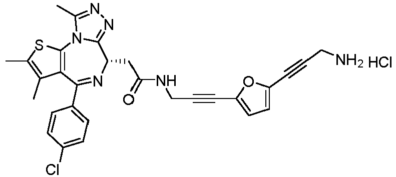

### Step 1: 2-bromo-5-iodofuran (Intermediate 4-1a)

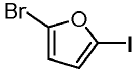

2-bromofuran (2.0 mmol, 1.0 eq), NIS (1.1 eq), and PTSA (0.1 eq) were dissolved in 4.0 mL of EtOH, and then the resulting mixture was stirred at 50 °C for 10 minutes. The reaction was terminated using Na₂S₂O₃. The reactant was extracted with EtOAc and H₂O to obtain an organic layer. The obtained organic layer was dried over Na₂SO₄, and then distilled under reduced pressure to synthesize a compound without a separate purification process.

### Step 2: tert-butyl (3-(5-bromofuran-2-yl)prop-2-yn-1-yl)carbamate (Intermediate 4-1b)

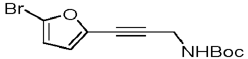

Intermediate 4-1b was synthesized in the same manner as in step 1 of Preparation Example 1-1 from Intermediate 4-1a.

### Step 3: tert-butyl (3-(5-(3-(1,3-dioxoisoindolin-2-yl)prop-1-yn-1-yl)furan-2-yl)prop-2-yn-1-yl)carbamate (Intermediate 4-1c)

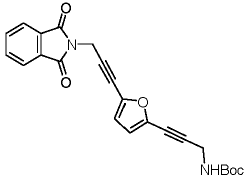

Intermediate 4-1c was synthesized in the same manner as in step 2 of Preparation Example 5-116 from Intermediate 4-1b.

### Step 4: 2-(3-(5-(3-aminoprop-1-yn-1-yl)furan-2-yl)prop-2-yn-1-yl)isoindoline-1,3-dione trifluoroacetic acid (Intermediate 4-1d)

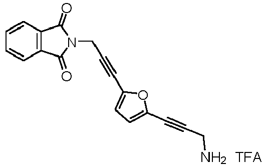

Intermediate 4-1c (0.18 mmol, 1.0 eq) was dissolved in 3.0 mL of DCM, and then TFA (excess) was added dropwise thereto, and the resulting mixture was stirred at room temperature for 1 hour. The reactant was distilled under reduced pressure to synthesize Intermediate 4-1d without a separate purification process.

### Step 5: (S)-2-(4-(4-chlorophenyl)-2,3,9-trimethyl-6H-thieno[3,2-f][1,2,4]triazolo[4,3-a][1,4]diazepin-6-yl)-N-(3-(5-(3-(1,3-dioxoisoindolin-2-yl)prop-1-yn-1-yl)furan-2-yl)prop-2-yn-1-yl)acetamide (Intermediate 4-1'a)

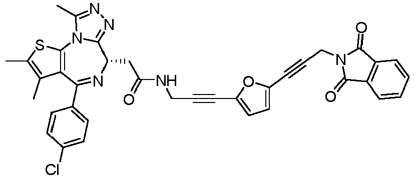

JQ1 carboxylic acid (0.2 mmol, 1.0 eq), Intermediate 4-1d (1.2 eq), and HATU (1.5 eq) were dissolved in dry DCM, and then a gas in a reactor was replaced with an Ar gas. DIPEA (3.0 eq) was added dropwise thereto, and then the resulting mixture was stirred at room temperature for 16 hours. The reactant was extracted with EtOAc and H₂O to obtain an organic layer. The obtained organic layer was dried over Na₂SO₄. The mixture was filtered under reduced pressure, distilled, and then purified through flash column to synthesize Intermediate 4-1'a.

### Step 6: (S)-N-(3-(5-(3-aminoprop-1-yn-1-yl)furan-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide hydrochloride

Example 4-1' was synthesized by sequentially applying the same manner as in step 3 of Preparation Example 5-116 and step 2 of Preparation Example 1-1 from Intermediate 4-1'a.

¹H NMR (500 MHz, DMSO-*d*₆) δ 8.83 (t, *J* = 5.6 Hz, 1H), 8.45 (s, 3H), 7.43-7.25 (m, 4H), 6.92 (d, *J* = 3.6 Hz, 1H), 6.86 (d, *J* = 3.6 Hz, 1H), 4.50 (dd, *J* = 8.5, 5.9 Hz, 1H), 4.27 - 4.13 (m, 2H), 4.03 (q, *J* = 5.6 Hz, 2H), 3.33 - 3.15 (m, 2H), 2.57 (s, 3H), 2.38 (d, *J* = 0.9Hz, 3H), 1.56 (d, *J* = 0.9 Hz, 3H); MS m/z [M+H]⁺ 557.28.

### [Preparation Example 4-10'] (S)-N-(3-(5-(3-aminoprop-1-yn-1-yl)thiophen-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide hydrochloride

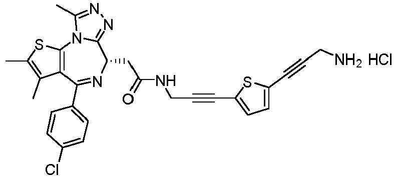

### Step 1: 2-(3-(5-(3-aminoprop-1-yn-1-yl)thiophen-2-yl)prop-2-yn-1-yl)isoindoline-1,3-dione trifluoroacetic acid (Intermediate 4-10d)

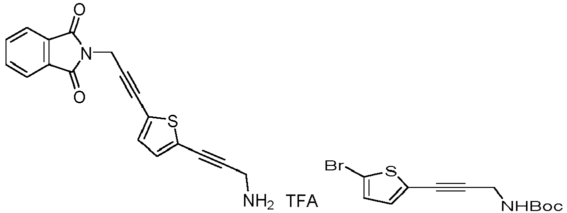

Intermediate 4-10d was synthesized in the same manner as in steps 1 to 4 of Preparation Example 4-1' from 2-bromothiophene via Intermediate 4-10b (tert-butyl (3-(5-bromothiophen-2-yl)prop-2-yn-1-yl)carbamate).

### Step 2: (S)-N-(3-(5-(3-aminoprop-1-yn-1-yl)thiophen-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide hydrochloride

Example 4-10' was synthesized in the same manner as in step 6 of Preparation Example 4-1' from Intermediate 4-10d.

¹H NMR (500 MHz, DMSO-d₆) δ 8.85 (s, 1H), 8.47 (s, 2H), 7.52 - 7.35 (m, 4H), 7.34 (d, *J =* 3.9 Hz, 1H), 7.31 (d, *J =* 3.8 Hz, 1H), 4.60 - 4.49 (m, 1H), 4.23 (qd, *J* = 18.0, 5.6 Hz, 2H), 4.10 - 4.02 (m, 2H), 3.41 - 3.28 (m, 1H), 3.27 - 3.17 (m, 1H), 2.62 (d, *J* = 2.9 Hz, 3H), 2.42 (d, *J* = 2.9 Hz, 3H), 1.61 (d, *J* = 5.5 Hz, 3H); MS m/z [M+H]⁺ 573.21.

### [Preparation Example 4-128'] ((S)-N-(3-(4-(3-aminoprop-1-yn-1-yl)phenyl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide hydrochloride

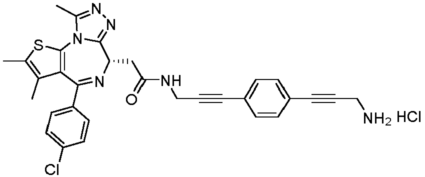

### Step 1: 3-(4-iodophenyl)prop-2-yn-1-amine hydrochloride (Intermediate 4-128a)

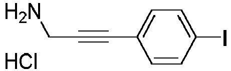

Intermediate 4-128a was synthesized in the same manner as in Preparation Example 1-1 from 1,4-diiodobenzene.

### Step 2: (S)-2-(4-(4-chlorophenyl)-2,3,9-trimethyl-6H-thieno[3,2-f][1,2,4]triazolo[4,3-a][1,4]diazepin-6-yl)-N-(3-(4-iodophenyl)prop-2-yn-1-yl)acetamide (Intermediate 4-128'a)

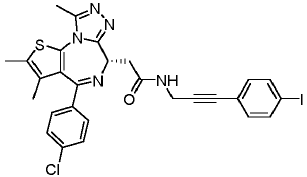

Intermediate 4-128'a was synthesized in the same manner as in step 5 of Preparation Example 4-1' from Intermediate 4-128a.

### Step 3: ((S)-N-(3-(4-(3-aminoprop-1-yn-1-yl)phenyl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide hydrochloride

Example 4-128' was synthesized in the same manner as in Preparation Example 1-1 from Intermediate 4 128'a.

¹H NMR (500 MHz, DMSO-*d*₆) δ 8.82 (t, *J* = 5.5 Hz, 1H), 8.35 (bs, 3H), 7.51 - 7.32 (m, 8H), 4.53 (dd, *J* = 8.4, 5.9 Hz, 1H), 4.21 (qd, *J* = 17.8, 5.5 Hz, 2H), 4.03 (q, *J* = 5.5 Hz, 2H), 3.35 - 3.20 (m, 2H), 2.60 (s, 3H), 2.41 (s, 3H), 1.60 (s, 3H); MS m/z [M+H]⁺ 567.25.

### [Preparation Example 4-129'] (S)-N-(3-(3-(3-aminoprop-1-yn-1-yl)phenyl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide hydrochloride

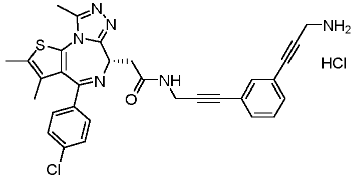

Example 4-129' was synthesized in the same manner as in Preparation Example 4-128' from 1,3-diiodobenze.

¹H NMR (500 MHz, DMSO-d₆)δ 8.82 (t, *J* = 5.5 Hz, 1H), 8.39 (bs, 3H), 7.54 - 7.32 (m, 8H), 4.53 (dd, *J* = 8.4, 5.9 Hz, 1H), 4.20 (qd, *J* = 17.7, 5.4 Hz, 2H), 4.01 (q, *J* = 5.6 Hz, 2H), 3.36 - 3.20 (m, 2H), 2.60 (s, 3H), 2.41 (s, 3H), 1.60 (s, 3H); MS m/z [M+H]⁺ 567.25.

### [Preparation Example 4-147'] (S)-N-(4-(5-(4-aminobut-1-yn-1-yl)furan-2-yl)but-3-yn-1-yl)-2-(4-(4-chlorophenyl)-2,3,9-trimethyl-6H-thieno[3,2-f][1,2,4]triazolo[4,3-a][1,4]diazepin-6-yl)acetamide hydrochloride

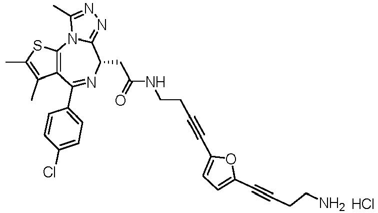

### Step 1: tert-butyl (4-(5-bromofuran-2-yl)but-3-yn-1-yl)carbamate (Intermediate 4-147a)

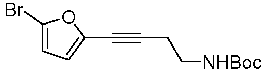

Intermediate 4-147a was synthesized in the same manner as in step 1 of Preparation Example 1-1 using Intermediate 4-1a and *tert*-butyl but-3-yn-1-ylcarbamate.

### Step 2: tert-butyl (4-(5-(4-(1,3-dioxoisoindolin-2-yl)but-1-yn-1-yl)furan-2-yl)but-3-yn-1-yl)carbamate (Intermediate 4-147b)

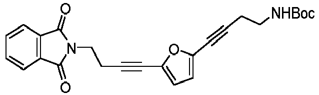

Intermediate 4-147b was synthesized in the same manner as in step 2 of Preparation Example 5-116 using Intermediate 4-147a and 2-(but-3-yn-1-yl)isoindoline-1,3-dione.

### Step 3: (S)-N-(4-(5-(4-aminobut-1-yn-1-yl)furan-2-yl)but-3-yn-1-yl)-2-(4-(4-chlorophenyl)-2,3,9-trimethyl-6H-thieno[3,2-f][1,2,4]triazolo[4,3-a][1,4]diazepin-6-yl)acetamide hydrochloride

Example 4-147' was synthesized in the same manner as in step 4 to 6 of Preparation Example 4-1' from Intermediate 4-147b.

¹H NMR (500 MHz, Methanol-*d*₄) δ 7.53 - 7.47 (m, 2H), 7.43 (dd, *J* = 8.7, 3.0 Hz, 2H), 6.56 (d, *J* = 3.5 Hz, 1H), 6.50 (d, *J* = 3.5 Hz, 1H), 4.98 (s, 1H), 3.51 - 3.44 (m, 2H), 3.44 - 3.40 (m, 2H), 3.17 - 3.12 (m, 2H), 2.86 - 2.83 (m, 5H), 2.70 (td, *J* = 6.6, 3.5 Hz, 2H), 2.50 - 2.44 (m, 2H), 1.73 - 1.66 (m, 2H); MS m/z [M+H]⁺ 585.21.

### [Preparation Example 4-177'] (S)-N-(3-(5-(3-aminoprop-1-yn-1-yl)pyridin-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide hydrochloride

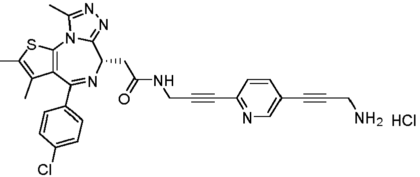

### Step 1: tert-butyl (3-(5-bromopyridin-2-yl)prop-2-yn-1-yl)carbamate (Intermediate 4-177a)

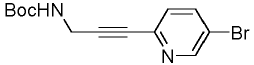

Intermediate 4-177a was synthesized in the same manner as in step 1 of Preparation Example 1-1 using 5-bromo-2-iodopyridine as a starting material.

### Step 2: tert-butyl (3-(5-(3-hydroxyprop-1-yn-1-yl)pyridin-2-yl)prop-2-yn-1-yl)carbamate (Intermediate 4-177b)

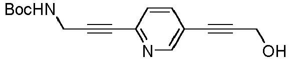

After Intermediate 4-177a (0.7 mmol, 1.0 eq), propargyl alcohol (2.0 eq), Pd(PPh₃)₄ (0.1 eq), Cul (0.3 eq), TBAI (3.0 eq), TEA (0.6 mL), and dry THF (2.4 mL) were mixed, a gas in a reactor was replaced with an Ar gas, and then the resulting mixture was reacted in M.W. at 100 °C for 50 minutes. The reactant was extracted with EtOAc and H₂O to obtain an organic layer. The obtained organic layer was dried over Na₂SO₄. The mixture was filtered under reduced pressure, distilled, and then purified through flash column to synthesize Intermediate 4-177b.

### Step 3: 3-(6-(3-aminoprop-1-yn-1-yl)pyridin-3-yl)prop-2-yn-1-ol hydrochloride (Intermediate 4-177c)

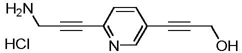

Intermediate 4-177c was synthesized in the same manner as in step 2 of Preparation Example 1-1 from Intermediate 4-177b.

### Step 4: 2,5-dioxopyrrolidin-1-yl (S)-2-(4-(4-chlorophenyl)-2,3,9-trimethyl-6H-thieno[3,2-f][1,2,4]triazolo[4,3-a][1,4]diazepin-6-yl)acetate (JQ1 NHS ester)

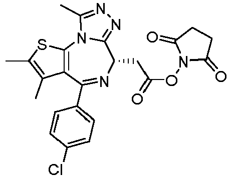

JQ1 carboxylic acid (1.0 mmol, 1.2 eq), *N*-hydroxysuccinimide (2.0 eq), EDC (2.0 eq), and DCM (15 mL) were mixed, a gas in a reactor was replaced with an Ar gas, and then the resulting mixture was stirred at room temperature overnight. The reactant was distilled under reduced pressure, and then purified through flash column to synthesize Intermediate JQ1 NHS ester.

### Step 5: (S)-2-(4-(4-chlorophenyl)-2,3,9-trimethyl-6H-thieno[3,2-f][1,2,4]triazolo[4,3-a][1,4]diazepin-6-yl)-N-(3-(5-(3-hydroxyprop-1-yn-1-yl)pyridin-2-yl)prop-2-yn-1-yl)acetamide (Intermediate 4-177'a)

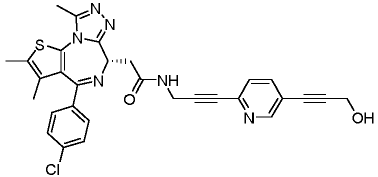

JQ1 NHS ester (0.3 mmol, 1.0 eq) and Intermediate 4-177c (1.0 eq) were dissolved in dry DCM, and then a gas in a reactor was replaced with an Ar gas. DIPEA (2.4 eq) was added dropwise thereto, and then the resulting mixture was stirred at room temperature overnight. The reactant was distilled under reduced pressure, and then purified through flash column to synthesize Intermediate 4-177'a.

### Step 6: (S)-N-(3-(5-(3-bromoprop-1-yn-1-yl)pyridin-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide (Intermediate 4-177'b)

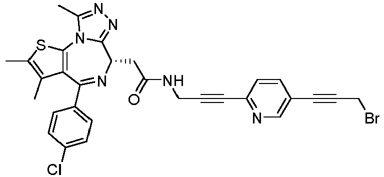

Intermediate 4-177'a (0.05 mmol, 1.0 eq) and CBr₄ (2.0 eq) were dissolved in dry DCM, and then a gas in a reactor was replaced with an Ar gas. PPh₃ (2.0 eq) was slowly added thereto, and then the resulting mixture was stirred at room temperature for 6 hours. The reactant was distilled under reduced pressure, and then purified through flash column to synthesize Intermediate 4-177'b.

### Step 7: (S)-2-(4-(4-chlorophenyl)-2,3,9-trimethyl-6H-thieno[3,2-f][1,2,4]triazolo[4,3-a][1,4]diazepin-6-yl)-N-(3-(5-(3-(1,3-dioxoisoindolin-2-yl)prop-1-yn-1-yl)pyridin-2-yl)prop-2-yn-1-yl)acetamide (Intermediate 4-177'c)

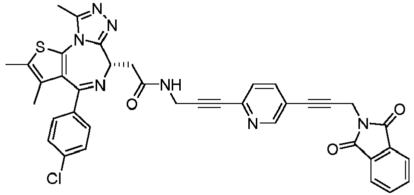

Intermediate 4-177'b (0.02 mmol, 1.0 eq), phthalimide (1.3 eq), and CS₂CO₃ (2.0 eq) were dissolved in ACN (0.6 mL) and THF (0.2 mL), and then the resulting mixture was stirred at room temperature overnight. The reactant was distilled under reduced pressure, and then purified through flash column to synthesize Intermediate 4-177c'.

### Step 8: (S)-N-(3-(5-(3-aminoprop-1-yn-1-yl)pyridin-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide hydrochloride

Example 4-177' was synthesized in the same manner as in step 6 of Preparation Example 4-1' from Intermediate 4-177'c.

¹H NMR (500 MHz, DMSO-*d*₆) δ 8.41 (s, 3H), 7.94 (s, 1H), 7.64 (s, 1H), 7.58 (d, *J* = 8.1 Hz, 1H), 7.46 (d, *J* = 8.3 Hz, 2H), 7.40 (d, *J* = 8.1 Hz, 2H), 4.56 (t, *J* = 6.7 Hz, 1H), 4.06 - 4.00 (m, 1H), 3.86 (s, 2H), 3.75 (s, 4H), 3.53 (s, 5H), 2.57 (s, 3H), 2.38 (s, 3H), 1.59 (s, 3H); MS m/z [M+H]⁺ 568.23.

### [Preparation Example 4-179'] (S)-N-(3-(6-(3-aminoprop-1-yn-1-yl)pyridin-3-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide hydrochloride

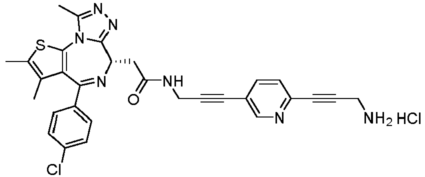

### Step 1: 3-(5-bromopyridin-2-yl)prop-2-yn-1-ol (Intermediate 4-179a)

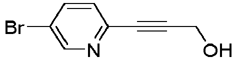

After 5-bromo-2-iodopyridine (1.0 mmol, 1.0 eq), propargylalcohol (1.1 eq), PdCl₂(PPh₃)₂ (0.05 eq), Cul (0.1 eq), TEA (1.0 mL), and dry THF (4.0 mL) were mixed, a gas in a reactor was replaced with an Ar gas, and then the resulting mixture was heated in M.W. at 100 °C for 30 minutes. The reactant was extracted with EtOAc and H₂O to obtain an organic layer. The obtained organic layer was dried over Na₂SO₄. The mixture was filtered under reduced pressure, distilled, and then purified through flash column to synthesize Intermediate 4-179a.

### Step 2: tert-butyl (3-(6-(3-hydroxyprop-1-yn-1-yl)pyridin-3-yl)prop-2-yn-1-yl)carbamate (Intermediate 4-179b)

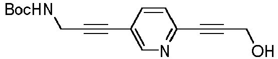

Intermediate 4-179b was synthesized in the same manner as in step 2 of Preparation Example 4-177' from Intermediate 4-179a and n-boc propargylamine.

### Step 3: 3-(5-(3-aminoprop-1-yn-1-yl)pyridin-2-yl)prop-2-yn-1-ol hydrochloride (Intermediate 4-179c)

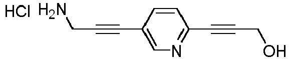

Intermediate 4-179c was synthesized in the same manner as in step 2 of Preparation Example 1-1 from Intermediate 4-179b.

### Step 4: (S)-N-(3-(6-(3-aminoprop-1-yn-1-yl)pyridin-3-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide hydrochloride

Example 4-179' was synthesized in the same manner as in steps 5 to 8 of Preparation Example 4-177' from Intermediate 4-179c.

¹H NMR (500 MHz, DMSO-*d*₆) δ 8.48 (t, *J* = 5.8 Hz, 1H), 8.14 (t, *J* = 5.7 Hz, 1H), 7.87 - 7.83 (m, 2H), 7.46 - 7.41 (m, 5H), 7.39 - 7.36 (m, 2H), 7.09 (q, *J* = 3.5 Hz, 2H), 4.46 (dd, *J* = 8.1, 6.1 Hz, 1H), 3.49 (s, 2H), 3.19 (d, *J* = 5.8 Hz, 3H), 3.14 - 3.09 (m, 2H), 3.05 (dq, *J* = 12.9, 6.2 Hz, 2H), 2.54 (d, *J* = 2.0 Hz, 3H), 2.35 (d, *J* = 3.1 Hz, 3H), 1.57 (s, 3H), 1.52 (d, *J* = 8.1 Hz, 2H), 1.48 (d, *J* = 7.6 Hz, 2H), 1.40 (s, 2H), 1.19 (s, 4H); MS m/z [M+H]⁺ 568.23.

### [Preparation Example 4-223'] (S)-N-(3-(5-(3-(aminooxy)prop-1-yn-1-yl)thiophen-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide hydrochloride

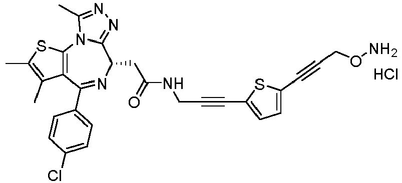

### Step 1: tert-butyl (3-(5-(3-hydroxyprop-1-yn-1-yl)thiophen-2-yl)prop-2-yn-1-yl)carbamate (Intermediate 4-223a)

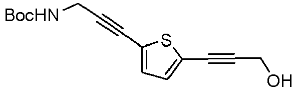

Intermediate 4-223a was synthesized in the same manner as in step 2 of Preparation Example 4-177' from Intermediate 4-10b.

### Step 2: tert-butyl (3-(5-(3-((1,3-dioxoisoindolin-2-yl)oxy)prop-1-yn-1-yl)thiophen-2-yl)prop-2-yn-1-yl)carbamate (Intermediate 4-223b)

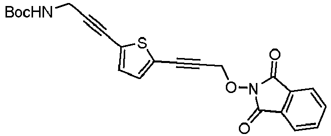

After PPh₃ (1.2 eq) was dissolved in dry THF, Ar replacement was performed, diisopropyl azodicarboxylate (1.0 eq) and Intermediate 4-223a (0.2 mmol, 1.0 eq) were added thereto at 0 °C, and then the resulting mixture was stirred for 5 minutes. N-hydroxyphthalimide (1.0 eq) was added thereto and then the mixture was stirred at room temperature for 5 hours. The reactant was distilled under reduced pressure, and then purified through flash column to synthesize Intermediate 4-223b.

### Step 3: 2-((3-(5-(3-aminoprop-1-yn-1-yl)thiophen-2-yl)prop-2-yn-1-yl)oxy)isoindoline-1,3-dione trifluoroacetic acid (Intermediate 4-223c)

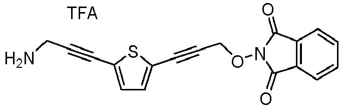

Intermediate 4-223c was synthesized in the same manner as in step 4 of Preparation Example 4-1' from Intermediate 4-223b.

### Step 4: (S)-2-(4-(4-chlorophenyl)-2,3,9-trimethyl-6H-thieno[3,2-f][1,2,4]triazolo[4,3-a][1,4]diazepin-6-yl)-N-(3-(5-(3-((1,3-dioxoisoindolin-2-yl)oxy)prop-1-yn-1-yl)thiophen-2-yl)prop-2-yn-1-yl)acetamide (Intermediate 4-223'a)

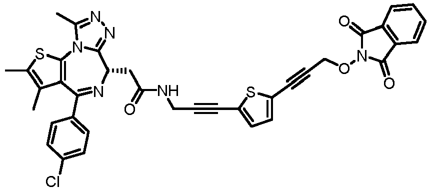

Intermediate 4-223'a was synthesized in the same manner as in step 5 of Preparation Example 4-1' from Intermediate 4-223c.

### Step 5: (S)-N-(3-(5-(3-(aminooxy)prop-1-yn-1-yl)thiophen-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide

Example 4-223' was synthesized by sequentially applying the same manner as in step 3 of Preparation Example 5-116 and step 2 of Preparation Example 1-1 from Intermediate 4-223'a.

¹H NMR (500 MHz, Methanol-*d*₄) δ 7.50 - 7.43 (m, 2H), 7.33 - 7.26 (m, 2H), 7.22 (d, *J* = 3.8 Hz, 1H), 7.19 (d, *J* = 3.9 Hz, 1H), 4.80 - 4.74 (m, 1H), 4.49 - 4.37 (m, 2H), 4.22 (d, *J* = 17.8 Hz, 1H), 3.51 (dd, *J* = 15.1, 9.6 Hz, 1H), 3.43 - 3.32 (m, 2H), 2.79 (s, 3H), 2.47 (s, 3H), 1.70 (s, 3H); MS m/z [M+H]⁺ 589.21.

### [Preparation Example 5-116'] methyl (S)-2-(3-aminoprop-1-yn-1-yl)-4-(3-(2-(4-(4-chlorophenyl)-2,3,9-trimethyl-6H-thieno[3,2-f][1,2,4]triazolo[4,3-a][1,4]diazepin-6-yl)acetamido)prop-1-yn-1-yl)benzoate hydrochloride

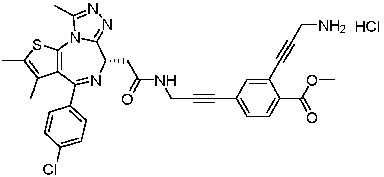

Example 5-116' was synthesized in the same manner as in Preparation Example 1-1' from Intermediate 5-116c.

¹H NMR (500 MHz, DMSO-*d*₆) δ 8.81 (t, *J* = 5.4 Hz, 1H), 8.38 (s, 3H), 7.88 (d, *J* = 8.2 Hz, 1H), 7.59 (d, *J* = 1.7 Hz, 1H), 7.55 (dd, *J* = 8.2, 1.7 Hz, 1H), 7.40 - 7.36 (m, 2H), 7.32 (d, *J* = 8.9 Hz, 2H), 4.50 (dd, *J* = 8.3, 6.2 Hz, 1H), 4.19 (t, *J* = 5.5 Hz, 2H), 4.00 (q, *J* = 5.7 Hz, 2H), 3.84 (s, 3H), 3.37 - 3.17 (m, 2H), 2.56 (s, 3H), 2.37 (d, *J* = 0.9 Hz, 3H), 1.56 (d, *J* = 0.9 Hz, 3H); MS m/z [M+H]⁺625.37.

### [Preparation Example II']

A TBM-UBM compound in which a compound (JQ1) showing the activity of a bromodomain-containing protein 4 (BRD4) inhibitor is linked to compounds corresponding to Formula II of the present invention was synthesized as follows.

### [Preparation Example 4-124'] (S)-N-(4-(3-aminoprop-1-yn-1-yl)phenyl)-3-(2-(4-(4-chlorophenyl)-2,3,9-trimethyl-6H-thieno[3,2-f][1,2,4]triazolo[4,3-a][1,4]diazepin-6-yl)acetamido)propanamide hydrochloride

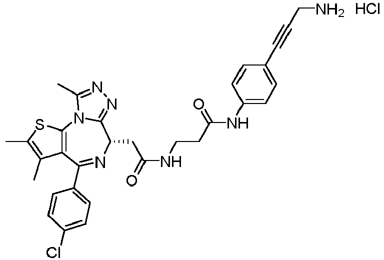

### Step 1: (S)-3-(2-(4-(4-chlorophenyl)-2,3,9-trimethyl-6H-thieno[3,2-f][1,2,4]triazolo[4,3-a][1,4]diazepin-6-yl)acetamido)propanoic acid (Intermediate 4-124'a)

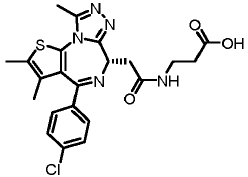

Intermediate 4-124'a was synthesized in the same manner as in step 5 of Preparation Example 4-177' from *β*-alanine.

### Step 2: (S)-N-(4-(3-aminoprop-1-yn-1-yl)phenyl)-3-(2-(4-(4-chlorophenyl)-2,3,9-trimethyl-6H-thieno[3,2-f][1,2,4]triazolo[4,3-a][1,4]diazepin-6-yl)acetamido)propanamide hydrochloride

Example 4-124' was synthesized in the same manner as in Preparation Example 1-1' from Intermediate 4-124'a and Intermediate 1-1a.

¹H NMR (500 MHz, DMSO-*d*₆) δ 10.29 (s, 1H), 8.38 (s, 3H), 7.73-7.60 (m, 2H), 7.52 (d, *J* = 8.6 Hz, 1H), 7.38 (tt, *J* = 8.7, 4.2 Hz, 5H), 4.51 (d, *J* = 6.9 Hz, 1H), 3.92 (d, *J =* 5.8 Hz, 2H), 3.40 - 3.22 (m, 4H), 3.30 - 3.00 (m, 2H), 2.63 - 2.57 (m, 3H), 2.56 - 2.50 (m, 2H), 2.38 (s, 3H), 1.60 - 1.54 (m, 3H); MS m/z [M+H]⁺ 600.32.

### [Preparation Example 4-125'] (S)-N-(4-(3-aminoprop-1-yn-1-yl)-3-(hydroxymethyl)phenyl)-3-(2-(4-(4-chlorophenyl)-2,3,9-trimethyl-6H-thieno[3,2-f][1,2,4]triazolo[4,3-a][1,4]diazepin-6-yl)acetamido)propanamide hydrochloride

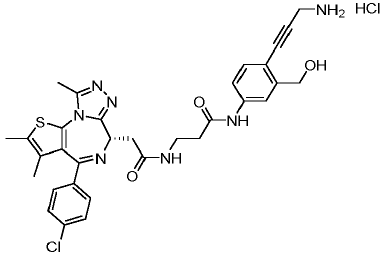

### Step 1: tert-butyl (S)-(3-(2-(((tert-butyldimethylsilyl)oxy)methyl)-4-(3-(2-(4-(4-chlorophenyl)-2,3,9-trimethyl-6H-thieno[3,2-f][1,2,4]triazolo[4,3-a][1,4]diazepin-6-yl)acetamido)propanamido)phenyl)prop-2-yn-1-yl)carbamate (Intermediate 4-125'a)

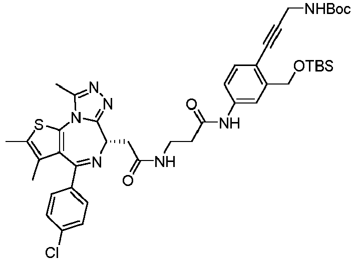

Intermediate 4-125'a was synthesized in the same manner as in step 1 of Preparation Intermediate 1-1' from Intermediate 4-124'a and Intermediate 3-3c.

### Step 2: (S)-N-(4-(3-aminoprop-1-yn-1-yl)-3-(hydroxymethyl)phenyl)-3-(2-(4-(4-chlorophenyl)-2,3,9-trimethyl-6H-thieno[3,2-f][1,2,4]triazolo[4,3-a][1,4]diazepin-6-yl)acetamido)propanamide hydrochloride

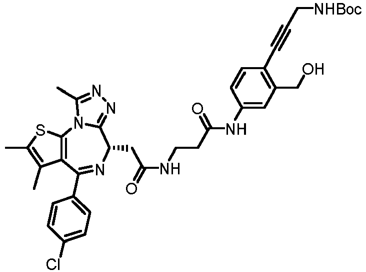

Example 4-125' was synthesized in the same manner as in steps 5 to 6 of Preparation Example 3-3' from Intermediate 4-125'a.

¹H NMR (500 MHz, DMSO-*d*₆) δ 10.24 (d, *J* = 11.4 Hz, 1H), 8.41 (d, *J* = 35.7 Hz, 3H), 7.76 (s, 1H), 7.63 - 7.57 (m, 1H), 7.42 - 7.35 (m, 4H), 7.29 (d, *J* = 8.3 Hz, 1H), 4.58 (s, 1H), 4.56 - 4.42 (m, 2H), 3.94 (d, *J* = 6.3 Hz, 2H), 3.42 - 3.30 (m, 2H), 3.29 - 3.10 (m, 2H), 2.59 (q, *J* = 4.9, 4.2 Hz, 3H), 2.53 (t, *J* = 7.0 Hz, 2H), 2.38 (s, 3H), 1.61 - 1.55 (m, 3H); MS m/z [M+H]⁺ 630.46.

### [Preparation Example 4-126'] methyl (S)-2-(3-aminoprop-1-yn-1-yl)-5-(3-(2-(4-(4-chlorophenyl)-2,3,9-trimethyl-6H-thieno[3,2-f][1,2,4]triazolo[4,3-a][1,4]diazepin-6-yl)acetamido)propanamido)benzoate hydrochloride

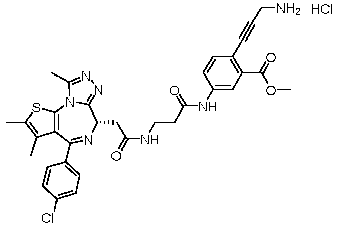

Example 4-126' was synthesized in the same manner as in Preparation Example 1-1' from Intermediate 4-124'a and Intermediate 3-1a.

¹H NMR (500 MHz, DMSO-*d*₆) δ 10.49 (d, *J* = 13.2 Hz, 1H), 8.39 (d, *J* = 25.3 Hz, 3H), 8.24 (d, *J* = 2.1 Hz, 1H), 7.83 (dd, *J* = 8.5, 2.3 Hz, 1H), 7.50 (d, *J* = 8.5 Hz, 1H), 7.43 - 7.33 (m, 4H), 4.50 (d, *J* = 5.6 Hz, 1H), 4.01 - 3.91 (m, 2H), 3.83 (s, 3H), 3.43 - 3.30 (m, 2H), 3.27 - 3.10 (m, 2H), 2.58 (dd, *J* = 3.9, 1.6 Hz, 3H), 2.54 (d, *J* = 6.8 Hz, 2H), 2.41 - 2.34 (m, 3H), 1.58 - 1.53 (m, 3H); MS m/z [M+H]⁺ 658.47.

### [Preparation Example 4-148'] (S)-5-(3-aminoprop-1-yn-1-yl)-N-(4-(2-(4-(4-chlorophenyl)-2,3,9-trimethyl-6H-thieno[3,2-f][1,2,4]triazolo[4,3-a][1,4]diazepin-6-yl)acetamido)but-2-yn-1-yl)furan-2-carboxamide hydrochloride

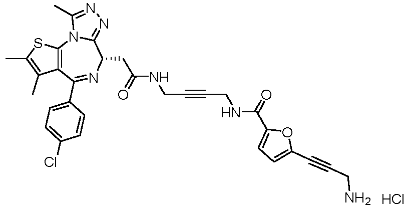

### Step 1: tert-butyl (4-aminobut-2-yn-1-yl)carbamate (Intermediate 4-148a)

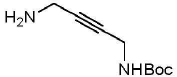

Intermediate 4-148a was synthesized in the same manner as in step 1 of Preparation Example 3-57' from but-2-yne-1,4-diamine.

### Step 2: (S)-N-(4-aminobut-2-yn-1-yl)-2-(4-(4-chlorophenyl)-2,3,9-trimethyl-6H-thieno[3,2-f][1,2,4]triazolo[4,3-a][1,4]diazepin-6-yl)acetamide hydrochloride (Intermediate 4-148'a)

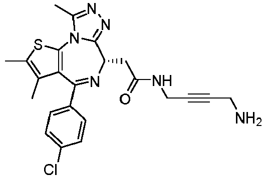

Intermediate 4-148'a was synthesized in the same manner as in Preparation Example 1-1' from JQ1 carboxylic acid and Intermediate 4-148a.

### Step 3: (S)-5-(3-aminoprop-1-yn-1-yl)-N-(4-(2-(4-(4-chlorophenyl)-2,3,9-trimethyl-6H-thieno[3,2-f][1,2,4]triazolo[4,3-a][1,4]diazepin-6-yl)acetamido)but-2-yn-1-yl)furan-2-carboxamide hydrochloride

Example 4-148' was synthesized in the same manner as in Preparation Example 1-1' from Intermediate 4-148'a and Intermediate 4-227b.

¹H NMR (500 MHz, DMSO-*d*₆) δ 8.96 (t, *J* = 5.7 Hz, 1H), 8.61 (t, *J* = 5.5 Hz, 1H), 8.41 (s, 3H), 7.45 (d, *J* = 8.7 Hz, 2H), 7.41 - 7.35 (m, 2H), 7.21 (d, *J* = 3.6 Hz, 1H), 6.97 (d, *J* = 3.6 Hz, 1H), 4.47 (dd, *J* = 8.2, 6.0 Hz, 1H), 4.08 - 3.97 (m, 3H), 3.28 - 3.12 (m, 2H), 2.56 (s, 3H), 2.37 (s, 3H), 1.58 (s, 3H). MS m/z [M+H]⁺ 614.25.

### [Preparation Example 4-149'] ((S)-5-(3-aminoprop-1-yn-1-yl)-N-(4-(2-(4-(4-chlorophenyl)-2,3,9-trimethyl-6H-thieno[3,2-f][1,2,4]triazolo[4,3-a][1,4]diazepin-6-yl)acetamido)butyl)furan-2-carboxamide hydrochloride

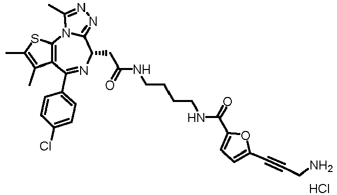

Example 4-149' was synthesized in the same manner as in steps 2 to 3 of Preparation Example 4-148' from *tert*-butyl(4-aminobutyl)carbamate.

¹H NMR (500 MHz, DMSO-*d*₆) δ 8.57 (t, *J* = 5.8 Hz, 1H), 8.50 (s, 3H), 8.24 (t, *J* = 5.6 Hz, 1H), 7.49 (d, *J* = 8.8 Hz, 2H), 7.41 (d, *J* = 8.7 Hz, 2H), 7.18 (d, *J* = 3.6 Hz, 1H), 6.99 (d, *J* = 3.6 Hz, 1H), 4.52 (dd, *J* = 8.3, 6.0 Hz, 1H), 4.08 (s, 2H), 3.26 - 3.18 (m, 4H), 3.17 - 3.08 (m, 2H), 2.61 (s, 3H), 2.41 (d, *J* = 0.9 Hz, 3H), 1.61 (d, *J* = 0.9 Hz, 3H), 1.52 (d, *J* = 7.0 Hz, 2H), 1.47 (d, *J* = 7.1 Hz, 2H); MS m/z [M+H]⁺ 618.16.

### [Preparation Example 4-161'] (S)-N-(3-(3-aminoprop-1-yn-1-yl)phenyl)-3-(2-(4-(4-chlorophenyl)-2,3,9-trimethyl-6H-thieno[3,2-f][1,2,4]triazolo[4,3-a][1,4]diazepin-6-yl)acetamido)propanamide hydrochloride

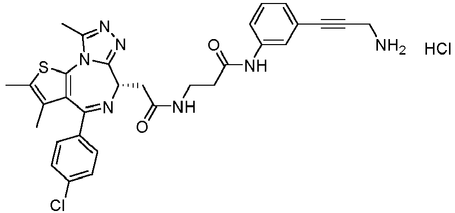

Example 4-161' was synthesized in the same manner as in Preparation Example 1-1' from Intermediate 4-124'a and Intermediate 1-3a.

MS m/z [M+H]⁺ 600.36.

### [Preparation Example 4-162'] (S)-N-(4-(3-aminoprop-1-yn-1-yl)phenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide hydrochloride

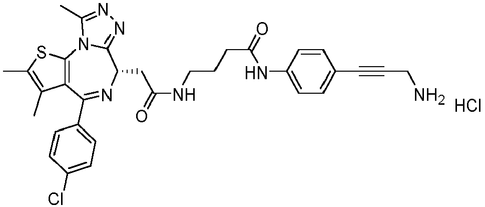

### Step 1: (S)-4-(2-(4-(4-chlorophenyl)-2,3,9-trimethyl-6H-thieno[3,2-f][1,2,4]triazolo[4,3-a][1,4]diazepin-6-yl)acetamido)butanoic acid (Intermediate 4-162'a)

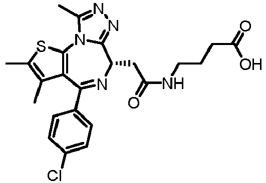

Intermediate 4-162'a was synthesized in the same manner as in step 5 of Preparation Example 4-177' from 4-aminobutanoic acid.

### Step 2: (S)-N-(4-(3-aminoprop-1-yn-1-yl)phenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide hydrochloride

Example 4-162' was synthesized in the same manner as in Preparation Example 1-1' from Intermediate 4-162'a and Intermediate 1-1a.

¹H NMR (500 MHz, DMSO-*d*₆) δ 10.19 (s, 1H), 8.33 (s, 3H), 8.25 (d, *J* = 5.7 Hz, 1H), 7.66 - 7.60 (m, 2H), 7.42 (d, *J* = 8.9 Hz, 2H), 7.39 - 7.32 (m, 4H), 4.48 (dd, *J* = 8.2, 6.1 Hz, 1H), 3.93 (q, *J* = 5.6 Hz, 2H), 3.27 - 3.05 (m, 4H), 2.57 (s, 3H), 2.39 - 2.36 (m, 3H), 2.36 - 2.30 (m, 2H), 1.74 (q, *J* = 7.3 Hz, 2H), 1.59 - 1.54 (m, 3H); MS m/z [M+H]⁺ 614.36.

### [Preparation Example 4-163'] (S)-N-(4-(3-aminoprop-1-yn-1-yl)phenyl)-5-(2-(4-(4-chlorophenyl)-2,3,9-trimethyl-6H-thieno[3,2-f][1,2,4]triazolo[4,3-a][1,4]diazepin-6-yl)acetamido)pentanamide hydrochloride

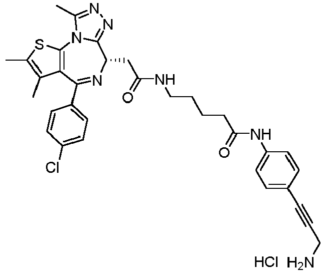

### Step 1: (S)-5-(2-(4-(4-chlorophenyl)-2,3,9-trimethyl-6H-thieno[3,2-f][1,2,4]triazolo[4,3-a][1,4]diazepin-6-yl)acetamido)pentanoic acid (Intermediate 4-163'a)

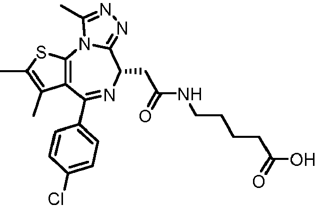

Intermediate 4-163'a was synthesized in the same manner as in step 5 of Preparation Example 4-177' from 5-aminopentanoic acid.

### Step 2: (S)-N-(4-(3-aminoprop-1-yn-1-yl)phenyl)-5-(2-(4-(4-chlorophenyl)-2,3,9-trimethyl-6H-thieno[3,2-f][1,2,4]triazolo[4,3-a][1,4]diazepin-6-yl)acetamido)pentanamide hydrochloride

Example 4-163' was synthesized in the same manner as in Preparation Example 1-1' from Intermediate 4-163'a and Intermediate 1-1a.

¹H NMR (500 MHz, DMSO-*d*₆) δ 10.24 (d, *J* = 2.3 Hz, 1H), 8.42 (s, 3H), 8.24 (t, *J* = 5.8 Hz, 1H), 7.64 (d, *J* = 8.3 Hz, 2H), 7.45 (d, *J* = 8.0 Hz, 2H), 7.39 - 7.29 (m, 4H), 4.51 (d, *J* = 6.7 Hz, 1H), 3.95 - 3.86 (m, 2H), 3.27 - 3.01 (m, 4H), 2.63 - 2.54 (m, 3H), 2.41 - 2.36 (m, 3H), 2.33 (t, *J* = 7.4 Hz, 2H), 1.60 (dd, *J* = 7.3, 3.1 Hz, 2H), 1.57 - 1.52 (m, 3H), 1.44 (p, *J* = 7.1 Hz, 2H); MS m/z [M+H]⁺ 628.46.

### [Preparation Example 4-164'] (S)-N-(3-(3-aminoprop-1-yn-1-yl)phenyl)-5-(2-(4-(4-chlorophenyl)-2,3,9-trimethyl-6H-thieno[3,2-f][1,2,4]triazolo[4,3-a][1,4]diazepin-6-yl)acetamido)pentanamide hydrochloride

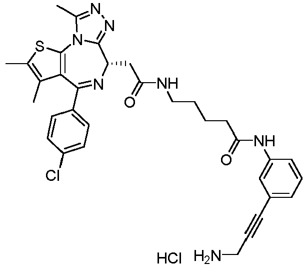

Example 4-164' was synthesized in the same manner as in Preparation Example 1-1' from Intermediate 4-163'a and Intermediate 1-3a.

¹H NMR (500 MHz, DMSO-*d*₆) δ 10.16 (d, *J* = 3.5 Hz, 1H), 8.40 (s, 3H), 8.24 (t, *J* = 5.8 Hz, 1H), 7.90 (d, *J* = 1.9 Hz, 1H), 7.49 (dd, *J* = 8.3, 1.9 Hz, 1H), 7.46 - 7.41 (m, 2H), 7.40 - 7.34 (m, 2H), 7.28 (t, *J* = 7.9 Hz, 1H), 7.07 (dt, *J* = 7.7, 1.3 Hz, 1H), 4.49 (dd, *J* = 8.3, 5.7 Hz, 1H), 3.97 - 3.85 (m, 2H), 3.26 - 2.98 (m, 4H), 2.58 (d, *J* = 1.1 Hz, 3H), 2.37 (d, *J* = 0.9 Hz, 3H), 2.31 (q, *J* = 8.5, 7.9 Hz, 2H), 1.58 (s, 2H), 1.56 (d, *J* = 0.9 Hz, 3H), 1.44 (p, *J* = 7.1 Hz, 2H); MS m/z [M+H]⁺ 628.46.

### [Preparation Example 4-165'] (S)-N-(4-(3-aminoprop-1-yn-1-yl)phenyl)-6-(2-(4-(4-chlorophenyl)-2,3,9-trimethyl-6H-thieno[3,2-f][1,2,4]triazolo[4,3-a][1,4]diazepin-6-yl)acetamido)hexanamide hydrochloride

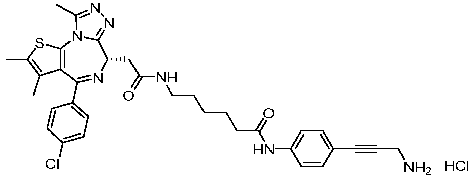

### Step 1: (S)-6-(2-(4-(4-chlorophenyl)-2,3,9-trimethyl-6H-thieno[3,2-f][1,2,4]triazolo[4,3-a][1,4]diazepin-6-yl)acetamido)hexanoic acid (Intermediate 4-165'a)

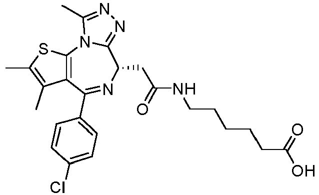

Intermediate 4-165'a was synthesized in the same manner as in step 5 of Preparation Example 4-177' from 6-aminohexanoic acid.

### Step 2: (S)-N-(4-(3-aminoprop-1-yn-1-yl)phenyl)-6-(2-(4-(4-chlorophenyl)-2,3,9-trimethyl-6H-thieno[3,2-f][1,2,4]triazolo[4,3-a][1,4]diazepin-6-yl)acetamido)hexanamide hydrochloride

Example 4-165' was synthesized in the same manner as in Preparation Example 1-1' from Intermediate 4-165'a and Intermediate 1-1a.

¹H NMR (500 MHz, DMSO-*d*₆) δ 10.10 (s, 1H), 8.25 (s, 3H), 8.17 (s, 1H), 7.66 - 7.58 (m, 2H), 7.50 - 7.42 (m, 2H), 7.40 - 7.29 (m, 4H), 4.49 - 4.42 (m, 1H), 3.94 (d, *J* = 5.7 Hz, 2H), 3.24 - 3.02 (m, 4H), 2.55 (d, *J* = 3.2 Hz, 3H), 2.37 (s, 3H), 2.34 - 2.25 (m, 2H), 1.57 (s, 3H), 1.56 (d, *J* = 10.1 Hz, 2H), 1.48 - 1.37 (m, 2H), 1.35 - 1.26 (m, 2H); MS m/z [M+H]⁺ 642.46.

### [Preparation Example 4-166'] ((S)-5-(3-aminoprop-1-yn-1-yl)-N-(3-(2-(4-(4-chlorophenyl)-2,3,9-trimethyl-6H-thieno[3,2-f][1,2,4]triazolo[4,3-a][1,4]diazepin-6-yl)acetamido)propyl)furan-2-carboxamide hydrochloride

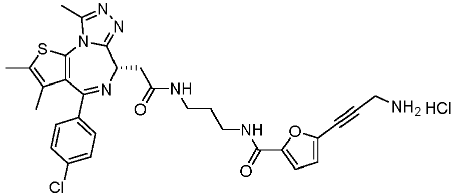

Intermediate 4-166'a ((S)-N-(3-aminopropyl)-2-(4-(4-chlorophenyl)-2,3,9-trimethyl-6H-thieno[3,2-f][1,2,4]triazolo[4,3-a][1,4]diazepin-6-yl)acetamide hydrochloride) was synthesized by sequentially applying step 2 of Preparation Example 5-77' and step 2 of Preparation Example 1-1 from *tert*-butyl (3-aminopropyl)carbamate, and then Example 4-166' was synthesized in the same manner as in Preparation Example 1-1' from Intermediate 4-166'a and 4-227b.

¹H NMR (500 MHz, DMSO-*d*₆) δ 8.57 (t, *J* = 5.8 Hz, 1H), 8.52 (s, 3H), 8.31 (t, *J* = 5.7 Hz, 1H), 7.48 (d, *J* = 8.8 Hz, 2H), 7.42 (d, *J* = 8.5 Hz, 2H), 7.19 (d, *J* = 3.6 Hz, 1H), 7.00 (d, *J* = 3.6 Hz, 1H), 4.53 (dd, *J* = 8.2, 6.1 Hz, 1H), 4.08 (t, *J* = 5.7 Hz, 2H), 3.29 - 3.20 (m, 4H), 3.19 - 3.11 (m, 2H), 2.61 (s, 3H), 2.41 (d, *J* = 0.9 Hz, 3H), 1.68 (t, *J* = 7.0 Hz, 2H), 1.63 - 1.60 (m, 3H); MS m/z [M+H]⁺ 604.16.

### [Preparation Example 4-167'] (S)-5-(3-aminoprop-1-yn-1-yl)-N-(5-(2-(4-(4-chlorophenyl)-2,3,9-trimethyl-6H-thieno[3,2-f][1,2,4]triazolo[4,3-a][1,4]diazepin-6-yl)acetamido)pentyl)furan-2-carboxamide hydrochloride

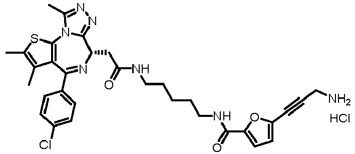

Example 4-167' was synthesized in the same manner as in Preparation Example 4-166' from tert-butyl (5-aminopentyl)carbamate.

¹H NMR (500 MHz, DMSO-*d*₆) δ 8.53 (dd, *J* = 10.3, 4.4 Hz, 4H), 8.24 (t, *J* = 5.5 Hz, 1H), 7.50 (d, *J* = 8.8 Hz, 2H), 7.42 (d, *J* = 8.5 Hz, 2H), 7.18 (d, *J* = 3.6 Hz, 1H), 6.98 (d, *J* = 3.6 Hz, 1H), 4.54 (dd, *J* = 8.2, 6.0 Hz, 2H), 4.07 (d, *J* = 5.6 Hz, 2H), 3.25 - 3.17 (m, 4H), 3.11 (dt, *J* = 30.0, 6.9 Hz, 2H), 2.61 (s, 3H), 2.41 (d, *J* = 1.0 Hz, 3H), 1.63 - 1.60 (m, 4H), 1.52 (q, *J* = 7.3 Hz, 2H), 1.46 (q, *J* = 7.4 Hz, 2H), 1.32 (q, *J* = 8.6, 8.1 Hz, 2H); MS m/z [M+H]⁺ 632.27.

### [Preparation Example 4-225'] (S)-5-(3-aminoprop-1-yn-1-yl)-N-(9-(2-(4-(4-chlorophenyl)-2,3,9-trimethyl-6H-thieno[3,2-f][1,2,4]triazolo[4,3-a][1,4]diazepin-6-yl)acetamido)nonyl)furan-2-carboxamide hydrochloride

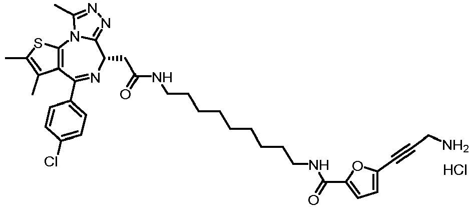

Example 4-225' was synthesized in the same manner as Preparation Example 4-166' from tert-butyl (9-aminononyl)carbamate.

¹H NMR (500 MHz, DMSO-*d*₆) δ 8.53 - 8.44 (m, 3H), 7.45 (d, *J* = 8.7 Hz, 2H), 7.40 - 7.37 (m, 2H), 7.36 (d, *J* = 8.7 Hz, 1H), 7.13 (d, *J* = 3.6 Hz, 1H), 7.10 (d, *J* = 3.1 Hz, 1H), 6.94 (d, *J* = 3.6 Hz, 1H), 4.49 (d, *J* = 2.4 Hz, 1H), 4.04 (p, *J* = 5.9 Hz, 2H), 3.34 - 3.30 (m, 2H), 3.17 (t, *J* = 9.3 Hz, 6=4H), 2.57 (s, 3H), 2.37 (d, *J* = 0.9 Hz, 3H), 1.58 (d, *J* = 1.0 Hz, 3H), 1.40 (dt, *J* = 15.1, 6.9 Hz, 4H), 1.20 (d, *J* = 9.9 Hz, 10H); MS m/z [M+H]⁺ 688.42.

### [Preparation Example 4-228'] (methyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzoate hydrochloride

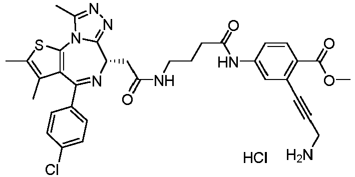

Example 4-228' was synthesized in the same manner as in step 2 of Preparation Example 5-77' from Intermediate 3-25a.

¹H NMR (500 MHz, DMSO-*d*₆) δ 10.50 (s, 1H), 8.40 (s, 3H), 8.29 (t, *J* = 5.6 Hz, 1H), 8.09 (d, *J* = 2.1 Hz, 1H), 7.85 (d, *J* = 8.7 Hz, 1H), 7.55 (dd, *J* = 8.7, 2.2 Hz, 1H), 7.43 - 7.36 (m, 4H), 4.49 (dd, *J* = 7.9, 6.4 Hz, 1H), 4.01 (q, *J* = 5.6 Hz, 2H), 3.79 (s, 3H), 3.53 (d, *J* = 4.6 Hz, 1H), 3.34 (q, *J* = 7.0 Hz, 1H), 3.27 - 3.06 (m, 5H), 2.57 (s, 3H), 2.37 (s, 3H), 1.73 (p, *J* = 7.1 Hz, 2H), 1.57 (s, 3H); MS m/z [M+H]⁺ 672.34.

### [Preparation Example 4-235'] ((S)-5-(3-aminoprop-1-yn-1-yl)-N-(8-(2-(4-(4-chlorophenyl)-2,3,9-trimethyl-6H-thieno[3,2-f][1,2,4]triazolo[4,3-a][1,4]diazepin-6-yl)acetamido)octyl)furan-2-carboxamide hydrochloride

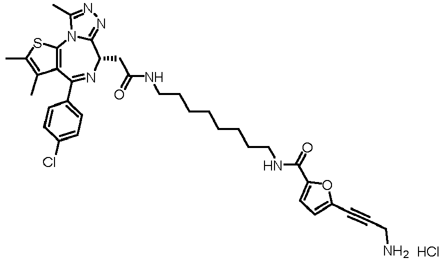

Example 4-235' was synthesized in the same manner as in Preparation Example 4-166' from Example tert-butyl (8-aminooctyl)carbamate.

¹H NMR (500 MHz, DMSO-*d*₆) δ 8.53 - 8.44 (m, 4H), 8.16 (t, *J* = 5.7 Hz, 1H), 7.47 - 7.43 (m, 2H), 7.41 - 7.36 (m, 2H), 7.13 (d, *J* = 3.6 Hz, 1H), 6.94 (d, *J* = 3.6 Hz, 1H), 4.48 (dd, *J* = 8.2, 6.0 Hz, 1H), 4.04 (q, *J* = 5.7 Hz, 2H), 3.37 - 3.17 (m, 2H), 3.17 - 2.99 (m, 4H), 2.57 (d, *J* = 1.1 Hz, 3H), 2.40 - 2.36 (m, 3H), 1.59 - 1.56 (m, 3H), 1.40 (dd, *J* = 15.3, 8.4 Hz, 4H), 1.28 - 1.16 (m, 8H); MS m/z [M+H]⁺ 674.42.

### [Preparation Example 5-1'] ethyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzoate hydrochloride

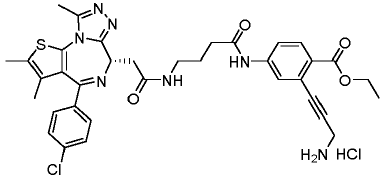

### Step 1: ethyl (S)-2-(3-((tert-butoxycarbonyl)amino)prop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzoate (Intermediate 5-1'a)

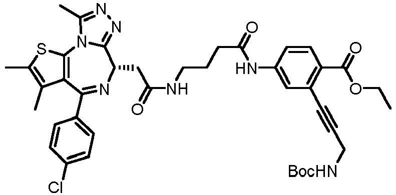

Intermediate 5-53'a (0.02 mmol, 1.0 eq) and K₂CO₃ (3.0 eq) were dissolved in 0.5 mL of dry DMF, and then a gas in a reactor was replaced with an Ar gas. After the reactant was stirred at room temperature for 30 minutes, iodoethane (3.0 eq) was added dropwise thereto, and then the resulting mixture was stirred at room temperature overnight. The reactant was purified through flash column to synthesize Intermediate 5-1'a.

### Step 2: ethyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzoate hydrochloride

Example 5-1' was synthesized in the same manner as in step 2 of Preparation Example 1-1 from Intermediate 5-1'a.

¹H NMR (500 MHz, DMSO-*d*₆) δ 10.44 (s, 1H), 8.37 (s, 3H), 8.30 (t, *J* = 5.7 Hz, 1H), 8.17 (d, *J* = 2.1 Hz, 1H), 7.89 (d, *J* = 8.7 Hz, 1H), 7.53 (dd, *J* = 8.7, 2.3 Hz, 1H), 7.45 (d, *J* = 8.9 Hz, 2H), 7.40 (d, *J* = 8.7 Hz, 2H), 4.50 (dd, *J* = 7.9, 6.3 Hz, 1H), 4.29 (q, *J* = 7.1 Hz, 2H), 4.04 (q, *J* = 5.6 Hz, 2H), 3.23 - 3.13 (m, 4H), 2.59 (s, 3H), 2.43 (dd, *J* = 7.7, 2.3 Hz, 2H), 2.41 (s, 3H), 1.78 (q, *J* = 7.3 Hz, 2H), 1.61 (s, 3H), 1.32 (d, *J* = 7.2 Hz, 3H); MS m/z [M+H]⁺ 686.48.

### [Preparation Example 5-2'] isopropyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzoate hydrochloride

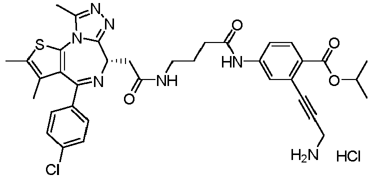

Example 5-2' was synthesized in the same manner as in Example 5-1' from Intermediate 5-53'a and 2-iodopropane.

¹H NMR (500 MHz, DMSO-*d*₆) δ 10.44 (d, *J* = 2.3 Hz, 1H), 8.37 (s, 3H), 8.30 (s, 1H), 8.17 (t, *J* = 2.3 Hz, 1H), 7.86 (dd, *J* = 8.7, 2.3 Hz, 1H), 7.52 (d, *J* = 8.6 Hz, 1H), 7.44 (dd, *J* = 8.8, 2.3 Hz, 2H), 7.40 (dd, *J* = 8.6, 2.3 Hz, 2H), 5.09 (ddd, *J* = 12.6, 6.3, 2.4 Hz, 1H), 4.54 - 4.43 (m, 1H), 4.06 - 4.01 (m, 2H), 3.24 - 3.15 (m, 4H), 2.59 (d, *J* = 2.3 Hz, 3H), 2.42 (s, 2H), 2.41 (d, *J* = 2.3 Hz, 3H), 1.79 - 1.74 (m, 2H), 1.61 (d, *J* = 2.3 Hz, 3H), 1.31 (dd, *J* = 6.3, 2.3 Hz, 6H); MS m/z [M+H]⁺ 700.39.

### [Preparation Example 5-4'] methyl (S)-2-(3-aminoprop-1-yn-1-yl)-4-(3-(2-(4-(4-chlorophenyl)-2,3,9-trimethyl-6H-thieno[3,2-f][1,2,4]triazolo[4,3-a][1,4]diazepin-6-yl)acetamido)propanamido)benzoate hydrochloride

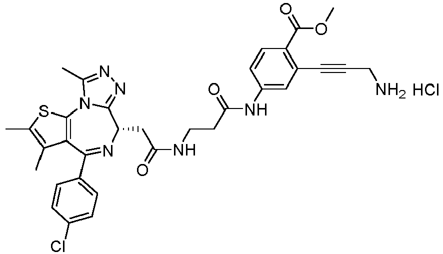

Example 5-4' was synthesized in the same manner as in Preparation Example 1-1' from Intermediate 4-124'a and Intermediate 3-25a.

¹H NMR (500 MHz, DMSO-*d*₆) δ 10.62 (s, 1H), 8.54-8.35 (m, 4H), 8.14 (d, *J*= 2.2 Hz, 1H), 7.90 (d, *J* = 8.7 Hz, 1H), 7.63 (dd, *J* = 8.7, 2.2 Hz, 1H), 7.47 - 7.37 (m, 4H), 4.53 (dd, *J* = 8.1, 6.0 Hz, 1H), 4.05 (q, *J* = 5.6 Hz, 2H), 3.84 (s, 3H), 3.41 (dd, *J* = 10.0, 6.1 Hz, 2H), 3.29 - 3.16 (m, 2H), 2.61 (s, 5H), 2.42 (d, *J* = 0.9 Hz, 3H), 1.61 (s, 3H); MS m/z [M+H]⁺ 658.38.

### [Preparation Example 5-5'] methyl (S)-2-(3-aminoprop-1-yn-1-yl)-4-(5-(2-(4-(4-chlorophenyl)-2,3,9-trimethyl-6H-thieno[3,2-f][1,2,4]triazolo[4,3-a][1,4]diazepin-6-yl)acetamido)pentanamido)benzoate hydrochloride

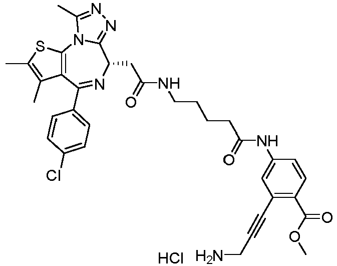

Example 5-5' was synthesized in the same manner as in step 2 of Preparation Example 5-77' from Intermediate 3-25a.

¹H NMR (500 MHz, DMSO-*d*₆) δ 10.39 (s, 1H), 8.33 (s, 3H), 8.25 (t, *J=* 5.6 Hz, 1H), 8.15 (d, *J* = 2.2 Hz, 1H), 7.89 (d, *J* = 8.7 Hz, 1H), 7.53 (dd, *J* = 8.7, 2.2 Hz, 1H), 7.46 (d, *J* = 8.8 Hz, 2H), 7.40 (d, *J* = 8.6 Hz, 2H), 4.50 (dd, *J* = 8.4, 5.9 Hz, 1H), 4.06 (q, *J* = 5.7 Hz, 2H), 3.83 (s, 3H), 3.26 (dd, *J* = 15.0, 8.4 Hz, 2H), 3.17 (dd, *J* = 10.5, 4.4 Hz, 2H), 2.59 (d, *J* = 2.8 Hz, 3H), 2.41 - 2.40 (m, 3H), 2.39 - 2.36 (m, 2H), 1.63 (d, *J* = 8.2 Hz, 2H), 1.60 (d, *J* = 0.8 Hz, 3H), 1.51 - 1.47 (m, 2H); MS m/z [M+H]⁺ 686.47.

### [Preparation Example 5-6'] methyl (S)-2-(3-aminoprop-1-yn-1-yl)-4-(6-(2-(4-(4-chlorophenyl)-2,3,9-trimethyl-6H-thieno[3,2-f][1,2,4]triazolo[4,3-a][1,4]diazepin-6-yl)acetamido)hexanamido)benzoate hydrochloride

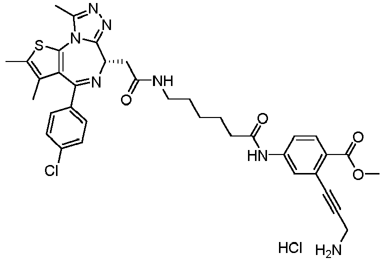

Example 5-6' was synthesized in the same manner as in step 2 of Preparation Example 5-77' from Intermediate 4-165'a and Intermediate 3-25a.

¹H NMR (500 MHz, DMSO-*d*₆) δ 10.36 (s, 1H), 8.32 (s, 3H), 8.21 (t, *J* = 5.7 Hz, 1H), 8.16 (d, *J* = 2.2 Hz, 1H), 7.89 (d, *J* = 8.6 Hz, 1H), 7.52 (dd, *J* = 8.7, 2.2 Hz, 1H), 7.49 (d, *J* = 8.7 Hz, 2H), 7.42 (d, *J* = 8.5 Hz, 2H), 4.50 (dd, *J* = 8.1, 6.1 Hz, 1H), 4.05 (dd, *J* = 11.2, 5.6 Hz, 2H), 3.83 (s, 3H), 3.25 - 3.18 (m, 2H), 2.67 - 2.62 (m, 2H), 2.59 (s, 3H), 2.41 (s, 3H), 2.38 - 2.33 (m, 4H), 1.62 (s, 3H), 1.46 (q, *J* = 7.4 Hz, 2H), 1.35 (d, *J* = 7.1 Hz, 2H); MS m/z [M+H]⁺ 700.47.

### [Preparation Example 5-8'] (S)-N-(4-(3-aminoprop-1-yn-1-yl)-3-(hydroxymethyl)phenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide hydrochloride

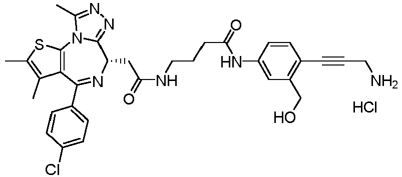

Example 5-8' was synthesized in the same manner as in Preparation Example 4-125' using Intermediate 3-3c and 4-162'a.

¹H NMR (500 MHz, DMSO-*d*₆) δ 10.15 (s, 1H), 8.30 (d, *J* = 5.3 Hz, 4H), 7.76 (d, *J* = 2.2 Hz, 1H), 7.63 (dd, *J* = 8.4, 2.2 Hz, 1H), 7.51 - 7.42 (m, 2H), 7.41 (d, *J* = 8.7 Hz, 2H), 7.33 (d, *J* = 8.4 Hz, 1H), 4.77 (s, 1H), 4.62 (s, 2H), 4.51 (dd, *J* = 8.1, 6.2 Hz, 1H), 4.01 (q, *J* = 5.6 Hz, 2H), 3.20 - 3.08 (m, 2H), 2.60 (s, 3H), 2.41 (d, *J* = 0.9 Hz, 3H), 2.39 (s, 2H), 2.02 - 1.94 (m, 2H), 1.81 - 1.72 (m, 2H), 1.61 (d, *J* = 1.0 Hz, 3H); MS m/z [M+H]⁺ 644.45.

### [Preparation Example 5-9'] (S)-N-(4-(3-aminoprop-1-yn-1-yl)-3-(hydroxymethyl)phenyl)-5-(2-(4-(4-chlorophenyl)-2,3,9-trimethyl-6H-thieno[3,2-f][1,2,4]triazolo[4,3-a][1,4]diazepin-6-yl)acetamido)pentanamide hydrochloride

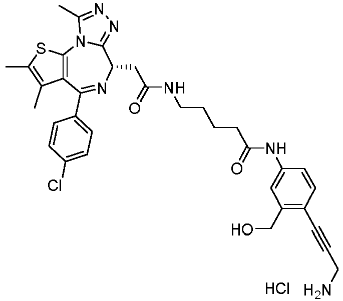

Example 5-9' was synthesized in the same manner as in Preparation Example 4-125' from Intermediate 4-163'a and Intermediate 3-3c.

¹H NMR (500 MHz, DMSO-*d*₆) δ 10.10 (s, 1H), 8.29 (s, 3H), 8.21 (t, *J* = 5.6 Hz, 1H), 7.70 (d, *J* = 2.2 Hz, 1H), 7.60 (dd, *J* = 8.4, 2.2 Hz, 1H), 7.43 (d, *J* = 8.8 Hz, 2H), 7.37 (d, *J* = 8.5 Hz, 2H), 7.28 (d, *J* = 8.4 Hz, 1H), 4.73 (s, 1H), 4.57 (s, 2H), 4.47 (dd, *J* = 8.4, 5.8 Hz, 1H), 3.96 (q, *J* = 5.7 Hz, 2H), 3.22 (dd, *J* = 15.0, 8.5 Hz, 2H), 3.13 (dd, *J* = 10.5, 4.5 Hz, 2H), 2.55 (s, 3H), 2.38 - 2.35 (m, 3H), 2.34 - 2.29 (m, 2H), 1.56 (d, *J* = 0.9 Hz, 3H), 1.48 - 1.39 (m, 4H); MS m/z [M+H]⁺ 658.34.

### [Preparation Example 5-12'] methyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butoxy)benzoate hydrochloride

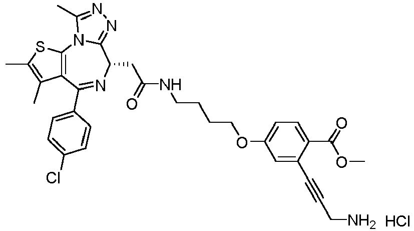

### Step 1: methyl 4-hydroxy-2-iodobenzoate (Intermediate 5-12a)

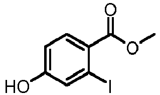

While the mixture of methyl 4-amino-2-iodobenzoate (1.0 mmol, 1.0 eq), H₂SO₄ (0.3 mL), and H₂O (3.0 mL) was stirred at 0 °C, Na₂NO₃ (1.0 eq), which was dissolved in a small amount of H₂O, was added dropwise thereto. After 30 minutes, the reactant was filtered, and then the filtrate was stirred under reflux for 30 minutes. Thereafter, the reaction temperature was decreased to room temperature, and then the stirring was performed for 18 hours. The reactant was extracted with Et₂O to obtain an organic layer. The obtained organic layer was dried over MgSO₄, then filtered under reduced pressure, and then distilled under reduced pressure to synthesize Intermediate 5-12a without further purification.

### Step 2: methyl 2-(3-((tert-butoxycarbonyl)amino)prop-1-yn-1-yl)-4-hydroxybenzoate (Intermediate 5-12b)

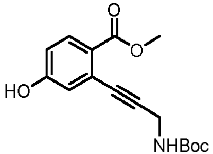

Intermediate 5-12b was synthesized in the same manner as in step 1 of Preparation Example 1-1 from Intermediate 5-12a.

### Step 3: (S)-N-(4-bromobutyl)-2-(4-(4-chlorophenyl)-2,3,9-trimethyl-6H-thieno[3,2-f][1,2,4]triazolo[4,3-a][1,4]diazepin-6-yl)acetamide (Intermediate 5-12'a)

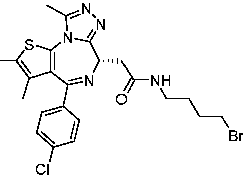

Intermediate 5-12'a was synthesized in the same manner as in step 5 of Preparation Example 4-1' from 4-bromobutan-1-amine hydrochloride.

### Step 4: methyl (S)-2-(3-((tert-butoxycarbonyl)amino)prop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butoxy)benzoate (Intermediate 5-12'b)

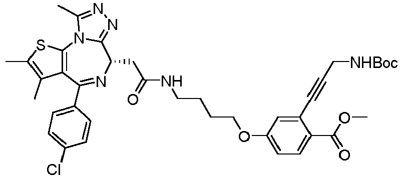

After Intermediate 5-12'a (0.03 mmol, 1.0 eq), Intermediate 5-12b (4.8 eq), K₂CO₃ (4.8 eq), and TBAI (0.1 eq) were added dropwise to dry DMF, a gas in a reactor was replaced with an Ar gas, and then the reaction was performed at 100 °C. After 2 hours, the reaction was terminated by adding H₂O to the reactant, and then a compound was extracted with DCM to obtain an organic layer. The obtained organic layer was dried over MgSO₄. The organic layer was filtered and distilled under reduced pressure, and then purified through flash column to synthesize Intermediate 5-12'b.

### Step 5: methyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butoxy)benzoate hydrochloride

Example 5-12' was synthesized in the same manner as in step 2 of Preparation Example 1-1 from Intermediate 5-12'b.

¹H NMR (500 MHz, DMSO-*d*₆) δ 8.35 (s, 3H), 8.27 (t, *J* = 5.8 Hz, 1H), 7.89 (d, *J* = 8.6 Hz, 1H), 7.43 (q, *J* = 8.8 Hz, 4H), 7.11 - 7.06 (m, 2H), 4.50 (dd, *J* = 8.3, 6.0 Hz, 1H), 4.11 - 4.00 (m, 4H), 3.82 (s, 3H), 3.28 - 3.14 (m, 4H), 2.59 (s, 3H), 2.41 (s, 3H), 2.03 - 1.95 (m, 2H), 1.78 (q, *J* = 7.3 Hz, 2H), 1.63 - 1.56 (m, 3H); MS m/z [M+H]⁺ 659.54.

### [Preparation Example 5-18'] methyl (S)-2-(3-aminoprop-1-yn-1-yl)-4-((3-(2-(4-(4-chlorophenyl)-2,3,9-trimethyl-6H-thieno[3,2-f][1,2,4]triazolo[4,3-a][1,4]diazepin-6-yl)acetamido)propyl)carbamoyl)benzoate hydrochloride

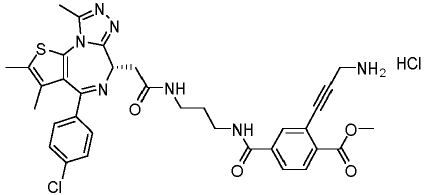

### Step 1: methyl (S)-2-bromo-4-((3-(2-(4-(4-chlorophenyl)-2,3,9-trimethyl-6H-thieno[3,2-f][1,2,4]triazolo[4,3-a][1,4]diazepin-6-yl)acetamido)propyl)carbamoyl)benzoate (Intermediate 5-18'a)

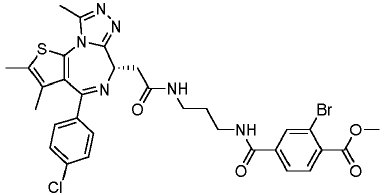

Intermediate 5-18'a was synthesized in the same manner as in step 5 of Preparation Example 4-1' from Intermediate 4-166'a and 3-bromo-4-(methoxycarbonyl)benzoic acid.

### Step 2: methyl (S)-2-(3-((tert-butoxycarbonyl)amino)prop-1-yn-1-yl)-4-((3-(2-(4-(4-chlorophenyl)-2,3,9-trimethyl-6H-thieno[3,2-f][1,2,4]triazolo[4,3-a][1,4]diazepin-6-yl)acetamido)propyl)carbamoyl)benzoate (Intermediate 5-18'b)

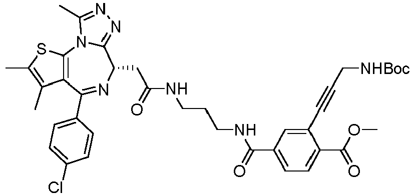

Intermediate 5-18'b was synthesized in the same manner as in step 2 of Preparation Example 4-179' from Intermediate 5-18'a.

### Step 3: methyl (S)-2-(3-aminoprop-1-yn-1-yl)-4-((3-(2-(4-(4-chlorophenyl)-2,3,9-trimethyl-6H-thieno[3,2-f][1,2,4]triazolo[4,3-a][1,4]diazepin-6-yl)acetamido)propyl)carbamoyl)benzoate hydrochloride

Example 5-18' was synthesized in the same manner as in step 2 of Preparation Example 1-1 from Intermediate 5-18'b.

¹H NMR (500 MHz, DMSO-*d*₆) δ 8.80 (t, *J* = 5.6 Hz, 1H), 8.42 (s, 3H), 8.30 (t, *J* = 5.7 Hz, 1H), 8.07 (t, *J* = 1.2 Hz, 1H), 7.99 - 7.97 (m, 2H), 7.49 - 7.39 (m, 4H), 4.51 (dd, *J* = 8.1, 6.1 Hz, 1H), 4.06 (q, *J* = 5.7 Hz, 2H), 3.89 (s, 3H), 3.38 - 3.10 (m, 6H), 2.60 (s, 3H), 2.41 (s, 3H), 1.72 (p, *J* = 6.9 Hz, 2H), 1.60 (s, 3H); MS m/z [M+H]⁺ 672.42.

### [Preparation Example 5-35'] methyl (S)-2-(3-aminoprop-1-yn-1-yl)-4-((4-(2-(4-(4-chlorophenyl)-2,3,9-trimethyl-6H-thieno[3,2-f][1,2,4]triazolo[4,3-a][1,4]diazepin-6-yl)acetamido)cyclohexyl)amino)benzoate trifluoroacetic acid

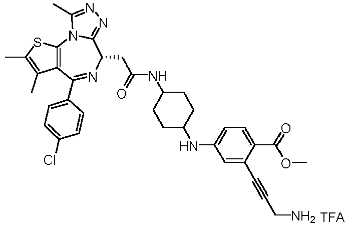

### Step 1: methyl 4-bromo-2-(3-(1,3-dioxoisoindolin-2-yl)prop-1-yn-1-yl)benzoate (Intermediate 5-35a)

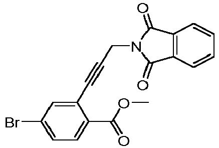

Methyl 4-bromo-2-iodobenzoate(2.0 mmol, 1.0 eq), n-propargylphthalimide (1.1 eq), Pd(PPh₃)₄ (0.01 eq), Cul (0.05 eq), and ACN (7.0 mL) were mixed, and then a gas in a reactor was replaced with an Ar gas. The reactant was stirred at room temperature for 3 hours, filtered under reduced pressure through celite, and then distilled under reduced pressure. The mixture was purified through flash column to synthesize Intermediate 5-35a.

### Step 2: methyl 4-((4-((tert-butoxycarbonyl)amino)cyclohexyl)amino)-2-(3-(1,3-dioxoisoindolin-2-yl)prop-1-yn-1-yl)benzoate (Intermediate 5-35b)

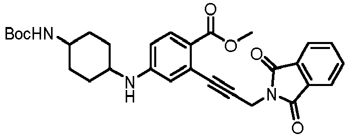

Intermediate 5-35b was synthesized in the same manner as in step 2 of Preparation Example 5- 40 from Intermediate 5-35a and *tert*-butyl(4-aminocyclohexyl)carbamate.

### Step 3: methyl (S)-4-((4-(2-(4-(4-chlorophenyl)-2,3,9-trimethyl-6H-thieno[3,2-f][1,2,4]triazolo[4,3-a][1,4]diazepin-6-yl)acetamido)cyclohexyl)amino)-2-(3-(1,3-dioxoisoindolin-2-yl)prop-1-yn-1-yl)benzoate (Intermediate 5-35'a)

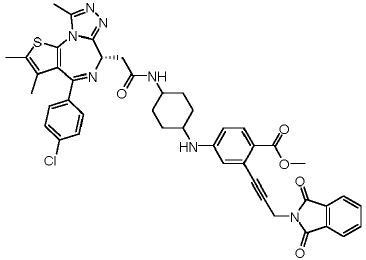

Intermediate 5-35'a was synthesized in the same manner as in steps 4 to 5 of Preparation Example 4-1' from Intermediate 5-35b.

### Step 4: methyl (S)-2-(3-aminoprop-1-yn-1-yl)-4-((4-(2-(4-(4-chlorophenyl)-2,3,9-trimethyl-6H-thieno[3,2-f][1,2,4]triazolo[4,3-a][1,4]diazepin-6-yl)acetamido)cyclohexyl)amino)benzoate trifluoroacetic acid

Example 5-35' was synthesized in the same manner as in step 5 of Preparation Example 5-85' from Intermediate 5-35'a.

¹H NMR (500 MHz, DMSO-*d*₆) δ 8.26 (s, 3H), 8.13 (t, *J* = 7.2 Hz, 1H), 7.67 (dd, *J* = 8.8, 4.8 Hz, 1H), 7.53 - 7.32 (m, 4H), 6.77 - 6.53 (m, 2H), 4.46 (dd, *J* = 8.4, 5.9 Hz, 1H), 3.99 (q, *J* = 5.7 Hz, 2H), 3.71 (s, 2H), 3.16 - 3.09 (m, 5H) 2.55 (s, 3H), 2.37 (s, 3H), 1.65 (d, *J* = 23.1 Hz, 8H), 1.98 - 1.84 (m, 2H), 1.57 (d, *J* = 4.7 Hz, 3H); MS m/z [M+H]⁺ 684.61.

### [Preparation Example 5-53'] (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzoic acid hydrochloride

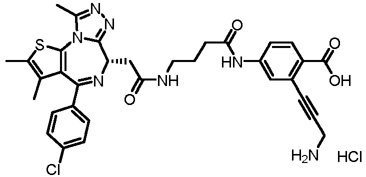

### Step 1: (S)-2-(3-((tert-butoxycarbonyl)amino)prop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzoic acid (Intermediate 5-53'a)

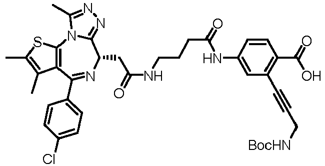

Intermediate 5-53'a was synthesized in the same manner as in step 1 of Preparation Example 3-2' from Intermediate 4-228'a.

### Step 2: (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzoic acid hydrochloride

Example 5-53' was synthesized in the same manner as in step 2 of Preparation Example 1-1 from Intermediate 5-53'a.

¹H NMR (500 MHz, DMSO-*d*₆) δ 10.39 (s, 1H), 8.34 (s, 4H), 8.15 (d, *J* = 2.3 Hz, 1H), 7.88 (d, *J* = 8.6 Hz, 1H), 7.46 - 7.41 (m, 5H), 4.51 (dd, *J* = 8.0, 6.4 Hz, 1H), 4.04 (q, *J* = 5.7 Hz, 2H), 3.67 (dd, *J* = 4.8, 1.4 Hz, 2H), 3.26 - 3.23 (m, 2H), 3.21 - 3.17 (m, 2H), 2.60 (s, 3H), 2.41 (s, 3H), 1.78 (d, *J* = 7.5 Hz, 2H), 1.61 (s, 3H); MS m/z [M+H]⁺ 658.37.

### [Preparation Example 5-59'] (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzamide hydrochloride

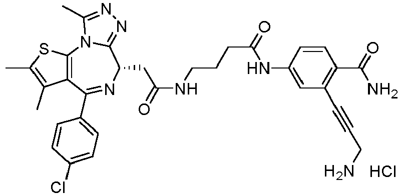

### Step 1: tert-butyl (S)-(3-(2-carbamoyl-5-(4-(2-(4-(4-chlorophenyl)-2,3,9-trimethyl-6H-thieno[3,2-f][1,2,4]triazolo[4,3-a][1,4]diazepin-6-yl)acetamido)butanamido)phenyl)prop-2-yn-1-yl)carbamate (Intermediate 5-59'a)

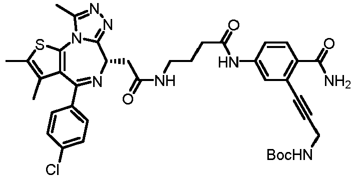

After Intermediate 5-53'a (0.04 mmol, 1.0 eq) was dissolved in 1.0 mL of DCM, a small amount of SOCl₂ was added dropwise thereto, and then the resulting mixture was stirred under reflux for 1 hour. After the reaction temperature was decreased to 0 °C, 2.0 mL of ammonium hydroxide was slowly added dropwise thereto, followed by stirring at room temperature for 18 hours. The reactant was dried over Na₂SO₄, and then filtered under reduced pressure. The mixture was vaporized under reduced pressure, and then purified through flash column to synthesize Intermediate 5-59'a.

### Step 2: (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzamide hydrochloride

Example 5-59' was synthesized in the same manner as in step 2 of Preparation Example 1-1 from Intermediate 5-59'a.

¹H NMR (500 MHz, DMSO-*d*₆) δ 10.27 (s, 1H), 8.31 (dd, *J* = 11.8, 6.3 Hz, 4H), 8.06 (d, *J* = 2.2 Hz, 1H), 7.65 (s, 1H), 7.60 (d, *J* = 8.5 Hz, 1H), 7.49 - 7.45 (m, 3H), 7.43 - 7.41 (m, 3H), 4.51 (dd, *J* = 7.9, 6.4 Hz, 1H), 4.02 (t, *J* = 5.6 Hz, 2H), 3.50 - 3.48 (m, 2H), 3.21 (ddd, *J* = 27.8, 11.7, 7.6 Hz, 4H), 2.60 (s, 3H), 2.42 - 2.41 (m, 3H), 1.78 (d, *J* = 7.4 Hz, 2H), 1.62 - 1.61 (m, 3H); MS m/z [M+H]⁺ 657.44.

### [Preparation Example 5-61'] (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)-N,N-dimethylbenzamide hydrochloride

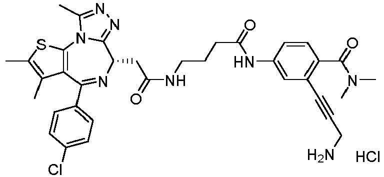

### Step 1: tert-butyl (S)-(3-(5-(4-(2-(4-(4-chlorophenyl)-2,3,9-trimethyl-6H-thieno[3,2-f][1,2,4]triazolo[4,3-a][1,4]diazepin-6-yl)acetamido)butanamido)-2-(dimethylcarbamoyl)phenyl)prop-2-yn-1-yl)carbamate (Intermediate 5-61'a)

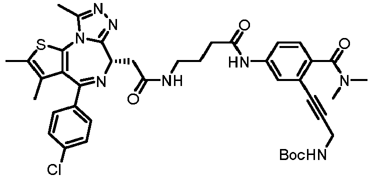

Intermediate 5-53'a (0.03 mmol, 1.0 eq) was dissolved in 0.8 mL of dry DCE, and then a gas in a reactor was replaced with an Ar gas. DEA (2.0 M in THF, 0.01 mL) was added dropwise thereto, followed by stirring at room temperature for 5 minutes. DCC (1.5 eq) and HOBt (1.5 eq) were further mixed, followed by reaction at 70 °C for 3 hours. The reactant was distilled under reduced pressure, and then purified through flash column to synthesize Intermediate 5-61'a.

### Step 2: (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)-N,N-dimethylbenzamide hydrochloride

Example 5-61' was synthesized in the same manner as in step 2 of Preparation Example 1-1 from Intermediate 5-61'a.

¹H NMR (500 MHz, DMSO-*d*₆) δ 10.27 (s, 1H), 8.36 (s, 3H), 8.33 - 8.30 (m, 1H), 8.03 (d, *J* = 2.1 Hz, 1H), 7.51 (dd, *J* = 8.4, 2.2 Hz, 1H), 7.46 (d, *J* = 8.8 Hz, 2H), 7.42 (d, *J* = 8.6 Hz, 2H), 7.26 (d, *J* = 8.4 Hz, 1H), 4.54 - 4.51 (m, 1H), 4.01 (d, *J* = 5.7 Hz, 2H), 3.71 - 3.67 (m, 2H), 3.27 - 3.16 (m, 4H), 3.01 (s, 3H), 2.81 (s, 3H), 2.60 (s, 3H), 2.41 (s, 3H), 1.80 - 1.76 (m, 2H), 1.61 (s, 3H); MS m/z [M+H]⁺ 685.61.

### [Preparation Example 5-63'] (S)-N-(3-(3-aminoprop-1-yn-1-yl)-4-methoxyphenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide trifluoroacetic acid

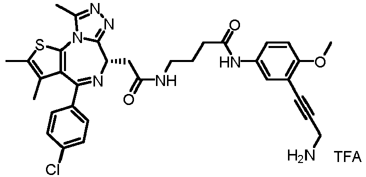

### Step 1: tert-butyl (3-(5-amino-2-methoxyphenyl)prop-2-yn-1-yl)carbamate (Intermediate 5-63a)

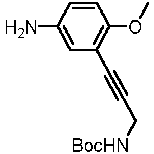

Intermediate 5-63a was synthesized in the same manner as in step 1 of Preparation Example 1-1 from 3-iodo-4-methoxyaniline.

### Step 2: tert-butyl (S)-(3-(5-(4-(2-(4-(4-chlorophenyl)-2,3,9-trimethyl-6H-thieno[3,2-f][1,2,4]triazolo[4,3-a][1,4]diazepin-6-yl)acetamido)butanamido)-2-methoxyphenyl)prop-2-yn-1-yl)carbamate(Intermediate 5-63'a)

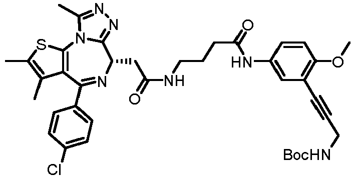

Intermediate 5-63'b was synthesized in the same manner as in step 5 of Preparation Example 4-1' from Intermediates 4-162'a and 5-63a.

### Step 3: (S)-N-(3-(3-aminoprop-1-yn-1-yl)-4-methoxyphenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide trifluoroacetic acid

Example 5-63' was synthesized in the same manner as in step 2 of Preparation Example 1-1' from Intermediate 5-63'a.

¹H NMR (500 MHz, DMSO-*d*₆) δ 9.89 (s, 1H), 8.27 (d, *J* = 6.5 Hz, 3H), 7.89 (d, *J* = 2.7 Hz, 1H), 7.47 - 7.39 (m, 6H), 7.04 (d, *J* = 9.1 Hz, 1H), 4.50 (dd, *J* = 8.2, 6.2 Hz, 1H), 4.03 (d, *J* = 5.6 Hz, 2H), 3.78 (s, 3H), 3.20 (ddt, *J* = 37.0, 23.1, 6.8 Hz, 6H), 2.60 (s, 3H), 2.41 (d, *J* = 0.9 Hz, 4H), 2.34 (t, *J* = 7.5 Hz, 2H), 1.79 - 1.73 (m, 2H), 1.62 - 1.61 (m, 3H); MS m/z [M+H]⁺ 644.45.

### [Preparation Example 5-65'] (S)-N-(3-(3-aminoprop-1-yn-1-yl)-4-methylphenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide hydrochloride

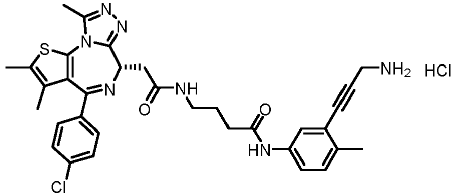

Example 5-65' was synthesized in the same manner of Preparation Example 5-77' from 3-iodo-4-methylaniline.

¹H NMR (500 MHz, DMSO-*d*₆) δ10.162 (s, 1H), 8.531 (brs, 3H), 8.351(t, *J* = 1.3Hz, 1H), 7.878(d, *J* = 0.2 Hz), 7.481 - 7.411 (m, 5H), 7.203(d, 1.1 Hz, 1H), 4.572 (t, *J* = 1.6 Hz, 1H), 4.016 (d, *J* = 0.2 Hz, 2H), 3.282 - 3.163 (m, 4H), 2.644 (s, 3H), 2.416 (s, 3H), 2.396 - 2.357 (m, 2H), 2.338 (s, 3H), 1.798 - 1.743 (m, 2H), 1.612 (s, 3H); MS m/z [M+H]⁺ 628.4.

### [Preparation Example 5-75'] (S)-N-(3-(3-aminoprop-1-yn-1-yl)-4-(trifluoromethyl)phenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide hydrochloride

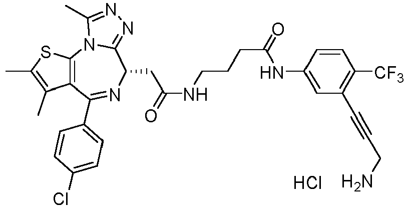

Example 5-75' was synthesized in the same manner as in Preparation Example 5-77' from 3-iodo-4-(trifluoromethyl)aniline.

¹H NMR (500 MHz, DMSO-*d*₆) δ 10.18 (s, 1H), 9.03 (s, 1H), 8.75 (s, 1H), 8.62 (s, 1H), 8.34 (s, 3H), 8.20 (s, 1H), 7.95 (s, 2H), 7.94 (s, 1H), 7.71 (s, 1H), 7.57 (d, *J* = 8.3 Hz, 2H), 7.25 (d, *J* = 8.5 Hz, 1H), 7.10 (s, 1H), 7.08 (s, 1H), 4.55 (s, 2H), 3.92 (s, 2H), 3.80 (s, 3H), 3.13 (s, 2H), 2.38 (s, 2H), 1.85 (s, 2H); MS m/z [M+H]⁺ 682.41.

### [Preparation Example 5-77'] (S)-N-(3-(3-aminoprop-1-yn-1-yl)-4-fluorophenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide hydrochloride

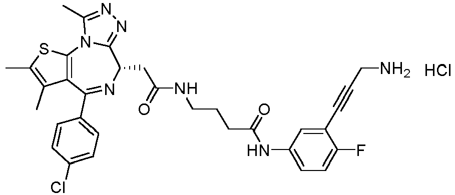

### Step 1: tert-butyl (3-(5-amino-2-fluorophenyl)prop-2-yn-1-yl)carbamate (Intermediate 5-77a)

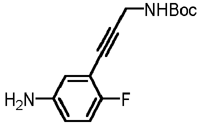

TEA (3.0 mL) was added dropwise to 4-Fluoro-3-iodoaniline (1.0 mmol, 1.0 eq), n-boc propargylamine (1.5 eq), Pd(PPh₃)₄ (0.1 eq), and Cul (0.2 eq). A gas in a reactor was replaced with an Ar gas, followed by stirring at 70 °C overnight. The resulting mixture was filtered under reduced pressure using celite, and then vaporized. The mixture was purified through flash column to synthesize Intermediate 5-77a.

### Step 2: (S)-N-(3-(3-aminoprop-1-yn-1-yl)-4-fluorophenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide hydrochloride

Example 5-77' was synthesized by sequentially applying the same manner as in step 5 of Preparation Example 4-1' and step 2 of Preparation Example 1-1 from Intermediates 4-162'a and 5-77a.

¹H NMR (400 MHz, DMSO-*d*₆) δ 10.368 (s, 1H), 8.530 (brs, 3H), 8.354 - 8.341 (m, 1H), 7.994 - 7.972 (m, 1H), 7.574 (s, 1H), 7.475 - 7.407 (m, 4H), 7.288 - 7.242 (m, 1H), 4.573 - 4.538 (m, 1H), 4.044 - 4.031 (m, 2H), 3.275 -3.132 (m, 4H), 2.632 (s, 3H), 2.414 (s, 3H), 2.390 - 2.371 (m, 2H), 1.779 - 1.747 (m, 2H), 1.611 (s, 3H); ¹⁹F NMR (400 MHz, DMSO-*d*₆) -117.332; MS m/z [M+H]⁺ 632.10.

### [Preparation Example 5-78'] methyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)-6-methylbenzoate hydrochloride

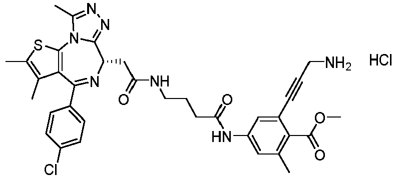

### Step 1: methyl 4-bromo-2-(3-((tert-butoxycarbonyl)amino)prop-1-yn-1-yl)-6-methylbenzoate (Intermediate 5-78a)

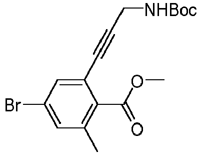

Intermediate 5-78a was synthesized in the same manner as in step 1 of Preparation Example 1-1 from methyl 4-bromo-2-iodo-6-methylbenzoate.

### Step 2: methyl 4-((tert-butoxycarbonyl)amino)-2-(3-((tert-butoxycarbonyl)amino)prop-1-yn-1-yl)-6-methylbenzoate (Intermediate 5-78b)

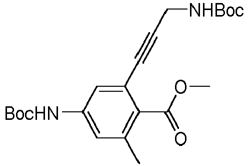

Intermediate 5-78a (0.5 mmol, 1.0 eq), tert-butyl carbamate (3.0 eq), BrettPhos Pd G3 (0.1 eq), CS₂CO₃ (3.0 eq), and dioxane (1.5 mL) were mixed and then the resulting mixture was stirred at 100 °C overnight. The reactant was extracted with EtOAc and H₂O to obtain an organic layer. The obtained organic layer was dried over Na₂SO₄. The mixture was filtered under reduced pressure, distilled, and then purified through flash column to synthesize Intermediate 5-78b.

### Step 3: methyl 4-amino-2-(3-aminoprop-1-yn-1-yl)-6-methylbenzoate (Intermediate 5-78c)

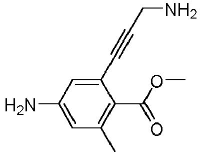

Intermediate 5-78c was synthesized in the same manner as in step 2 of Preparation Example 1-1 from Intermediate 5-78b.

### Step 4: methyl 4-amino-2-(3-((tert-butoxycarbonyl)amino)prop-1-yn-1-yl)-6-methylbenzoate (Intermediate 5-78d)

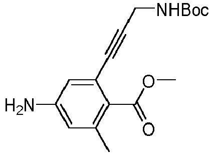

After Intermediate 5-78c (0.1 mmol, 1.0 eq) was dissolved in 3.0 mL of DCM, TEA (3.0 eq) and Boc₂O (1.0 eq) were slowly added dropwise thereto at 0 °C, and stirred for 2 hours. The reactant was extracted with DCM and H₂O to obtain an organic layer. The obtained organic layer was dried over Na₂SO₄. The mixture was filtered under reduced pressure, distilled, and then purified through flash column to synthesize Intermediate 5-78d.

### Step 5: methyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)-6-methylbenzoate hydrochloride

Example 5-78' was synthesized in the same manner as in steps 2 of Preparation Example 5-77' from Intermediate 5-78d.

¹H NMR (400 MHz, DMSO-*d*₆) δ 10.38 (s, 1H), 8.50 (s, 3H), 8.34 (t, *J* = 14 Hz, 1H), 7.86 (s, 1H), 7.48 - 7.41 (m, 5H), 4.56 - 4.53 (m, 1H), 4.00 - 3.98 (m, 2H), 3.86 (s, 3H), 3.27 - 3.15 (m, 2H), 2.62 (s 3H), 2.41 (s, 5H), 2.25 (s, 3H), 1.80 -1.76 (m, 2H), 1.61 (s, 3H); MS m/z [M+H]⁺ 686.4.

### [Preparation Example 5-81'] (S)-N-(3-(3-aminoprop-1-yn-1-yl)-4-(2-(trifluoromethyl)pyrimidin-5-yl)phenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide hydrochloride

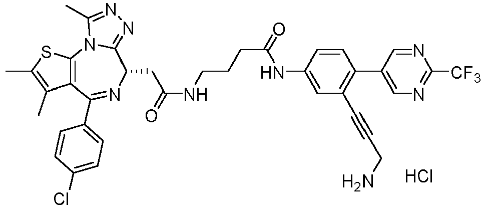

### Step 1: tert-butyl (3-(2-bromo-5-nitrophenyl)prop-2-yn-1-yl)carbamate (Intermediate 5-81a)

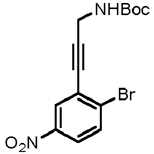

Intermediate 5-81a was synthesized in the same manner as in step 1 of Preparation Example 5-89' from bromo-2-iodo-4-nitrobenzene.

### Step 2: tert-butyl (3-(5-nitro-2-(2-(trifluoromethyl)pyrimidin-5-yl)phenyl)prop-2-yn-1-yl)carbamate (Intermediate 5-81b)

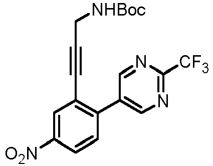

Intermediate 5-81a (3.0 mmol, 1.0 eq), (2-(trifluoromethyl)pyrimidin-5-yl)boronic acid (1.1 eq), Pd(dppf)Cl₂ (0.1 eq), K₂CO₃ (2.0 eq), and dioxane/H₂O (10.0 mL) were mixed, and then the resulting mixture was stirred at 50 °C for 12 hours. The reactant was purified through flash column to synthesize Intermediate Compound 5-81b.

### Step 3: tert-butyl (3-(5-amino-2-(2-(trifluoromethyl)pyrimidin-5-yl)phenyl)prop-2-yn-1-yl)carbamate (Intermediate 5-81c)

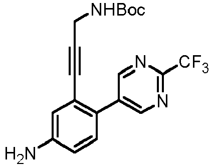

Intermediate 5-81b (1.5 mmol, 1.0 eq), Fe (3.0 eq), NH₄Cl (5.0 eq), and EtOH/H₂O were stirred at 80 °C for 2 hours. The reactant was purified through flash column to synthesize Intermediate 5-81c.

### Step 4: (S)-N-(3-(3-aminoprop-1-yn-1-yl)-4-(2-(trifluoromethyl)pyrimidin-5-yl)phenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide hydrochloride

Example 5-81' was synthesized in the same manner as in steps 2 to 3 of Preparation Example 5-77' from Intermediate 5-81c.

¹H NMR (500 MHz, DMSO-*d*₆) δ 10.36 (s, 1H), 9.25 (s, 2H), 8.28 (dd, *J* = 13.5, 7.6 Hz, 4H), 8.18 (t, *J* = 1.3 Hz, 1H), 7.62 (d, *J* = 1.8 Hz, 2H), 7.42 (d, *J* = 8.9 Hz, 2H), 7.40 - 7.35 (m, 2H), 4.48 (dd, *J* = 8.1, 6.2 Hz, 1H), 3.91 (q, *J* = 5.6 Hz, 2H), 3.51 (s, 1H), 3.27 - 3.07 (m, 4H), 2.56 (s, 3H), 2.43 - 2.34 (m, 5H), 1.76 (q, *J* = 7.3 Hz, 2H), 1.58 (d, *J* = 0.9 Hz, 3H); MS m/z [M+H]⁺ 760.44.

### [Preparation Example 5-89'] methyl (S)-3-(3-aminoprop-1-yn-1-yl)-5-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzoate hydrochloride

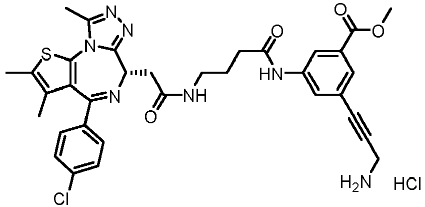

### Step 1: methyl 3-amino-5-(3-((tert-butoxycarbonyl)amino)prop-1-yn-1-yl)benzoate (Intermediate 5-89a)

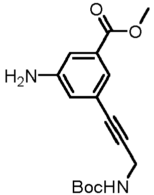

Methyl 3-amino-5-bromobenzoate (4.0 mmol, 1.0 eq), n-boc propargylamine (2.0 eq), Xphos Pd G₄ (0.03 eq), Cul (0.1 eq), Cs₂CO₃ (3.0 eq), and dry toluene (10.0 mL) were mixed, and then the resulting mixture was stirred at 100 °C for 12 hours. The reactant was filtered under reduced pressure through celite. The mixture was vaporized under reduced pressure, and then purified through flash column to synthesize Intermediate 5-89a.

### Step 2: methyl (S)-3-(3-aminoprop-1-yn-1-yl)-5-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzoate hydrochloride

Intermediate 5-89' was synthesized in the same manner as in steps 2 to 3 of Preparation Example 5-77' from Intermediate 5-89a.

¹H NMR (500 MHz, DMSO-*d*₆) δ 10.26 (s, 1H), 8.26 (t, *J*= 5.8 Hz, 1H), 8.13 (s, 1H), 7.96 (s, 1H), 7.54 (s, 1H), 7.44 - 7.35 (m, 6H), 4.46 (dd, *J*= 8.3, 6.0 Hz, 1H), 3.81 (s, 3H), 3.57 (s, 3H), 3.17 (d, *J*= 6.4 Hz, 2H), 3.11 - 3.08 (m, 2H), 2.55 (s, 3H), 2.36 (s, 3H), 1.74 (q, *J*= 7.2 Hz, 3H), 1.57 (s, 4H); MS m/z [M+H]⁺ 672.52.

### [Preparation Example 5-90'] methyl (S)-4-(3-aminoprop-1-yn-1-yl)-2-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzoate hydrochloride

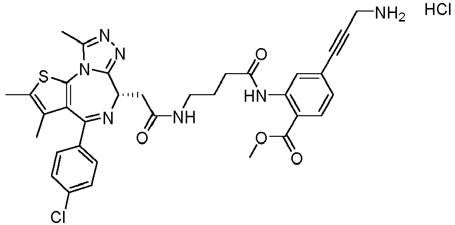

Example 5-90' was synthesized in the same manner as in Example 5-77' from methyl 2-amino-4-iodobenzoate.

¹H NMR (500 MHz, DMSO-*d*₆) δ 10.61 (s, 1H), 8.36 (d, *J=* 1.7 Hz, 1H), 8.29 (t, *J*= 5.7 Hz, 1H), 7.87 (d, *J*= 8.2 Hz, 1H), 7.49 - 7.38 (m, 6H), 7.16 (dd, *J*= 8.2, 1.7 Hz, 1H), 4.49 (dd, *J*= 8.3, 5.9 Hz, 1H), 3.81 (s, 3H), 3.52 (s, 2H), 3.26 - 3.22 (m, 2H), 3.14 (ddd, *J*= 16.9, 7.9, 5.6 Hz, 2H), 2.58 (d, *J*= 2.6 Hz, 3H), 2.40 (d, *J*= 0.9 Hz, 4H), 1.80 (dd, *J*= 13.9, 6.7 Hz, 3H), 1.61 (t, *J*= 1.2 Hz, 4H); MS m/z [M+H]⁺ 672.52.

### [Preparation Example 5-96'] methyl (S)-3-(3-aminoprop-1-yn-1-yl)-6-(4-(2-(4-(4-chlorophenyl)-2,3,9-trimethyl-6H-thieno[3,2-f][1,2,4]triazolo[4,3-a][1,4]diazepin-6-yl)acetamido)butanamido)picolinate hydrochloride

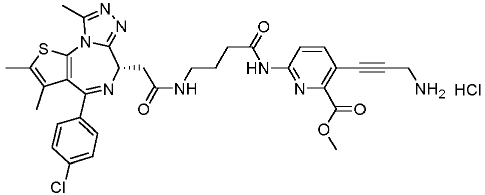

### Step 1: methyl 6-amino-3-(3-((tert-butoxycarbonyl)amino)prop-1-yn-1-yl)picolinate (Intermediate 5-96a)

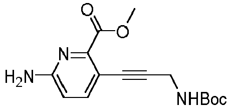

Intermediate 5-96a was synthesized in the same manner as in step 1 of Preparation Example 5-89' using methyl 6-amino-3-bromopicolinate.

### Step 2: methyl (S)-3-(3-aminoprop-1-yn-1-yl)-6-(4-(2-(4-(4-chlorophenyl)-2,3,9-trimethyl-6H-thieno[3,2-f][1,2,4]triazolo[4,3-a][1,4]diazepin-6-yl)acetamido)butanamido)picolinate hydrochloride

Example 5-96' was synthesized in the same manner as in steps 2 to 3 of Preparation Example 5-77' from Intermediate 5-96a.

¹H NMR (500 MHz, DMSO-*d*₆) δ 10.99 (s, 1H), 8.30 (d, *J* = 8.8 Hz, 1H), 8.26 (s, 4H), 8.22 (t, *J* = 5.7 Hz, 1H), 7.97 (d, *J* = 8.7 Hz, 1H), 7.45 - 7.39 (m, 2H), 7.39 - 7.33 (m, 2H), 4.46 (dd, *J* = 8.3, 6.0 Hz, 1H), 4.02 (q, *J* = 5.6 Hz, 2H), 3.85 (s, 3H), 3.26 - 3.04 (m, 4H), 2.55 (s, 5H), 2.37 (d, *J=* 0.8 Hz, 3H), 1.72 (p, *J=* 7.2 Hz, 2H), 1.57 (d, *J=* 0.8 Hz, 3H); MS m/z [M+H]⁺ 673.42.

### [Preparation Example 5-104'] (S)-N-(3-(3-aminoprop-1-yn-1-yl)phenyl)-6-(2-(4-(4-chlorophenyl)-2,3,9-trimethyl-6H-thieno[3,2-f][1,2,4]triazolo[4,3-a][1,4]diazepin-6-yl)acetamido)hexanamide hydrochloride

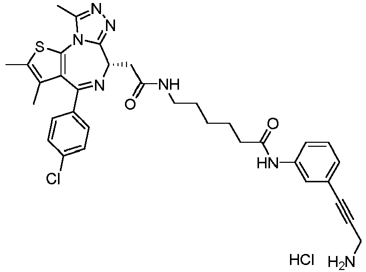

Example 5-104' was synthesized in the same manner as in steps 2 of Preparation Example 5-77' from Intermediate 4-165'a and Intermediate 1-3a.

¹H NMR (500 MHz, DMSO-*d*₆) δ 10.09 (s, 1H), 8.35 (s, 3H), 8.19 (s, 1H), 7.92 (s, 1H), 7.46 (d, *J* = 8.8 Hz, 4H), 7.38 (d, *J* = 8.5 Hz, 2H), 7.29 (t, *J* = 7.9 Hz, 1H), 7.07 (d, *J* = 10.1 Hz, 1H), 4.50 - 4.46 (m, 1H), 3.96 (t, *J*= 5.6 Hz, 2H), 3.24-3.12 (m, 2H), 3.10 - 3.03 (m, 2H), 2.57 (s, 3H), 2.37 (s, 3H), 2.28 (t, *J* = 7.5 Hz, 2H), 1.57 (d, *J* = 7.2 Hz, 6H), 1.47 - 1.40 (m, 2H), 1.34 - 1.27 (m, 2H); MS m/z [M+H]⁺ 642.45.

### [Preparation Example 5-106'] methyl (S)-2-(3-aminoprop-1-yn-1-yl)-4-(5-(2-(4-(4-chlorophenyl)-2,3,9-trimethyl-6H-thieno[3,2-f][1,2,4]triazolo[4,3-a][1,4]diazepin-6-yl)ethoxy)-5-oxopentanamido)benzoate hydrochloride

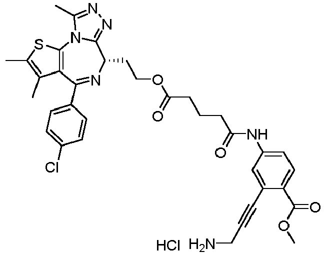

### Step 1: methyl (S)-2-(4-(4-chlorophenyl)-2,3,9-trimethyl-6H-thieno[3,2-f][1,2,4]triazolo[4,3-a][1,4]diazepin-6-yl)acetate (Intermediate 5-106'a)

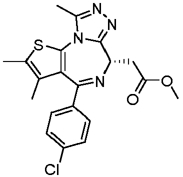

JQ1 carboxylic acid (1.5 mmol, 1.0 eq) was dissolved in 8.0 mL of MeOH, and then SOCl₂ (3.0 eq) was added dropwise thereto. After the resulting mixture was stirred at room temperature for 3 hours, the solvent was removed under reduced pressure, and then DCM was added dropwise thereto, followed by removing the remaining solvent through reduced pressure again. After the compound was dissolved in a small amount of EtOAc, solids generated by recrystallization using n-hexane were filtered to synthesize Intermediate 5-106'a without a further purification process.

### Step 2: (S)-2-(4-(4-chlorophenyl)-2,3,9-trimethyl-6H-thieno[3,2-f][1,2,4]triazolo[4,3-a][1,4]diazepin-6-yl)ethan-1-ol (Intermediate 5-106'b)

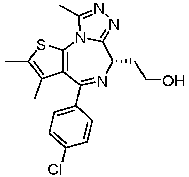

Intermediate 5-106'a (0.3 mmol, 1.0 eq) was dissolved in 3.0 mL of dry THF, followed by stirring at 0 °C. 1.0 M lithium aluminium hydride solution in THF (1.5 eq) was slowly added dropwise thereto for 10 minutes. After 5 minutes, the reaction was terminated by adding ice water. The mixture was acidified to pH 3 by adding 2N HCl aqueous solution dropwise thereto. The mixture was extracted with EtOAc and H₂O to obtain an organic layer. The obtained organic layer was then washed with aqueous NaHCOs and NaCl solutions. The organic layer was dried over Na₂SO₄, filtered under reduced pressure, and then vaporized to synthesize Intermediate 5-106'b without a further purification process.

### Step 3: (S)-5-(2-(4-(4-chlorophenyl)-2,3,9-trimethyl-6H-thieno[3,2-f][1,2,4]triazolo[4,3-a][1,4]diazepin-6-yl)ethoxy)-5-oxopentanoic acid (Intermediate 5-106'c)

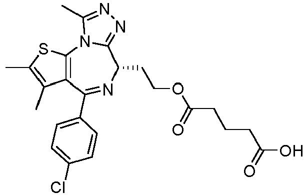

Intermediate 5-106'b (0.1 mmol, 1.0 eq) and glutaric acid (2.0 eq) were dissolved in dry DCM, followed by stirring at room temperature. DCC (1.1 eq) and DMAP (1.1 eq) were added dropwise thereto, followed by stirring for 18 hours. The precipitate generated after the reaction was filtered, and then the filtrate was purified through flash column to synthesize Intermediate 5-106'c.

### Step 4: methyl (S)-2-(3-aminoprop-1-yn-1-yl)-4-(5-(2-(4-(4-chlorophenyl)-2,3,9-trimethyl-6H-thieno[3,2-f][1,2,4]triazolo[4,3-a][1,4]diazepin-6-yl)ethoxy)-5-oxopentanamido)benzoate hydrochloride

Example 5-106' was synthesized in the same manner as in steps 2 of Preparation Example 5-77' from Intermediate 5-106'c and Intermediate 3-25a.

¹H NMR (500 MHz, DMSO-*d*₆) δ 7.89-7.82 (m, 1H), 7.50-7.45 (m, 7H), 7.21 (s, 1H), 6.67 (s, 1H), 4.44 - 4.39 (m, 2H), 4.18 - 4.15 (m, 1H), 4.05 (s, 2H), 3.79 (s, 3H), 2.65 - 2.63 (m, 2H), 2.59 (s, 3H), 2.36 (s, 3H), 2.18 (t, *J* = 7.3 Hz, 2H), 2.02 (s, 2H), 1.60 (s, 3H); MS m/z [M+H]⁺ 687.31.

### [Preparation Example 5-107'] (S)-N-(3-(3-aminoprop-1-yn-1-yl)phenyl)-8-(2-(4-(4-chlorophenyl)-2,3,9-trimethyl-6H-thieno[3,2-f][1,2,4]triazolo[4,3-a][1,4]diazepin-6-yl)acetamido)octanamide hydrochloride

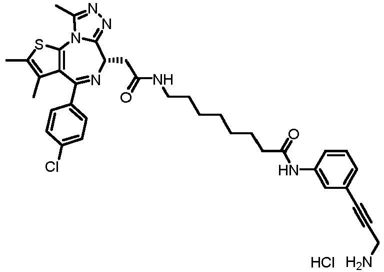

### Step 1: methyl (S)-8-(2-(4-(4-chlorophenyl)-2,3,9-trimethyl-6H-thieno[3,2-f][1,2,4]triazolo[4,3-a][1,4]diazepin-6-yl)acetamido)octanoate (Intermediate 5-107'a)

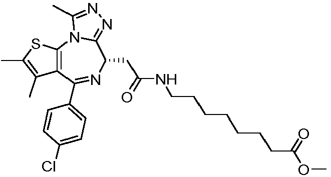

Intermediate 5-107'a was synthesized in the same manner as in step 5 of Preparation Example 4-1' from methyl 8-aminooctanoate.

### Step 2: (S)-8-(2-(4-(4-chlorophenyl)-2,3,9-trimethyl-6H-thieno[3,2-f][1,2,4]triazolo[4,3-a][1,4]diazepin-6-yl)acetamido)octanoic acid (Intermediate 5-107'b)

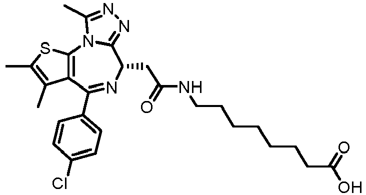

Intermediate 5-107'b was synthesized in the same manner as in Preparation Example step 1 of 3-2' from Intermediate 5-107'a.

### Step 3: (S)-N-(3-(3-aminoprop-1-yn-1-yl)phenyl)-8-(2-(4-(4-chlorophenyl)-2,3,9-trimethyl-6H-thieno[3,2-f][1,2,4]triazolo[4,3-a][1,4]diazepin-6-yl)acetamido)octanamide hydrochloride

Example 5-107' was synthesized in the same manner as in steps 2 of Preparation Example 5-77' from Intermediate 5-107'b and Intermediate 1-3a.

¹H NMR (500 MHz, DMSO-*d*₆) δ 10.12 (s, 1H), 8.42 (s, 3H), 8.17 (t, *J* = 5.6 Hz, 1H), 7.90 (t, *J* = 1.9 Hz, 1H), 7.50 - 7.43 (m, 3H), 7.41 - 7.36 (m, 2H), 7.28 (t, *J* = 7.9 Hz, 1H), 7.07 (dt, *J* = 7.7, 1.3 Hz, 1H), 4.49 (dd, *J* = 8.0, 6.1 Hz, 1H), 3.95 (q, *J* = 5.7 Hz, 2H), 3.24 - 3.12 (m, 2H), 3.06 (dq, *J* = 22.0, 6.4 Hz, 2H), 2.58 (s, 3H), 2.37 (d, *J* = 1.0 Hz, 3H), 2.27 (t, *J* = 7.5 Hz, 2H), 1.58 (d, *J* = 1.0 Hz, 3H), 1.56 - 1.50 (m, 2H), 1.42 - 1.36 (m, 2H), 1.25 (s, 6H); MS m/z [M+H]⁺ 670.53.

### [Preparation Example 5-108'] (S)-N-(3-(3-aminoprop-1-yn-1-yl)phenyl)-11-(2-(4-(4-chlorophenyl)-2,3,9-trimethyl-6H-thieno[3,2-f][1,2,4]triazolo[4,3-a][1,4]diazepin-6-yl)acetamido)undecanamide hydrochloride

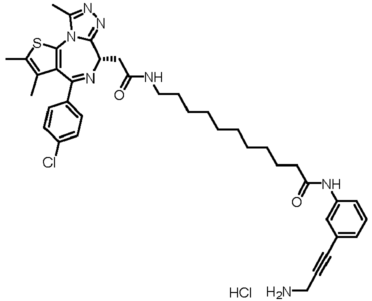

Example 5-108' was synthesized in the same manner as in Preparation Example 5-107' from methyl 11-aminoundecanoate.

¹H NMR (500 MHz, DMSO-*d*₆) δ 10.05 (s, 1H), 8.34 (s, 3H), 8.15 (t, *J=* 5.6 Hz, 1H), 7.91 (s, 1H), 7.44 (d, *J* = 8.8 Hz, 4H), 7.38 (d, *J* = 8.6 Hz, 2H), 7.29 (t, *J* = 7.9 Hz, 1H), 7.07 (d, *J* = 7.7 Hz, 1H), 4.50 - 4.45 (m, 1H), 3.96 (q, *J* = 5.7 Hz, 2H), 3.25 - 3.17 (m, 2H), 3.15 - 3.05 (m, 2H), 2.56 (s, 3H), 2.37 (s, 3H), 2.26 (t, *J* = 7.5 Hz, 2H), 1.58 (s, 3H), 1.56 - 1.50 (m, 2H), 1.41 - 1.36 (m, 2H), 1.22 (s, 14H); MS m/z [M+H]⁺ 712.58.

### [Preparation Example 5-109'] (S)-2-(4-(4-chlorophenyl)-2,3,9-trimethyl-6H-thieno[3,2-f][1,2,4]triazolo[4,3-a][1,4]diazepin-6-yl)ethyl 11-((3-(3-aminoprop-1-yn-1-yl)phenyl)amino)-11-oxoundecanoate hydrochloride

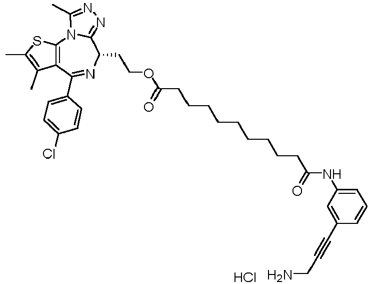

Example 5-109' was synthesized in the same manner as in steps 3 to 4 of Preparation Example 5-106' using Intermediate 5-106'b, undecanedioic acid, and Intermediate 1-3a via Intermediate 5-109'a ((S)-11-(2-(4-(4-chlorophenyl)-2,3,9-trimethyl-6H-thieno[3,2-f][1,2,4]triazolo[4,3-a][1,4]diazepin-6-yl)ethoxy)-11-oxoundecanoic acid).

¹H NMR (500 MHz, DMSO-*d*₆) δ 10.03 (s, 1H), 8.28 (s, 3H), 7.97 (d, *J* = 2.0 Hz, 1H), 7.49 (t, *J* = 9.1 Hz, 6H), 7.33 (t, *J* = 7.9 Hz, 1H), 7.11 (dt, *J* = 7.6, 1.3 Hz, 1H), 4.48 - 4.42 (m, 1H), 4.38 (dt, *J* = 11.1, 6.2 Hz, 1H), 4.14 (dd, *J* = 8.6, 5.6 Hz, 1H), 4.01 (q, *J* = 5.6 Hz, 3H), 2.59 (s, 3H), 2.39 (d, *J* = 0.9 Hz, 4H), 2.29 (d, *J* = 7.3 Hz, 2H), 2.26 (d, *J* = 7.2 Hz, 2H), 1.61 (d, *J* = 0.9 Hz, 3H), 1.56 (s, 2H), 1.44 (s, 2H), 1.26 (d, *J* = 7.8 Hz, 4H), 1.18 (s, 6H); MS m/z [M+H]⁺ 713.58.

### [Preparation Example 5-110'] methyl (S)-2-(3-aminoprop-1-yn-1-yl)-4-(11-(2-(4-(4-chlorophenyl)-2,3,9-trimethyl-6H-thieno[3,2-f][1,2,4]triazolo[4,3-a][1,4]diazepin-6-yl)ethoxy)-11-oxoundecanamido)benzoate hydrochloride

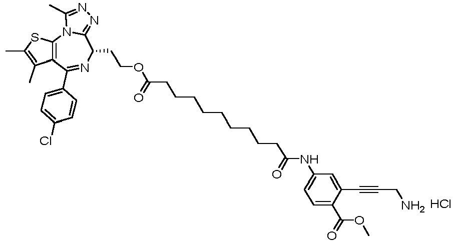

Example 5-110' was synthesized in the same manner as in steps 2 of Preparation Example 5-77' from Intermediate 5-109'a and Intermediate 3-25a.

¹H NMR (500 MHz, DMSO-*d*₆) δ 10.37 (d, *J* = 16.6 Hz, 1H), 8.35 (s, 2H), 8.09 (q, *J* = 2.4 Hz, 1H), 7.88 - 7.83 (m, 1H), 7.54 - 7.38 (m, 5H), 4.41 (dt, *J* = 10.8, 7.0 Hz, 1H), 4.34 (dt, *J* = 11.2, 6.1 Hz, 1H), 4.11 (dd, *J* = 8.6, 5.6 Hz, 1H), 4.04 - 3.98 (m, 2H), 3.79 (s, 3H), 2.64 - 2.58 (m, 2H), 2.56 (d, *J* = 1.3 Hz, 3H), 2.35 (s, 3H), 2.30 (t, *J* = 7.3 Hz, 2H), 2.21 (t, *J* = 7.3 Hz, 2H), 1.57 (s, 3H), 1.52 (t, *J* = 7.2 Hz, 2H), 1.39 (d, *J* = 8.0 Hz, 2H), 1.20 (s, 4H), 1.14 (s, 6H); MS m/z [M+H]⁺ 771.53.

### [Preparation Example 5-115'] methyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetoxy)butanamido)benzoate hydrochloride

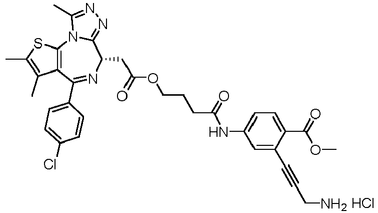

### Step 1: tert-butyl (S)-4-(2-(4-(4-chlorophenyl)-2,3,9-trimethyl-6H-thieno[3,2-f][1,2,4]triazolo[4,3-a][1,4]diazepin-6-yl)acetoxy)butanoate (Intermediate 5-115'a)

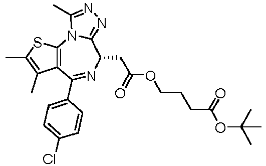

Intermediate 5-115'a was synthesized in the same manner as in step 5 of Preparation Example 4-1' from *tert*-butyl 4-hydroxybutanoate.

### Step 2: (S)-4-(2-(4-(4-chlorophenyl)-2,3,9-trimethyl-6H-thieno[3,2-f][1,2,4]triazolo[4,3-a][1,4]diazepin-6-yl)acetoxy)butanoic acid (Intermediate 5-115'b)

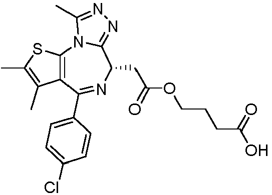

Intermediate 5-115'b was synthesized in the same manner as in step 4 of Preparation Example 4-1' from Intermediate 5-115'a.

### Step 3: methyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetoxy)butanamido)benzoate hydrochloride

Example 5-115' was synthesized in the same manner as in step 2 to 3 of Preparation Example 5-77' from Intermediate 5-115'b and Intermediate 3-25a.

¹H NMR (500 MHz, DMSO-*d*₆) δ 10.40 (s, 1H), 8.31 (s, 2H), 8.15 (d, *J* = 2.1 Hz, 1H), 7.89 (d, *J* = 8.7 Hz, 1H), 7.53 - 7.39 (m, 7H), 4.51 - 4.44 (m, 2H), 4.16 (d, *J* = 5.9 Hz, 3H), 4.07 (d, *J* = 5.6 Hz, 3H), 3.83 (d, *J* = 1.5 Hz, 3H), 3.65 - 3.59 (m, 1H), 3.47 - 3.37 (m, 3H), 3.14 (dd, *J* = 7.3, 4.3 Hz, 1H), 2.69 (d, *J* = 1.4 Hz, 3H), 2.60 (d, *J* = 2.6 Hz, 3H), 2.42 - 2.40 (m, 3H), 1.93 (t, *J* = 6.9 Hz, 2H), 1.62 - 1.60 (m, 3H); MS m/z [M+H]⁺ 673.42.

### [Preparation Example 5-148'] methyl (S)-2-(3-acetamidoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzoate

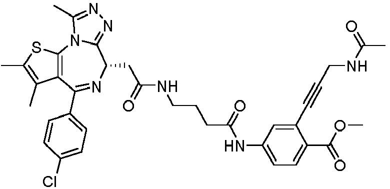

Example 4-228' (0.02 mmol, 1.0 eq) was dissolved in 0.6 mL of DCM, and then TEA (9 µL) and Ac₂O (2.4 µL) were added dropwise thereto at 0 °C, and the resulting mixture was stirred at room temperature for 40 minutes. The reactant was distilled under reduced pressure, and then purified through flash column to synthesize Example 5-148'.

¹H NMR (400 MHz, DMSO-*d*₆) *δ* 10.31 (s, 1H), 8.42 (t, *J* = 5.5 Hz, 1H), 8.30 (t, *J* = 5.7 Hz, 1H), 7.91 (d, *J* = 2.2 Hz, 1H), 7.84 (d, *J* = 8.7 Hz, 1H), 7.60 (dd, *J* = 8.7, 2.2 Hz, 1H), 7.47 - 7.43 (m, 2H), 7.43 - 7.39 (m, 2H), 4.51 (dd, *J* = 8.1, 6.2 Hz, 1H), 4.13 (d, *J* = 5.5 Hz, 2H), 3.80 (s, 3H), 3.28 - 3.13 (m, 4H), 2.60 (s, 3H), 2.43 - 2.39 (m, 5H), 1.86 (s, 3H), 1.77 (p, *J* = 7.6 Hz, 2H), 1.61 (d, *J* = 0.9 Hz, 3H); MS m/z [M+H]⁺ 714.48.

### [Preparation Example 5-149'] methyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)-N-methylacetamido)butanamido)benzoate hydrochloride

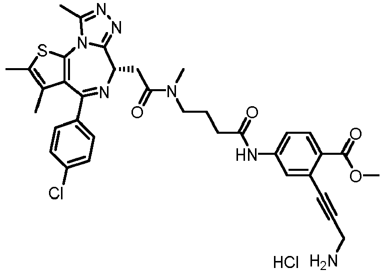

### Step 1: (S)-4-(2-(4-(4-chlorophenyl)-2,3,9-trimethyl-6H-thieno[3,2-f][1,2,4]triazolo[4,3-a][1,4]diazepin-6-yl)-N-methylacetamido)butanoic acid (Intermediate 5-149'a)

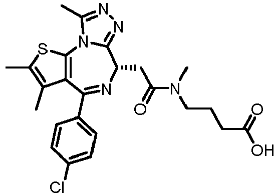

Intermediate 5-149'a was synthesized by sequentially applying the same manner as in step 5 of Preparation Example 4-1' and step 1 of Preparation Example 3-2' from methyl 4-(methylamino)butanoate hydrochloride.

### Step 2: methyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)-N-methylacetamido)butanamido)benzoate hydrochloride

Example 5-149' was synthesized in the same manner as in steps 2 of Preparation Example 5-77' from Intermediate 5-149'a and Intermediate 3-25a.

¹H NMR (400 MHz, DMSO-*d*₆) δ 10.667-10.521 (m, 1H), 8.566(brs, 3H), 8.160 -8.138 (m, 1H), 7.889 - 7.823 (m, 1H), 7.525 - 7.512 (m, 1H). 7.508 - 7.409 (m, 4H), 4.572 (m, 1H), 4.052 - 4.032 (m, 2H), 3.841 (s, 3H), 3.713 - 3.501 (m, 2H), 3.407 -3.391 (m, 2H), 3.148 (s, 3H), 2.640 (s, 3H), 2.499 (s, 3H), 2.430 - 2.360 (m, 2H), 1.827 - 1.810 (m, 1H), 1.793 -1.635 (m, 1H), 1.626 (s, 3H); MS m/z [M+H]⁺ 686.2.

### [Preparation Example 5-150'] methyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)-N-methylbutanamido)benzoate hydrochloride

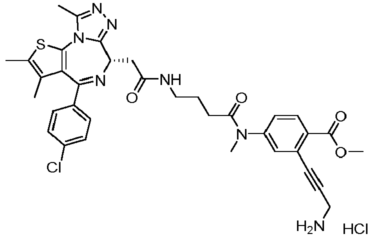

### Step 1: methyl 2-(3-((tert-butoxycarbonyl)amino)prop-1-yn-1-yl)-4-(methylamino)benzoate (Intermediate 5-150a)

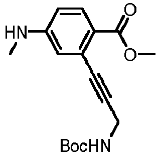

Intermediate 3-25a (0.6 mmol, 1.0 eq), MeB(OH)₂ (2.5 eq), Cu(OAc)₂ (2.5 eq), pyridine (3.5 eq), and dioxane (6.0 mL) were mixed, and then a gas in a reactor was replaced with an Ar gas. The reactant was stirred at 110 °C overnight. The reactant was filtered under reduced pressure through celite and then vaporized. The mixture was purified through flash column to synthesize Intermediate 5-150a.

### Step 2: methyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)-N-methylbutanamido)benzoate hydrochloride

Example 5-150' was synthesized in the same manner as in steps 2 of Example 5-77' from Intermediate 5-150a.

¹H NMR (400 MHz, DMSO-*d*₆) δ 8.57 (brs, 3H), 8.26 (t, *J*= 13 Hz, 1H), 7.93 (d, *J* = 21 Hz, 1H), 7.61 (m, 1H), 7.49 (m, 3H), 7.42 (m, 2H), 4.54 (m, 1H), 4.02 (m, 2H), 3.88 (s, 3H), 3.25 (m, 5H), 3.16 (m, 2H), 2.64 (s, 3H), 2.42 (s, 3H), 2.33 (m, 2H), 1.68 (m, 2H), 1.62 (s, 3H); MS m/z [M+H]⁺ 686.3.

### [Preparation Example III']

ATBM-UBM compound in which a compound (JQ1) showing the activity of a bromodomain-containing protein 4 (BRD4) inhibitor is linked to compounds corresponding to Formula III of the present invention was synthesized as follows.

### [Preparation Example 4-2'] methyl (S)-2-(3-aminoprop-1-yn-1-yl)-5-(4-(2-(4-(4-chlorophenyl)-2,3,9-trimethyl-6H-thieno[3,2-f][1,2,4]triazolo[4,3-a][1,4]diazepin-6-yl)acetyl)piperazin-1-yl)benzoate hydrochloride

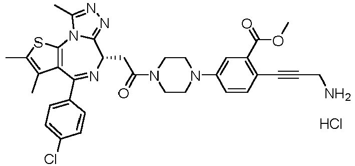

### Step 1: tert-butyl 4-(3-(methoxycarbonyl)phenyl)piperazine-1-carboxylate (Intermediate 4-2a)

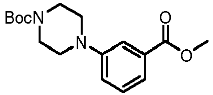

Intermediate 4-2a was synthesized in the same manner as in step 1 of Preparation Example 4-226' from methyl 3-iodobenzoate.

### Step 2: tert-butyl 4-(4-bromo-3-(methoxycarbonyl)phenyl)piperazine-1-carboxylate (Intermediate 4-2b)

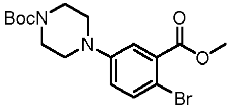

Intermediate 4-2a (2.0 mmol, 1.0 eq) was dissolved in dry DCM, and then NBS (1.2 eq) was added thereto, followed by stirring at room temperature for 30 minutes. The reactant was extracted with DCM and H₂O to obtain an organic layer. The obtained organic layer was dried over Na₂SO₄. The mixture was filtered under reduced pressure, distilled, and then purified through flash column to synthesize Intermediate 4-2b.

### Step 3: methyl 2-bromo-5-(piperazin-1-yl)benzoate hydrochloride (Intermediate 4-2c)

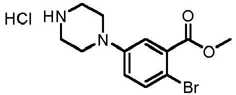

Intermediate 4-2c was synthesized in the same manner as in step 2 of Preparation Example 1-1 from Intermediate 4-2b.

### Step 4: methyl (S)-2-bromo-5-(4-(2-(4-(4-chlorophenyl)-2,3,9-trimethyl-6H-thieno[3,2-f][1,2,4]triazolo[4,3-a][1,4]diazepin-6-yl)acetyl)piperazin-1-yl)benzoate (Intermediate 4-2'a)

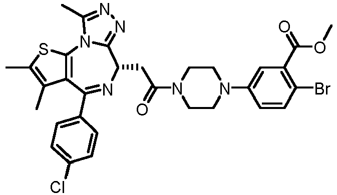

Intermediate 4-2'a was synthesized in the same manner as in step 5 of Preparation Example 4-1' from Intermediate 4-2c.

### Step 5: methyl (S)-2-(3-aminoprop-1-yn-1-yl)-5-(4-(2-(4-(4-chlorophenyl)-2,3,9-trimethyl-6H-thieno[3,2-f][1,2,4]triazolo[4,3-a][1,4]diazepin-6-yl)acetyl)piperazin-1-yl)benzoate hydrochloride

Example 4-2' was synthesized in the same manner as in steps 4 to 5 of Preparation Example 5-140' from Intermediate 4-2'a.

MS m/z [M+H]⁺ 656.41.

### [Preparation Example 4-226'] (S)-1-(4-(4-(3-aminoprop-1-yn-1-yl)phenyl)piperazin-1-yl)-2-(4-(4-chlorophenyl)-2,3,9-trimethyl-6H-thieno[3,2-f][1,2,4]triazolo[4,3-a][1,4]diazepin-6-yl)ethan-1-one hydrochloride

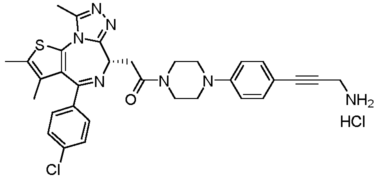

### Step 1: tert-butyl 4-phenylpiperazine-1-carboxylate (Intermediate 4-226a)

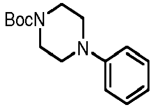

lodobenzene (1.4 mmol, 1.0 eq), *tert*-butyl piperazine-1-carboxylate (1.1 eq), Pd(OH)₂ (0.1 eq), BINAP (0.1 eq), CS₂CO₃ (2.0 eq), and dry toluene were mixed, and then a gas in a reactor was replaced with an Ar gas, followed by stirring at 100 °C overnight. The reactant was filtered through celite and then concentrated under reduced pressure, and purified through flash column to synthesize Intermediate 4-226a.

### Step 2: tert-butyl 4-(4-iodophenyl)piperazine-1-carboxylate (Intermediate 4-226b)

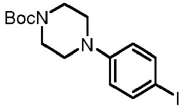

Intermediate 4-226a (2.0 mmol, 1.0 eq) was dissolved in dry DCM, and then NIS (1.2 eq) was added thereto, followed by stirring at room temperature for 30 minutes. The reactant was extracted with DCM and H₂O to obtain an organic layer. The obtained organic layer was dried over Na₂SO₄. The mixture was filtered under reduced pressure, distilled, and then purified through flash column to synthesize Intermediate 4-226b.

### Step 3: 1-(4-iodophenyl)piperazine hydrochloride (Intermediate 4-226c)

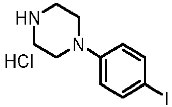

Intermediate 4-226c was synthesized in the same manner as in step 2 of Preparation Example 1-1 from Intermediate 4-226b.

### Step 4: (S)-2-(4-(4-chlorophenyl)-2,3,9-trimethyl-6H-thieno[3,2-f][1,2,4]triazolo[4,3-a][1,4]diazepin-6-yl)-1-(4-(4-iodophenyl)piperazin-1-yl)ethan-1-one (Intermediate 4-226'a)

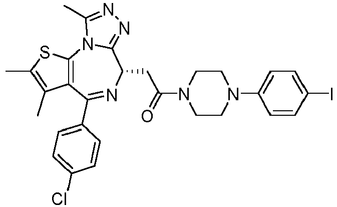

Intermediate 4-226'a was synthesized in the same manner as in step 5 of Preparation Example 4-1' from Intermediate 4-226c.

### Step 5: (S)-1-(4-(4-(3-aminoprop-1-yn-1-yl)phenyl)piperazin-1-yl)-2-(4-(4-chlorophenyl)-2,3,9-trimethyl-6H-thieno[3,2-f][1,2,4]triazolo[4,3-a][1,4]diazepin-6-yl)ethan-1-one hydrochloride

Example 4-226' was synthesized in the same manner as in Preparation Example 1-1 from Intermediate 4-226'a.

¹H NMR (500 MHz, DMSO-*d*₆) δ 8.34 - 8.12 (s, 3H), 7.48-7.39 (m, 4H), 7.33-7.25 (m, 1H), 7.03 - 6.98 (m, 1H), 6.97 - 6.94 (m, 1H), 4.59 (tt, *J* = 6.9, 2.6 Hz, 1H), 4.06 - 4.02 (m, 2H), 3.82 - 3.76 (m, 1H), 3.66 - 3.56 (m, 2H), 3.53 (s, 3H), 3.51 - 3.41 (m, 2H), 3.40 - 3.28 (m, 2H), 3.19 (d, *J* = 26.3 Hz, 2H), 2.60 (s, 3H), 2.38 (s, 3H), 1.59 (s, 3H); MS m/z [M+H]⁺ 598.45.

### [Preparation Example 4-227'] (S)-1-(4-(5-(3-aminoprop-1-yn-1-yl)furan-2-carbonyl)piperazin-1-yl)-2-(4-(4-chlorophenyl)-2,3,9-trimethyl-6H-thieno[3,2-f][1,2,4]triazolo[4,3-a][1,4]diazepin-6-yl)ethan-1-one hydrochloride

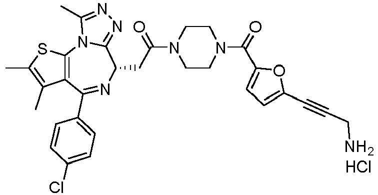

### Step 1: methyl 5-(3-((tert-butoxycarbonyl)amino)prop-1-yn-1-yl)furan-2-carboxylate (Intermediate 4-227a)

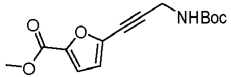

Intermediate 4-227a was synthesized in the same manner as in step 2 of Preparation Example 4-179' from methyl 5-bromo-2-furancarboxylate.

### Step 2: 5-(3-((tert-butoxycarbonyl)amino)prop-1-yn-1-yl)furan-2-carboxylic acid (Intermediate 4-227b)

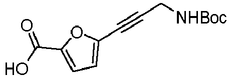

Intermedicate 4-227b was synthesized in the same manner as in step 1 of Preparation Example 3-2' from Intermediate 4-227a.

### Step 3: (S)-1-(4-(5-(3-aminoprop-1-yn-1-yl)furan-2-carbonyl)piperazin-1-yl)-2-(4-(4-chlorophenyl)-2,3,9-trimethyl-6H-thieno[3,2-f][1,2,4]triazolo[4,3-a][1,4]diazepin-6-yl)ethan-1-one hydrochloride

Example 4-227' was synthesized in the same manner as in Preparation Example 5-7' from Intermediate 5-140'b and Intermediate 4-227b.

¹H NMR (500 MHz, DMSO-*d*₆) δ 8.41 (s, 3H), 7.51 - 7.45 (m, 2H), 7.45 - 7.38 (m, 2H), 7.09 (d, *J* = 3.6 Hz, 1H), 7.01 (d, *J* = 3.6 Hz, 1H), 4.56 (t, *J* = 6.7 Hz, 1H), 4.06 (q, *J* = 5.7 Hz, 2H), 3.75 (d, *J* = 4.4 Hz, 2H), 3.66 - 3.62 (m, 4H), 3.43 - 3.36 (m, 4H), 2.57 (s, 3H), 2.38 (s, 3H), 1.59 (s, 3H); MS m/z [M+H]⁺ 616.33.

### [Preparation Example 5-7'] methyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetyl)piperazin-1-yl)benzoate hydrochloride

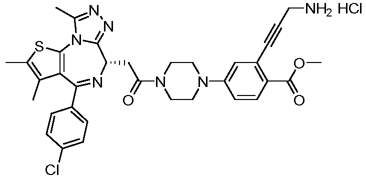

### Step 1: methyl (S)-2-(3-((tert-butoxycarbonyl)amino)prop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetyl)piperazin-1-yl)benzoate (Intermediate 5-7'a)

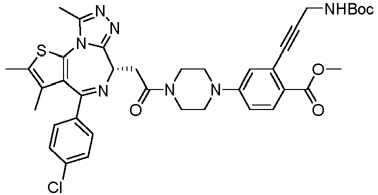

Intermediate 5-7'a was synthesized in the same manner as in step 5 of Preparation Example 4-1' from Intermediate 5-7b.

### Step 2: methyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetyl)piperazin-1-yl)benzoate hydrochloride

Example 5-7' was synthesized in the same manner as in step 2 of Preparation Example 1-1 from Intermediate 5-7'a.

¹H NMR (500 MHz, DMSO-*d*₆) δ 8.33 (s, 3H), 7.84 (d, *J* = 9.0 Hz, 1H), 7.48 (d, *J* = 8.8 Hz, 2H), 7.43 (d, *J* = 8.7 Hz, 2H), 7.10 - 7.07 (m, 1H), 7.03 (d, *J* = 2.6 Hz, 1H), 4.60 (t, *J* = 6.8 Hz, 1H), 4.04 (d, *J* = 5.7 Hz, 2H), 3.80 (s, 3H), 3.71 - 3.61 (m, 8H), 3.39 - 3.34 (m, 2H), 2.60 (d, *J* = 1.1 Hz, 3H), 2.42 (d, *J* = 0.9 Hz, 3H), 1.63 (d, *J* = 1.0 Hz, 3H); MS m/z [M+H]⁺ 656.42.

### [Preparation Example 5-22'] methyl (S)-2-(3-aminoprop-1-yn-1-yl)-4-((1-(2-(4-(4-chlorophenyl)-2,3,9-trimethyl-6H-thieno[3,2-f][1,2,4]triazolo[4,3-a][1,4]diazepin-6-yl)acetyl)piperidin-4-yl)carbamoyl)benzoate hydrochloride

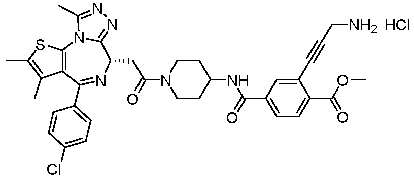

### Step 1: tert-butyl 4-(3-bromo-4-(methoxycarbonyl)benzamido)piperidine-1-carboxylate (Intermediate 5-22a)

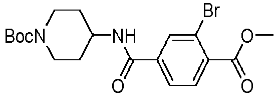

Intermediate 5-22a was synthesized in the same manner as in step 5 of Preparation Example 4-1' using 3-bromo-4-(methoxycarbonyl)benzoic acid (0.2 mmol, 1.0 eq) and *tert*-butyl 4-aminopiperidine-1-carboxylate as starting materials.

### Step 2: methyl 2-bromo-4-(piperidin-4-ylcarbamoyl)benzoate hydrochloride (Intermediate 5-22b)

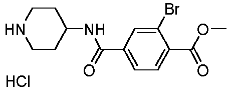

Intermediate 5-22b was synthesized in the same manner as in step 2 of Preparation Example 1-1 from Intermediate 5-22a.

### Step 3: methyl (S)-2-(3-aminoprop-1-yn-1-yl)-4-((1-(2-(4-(4-chlorophenyl)-2,3,9-trimethyl-6H-thieno[3,2-f][1,2,4]triazolo[4,3-a][1,4]diazepin-6-yl)acetyl)piperidin-4-yl)carbamoyl)benzoate hydrochloride

Example 5-22' was synthesized in the same manner as in steps 4 to 5 of Preparation Example 4-2' from Intermediate 5-22b via Intermediate 5-22'a (methyl (S)-2-bromo-4-((1-(2-(4-(4-chlorophenyl)-2,3,9-trimethyl-6H-thieno[3,2-f][1,2,4]triazolo[4,3-a][1,4]diazepin-6-yl)acetyl)piperidin-4-yl)carbamoyl)benzoate).

¹H NMR (500 MHz, DMSO-*d*₆) δ 8.75 (dd, *J* = 7.6, 3.6 Hz, 1H), 8.56 (s, 3H), 8.10 (dd, *J* = 5.2, 1.7 Hz, 1H), 8.04 - 7.96 (m, 2H), 7.53 - 7.50 (m, 2H), 7.46 (dd, *J* = 8.6, 4.8 Hz, 2H), 4.65 - 4.62 (m, 1H), 4.35 (d, *J* = 13.0 Hz, 1H), 4.17 (s, 1H), 4.06 (d, *J* = 5.7 Hz, 2H), 3.91 (d, *J* = 1.5 Hz, 3H), 3.68 (dd, *J* = 16.5, 7.5 Hz, 1H), 3.44 (dt, *J* = 16.4, 6.6 Hz, 1H), 3.28 (t, *J* = 12.6 Hz, 1H), 2.79 (q, *J* = 12.4, 12.0 Hz, 1H), 2.64 (s, 3H), 2.43 (d, *J* = 0.9 Hz, 3H), 1.96 (d, *J* = 12.5 Hz, 1H), 1.84 (s, 1H), 1.64 (s, 3H), 1.42 (td, *J* = 12.7, 11.5, 5.9 Hz, 1H); MS m/z [M+H]⁺ 698.50.

### [Preparation Example 5-23'] methyl (S)-2-(3-aminoprop-1-yn-1-yl)-4-(1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamido)benzoate hydrochloride

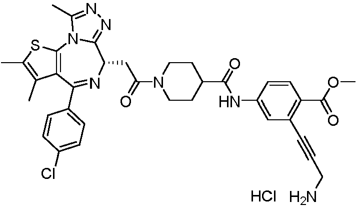

Example 5-23' was synthesized in the same manner as in Preparation Example 5-7' from Intermediate 5-25'b and Intermediate 3-25a.

¹H NMR (500 MHz, DMSO-*d*₆) δ 10.57 (d, *J* = 8.6 Hz, 1H), 8.41 (s, 3H), 8.17 (dd, *J* = 20.9, 2.2 Hz, 1H), 7.91 (dd, *J* = 8.7, 3.5 Hz, 1H), 7.61 (ddd, *J* = 24.0, 8.7, 2.2 Hz, 1H), 7.51 (d, *J* = 8.7 Hz, 2H), 7.45 (t, *J* = 8.9 Hz, 2H), 4.60 (t, *J* = 6.7 Hz, 1H), 4.42 (d, *J* = 3.7 Hz, 1H), 4.23 (d, *J* = 13.5 Hz, 1H), 4.06 (q, *J* = 5.6 Hz, 2H), 3.84 (d, *J* = 1.8 Hz, 3H), 3.64 (ddd, *J* = 20.5, 16.4, 7.4 Hz, 1H), 3.50 - 3.34 (m, 1H), 3.22 (q, *J* = 11.5 Hz, 1H), 2.71 (t, *J* = 12.5 Hz, 2H), 2.61 (s, 3H), 2.42 (s, 3H), 1.95 (d, *J* = 13.3 Hz, 1H), 1.84 (s, 1H), 1.70 (t, *J* = 11.0 Hz, 1H), 1.64 (s, 3H), 1.52 - 1.42 (m, 1H); MS m/z [M+H]⁺ 698.3.

### [Preparation Example 5-25'] methyl (S)-2-(3-aminoprop-1-yn-1-yl)-4-(3-(1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamido)propanamido)benzoate hydrochloride

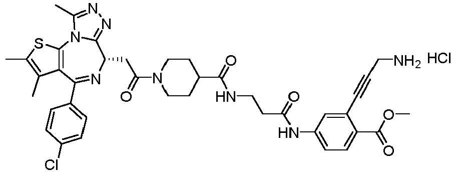

### Step 1: 3-(1,3-dioxoisoindolin-2-yl)propanoic acid (Intermediate 5-25a)

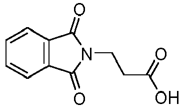

*β*-Alanine (2.0 mmol, 2.0 eq) and phthalic anhydride (1.0 eq) were dissolved in 0.6 mL of acetic acid, followed by stirring at 120 °C overnight. The reactant was extracted with DCM and H₂O to obtain an organic layer. The obtained organic layer was dried over Na₂SO₄. The mixture was filtered under reduced pressure, distilled, and then purified through flash column to synthesize Intermediate 5-25a.

### Step 2: methyl 2-(3-((tert-butoxycarbonyl)amino)prop-1-yn-1-yl)-4-(3-(1,3-dioxoisoindolin-2-yl)propanamido)benzoate (Intermediate 5-25b)

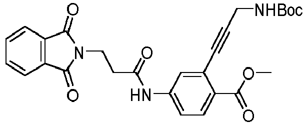

Intermediate 5-25b was synthesized in the same manner as in step 5 of Preparation Example 4-1' from Intermediate 5-25a and Intermediate 3-25a.

### Step 3: methyl 4-(3-aminopropanamido)-2-(3-((tert-butoxycarbonyl)amino)prop-1-yn-1-yl)benzoate (Intermediate 5-25c)

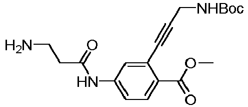

Intermediate 5-25c was synthesized in the same manner as in step 3 of Preparation Example 5-116 from Intermediate 5-25b.

### Step 4: methyl (S)-1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxylate (Intermediate 5-25'a)

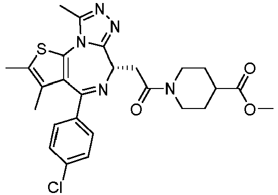

Intermediate 5-25'a was synthesized in the same manner as in step 5 of Preparation Example 4-1' from methyl piperidine-4-carboxylate.

### Step 5: (S)-1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxylic acid (Intermediate 5-25'b)

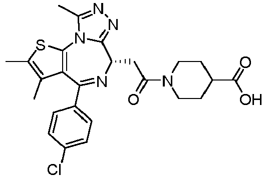

Intermediate 5-25'b was synthesized in the same manner as in step 1 of Preparation Example 3-2' from Intermediate 5- 25'a.

### Step 6: methyl (S)-2-(3-aminoprop-1-yn-1-yl)-4-(3-(1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamido)propanamido)benzoate hydrochloride

Example 5-25' was synthesized in the same manner as in Preparation Example 5-7' from Intermediate 5-25'b and Intermediate 5-25c.

¹H NMR (500 MHz, DMSO-*d*₆) δ 10.44 (s, 1H), 8.34 (s, 3H), 8.13 (t, *J* = 2.3 Hz, 1H), 8.00 (dd, *J* = 10.2, 5.4 Hz, 1H), 7.90 (d, *J* = 8.7 Hz, 1H), 7.56 (dd, *J* = 8.7, 2.2 Hz, 1H), 7.50 (d, *J* = 4.7 Hz, 2H), 7.43 (t, *J* = 7.3 Hz, 2H), 4.57 (t, *J* = 6.7 Hz, 1H), 4.33 (t, *J* = 14.5 Hz, 1H), 4.14 (s, 1H), 4.06 (t, *J* = 5.7 Hz, 2H), 3.83 (s, 3H), 3.62 (s, 1H), 3.58 (s, 2H), 3.34 (d, *J* = 6.6 Hz, 2H), 3.14 (s, 1H), 2.63 (dp, *J* = 5.5, 1.8 Hz, 2H), 2.60 (d, *J* = 2.0 Hz, 3H), 2.54 (d, *J* = 2.7 Hz, 1H), 2.42 (d, *J* = 0.8 Hz, 3H), 2.36 (p, *J* = 1.9 Hz, 1H), 2.00 (d, *J* = 7.6 Hz, 1H), 1.76 (d, *J* = 13.0 Hz, 1H), 1.68 (d, *J* = 4.8 Hz, 1H), 1.63 (d, *J* = 0.9 Hz, 3H); MS m/z [M+H]⁺ 769.43.

### [Preparation Example 5-26'] methyl (S)-2-(3-aminoprop-1-yn-1-yl)-4-(4-(1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamido)butanamido)benzoate hydrochloride

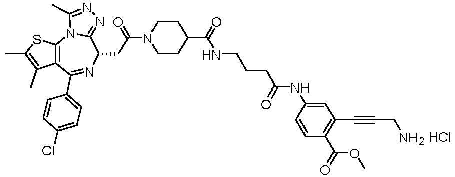

### Step 1: methyl 4-aminobutanoate (Intermediate 5-26a)

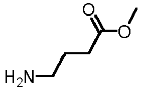

4-Aminobutanoic acid (14.0 mmol, 1.0 eq) was dissolved in 20.0 mL of MeOH, and then a gas in a reactor was replaced with an Ar gas. SOCl₂ (1.7 mL) was added dropwise at 0 °C to the reactant, followed by stirring at room temperature overnight. The reactant was distilled under reduced pressure, and then recrystallized with EtOAc to synthesize Intermediate 5-26a.

### Step 2: (S)-4-(1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamido)butanoic acid (Intermediate 5-26'b)

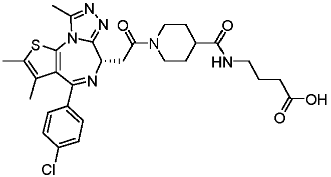

Intermediate Example 5-26'b synthesized in the same manner as in steps 4 to 5 of Preparation Example 5-25' from Intermediate 5-25'b and Intermediate 5-26a.

### Step 3: methyl (S)-2-(3-aminoprop-1-yn-1-yl)-4-(4-(1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamido)butanamido)benzoate hydrochloride

Example 5-26' was synthesized in the same manner as in Preparation Example 5-7' from Intermediate 5-26'b and Intermediate 3-25a.

¹H NMR (500 MHz, DMSO-*d*₆) δ 10.50 (s, 1H), 8.39 (s, 3H), 8.08 (s, 1H), 7.95 - 7.88 (m, 1H), 7.84 (d, *J* = 8.6 Hz, 1H), 7.59 - 7.53 (m, 1H), 7.50 - 7.38 (m, 4H), 4.59 - 4.52 (m, 1H), 4.30 (s, 2H), 4.02 (s, 2H), 3.77 (s, 3H), 3.63 - 3.47 (m, 1H), 3.43 - 3.30 (m, 1H), 3.16 - 3.01 (m, 4H), 2.62 - 2.56 (m, 3H), 2.37 (s, 3H), 1.67 (s, 4H), 1.57 (s, 3H), 1.32 (dd, *J* = 10.4, 6.7 Hz, 2H) 1.26 - 1.13 (m, 2H), 1.09 - 0.99 (m, 2H); MS m/z [M+H]⁺ 783.31.

### [Preparation Example 5-27'] methyl (S)-2-(3-aminoprop-1-yn-1-yl)-4-((3-(1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamido)propyl)carbamoyl)benzoate hydrochloride

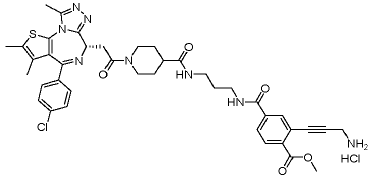

### Step 1: (S)-N-(3-aminopropyl)-1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamide trifluoroacetic acid (Intermediate 5-27'b)

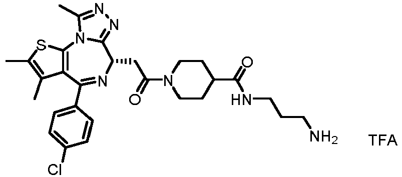

Intermediate 5-27'b was synthesized in the same manner as in steps 1 to 2 of Preparation Example 5-140' from Intermediate 5-25'b and tert-butyl (3-aminopropyl)carbamate.

### Step 2: methyl (S)-2-(3-aminoprop-1-yn-1-yl)-4-((3-(1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamido)propyl)carbamoyl)benzoate hydrochloride

Example 5-27' was synthesized in the same manner as in step 5 of Preparation Example 5-7' and Preparation Example 1-1 from Intermediate 5-27'b and 3-iodo-4-(methoxycarbonyl)benzoic acid.

¹H NMR (500 MHz, DMSO-*d*₆) δ 8.76 (d, *J* = 3.9 Hz, 1H), 8.37 (s, 3H), 8.02 (d, *J* = 1.9 Hz, 1H), 7.97 - 7.85 (m, 3H), 7.45 (d, *J* = 8.7 Hz, 2H), 7.40 (dd, *J* = 12.3, 8.5 Hz, 2H), 4.54 (t, *J* = 6.7 Hz, 1H), 4.32 (s, 1H), 4.11 (s, 1H), 4.02 (q, *J* = 5.6 Hz, 2H), 3.85 (s, 3H), 3.58 - 3.47 (m, 4H), 3.25 (d, *J* = 6.0 Hz, 2H), 3.12 - 3.00 (m, 4H), 2.56 (s, 3H), 2.38 (s, 3H), 1.77 - 1.71 (m, 1H), 1.63 (d, *J* = 3.7 Hz, 2H), 1.59 (s, 3H), 1.41 - 1.30 (m. 1H), 1.25 - 1.18 (m, 1H); MS m/z [M+H]⁺ 783.61.

### [Preparation Example 5-28'] methyl (S)-2-(3-aminoprop-1-yn-1-yl)-4-(3-(1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamido)prop-1-yn-1-yl)benzoate hydrochloride

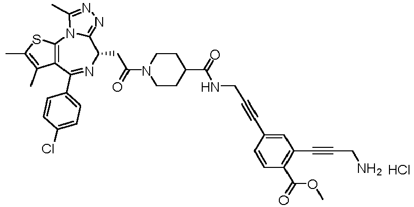

Example 5-28' was synthesized in the same manner as in Preparation Example 5-7' from Intermediate 5-25'b and Intermediate 5-116c.

¹H NMR (500 MHz, DMSO-*d*₆) δ 8.45 (s, 4H), 7.86 (dd, *J* = 8.2, 2.8 Hz, 1H), 7.57 (t, *J* = 2.0 Hz, 1H), 7.55 - 7.50 (m, 1H), 7.49 - 7.45 (m, 3H), 7.41 (dd, *J* = 12.6, 8.4 Hz, 1H), 4.57 (t, *J* = 6.7 Hz, 1H), 4.33 (t, *J* = 13.2 Hz, 1H), 4.13 (dd, *J* = 7.7, 5.6 Hz, 4H), 4.00 (t, *J* = 5.7 Hz, 4H), 3.83 (s, 4H), 3.62 - 3.37 (m, 3H), 3.13 (q, *J* = 11.1, 9.5 Hz, 1H), 2.59 (s, 3H), 2.40 - 2.38 (m, 3H), 1.59 (s, 3H); MS m/z [M+H]⁺ 736.46.

### [Preparation Example 5-29'] methyl (S)-2-(3-aminoprop-1-yn-1-yl)-4-(4-((1-(2-(4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperazin-1-yl)benzoate hydrochloride

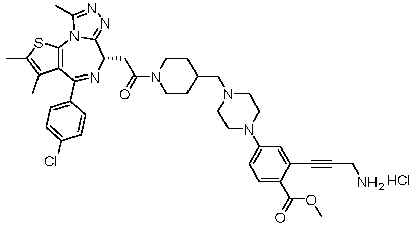

Example 5-29' was synthesized in the same manner as in Preparation Example 5-7' from JQ1 carboxylic acid and Intermediate 5-29d.

¹H NMR (500 MHz, DMSO-*d*₆) δ 9.46 (s, 1H), 8.31 (s, 3H), 7.85 (d, *J* = 8.9 Hz, 1H), 7.53 - 7.47 (m, 1H), 7.44 (dd, *J* = 8.5, 2.6 Hz, 2H), 7.15 (d, *J* = 9.0 Hz, 1H), 7.10 (s, 1H), 4.58 (t, *J* = 6.7 Hz, 1H), 4.38 (d, *J* = 12.7 Hz, 1H), 4.20 (s, 1H), 4.04 (dd, *J* = 10.6, 6.3 Hz, 1H), 3.81 (s, 2H), 3.65 (s, 2H), 2.61 - 2.59 (m, 3H), 2.44 - 2.40 (m, 3H), 1.88 (s, 1H), 1.76 (d, *J* = 12.0 Hz, 1H), 1.63 (s, 3H), 1.27 - 1.22 (m, 3H); MS m/z [M+H]⁺ 753.54.

### [Preparation Example 5-39'] methyl (S)-2-(3-aminoprop-1-yn-1-yl)-4-(2-(2-(4-(4-chlorophenyl)-2,3,9-trimethyl-6H-thieno[3,2-f][1,2,4]triazolo[4,3-a][1,4]diazepin-6-yl)acetyl)-2,7-diazaspiro[3.5]nonane-7-carbonyl)benzoate trifluoroacetic acid

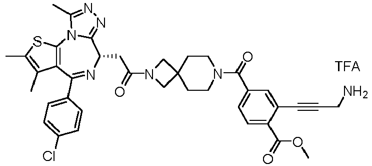

Example 5-39' was synthesized in the same manner as in steps 4 to 5 of Preparation Example 5-85' from Intermediate 5-39c.

¹H NMR (500 MHz, DMSO-*d*₆) δ 8.29 (s, 3H), 7.94 (d, *J* = 8.0 Hz, 1H), 7.56 (d, *J* = 1.7 Hz, 1H), 7.52 (dd, *J* = 8.0, 1.7 Hz, 1H), 7.45 (d, *J* = 9.6 Hz, 2H), 7.39 (s, 2H), 4.03 (s, 3H), 3.85 (s, 3H), 3.61 (s, 2H), 3.22 (t, *J* = 14.2 Hz, 4H), 3.10 (d, *J* = 21.5 Hz, 1H), 2.56 (s, 3H), 2.37 (s, 3H), 1.79 (s, 2H), 1.69 (s, 2H), 1.58 (s, 3H); MS m/z [M+H]⁺ 724.47.

### [Preparation Example 5-40'] methyl (S)-2-(3-aminoprop-1-yn-1-yl)-4-(6-(2-(4-(4-chlorophenyl)-2,3,9-trimethyl-6H-thieno[3,2-f][1,2,4]triazolo[4,3-a][1,4]diazepin-6-yl)acetyl)-2,6-diazaspiro[3.3]heptan-2-yl)benzoate trifluoroacetic acid

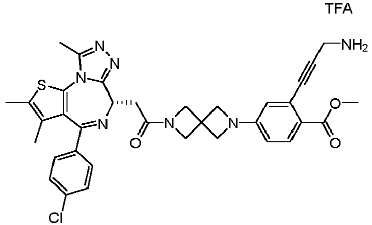

Example 5-40' was synthesized in the same manner as in steps 4 to 5 of Preparation Example 5-85' from Intermediate 5-40b.

¹H NMR (500 MHz, DMSO-*d*₆) δ 8.26 (s, 3H), 7.76 (s, 1H), 7.47 (d, *J* = 8.6 Hz, 2H), 7.39 (d, *J* = 8.2 Hz, 2H), 6.54 - 6.43 (m, 2H), 4.59 - 4.40 (m, 3H), 4.15 - 3.96 (m, 8H), 3.73 (s, 3H), 3.22 (dd, *J* = 15.5, 7.2 Hz, 1H), 3.09 (dd, *J* = 15.8, 7.0 Hz, 1H), 2.56 (s, 3H), 2.37 (s, 3H), 1.58 (s, 3H); MS m/z [M+H]⁺ 668.53.

### [Preparation Example 5-41'] methyl (S)-2-(3-aminoprop-1-yn-1-yl)-4-(6-(2-(4-(4-chlorophenyl)-2,3,9-trimethyl-6H-thieno[3,2-f][1,2,4]triazolo[4,3-a][1,4]diazepin-6-yl)acetyl)-2,6-diazaspiro[3.3]heptane-2-carbonyl)benzoate trifluoroacetic acid

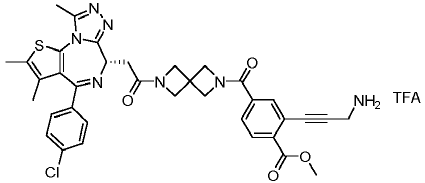

Example 5-41' was synthesized in the same manner as in steps 4 to 5 of Preparation Example 5-85' from Intermediate 5-41c.

¹H NMR (500 MHz, DMSO-*d*₆) δ 8.29 (s, 3H), 7.94 (dd, *J* = 8.3, 4.8 Hz, 1H), 7.80 (s, 1H), 7.73 (d, *J* = 8.2 Hz, 1H), 7.44 (dd, *J* = 18.8, 8.3 Hz, 2H), 7.41 - 7.36 (m, 2H), 4.55 - 4.40 (m, 3H), 4.25 (s, 8H), 4.07 - 4.00 (m, 3H), 3.85 (s, 3H), 3.22 - 3.13 (m, 1H), 3.13 - 3.03 (m, 1H), 2.56 (s, 3H), 2.37 (s, 3H), 1.57 (s, 3H); MS m/z [M+H]⁺ 696.60.

### [Preparation Example 5-44'] methyl 2-(3-aminoprop-1-yn-1-yl)-4-(5-(2-((S)-4-(4-chlorophenyl)-2,3,9-trimethyl-6H-thieno[3,2-f][1,2,4]triazolo[4,3-a][1,4]diazepin-6-yl)acetyl)hexahydropyrrolo[3,4-c]pyrrol-2(1H)-yl)benzoate trifluoroacetic acid

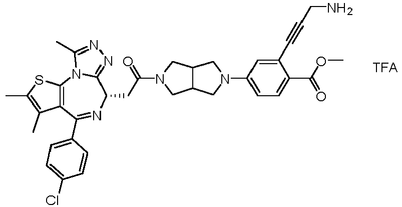

### Step 1: tert-butyl 5-(3-(3-(1,3-dioxoisoindolin-2-yl)prop-1-yn-1-yl)-4-(methoxycarbonyl)phenyl)hexahydropyrrolo[3,4-c]pyrrole-2(1H)-carboxylate (Intermediate 5-44a)

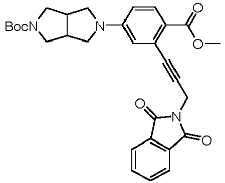

Intermediate 5-44a was synthesized in the same manner as in step 1 of Preparation Example 5- 168 using Intermediate 5-35a and *tert*-butyl hexahydropyrrolo[3,4-c]pyrrole-2(1H)-carboxylate as starting materials.

### Step 2: methyl 2-(3-(1,3-dioxoisoindolin-2-yl)prop-1-yn-1-yl)-4-(hexahydropyrrolo[3,4-c]pyrrol-2(1H)-yl)benzoate hydrochloride (Intermediate 5-44b)

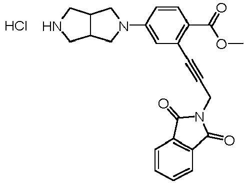

Intermediate 5-44b was synthesized in the same manner as in step 2 of Preparation Example 1-1 using Intermediate 5-44a as a starting material.

### Step 3: methyl 2-(3-aminoprop-1-yn-1-yl)-4-(5-(2-((S)-4-(4-chlorophenyl)-2,3,9-trimethyl-6H-thieno[3,2-f][1,2,4]triazolo[4,3-a][1,4]diazepin-6-yl)acetyl)hexahydropyrrolo[3,4-c]pyrrol-2(1H)-yl)benzoate trifluoroacetic acid

Example 5-44' was synthesized in the same manner as in steps 4 to 5 of Preparation Example 5-85' using Intermediate 5-44b as a starting material.

1H NMR (500 MHz, DMSO-*d*₆) δ 8.26 (s, 3H), 7.82 - 7.74 (m, 1H), 7.49 - 7.44 (m, 1H), 7.42 - 7.37 (m, 2H), 7.37 - 7.32 (m, 2H), 6.63 - 6.58 (m, 1H), 4.52 - 4.49 (m, 1H), 4.00 (s, 2H), 3.74 (d, *J* = 2.0 Hz, 3H), 3.58 (td, *J* = 13.3, 12.0, 6.4 Hz, 4H), 3.34 - 3.28 (m, 2H), 3.21 (h, *J* = 5.6 Hz, 2H), 3.15 (s, 1H), 3.03 (s, 1H), 2.55 (d, *J* = 1.0 Hz, 3H), 2.38 - 2.36 (m, 3H), 1.60 - 1.58 (m, 3H); MS m/z [M+H]⁺ 682.41.

### [Preparation Example 5-85'] methyl (S)-2-(3-aminoprop-1-yn-1-yl)-4-(((1-(2-(4-(4-chlorophenyl)-2,3,9-trimethyl-6H-thieno[3,2-f][1,2,4]triazolo[4,3-a][1,4]diazepin-6-yl)acetyl)piperidin-4-yl)methyl)amino)benzoate trifluoroacetic acid

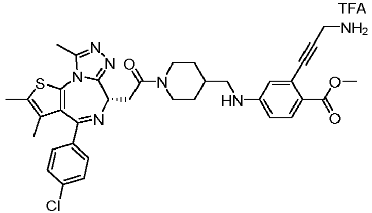

### Step 1: tert-butyl 4-(((3-iodo-4-(methoxycarbonyl)phenyl)amino)methyl)piperidine-1-carboxylate (Intermediate 5-85a)

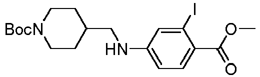

Methyl 4-amino-2-iodobenzoate (0.9 mmol, 1.0 eq) and boc-piperidine carboxaldehyde (1.2 eq) were dissolved in dry DCM, followed by stirring at room temperature for 15 minutes. Sodium triacetoxyborohydride (1.4 eq) was added thereto, and then the resulting mixture was stirred at room temperature for 15 hours. The reactant was extracted with DCM and H₂O to obtain an organic layer. The obtained organic layer was dried over Na₂SO₄. The mixture was filtered under reduced pressure, distilled, and then purified through flash column to synthesize Intermediate 5-85a.

### Step 2: tert-butyl 4-(((3-(3-(1,3-dioxoisoindolin-2-yl)prop-1-yn-1-yl)-4-(methoxycarbonyl)phenyl)amino)methyl)piperidine-1-carboxylate (Intermediate 5-85b)

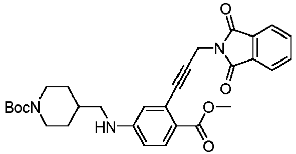

After Intermediate 5-85a (0.2 mmol, 1.0 eq), n-propargylphthalimide (1.5 eq), Pd(PPh₃)₄ (0.01 eq), Cul (0.03 eq), TEA (0.2 mL), and DMF (0.8 mL) were mixed, a gas in a reactor was replaced with an Ar gas, and then the resulting mixture was stirred at room temperature overnight. The reactant was extracted with DCM and H₂O to obtain an organic layer. The obtained organic layer was dried over Na₂SO₄. The mixture was filtered under reduced pressure, distilled, and then purified through flash column to synthesize Intermediate 5-85b.

### Step 3: methyl 2-(3-(1,3-dioxoisoindolin-2-yl)prop-1-yn-1-yl)-4-((piperidin-4-ylmethyl)amino)benzoate trifluoroacetic acid (Intermediate 5-85c)

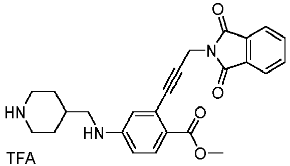

Intermediate 5-85c was synthesized in the same manner as in step 4 of Preparation Example 4-1' from Intermediate 5-85b.

### Step 4: methyl (S)-4-(((1-(2-(4-(4-chlorophenyl)-2,3,9-trimethyl-6H-thieno[3,2-f][1,2,4]triazolo[4,3-a][1,4]diazepin-6-yl)acetyl)piperidin-4-yl)methyl)amino)-2-(3-(1,3-dioxoisoindolin-2-yl)prop-1-yn-1-yl)benzoate (Intermediate 5-85'a)

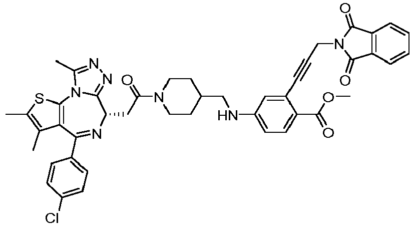

Intermediate 5-85'a was synthesized in the same manner as in step 5 of Preparation Example 4-1' from Intermediate 5-85c.

### Step 5: methyl (S)-2-(3-aminoprop-1-yn-1-yl)-4-(((1-(2-(4-(4-chlorophenyl)-2,3,9-trimethyl-6H-thieno[3,2-f][1,2,4]triazolo[4,3-a][1,4]diazepin-6-yl)acetyl)piperidin-4-yl)methyl)amino)benzoate trifluoroacetic acid

Intermediate 5-85'a (0.1 mmol, 1.0 eq), hydrazine monohydrate (3.0 eq), and EtOH (1.0 mL) were stirred at 40 °C for 16 hours. The reactant was distilled under reduced pressure, and then purified with prep HPLC to synthesize Example 5-85'.

¹H NMR (500 MHz, DMSO-*d*₆) δ 8.27 (s, 3H), 7.72 (dd, *J* = 8.7, 2.0 Hz, 1H), 7.49 (dd, *J* = 8.9, 1.8 Hz, 2H), 7.43 (dd, *J* = 8.6, 6.6 Hz, 2H), 6.82 (s, 1H), 6.71 - 6.65 (m, 2H), 4.58 (t, *J* = 6.7 Hz, 1H), 4.39 (d, *J* = 12.9 Hz, 1H), 4.18 (d, *J* = 13.6 Hz, 1H), 4.03 (q, *J* = 5.6 Hz, 2H), 3.75 (s, 3H), 3.69 - 3.58 (m, 2H), 3.12 (s, 2H), 3.00 (s, 2H), 2.63 (ddt, *J* = 7.5, 3.7, 1.9 Hz, 2H), 2.60 (d, *J* = 2.6 Hz, 3H), 2.42 (d, *J* = 1.0 Hz, 3H), 1.85 (s, 2H), 1.76 (s, 1H), 1.63 (s, 3H); MS m/z [M+H]⁺ 684.51.

### [Preparation Example 5-86'] methyl (S)-2-(3-aminoprop-1-yn-1-yl)-4-((1-(2-(4-(4-chlorophenyl)-2,3,9-trimethyl-6H-thieno[3,2-f][1,2,4]triazolo[4,3-a][1,4]diazepin-6-yl)acetyl)piperidin-4-yl)amino)benzoate

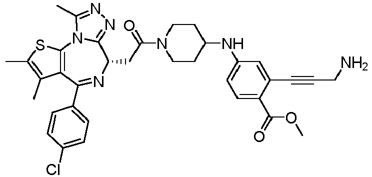

### Step 1: methyl (S)-4-((1-(2-(4-(4-chlorophenyl)-2,3,9-trimethyl-6H-thieno[3,2-f][1,2,4]triazolo[4,3-a][1,4]diazepin-6-yl)acetyl)piperidin-4-yl)amino)-2-(3-(1,3-dioxoisoindolin-2-yl)prop-1-yn-1-yl)benzoate (Intermediate 5-86'a)

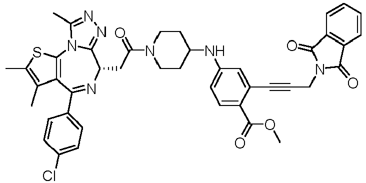

Intermediate 5-86'a was synthesized in the same manner as in step 5 of Preparation Example 4-1' from Intermediate 5-86c.

### Step 2: methyl (S)-2-(3-aminoprop-1-yn-1-yl)-4-((1-(2-(4-(4-chlorophenyl)-2,3,9-trimethyl-6H-thieno[3,2-f][1,2,4]triazolo[4,3-a][1,4]diazepin-6-yl)acetyl)piperidin-4-yl)amino)benzoate

Example 5-86' was synthesized in the same manner as in step 3 of Preparation Example 5-116 from Intermediate 5-86'a.

¹H NMR (500 MHz, DMSO-*d*₆) δ 10.48 (s, 1H), 8.36 (s, 3H), 8.28 (t, *J* = 5.7 Hz, 1H), 8.19 (d, *J=* 2.2 Hz, 1H), 7.71 (d, *J* = 8.8 Hz, 1H), 7.58 (d, *J* = 9.2 Hz, 1H), 7.39 (q, *J* = 8.8 Hz, 5H), 4.47 (dd, *J* = 8.1, 6.2 Hz, 1H), 4.04 (q, *J* = 5.6 Hz, 2H), 3.23 - 3.10 (m, 4H), 2.56 (s, 3H), 2.41 - 2.39 (m, 2H), 2.37 (d, *J* = 0.9 Hz, 3H), 1.74 (q, *J* = 7.2 Hz, 2H), 1.57 (d, *J* = 0.9 Hz, 3H); MS m/z [M+H]⁺ 670.53.

### [Preparation Example 5-139'] (S)-2-(3-aminoprop-1-yn-1-yl)-4-(((1-(2-(4-(4-chlorophenyl)-2,3,9-trimethyl-6H-thieno[3,2-f][1,2,4]triazolo[4,3-a][1,4]diazepin-6-yl)acetyl)piperidin-4-yl)methyl)amino)benzoic acid trifluoroacetic acid

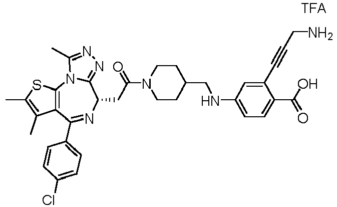

### Step 1: (S)-4-(((1-(2-(4-(4-chlorophenyl)-2,3,9-trimethyl-6H-thieno[3,2-f][1,2,4]triazolo[4,3-a][1,4]diazepin-6-yl)acetyl)piperidin-4-yl)methyl)amino)-2-(3-(1,3-dioxoisoindolin-2-yl)prop-1-yn-1-yl)benzoic acid (Intermediate 5-139'a)

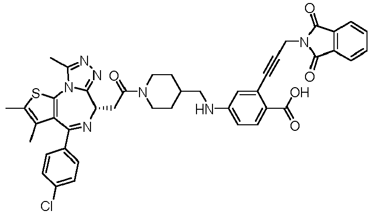

Intermediate 5-139'a was synthesized in the same manner as in step 1 of Preparation Example 3-2' from Intermediate 5-85'a.

### Step 2: (S)-2-(3-aminoprop-1-yn-1-yl)-4-(((1-(2-(4-(4-chlorophenyl)-2,3,9-trimethyl-6H-thieno[3,2-f][1,2,4]triazolo[4,3-a][1,4]diazepin-6-yl)acetyl)piperidin-4-yl)methyl)amino)benzoic acid trifluoroacetic acid

Example 5-139' was synthesized in the same manner as in step 5 of Preparation Example 5-85' from Intermediate 5-139'a.

¹H NMR (500 MHz, DMSO-*d*₆) δ 8.31 (s, 3H), 7.84 - 7.79 (m, 1H), 7.49 (dd, *J* = 8.7, 2.8 Hz, 3H), 7.43 (dd, *J* = 8.7, 7.1 Hz, 3H), 7.17 (s, 1H), 6.86 (dd, *J* = 8.8, 2.3 Hz, 1H), 6.67 (d, *J* = 2.1 Hz, 1H), 6.58 (s, 1H), 4.61 - 4.54 (m, 1H), 4.40 (d, *J* = 12.9 Hz, 1H), 4.19 (d, *J* = 12.9 Hz, 1H), 3.92 (d, *J* = 5.8 Hz, 2H), 3.66 - 3.61 (m, 2H), 3.09 (s, 3H), 2.62 (d, *J* = 1.8 Hz, 2H), 2.60 (d, *J* = 3.5 Hz, 3H), 2.42 (d, *J* = 1.0 Hz, 3H), 1.89 - 1.76 (m, 3H), 1.63 (d, *J* = 0.9 Hz, 3H); MS m/z [M+H]⁺ 670.53.

### [Preparation Example 5-140'] methyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetyl)piperazine-1-carbonyl)benzoate hydrochloride

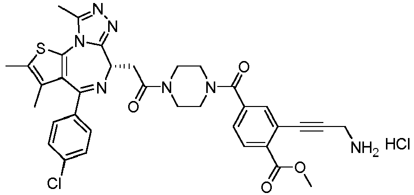

### Step 1: tert-butyl (S)-4-(2-(4-(4-chlorophenyl)-2,3,9-trimethyl-6H-thieno[3,2-f][1,2,4]triazolo[4,3-a][1,4]diazepin-6-yl)acetyl)piperazine-1-carboxylate (Intermediate 5-140'a)

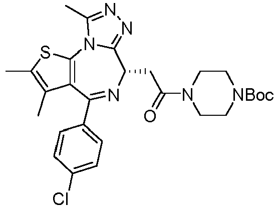

Intermediate 5-140'a was synthesized in the same manner as in step 5 of Preparation Example 4-1' from tert-butyl piperazine-1-carboxylate.

### Step 2: (S)-2-(4-(4-chlorophenyl)-2,3,9-trimethyl-6H-thieno[3,2-f][1,2,4]triazolo[4,3-a][1,4]diazepin-6-yl)-1-(piperazin-1-yl)ethan-1-one trifluoroacetic acid (Intermediate 5-140'b)

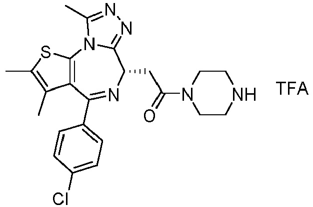

Example 5-140'b was synthesized in the same manner as in step 4 of Preparation Example 4-1' from Intermediate 5-140'a.

### Step 3: methyl (S)-2-bromo-4-(4-(2-(4-(4-chlorophenyl)-2,3,9-trimethyl-6H-thieno[3,2-f][1,2,4]triazolo[4,3-a][1,4]diazepin-6-yl)acetyl)piperazine-1-carbonyl)benzoate (Intermediate 5-140'c)

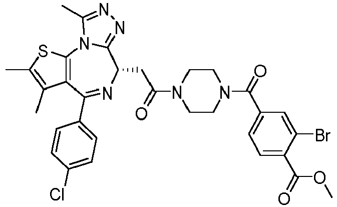

Intermediate 5-140'c was synthesized in the same manner as in step 5 of Preparation Example 4-1' from Intermediate 5-140'b and 3-bromo-4-(methoxycarbonyl)benzoic acid.

### Step 4: methyl (S)-2-(3-((tert-butoxycarbonyl)amino)prop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetyl)piperazine-1-carbonyl)benzoate (Intermediate 5-140'd)

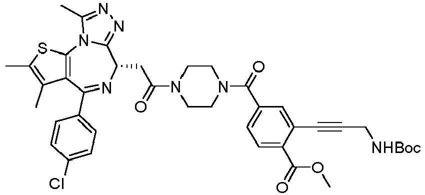

Intermediate 5-140'd was synthesized in the same manner as in step 2 of Preparation Example 4-179' from Intermediate 5-140'c.

### Step 5: methyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetyl)piperazine-1-carbonyl)benzoate hydrochloride

Example 5-140' was synthesized in the same manner as in step 2 of Preparation Example 1-1 from Intermediate 5-140'd.

¹H NMR (500 MHz, DMSO-*d*₆) δ 8.41 (s, 3H), 7.94 (s, 1H), 7.64 (s, 1H), 7.58 (d, *J* = 8.1 Hz, 1H), 7.46 (d, *J* = 8.3 Hz, 2H), 7.40 (d, *J* = 8.1 Hz, 2H), 4.56 (t, *J* = 6.7 Hz, 1H), 4.06 - 4.00 (m, 2H), 3.86 (s, 3H), 3.81 - 3.68 (m, 4H), 3.54 - 3.43 (m, 5H), 3.27 - 3.19 (m, 1H), 2.57 (s, 3H), 2.38 (s, 3H), 1.59 (s, 3H); MS m/z [M+H]⁺ 684.51.

### [Preparation Example 5-152'] methyl (S)-2-(3-aminoprop-1-yn-1-yl)-4-(1-(2-(4-(4-chlorophenyl)-2,3,9-trimethyl-6H-thieno[3,2-f][1,2,4]triazolo[4,3-a][1,4]diazepin-6-yl)acetyl)-N-methylpiperidine-4-carboxamido)benzoate trifluoroacetic acid

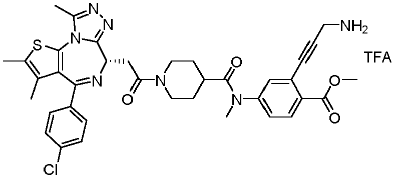

### Step 1: methyl (S)-4-(1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamido)-2-(3-(1,3-dioxoisoindolin-2-yl)prop-1-yn-1-yl)benzoate (Intermediate 5-152'a)

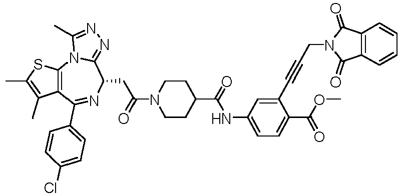

Example 5-23' (0.04 mmol, 1.0 eq) and N-hydroxyphthalimide (1.0 eq) were dissolved in 2.0 mL of 0.1 M phosphate buffer (pH 7.0), followed by stirring at room temperature for 16 hours. The reactant was extracted with DCM and H₂O to obtain an organic layer. The obtained organic layer was dried over Na₂SO₄. The mixture was filtered under reduced pressure, distilled, and then purified through flash column to synthesize Intermediate 5-152'a.

### Step 2: methyl (S)-4-(1-(2-(4-(4-chlorophenyl)-2,3,9-trimethyl-6H-thieno[3,2-f][1,2,4]triazolo[4,3-a][1,4]diazepin-6-yl)acetyl)-N-methylpiperidine-4-carboxamido)-2-(3-(1,3-dioxoisoindolin-2-yl)prop-1-yn-1-yl)benzoate (Intermediate 5-152'b)

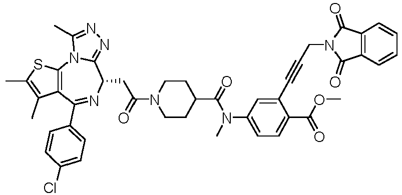

Intermediate 5-152'b was synthesized in the same manner as in step 1 of Preparation Example 5-153' from Intermediate 5-152'a.

### Step 3: methyl (S)-2-(3-aminoprop-1-yn-1-yl)-4-(1-(2-(4-(4-chlorophenyl)-2,3,9-trimethyl-6H-thieno[3,2-f][1,2,4]triazolo[4,3-a][1,4]diazepin-6-yl)acetyl)-N-methylpiperidine-4-carboxamido)benzoate trifluoroacetic acid

Example 5-152' was synthesized in the same manner as in step 5 of Preparation Example 5-85' from Intermediate 5-152'b.

¹H NMR (500 MHz, DMSO-*d*₆) δ 7.96 (s, 1H), 7.57 (s, 1H), 7.52 - 7.44 (m, 4H), 7.39 (s, 1H), 4.57 - 4.51 (m, 1H), 4.32 - 4.31 (m, 1H), 4.09 (s, 2H), 3.87 (s, 3H), 3.72 - 3.56 (m, 4H), 3.44 (s, 3H), 2.58 (s, 3H), 2.39 (s, 3H), 1.77 - 1.67 (m, 1H), 1.60 (s, 3H), 1.42 - 1.31 (m, 1H), 1.00 - 0.95 (m, 1H), 0.89 - 0.80 (s, 1H); MS m/z [M+H]⁺ 712.58.

### [Preparation Example 5-153'] methyl (S)-2-(3-aminoprop-1-yn-1-yl)-4-((1-(2-(4-(4-chlorophenyl)-2,3,9-trimethyl-6H-thieno[3,2-f][1,2,4]triazolo[4,3-a][1,4]diazepin-6-yl)acetyl)piperidin-4-yl)(methyl)carbamoyl)benzoate trifluoroacetic acid

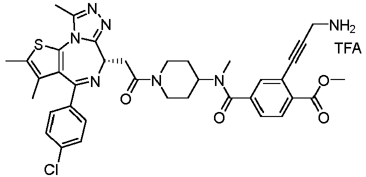

### Step 1: methyl (S)-2-bromo-4-((1-(2-(4-(4-chlorophenyl)-2,3,9-trimethyl-6H-thieno[3,2-f][1,2,4]triazolo[4,3-a][1,4]diazepin-6-yl)acetyl)piperidin-4-yl)(methyl)carbamoyl)benzoate (Intermediate 5-153'a)

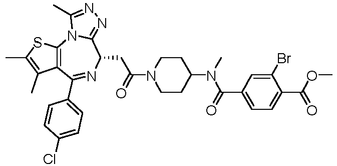

Intermediate 5-22'a (0.06 mmol, 1.0 eq) was dissolved in 0.5 mL of dry DMF, and then NaH (1.3 eq) and Mel (5.0 eq) were added thereto, followed by stirring at room temperature for 2 hours. The reactant was extracted with EtOAc and H₂O to obtain an organic layer. The obtained organic layer was dried over Na₂SO₄. The mixture was filtered under reduced pressure, distilled, and then purified through flash column to synthesize Intermediate 5-153'a.

### Step 2: methyl (S)-2-(3-((tert-butoxycarbonyl)amino)prop-1-yn-1-yl)-4-((1-(2-(4-(4-chlorophenyl)-2,3,9-trimethyl-6H-thieno[3,2-f][1,2,4]triazolo[4,3-a][1,4]diazepin-6-yl)acetyl)piperidin-4-yl)(methyl)carbamoyl)benzoate (Intermediate 5-153'b)

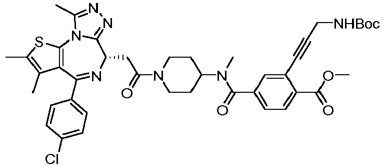

Intermediate 5-153'b was synthesized in the same manner as in step 2 of Preparation Example 4-179' from Intermediate 5-153'a.

### Step 3: methyl (S)-2-(3-aminoprop-1-yn-1-yl)-4-((1-(2-(4-(4-chlorophenyl)-2,3,9-trimethyl-6H-thieno[3,2-f][1,2,4]triazolo[4,3-a][1,4]diazepin-6-yl)acetyl)piperidin-4-yl)(methyl)carbamoyl)benzoate trifluoroacetic acid

Intermediate 5-153'b (0.5 mmol, 1.0 eq) was dissolved in 3.0 mL of DCM, and then a HCl solution (4.0 M in dioxane, excess) was added dropwise thereto, followed by stirring at room temperature for 2 hours. The reactant was distilled under reduced pressure, and then purified through prep HPLC to synthesize Example 5-153'.

¹H NMR (500 MHz, DMSO-*d*₆) δ 8.30 (s, 3H), 7.98 (d, *J* = 8.1 Hz, 1H), 7.62 - 7.55 (m, 2H), 7.52 - 7.40 (m, 5H), 4.58 (s,2H), 4.07 (d, *J* = 5.6 Hz, 2H), 3.89 (s, 3H), 3.68 (d, *J* = 9.6 Hz, 2H), 3.51 (s, 3H), 2.89 (d, *J* = 13.5 Hz, 2H), 2.75 (s, 2H), 2.60 (s, 3H), 2.42 (s, 3H), 1.63 (s, 3H); MS m/z [M+H]⁺ 712.48.

### [Preparation Example 5-154'] methyl (S)-4-(1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamido)-2-(3-(sulfamoylamino)prop-1-yn-1-yl)benzoate trifluoroacetic acid

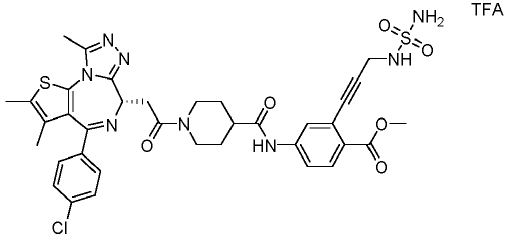

Sulfamoyl chloride (0.048 mmol, 1.1 eq) was dissolved in 0.2 mL of DCM and added dropwise at 0 °C to a mixed solution of Example 5-23' (0.043 mmol, 1.0 eq), TEA (2.5 eq), and DCM (0.8 mL), and then the resulting mixture was stirred at room temperature overnight. The reactant was extracted with EtOAc and H₂O to obtain an organic layer. The obtained organic layer was dried over Na₂SO₄. The mixture was filtered under reduced pressure, distilled, and then purified with prep HPLC to synthesize Example 5-154'.

¹H NMR (500 MHz, DMSO-*d*₆) δ 10.36 (d, *J* = 15.9 Hz, 1H), 7.95 (d, J=2.1 Hz, 1H), 7.87 (dd, *J* = 8.7, 3.7 Hz, 1H), 7.68 - 7.60 (m, 1H), 7.51 - 7.43 (m, 4H), 7.14 - 7.11 (m, 1H), 6.67 (s, 3H), 4.59 (t, *J* = 6.7 Hz, 1H), 4.45 - 4.39 (m, 1H), 4.25 - 4.20 (m, 1H), 3.97 (d, *J* = 5.7 Hz, 2H), 3.82 (s, 3H), 3.65 - 3.56 (m, 1H), 3.22 - 3.20 (m, 1H), 2.73 - 2.62 (m, 3H), 2.60 (s, 3H), 2.42 (s, 3H), 1.96 - 1.92 (m, 1H), 1.87 - 1.81 (m, 1H), 1.73 - 1.66 (m, 1H), 1.64 (s, 3H), 1.47 (t, *J* = 14.9 Hz, 1H), MS m/z [M+H]⁺ 777.32,

### [Preparation Example 5-155'] methyl (S)-2-((1-aminocyclopropyl)ethynyl)-4-(1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamido)benzoate trifluoroacetic acid

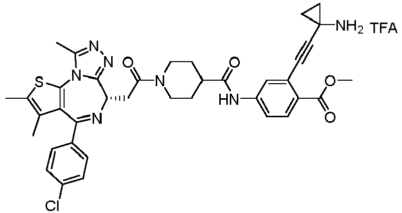

### Step 1: methyl 4-amino-2-((1-((tert-butoxycarbonyl)amino)cyclopropyl)ethynyl)benzoate (Intermediate 5-155a)

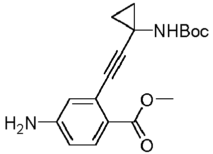

Intermedicate 5-155a was synthesized in the same manner as in Preparation Example 1-1 from methyl 4-amino-2-iodobenzoate and tert-butyl (1-ethynylcyclopropyl)carbamate.

### Step 2: methyl (S)-2-((1-((tert-butoxycarbonyl)amino)cyclopropyl)ethynyl)-4-(1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamido)benzoate (Intermediate 5-155'a)

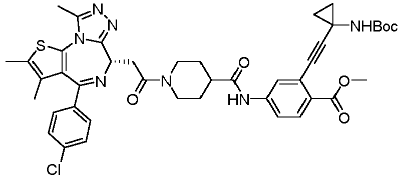

Intermediate 5-155'a was synthesized in the same manner as in step 5 of Preparation Example 4-1' from Intermediate 5-25'b and Intermediate 5-155a.

### Step 3: methyl (S)-2-((1-aminocyclopropyl)ethynyl)-4-(1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamido)benzoate trifluoroacetic acid

Example 5-155' was synthesized in the same manner as in step 3 of Preparation Example 5-153' from Intermediate 5-155'a.

¹H NMR (500 MHz, DMSO-*d*₆) δ 10.43 (d, *J* = 10.9 Hz, 1H), 8.77 (s, 2H), 8.14 (dd, *J* = 22.6, 2.2 Hz, 1H), 7.92 (dd, *J* = 8.7, 3.5 Hz, 1H), 7.59 - 7.48 (m, 3H), 7.45 (t, *J* = 8.6 Hz, 2H), 4.60 (d, *J* = 6.8 Hz, 1H), 3.82 (d, *J* = 2.3 Hz, 3H), 3.61 (dd, *J* = 16.3, 7.5 Hz, 1H), 3.44 - 3.36 (m, 1H), 3.22 (q, *J* = 11.2 Hz, 1H), 2.69 (t, *J* = 12.1 Hz, 2H), 2.61 (s, 3H), 2.42 (d, *J* = 1.0 Hz, 3H), 1.94 (d, *J* = 12.6 Hz, 1H), 1.83 (s, 1H), 1.74 - 1.66 (m, 1H), 1.64 (s, 3H), 1.48 (d, *J* = 15.8 Hz, 1H), 1.42 - 1.38 (m, 2H), 1.36 - 1.33 (m, 2H), 1.26 - 1.21 (m, 2H); MS m/z [M+H]⁺ 724.47.

### [Preparation Example 5-157'] methyl 4-(1-(2-((S)-4-(4-chlorophenyl)-2,3,9-trimethyl-6H-thieno[3,2-f][1,2,4]triazolo[4,3-a][1,4]diazepin-6-yl)acetyl)piperidine-4-carboxamido)-2-(3-(((1-(isobutyryloxy)ethoxy)carbonyl)amino)prop-1-yn-1-yl)benzoate

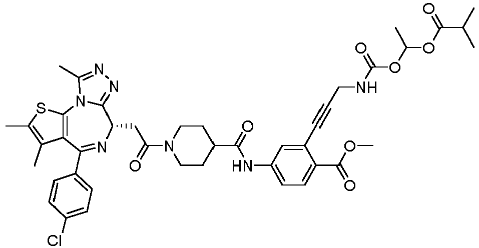

1-(((4-nitrophenoxy)carbonyl)oxy)ethyl isobutyrate (1.0 eq) was added at 0 °C to a mixed solution of Example 5-23' (0.027 mmol, 1.0 eq), HOBt (1.0 eq), DIPEA (3.0 eq), and DMF (0.5 mL), followed by stirring for 20 minutes. The reactant was filtered under reduced pressure, purified through flash column, and further purified with prep HPLC to synthesize Example 5-157'.

¹H NMR (500 MHz, DMSO-*d*₆) δ 10.33 (d, *J* = 16.3 Hz, 1H), 8.07 - 8.01 (m, 1H), 7.93 (dd, *J* = 6.5, 2.2 Hz, 1H), 7.86 (dd, *J* = 8.7, 3.6 Hz, 1H), 7.63 (dt, *J* = 8.7, 2.5 Hz, 1H), 7.50 (d, *J* = 8.4 Hz, 2H), 7.48 - 7.41 (m, 2H), 6.70 (q, *J* = 5.4 Hz, 1H), 4.59 (t, *J* = 6.7 Hz, 1H), 4.46 - 4.38 (m, 1H), 4.27 - 4.19 (m, 1H), 4.13 - 4.02 (m, 2H), 3.80 (d, *J* = 1.2 Hz, 3H), 3.22 (q, *J* = 11.6 Hz, 1H), 2.75 - 2.64 (m, 2H), 2.61 (s, 3H), 2.42 (s, 3H), 1.97 - 1.91 (m, 1H), 1.87 - 1.80 (m, 1H), 1.74 - 1.67 (m, 1H), 1.64 (s, 3H), 1.52 - 1.44 (m, 1H), 1.41 (d, *J* = 5.4 Hz, 3H), 1.06 (dd, *J* = 7.0, 4.5 Hz, 6H); MS m/z [M+H]⁺ 856.44

### [Preparation Example 5-158'] methyl (S)-4-(1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamido)-2-(3-(sulfamoyloxy)prop-1-yn-1-yl)benzoate trifluoroacetic acid

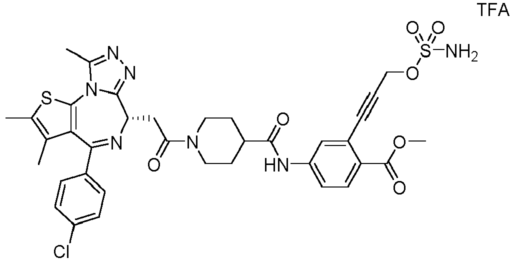

### Step 1: tert-butyl 4-((3-iodo-4-(methoxycarbonyl)phenyl)carbamoyl)piperidine-1-carboxylate (Intermediate 5-158'a)

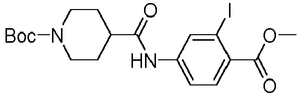

Intermediate 5-158'a was synthesized in the same manner as in step 5 of Preparation Example 4-1' using 1-(*tert*-butoxycarbonyl)piperidine-4-carboxylic acid and methyl 4-amino-2-iodobenzoate as starting materials.

### Step 2: methyl 2-iodo-4-(piperidine-4-carboxamido)benzoate dihydrochloride (Intermediate 5-158'b)

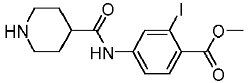

Intermediate 5-158'b was synthesized in the same manner as in step 2 of Preparation Example 1-1 from Intermediate 5-158'a.

### Step 3: methyl (S)-4-(1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamido)-2-iodobenzoate (Intermediate 5-158'c)

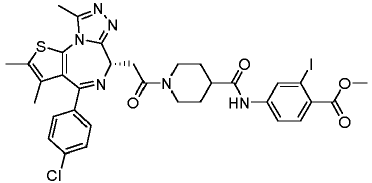

Intermediate 5-158'c was synthesized in the same manner as in step 5 of Preparation Example 4-1' from JQ1 carboxylic acid and Intermediate 5-158'b.

### Step 4: methyl (S)-4-(1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamido)-2-(3-hydroxyprop-1-yn-1-yl)benzoate (Intermediate 5-158'd)

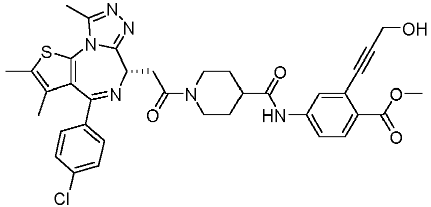

After Intermediate 5-158'c (0.3 mmol, 1 eq), propargylalcohol (3.0 eq), Pd(PPh₃)₄ (0.02 eq), Cul (0.1 eq), TEA (1.0 mL), and DMF (1.0 mL) were mixed, a gas in a reactor was replaced with an Ar gas, and then the resulting mixture was stirred at room temperature overnight. The mixture was purified through flash column to synthesize Intermediate 5-158'd.

### Step 5: methyl (S)-4-(1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamido)-2-(3-(sulfamoyloxy)prop-1-yn-1-yl)benzoate trifluoroacetic acid

Example 5-158' was synthesized in the same manner as in Preparation Example 5-154' from Intermediate 5-158'd.

¹H NMR (500 MHz, DMSO-*d*₆) δ 10.40 (d, *J* = 16.9 Hz, 1H), 8.00 (dd, *J* = 7.6, 2.2 Hz, 1H), 7.91 (dd, *J* = 8.7, 3.8 Hz, 1H), 7.72 (s, 2H), 7.69 (dd, *J* = 8.5, 2.5 Hz, 1H), 7.53 - 7.42 (m, 4H), 7.28 (s, 1H), 6.69 (s, 1H), 5.02 (s, 2H), 4.59 (t, *J* = 6.7 Hz, 1H), 4.42 (s, 1H), 4.23 (s, 1H), 3.82 (d, *J* = 1.1 Hz, 3H), 3.67 - 3.54 (m, 1H), 2.68 (s, 2H), 2.60 (s, 3H), 2.42 (s, 3H), 1.91 (s, 1H), 1.84 (s, 1H), 1.70 (d, *J* = 13.0 Hz, 1H), 1.64 (s, 3H), 1.47 (d, *J* = 6.3 Hz, 1H).

MS m/z [M+H]⁺ 778.42.

### [Preparation Example 5-162'] methyl 4-(6-(2-((S)-4-(4-chlorophenyl)-2,3,9-trimethyl-6H-thieno[3,2-f][1,2,4]triazolo[4,3-a][1,4]diazepin-6-yl)acetyl)-2,6-diazaspiro[3.3]heptan-2-yl)-2-(3-(((1-(isobutyryloxy)ethoxy)carbonyl)amino)prop-1-yn-1-yl)benzoate

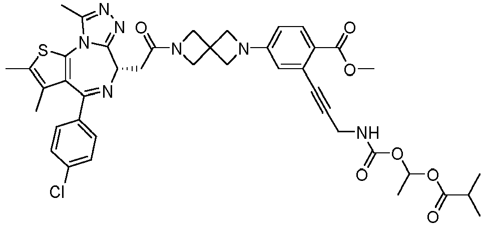

Example 5-162' was synthesized in the same manner as Preparation Example 5-157' from Example 5-40'.

¹H NMR (500 MHz, DMSO-*d*₆) δ 7.98 (t, *J* = 5.8 Hz, 1H), 7.72 (d, *J* = 8.5 Hz, 1H), 7.50 - 7.45 (m, 2H), 7.42 - 7.37 (m, 2H), 6.66 (q, *J* = 5.4 Hz, 1H), 6.46 - 6.41 (m, 2H), 4.57 - 4.47 (m, 2H), 4.45 (t, *J* = 7.1 Hz, 1H), 4.11 (d, *J* = 3.1 Hz, 2H), 4.09 - 4.06 (m, 3H), 4.05 - 4.03 (m, 3H), 3.70 (s, 3H), 3.21 (dd, *J* = 15.5, 6.9 Hz, 1H), 3.11 (dd, *J* = 15.5, 7.2 Hz, 1H), 2.57 (s, 3H), 2.37 (d, *J* = 1.1 Hz, 3H), 1.59 (d, *J* = 1.0 Hz, 3H), 1.37 (d, *J* = 5.4 Hz, 3H), 1.03 (dd, *J* = 7.0, 3.9 Hz, 6H).

### [Preparation Example 5-168'] methyl (S)-2-(3-aminoprop-1-yn-1-yl)-4-(7-(2-(4-(4-chlorophenyl)-2,3,9-trimethyl-6H-thieno[3,2-f][1,2,4]triazolo[4,3-a][1,4]diazepin-6-yl)acetyl)-2,7-diazaspiro[3.5]nonan-2-yl)benzoate hydrochloride

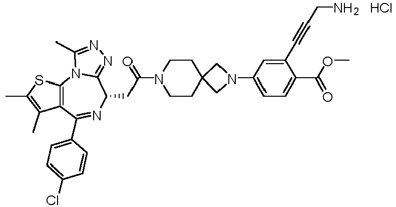

Preparation Example 5-168' was synthesized in the same manner as in steps 4 to 5 of Preparation Example 5-85' from JQ1 carboxylic acid and Intermediate 5-168b.

¹H NMR (400 MHz, DMSO-*d*₆) δ 7.76 (d, *J* = 2.3 Hz, 1H), 7.49 (s, 2H), 7.45 (s, 2H), 6.45 (s, 2H), 4.58 (t, *J* = 1.7 Hz, 1H), 3.74 (m, 6H), 3.64 (m, 5H), 3.48 (m, 2H), 3.28 (m, 2H), 2.60 (s, 3H), 2.42 (s, 3H), 1.88 (s, 2H), 1.70 (s, 2H), 1.63 (s, 3H); MS m/z [M+H]⁺ 696.2.

### [Preparation Example IV']

A TBM-UBM compound in which a compound (JQ1) showing the activity of a bromodomain-containing protein 4 (BRD4) inhibitor is linked to compounds corresponding to Formula IV of the present invention was synthesized as follows.

### [Preparation Example 5-32'] methyl (S)-2-(3-aminoprop-1-yn-1-yl)-4-(4-(3-(2-(4-(4-chlorophenyl)-2,3,9-trimethyl-6H-thieno[3,2-f][1,2,4]triazolo[4,3-a][1,4]diazepin-6-yl)acetamido)propanoyl)piperazin-1-yl)benzoate hydrochloride

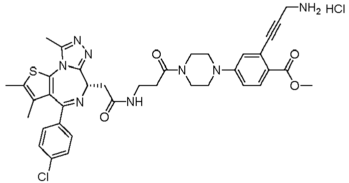

Example 5-32' was synthesized in the same manner as in Preparation Example 5-7' from Intermediate 4-124'a and Intermediate 5-7b.

¹H NMR (500 MHz, DMSO-*d*₆) δ 8.34 (s, 3H), 8.24 (t, *J=* 5.8 Hz, 1H), 7.78 (d, *J* = 8.9 Hz, 1H), 7.46 - 7.43 (m, 2H), 7.39 (d, *J* = 8.6 Hz, 2H), 7.00 (dd, *J* = 9.0, 2.7 Hz, 1H), 6.97 (d, *J* = 2.6 Hz, 1H), 4.47 (t, *J* = 7.1 Hz, 1H), 3.98 (q, *J* = 5.6 Hz, 2H), 3.75 (s, 3H), 3.59 - 3.54 (m, 3H), 3.39 - 3.26 (m, 9H), 3.19 (d, *J* = 7.2 Hz, 2H), 2.55 (s, 3H), 2.37 (s, 3H), 1.58 (s, 3H); MS m/z [M+H]⁺ 727.47.

### [Preparation Example 5-33'] methyl (S)-2-(3-aminoprop-1-yn-1-yl)-5-(4-(3-(2-(4-(4-chlorophenyl)-2,3,9-trimethyl-6H-thieno[3,2-f][1,2,4]triazolo[4,3-a][1,4]diazepin-6-yl)acetamido)propanoyl)piperazin-1-yl)benzoate hydrochloride

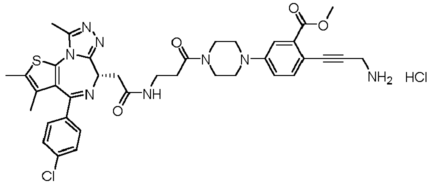

### Step 1: methyl 5-bromo-2-(3-((tert-butoxycarbonyl)amino)prop-1-yn-1-yl)benzoate (Intermediate 5-33a)

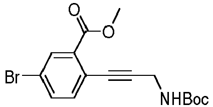

Intermediate 5-33a was synthesized in the same manner as in step 1 of Preparation Example 1-1 from methyl 5-bromo-2-iodobenzoate.

### Step 2: methyl 2-(3-((tert-butoxycarbonyl)amino)prop-1-yn-1-yl)-5-(piperazin-1-yl)benzoate (Intermediate 5-33b)

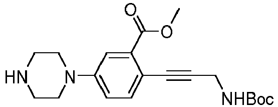

Intermediate 5-33b was synthesized in the same manner as in step 1 of Preparation Example 5-40 from Intermediate 5-33a and piperazine.

### Step 3: methyl (S)-2-(3-aminoprop-1-yn-1-yl)-5-(4-(3-(2-(4-(4-chlorophenyl)-2,3,9-trimethyl-6H-thieno[3,2-f][1,2,4]triazolo[4,3-a][1,4]diazepin-6-yl)acetamido)propanoyl)piperazin-1-yl)benzoate hydrochloride

Example 5-33' was synthesized in the same manner as in Preparation Example 1-1' from Intermediate 4-124'a and Intermediate 5-33b.

¹H NMR (500 MHz, DMSO-*d*₆) δ 8.26 (d, *J* = 26.6 Hz, 3H), 7.45 (dd, *J* = 8.9, 1.7 Hz, 2H), 7.43 - 7.36 (m, 3H), 7.29 (d, *J* = 2.8 Hz, 1H), 7.13 (dd, *J* = 8.8, 2.6 Hz, 1H), 7.07 (s, 1H), 4.48 (t, *J* = 7.1 Hz, 1H), 3.95 (q, *J* = 5.6 Hz, 1H), 3.81 (s, 3H), 3.36 - 3.27 (m, 5H), 3.27 - 3.18 (m, 5H), 2.56 (d, *J* = 1.3 Hz, 3H), 2.51 (q, *J* = 6.4, 6.0 Hz, 3H), 2.37 (s, 3H), 1.58 (s, 3H); MS m/z [M+H]⁺ 727.47.

### [Preparation Example 5-111'] (S)-5-(4-(3-aminoprop-1-yn-1-yl)phenyl)-N-(7-(2-(4-(4-chlorophenyl)-2,3,9-trimethyl-6H-thieno[3,2-f][1,2,4]triazolo[4,3-a][1,4]diazepin-6-yl)acetamido)heptyl)furan-2-carboxamide hydrochloride

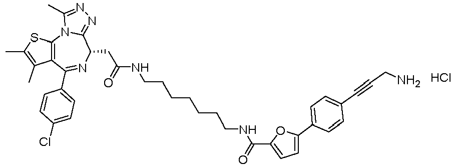

### Step 1: (S)-N-(7-aminoheptyl)-2-(4-(4-chlorophenyl)-2,3,9-trimethyl-6H-thieno[3,2-f][1,2,4]triazolo[4,3-a][1,4]diazepin-6-yl)acetamide hydrochloride (Intermediate 5-111'a)

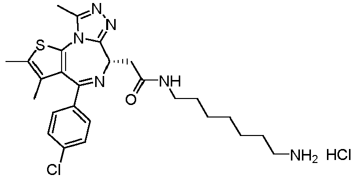

Intermediate 5-111'a was synthesized in the same manner as in Preparation Example 1-1'from *tert*-butyl (7-aminopropyl)carbamate.

### Step 2: (S)-5-(4-bromophenyl)-N-(7-(2-(4-(4-chlorophenyl)-2,3,9-trimethyl-6H-thieno[3,2-f][1,2,4]triazolo[4,3-a][1,4]diazepin-6-yl)acetamido)heptyl)furan-2-carboxamide (Intermediate 5-111'b)

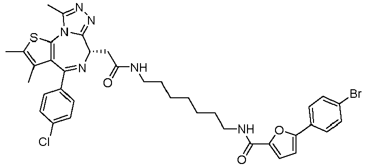

Intermediate 5-111'b was synthesized in the same manner as in step 5 of Preparation Example 4-1' from Intermediate 5-111'a and 5-(4-bromophenyl)-2-furoic acid.

### Step 3: (S)-5-(4-(3-aminoprop-1-yn-1-yl)phenyl)-N-(7-(2-(4-(4-chlorophenyl)-2,3,9-trimethyl-6H-thieno[3,2-f][1,2,4]triazolo[4,3-a][1,4]diazepin-6-yl)acetamido)heptyl)furan-2-carboxamide hydrochloride

Example 5-111' was synthesized in the same manner as in steps 2 to 3 of Preparation Example 4-179' from Intermediate 5-111'b.

¹H NMR (500 MHz, DMSO-*d*₆) δ 8.48 (t, *J* = 5.8 Hz, 1H), 8.14 (t, *J* = 5.7 Hz, 1H), 7.87 - 7.83 (m, 2H), 7.46 - 7.41 (m, 5H), 7.39 - 7.36 (m, 2H), 7.09 (q, *J* = 3.5 Hz, 2H), 4.46 (dd, *J* = 8.1, 6.1 Hz, 1H), 3.49 (s, 2H), 3.19 (d, *J* = 5.8 Hz, 3H), 3.14 - 3.09 (m, 2H), 3.05 (dq, *J* = 12.9, 6.2 Hz, 2H), 2.54 (d, *J* = 2.0 Hz, 3H), 2.35 (d, *J* = 3.1 Hz, 3H), 1.57 (s, 3H), 1.52 (d, *J* = 8.1 Hz, 2H), 1.48 (d, *J* = 7.6 Hz, 2H), 1.40 (s, 2H), 1.19 (s, 4H); MS m/z [M+H]⁺ 736.56.

### [Preparation Example 5-164'] methyl (S)-2-(3-aminoprop-1-yn-1-yl)-4-(4-(3-(2-(4-(4-chlorophenyl)-2,3,9-trimethyl-6H-thieno[3,2-f][1,2,4]triazolo[4,3-a][1,4]diazepin-6-yl)acetamido)propyl)piperazin-1-yl)benzoate hydrochloride

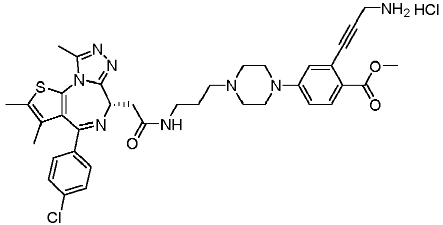

### Step 1: methyl 4-(4-(3-((((9H-fluoren-9-yl)methoxy)carbonyl)amino)propyl)piperazin-1-yl)-2-(3-((tert-butoxycarbonyl)amino)prop-1-yn-1-yl)benzoate (Intermediate 5-164a)

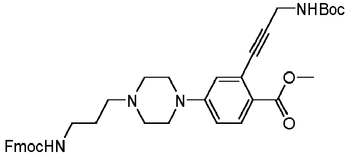

Intermediate 5-7b (0.1 mmol, 1.0 eq) was dissolved in 3.0 mL of DCM, and then NaBH(OAc)₃ (3.0 eq), (9H-fluoren-9-yl)methyl (3-oxopropyl)carbamate (1.2 eq), and acetic acid (1.0 eq) were slowly added thereto at 0 °C, followed by stirring for 12 hours. The reactant was extracted with DCM and H₂O to obtain an organic layer. The obtained organic layer was dried over Na₂SO₄. The mixture was filtered under reduced pressure, distilled, and then purified through flash column to synthesize Intermediate 5-164a.

### Step 2: methyl 4-(4-(3-aminopropyl)piperazin-1-yl)-2-(3-((tert-butoxycarbonyl)amino)prop-1-yn-1-yl)benzoate formic acid (Intermediate 5-164b)

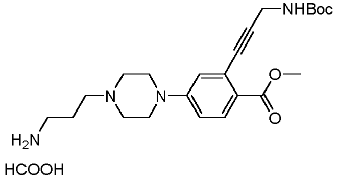

Intermediate 5-164a (0.1 mmol, 1.0 eq) was dissolved in 2.0 mL of piperidine/DMF (1:1), followed by stirring at room temperature for 0.5 hours. The reactant was concentrated under reduced pressure, and then purified through prep HPLC to synthesize Intermediate 5-164b.

### Step 3: methyl (S)-2-(3-aminoprop-1-yn-1-yl)-4-(4-(3-(2-(4-(4-chlorophenyl)-2,3,9-trimethyl-6H-thieno[3,2-f][1,2,4]triazolo[4,3-a][1,4]diazepin-6-yl)acetamido)propyl)piperazin-1-yl)benzoate hydrochloride

Example 5-164' was synthesized in the same manner as in step 2 of Preparation Example 5-77' from JQ1 carboxylic acid and Intermediate 5-164b.

¹H NMR (400 MHz, DMSO-*d*₆) δ 11.37 (s, 1H), 8.65 - 8.47 (m, 3H), 7.84 (d, *J* = 2.2 Hz, 1H), 7.53 (d, *J* = 2.2 Hz, 2H), 7.44 (d, *J* = 2.1 Hz, 2H), 7.19 (s, 1H), 7.18 - 7.12 (m, 1H), 4.59 - 4.57 (m, 1H), 4.05 - 3.99 (m, 4H), 3.81 (s, 3H), 3.61 - 3.56 (m, 2H), 3.43 - 3.39 (m, 2H), 3.30 - 3.27 (m, 3H), 3.17 - 3.12 (m, 5H), 2.64 (s, 3H), 2.42 (m, 3H), 1.96 - 1.91 (m, 2H), 1.62 (s, 3H); MS m/z [M+H]⁺ 713.5.

### [Preparation Example 5-165'] methyl (S)-2-(3-aminoprop-1-yn-1-yl)-5-(4-(3-(2-(4-(4-chlorophenyl)-2,3,9-trimethyl-6H-thieno[3,2-f][1,2,4]triazolo[4,3-a][1,4]diazepin-6-yl)acetamido)propyl)piperazin-1-yl)benzoate hydrochloride

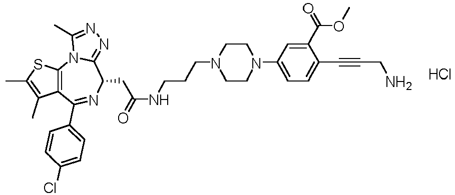

### Step 1: methyl 5-(4-(3-((((9H-fluoren-9-yl)methoxy)carbonyl)amino)propyl)piperazin-1-yl)-2-(3-((tert-butoxycarbonyl)amino)prop-1-yn-1-yl)benzoate (Intermediate 5-165a)

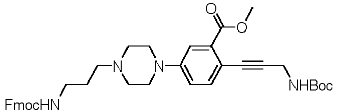

Intermediate 5-165a was synthesized in the same manner as in step 1 of Preparation Example 5-164' using Intermediate 5-33b as a starting material.

### Step 2: methyl 5-(4-(3-aminopropyl)piperazin-1-yl)-2-(3-((tert-butoxycarbonyl)amino)prop-1-yn-1-yl)benzoate (Intermediate 5-165b)

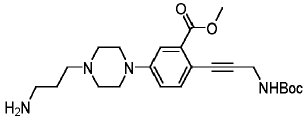

Intermediate 5-165b was synthesized in the same manner as in step 4 of Example 5-29 using Intermediate 5-165a as a starting material.

### Step 3: methyl (S)-2-(3-aminoprop-1-yn-1-yl)-5-(4-(3-(2-(4-(4-chlorophenyl)-2,3,9-trimethyl-6H-thieno[3,2-f][1,2,4]triazolo[4,3-a][1,4]diazepin-6-yl)acetamido)propyl)piperazin-1-yl)benzoate hydrochloride

Example 5-165' was synthesized in the same manner as in step 2 of Example 5-77' using Intermediate 5-165b as a starting material.

¹H NMR (400 MHz, DMSO-*d*₆) δ 8.21 (t, *J* = 1.4 Hz, 1H), 7.48 (d, *J* = 2.1 Hz, 2H), 7.42 (d, *J* = 2.1 Hz, 2H), 7.32 (d, *J* = 2.2 Hz, 1H), 7.25 (s, 1H), 7.11 (m, 1H), 4.50 (t, *J* = 1.7 Hz, 1H), 3.82 (s, 3H), 3.48 (s, 2H), 3.24 - 3.20 (m, 6H), 3.15 (m, 2H), 2.59 (s, 3H), 2.41 (s, 4H), 2.37 (s, 3H), 2.34 (m, 2H), 1.62 (s, 5H); MS m/z [M+H]⁺ 713.4.

### [Preparation Example I"]

A TBM-UBM compound in which a compound (Alisertib) showing the activity of an Aurora A kinase inhibitor is linked to compounds corresponding to Formula I of the present invention was synthesized as follows.

### [Preparation Example 1-1"] N-(4-(3-aminoprop-1-yn-1-yl)phenyl)-4-((9-chloro-7-(2-fluoro-6-methoxyphenyl)-5H-benzo[c]pyrimido[4,5-e]azepin-2-yl)amino)-2-methoxybenzamide trifluoroacetic acid

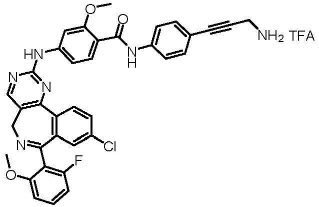

Example 1-1" was synthesized in the same manner as in steps 2 to 3 of Preparation Example 5-155' from commercially available Alisertib (4-((9-chloro-7-(2-fluoro-6-methoxyphenyl)-5H-benzo[c]pyrimido[4,5-e]azepin-2-yl)amino)-2-methoxybenzoic acid) and Intermediate 1-1a.

¹H NMR (500 MHz, DMSO-*d*₆) δ 10.23 (s, 1H), 10.10 (s, 1H), 8.71 (d, *J* = 3.6 Hz, 1H), 8.27 (d, *J* = 8.5 Hz, 1H), 8.22 (s, 3H), 7.98 (s, 1H), 7.79 (dd, *J* = 9.5, 6.9 Hz, 3H), 7.70 (d, *J* = 8.5 Hz, 1H), 7.41 (dddd, *J* = 12.3, 9.9, 8.5, 1.8 Hz, 4H), 3.98 - 3.94 (m, 2H), 3.92 (s, 3H); MS m/z [M+H]⁺ 647.45.

### [Preparation Example 1-3"] N-(3-(3-aminoprop-1-yn-1-yl)phenyl)-4-((9-chloro-7-(2-fluoro-6-methoxyphenyl)-5H-benzo[c]pyrimido[4,5-e]azepin-2-yl)amino)-2- methoxybenzamide trifluoroacetic acid

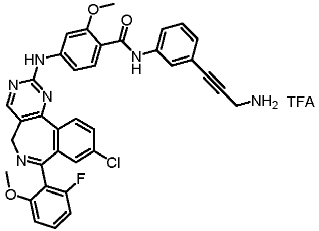

Example 1-3" was synthesized in the same manner as in steps 2 to 3 of Preparation Example 5-155' from Alisertib and Intermediate 1-3a.

¹H NMR (500 MHz, DMSO-*d*₆) δ 10.27 (s, 1H), 10.07 (s, 1H), 8.74 (s, 1H), 8.31 (d, *J* = 8.8 Hz, 4H), 8.11 (s, 1H), 8.01 (s, 1H), 7.83 (dd, *J* = 8.5, 2.2 Hz, 1H), 7.74 (d, *J* = 8.6 Hz, 1H), 7.66 - 7.63 (m, 1H), 7.48 (dd, *J* = 8.6, 1.9 Hz, 1H), 7.45 - 7.37 (m, 2H), 7.23 (s, 1H), 7.18 (d, *J* = 7.7 Hz, 1H), 4.03 (d, *J* = 5.6 Hz, 2H), 3.96 (s, 6H), 3.80 (s, 2H); MS m/z [M+H]⁺ 647.45.

### [Preparation Example 3-25"] methyl 2-(3-aminoprop-1-yn-1-yl)-4-(4-((9-chloro-7-(2-fluoro-6-methoxyphenyl)-5H-benzo[c]pyrimido[4,5-e]azepin-2-yl)amino)-2-methoxybenzamido)benzoate trifluoroacetic acid

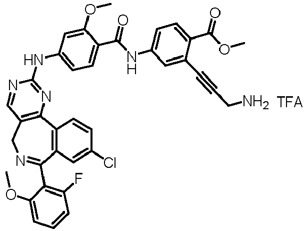

Example 3-25" was synthesized in the same manner as in steps 2 to 3 of Preparation Example 5-155' from Alisertib and Intermediate 3-25a.

¹H NMR (500 MHz, DMSO-*d*₆) δ 10.30 (d, *J* = 4.6 Hz, 2H), 8.75 (s, 1H), 8.35 (s, 2H), 8.30 (d, *J* = 2.6 Hz, 3H), 8.02 (s, 1H), 7.93 (d, *J* = 8.6 Hz, 1H), 7.83 (dd, *J* = 8.5, 2.2 Hz, 1H), 7.73 (td, *J* = 6.4, 3.3 Hz, 2H), 7.48 (dd, *J* = 8.6, 1.9 Hz, 1H), 7.43 (td, *J* = 8.4, 6.8 Hz, 1H), 7.23 (s, 1H), 7.04 - 6.77 (m, 1H), 4.08 (s, 2H), 3.96 (s, 6H), 3.85 (s, 5H); MS m/z [M+H]⁺ 705.39.

### [Preparation Example 3-49"] N-(4-(3-amino-4-hydroxybut-1-yn-1-yl)phenyl)-4-((9-chloro-7-(2-fluoro-6-methoxyphenyl)-5H-benzo[c]pyrimido[4,5-e]azepin-2-yl)amino)-2-methoxybenzamide trifluoroacetic acid

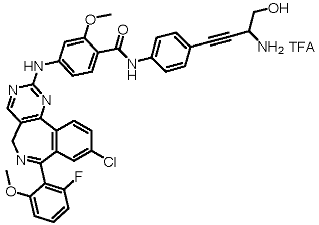

Example 3-49" was synthesized in the same manner as in step 2 to 3 of Preparation Example 5-155' from Intermediate 3-49b.

¹H NMR (500 MHz, DMSO-*d*₆) δ 10.23 (s, 1H), 10.10 (s, 1H), 8.70 (s, 1H), 8.40 (d, *J* = 5.3 Hz, 3H), 8.27 (d, *J* = 8.5 Hz, 1H), 7.97 (s, 1H), 7.82 - 7.76 (m, 4H), 7.70 (d, *J* = 8.6 Hz, 1H), 7.46 - 7.35 (m, 5H), 7.19 (s, 1H), 4.32 - 4.26 (m, 1H), 3.92 (s, 3H), 3.74 (dd, *J* = 11.3, 4.4 Hz, 1H), 3.63 (dd, *J* = 11.2, 7.0 Hz, 1H); MS m/z [M+H]⁺ 677.52.

### [Preparation Example 4-1"] N-(3-(5-(3-aminoprop-1-yn-1-yl)furan-2-yl)prop-2-yn-1-yl)-4-((9-chloro-7-(2-fluoro-6-methoxyphenyl)-5H-benzo[c]pyrimido[4,5-e]azepin-2-yl)amino)-2-methoxybenzamide trifluoroacetic acid

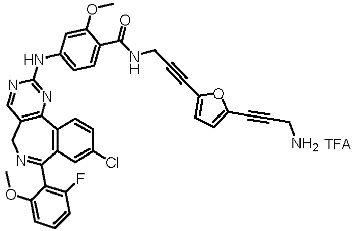

Example 4-1" was synthesized in the same manner as in steps 4 to 5 of Preparation Example 5-85' from Alisertib and Intermediate 4-1d.

¹H NMR (500 MHz, DMSO-*d*₆) δ 10.22 (s, 1H), 8.70 (s, 1H), 8.52 (t, *J* = 5.7 Hz, 1H), 8.28 (s, 2H), 8.26 (s, 3H), 7.96 (s, 1H), 7.83 (d, *J* = 8.7 Hz, 1H), 7.79 (dd, *J* = 8.5, 2.2 Hz, 1H), 7.42 - 7.34 (m, 3H), 7.19 (s, 1H), 6.91 (d, *J* = 3.6 Hz, 1H), 6.84 (d, *J* = 3.6 Hz, 1H), 4.32 (d, *J* = 5.7 Hz, 2H), 4.04 (d, *J* = 5.5 Hz, 2H), 3.90 (s, 6H); MS m/z [M+H]⁺ 675.42.

### [Preparation Example 5-116"] methyl 2-(3-aminoprop-1-yn-1-yl)-4-(3-(4-((9-chloro-7-(2-fluoro-6-methoxyphenyl)-5H-benzo[c]pyrimido[4,5-e]azepin-2-yl)amino)-2-methoxybenzamido)prop-1-yn-1-yl)benzoate trifluoroacetic acid

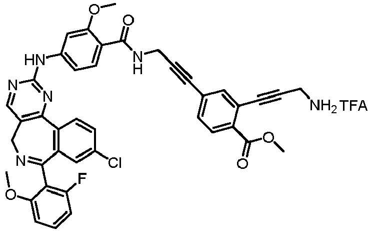

Example 5-116" was synthesized in the same manner as in steps 2 to 3 of Preparation Example 5-155' from Alisertib and Intermediate 5-116c.

¹H NMR (500 MHz, DMSO-*d*₆) δ 10.21 (d, *J* = 2.6 Hz, 1H), 8.70 (s, 1H), 8.52 (t, *J* = 5.7 Hz, 1H), 8.29 - 8.19 (m, 5H), 7.96 (s, 1H), 7.85 (dd, *J* = 11.4, 8.4 Hz, 2H), 7.79 (dd, *J* = 8.5, 2.3 Hz, 1H), 7.59 - 7.51 (m, 2H), 7.42 - 7.35 (m, 3H), 7.19 (s, 1H), 4.33 (d, *J* = 5.7 Hz, 2H), 4.01 (q, *J* = 5.6 Hz, 2H), 3.91 (s, 3H), 3.82 (s, 3H); MS m/z [M+H]⁺ 743.45.

### [Preparation Example II"]

A TBM-UBM compound in which a compound (Alisertib) showing the activity of an Aurora A kinase inhibitor is linked to compounds corresponding to Formula II of the present invention was synthesized as follows.

### [Preparation Example 4-165"] N-(6-((4-(3-aminoprop-1-yn-1-yl)phenyl)amino)-6-oxohexyl)-4-((9-chloro-7-(2-fluoro-6-methoxyphenyl)-5H-benzo[c]pyrimido[4,5-e]azepin-2-yl)amino)-2-methoxybenzamide trifluoroacetic acid

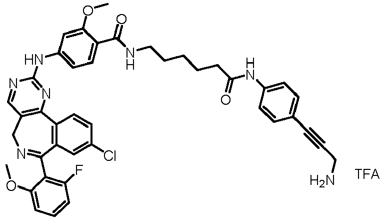

### Step 1: methyl 6-(4-((9-chloro-7-(2-fluoro-6-methoxyphenyl)-5H-benzo[c]pyrimido[4,5-e]azepin-2-yl)amino)-2-methoxybenzamido)hexanoate(Intermediate 4-165"a)

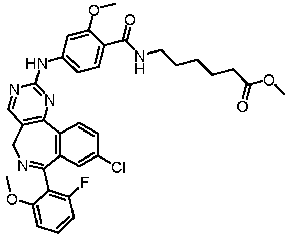

Intermediate 4-165"a was synthesized in the same manner as in step 5 of Preparation Example 4-1' using Alisertib and methyl 6-aminohexanoate as starting materials.

### Step 2: potassium 6-(4-((9-chloro-7-(2-fluoro-6-methoxyphenyl)-5H-benzo[c]pyrimido[4,5-e]azepin-2-yl)amino)-2-methoxybenzamido)hexanoate (Intermediate 4-165"b)

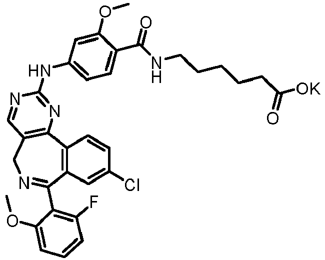

Intermediate 4-165"a (0.04 mmol, 1.0 eq), potassium trimethylsilanolate (4.0 eq), and THF (0.5 mL) were mixed, and then the mixture was stirred at room temperature for 3 hours. The reactant was filtered under reduced pressure to synthesize Intermediate 4-165"b without further purification.

### Step 3: tert-butyl (3-(4-(6-(4-((9-chloro-7-(2-fluoro-6-methoxyphenyl)-5H-benzo[c]pyrimido[4,5-e]azepin-2-yl)amino)-2-methoxybenzamido)hexanamido)phenyl)prop-2-yn-1-yl)carbamate (Intermediate 4-165"c)

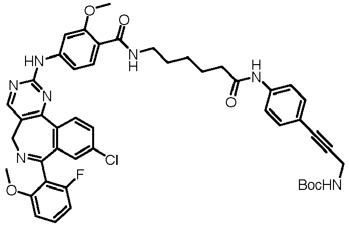

Intermediate 4-165"c was synthesized in the same manner as in step 5 of Preparation Example 4-1' using Intermediates 4-165"b and 1-1a as starting materials.

### Step 4: N-(6-((4-(3-aminoprop-1-yn-1-yl)phenyl)amino)-6-oxohexyl)-4-((9-chloro-7-(2-fluoro-6-methoxyphenyl)-5H-benzo[c]pyrimido[4,5-e]azepin-2-yl)amino)-2-methoxybenzamide trifluoroacetic acid

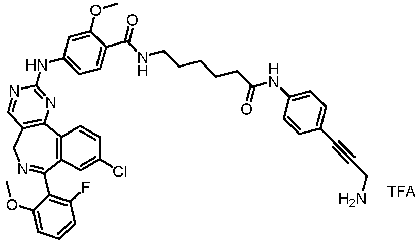

Example 4-165" was synthesized in the same manner as in step 3 of Preparation Example 5-153' from Intermediate 4-165"c.

¹H NMR (500 MHz, DMSO-*d*₆) δ 10.15 (s, 1H), 10.07 (s, 1H), 8.68 (s, 1H), 8.26 (d, *J* = 8.6 Hz, 1H), 8.20 (s, 3H), 8.01 (s, 1H), 7.91 (s, 1H), 7.80 - 7.77 (m, 1H), 7.77 - 7.74 (m, 2H), 7.63 - 7.60 (m, 2H), 7.38 (d, *J* = 7.0 Hz, 1H), 7.37 - 7.34 (m, 4H), 3.94 (d, *J* = 5.7 Hz, 2H), 3.87 (s, 6H), 2.31 (d, *J* = 7.7 Hz, 4H), 1.60 (d, *J* = 7.4 Hz, 2H), 1.51 (t, *J* = 7.3 Hz, 2H), 1.32 (d, *J* = 7.2 Hz, 2H); MS m/z [M+H]⁺ 760.44.

### [Preparation Example 4-166"] 5-(3-aminoprop-1-yn-1-yl)-N-(3-(4-((9-chloro-7-(2-fluoro-6-methoxyphenyl)-5H-benzo[c]pyrimido[4,5-e]azepin-2-yl)amino)-2-methoxybenzamido)propyl)furan-2-carboxamide trifluoroacetic acid

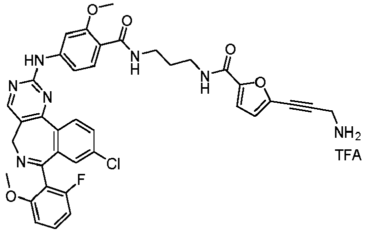

### Step 1: tert-butyl (3-(4-((9-chloro-7-(2-fluoro-6-methoxyphenyl)-5H-benzo[c]pyrimido[4,5-e]azepin-2-yl)amino)-2-methoxybenzamido)propyl)carbamate (Intermediate 4-166"a)

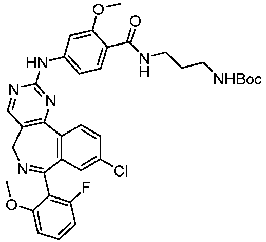

Intermediate 4-166"a was synthesized in the same manner as in step 1 of Preparation Example 4-165" using *tert*-butyl (3-aminopropyl)carbamate.

### Step 2: N-(3-aminopropyl)-4-((9-chloro-7-(2-fluoro-6-methoxyphenyl)-5H-benzo[c]pyrimido[4,5-e]azepin-2-yl)amino)-2-methoxybenzamide hydrochloride(Intermediate 4-166"b)

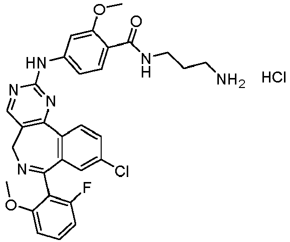

Intermediate 4-166"b was synthesized in the same manner as in step 2 of Preparation Example 1-1 from Intermediate 4-166"a.

### Step 3: tert-butyl (3-(5-((3-(4-((9-chloro-7-(2-fluoro-6-methoxyphenyl)-5H-benzo[c]pyrimido[4,5-e]azepin-2-yl)amino)-2-methoxybenzamido)propyl)carbamoyl)furan-2-yl)prop-2-yn-1-yl)carbamate (Intermediate 4-166"c)

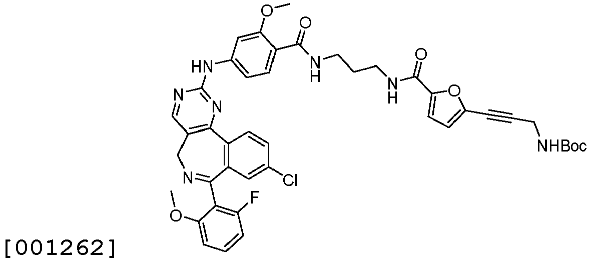

Intermediate 4-166"c was synthesized in the same manner as in step 1 of Preparation Example 1-1' from Intermediate 4-166"b and Intermediate 4-227b.

### Step 4: 5-(3-aminoprop-1-yn-1-yl)-N-(3-(4-((9-chloro-7-(2-fluoro-6-methoxyphenyl)-5H-benzo[c]pyrimido[4,5-e]azepin-2-yl)amino)-2-methoxybenzamido)propyl)furan-2-carboxamide trifluoroacetic acid

Example 4-166" was synthesized in the same manner as in step 3 of Preparation Example 5-153' from Intermediate 4-166"c.

¹H NMR (500 MHz, DMSO-*d*₆) δ 10.20 (s, 1H), 8.72 (s, 1H), 8.60 (t, *J* = 6.0 Hz, 1H), 8.39 - 8.25 (m, 6H), 7.97 (s, 1H), 7.84 - 7.81 (m, 2H), 7.44 - 7.40 (m, 2H), 7.22 (s, 1H), 7.19 (d, *J* = 3.6 Hz, 1H), 7.01 (d, *J* = 3.6 Hz, 1H), 4.11 (d, *J* = 5.6 Hz, 2H), 3.94 (s, 6H), 3.30 (dt, *J* = 12.9, 6.4 Hz, 6H), 1.70 (q, *J* = 6.7 Hz, 2H); MS m/z [M+H]⁺ 722.47.

### [Preparation Example 4-228"] methyl 2-(3-aminoprop-1-yn-1-yl)-4-(4-(4-((9-chloro-7-(2-fluoro-6-methoxyphenyl)-5H-benzo[c]pyrimido[4,5-e]azepin-2-yl)amino)-2-methoxybenzamido)butanamido)benzoate trifluoroacetic acid

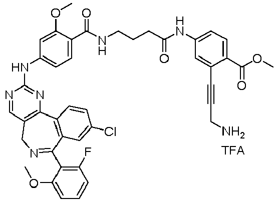

### Step 1: 2,5-dioxopyrrolidin-1-yl 4-((9-chloro-7-(2-fluoro-6-methoxyphenyl)-5H-benzo[c]pyrimido[4,5-e]azepin-2-yl)amino)-2-methoxybenzoate (Intermediate 4-228"a)

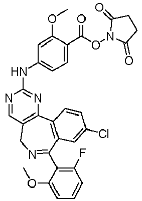

Intermediate 4-228"a was synthesized in the same manner as in step 4 of Preparation Example 4-177' from Alisertib.

### Step 2: 4-(4-((9-chloro-7-(2-fluoro-6-methoxyphenyl)-5H-benzo[c]pyrimido[4,5-e]azepin-2-yl)amino)-2-methoxybenzamido)butanoic acid (Intermediate 4-228"b)

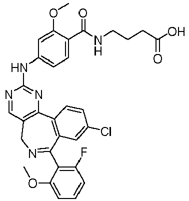

Intermediate 4-228"b was synthesized in the same manner as in step 5 of Preparation Example 4-177' from Intermediate 4-228"a and 4-aminobutanoic acid.

### Step 3: methyl 2-(3-aminoprop-1-yn-1-yl)-4-(4-(4-((9-chloro-7-(2-fluoro-6-methoxyphenyl)-5H-benzo[c]pyrimido[4,5-e]azepin-2-yl)amino)-2-methoxybenzamido)butanamido)benzoate trifluoroacetic acid

Example 4-228" was synthesized in the same manner as in steps 2 to 3 of Preparation Example 5-155' from Intermediate 4-228"b and Intermediate 3-25a.

¹H NMR (500 MHz, DMSO-*d*₆) δ 10.40 (s, 1H), 10.19 (s, 1H), 8.72 (s, 1H), 8.29 (d, *J* = 8.6 Hz, 5H), 8.16 - 8.13 (m, 2H), 7.94 (s, 1H), 7.89 (d, *J* = 8.6 Hz, 1H), 7.84 - 7.79 (m, 2H), 7.52 (dd, *J* = 8.7, 2.2 Hz, 1H), 7.44 - 7.39 (m, 2H), 7.22 (s, 1H), 4.07 (d, *J* = 5.7 Hz, 2H), 3.91 (s, 3H), 3.81 (s, 6H), 3.35 (q, *J* = 6.6 Hz, 4H), 2.41 (t, *J* = 7.3 Hz, 2H), 1.84 (q, *J* = 7.2 Hz, 2H); MS m/z [M+H]⁺ 760.51.

### [Preparation Example 5-8"] N-(4-((4-(3-aminoprop-1-yn-1-yl)-3-(hydroxymethyl)phenyl)amino)-4-oxobutyl)-4-((9-chloro-7-(2-fluoro-6-methoxyphenyl)-5H-benzo[c]pyrimido[4,5-e]azepin-2-yl)amino)-2-methoxybenzamide trifluoroacetic acid

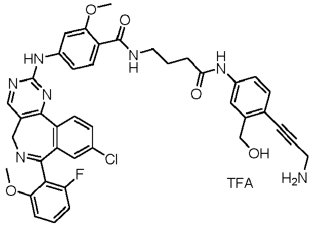

Example 5-8" was synthesized by preparing the compound in the same manner of Preparation Example 5-8' from Alisertib and performing further purification through prep HPLC.

¹H NMR (500 MHz, DMSO-*d*₆) δ 10.19 (s, 1H), 10.16 (s, 1H), 8.72 (s, 1H), 8.30 (d, *J* = 8.5 Hz, 1H), 8.25 (s, 4H), 8.14 (t, *J* = 5.8 Hz, 1H), 7.95 (s, 1H), 7.84 - 7.77 (m, 2H), 7.76 (d, *J* = 2.2 Hz, 1H), 7.62 (dd, *J* = 8.4, 2.2 Hz, 1H), 7.41 (ddd, *J* = 8.6, 6.9, 1.7 Hz, 3H), 7.33 (d, *J* = 8.4 Hz, 1H), 7.21 (d, *J* = 9.2 Hz, 1H), 4.62 (s, 2H), 4.01 (q, *J* = 5.6 Hz, 2H), 3.92 (s, 6H), 3.16 (s, 4H), 2.38 (s, 2H), 1.84 (s, 2H); MS m/z [M+H]⁺ 762.54.

### [Preparation Example 5-18"] methyl 2-(3-aminoprop-1-yn-1-yl)-4-((3-(4-((9-chloro-7-(2-fluoro-6-methoxyphenyl)-5H-benzo[c]pyrimido[4,5-e]azepin-2-yl)amino)-2-methoxybenzamido)propyl)carbamoyl)benzoate hydrochloride

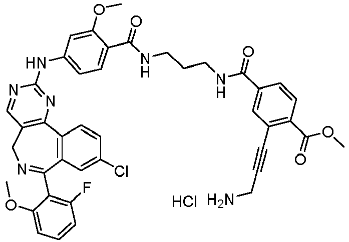

Example 5-18" was synthesized in the same manner as in Preparation Example 5-18' from Alisertib.

¹H NMR (500 MHz, DMSO-*d*₆) δ 10.22 (s, 1H), 8.89 (t, *J* = 5.8 Hz, 1H), 8.73 (s, 1H), 8.45 (s, 3H), 8.34 - 8.27 (m, 2H), 8.09 (t, *J* = 1.2 Hz, 1H), 7.99 (d, *J* = 1.2 Hz, 2H), 7.96 (s, 1H), 7.88 - 7.79 (m, 3H), 7.48 - 7.38 (m, 3H), 7.25 (s, 1H), 4.06 (q, *J* = 5.6 Hz, 2H), 3.94 (s, 6H), 3.89 (s, 3H), 3.87 (s, 2H), 3.34 (q, *J* = 7.4 Hz, 4H), 1.75 (t, *J* = 6.7 Hz, 2H); MS m/z [M+H]⁺ 790.41.

### [Preparation Example 5-35"] methyl 2-(3-aminoprop-1-yn-1-yl)-4-((4-(4-((9-chloro-7-(2-fluoro-6-methoxyphenyl)-5H-benzo[c]pyrimido[4,5-e]azepin-2-yl)amino)-2-methoxybenzamido)cyclohexyl)amino)benzoate trifluoroacetic acid

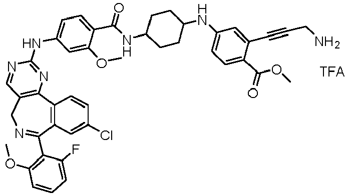

Example 5-35" was synthesized in the same manner as in Preparation Example 5-35' from Alisertib.

¹H NMR (500 MHz, DMSO-*d*₆) δ 10.19 (s, 1H), 8.69 (d, *J =* 3.5 Hz, 1H), 8.29 - 8.21 (m, 6H), 7.99 (d, *J* = 7.1 Hz, 2H), 7.81 (d, *J* = 8.6 Hz, 1H), 7.78 (dd, *J* = 8.5, 2.2 Hz, 2H), 7.68 (dd, *J* = 8.9, 1.5 Hz, 1H), 7.42 - 7.35 (m, 3H), 6.69 (s, 2H), 3.99 (q, *J* = 5.7 Hz, 2H), 3.95 (s, 3H), 3.89 (s, 1H), 3.71 (s, 1H), 3.44 (s, 1H), 2.59 (dp, *J* = 5.5, 1.8 Hz, 2H), 1.94 (d, *J* = 11.0 Hz, 2H), 1.71 (t, *J* = 13.3 Hz, 4H), 1.49 (d, *J* = 9.0 Hz, 2H); MS m/z [M+H]⁺ 802.50.

### [Preparation Example 5-81"] N-(4-((3-(3-aminoprop-1-yn-1-yl)-4-(2-(trifluoromethyl)pyrimidin-5-yl)phenyl)amino)-4-oxobutyl)-4-((9-chloro-7-(2-fluoro-6-methoxyphenyl)-5H-benzo[c]pyrimido[4,5-e]azepin-2-yl)amino)-2-methoxybenzamide trifluoroacetic acid

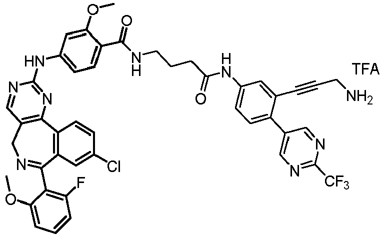

Example 5-81" was synthesized in the same manner as in Preparation Example 5-81' from Alisertib.

¹H NMR (500 MHz, DMSO-*d*₆) δ 10.34 (s, 1H), 10.19 (s, 1H), 9.28 (s, 2H), 8.72 (s, 1H), 8.29 (d, *J* = 8.6 Hz, 1H), 8.26 - 8.17 (m, 4H), 8.14 (t, *J* = 5.8 Hz, 1H), 7.94 (s, 1H), 7.85 - 7.76 (m, 2H), 7.66 - 7.60 (m, 2H), 7.45 - 7.37 (m, 3H), 7.22 (s, 1H), 3.98 - 3.93 (m, 2H), 3.92 (s, 6H), 3.37 (d, *J* = 6.4 Hz, 4H), 2.42 (d, *J* = 7.4 Hz, 2H), 1.87 (t, *J* = 7.1 Hz, 2H); MS m/z [M+H]⁺ 878.42.

### [Preparation Example 5-104"] N-(6-((3-(3-aminoprop-1-yn-1-yl)phenyl)amino)-6-oxohexyl)-4-((9-chloro-7-(2-fluoro-6-methoxyphenyl)-5H-benzo[c]pyrimido[4,5-e]azepin-2-yl)amino)-2-methoxybenzamide trifluoroacetic acid

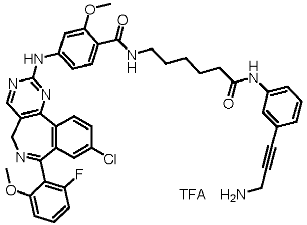

Example 5-104" was synthesized in the same manner as in steps 3 to 4 of Preparation Example 4-165" from Intermediate 4-165"b and Intermediate 1-3a.

¹H NMR (500 MHz, DMSO-*d*₆) δ 10.15 (s, 1H), 10.01 (s, 1H), 8.68 (s, 1H), 8.26 (d, *J* = 8.5 Hz, 2H), 8.23 (s, 3H), 8.01 (t, *J* = 5.8 Hz, 1H), 7.93 (t, *J* = 1.9 Hz, 1H), 7.91 (s, 1H), 7.78 (dd, *J* = 8.5, 2.2 Hz, 1H), 7.75 (d, *J* = 8.6 Hz, 1H), 7.43 - 7.39 (m, 2H), 7.37 (dt, *J* = 8.7, 1.8 Hz, 2H), 7.29 (t, *J* = 7.9 Hz, 1H), 7.07 (dt, *J* = 7.6, 1.4 Hz, 1H), 3.97 (d, *J* = 5.7 Hz, 2H), 3.86 (s, 3H), 2.29 (t, *J* = 7.4 Hz, 4H), 1.60 (d, *J* = 7.5 Hz, 2H), 1.50 (d, *J* = 7.5 Hz, 2H), 1.32 (d, *J* = 7.4 Hz, 2H); MS m/z [M+H]⁺ 760.44.

### [Preparation Example 5-150"] methyl 2-(3-aminoprop-1-yn-1-yl)-4-(4-(4-((9-chloro-7-(2-fluoro-6-methoxyphenyl)-5H-benzo[c]pyrimido[4,5-e]azepin-2-yl)amino)-2-methoxybenzamido)-N-methylbutanamido)benzoate hydrochloride

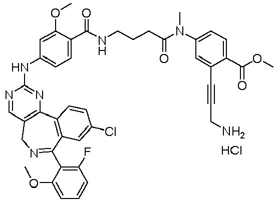

Example 5-150" was synthesized in the same manner as in Preparation Example 5-150' from Alisertib.

¹H NMR (500 MHz, DMSO-*d*₆) δ 10.15 (s, 1H), 8.69 (s, 1H), 8.29 - 8.23 (m, 5H), 7.99 (s, 1H), 7.89 (s, 1H), 7.87 (d, *J* = 8.4 Hz, 1H), 7.79 (dd, J = 8.5, 2.2 Hz, 1H), 7.71 (d, *J* = 8.6 Hz, 1H), 7.54 (d, *J* = 2.3 Hz, 1H), 7.47 (dd, *J* = 8.5, 2.2 Hz, 1H), 7.42 - 7.34 (m, 3H), 7.19 (s, 1H), 4.02 (q, *J* = 5.6 Hz, 2H), 3.83 (s, 3H), 3.80 (s, 6H), 3.23 - 3.16 (m, 7H), 2.23 (s, 2H), 1.71 (p, *J* = 6.9 Hz, 2H); MS m/z [M+H]⁺ 804.39

### [Preparation Example III"]

A TBM-UBM compound in which a compound (Alisertib) showing the activity of an Aurora A kinase inhibitor is linked to compounds corresponding to Formula III of the present invention was synthesized as follows.

### [Preparation Example 5-23"] methyl 2-(3-aminoprop-1-yn-1-yl)-4-(1-(4-((9-chloro-7-(2-fluoro-6-methoxyphenyl)-5H-benzo[c]pyrimido[4,5-e]azepin-2-yl)amino)-2-methoxybenzoyl)piperidine-4-carboxamido)benzoate hydrochloride

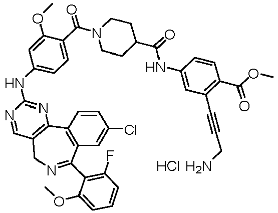

Example 5-23" was synthesized in the same manner as in Preparation Example 5-23' from Alisertib.

¹H NMR (500 MHz, DMSO-*d*₆) δ 10.46 (s, 1H), 10.04 (s, 1H), 8.66 (s, 1H), 8.33 (s, 3H), 8.26 (d, *J* = 8.5 Hz, 2H), 8.12 (d, *J* = 16.3 Hz, 1H), 7.86 (d, *J* = 8.7 Hz, 2H), 7.78 (dd, *J* = 8.5, 2.2 Hz, 1H), 7.54 - 7.52 (m, 1H), 7.39 (td, *J* = 8.4, 6.9 Hz, 2H), 7.34 (s, 1H), 7.19 (d, *J* = 10.7 Hz, 1H), 7.13 - 7.02 (m, 1H), 4.51 (s, 1H), 4.01 (t, *J* = 5.6 Hz, 2H), 3.79 (s, 3H), 3.77 (s, 3H), 2.76 (t, *J* = 12.4 Hz, 2H), 2.59 (dp, *J* = 5.5, 1.9 Hz, 2H), 1.87 (s, 2H), 1.71 (s, 2H); MS m/z [M+H]⁺ 816.48.

### [Preparation Example 5-28"] methyl 2-(3-aminoprop-1-yn-1-yl)-4-(3-(1-(4-((9-chloro-7-(2-fluoro-6-methoxyphenyl)-5H-benzo[c]pyrimido[4,5-e]azepin-2-yl)amino)-2-methoxybenzoyl)piperidine-4-carboxamido)prop-1-yn-1-yl)benzoate trifluoroacetic acid

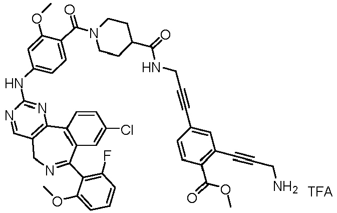

Example 5-28" was synthesized in the same manner as in Preparation Example 5-28' from Alisertib.

¹H NMR (500 MHz, DMSO-*d*₆) δ 10.02 (s, 1H), 8.65 (s, 1H), 8.40 (s, 1H), 8.26 (s, 3H), 8.24 (s, 2H), 7.86 (d, *J* = 8.1 Hz, 1H), 7.84 (s, 1H), 7.79 - 7.75 (m, 1H), 7.55 (s, 1H), 7.52 (d, *J* = 8.2 Hz, 1H), 7.41 - 7.35 (m, 1H), 7.33 (s, 1H), 7.17 (d, *J* = 7.5 Hz, 1H), 7.16 - 7.07 (m, 1H), 7.12 - 7.00 (m, 1H), 4.46 (s, 1H), 4.11 (s, 2H), 4.01 (q, *J* = 5.6 Hz, 2H), 3.82 (s, 3H), 3.76 (s, 6H), 2.93 (d, *J* = 47.2 Hz, 2H), 2.72 (t, *J* = 12.7 Hz, 2H), 1.75 (d, *J* = 12.7 Hz, 2H), 1.65 - 1.29 (m, 2H); MS m/z [M+H]⁺ 854.44.

### [Preparation Example 5-29"] methyl 2-(3-aminoprop-1-yn-1-yl)-4-(4-((1-(4-((9-chloro-7-(2-fluoro-6-methoxyphenyl)-5H-benzo[c]pyrimido[4,5-e]azepin-2-yl)amino)-2-methoxybenzoyl)piperidin-4-yl)methyl)piperazin-1-yl)benzoate trifluoroacetic acid

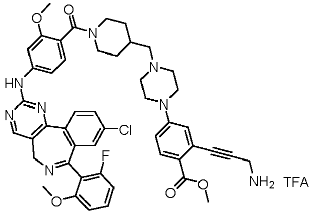

Example 5-29" was synthesized in the same manner as in steps 2 to 3 of Preparation Example 5-155' from Alisertib and Intermediate 5-29d.

¹H NMR (500 MHz, DMSO-*d*₆) δ 10.04 (s, 1H), 9,60 (s, 1H), 8.66 (s, 1H), 8.30 (s, 3H), 8.25 (d, *J* = 8.5 Hz, 1H), 7.81 (d, *J* = 8.9 Hz, 2H), 7.77 (d, *J* = 8.8 Hz, 1H), 7.39 (td, *J* = 8.4, 6.8 Hz, 1H), 7.33 (s, 1H), 7.17 (d, *J* = 9.3 Hz, 1H), 7.11 (d, *J* = 2.6 Hz, 1H), 7.09 (d, *J* = 2.6 Hz, 1H), 7.06 (d, *J* = 2.7 Hz, 2H), 4.48 (d, *J* = 12.7 Hz, 1H), 4.00 (d, *J* = 5.6 Hz, 3H), 3.77 (s, 6H), 3.40 (d, *J* = 13.0 Hz, 2H), 3.18 (s, 3H), 3.08 (s, 5H), 2.91 (s, 1H), 2.73 (t, *J* = 12.4 Hz, 1H), 2.08 (s, 1H), 1.83 (d, *J* = 11.0 Hz, 1H), 1.64 (d, *J* = 24.6 Hz, 1H), 1.16 (d, *J* = 37.6 Hz, 3H), 1.00 (d, *J* = 6.1 Hz, 1H); MS m/z [M+H]⁺ 871.53.

### [Preparation Example 5-40"] methyl 2-(3-aminoprop-1-yn-1-yl)-4-(6-(4-((9-chloro-7-(2-fluoro-6-methoxyphenyl)-5H-benzo[c]pyrimido[4,5-e]azepin-2-yl)amino)-2-methoxybenzoyl)-2,6-diazaspiro[3.3]heptan-2-yl)benzoate trifluoroacetic acid

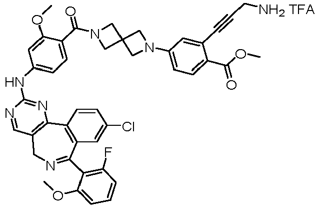

Example 5-40" was synthesized in the same manner as in Preparation Example 5-40' from Alisertib.

¹H NMR (500 MHz, DMSO-*d*₆) δ 10.08 (s, 1H), 8.67 (s, 1H), 8.30 - 8.16 (m, 5H), 7.87 (s, 1H), 7.80 - 7.73 (m, 3H), 7.42 - 7.35 (m, 1H), 7.32 (dd, *J* = 8.5, 1.8 Hz, 1H), 7.25 (d, *J* = 8.4 Hz, 1H), 7.18 (s, 1H), 6.45 (dd, *J* = 8.8, 2.5 Hz, 1H), 6.41 (d, *J* = 2.4 Hz, 1H), 4.17 (s, 2H), 4.12 (s, 2H), 4.09 (d, *J* = 8.7 Hz, 2H), 4.05 (s, 2H), 3.99 (q, *J* = 5.6 Hz, 2H), 3.81 (s, 3H), 3.73 (s, 3H); MS m/z [M+H]⁺ 786.41.

### [Preparation Example IV"]

A TBM-UBM compound in which a compound (Alisertib) showing the activity of an Aurora A kinase inhibitor is linked to compounds corresponding to Formula IV of the present invention was synthesized as follows.

### [Preparation Example 5-32"] methyl 2-(3-aminoprop-1-yn-1-yl)-4-(4-(3-(4-((9-chloro-7-(2-fluoro-6-methoxyphenyl)-5H-benzo[c]pyrimido[4,5-e]azepin-2-yl)amino)-2-methoxybenzamido)propanoyl)piperazin-1-yl)benzoate trifluoroacetic acid

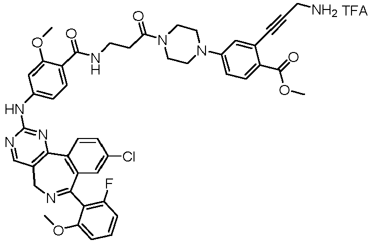

### Step 1: 3-(4-((9-chloro-7-(2-fluoro-6-methoxyphenyl)-5H-benzo[c]pyrimido[4,5-e]azepin-2-yl)amino)-2-methoxybenzamido)propanoic acid (Intermediate 5-32"a)

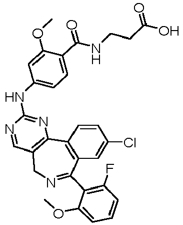

Intermediate 5-32"a was synthesized in the same manner as in step 1 of Preparation Example 4-124' from Alisertib.

### Step 2: methyl 2-(3-aminoprop-1-yn-1-yl)-4-(4-(3-(4-((9-chloro-7-(2-fluoro-6-methoxyphenyl)-5H-benzo[c]pyrimido[4,5-e]azepin-2-yl)amino)-2-methoxybenzamido)propanoyl)piperazin-1-yl)benzoate trifluoroacetic acid

Example 5-32" was synthesized in the same manner as in Preparation Example 1-1' from Intermediate 5-32"a and Intermediate 5-7b, and then further purified through prep HPLC.

¹H NMR (500 MHz, DMSO-*d*₆) δ 10.18 (s, 1H), 8.69 (s, 1H), 8.39 (t, *J=* 5.9 Hz, 1H), 8.27 (s, 1H), 8.25 (s, 3H), 7.94 (s, 1H), 7.83 (d, *J* = 8.6 Hz, 1H), 7.80 (d, *J* = 2.2 Hz, 1H), 7.77 (d, *J* = 8.9 Hz, 2H), 7.41 - 7.38 (m, 1H), 7.37 (dd, *J* = 8.5, 1.8 Hz, 2H), 7.19 (s, 1H), 7.01 (dd, *J* = 9.1, 2.7 Hz, 1H), 6.95 (d, *J* = 2.7 Hz, 1H), 3.99 (q, *J* = 5.6 Hz, 2H), 3.88 (s, 3H), 3.74 (s, 6H), 3.50 (d, *J* = 6.3 Hz, 8H), 3.34 (s, 2H), 3.31 (d, *J* = 5.5 Hz, 2H), 2.62 - 2.59 (m, 2H); MS m/z [M+H]⁺ 845.55.

### [Preparation Example I‴]

A TBM-UBM compound in which a compound (N-cyclopropyl-4'-(4-(2-hydroxyethoxy)benzoyl)-6-methyl-[1,1'-biphenyl]-3-carboxamide) showing the activity of a p38 MAPK inhibitor is linked to compounds corresponding to Formula I of the present invention was synthesized as follows.

### [Preparation Example 4-166‴] 2-(4-(5'-(cyclopropylcarbamoyl)-2'-methyl-[1,1'-biphenyl]-4-carbonyl)phenoxy)ethyl (3-(5-(3-aminoprop-1-yn-1-yl)furan-2-carboxamido)propyl)carbamate trifluoroacetic acid

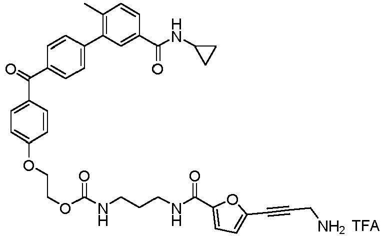

### Step 1: tert-butyl (3-(5-((3-((((9H-fluoren-9-yl)methoxy)carbonyl)amino)propyl)carbamoyl)furan-2-yl)prop-2-yn-1-yl)carbamate (Intermediate 4-166a)

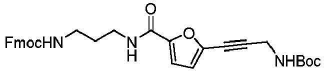

Intermediate 4-166a was synthesized in the same manner as in step 5 of Preparation Example 4-1' using (9H-Fluoren-9-yl)methyl (3-aminopropyl)carbamate as a starting material.

### Step 2: tert-butyl (3-(5-((3-aminopropyl)carbamoyl)furan-2-yl)prop-2-yn-1-yl)carbamate (Intermediate 4-166b)

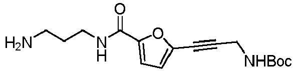

Intermediate 4-166b was synthesized in the same manner as in step 4 of Preparation Example 5-29 using Intermediate 4-166a as a starting material.

### Step 3: (4-bromophenyl)(4-(2-hydroxyethoxy)phenyl)methanone (Intermediate 4-166‴a)

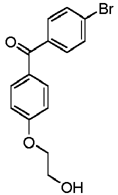

(4-Bromophenyl)(4-hydroxyphenyl)methanone (1.0 mmol, 1.0 eq), 2-bromoethan-1-ol (1.5 eq), and K₂CO₃ (2.0 eq) were stirred in an acetone solvent at 85 °C for 16 hours. The reactant was purified through flash column to synthesize Intermediate 4-166‴a.

### Step 4: N-cyclopropyl-4'-(4-(2-hydroxyethoxy)benzoyl)-6-methyl-[1,1'-biphenyl]-3-carboxamide (Intermediate 4-166‴b)

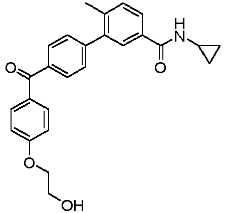

After Intermediate 4-166‴a (0.7 mmol, 1.0 eq), N-cyclopropyl-4-methyl-3-(4,4,5,5-tetramethyl-1,3,2-dioxaborolan-2-yl)benzamide (1.5 eq), Pd(PPh₃)₄ (0.1 eq), K₂CO₃ (2.0 eq), and DMF (3.0 mL) were mixed, a gas in a reactor was replaced with an Ar gas, and then heated at 90 °C for 16 hours. The reactant was extracted with EtOAc and H₂O to obtain an organic layer. The obtained organic layer was dried over Na₂SO₄. The mixture was filtered under reduced pressure, distilled, and then purified through flash column to synthesizelntermediate 4-166‴b.

### Step 5: 2-(4-(5'-(cyclopropylcarbamoyl)-2'-methyl-[1,1'-biphenyl]-4-carbonyl)phenoxy)ethyl (4-nitrophenyl) carbonate (Intermediate 4-166‴c)

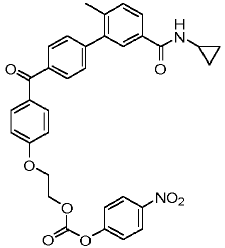

Intermediate 4-166‴b (0.7 mmol, 1.0 eq), 4-nitrophenyl chloroformate (1.1 eq), and TEA (2.0 eq) were stirred in a THF solvent at 0 °C for 2 hours. The reactant was purified through flash column to synthesize Intermediate 4-166‴c.

### Step 6: i-butyl (3-(5-((3-(((2-(4-(5'-(cyclopropylcarbamoyl)-2'-methyl-[1,1'-biphenyl]-4-carbonyl)phenoxy)ethoxy)carbonyl)amino)propyl)carbamoyl)furan-2-yl)prop-2-yn-1-yl)carbamate (Intermediate 4-166‴d)

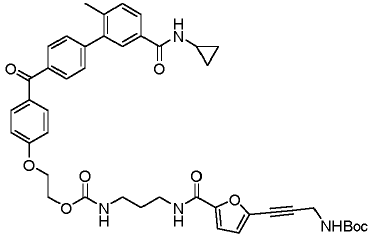

Intermediate 4-166‴c (0.5 mmol, 1.0 eq), Intermediate 4-166b (1.0 eq), and DIPEA (3.0 eq) were stirred in a DMF solvent at room temperature for 90 minutes. The reactant was purified through flash column to synthesize Intermediate 4-166‴d.

### Step 7: 2-(4-(5'-(cyclopropylcarbamoyl)-2'-methyl-[1,1'-biphenyl]-4-carbonyl)phenoxy)ethyl (3-(5-(3-aminoprop-1-yn-1-yl)furan-2-carboxamido)propyl)carbamate trifluoroacetic acid

Example 4-166‴ was synthesized in the same manner as in step 3 of Preparation Example 5-155' using Intermediate 4-166‴d as a starting material.

1H NMR (500 MHz, DMSO-*d*₆) δ 8.50 (t, *J* = 5.8 Hz, 1H), 8.46 (d, *J* = 4.2 Hz, 1H), 8.35 (s, 2H), 7.79 (dd, *J* = 8.5, 6.4 Hz, 5H), 7.74 (d, *J* = 1.9 Hz, 1H), 7.57 (d, *J* = 8.3 Hz, 2H), 7.42 (d, *J* = 8.0 Hz, 1H), 7.34 (t, *J* = 5.7 Hz, 1H), 7.17 - 7.12 (m, 3H), 6.99 (d, *J* = 3.7 Hz, 1H), 4.33 - 4.26 (m, 4H), 4.10 (s, 2H), 3.21 (q, *J* = 6.7 Hz, 2H), 3.04 - 2.98 (m, 2H), 2.85 (td, *J* = 7.3, 3.8 Hz, 1H), 2.31 (s, 3H), 1.62 (p, *J* = 7.0 Hz, 2H), 0.68 (td, *J* = 7.1, 4.7 Hz, 2H), 0.59 - 0.54 (m, 2H); MS m/z [M+H]⁺ 663.53.

### [Preparation Example 5-106‴] methyl 2-(3-aminoprop-1-yn-1-yl)-4-(5-(2-(4-(5'-(cyclopropylcarbamoyl)-2'-methyl-[1,1'-biphenyl]-4-carbonyl)phenoxy)ethoxy)-5-oxopentanamido)benzoate hydrochloride

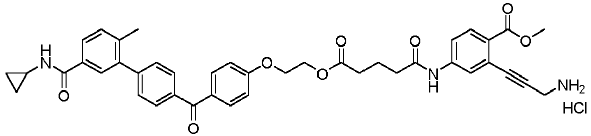

### Step 1: 5-((3-(3-((tert-butoxycarbonyl)amino)prop-1-yn-1-yl)-4-(methoxycarbonyl)phenyl)amino)-5-oxopentanoic acid (Intermediate 5-106a)

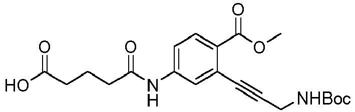

Intermediate 5-106a was synthesized in the same manner as in step 3 of Preparation Example 5-106' using Intermediate 3-25a as a starting material.

### Step 2: methyl 2-(3-aminoprop-1-yn-1-yl)-4-(5-(2-(4-(5'-(cyclopropylcarbamoyl)-2'-methyl-[1,1'-biphenyl]-4-carbonyl)phenoxy)ethoxy)-5-oxopentanamido)benzoate hydrochloride

Example 5-106‴ was synthesized in the same manner as in step 2 of Preparation Example 5-77' from Intermediate 5-106a and Intermediate 4-166‴c.

1H NMR (500 MHz, DMSO-*d*₆) δ 10.36 (s, 1H), 8.42 (d, *J =* 4.2 Hz, 1H), 8.08 (d, *J* = 2.2 Hz, 1H), 7.84 (d, *J* = 8.7 Hz, 1H), 7.78 - 7.72 (m, 5H), 7.70 (d, *J* = 2.0 Hz, 1H), 7.55 - 7.50 (m, 2H), 7.48 (dd, *J* = 8.7, 2.2 Hz, 1H), 7.38 (d, *J* = 8.0 Hz, 1H), 7.12 - 7.06 (m, 2H), 4.39 - 4.35 (m, 2H), 4.31 - 4.26 (m, 2H), 3.99 (s, 2H), 3.78 (s, 3H), 2.82 (tt, *J* = 7.3, 4.0 Hz, 1H), 2.41 - 2.35 (m, 4H), 2.27 (s, 3H), 1.81 (p, *J* = 7.5 Hz, 2H), 0.67 - 0.62 (m, 2H), 0.54 - 0.50 (m, 2H); MS m/z [M+H]⁺ 716.48.

### [Preparation Example II‴]

A TBM-UBM compound in which a compound (N-cyclopropyl-4'-(4-(2-hydroxyethoxy)benzoyl)-6-methyl-[1,1'-biphenyl]-3-carboxamide) showing the activity of a p38 MAPK inhibitor is linked to compounds corresponding to Formula II of the present invention was synthesized as follows.

### [Preparation Example 1-1‴] 2-(4-(5'-(cyclopropylcarbamoyl)-2'-methyl-[1,1'-biphenyl]-4-carbonyl)phenoxy)ethyl (4-(3-aminoprop-1-yn-1-yl)phenyl)carbamate hydrochloride

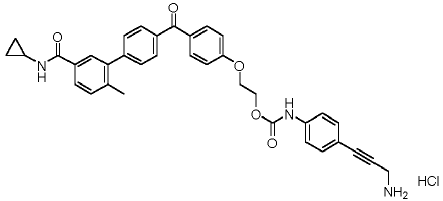

### Step 1: 2-(4-(5'-(cyclopropylcarbamoyl)-2'-methyl-[1,1'-biphenyl]-4-carbonyl)phenoxy)ethyl (4-(3-((tert-butoxycarbonyl)amino)prop-1-yn-1-yl)phenyl)carbamate (Intermediate 1-1‴a)

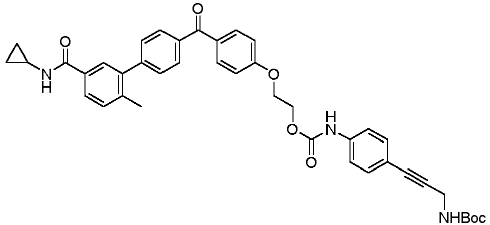

Intermediate 1-1‴a was synthesized in the same manner as in step 4 of Preparation Example 5-140‴ using Intermediate 4-166‴c and Intermediate 1-1a as starting materials.

### Step 2: 2-(4-(5'-(cyclopropylcarbamoyl)-2'-methyl-[1,1'-biphenyl]-4-carbonyl)phenoxy)ethyl (4-(3-aminoprop-1-yn-1-yl)phenyl)carbamate

Example 1-1‴, was synthesized in the same manner as in step 1 of Preparation Example 1-1 from Intermediate 1-1‴a.

1H NMR (500 MHz, DMSO-*d*₆) δ 8.42 (d, *J* = 4.3 Hz, 1H), 8.24 (s, 3H), 7.90 (t, *J* = 5.7 Hz, 1H), 7.86 (d, *J* = 8.2 Hz, 1H), 7.75 (ddd, *J* = 8.4, 4.5, 2.4 Hz, 5H), 7.71 (d, *J* = 2.0 Hz, 1H), 7.57 (d, *J* = 1.7 Hz, 1H), 7.52 (dd, *J* = 6.8, 4.8 Hz, 3H), 7.38 (d, *J* = 8.0 Hz, 1H), 7.13 - 7.08 (m, 2H), 4.33 (d, *J* = 4.7 Hz, 2H), 4.27 (s, 2H), 4.06 (d, *J* = 5.7 Hz, 2H), 4.00 (s, 2H), 3.83 (d, *J* = 3.7 Hz, 3H), 2.81 (td, *J* = 7.3, 3.8 Hz, 1H), 2.28 (s, 3H), 0.67 - 0.63 (m, 2H), 0.52 (dt, *J* = 6.9, 4.4 Hz, 2H); MS m/z [M+H]⁺ 684.51

### [Preparation Example 3-49‴] 2-(4-(5'-(cyclopropylcarbamoyl)-2'-methyl-[1,1'-biphenyl]-4-carbonyl)phenoxy)ethyl (4-(3-amino-4-hydroxybut-1-yn-1-yl)phenyl)carbamate hydrochloride

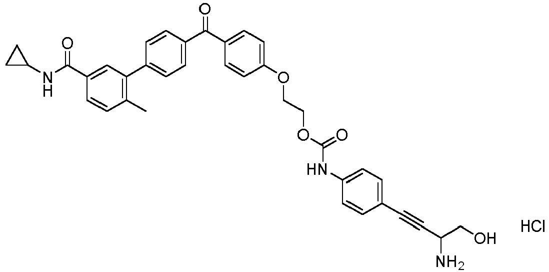

### Step 1: 2-(4-(5'-(cyclopropylcarbamoyl)-2'-methyl-[1,1'-biphenyl]-4-carbonyl)phenoxy)ethyl (4-(3-amino-4-hydroxybut-1-yn-1-yl)phenyl)carbamate hydrochloride

Example 3-49‴ was synthesized in the same manner as in steps 1 to 2 of Preparation Example 1-1‴ using Intermediate 3-49b as a starting material.

1H NMR (500 MHz, DMSO-*d*₆) δ 10.13 (s, 1H), 8.46 (d, *J =* 4.3 Hz, 1H), 8.40 (s, 2H), 7.80 (dd, *J* = 12.2, 8.5 Hz, 5H), 7.74 (d, *J=* 1.9 Hz, 1H), 7.55 (dd, *J=* 16.1, 8.6 Hz, 4H), 7.41 (dd, *J* = 11.5, 8.4 Hz, 3H), 7.16 (d, *J =* 8.9 Hz, 2H), 5.74 (t, *J =* 5.3 Hz, 1H), 4.52 - 4.45 (m, 2H), 4.41 - 4.35 (m, 2H), 4.31 (s, 1H), 3.79 - 3.73 (m, 1H), 3.67 - 3.60 (m, 1H), 2.88 - 2.82 (m, 1H), 2.31 (s, 3H), 0.68 (dd, *J* = 7.1, 2.4 Hz, 2H), 0.56 (dd, *J=* 4.1, 2.4 Hz, 2H); MS m/z [M+H]⁺ 618.47.

### [Preparation Example 5-116‴] methyl 2-(3-aminoprop-1-yn-1-yl)-4-(3-(((2-(4-(5'-(cyclopropylcarbamoyl)-2'-methyl-[1,1'-biphenyl]-4-carbonyl)phenoxy)ethoxy)carbonyl)amino)prop-1-yn-1-yl)benzoate hydrochloride

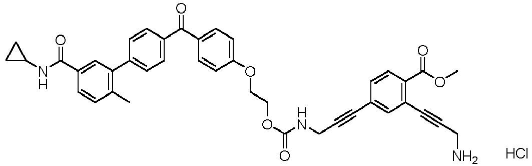

### Step 1: methyl 2-(3-aminoprop-1-yn-1-yl)-4-(3-(((2-(4-(5'-(cyclopropylcarbamoyl)-2'-methyl-[1,1'-biphenyl]-4-carbonyl)phenoxy)ethoxy)carbonyl)amino)prop-1-yn-1-yl)benzoate hydrochloride

Example 5-116‴ was synthesized in the same manner as in steps 1 to 2 of Preparation Example 1-1‴ using Intermediate 5-116c as a starting material.

1H NMR (500 MHz, DMSO-*d*₆) δ 8.42 (d, *J* = 4.3 Hz, 1H), 8.24 (s, 3H), 7.90 (t, *J* = 5.7 Hz, 1H), 7.86 (d, *J* = 8.2 Hz, 1H), 7.75 (ddd, *J* = 8.4, 4.5, 2.4 Hz, 5H), 7.71 (d, *J* = 2.0 Hz, 1H), 7.57 (d, *J* = 1.7 Hz, 1H), 7.52 (dd, *J* = 6.8, 4.8 Hz, 3H), 7.38 (d, *J* = 8.0 Hz, 1H), 7.13 - 7.08 (m, 2H), 4.33 (d, *J* = 4.7 Hz, 2H), 4.27 (s, 2H), 4.06 (d, *J* = 5.7 Hz, 2H), 4.00 (s, 2H), 3.83 (d, *J* = 3.7 Hz, 3H), 2.81 (td, *J* = 7.3, 3.8 Hz, 1H), 2.28 (s, 3H), 0.67 - 0.63 (m, 2H), 0.52 (dt, *J* = 6.9, 4.4 Hz, 2H); MS m/z [M+H]⁺ 684.51

### [Preparation Example III‴]

A TBM-UBM compound in which a compound (N-cyclopropyl-4'-(4-(2-hydroxyethoxy)benzoyl)-6-methyl-[1,1'-biphenyl]-3-carboxamide) showing the activity of a p38 MAPK inhibitor is linked to compounds corresponding to Formula III of the present invention was synthesized as follows.

### [Preparation Example 5-39‴] 2-(4-(5'-(cyclopropylcarbamoyl)-2'-methyl-[1,1'-biphenyl]-4-carbonyl)phenoxy)ethyl 7-(3-(3-aminoprop-1-yn-1-yl)-4-(methoxycarbonyl)benzoyl)-2,7-diazaspiro[3.5]nonane-2-carboxylate trifluoroacetic acid

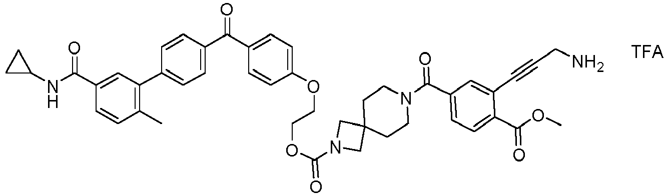

Example 5-39‴ was synthesized in the same manner as in steps 4 to 5 of Preparation Example 5-140‴ using Intermediate 5-39c as a starting material.

1H NMR (500 MHz, DMSO-*d*₆) δ 8.46 (d, *J* = 4.3 Hz, 1H), 8.31 (s, 2H), 7.82 - 7.76 (m, 5H), 7.74 (d, *J* = 1.9 Hz, 1H), 7.61 - 7.55 (m, 3H), 7.52 - 7.46 (m, 2H), 7.42 (d, *J* = 8.3 Hz, 1H), 7.14 (d, *J* = 8.9 Hz, 2H), 4.33 (s, 2H), 4.29 (s, 2H), 3.99 (s, 2H), 3.66 (s, 7H), 3.17 (d, *J* = 8.5 Hz, 2H), 2.85 (td, *J* = 7.3, 3.8 Hz, 1H), 2.31 (s, 3H), 1.75 (s, 2H), 1.65 (s, 2H), 0.70 - 0.65 (m, 2H), 0.56 (dt, *J* = 7.2, 4.4 Hz, 2H); MS m/z [M+H]⁺ 783.51.

### [Preparation Example 5-140‴] 2-(4-(5'-(cyclopropylcarbamoyl)-2'-methyl-[1,1'-biphenyl]-4-carbonyl)phenoxy)ethyl 4-(3-(3-aminoprop-1-yn-1-yl)-4-(methoxycarbonyl)benzoyl)piperazine-1-carboxylate trifluoroacetic acid

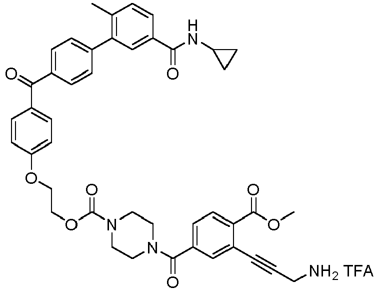

### Step 1 tert-butyl 4-(3-iodo-4-(methoxycarbonyl)benzoyl)piperazine-1-carboxylate (Intermediate 5-140a)

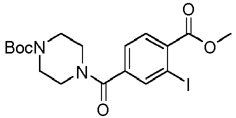

Intermediate 5-140a was synthesized in the same manner as in step 5 of Preparation Example 4-1' using tert-Butyl piperazine-1-carboxylate and 3-iodo-4-(methoxycarbonyl)benzoic acid as starting materials.

### Step 2: tert-butyl 4-(3-(3-(1,3-dioxoisoindolin-2-yl)prop-1-yn-1-yl)-4-(methoxycarbonyl)benzoyl)piperazine-1-carboxylate (Intermediate 5-140b)

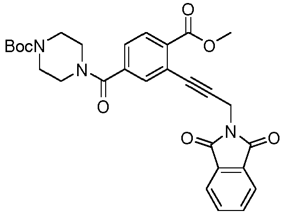

Intermediate 5-140b was synthesized in the same manner as in step 1 of Preparation Example 5-35' from Intermediate 5-140a.

### Step 3: methyl 2-(3-(1,3-dioxoisoindolin-2-yl)prop-1-yn-1-yl)-4-(piperazine-1-carbonyl)benzoate trifluoroacetic acid (Intermediate 5-140c)

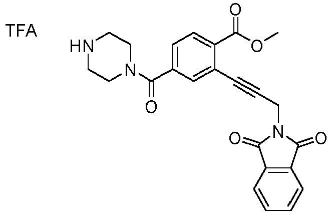

Intermediate 5-140c was synthesized in the same manner as in step 4 of Preparation Example 4-1' from Intermediate 5-140b.

### Step 4: 2-(4-(5'-(cyclopropylcarbamoyl)-2'-methyl-[1,1'-biphenyl]-4-carbonyl)phenoxy)ethyl 4-(3-(3-(1,3-dioxoisoindolin-2-yl)prop-1-yn-1-yl)-4-(methoxycarbonyl)benzoyl)piperazine-1-carboxylate (Intermediate 5-140‴a)

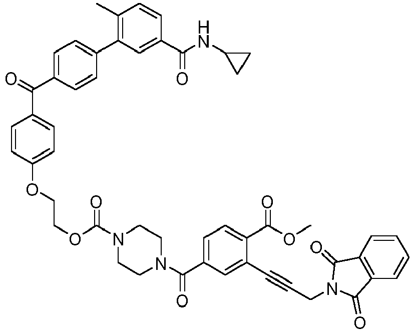

Intermediate 5-140‴a was synthesized in the same manner as in step 5 of Preparation Example 4-1' from Intermediates 5-140c and 4-166‴c.

### Step 5: 2-(4-(5'-(cyclopropylcarbamoyl)-2'-methyl-[1,1'-biphenyl]-4-carbonyl)phenoxy)ethyl 4-(3-(3-aminoprop-1-yn-1-yl)-4-(methoxycarbonyl)benzoyl)piperazine-1-carboxylate trifluoroacetic acid

Example 5-140‴ was synthesized in the same manner as in step 5 of Preparation Example 5-85' from Intermediate 5-140‴a.

1H NMR (500 MHz, DMSO-*d*₆) δ 8.42 (d, *J* = 4.3 Hz, 1H), 8.26 (s, 3H), 8.17 (d, *J* = 8.5 Hz, 1H), 8.06 - 8.00 (m, 1H), 7.92 (d, *J* = 8.1 Hz, 1H), 7.78 - 7.73 (m, 5H), 7.71 (d, *J* = 2.0 Hz, 1H), 7.57 (d, *J* = 1.7 Hz, 1H), 7.53 (d, *J* = 8.2 Hz, 2H), 7.38 (d, *J* = 8.1 Hz, 1H), 7.13 (dd, *J* = 8.9, 2.6 Hz, 1H), 4.36 (d, *J* = 5.0 Hz, 2H), 4.30 (s, 2H), 4.03 (q, *J* = 5.7 Hz, 2H), 3.84 (s, 3H), 3.34 (s, 4H), 3.24 (s, 4H), 2.81 (dt, *J* = 7.4, 3.7 Hz, 1H), 2.28 (s, 3H), 0.65 (td, *J* = 7.1, 4.7 Hz, 2H), 0.54 - 0.48 (m, 2H); MS m/z [M+H]⁺ 743.55.

### [Experimental Examples]

### [Experimental Example 1]

### Confirmation of Degradation Effects of BRD4 Degrader in MM.1S Cell Lines

To confirm that the compounds (BRD4 degraders) of Preparation Examples I' to IV' reduce the amount of BRD4 proteins, experiments were performed using MM.1S cell lines, which are B cells derived from the blood of multiple myeloma patients.

Specifically, the MM.1S cells were cultured in RPMI-1640 medium supplemented with 10% fetal bovine serum and Penicillin/Streptomycin (100 U/mL), transferred to a 6-well plate, and after 24 hours, the BRD4 degraders were diluted in the culture medium and the cells were treated with 10 µM each. After treatment, the cells were incubated in a CO₂ incubator for 24 hours. Through protein immunoblotting (Western Blot), the degrading capacity of each compound against BRD4 proteins in cells was confirmed. After cell culture was completed according to the experimental conditions, the cells were collected and centrifuged at 1,800 rpm for 3 minutes. The cells in the precipitate were lysed with RIPA (25 mM Tris-HCl pH 7.6, 150 mM NaCl, 1% NP-40, 1% sodium deoxycholate, 0.1% SDS, 1x Protease Inhibitor Cocktail), 5X sample buffer was added, and then heated at 100 °C for 15 minutes. The prepared sample was electrophoresed with sodium dodecyl sulfate poly acrylamide gel electrophoresis (SDS-PAGE), and then the proteins in the gel were transferred to a PVDF membrane using Trans-Blot Turbo. The membrane was treated with a 5% bovine serum albumin (BSA) solution and blocked at room temperature for 2 hours, and then BRD4 and β-actin antibodies were diluted in the 5% BSA solution to react with the membrane at 4 °C overnight. After washed with PBST (0.5% Tween20) 5 times, horse radish peroxidase (HRP)-conjugated secondary antibodies were diluted in PBST to react at room temperature for 2 hours, and then washed again with a PBST solution 5 times. After enzyme-substrate reaction was carried out using a HRP substrate, a signal was measured with chemi-doc equipment, and the densitometry of a band corresponding to the BRD4 protein detected in the Preparation Example Compounds was quantitatively analyzed. The case when the degrading capacity of each BRD4 degrader was reduced by less than 40% is indicated as +, the case when the degrading capacity was reduced by 40% to 70% (exclusive of 70) is indicated as ++, the case when the degrading capacity was reduced by 70% or more is indicated as +++ in Tables 16 to 19 below. As shown in the following Tables, it was confirmed that the compounds of Preparation Examples I' to IV' according to the present invention have excellent degrading capacity.

**[Table 16]**

| No. | BRD4 degrading capacity (%) | No. | BRD4 degrading capacity (%) |
|---|---|---|---|
| 1-1' | ++ | 3-25' | +++ |
| 1-3' | ++ | 3-26' | ++ |
| 3-1' | +++ | 3-27' | ++ |
| 3-2' | ++ | 3-49' | ++ |
| 3-3' | +++ | 3-51' | +++ |
| 3-8' | + | 3-57' | ++ |
| 4-1' | ++ | 4-147' | ++ |
| 4-10' | +++ | 4-179' | + |
| 4-128' | ++ | 4-223' | ++ |
| 4-129' | +++ | 5-116' | +++ |

**[Table 17]**

| No. | BRD4 degrading capacity (%) | No. | BRD4 degrading capacity (%) |
|---|---|---|---|
| 4-124' | +++ | 5-35' | +++ |
| 4-125' | +++ | 5-53' | ++ |
| 4-126' | ++ | 5-59' | +++ |
| 4-148' | + | 5-61' | ++ |
| 4-149' | +++ | 5-63' | ++ |
| 4-161' | +++ | 5-65' | + |
| 4-162' | +++ | 5-75' | +++ |
| 4-163' | +++ | 5-77' | + |
| 4-164' | ++ | 5-78' | + |
| 4-165' | +++ | 5-81' | +++ |
| 4-166' | +++ | 5-89' | + |
| 4-167' | ++ | 5-90' | +++ |
| 4-225' | +++ | 5-96' | +++ |
| 4-228' | +++ | 5-104' | ++ |
| 4-235' | ++ | 5-106' | + |
| 5-1' | +++ | 5-107' | ++ |
| 5-2' | ++ | 5-108' | +++ |
| 5-4' | ++ | 5-109' | +++ |
| 5-5' | ++ | 5-110' | ++ |
| 5-6' | ++ | 5-115' | +++ |
| 5-8' | ++ | 5-148' | + |
| 5-9' | ++ | 5-149' | +++ |
| 5-12' | +++ | 5-150' | +++ |
| 5-18' | +++ | | |

**[Table 18]**

| No. | BRD4 degrading capacity (%) | No. | BRD4 degrading capacity (%) |
|---|---|---|---|
| 4-2' | +++ | 5-44' | ++ |
| 4-226' | +++ | 5-85' | +++ |
| 4-227' | ++ | 5-86' | +++ |
| 5-7' | + | 5-139' | + |
| 5-22' | +++ | 5-140' | +++ |
| 5-23' | +++ | 5-152' | +++ |
| 5-25' | +++ | 5-153' | +++ |
| 5-26' | ++ | 5-154' | +++ |
| 5-27' | ++ | 5-155' | ++ |
| 5-28' | +++ | 5-157' | +++ |
| 5-29' | +++ | 5-158' | +++ |
| 5-39' | ++ | 5-162' | +++ |
| 5-40' | +++ | 5-168' | +++ |
| 5-41' | +++ | | |

**[Table 19]**

| No. | BRD4 degrading capacity (%) | No. | BRD4 degrading capacity (%) |
|---|---|---|---|
| 5-32' | +++ | 5-164' | +++ |
| 5-33' | ++ | 5-165' | +++ |
| 5-111' | ++ | | |

### [Experimental Example 2]

### Confirmation of Degradation Effects of Aurora A Degrader in MM.1S Cell Lines

To confirm that the compounds (Aurora A degraders) of Preparation Examples I" to IV" reduce the amount of Aurora A proteins, experiments were performed using MM.1S cell lines, which are B cells derived from the blood of multiple myeloma patients.

Specifically, the MM.1S cells were cultured in RPMI-1640 medium containing 10% fetal bovine serum (FBS), 1% penicillin, and streptomycin (100 U/mL), transferred to a 6-well plate, and after 24 hours, the Aurora A degraders were diluted in the RPMI culture medium and cells were treated with 5 µM concentration thereof. After treatment, the cells were incubated in a CO₂ incubator for 24 hours. Through protein immunoblotting (Western Blot), the degrading capacity of each compound against Aurora A proteins in cells was confirmed. After cell culture was completed according to the experimental conditions, the cells were collected and centrifuged at 1,800 rpm for 3 minutes. The cells in the precipitate were lysed with RIPA (25 mM Tris-HCl pH 7.6, 150 mM NaCl, 1% NP-40, 1% sodium deoxycholate, 0.1% SDS, 1x Protease Inhibitor Cocktail), 5X sample buffer was added, and then heated at 100 °C for 15 minutes. The prepared sample was electrophoresed with sodium dodecyl sulfate poly acrylamide gel electrophoresis (SDS-PAGE), and then the proteins in the gel were transferred to a PVDF membrane using Trans-Blot Turbo. The membrane was treated with a 5% bovine serum albumin (BSA) solution and blocked at room temperature for 2 hours, and then Aurora A and β-actin antibodies were diluted in the 5% BSA solution to react with the membrane at 4 °C overnight. After washed with PBST (0.5% Tween20) 5 times, horse radish peroxidase (HRP)-conjugated secondary antibodies were diluted in PBST to react at room temperature for 2 hours, and then washed again with a PBST solution 5 times. After enzyme-substrate reaction was carried out using a HRP substrate, a signal was measured with chemi-doc equipment, and the densitometry of a band corresponding to the Aurora A protein detected in the Preparation Example Compounds was quantitatively analyzed. The case when the degrading capacity of each Aurora A degrader was reduced by less than 40% is indicated as +, the case when the degrading capacity was reduced by 40% to 70% (exclusive of 70) is indicated as ++, the case when the degrading capacity was reduced by 70% or more is indicated as +++ in Tables 20 to 23 below. It was confirmed that the compound of Formula 2, which is a control group, exhibited the degrading capacity of 0%, whereas the compounds of Preparation Examples I" to IV" according to the present invention have excellent degrading capacity.

**[Table 20]**

| No. | Aurora A degrading capacity (%) | No. | Aurora A degrading capacity (%) |
|---|---|---|---|
| 1-1" | +++ | 3-25" | + |
| 1-3" | +++ | 3-49" | +++ |
| 4-1" | +++ | 5-116" | +++ |

**[Table 21]**

| No. | Aurora A degrading capacity (%) | No. | Aurora A degrading capacity (%) |
|---|---|---|---|
| 4-165" | +++ | 5-35" | +++ |
| 4-166" | + | 5-81" | +++ |
| 4-228" | ++ | 5-104" | +++ |
| 5-8" | + | 5-150" | + |
| 5-18" | +++ | | |

**[Table 22]**

| No. | Aurora A degrading capacity (%) | No. | Aurora A degrading capacity (%) |
|---|---|---|---|
| 5-23" | + | 5-29" | +++ |
| 5-28" | +++ | 5-40" | +++ |

**[Table 23]**

| No. | Aurora A degrading capacity (%) | No. | Aurora A degrading capacity (%) |
|---|---|---|---|
| 5-32" | ++ | | |

### [Experimental Example 3]

### Confirmation of Degradation Effects of p38 Degrader in Macrophages

To confirm that the compounds (p38 degraders) of Preparation Examples I‴ to III‴ reduce the amount of phospho-p38 (p-p38) proteins, experiments were performed using Raw 264.7 macrophages. Since p38 MAPK alpha is activated through the process of phosphorylation of threonine 180 (Thr180) and tyrosine 182 (Tyr182) residues by MKK3, MKK6, and the like, which are upstream regulators, phosphorylation of these residues was generally detected using an antibody (3D7 rabbit monoclonal antibody from Cell Signaling, Inc.) that specifically recognizes phospho-p38 (p-p38) MAPK in which p38 MAPK alpha was activated.

Specifically, the RAW 264.7 cells were cultured in DMEM medium containing 10% fetal bovine serum (FBS), 1% penicillin, and streptomycin (100 U/mL), transferred to a 6-well plate, and after 24 hours, the p38 degraders were diluted in the DMEM culture medium and the cells were treated with 10 µM concentration thereof. After treatment, the cells were incubated in a CO₂ incubator for 24 hours. Through protein immunoblotting (Western Blot), the degrading capacity of each compound against p38 and p-p38 proteins in cells was confirmed. After cell culture was completed according to the experimental conditions, the cells were collected and centrifuged at 1,800 rpm for 3 minutes. The cells in the precipitate were lysed with RIPA (25 mM Tris-HCl pH 7.6, 150 mM NaCl, 1% NP-40, 1% sodium deoxycholate, 0.1% SDS, 1x Protease Inhibitor Cocktail), 5X sample buffer was added, and then heated at 100 °C for 15 minutes. The prepared sample was electrophoresed with sodium dodecyl sulfate poly acrylamide gel electrophoresis (SDS-PAGE), and then the proteins in the gel were transferred to a PVDF membrane using Trans-Blot Turbo. The membrane was treated with a 5% bovine serum albumin (BSA) solution and blocked at room temperature for 2 hours, and then p-p38, p38, and β-actin antibodies were diluted in the 5% BSA solution to react with the membrane at 4 °C overnight. After washed with PBST (phosphate-based saline containing 0.5% Tween20) 5 times, horse radish peroxidase (HRP)-conjugated secondary antibodies were diluted in PBST to react at room temperature for 2 hours, and then washed again with a PBST solution 5 times. After enzyme-substrate reaction was carried out using a HRP substrate, a signal was measured with chemi-doc equipment, and the densitometry of bands corresponding to the p-p38 and p38 proteins detected in the Preparation Example Compounds was quantitatively analyzed. The case when the degrading capacity of each p38 degrader was reduced by less than 40% is indicated as +, the case when the degrading capacity was reduced by 40% to 70% (exclusive of 70) is indicated as ++, the case when the degrading capacity was reduced by 70% or more is indicated as +++ in Tables 24 to 26 below. As shown in the following Tables, it was confirmed that the compounds of Preparation Examples I‴ to III‴ according to the present invention have excellent degrading capacity.

**[Table 24]**

| No. | p38 degrading capacity (%) | No. | p38 degrading capacity (%) |
|---|---|---|---|
| 1-1‴ | +++ | 5-116‴ | +++ |
| 3-49‴ | +++ | | |

**[Table 25]**

| No. | p38 degrading capacity (%) | No. | p38 degrading capacity (%) |
|---|---|---|---|
| 4-166''' | +++ | 5-106''' | +++ |

**[Table 26]**

| No. | p38 degrading capacity (%) | No. | p38 degrading capacity (%) |
|---|---|---|---|
| 5-140''' | +++ | | |

### [Experimental Example 4]

### Confirmation of Direct Binding of Ubiquitin Using Pulldown Assay

To confirm whether the compounds represented by Formula I to Formula IV of the present invention bind to ubiquitin and polyubiquitinated via various E3 ligase complexes, *in vitro* ubiquitination assay was performed. As E3 ligases, ITCH/AIP4, NEDD4, and cIAP-1/HUAP-2 proteins (manufacturer: R&D System) were used.

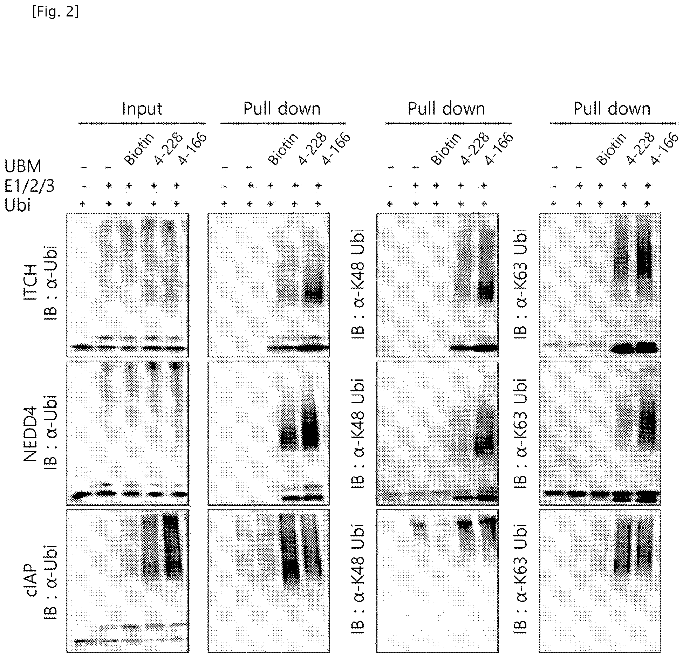

Specifically, 10X buffer and 100 µg of ubiquitin were used for a first lane, and 10X buffer and 100 µg of ubiquitin, 5 nM of E1, 100 nM of E2, 14 nM of E3, and 1 mM of ATP were used for second to sixth lanes. For the third lane, biotin was added at 40 µM as a negative control group, and for the fourth and fifth lanes, biotin-bound compounds of Preparation Examples 4-228 and 4-166 were added at 40 µM, respectively. Each sample was reacted in a 37 °C incubator for 3 hours, and then 5 µL of each sample was heated at 100 °C for 10 minutes by adding 5X sample buffer for input results. The remaining sample solution was reacted at 4 °C for 3 hours by adding agarose bead bound with Streptavidin, washed 5 times with TritonX-100 lysis buffer, and then heated at 100 °C for 10 minutes by adding 2X sample buffer. The Input sample and the pulldown sample were electrophoresed with SDS-PAGE, and then the proteins in the gel were transferred to the PVDF membrane using Trans-Blot Turbo. The membrane was treated with a 5% bovine serum albumin (BSA) solution, blocked at room temperature for 1 hour, and then each of ubiquitin antibody, K63-linked polyubiquitin antibody, and K48-linked polyubiquitin antibody was diluted in the 5% BSA solution, and reacted with the PVDF membrane at 4 °C overnight. After washed with PBST (phosphate-based saline containing 0.5% Tween20) 5 times, horse radish peroxidase (HRP)-conjugated secondary antibodies were diluted in PBST to react at room temperature for 2 hours, and then washed again with a PBST solution 5 times. After enzyme-substrate reaction was carried out using a HRP substrate, a signal was measured with chemi-doc equipment, and the results are shown in FIG. 2.

As a result, as shown in FIG. 2, it was confirmed that the compounds represented by Formula I to Formula IV of the present invention are acceptors that directly bind to ubiquitin.

### [Experimental Example 5]

### Confirmation of Direct Binding to Ubiquitin Using Intact Protein Analysis

To confirm that the compounds represented by Formulae V to VIII of the present invention directly bind to ubiquitin, *in vitro* ubiquitination assay was performed, and then the compounds of Preparation Examples I' to IV‴ (BRD4 degraders) bound to ubiquitin, a BRD4 inhibitor, and ubiquitin were detected using intact protein analysis. The intact protein analysis is a method capable of measuring a compound bound to a protein by mass spectrometry without protease pretreatment. Experiments were performed using ITCH/AIP4 proteins (manufacturer: R&D System), which are E3 ligases, in order to bind ubiquitin and BRD4 degraders *in vitro.*

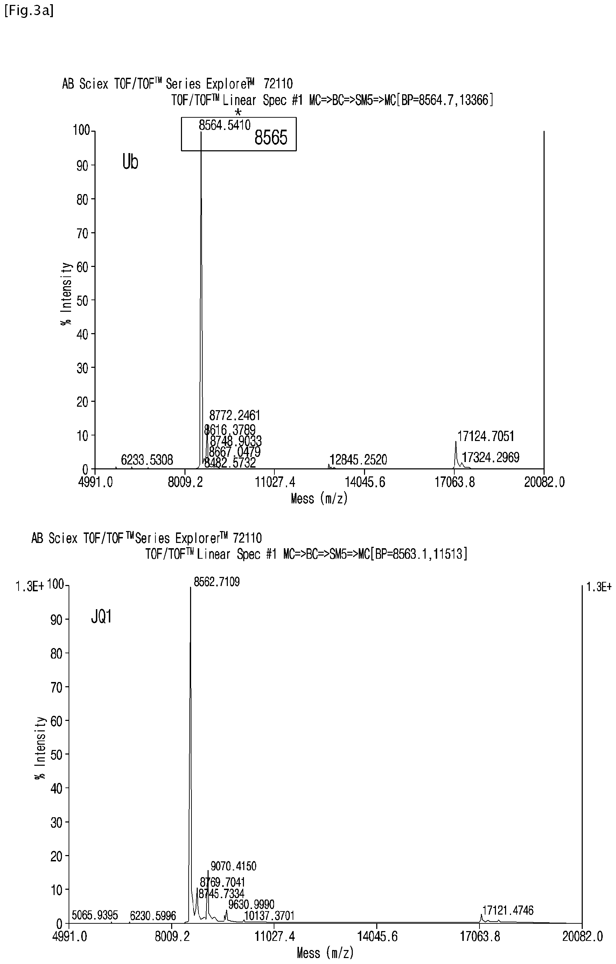

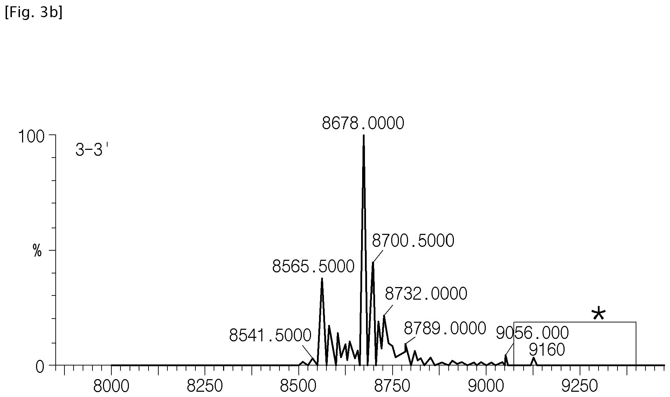

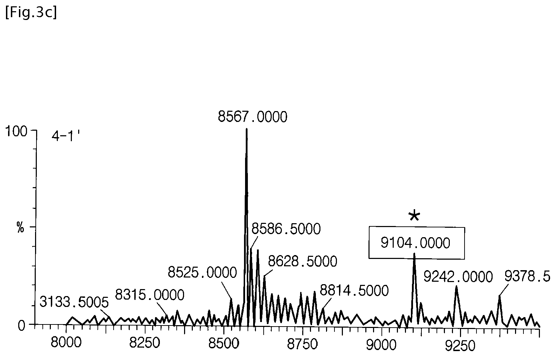

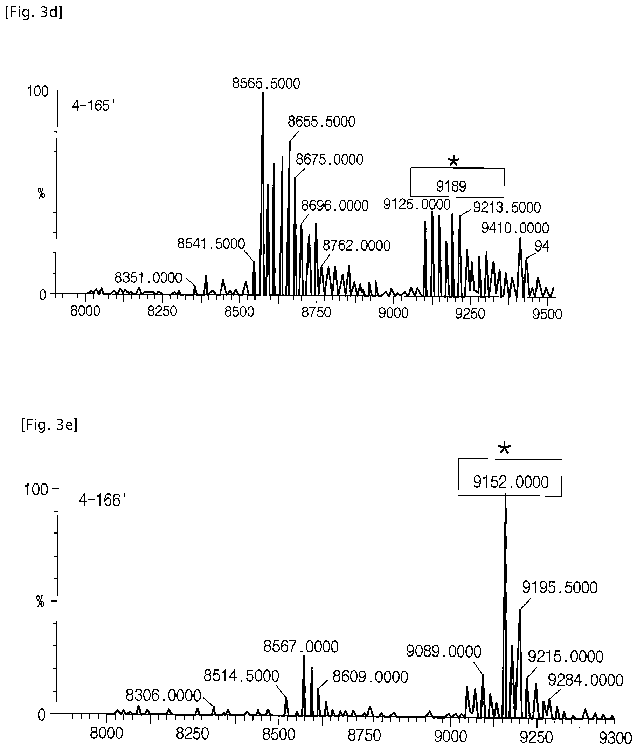

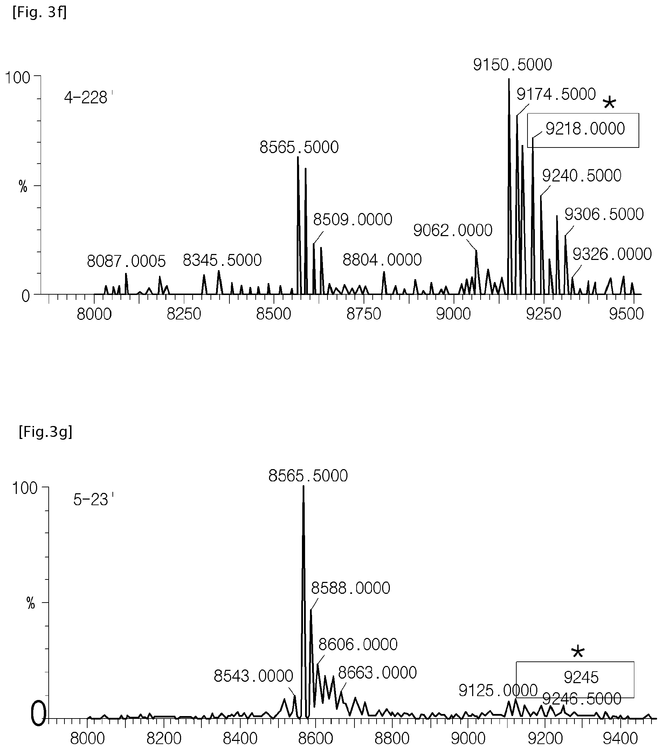

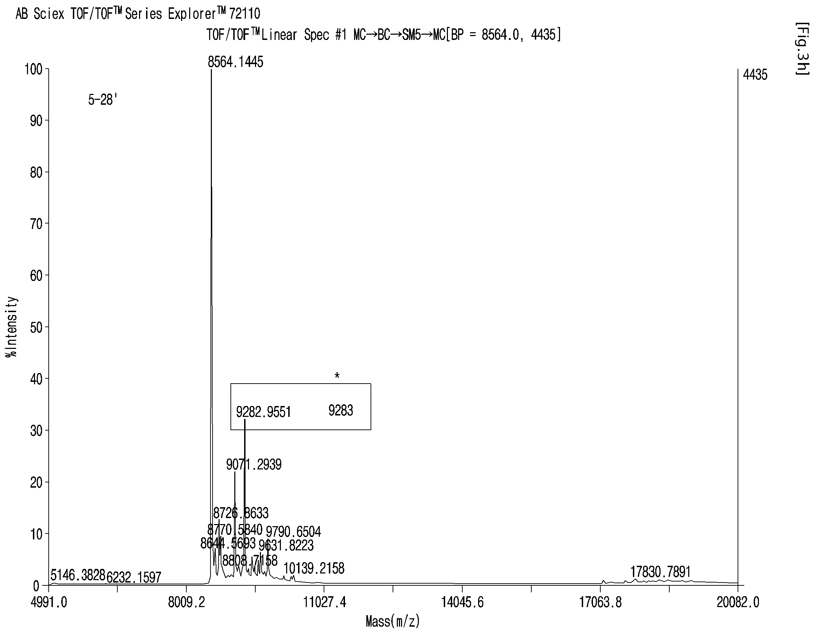

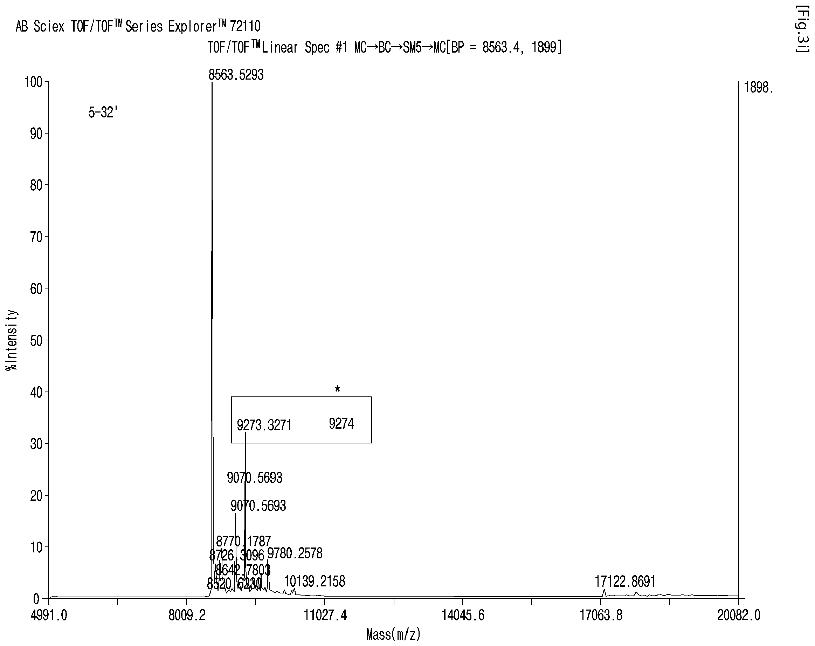

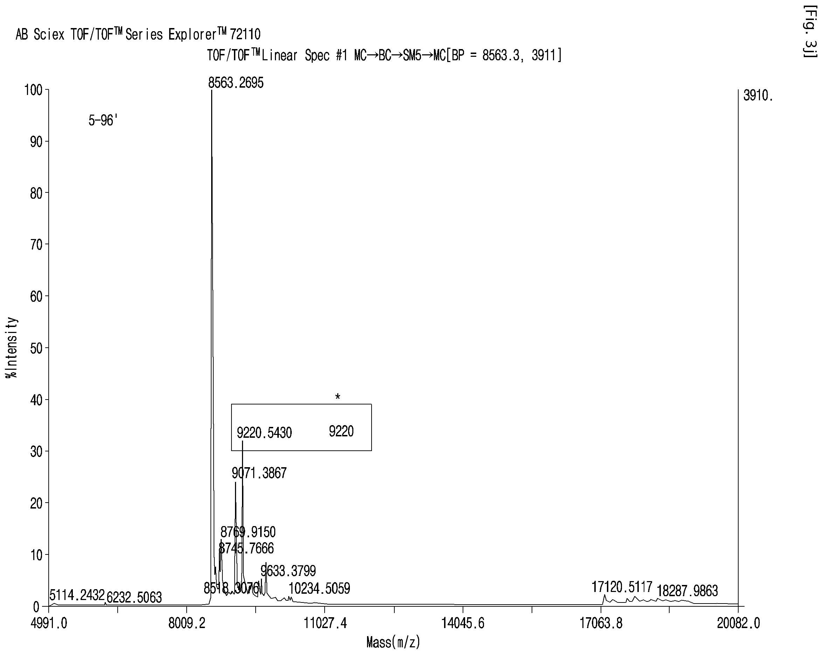

Specifically, 10 µM of each degrader was mixed with 1 mg/mL of ubiquitin, 10X buffer, 5 nM of E1, 100 nM of E2, 14 nM of E3, and 1 mM of ATP to react in a 37 °C incubator for 4 hours. To the reacted samples, acetonitrile and formic acid were added for UPLC-Q/TOF or MALDI-TOF analysis, resulting in the final concentrations of 50% acetonitrile and 0.1% formic acid, respectively. For UPLC-Q/TOF, a Micromass Q-TOF micro Synapt High-definition mass spectrometer equipped with ESI was used to perform the mass spectrometry. For optimum analysis conditions, the mass spectrometry was performed under the conditions of a capillary voltage of 2.7 kV, an energy source temperature of 120 °C, an elution temperature of 400 °C, a cone gas speed of 10 Uh, an elution gas speed of 800 Uh, a minimum voltage of 2 V, and a maximum voltage of 30 V. For the accuracy of mass spectrometry, the mass spectrometry was performed with the comparison with the reference value in a positive ion mode. The experimental results were analyzed using MassLynx 4.0 program, and the mass spectrometry values are shown in FIG. 3. In addition, a mass spectrum in MALDI-TOF was obtained in linear cation mode using a MALDI-TOF/TOF 5800 system (AB SCIEX, USA). The experimental results were analyzed using TOF/TOFTM Series ExplorerTM software version 4.0.

As a result, as shown in FIG. 3, it was confirmed that the molecular weight peak of the size of proteins bound to ubiquitin was not detected in the control group of Formula 1 (JQ1), but the ubiquitin and the BRD4 degrader were directly bound through peak detection in which the molecular weight of the ubiquitin reacted with the BRD4 degrader was increased by the size of the degrader.

Although the preferred embodiments of the present invention have been exemplarily described, the scope of the present invention is not limited to the specific embodiments as described above. Therefore, it will be apparent to those skilled in the art that modifications and variations can be made without departing from the spirit and scope of the invention as defined by the appended claims.

## Claims

1. A compound represented by Formula I to Formula IV below, or a pharmaceutically acceptable salt, isomer, solvate, hydrate, or prodrug thereof: 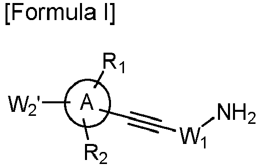 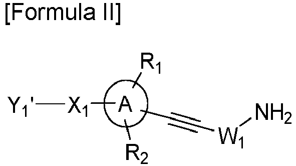 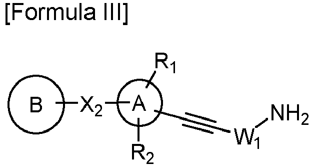 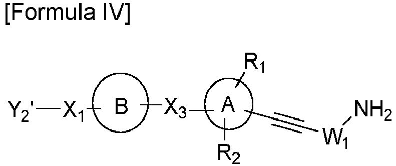
wherein, in Formula I to Formula IV above, each independently,
W₁ is any one selected from the group consisting of -alkylene-, -alkylene-O-, - alkylene-OSO₂-, and -alkylene-N(R')SO₂- unsubstituted or substituted with at least one selected from the group consisting of deuterium, alkyl, C(O)OR', C(O)N(R')(R"), alkylene-C(O)OR', alkylene-C(O)N(R')(R"), alkylene-OR', alkylene-SR', alkylene-N(R')(R"), alkylene-OP(O)(OR')(OR"), alkylene-P(O)(OR')(OR"), and alkylene-OP(O)(R')(R");
A is any one selected from the group consisting of aryl, heteroaryl, cycloalkyl, heterocycloalkyl, and heterocycloalkenyl;
R₁ and R₂ are each independently any one selected from the group consisting of hydrogen, alkyl, alkenyl, alkynyl, halogen, oxo, CN, NO₂, OR', SR', N(R')(R"), O-(alkylene)-OR', -N(R')-(alkylene)-OR', C(O)R', C(NH)R', C(S)R', C(O)OR', C(O)N(R')(R"), P(O)(R')(R"), P(O)(OR')(OR"), C(NH)N(R')(R"), C(S)OR', C(S)N(R')(R"), C(O)O-(alkylene)-OC(O)R', C(O)O-(alkylene)-methacryloyloxy, (alkylene)-OR', (alkylene)-SR', (alkylene)-N(R')(R"), (alkynylene)-N(R')(R" ), (alkylene)-P(O)(R')(R")-, (alkylene)-P(O)(OR')(OR"), (alkylene)-OP(O)(R')(R"), (alkylene)-OP(O)(OR')(OR"), (alkylene)-OP(O)(benzyloxy)₂, (alkylene)-OP(O)(phenoxy)₂, (alkylene)-OP(O)(phenoxy)(NH-CH(R')C(O)OR"), (alkylene)-C(O)OR', (alkenylene)-C(O)OR', (alkynylene)-C(O)OR', (alkylene)-C(O)N(R')(R"), (alkenylene)-C(NH)N(R')(R"), (alkynylene)-C(S)N(R')(R"), (alkylene)-C(O)R', (alkylene)-C(NH)R', (alkylene)-C(S)R', aryl, heteroaryl, heterocycloalkyl, and heterocycloalkenyl, optionally the R₁ and R₂ above are each independently substituted with at least one halogen, and when R₁ and R₂ above are each independently aryl, heteroaryl, heterocycloalkyl, or heterocycloalkenyl, optionally the R₁ and R₂ above are each independently substituted with alkyl or N(R')(R") unsubstituted or substituted with at least one halogen at one or more positions;
X₁ is a direct linkage, any one selected from among X1a group consisting of-N(R')-, -O-, -S-, -C(O)-, -SO-, SO₂-, =N-O-, and -O-N=, X1b group consisting of -OC(O)-, -C(O)O-, -N(R')C(O)-, -C(O)N(R')-, -N(R')SO-, -SON(R')-, -N(R')SO₂-, and -SO₂N(R')-, and X1c group consisting of -alkylene-, -alkenylene-, and -alkynylene-, or that two or more thereof are linked to each other;
X₂ is a direct linkage, any one selected from among X2a group consisting of -N(R')-, -O-, -S-, -C(O)-, -SO-, SO₂-, =N-O-, and -O-N=, X2b group consisting of -OC(O)-, - N(R')C(O)-, -N(R')C(O)O-, -N(R')SO₂-O-, and -N(R')C(S)O-, X2c group consisting of - C(O)O-, -C(O)N(R')-, -O-SO₂-N(R')-, -O-C(S)N(R')-, and -O-C(O)N(R')-, and X2d group consisting of -alkylene-, -alkenylene-, and -alkynylene-, or that two or more thereof are linked to each other;
X₃ is a direct linkage, or any one selected from the group consisting of -alkylene-, - O-, -S-, -C(O)-, -N(R')-, -SO-, -SO₂-, and a combination thereof;
B is any one selected from the group consisting of aryl, heteroaryl, cycloalkyl, heterocycloalkyl, and heterocycloalkenyl, which are unsubstituted or substituted with at least one selected from the group consisting of hydroxy, alkoxy, and oxo, or that two or more selected from the group consisting of aryl, heteroaryl, cycloalkyl, heterocycloalkyl, and heterocycloalkenyl, which are unsubstituted or substituted with at least one selected from the group consisting of hydroxy, alkoxy, and oxo, are directly linked, or linked via -alkylene-, -O-, -S-, -C(O)-, -N(R')-, -SO-, -SO₂-, or a combination thereof;
W₂' is hydrogen or any one selected from the group consisting of alkyl, alkenyl, and alkynyl unsubstituted or substituted with at least one selected from the group consisting of deuterium, halogen, alkyl, -alkylene-C(O)OR', alkylene-C(O)N(R')(R"), alkylene-OR', alkylene-SR', alkylene-N(R')(R"), alkylene-OP(O)(OR')(OR"), alkylene-P(O)(OR')(OR"), alkylene-OP(O)(R')(R"), -N(R')(R"), -OR', -SR', -C(O)R', -SOR', -SO₂R', =N-OR', -C(O)OR', -C(O)N(R')(R"), -OC(O)OR', -OC(O)N(R')(R"), -N(R')C(O)O(R"), -N(R')SO(R'), - SON(R')(R"), -N(R')SO₂(R"), and -SO₂N(R')(R");
Y₁' is any one selected from the group consisting of alkyl, alkenyl, alkynyl, (CH₃CH₂O)-(CH₂CH₂O)ₙ₋₁CH₂-, and (CH₃CH₂O)-(CH₂CH₂O)ₙ₋₁CH₂CH₂-, wherein n is 1-4, unsubstituted or substituted with at least one selected from the group consisting of halogen, -N(R')(R"), -OR', -SR', -C(O)R', -SOR', SO₂R', =N-OR', -C(O)OR', -C(O)N(R')(R"), - N(R')SO(R'), -SON(R')(R"), -OC(O)OR', -OC(O)N(R')(R"), -N(R')C(O)O(R"), -N(R')SO₂(R"), and -SO₂N(R')(R");
Y₂' is any one selected from the group consisting of alkyl, alkenyl, alkynyl, heterocycloalkyl, and heterocycloalkenyl unsubstituted or substituted with at least one selected from the group consisting of halogen, -N(R')(R"), -OR', -SR', -C(O)R', -SOR', SO₂R', =N-OR', -C(O)OR', -C(O)N(R')(R"), -OC(O)OR', -OC(O)N(R')(R"), -N(R')C(O)O(R"), -N(R')SO(R'), -SON(R')(R"), -N(R')SO₂(R"), and -SO₂N(R')(R"); and
R' and R" in the functional groups are each independently hydrogen or alkyl.

2. The compound of claim 1, wherein, in Formula I to Formula IV above, each independently,
the W₁ above is any one selected from the group consisting of -alkylene-, -alkylene-O-, -alkylene-OSO₂-, and -alkylene-N(R')SO₂- unsubstituted or substituted with at least one selected from the group consisting of C(O)OR', C(O)N(R')(R"), alkylene-OR', alkylene-SR', and alkylene-OP(O)(OR')(OR");
the A above is any one selected from the group consisting of aryl, 5- to 10-membered heteroaryl containing at least one selected from the group consisting of N, O, and S, 3- to 10-membered cycloalkyl, 3- to 10-membered heterocycloalkyl containing at least one selected from the group consisting of N, O, and S, and 3- to 10-membered heterocycloalkenyl containing at least one selected from the group consisting of N, O, and S;
the R₁ and R₂ above are each independently any one selected from the group consisting of hydrogen, alkyl, alkenyl, halogen, OR', N(R')(R"), O-(alkylene)-OR', C(O)R', C(O)OR', C(O)N(R')(R"), C(NH)N(R')(R"), C(O)O-(alkylene)-OC(O)R', C(O)O-(alkylene)-methacryloyloxy, (alkylene)-OR', (alkylene)-SR', (alkylene)-N(R')(R"), (alkynylene)-N(R')(R"), (alkylene)-OP(O)(OR')(OR"), (alkylene)-OP(O)(benzyloxy)₂, (alkylene)-OP(O)(phenoxy)₂, (alkylene)-OP(O)(phenoxy)(NH-CH(R')C(O)OR"), (alkylene)-C(O)OR', aryl, heteroaryl, heterocycloalkyl, and heterocycloalkenyl, optionally the R₁ and R₂ above are each independently substituted with at least one halogen, and when R₁ and R₂ above are each independently aryl, heteroaryl, heterocycloalkyl, or heterocycloalkenyl, optionally the R₁ and R₂ above are each independently substituted with alkyl or N(R')(R") unsubstituted or substituted with at least one halogen at one or more positions;
the X₁ above is a direct linkage, any one selected from among the X1a group and the X1b group, or that any one selected from among the X1a group and the X1b group and any one selected from the X1c group are linked in the form of X1a-X1c or X1b-X1c;
the X₂ above is a direct linkage, or any one selected from the X2a group, the X2b group, and the X2c group, or that two or more thereof are linked to each other, or that any one or two or more selected from the X2b group, the X2c group, and the X2d group are linked in the form of X2b-X2d-X2b, X2b-X2d-X2c, X2c-X2d-X2b, or X2c-X2d-X2c;
the X₃ above is a direct linkage, or any one selected from the group consisting of - C(O)-, -N(R')-, and a combination thereof;
the B above is any one selected from the group consisting of aryl, 5- to 10-membered heteroaryl, 3- to 10-membered heterocycloalkyl, and 3- to 10-membered heterocycloalkenyl, which are unsubstituted or substituted with at least one selected from the group consisting of hydroxy, alkoxy, and oxo, or that two or more selected from the group consisting of aryl, 5- to 10-membered heteroaryl, 3- to 10-membered heterocycloalkyl, and 3- to 10-membered heterocycloalkenyl, which are unsubstituted or substituted with at least one selected from the group consisting of hydroxy and oxo, are directly linked, or linked via -alkylene-, -C(O)-, -N(R')-, or a combination thereof;
the W₂' above is hydrogen or any one selected from the group consisting of alkenyl and alkynyl unsubstituted or substituted with at least one selected from the group consisting of deuterium, halogen, alkyl, -alkylene-C(O)OR', alkylene-C(O)N(R')(R"), alkylene-OR', alkylene-SR', alkylene-N(R')(R"), alkylene-OP(O)(OR')(OR"), alkylene-P(O)(OR')(OR"), alkylene-OP(O)(R')(R"), -N(R')(R"), -OR', -SR', -C(O)R', -SOR', -SO₂R', =N-OR', -C(O)OR', -C(O)N(R')(R"), -OC(O)OR', -OC(O)N(R')(R"), -N(R')C(O)O(R"), -N(R')SO(R'), - SON(R')(R"), -N(R')SO₂(R"), and -SO₂N(R')(R");
the Y₁' above is any one selected from the group consisting of alkyl, alkenyl, alkynyl, (CH₃CH₂O)-(CH₂CH₂O)ₙ₋₁CH₂-, and (CH₃CH₂O)-(CH₂CH₂O)ₙ₋₁CH₂CH₂-, wherein n is 1-2, unsubstituted or substituted with at least one selected from the group consisting of halogen, -N(R')(R"), -OR', -SR', -C(O)R', -SOR', SO₂R', =N-OR', -C(O)OR', -C(O)N(R')(R"), - OC(O)OR', -OC(O)N(R')(R"), -N(R')C(O)O(R"), -N(R')SO(R'), -SON(R')(R"), -N(R')SO₂(R"), and -SO₂N(R')(R"); and
the Y₂' above is any one selected from the group consisting of alkyl, alkenyl, alkynyl, and heterocycloalkyl unsubstituted or substituted with at least one selected from the group consisting of halogen, -N(R')(R"), -OR', -SR', -C(O)R', -SOR', SO₂R', =N-OR', -C(O)OR', - C(O)N(R')(R"), -OC(O)OR', -OC(O)N(R')(R"), -N(R')C(O)O(R"), -N(R')SO(R'), - SON(R')(R"), -N(R')SO₂(R"), and -SO₂N(R')(R"),
or a pharmaceutically acceptable salt, isomer, solvate, hydrate, or prodrug thereof.

3. The compound of claim 2, wherein, in Formula I to Formula IV above, each independently,
the W₁ above is any one selected from the group consisting of -alkylene-, -alkylene-O-, -alkylene-OSO₂-, and -alkylene-N(R')SO₂- unsubstituted or substituted with at least one selected from the group consisting of alkylene-OR' and alkylene-OP(O)(OR')(OR");
the A above is any one selected from the group consisting of benzene, furan, benzofuran, pyridine, thiophene, imidazole, and oxazole;
the R₁ and R₂ above are each independently any one selected from the group consisting of hydrogen, alkyl, OR', C(O)R', C(O)OR', C(O)N(R')(R"), (alkylene)-OR', (alkylene)-SR', (alkylene)-OP(O)(OR')(OR"), heteroaryl, and heterocycloalkyl, optionally the R₁ and R₂ above are each independently substituted with at least one halogen, and when R₁ and R₂ above are each independently heteroaryl or heterocycloalkyl, optionally the R₁ and R₂ above are each independently substituted with alkyl or N(R')(R") unsubstituted or substituted with at least one halogen at one or more positions;
the X₁ above is a direct linkage, or the X1a group consists of -N(R')- and -O-, and the X1b group consists of -C(O)O-, -N(R')C(O)-, and -C(O)N(R')-,
the X₂ above is a direct linkage, or the X2a group consists of N(R')-, -O-, -C(O)-, and =N-O-, the X2b group consists of -N(R')C(O)-, and the X2c group consists of - C(O)N(R')-,
the B above is any one selected from the group consisting of 5- to 10-membered heteroaryl, 3- to 10-membered heterocycloalkyl, and 3- to 10-membered heterocycloalkenyl, which are unsubstituted or substituted with at least one selected from the group consisting of hydroxy and oxo, or two or more selected from the group consisting of 5- to 10-membered heteroaryl, 3- to 10-membered heterocycloalkyl, and 3- to 10-membered heterocycloalkenyl, which are unsubstituted, are directly linked, or linked via -alkylene-, -C(O)-, -N(R')-, or a combination thereof;
the W₂' is hydrogen, or alkynyl unsubstituted or substituted with at least one selected from the group consisting of halogen, alkylene-OR', alkylene-OP(O)(OR')(OR"), - N(R')(R"), -OR', and -C(O)OR';
the Y₁' is any one selected from the group consisting of alkyl, alkenyl, and alkynyl unsubstituted or substituted with at least one selected from the group consisting of halogen, -N(R')(R"), -OR', and -C(O)OR'; and
the Y₂' is any one selected from the group consisting of alkyl and heterocycloalkyl unsubstituted or substituted with at least one selected from the group consisting of halogen, -N(R')(R"), -OR', and -C(O)OR',
or a pharmaceutically acceptable salt, isomer, solvate, hydrate, or prodrug thereof

4. The compound of claim 3, wherein the compound is at least one selected from the group consisting of compounds listed in Tables 1 to 4 below, or a pharmaceutically acceptable salt, isomer, solvate, hydrate, or prodrug thereof:
**[Table 1]**
| No. | Compound |
|---|---|
| 1-1 | 4-(3-aminoprop-1-yn-1-yl)aniline hydrochloride |
| 1-3 | 3-(3-aminoprop-1-yn-1-yl)aniline hydrochloride |
| 3-1 | methyl 5-amino-2-(3-aminoprop-1-yn-1-yl)benzoate |
| 3-2 | 5-amino-2-(3-aminoprop-1-yn-1-yl)benzoic acid |
| 3-3 | (5-amino-2-(3-aminoprop-1-yn-1-yl)phenyl)methanol |
| 3-6 | (5-amino-2-(3-aminoprop-1-yn-1-yl)phenyl)methanethiol |
| 3-7 | 5-amino-2-(3-aminoprop-1-yn-1-yl)benzyl dimethyl phosphate |
| 3-8 | 5-amino-2-(3-aminoprop-1-yn-1-yl)benzyl dihydrogen phosphate |
| 3-9 | 5-amino-2-(3-aminoprop-1-yn-1-yl)benzyl di-tert-butyl phosphate |
| 3-10 | 5-amino-2-(3-aminoprop-1-yn-1-yl)benzyl dibenzyl phosphate |
| 3-11 | 5-amino-2-(3-aminoprop-1-yn-1-yl)benzyl diisopropyl phosphate |
| 3-12 | isopropyl (((5-amino-2-(3-aminoprop-1-yn-1-yl)benzyl)oxy)(phenoxy)phosphoryl)-L-alaninate |
| 3-25 | methyl 4-amino-2-(3-aminoprop-1-yn-1-yl)benzoate hydrochloride |
| 3-26 | 4-amino-2-(3-aminoprop-1-yn-1-yl)benzoic acid |
| 3-27 | (4-amino-2-(3-aminoprop-1-yn-1-yl)phenyl)methanol |
| 3-28 | (4-amino-2-(3-aminoprop-1-yn-1-yl)phenyl)methanethiol |
| 3-30 | 4-amino-2-(3-aminoprop-1-yn-1-yl)benzyl dimethyl phosphate |
| 3-31 | 4-amino-2-(3-aminoprop-1-yn-1-yl)benzyl di-tert-butyl phosphate |
| 3-32 | 4-amino-2-(3-aminoprop-1-yn-1-yl)benzyl dihydrogen phosphate |
| 3-33 | 4-amino-2-(3-aminoprop-1-yn-1-yl)benzyl diphenyl phosphate |
| 3-34 | 4-amino-2-(3-aminoprop-1-yn-1-yl)benzyl diisopropyl phosphate |
| 3-35 | isopropyl ((4-amino-2-(3-aminoprop-1-yn-1-yl)phenethoxy)(phenoxy)phosphoryl)-L-alaninate |
| 3-49 | 2-amino-4-(4-aminophenyl)but-3-yn-1-ol hydrochloride |
| 3-51 | 2-amino-4-(3-aminophenyl)but-3-yn-1-ol |
| 3-53 | 2-amino-4-(4-aminophenyl)but-3-yn-1-yl di-tert-butyl phosphate |
| 3-55 | 2-amino-4-(3-aminophenyl)but-3-yn-1-yl di-tert-butyl phosphate |
| 3-57 | 2-amino-4-(4-aminophenyl)but-3-yn-1-yl dihydrogen phosphate |
| 3-60 | 2-amino-4-(3-aminophenyl)butyl dihydrogen phosphate |
| 4-1 | 3,3'-(furan-2,5-diyl)bis(prop-2-yn-1-amine) |
| 4-10 | 3,3'-(thiophene-2,5-diyl)bis(prop-2-yn-1-amine) |
| 4-13 | 3,3'-(1H-pyrrole-2,5-diyl)bis(prop-2-yn-1-amine) |
| 4-16 | 3,3'-(furan-2,4-diyl)bis(prop-2-yn-1-amine) |
| 4-19 | 3,3'-(thiophene-2,4-diyl)bis(prop-2-yn-1-amine) |
| 4-25 | 3,3'-(furan-2,4-diyl)bis(prop-2-yn-1-amine) |
| 4-28 | 3,3'-(thiophene-2,4-diyl)bis(prop-2-yn-1-amine) |
| 4-31 | 3,3'-(1H-pyrrole-2,4-diyl)bis(prop-2-yn-1-amine) |
| 4-34 | 3,3'-(furan-3,4-diyl)bis(prop-2-yn-1-amine) |
| 4-40 | 3,3'-(1H-pyrrole-3,4-diyl)bis(prop-2-yn-1-amine) |
| 4-43 | 3,3'-(furan-2,3-diyl)bis(prop-2-yn-1-amine) |
| 4-52 | 3,3'-(furan-2,3-diyl)bis(prop-2-yn-1-amine) |
| 4-61 | 4-(5-(3-aminoprop-1-yn-1-yl)furan-2-yl)but-3-yn-1-amine |
| 4-62 | 4-(5-(3-aminoprop-1-yn-1-yl)thiophen-2-yl)but-3-yn-1-amine |
| 4-63 | 4-(5-(3-aminoprop-1-yn-1-yl)-1H-pyrrol-2-yl)but-3-yn-1-amine |
| 4-64 | 5-(5-(3-aminoprop-1-yn-1-yl)furan-2-yl)pent-4-yn-1-amine |
| 4-65 | 5-(5-(3-aminoprop-1-yn-1-yl)thiophen-2-yl)pent-4-yn-1-amine |
| 4-66 | 5-(5-(3-aminoprop-1-yn-1-yl)-1H-pyrrol-2-yl)pent-4-yn-1-amine |
| 4-67 | 6-(5-(3-aminoprop-1-yn-1-yl)furan-2-yl)hex-5-yn-1-amine |
| 4-68 | 6-(5-(3-aminoprop-1-yn-1-yl)thiophen-2-yl)hex-5-yn-1-amine |
| 4-69 | 6-(5-(3-aminoprop-1-yn-1-yl)-1H-pyrrol-2-yl)hex-5-yn-1-amine |
| 4-70 | 7-(5-(3-aminoprop-1-yn-1-yl)furan-2-yl)hept-6-yn-1-amine |
| 4-71 | 7-(5-(3-aminoprop-1-yn-1-yl)thiophen-2-yl)hept-6-yn-1-amine |
| 4-72 | 7-(5-(3-aminoprop-1-yn-1-yl)-1H-pyrrol-2-yl)hept-6-yn-1-amine |
| 4-73 | 8-(5-(3-aminoprop-1-yn-1-yl)furan-2-yl)oct-7-yn-1-amine |
| 4-74 | 8-(5-(3-aminoprop-1-yn-1-yl)thiophen-2-yl)oct-7-yn-1-amine |
| 4-75 | 8-(5-(3-aminoprop-1-yn-1-yl)-1H-pyrrol-2-yl)oct-7-yn-1-amine |
| 4-128 | 3,3'-(1,4-phenylene)bis(prop-2-yn-1-amine) |
| 4-129 | 3,3'-(1,3-phenylene)bis(prop-2-yn-1-amine) |
| 4-130 | 3,3'-(1H-imidazole-2,5-diyl)bis(prop-2-yn-1-amine) |
| 4-131 | 3,3'-(oxazole-2,5-diyl)bis(prop-2-yn-1-amine) |
| 4-147 | 4,4'-(furan-2,5-diyl)bis(but-3-yn-1-amine) |
| 4-177 | 3,3'-(pyridine-2,5-diyl)bis(prop-2-yn-1-amine) |
| 4-178 | 3,3'-(pyridine-2,6-diyl)bis(prop-2-yn-1-amine) |
| 4-179 | 3,3'-(pyridine-2,5-diyl)bis(prop-2-yn-1-amine) |
| 4-180 | 3-(5-(3-aminoprop-1-yn-1-yl)furan-2-yl)prop-2-yn-1, 1-d2-1-amine |
| 4-181 | 2-amino-4-(5-(3-aminoprop-1-yn-1-yl)furan-2-yl)but-3-ynoic acid |
| 4-182 | methyl 2,5-bis(3-aminoprop-1-yn-1-yl)furan-3-carboxylate |
| 4-183 | dimethyl 2,5-bis(3-aminoprop-1-yn-1-yl)furan-3,4-dicarboxylate |
| 4-184 | methyl 2,5-bis(3-aminoprop-1-yn-1-yl)furan-3-carboxylate |
| 4-185 | (2,5-bis(3-aminoprop-1-yn-1-yl)furan-3-yl)methanol |
| 4-186 | (2,5-bis(3-aminoprop-1-yn-1-yl)furan-3-yl)methanol |
| 4-187 | (2,5-bis(3-aminoprop-1-yn-1-yl)furan-3,4-diyl)dimethanol |
| 4-188 | (2,5-bis(3-aminoprop-1-yn-1-yl)furan-3-yl)methyl dihydrogen phosphate |
| 4-189 | (2,5-bis(3-aminoprop-1-yn-1-yl)furan-3-yl)methyl dihydrogen phosphate |
| 4-200 | 4-(5-(3-aminoprop-1-yn-1-yl)furan-2-yl)but-3-yn-1-amine |
| 4-201 | 4-(5-(3-aminoprop-1-yn-1-yl)furan-3-yl)but-3-yn-1-amine |
| 4-202 | 4,4'-(furan-2,4-diyl)bis(but-3-yn-1-amine) |
| 4-218 | 3,3'-(5-methylthiophene-2,4-diyl)bis(prop-2-yn-1-amine) |
| 4-219 | 4-(5-(3-aminoprop-1-yn-1-yl)-2-methylthiophen-3-yl)but-3-yn-1-amine |
| 4-223 | 3-(5-(3-(aminooxy)prop-1-yn-1-yl)thiophen-2-yl)prop-2-yn-1-amine |
| 5-52 | ethyl 2,5-bis(3-aminoprop-1-yn-1-yl)oxazole-4-carboxylate |
| 5-116 | methyl 2,4-bis(3-aminoprop-1-yn-1-yl)benzoate dihydrochloride |
**[Table 2]**
| No. | Compound |
|---|---|
| 4-6 | 2-(3-aminoprop-1-yn-1-yl)-5-(3-aminopropanamido)benzoic acid |
| 4-109 | methyl 2-(4-aminobut-1-yn-1-yl)-5-(3-aminopropanamido)benzoate |
| 4-110 | 2-(4-aminobut-1-yn-1-yl)-5-(3-aminopropanamido)benzoic acid |
| 4-111 | 3-amino-N-(4-(4-aminobut-1-yn-1-yl)-3-(hydroxymethyl)phenyl)propanamide |
| 4-112 | methyl 2-(5-aminopent-1-yn-1-yl)-5-(3-aminopropanamido)benzoate |
| 4-113 | 2-(5-aminopent-1-yn-1-yl)-5-(3-aminopropanamido)benzoic acid |
| 4-114 | 3-amino-N-(4-(5-aminopent-1-yn-1-yl)-3-(hydroxymethyl)phenyl)propanamide |
| 4-115 | methyl 2-(6-aminohex-1-yn-1-yl)-5-(3-aminopropanamido)benzoate |
| 4-116 | 2-(6-aminohex-1-yn-1-yl)-5-(3-aminopropanamido)benzoic acid |
| 4-117 | 3-amino-N-(4-(6-aminohex-1-yn-1-yl)-3-(hydroxymethyl)phenyl)propanamide |
| 4-118 | methyl 2-(7-aminohept-1-yn-1-yl)-5-(3-aminopropanamido)benzoate |
| 4-119 | 2-(7-aminohept-1-yn-1-yl)-5-(3-aminopropanamido)benzoic acid |
| 4-120 | 3-amino-N-(4-(7-aminohept-1-yn-1-yl)-3-(hydroxymethyl)phenyl)propanamide |
| 4-121 | methyl 2-(8-aminooct-1-yn-1-yl)-5-(3-aminopropanamido)benzoate |
| 4-122 | 2-(8-aminooct-1-yn-1-yl)-5-(3-aminopropanamido)benzoic acid |
| 4-123 | 3-amino-N-(4-(8-aminooct-1-yn-1-yl)-3-(hydroxymethyl)phenyl)propanamide |
| 4-124 | 3-amino-N-(4-(3-aminoprop-1-yn-1-yl)phenyl)propanamide |
| 4-125 | 3-amino-N-(4-(3-aminoprop-1-yn-1-yl)-3-(hydroxymethyl)phenyl)propanamide |
| 4-126 | methyl 2-(3-aminoprop-1-yn-1-yl)-5-(3-aminopropanamido)benzoate |
| 4-127 | 3-amino-N-(4-(3-aminoprop-1-yn-1-yl)-3,5-bis(hydroxymethyl)phenyl)propanamide |
| 4-148 | N-(4-aminobut-2-yn-1-yl)-5-(3-aminoprop-1-yn-1-yl)furan-2-carboxamide |
| 4-149 | N-(4-aminobutyl)-5-(3-aminoprop-1-yn-1-yl)furan-2-carboxamide |
| 4-150 | 5-(4-aminobut-1-yn-1-yl)-N-(4-aminobut-2-yn-1-yl)furan-2-carboxamide |
| 4-161 | 3-amino-N-(3-(3-aminoprop-1-yn-1-yl)phenyl)propanamide |
| 4-162 | 4-amino-N-(4-(3-aminoprop-1-yn-1-yl)phenyl)butanamide dihydrochloride |
| 4-163 | 5-amino-N-(4-(3-aminoprop-1-yn-1-yl)phenyl)pentanamide |
| 4-164 | 5-amino-N-(3-(3-aminoprop-1-yn-1-yl)phenyl)pentanamide |
| 4-165 | 6-amino-N-(4-(3-aminoprop-1-yn-1-yl)phenyl)hexanamide |
| 4-166 | 5-(3-aminoprop-1-yn-1-yl)-N-(3-aminopropyl)furan-2-carboxamide dihydrochloride |
| 4-167 | N-(5-aminopentyl)-5-(3-aminoprop-1-yn-1-yl)furan-2-carboxamide |
| 4-168 | 4-amino-N-(4-(3-aminoprop-1-yn-1-yl)-3-methoxyphenyl)butanamide |
| 4-169 | 5-amino-N-(4-(3-aminoprop-1-yn-1-yl)-3-methoxyphenyl)pentanamide |
| 4-170 | 5-amino-N-(3-(3-aminoprop-1-yn-1-yl)-4-methoxyphenyl)pentanamide |
| 4-171 | 6-amino-N-(4-(3-aminoprop-1-yn-1-yl)-3-methoxyphenyl)hexanamide |
| 4-192 | N-(4-aminobutyl)-5-(3-aminoprop-1-yn-1-yl)-3-methylfuran-2-carboxamide |
| 4-199 | 4-amino-N-((5-(3-aminoprop-1-yn-1-yl)furan-2-yl)methyl)butanamide |
| 4-203 | N-(4-aminobutyl)-4-(3-aminoprop-1-yn-1-yl)furan-2-carboxamide |
| 4-204 | 4-(4-aminobut-1-yn-1-yl)-N-(4-aminobutyl)furan-2-carboxamide |
| 4-205 | 4-(3-aminoprop-1-yn-1-yl)-N-(3-aminopropyl)furan-2-carboxamide |
| 4-206 | N-(5-aminopentyl)-4-(3-aminoprop-1-yn-1-yl)furan-2-carboxamide |
| 4-209 | N-(4-aminobutyl)-5-(3-aminoprop-1-yn-1-yl)benzofuran-2-carboxamide |
| 4-210 | 5-(3-aminoprop-1-yn-1-yl)-N-(3-aminopropyl)benzofuran-2-carboxamide |
| 4-214 | N-(4-aminobutyl)-5-(3-aminoprop-1-yn-1-yl)-7-methoxybenzofuran-2-carboxamide |
| 4-215 | N-(4-aminobutyl)-5-(3-aminoprop-1-yn-1-yl)-6-methoxybenzofuran-2-carboxamide |
| 4-216 | 5-(3-aminoprop-1-yn-1-yl)-N-(3-aminopropyl)-6-methoxybenzofuran-2-carboxamide |
| 4-217 | 5-(3-aminoprop-1-yn-1-yl)-N-(3-aminopropyl)-7-methoxybenzofuran-2-carboxamide |
| 4-224 | N-(7-aminoheptyl)-5-(3-aminoprop-1-yn-1-yl)furan-2-carboxamide |
| 4-225 | N-(9-aminononyl)-5-(3-aminoprop-1-yn-1-yl)furan-2-carboxamide |
| 4-228 | methyl 4-(4-aminobutanamido)-2-(3-aminoprop-1-yn-1-yl)benzoate dihydrochloride |
| 4-229 | N-(4-aminobutyl)-4-(3-aminoprop-1-yn-1-yl)benzamide |
| 4-231 | 5-(3-aminoprop-1-yn-1-yl)-N-(2-aminopyridin-4-yl)furan-2-carboxamide |
| 4-232 | 5-(3-(aminooxy)prop-1-yn-1-yl)-N-(3-aminopropyl)furan-2-carboxamide |
| 4-233 | methyl 2-amino-4-(5-((3-aminopropyl)carbamoyl)furan-2-yl)but-3-ynoate |
| 4-234 | methyl 2-(3-(aminooxy)prop-1-yn-1-yl)-5-((3-aminopropyl)carbamoyl)furan-3-carboxylate |
| 4-235 | N-(8-aminooctyl)-5-(3-aminoprop-1-yn-1-yl)furan-2-carboxamide |
| 5-1 | ethyl 4-(4-aminobutanamido)-2-(3-aminoprop-1-yn-1-yl)benzoate |
| 5-2 | isopropyl 4-(4-aminobutanamido)-2-(3-aminoprop-1-yn-1-yl)benzoate |
| 5-3 | cyclopropyl 4-(4-aminobutanamido)-2-(3-aminoprop-1-yn-1-yl)benzoate |
| 5-4 | methyl 2-(3-aminoprop-1-yn-1-yl)-4-(3-aminopropanamido)benzoate |
| 5-5 | methyl 4-(5-aminopentanamido)-2-(3-aminoprop-1-yn-1-yl)benzoate |
| 5-6 | methyl 4-(6-aminohexanamido)-2-(3-aminoprop-1-yn-1-yl)benzoate |
| 5-8 | 4-amino-N-(4-(3-aminoprop-1-yn-1-yl)-3-(hydroxymethyl)phenyl)butanamide trifluoroacetic acid |
| 5-9 | 5-amino-N-(4-(3-aminoprop-1-yn-1-yl)-3-(hydroxymethyl)phenyl)pentanamide |
| 5-10 | methyl 4-((4-aminobutyl)amino)-2-(3-aminoprop-1-yn-1-yl)benzoate |
| 5-11 | methyl 2-(3-aminoprop-1-yn-1-yl)-4-((3-aminopropyl)amino)benzoate |
| 5-12 | methyl 4-(4-aminobutoxy)-2-(3-aminoprop-1-yn-1-yl)benzoate |
| 5-13 | methyl 2-(3-aminoprop-1-yn-1-yl)-4-(3-aminopropoxy)benzoate |
| 5-14 | methyl 2-(3-aminoprop-1-yn-1-yl)-4-((3-aminopropanoyl)oxy)benzoate |
| 5-15 | methyl 4-((4-aminobutanoyl)oxy)-2-(3-aminoprop-1-yn-1-yl)benzoate |
| 5-16 | methyl 4-((5-aminopentanoyl)oxy)-2-(3-aminoprop-1-yn-1-yl)benzoate |
| 5-17 | methyl 4-((6-aminohexanoyl)oxy)-2-(3-aminoprop-1-yn-1-yl)benzoate |
| 5-18 | methyl 2-(3-aminoprop-1-yn-1-yl)-4-((3-aminopropyl)carbamoyl)benzoate dihydrochloride |
| 5-19 | methyl 4-((2-aminoethyl)carbamoyl)-2-(3-aminoprop-1-yn-1-yl)benzoate |
| 5-20 | methyl 4-((4-aminobutyl)carbamoyl)-2-(3-aminoprop-1-yn-1-yl)benzoate |
| 5-21 | methyl 4-((5-aminopentyl)carbamoyl)-2-(3-aminoprop-1-yn-1-yl)benzoate |
| 5-30 | methyl 4-((4-aminobut-2-yn-1-yl)carbamoyl)-2-(3-aminoprop-1-yn-1-yl)benzoate |
| 5-31 | methyl (Z)-4-((4-aminobut-2-en-1-yl)carbamoyl)-2-(3-aminoprop-1-yn-1-yl)benzoate |
| 5-34 | methyl 2-(3-aminoprop-1-yn-1-yl)-4-(((1r,4r)-4-hydroxycyclohexyl)amino)benzoate |
| 5-35 | methyl 4-((4-aminocyclohexyl)amino)-2-(3-aminoprop-1-yn-1-yl)benzoate |
| 5-49 | methyl 4-(4-aminobicyclo[2.2.2]octane-1-carboxamido)-2-(3-aminoprop-1-yn-1-yl)benzoate |
| 5-50 | methyl 4-(1-(2-aminoethyl)cyclopropane-1-carboxamido)-2-(3-aminoprop-1-yn-1-yl)benzoate |
| 5-53 | 4-(4-aminobutanamido)-2-(3-aminoprop-1-yn-1-yl)benzoic acid |
| 5-54 | difluoromethyl 4-(4-aminobutanamido)-2-(3-aminoprop-1-yn-1-yl)benzoate |
| 5-55 | 4-amino-N-(3-(3-aminoprop-1-yn-1-yl)-4-(1-(trifluoromethyl)cyclopropane-1-carbonyl)phenyl)butanamide |
| 5-56 | 4-amino-N-(3-(3-aminoprop-1-yn-1-yl)-4-(1-fluorocyclopropane-1-carbonyl)phenyl)butanamide |
| 5-57 | 4-amino-N-(3-(3-aminoprop-1-yn-1-yl)-4-(1-fluorocyclobutane-1-carbonyl)phenyl)butanamide |
| 5-58 | 1,1-difluoroethyl 4-(4-aminobutanamido)-2-(3-aminoprop-1-yn-1-yl)benzoate |
| 5-59 | 4-(4-aminobutanamido)-2-(3-aminoprop-1-yn-1-yl)benzamide |
| 5-60 | 4-(4-aminobutanamido)-2-(3-aminoprop-1-yn-1-yl)-N-methylbenzamide |
| 5-61 | 4-(4-aminobutanamido)-2-(3-aminoprop-1-yn-1-yl)-N,N-dimethylbenzamide |
| 5-62 | 4-amino-N-(3-(3-aminoprop-1-yn-1-yl)-4-hydroxyphenyl)butanamide |
| 5-63 | 4-amino-N-(3-(3-aminoprop-1-yn-1-yl)-4-methoxyphenyl)butanamide |
| 5-64 | 4-amino-N-(3-(3-aminoprop-1-yn-1-yl)-4-(2-methoxyethoxy)phenyl)butanamide |
| 5-65 | 4-amino-N-(3-(3-aminoprop-1-yn-1-yl)-4-methylphenyl)butanamide |
| 5-66 | N-(4-acetyl-3-(3-aminoprop-1-yn-1-yl)phenyl)-4-aminobutanamide |
| 5-67 | 4-amino-N-(3-(3-aminoprop-1-yn-1-yl)-4-(2-hydroxyethyl)phenyl)butanamide |
| 5-68 | 4-amino-N-(4-(aminomethyl)-3-(3-aminoprop-1-yn-1-yl)phenyl)butanamide |
| 5-69 | 4-amino-N-(4-amino-3-(3-aminoprop-1-yn-1-yl)phenyl)butanamide |
| 5-70 | 4-amino-N-(3-(3-aminoprop-1-yn-1-yl)-4-(5-methyl-1,3,4-oxadiazol-2-yl)phenyl)butanamide |
| 5-71 | 4-amino-N-(3-(3-aminoprop-1-yn-1-yl)-4-(3-methyl-1 ,2,4-oxadiazol-5-yl)phenyl)butanamide |
| 5-72 | 4-amino-N-(3-(3-aminoprop-1-yn-1-yl)-4-(3-(methylamino)oxetan-3-yl)phenyl)butanamide |
| 5-73 | (E)-4-amino-N-(3-(3-aminoprop-1-yn-1-yl)-4-(2-fluoroprop-1-en-1-yl)phenyl)butanamide |
| 5-74 | 4-amino-N-(3-(3-aminoprop-1-yn-1-yl)-4-carbamimidoylphenyl)butanamide |
| 5-75 | 4-amino-N-(3-(3-aminoprop-1-yn-1-yl)-4-(trifluoromethyl)phenyl)butanamide |
| 5-76 | 4-amino-N-(3-(3-aminoprop-1-yn-1-yl)-4-chlorophenyl)butanamide |
| 5-77 | 4-amino-N-(3-(3-aminoprop-1-yn-1-yl)-4-fluorophenyl)butanamide |
| 5-78 | methyl 4-(4-aminobutanamido)-2-(3-aminoprop-1-yn-1-yl)-6-methylbenzoate |
| 5-79 | methyl 4-(5-aminopent-1-yn-1-yl)-2-(3-aminoprop-1-yn-1-yl)benzoate |
| 5-80 | methyl 4-(4-aminobut-1-yn-1-yl)-2-(3-aminoprop-1-yn-1-yl)benzoate |
| 5-81 | 4-amino-N-(3-(3-aminoprop-1-yn-1-yl)-4-(2-(trifluoromethyl)pyrimidin-5-yl)phenyl)butanamide dihydrochloride |
| 5-82 | 4-(4-aminobutanamido)-2-(3-aminoprop-1-yn-1-yl)benzyl dihydrogen phosphate |
| 5-83 | 4-(4-aminobutanamido)-2-(3-aminoprop-1-yn-1-yl)benzyl di-tert-butyl phosphate |
| 5-87 | methyl 4-(4-aminobutanamido)-2-(3,4-diamino-4-oxobut-1-yn-1-yl)benzoate |
| 5-89 | methyl 3-(4-aminobutanamido)-5-(3-aminoprop-1-yn-1-yl)benzoate |
| 5-90 | methyl 2-(4-aminobutanamido)-4-(3-aminoprop-1-yn-1-yl)benzoate |
| 5-92 | methyl 4-(4-aminobutanamido)-2-((1-aminocyclopropyl)ethynyl)benzoate |
| 5-93 | 2-amino-4-(5-(4-aminobutanamido)-2-(methoxycarbonyl)phenyl)but-3-ynoic acid |
| 5-94 | methyl 2-(3-amino-4-methoxy-4-oxobut-1-yn-1-yl)-4-(4-aminobutanamido)benzoate |
| 5-95 | methyl 2-(3-amino-4-hydroxybut-1-yn-1-yl)-4-(4-aminobutanamido)benzoate |
| 5-96 | methyl 6-(4-aminobutanamido)-3-(3-aminoprop-1-yn-1-yl)picolinate |
| 5-97 | dimethyl 4-(4-aminobutanamido)-2-(3-aminoprop-1-yn-1-yl)isophthalate |
| 5-98 | methyl 2-(4-aminobutanamido)-6-(3-aminoprop-1-yn-1-yl)nicotinate |
| 5-99 | 2-(4-(4-aminobutanamido)-2-(3-aminoprop-1-yn-1-yl)phenyl)acetic acid |
| 5-100 | ethyl 2-(4-(4-aminobutanamido)-2-(3-aminoprop-1-yn-1-yl)phenyl)acetate |
| 5-101 | acetoxymethyl 4-(4-am inobutanam ido)-2-(3-am inoprop-1-yn-1-yl)benzoate |
| 5-102 | (methacryloyloxy)methyl 4-(4-aminobutanamido)-2-(3-aminoprop-1-yn-1-yl)benzoate |
| 5-103 | (pivaloyloxy)methyl 4-(4-aminobutanamido)-2-(3-aminoprop-1-yn-1-yl)benzoate |
| 5-104 | 6-amino-N-(3-(3-aminoprop-1-yn-1-yl)phenyl)hexanamide |
| 5-106 | 5-((3-(3-aminoprop-1-yn-1-yl)-4-(methoxycarbonyl)phenyl)amino)-5-oxopentanoic acid |
| 5-107 | 8-amino-N-(3-(3-aminoprop-1-yn-1-yl)phenyl)octanamide |
| 5-108 | 11-amino-N-(3-(3-aminoprop-1-yn-1-yl)phenyl)undecanamide |
| 5-109 | 11-((3-(3-aminoprop-1-yn-1-yl)phenyl)amino)-11-oxoundecanoic acid hydrochloride |
| 5-110 | 11-((3-(3-aminoprop-1-yn-1-yl)-4-(methoxycarbonyl)phenyl)amino)-11-oxoundecanoic acid hydrochloride |
| 5-113 | methyl 4-(5-((7-aminoheptyl)amino)-5-oxopentanamido)-2-(3-aminoprop-1-yn-1-yl)benzoate |
| 5-115 | methyl 2-(3-aminoprop-1-yn-1-yl)-4-(4-hydroxybutanamido)benzoate |
| 5-121 | N-(7-aminoheptyl)-2,4-bis(3-aminoprop-1-yn-1-yl)benzamide |
| 5-122 | methyl 4-(3-(4-aminobutanamido)prop-1-yn-1-yl)-2-(3-aminoprop-1-yn-1-yl)benzoate |
| 5-123 | 4-amino-N-(3-(3-(3-aminoprop-1-yn-1-yl)phenyl)prop-2-yn-1-yl)butanamide |
| 5-126 | 20-amino-N-(4-(3-aminoprop-1-yn-1-yl)phenyl)-3,6,9,12,15,18-hexaoxaicosanamide |
| 5-127 | 20-amino-N-(3-(3-aminoprop-1-yn-1-yl)phenyl)-3,6,9,12,15,18-hexaoxaicosanamide |
| 5-135 | 8-amino-N-(4-(3-aminoprop-1-yn-1-yl)-3-(hydroxymethyl)phenyl)octanamide |
| 5-148 | methyl 2-(3-acetamidoprop-1-yn-1-yl)-4-(4-aminobutanamido)benzoate |
| 5-149 | methyl 2-(3-aminoprop-1-yn-1-yl)-4-(4-(methylamino)butanamido)benzoate |
| 5-150 | methyl 4-(4-amino-N-methylbutanamido)-2-(3-aminoprop-1-yn-1-yl)benzoate hydrochloride |
| 5-169 | 2-(3-aminoprop-1-yn-1-yl)-N-(3-aminopropyl)oxazole-5-carboxamide |
| 5-170 | 4-amino-N-(3-(3-aminoprop-1-yn-1-yl)-4-morpholinophenyl)butanamide |
| 5-171 | 4-amino-N-(3-(3-aminoprop-1-yn-1-yl)-4-(4-methylpiperazin-1-yl)phenyl)butanamide |
**[Table 3]**
| No. | Compound |
|---|---|
| 4-2 | methyl 2-(3-aminoprop-1-yn-1-yl)-5-(piperazin-1-yl)benzoate hydrochloride |
| 4-3 | 2-(3-aminoprop-1-yn-1-yl)-5-(piperazin-1-yl)benzoic acid |
| 4-4 | (2-(3-aminoprop-1-yn-1-yl)-5-(piperazin-1-yl)phenyl)methanol |
| 4-94 | methyl 2-(4-aminobut-1-yn-1-yl)-5-(piperazin-1-yl)benzoate |
| 4-95 | 2-(4-aminobut-1-yn-1-yl)-5-(piperazin-1-yl)benzoic acid |
| 4-96 | (2-(4-aminobut-1-yn-1-yl)-5-(piperazin-1-yl)phenyl)methanol |
| 4-97 | methyl 2-(5-aminopent-1-yn-1-yl)-5-(piperazin-1-yl)benzoate |
| 4-98 | 2-(5-aminopent-1-yn-1-yl)-5-(piperazin-1-yl)benzoic acid |
| 4-99 | (2-(5-aminopent-1-yn-1-yl)-5-(piperazin-1-yl)phenyl)methanol |
| 4-100 | methyl 2-(6-aminohex-1-yn-1-yl)-5-(piperazin-1-yl)benzoate |
| 4-101 | 2-(6-aminohex-1-yn-1-yl)-5-(piperazin-1-yl)benzoic acid |
| 4-102 | (2-(6-aminohex-1-yn-1-yl)-5-(piperazin-1-yl)phenyl)methanol |
| 4-103 | methyl 2-(7-aminohept-1-yn-1-yl)-5-(piperazin-1-yl)benzoate |
| 4-104 | 2-(7-aminohept-1-yn-1-yl)-5-(piperazin-1-yl)benzoic acid |
| 4-105 | (2-(7-aminohept-1-yn-1-yl)-5-(piperazin-1-yl)phenyl)methanol |
| 4-106 | methyl 2-(8-aminooct-1-yn-1-yl)-5-(piperazin-1-yl)benzoate |
| 4-107 | 2-(8-aminooct-1-yn-1-yl)-5-(piperazin-1-yl)benzoic acid |
| 4-108 | (2-(8-aminooct-1-yn-1-yl)-5-(piperazin-1-yl)phenyl)methanol |
| 4-134 | 3-(4-([1,4'-bipiperidin]-4-yl)phenyl)prop-2-yn-1-amine |
| 4-135 | 3-(4-(1-(azetidin-3-yl)piperidin-4-yl)phenyl)prop-2-yn-1-amine |
| 4-136 | 3-(4-(piperidin-4-yl)phenyl)prop-2-yn-1-amine |
| 4-146 | 3-(4-(1,2,3,6-tetrahydropyridin-4-yl)phenyl)prop-2-yn-1-amine |
| 4-226 | 3-(4-(piperazin-1-yl)phenyl)prop-2-yn-1-amine |
| 4-227 | (5-(3-aminoprop-1-yn-1-yl)furan-2-yl)(piperazin-1-yl)methanone |
| 5-7 | methyl 2-(3-aminoprop-1-yn-1-yl)-4-(piperazin-1-yl)benzoate hydrochloride |
| 5-22 | methyl 2-(3-aminoprop-1-yn-1-yl)-4-(piperidin-4-ylcarbamoyl)benzoate |
| 5-23 | methyl 2-(3-aminoprop-1-yn-1-yl)-4-(piperidine-4-carboxamido)benzoate hydrochloride |
| 5-24 | methyl 2-(3-aminoprop-1-yn-1-yl)-4-(2-(piperidine-4-carboxamido)acetamido)benzoate |
| 5-25 | methyl 2-(3-aminoprop-1-yn-1-yl)-4-(3-(piperidine-4-carboxamido)propanamido)benzoate |
| 5-26 | methyl 2-(3-aminoprop-1-yn-1-yl)-4-(4-(piperidine-4-carboxamido)butanamido)benzoate |
| 5-27 | methyl 2-(3-aminoprop-1-yn-1-yl)-4-((3-(piperidine-4-carboxamido)propyl)carbamoyl)benzoate |
| 5-28 | methyl 2-(3-aminoprop-1-yn-1-yl)-4-(3-(piperidine-4-carboxamido)prop-1-yn-1-yl)benzoate dihydrochloride |
| 5-29 | methyl 2-(3-aminoprop-1-yn-1-yl)-4-(4-(piperidin-4-ylmethyl)piperazin-1-yl)benzoate dihydrochloride |
| 5-36 | methyl 2-(3-aminoprop-1-yn-1-yl)-4-(piperidin-4-yloxy)benzoate |
| 5-37 | methyl 2-(3-aminoprop-1-yn-1-yl)-4-(4-(azetidin-3-yl)piperazin-1-yl)benzoate |
| 5-38 | methyl 2-(3-aminoprop-1-yn-1-yl)-4-(2,7-diazaspiro[3.5]nonan-7-yl)benzoate |
| 5-39 | methyl 2-(3-aminoprop-1-yn-1-yl)-4-(2,7-diazaspiro[3.5]nonane-7-carbonyl)benzoate dihydrochloride |
| 5-40 | methyl 2-(3-aminoprop-1-yn-1-yl)-4-(2,6-diazaspiro[3.3]heptan-2-yl)benzoate dihydrochloride |
| 5-41 | methyl 2-(3-aminoprop-1-yn-1-yl)-4-(2,6-diazaspiro[3.3]heptane-2-carbonyl)benzoate dihydrochloride |
| 5-42 | methyl 4-((2-azaspiro[3.3]heptan-6-yl)carbamoyl)-2-(3-aminoprop-1-yn-1-yl)benzoate |
| 5-43 | methyl 4-(((2-azaspiro[3.3]heptan-6-ylidene)amino)oxy)-2-(3-aminoprop-1-yn-1-yl)benzoate |
| 5-44 | methyl 2-(3-aminoprop-1-yn-1-yl)-4-(hexahydropyrrolo[3,4-c]pyrrol-2(1H)-yl)benzoate |
| 5-45 | methyl 2-(3-aminoprop-1-yn-1-yl)-4-(octahydropyrrolo[3,4-c]pyrrole-2-carbonyl)benzoate |
| 5-46 | methyl 2-(3-aminoprop-1-yn-1-yl)-4-(4-(azetidin-3-yl)piperidin-1-yl)benzoate |
| 5-47 | methyl 2-(3-aminoprop-1-yn-1-yl)-4-(3-(piperidin-4-yl)azetidin-1-yl)benzoate |
| 5-48 | methyl 2-(3-aminoprop-1-yn-1-yl)-4-(3,6-diazabicyclo[3.1.1]heptan-3-yl)benzoate |
| 5-85 | methyl 2-(3-aminoprop-1-yn-1-yl)-4-((piperidin-4-ylmethyl)amino)benzoate |
| 5-86 | methyl 2-(3-aminoprop-1-yn-1-yl)-4-(piperidin-4-ylamino)benzoate dihydrochloride |
| 5-88 | 4-(4-(3-aminoprop-1-yn-1-yl)phenyl)piperidin-4-ol |
| 5-112 | methyl 3-(3-aminoprop-1-yn-1-yl)-6-(piperidine-4-carboxamido)picolinate |
| 5-118 | methyl 2-(3-aminoprop-1-yn-1-yl)-5-(3,6-diazabicyclo[3.1.1]heptan-3-yl)benzoate |
| 5-119 | methyl 2-(3-aminoprop-1-yn-1-yl)-5-(3-oxopiperazin-1-yl)benzoate |
| 5-120 | methyl 2-(3-aminoprop-1-yn-1-yl)-4-(4-(piperidine-4-carbonyl)piperazin-1-yl)benzoate |
| 5-124 | (5-(4-(3-aminoprop-1-yn-1-yl)phenyl)furan-2-yl)(4-(piperidin-4-ylmethyl)piperazin-1-yl)methanone |
| 5-125 | (5-(4-(3-aminoprop-1-yn-1-yl)phenyl)furan-2-yl)(piperazin-1-yl)methanone |
| 5-129 | 5-(4-(3-aminoprop-1-yn-1-yl)phenyl)-N-(piperidin-4-yl)furan-2-carboxamide |
| 5-131 | N-(4-(3-aminoprop-1-yn-1-yl)phenyl)piperidine-4-carboxamide |
| 5-134 | N-(4-(3-aminoprop-1-yn-1-yl)-3-(hydroxymethyl)phenyl)piperidine-4-carboxamide |
| 5-139 | 2-(3-aminoprop-1-yn-1-yl)-4-((piperidin-4-ylmethyl)amino)benzoic acid |
| 5-140 | methyl 2-(3-aminoprop-1-yn-1-yl)-4-(piperazine-1-carbonyl)benzoate |
| 5-152 | methyl 2-(3-aminoprop-1-yn-1-yl)-4-(N-methylpiperidine-4-carboxamido)benzoate |
| 5-153 | methyl 2-(3-aminoprop-1-yn-1-yl)-4-(methyl(piperidin-4-yl)carbamoyl)benzoate |
| 5-154 | methyl 4-(piperidine-4-carboxamido)-2-(3-(sulfamoylamino)prop-1-yn-1-yl)benzoate |
| 5-155 | methyl 2-((1-aminocyclopropyl)ethynyl)-4-(piperidine-4-carboxamido)benzoate |
| 5-156 | methyl 2-(3-(((4-chlorophenyl)(5-fluoro-2-hydroxyphenyl)methylene)amino)prop-1-yn-1-yl)-4-(piperidine-4-carboxamido)benzoate |
| 5-157 | methyl 2-(3-(((1-(isobutyryloxy)ethoxy)carbonyl)amino)prop-1-yn-1-yl)-4-(piperidine-4-carboxamido)benzoate |
| 5-158 | methyl 4-(piperidine-4-carboxamido)-2-(3-(sulfamoyloxy)prop-1-yn-1-yl)benzoate |
| 5-159 | methyl 2-(3-amino-4-mercaptobut-1-yn-1-yl)-4-(piperidin-4-ylcarbamoyl)benzoate |
| 5-160 | 5-(3-(3-aminoprop-1-yn-1-yl)phenyl)-N-(piperidin-4-yl)furan-2-carboxamide |
| 5-162 | methyl 2-(3-(((1-(isobutyryloxy)ethoxy)carbonyl)amino)prop-1-yn-1-yl)-4-(2,6-diazaspiro[3.3]heptan-2-yl)benzoate |
| 5-167 | methyl 3-(3-aminoprop-1-yn-1-yl)-6-(piperidine-4-carboxamido)picolinate |
| 5-168 | methyl 2-(3-aminoprop-1-yn-1-yl)-4-(2,7-diazaspiro[3.5]nonan-2-yl)benzoate dihydrochloride |
**[Table 4]**
| No. | Compound |
|---|---|
| 4-207 | N-(4-aminobut-2-yn-1-yl)-5-(4-(3-aminoprop-1-yn-1-yl)phenyl)furan-2-carboxamide |
| 4-208 | N-(4-aminobutyl)-5-(4-(3-aminoprop-1-yn-1-yl)phenyl)furan-2-carboxamide |
| 4-211 | 5-(4-(3-aminoprop-1-yn-1-yl)phenyl)-N-(3-aminopropyl)furan-2-carboxamide |
| 5-32 | methyl 2-(3-aminoprop-1-yn-1-yl)-4-(4-(3-aminopropanoyl)piperazin-1-yl)benzoate |
| 5-33 | methyl 2-(3-aminoprop-1-yn-1-yl)-5-(4-(3-aminopropanoyl)piperazin-1-yl)benzoate |
| 5-51 | methyl 4-(5-amino-1,3,4-oxadiazol-2-yl)-2-(3-aminoprop-1-yn-1-yl)benzoate |
| 5-111 | N-(7-aminoheptyl)-5-(4-(3-aminoprop-1-yn-1-yl)phenyl)furan-2-carboxamide |
| 5-114 | methyl 4-(4-(6-aminohexanoyl)piperazin-1-yl)-2-(3-aminoprop-1-yn-1-yl)benzoate |
| 5-117 | methyl 4-(4-aminopiperidin-1-yl)-2-(3-aminoprop-1-yn-1-yl)benzoate |
| 5-128 | (4-aminopiperidin-1-yl)(5-(4-(3-aminoprop-1-yn-1-yl)phenyl)furan-2-yl)methanone |
| 5-130 | N-(4-aminobutyl)-1-(5-(4-(3-aminoprop-1-yn-1-yl)phenyl)furan-2-carbonyl)piperidine-4-carboxamide |
| 5-132 | 1-(4-aminobutanoyl)-N-(4-(3-aminoprop-1-yn-1-yl)phenyl)piperidine-4-carboxamide |
| 5-133 | 1-(4-aminobutanoyl)-N-(4-(3-aminoprop-1-yn-1-yl)-3-(hydroxymethyl)phenyl)piperidine-4-carboxamide |
| 5-136 | N-(7-aminoheptyl)-5-(4-(3-aminoprop-1-yn-1-yl)-3-(hydroxymethyl)phenyl)furan-2-carboxamide |
| 5-137 | N-(4-aminobutyl)-5-(6-(3-aminoprop-1-yn-1-yl)pyridin-3-yl)furan-2-carboxamide |
| 5-138 | N-(7-aminoheptyl)-5-(6-(3-aminoprop-1-yn-1-yl)pyridin-3-yl)furan-2-carboxamide |
| 5-141 | methyl 4-(5-((4-aminobutyl)carbamoyl)furan-2-yl)-2-(3-aminoprop-1-yn-1-yl)benzoate |
| 5-142 | N-(4-aminobutyl)-5-(4-(3-aminoprop-1-yn-1-yl)-3-(hydroxymethyl)phenyl)furan-2-carboxamide |
| 5-143 | N-(4-aminobutyl)-5-(3-(3-aminoprop-1-yn-1-yl)phenyl)furan-2-carboxamide |
| 5-144 | methyl 5-(5-((4-aminobutyl)carbamoyl)furan-2-yl)-2-(3-aminoprop-1-yn-1-yl)benzoate |
| 5-145 | methyl 5-(5-((7-aminoheptyl)carbamoyl)furan-2-yl)-2-(3-aminoprop-1-yn-1-yl)benzoate |
| 5-164 | methyl 2-(3-aminoprop-1-yn-1-yl)-4-(4-(3-aminopropyl)piperazin-1-yl)benzoate |
| 5-165 | methyl 2-(3-aminoprop-1-yn-1-yl)-5-(4-(3-aminopropyl)piperazin-1-yl)benzoate |

5. The compound of claim 1, wherein the compound is a ubiquitin-binding compound, or a pharmaceutically acceptable salt, isomer, solvate, hydrate, or prodrug thereof.

6. A compound having a chemical structure of TBM-UBM, wherein the TBM is a moiety bound to a target protein or polypeptide, and
the UBM is a chemical structure represented by Formula V to Formula VIII below, or a pharmaceutically acceptable salt, isomer, solvate, hydrate, or prodrug thereof: 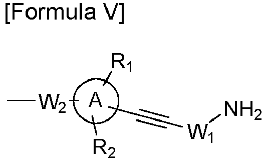 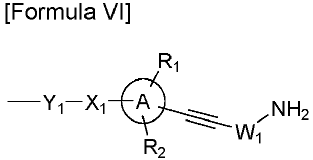 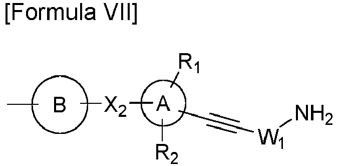 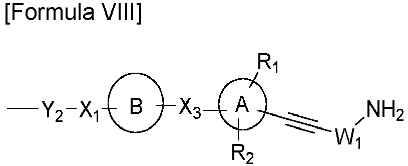
wherein, in Formula V to Formula VIII above, each independently,
W₁ is any one selected from the group consisting of -alkylene-, -alkylene-O-, - alkylene-OSO₂-, and -alkylene-N(R')SO₂- unsubstituted or substituted with at least one selected from the group consisting of deuterium, alkyl, C(O)OR', C(O)N(R')(R"), alkylene-C(O)OR', alkylene-C(O)N(R')(R"), alkylene-OR', alkylene-SR', alkylene-N(R')(R"), alkylene-OP(O)(OR')(OR"), alkylene-P(O)(OR')(OR"), and alkylene-OP(O)(R')(R");
A is any one selected from the group consisting of aryl, heteroaryl, cycloalkyl, heterocycloalkyl, and heterocycloalkenyl;
R₁ and R₂ are each independently any one selected from the group consisting of hydrogen, alkyl, alkenyl, alkynyl, halogen, oxo, CN, NO₂, OR', SR', N(R')(R"), O-(alkylene)-OR', -N(R')-(alkylene)-OR', C(O)R', C(NH)R', C(S)R', C(O)OR', C(O)N(R')(R"), P(O)(R')(R"), P(O)(OR')(OR"), C(NH)N(R')(R"), C(S)OR', C(S)N(R')(R"), C(O)O-(alkylene)-OC(O)R', C(O)O-(alkylene)-methacryloyloxy, (alkylene)-OR', (alkylene)-SR', (alkylene)-N(R')(R"), (alkynylene)-N(R')(R"), (alkylene)-P(O)(R')(R")-, (alkylene)-P(O)(OR')(OR"), (alkylene)-OP(O)(R')(R"), (alkylene)-OP(O)(OR')(OR"), (alkylene)-OP(O)(benzyloxy)₂, (alkylene)-OP(O)(phenoxy)₂, (alkylene)-OP(O)(phenoxy)(NH-CH(R')C(O)OR"), (alkylene)-C(O)OR', (alkenylene)-C(O)OR', (alkynylene)-C(O)OR', (alkylene)-C(O)N(R')(R"), (alkenylene)-C(NH)N(R')(R"), (alkynylene)-C(S)N(R')(R"), (alkylene)-C(O)R', (alkylene)-C(NH)R', (alkylene)-C(S)R', aryl, heteroaryl, heterocycloalkyl, and heterocycloalkenyl, optionally the R₁ and R₂ above are each independently substituted with at least one halogen, and when R₁ and R₂ above are each independently aryl, heteroaryl, heterocycloalkyl, or heterocycloalkenyl, optionally the R₁ and R₂ above are each independently substituted with alkyl or N(R')(R") unsubstituted or substituted with at least one halogen at one or more positions;
X₁ is a direct linkage, any one selected from among X1a group consisting of-N(R')-, -O-, -S-, -C(O)-, -SO-, SO₂-, =N-O-, and -O-N=, X1b group consisting of -OC(O)-, -C(O)O-, -N(R')C(O)-, -C(O)N(R')-, -N(R')SO-, -SON(R')-, -N(R')SO₂-, and -SO₂N(R')-, and X1c group consisting of -alkylene-, -alkenylene-, and -alkynylene-, or that two or more thereof are linked to each other;
X₂ is a direct linkage, any one selected from among X2a group consisting of -N(R')-, -O-, -S-, -C(O)-, -SO-, SO₂-, =N-O-, and -O-N=, X2b group consisting of -OC(O)-, - N(R')C(O)-, -N(R')C(O)O-, -N(R')SO₂-O-, and -N(R')C(S)O-, X2c group consisting of - C(O)O-, -C(O)N(R')-, -O-SO₂-N(R')-, -O-C(S)N(R')-, and -O-C(O)N(R')-, and X2d group consisting of -alkylene-, -alkenylene-, and -alkynylene-, or that two or more thereof are linked to each other;
X₃ is a direct linkage, or any one selected from the group consisting of -alkylene-, - O-, -S-, -C(O)-, -N(R')-, -SO-, -SO₂-, and a combination thereof;
B is any one selected from the group consisting of aryl, heteroaryl, cycloalkyl, heterocycloalkyl, and heterocycloalkenyl, which are unsubstituted or substituted with at least one selected from the group consisting of hydroxy, alkoxy, and oxo, or that two or more selected from the group consisting of aryl, heteroaryl, cycloalkyl, heterocycloalkyl, and heterocycloalkenyl, which are unsubstituted or substituted with at least one selected from the group consisting of hydroxy, alkoxy, and oxo, are directly linked, or linked via -alkylene-, -O-, -S-, -C(O)-, -N(R')-, -SO-, -SO₂-, or a combination thereof;
W₂ is a direct linkage, or any one selected from the group consisting of -alkylene-, -alkenylene-, and -alkynylene- unsubstituted or substituted with at least one selected from the group consisting of deuterium, alkyl, C(O)OR', C(O)N(R')(R"), alkylene-C(O)OR', alkylene-C(O)N(R')(R"), alkylene-OR', alkylene-SR', alkylene-N(R')(R"), alkylene-OP(O)(OR')(OR"), alkylene-P(O)(OR')(OR"), and alkylene-OP(O)(R')(R");
Y₁ is any one selected from the group consisting of a direct linkage, alkylene, alkenylene, alkynylene, -(CH₂CH₂O)ₙCH₂-, and -(CH₂CH₂O)ₙCH₂CH₂-, wherein n is 1-4;
Y₂ is any one selected from the group consisting of a direct linkage, alkylene, alkenylene, alkynylene, heterocycloalkylene, and heterocycloalkenylene;
the TBM and the UBM are covalently linked to each other, and the covalent bond site is a direct linkage, or the TBM and the UBM are each modified to form any one selected from the group consisting of -N(R')-, -O-, -C(R')(R")-O-, -S-, -C(O)-, -SO-, SO₂-, =N-O-, -ON=, -OC(O)-, -C(O)O-, -N(R')C(O)-, -C(O)N(R')-, -OC(O)O-, -OC(O)N(R')-, -N(R')C(O)O-, - N(R')SO-, -SON(R')-, -N(R')SO₂-, and -SO₂N(R')-; and
R' and R" in the functional groups are each independently hydrogen or alkyl.

7. The compound of claim 6, wherein, in Formula V to Formula VIII above, each independently,
the W₁ above is any one selected from the group consisting of -alkylene-, -alkylene-O-, -alkylene-OSO₂-, and -alkylene-N(R')SO₂- unsubstituted or substituted with at least one selected from the group consisting of C(O)OR', C(O)N(R')(R"), alkylene-OR', alkylene-SR', and alkylene-OP(O)(OR')(OR");
the A above is any one selected from the group consisting of aryl, 5- to 10-membered heteroaryl containing at least one selected from the group consisting of N, O, and S, 3- to 10-membered cycloalkyl, 3- to 10-membered heterocycloalkyl containing at least one selected from the group consisting of N, O, and S, and 3- to 10-membered heterocycloalkenyl containing at least one selected from the group consisting of N, O, and S;
the R₁ and R₂ above are each independently any one selected from the group consisting of hydrogen, alkyl, alkenyl, halogen, OR', N(R')(R"), O-(alkylene)-OR', C(O)R', C(O)OR', C(O)N(R')(R"), C(NH)N(R')(R"), C(O)O-(alkylene)-OC(O)R', C(O)O-(alkylene)-methacryloyloxy, (alkylene)-OR', (alkylene)-SR', (alkylene)-N(R')(R"), (alkynylene)-N(R')(R"), (alkylene)-OP(O)(OR')(OR"), (alkylene)-OP(O)(benzyloxy)₂, (alkylene)-OP(O)(phenoxy)₂, (alkylene)-OP(O)(phenoxy)(NH-CH(R')C(O)OR"), (alkylene)-C(O)OR', aryl, heteroaryl, heterocycloalkyl, and heterocycloalkenyl, optionally the R₁ and R₂ above are each independently substituted with at least one halogen, and when R₁ and R₂ above are each independently aryl, heteroaryl, heterocycloalkyl, or heterocycloalkenyl, optionally the R₁ and R₂ above are each independently substituted with alkyl or N(R')(R") unsubstituted or substituted with at least one halogen at one or more positions;
the X₁ above is a direct linkage, any one selected from the X1a group and the X1b group, or that any one selected from the X₁a group and the X₁b group and any one selected from the X1c group are linked in the form of X1a-X1c orX1b-X1c;
the X₂ above is a direct linkage, or any one selected from the X2a group, the X2b group, and the X2c group, or that two or more thereof are linked to each other, or that any one or two or more selected from the X2b group, the X2c group, and the X2d group are linked in the form of X2b-X2d-X2b, X2b-X2d-X2c, X2c-X2d-X2b, or X2c-X2d-X2c;
the X₃ above is a direct linkage, or any one selected from the group consisting of - C(O)-, -N(R')-, and a combination thereof;
the B above is any one selected from the group consisting of aryl, 5- to 10-membered heteroaryl, 3- to 10-membered heterocycloalkyl, and 3- to 10-membered heterocycloalkenyl, which are unsubstituted or substituted with at least one selected from the group consisting of hydroxy, alkoxy, and oxo, or that two or more selected from the group consisting of aryl, 5- to 10-membered heteroaryl, 3- to 10-membered heterocycloalkyl, and 3- to 10-membered heterocycloalkenyl, which are unsubstituted or substituted with at least one selected from the group consisting of hydroxy and oxo, are directly linked, or linked via -alkylene-, -C(O)-, -N(R')-, or a combination thereof;
the W₂ is a direct linkage, or any one selected from the group consisting of - alkenylene- and -alkynylene- unsubstituted or substituted with at least one selected from the group consisting of alkylene-OR', alkylene-SR', and alkylene-OP(O)(OR')(OR");
the Y₁ is any one selected from the group consisting of a direct linkage, alkylene, alkenylene, alkynylene, -(CH₂CH₂O)ₙCH₂-, and -(CH₂CH₂O)ₙCH₂CH₂-, wherein n is 1-2;
the Y₂ is any one selected from the group consisting of a direct linkage, alkylene, alkenylene, alkynylene, and heterocycloalkylene; and
the covalent bond site at which the TBM and the UBM are linked to each other is a direct linkage, or the TBM and the UBM are each modified to form any one selected from the group consisting of -N(R')-, -O-, -C(R')(R")-O-, -OC(O)-, -OC(O)N(R')-, and -C(O)N(R')-,
or a pharmaceutically acceptable salt, isomer, solvate, hydrate, or prodrug thereof.

8. The compound of claim 7, wherein, in Formula V to Formula VIII above, each independently,
the W₁ above is any one selected from the group consisting of -alkylene-, -alkylene-O-, -alkylene-OSO₂-, and -alkylene-N(R')SO₂- unsubstituted or substituted with at least one selected from the group consisting of alkylene-OR' and alkylene-OP(O)(OR')(OR");
the A above is any one selected from the group consisting of benzene, furan, benzofuran, pyridine, thiophene, imidazole, and oxazole;
the R₁ and R₂ above are each independently any one selected from the group consisting of hydrogen, alkyl, OR', C(O)R', C(O)OR', C(O)N(R')(R"), (alkylene)-OR', (alkylene)-SR', (alkylene)-OP(O)(OR')(OR"), heteroaryl, and heterocycloalkyl, optionally the R₁ and R₂ above are each independently substituted with at least one halogen, and when R₁ and R₂ above are each independently heteroaryl or heterocycloalkyl, optionally the R₁ and R₂ above are each independently substituted with alkyl or N(R')(R") unsubstituted or substituted with at least one halogen at one or more positions;
the X₁ above is a direct linkage, or the X1a group consists of -N(R')- and -O-, and the X1b group consists of -C(O)O-, -N(R')C(O)-, and -C(O)N(R')-,
the X₂ above is a direct linkage, or the X2a group consists of N(R')-, -O-, -C(O)-, and =N-O-, the X2b group consists of -N(R')C(O)-, and the X2c group consists of - C(O)N(R')-,
the B above is any one selected from the group consisting of 5- to 10-membered heteroaryl, 3- to 10-membered heterocycloalkyl, and 3- to 10-membered heterocycloalkenyl, which are unsubstituted or substituted with at least one selected from the group consisting of hydroxy and oxo, or two or more selected from the group consisting of 5- to 10-membered heteroaryl, 3- to 10-membered heterocycloalkyl, and 3- to 10-membered heterocycloalkenyl, which are unsubstituted, are directly linked, or linked via -alkylene-, -C(O)-, -N(R')-, or a combination thereof;
the W₂ is a direct linkage, or -alkynylene- unsubstituted or substituted with at least one selected from the group consisting of alkylene-OR' and alkylene-OP(O)(OR')(OR");
the Y₁ is any one selected from the group consisting of a direct linkage, alkylene, alkenylene, and alkynylene;
the Y₂ is any one selected from the group consisting of a direct linkage, alkylene and heterocycloalkylene; and
the covalent bond site at which the TBM and the UBM are linked to each other may be a direct linkage, or the TBM and the UBM are each modified to form any one selected from the group consisting of -O-, -C(R')(R")-O-, -OC(O)- -OC(O)N(R')-, and -C(O)N(R')-,
or a pharmaceutically acceptable salt, isomer, solvate, hydrate, or prodrug thereof.

9. The compound of any one of claims 6 to 8, wherein the compound degrades the target protein or polypeptide to which the TBM binds, or a pharmaceutically acceptable salt, isomer, solvate, hydrate, or prodrug thereof.

10. The compound of any one of claims 6 to 8, wherein the target protein or polypeptide to which the TBM binds is at least one selected from the group consisting of proteins associated with an apoptosis mechanism including B7.1, B7, TINFRIm, TNFR2, NADPH oxidase and Bcl2Bax, a MAP/ERK signal pathway-related protein including Ras and Raf, proteins associated with the NF-κB signal pathway, proteins associated with the mTOR signal pathway, proteins associated with the wnt signal pathway, proteins associated with the AKT signal pathway, proteins associated with the JAK-STAT signal pathway, NOX complexes and proteins associated with the signal pathway thereof, vascular endothelial growth factor and receptors and proteins associated with the signal pathway thereof, TLR receptors and proteins associated with the signal pathway thereof, G protein-coupled receptors and proteins associated with the signal pathway thereof, TCR-MHC complexrelated receptors and proteins associated with the signal pathway thereof, cell cycle checkpoint proteins and proteins associated with the signal pathway thereof, proteins including a factor that modulates gene transcription and translation, proteins associated with gene replication and regulatory factors thereof, proteins including histone protein regulatory factors for gene expression, factors that regulate epigenetics, and the related proteins, proteins that constitute and modulate cytoskeleton, a protein that modulates intracellular transport and proteins that modulate the protein, a C5a receptor, dopamine receptors, 5HT receptors, chemokine receptors, histamine receptors, CD40/CD40L, CD23, CD124, CD4, CD5, VCAM, VLA-4 integrin, selectin, ICOS, ICAM1, IL-2 receptors, IL-1 receptors, TNF-alpha receptors, and other related cytokine receptors and chemokine receptors, oxytocin receptors, glycine receptors, noradrenaline reuptake receptors, endothelin receptors, estrogen receptors, androgen receptors, adenosine receptors, a TrkA receptor for NGF, a vitronectin receptor, an integrin receptor, neurokinins and receptors, epithelial growth factor receptors, purinergic receptors (P2Y1, P2Y2, P2Y4, P2Y6, and P2X1-7), EGF receptors, HMG-CoA reductase, PDE V phosphodiesterase type, PDE IV phosphodiesterase type 4, PDE I, PDEII, PDEIII, nitric oxide (NO) synthase, cyclo-oxygenase 1, cyclo-oxygenase 2, 5-lipoxygenase, tryptase, serine protease, thymidylate synthase, purine nucleoside phosphorylase, GAPDH trypanosomal, glycogen phosphorylase, carbonic anhydrase, analogs thereof, P-glycoproteins and MRP, tyrosine kinase, p56, 1ck, Cat+ channels, inosine monophosphate dehydrogenase, interleukin-1 converting enzyme, caspases-1, glycinamide ribonucleotide formyl transferase, poly(ADP-ribose) polymerase, acetylcholinesterase, cyclin dependent kinases, microsomal transfer protein inhibitors, bile acid transport inhibitors, 5 alpha reductase inhibitors, angiotensin 11, adenosine receptors, adenosine kinase and AMP deaminase, farnesyltransferases, geranylgeranyl transferase, beta-amyloid, tyrosine kinase Her-21/neu, telomerase inhibition, cytosolic phospholipase A2 and tyrosine kinase, acetyl-CoA carboxylase, adenylosuccinate synthetase, protoporphyrinogen oxidase, and enolpyruvylshikimate-phosphate synthase, ecdysone 20-monooxygenase, ion channel of the GABA gated chloride channel, voltagesensitive sodium channel protein, sodium channel, calcium release channel proteins, chloride channel proteins and polypeptides derived from these proteins, bacterial or viral proteins and polypeptides derived therefrom, such as HCV NS3 protease, HCV NS3 RNA helicase, rhinovirus 3C protease, herpes simplex virus-1 (HSV-I) protease, cytomegalovirus (CMV) protease, multidrug resistance (MDR) proteins, HIV 1 protease, HIV 1 integrase, influenza, neuraminidase, and hepatitis B reverse transcriptase, and proteins and polypeptides such as vasoactive intestinal peptides, prions, neuropeptide Y, and receptors, or a pharmaceutically acceptable salt, isomer, solvate, hydrate, or prodrug thereof.

11. The compound of any one of claims 6 to 8, wherein the target protein or polypeptide to which the TBM binds is at least one selected from the group consisting of a bromodomain-containing protein, an Aurora kinase A, and a p38 MAP kinase, or a pharmaceutically acceptable salt, isomer, solvate, hydrate, or prodrug thereof.

12. The compound of any one of claims 6 to 8, wherein the TBM is any one selected from the group consisting of a BRD4 inhibitor, an Aurora A inhibitor, and a p38 inhibitor, or a pharmaceutically acceptable salt, isomer, solvate, hydrate, or prodrug thereof.

13. The compound of any one of claims 6 to 8, wherein the TBM has any one chemical structure selected from the group consisting of Formulae 1 to 3 below, or a pharmaceutically acceptable salt, isomer, solvate, hydrate, or prodrug thereof:

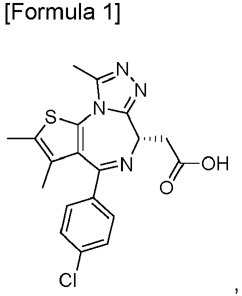

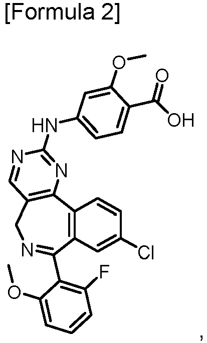

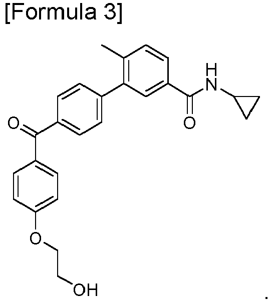

14. The compound of any one of claims 6 to 8, wherein the compound has any one chemical structure selected from the group consisting of Formulae 4 to 6 below, or a pharmaceutically acceptable salt, isomer, solvate, hydrate, or prodrug thereof: 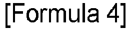 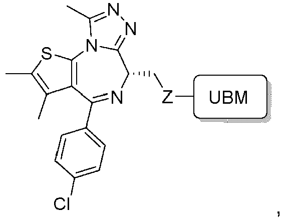 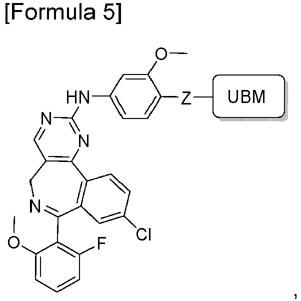 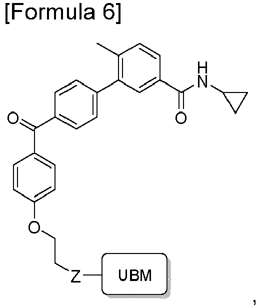
wherein, in the Formula 4 to Formula 6 above, Z is each independently a direct linkage, or any one selected from the group consisting of -N(R')-, -O-, -C(R')(R")-O-, -S-, - C(O)-, -SO-, SO₂-, =N-O-, -O-N=, -OC(O)-, -C(O)O-, -N(R')C(O)-, -C(O)N(R')-, -OC(O)O-, - OC(O)N(R')-, -N(R')C(O)O-, -N(R')SO-, -SON(R')-, -N(R')SO₂-, and -SO₂N(R')-; and
R' and R" in the functional groups are each independently hydrogen or alkyl.

15. The compound of any one of claims 6 to 8, wherein the compound is at least one selected from the group consisting of compounds listed in Tables 5 to 15 below, or a pharmaceutically acceptable salt, isomer, solvate, hydrate, or prodrug thereof:
**[Table 5]**
| No. | Compound |
|---|---|
| 1-1' | (S)-N-(4-(3-aminoprop-1-yn-1-yl)phenyl)-2-(4-(4-chlorophenyl)-2,3,9-trimethyl-6H-thieno[3,2-f][1,2,4]triazolo[4,3-a][1,4]diazepin-6-yl)acetamide hydrochloride |
| 1-3' | (S)-N-(3-(3-aminoprop-1-yn-1-yl)phenyl)-2-(4-(4-chlorophenyl)-2,3,9-trimethyl-6H-thieno[3,2-f][1,2,4]triazolo[4,3-a][1,4]diazepin-6-yl)acetamide hydrochloride |
| 3-1' | methyl (S)-2-(3-aminoprop-1-yn-1-yl)-5-(2-(4-(4-chlorophenyl)-2,3,9-trimethyl-6H-thieno[3,2-f][1,2,4]triazolo[4,3-a][1,4]diazepin-6-yl)acetamido)benzoate hydrochloride |
| 3-2' | (S)-2-(3-aminoprop-1-yn-1-yl)-5-(2-(4-(4-chlorophenyl)-2,3,9-trimethyl-6H-thieno[3,2-f][1,2,4]triazolo[4,3-a][1,4]diazepin-6-yl)acetamido)benzoic acid hydrochloride |
| 3-3' | (S)-N-(4-(3-aminoprop-1-yn-1-yl)-3-(hydroxymethyl)phenyl)-2-(4-(4-chlorophenyl)-2,3,9-trimethyl-6H-thieno[3,2-f][1,2,4]triazolo[4,3-a][1,4]diazepin-6-yl)acetamide hydrochloride |
| 3-6' | (S)-N-(4-(3-aminoprop-1-yn-1-yl)-3-(mercaptomethyl)phenyl)-2-(4-(4-chlorophenyl)-2,3,9-trimethyl-6H-thieno[3,2-f][1,2,4]triazolo[4,3-a][1,4]diazepin-6-yl)acetamide |
| 3-7' | (S)-2-(3-aminoprop-1-yn-1-yl)-5-(2-(4-(4-chlorophenyl)-2,3,9-trimethyl-6H-thieno[3,2-f][1,2,4]triazolo[4,3-a][1,4]diazepin-6-yl)acetamido)benzyl dimethyl phosphate |
| 3-8' | (S)-2-(3-aminoprop-1-yn-1-yl)-5-(2-(4-(4-chlorophenyl)-2,3,9-trimethyl-6H-thieno[3,2-f][1,2,4]triazolo[4,3-a][1,4]diazepin-6-yl)acetamido)benzyl dihydrogen phosphate trifluoroacetic acid |
| 3-9' | (S)-2-(3-aminoprop-1-yn-1-yl)-5-(2-(4-(4-chlorophenyl)-2,3,9-trimethyl-6H-thieno[3,2-f][1,2,4]triazolo[4,3-a][1,4]diazepin-6-yl)acetamido)benzyl di-tert-butyl phosphate |
| 3-10' | (S)-2-(3-aminoprop-1-yn-1-yl)-5-(2-(4-(4-chlorophenyl)-2,3,9-trimethyl-6H-thieno[3,2-f][1,2,4]triazolo[4,3-a][1,4]diazepin-6-yl)acetamido)benzyl dibenzyl phosphate |
| 3-11' | (S)-2-(3-aminoprop-1-yn-1-yl)-5-(2-(4-(4-chlorophenyl)-2,3,9-trimethyl-6H-thieno[3,2-f][1,2,4]triazolo[4,3-a][1,4]diazepin-6-yl)acetamido)benzyl diisopropyl phosphate |
| 3-12' | isopropyl (((2-(3-aminoprop-1-yn-1-yl)-5-(2-((S)-4-(4-chlorophenyl)-2,3,9-trimethyl-6H-thieno[3,2-f][1,2,4]triazolo[4,3-a][1,4]diazepin-6-yl)acetamido)benzyl)oxy)(phenoxy)phosphoryl)-L-alaninate |
| 3-25' | methyl (S)-2-(3-aminoprop-1-yn-1-yl)-4-(2-(4-(4-chlorophenyl)-2,3,9-trimethyl-6H-thieno[3,2-f][1,2,4]triazolo[4,3-a][1,4]diazepin-6-yl)acetamido)benzoate hydrochloride |
| 3-26' | (S)-2-(3-aminoprop-1-yn-1-yl)-4-(2-(4-(4-chlorophenyl)-2,3,9-trimethyl-6H-thieno[3,2-f][1,2,4]triazolo[4,3-a][1,4]diazepin-6-yl)acetamido)benzoic acid hydrochloride |
| 3-27' | (S)-N-(3-(3-aminoprop-1-yn-1-yl)-4-(hydroxymethyl)phenyl)-2-(4-(4-chlorophenyl)-2,3,9-trimethyl-6H-thieno[3,2-f][1,2,4]triazolo[4,3-a][1,4]diazepin-6-yl)acetamide hydrochloride |
| 3-28' | (S)-N-(3-(3-aminoprop-1-yn-1-yl)-4-(mercaptomethyl)phenyl)-2-(4-(4-chlorophenyl)-2,3,9-trimethyl-6H-thieno[3,2-f][1,2,4]triazolo[4,3-a][1,4]diazepin-6-yl)acetamide |
| 3-30' | (S)-2-(3-aminoprop-1-yn-1-yl)-4-(2-(4-(4-chlorophenyl)-2,3,9-trimethyl-6H-thieno[3,2-f][1,2,4]triazolo[4,3-a][1,4]diazepin-6-yl)acetamido)benzyl dimethyl phosphate |
| 3-31' | (S)-2-(3-aminoprop-1-yn-1-yl)-4-(2-(4-(4-chlorophenyl)-2,3,9-trimethyl-6H-thieno[3,2-f][1,2,4]triazolo[4,3-a][1,4]diazepin-6-yl)acetamido)benzyl di-tert-butyl phosphate |
| 3-32' | (S)-2-(3-aminoprop-1-yn-1-yl)-4-(2-(4-(4-chlorophenyl)-2,3,9-trimethyl-6H-thieno[3,2-f][1,2,4]triazolo[4,3-a][1,4]diazepin-6-yl)acetamido)benzyl dihydrogen phosphate |
| 3-33' | (S)-2-(3-aminoprop-1-yn-1-yl)-4-(2-(4-(4-chlorophenyl)-2,3,9-trimethyl-6H-thieno[3,2-f][1,2,4]triazolo[4,3-a][1,4]diazepin-6-yl)acetamido)benzyl diphenyl phosphate |
| 3-34' | (S)-2-(3-aminoprop-1-yn-1-yl)-4-(2-(4-(4-chlorophenyl)-2,3,9-trimethyl-6H-thieno[3,2-f][1,2,4]triazolo[4,3-a][1,4]diazepin-6-yl)acetamido)benzyl diisopropyl phosphate |
| 3-35' | isopropyl (((2-(3-aminoprop-1-yn-1-yl)-4-(2-((S)-4-(4-chlorophenyl)-2,3,9-trimethyl-6H-thieno[3,2-f][1,2,4]triazolo[4,3-a][1,4]diazepin-6-yl)acetamido)benzyl)oxy)(phenoxy)phosphoryl)-L-alaninate |
| 3-49' | N-(4-(3-amino-4-hydroxybut-1-yn-1-yl)phenyl)-2-((S)-4-(4-chlorophenyl)-2,3,9-trimethyl-6H-thieno[3,2-f][1,2,4]triazolo[4,3-a][1,4]diazepin-6-yl)acetamide hydrochloride |
| 3-51' | N-(3-(3-amino-4-hydroxybut-1-yn-1-yl)phenyl)-2-((S)-4-(4-chlorophenyl)-2,3,9-trimethyl-6H-thieno[3,2-f][1,2,4]triazolo[4,3-a][1,4]diazepin-6-yl)acetamide hydrochloride |
| 3-53' | 2-amino-4-(4-(2-((S)-4-(4-chlorophenyl)-2,3,9-trimethyl-6H-thieno[3,2-f][1,2,4]triazolo[4,3-a][1,4]diazepin-6-yl)acetamido)phenyl)but-3-yn-1-yl di-tert-butyl phosphate |
| 3-55' | 2-amino-4-(3-(2-((S)-4-(4-chlorophenyl)-2,3,9-trimethyl-6H-thieno[3,2-f][1,2,4]triazolo[4,3-a][1,4]diazepin-6-yl)acetamido)phenyl)but-3-yn-1-yl di-tert-butyl phosphate |
| 3-57' | 2-amino-4-(4-(2-((S)-4-(4-chlorophenyl)-2,3,9-trimethyl-6H-thieno[3,2-f][1,2,4]triazolo[4,3-a][1,4]diazepin-6-yl)acetamido)phenyl)but-3-yn-1-yl dihydrogen phosphate trifluoroacetic acid |
| 3-60' | 2-amino-4-(3-(2-((S)-4-(4-chlorophenyl)-2,3,9-trimethyl-6H-thieno[3,2-f][1,2,4]triazolo[4,3-a][1,4]diazepin-6-yl)acetamido)phenyl)butyl dihydrogen phosphate |
| 4-1' | (S)-N-(3-(5-(3-aminoprop-1-yn-1-yl)furan-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide hydrochloride |
| 4-10' | (S)-N-(3-(5-(3-aminoprop-1-yn-1-yl)thiophen-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide hydrochloride |
| 4-13' | (S)-N-(3-(5-(3-aminoprop-1-yn-1-yl)-1H-pyrrol-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-16' | (S)-N-(3-(4-(3-aminoprop-1-yn-1-yl)furan-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-19' | (S)-N-(3-(4-(3-aminoprop-1-yn-1-yl)thiophen-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-25' | (S)-N-(3-(5-(3-aminoprop-1-yn-1-yl)furan-3-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-28' | (S)-N-(3-(5-(3-aminoprop-1-yn-1-yl)thiophen-3-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-31' | (S)-N-(3-(5-(3-aminoprop-1-yn-1-yl)-1H-pyrrol-3-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-34' | (S)-N-(3-(4-(3-aminoprop-1-yn-1-yl)furan-3-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-40' | (S)-N-(3-(4-(3-aminoprop-1-yn-1-yl)-1H-pyrrol-3-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-43' | (S)-N-(3-(3-(3-aminoprop-1-yn-1-yl)furan-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-52' | (S)-N-(3-(2-(3-aminoprop-1-yn-1-yl)furan-3-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-61' | (S)-N-(3-(5-(4-aminobut-1-yn-1-yl)furan-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-62' | (S)-N-(3-(5-(4-aminobut-1-yn-1-yl)thiophen-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-63' | (S)-N-(3-(5-(4-aminobut-1-yn-1-yl)-1H-pyrrol-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-64' | (S)-N-(3-(5-(5-aminopent-1-yn-1-yl)furan-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-65' | (S)-N-(3-(5-(5-aminopent-1-yn-1-yl)thiophen-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-66' | (S)-N-(3-(5-(5-aminopent-1-yn-1-yl)-1H-pyrrol-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-67' | (S)-N-(3-(5-(6-aminohex-1-yn-1-yl)furan-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-68' | (S)-N-(3-(5-(6-aminohex-1-yn-1-yl)thiophen-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-69' | (S)-N-(3-(5-(6-aminohex-1-yn-1-yl)-1H-pyrrol-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-70' | (S)-N-(3-(5-(7-aminohept-1-yn-1-yl)furan-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-71' | (S)-N-(3-(5-(7-aminohept-1-yn-1-yl)thiophen-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-72' | (S)-N-(3-(5-(7-aminohept-1-yn-1-yl)-1H-pyrrol-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-73' | (S)-N-(3-(5-(8-aminooct-1-yn-1-yl)furan-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-74' | (S)-N-(3-(5-(8-aminooct-1-yn-1-yl)thiophen-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-75' | (S)-N-(3-(5-(8-aminooct-1-yn-1-yl)-1H-pyrrol-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-128' | ((S)-N-(3-(4-(3-aminoprop-1-yn-1-yl)phenyl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide hydrochloride |
| 4-129' | (S)-N-(3-(3-(3-aminoprop-1-yn-1-yl)phenyl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide hydrochloride |
| 4-130' | (S)-N-(3-(2-(3-aminoprop-1-yn-1-yl)-1H-imidazol-5-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-131' | (S)-N-(3-(2-(3-aminoprop-1-yn-1-yl)oxazol-5-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-147' | (S)-N-(4-(5-(4-aminobut-1-yn-1-yl)furan-2-yl)but-3-yn-1-yl)-2-(4-(4-chlorophenyl)-2,3,9-trimethyl-6H-thieno[3,2-f][1,2,4]triazolo[4,3-a][1,4]diazepin-6-yl)acetamide hydrochloride |
| 4-177' | (S)-N-(3-(5-(3-aminoprop-1-yn-1-yl)pyridin-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide hydrochloride |
| 4-178' | (S)-N-(3-(6-(3-aminoprop-1-yn-1-yl)pyridin-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-179' | (S)-N-(3-(6-(3-aminoprop-1-yn-1-yl)pyridin-3-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide hydrochloride |
| 4-180' | (S)-N-(3-(5-(3-aminoprop-1-yn-1-yl-3,3-d2)furan-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-181' | 2-amino-4-(5-(3-(2-((S)-4-(4-chlorophenyl)-2,3,9-trimethyl-6H-thieno[3,2-f][1,2,4]triazolo[4,3-a][1,4]diazepin-6-yl)acetamido)prop-1-yn-1-yl)furan-2-yl)but-3-ynoic acid |
| 4-182' | methyl (S)-2-(3-aminoprop-1-yn-1-yl)-5-(3-(2-(4-(4-chlorophenyl)-2,3,9-trimethyl-6H-thieno[3,2-f][1,2,4]triazolo[4,3-a][1,4]diazepin-6-yl)acetamido)prop-1-yn-1-yl)furan-3-carboxylate |
| 4-183' | dimethyl (S)-2-(3-aminoprop-1-yn-1-yl)-5-(3-(2-(4-(4-chlorophenyl)-2,3,9-trimethyl-6H-thieno[3,2-f][1,2,4]triazolo[4,3-a][1,4]diazepin-6-yl)acetamido)prop-1-yn-1-yl)furan-3,4-dicarboxylate |
| 4-184' | methyl (S)-5-(3-aminoprop-1-yn-1-yl)-2-(3-(2-(4-(4-chlorophenyl)-2,3,9-trimethyl-6H-thieno[3,2-f][1,2,4]triazolo[4,3-a][1,4]diazepin-6-yl)acetamido)prop-1-yn-1-yl)furan-3-carboxylate |
| 4-185' | (S)-N-(3-(5-(3-aminoprop-1-yn-1-yl)-4-(hydroxymethyl)furan-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-186' | (S)-N-(3-(5-(3-aminoprop-1-yn-1-yl)-3-(hydroxymethyl)furan-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-187' | (S)-N-(3-(5-(3-aminoprop-1-yn-1-yl)-3,4-bis(hydroxymethyl)furan-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-188' | (S)-(5-(3-aminoprop-1-yn-1-yl)-2-(3-(2-(4-(4-chlorophenyl)-2,3,9-trimethyl-6H-thieno[3,2-f][1,2,4]triazolo[4,3-a][1,4]diazepin-6-yl)acetamido)prop-1-yn-1-yl)furan-3-yl)methyl dihydrogen phosphate |
| 4-189' | (S)-(2-(3-aminoprop-1-yn-1-yl)-5-(3-(2-(4-(4-chlorophenyl)-2,3,9-trimethyl-6H-thieno[3,2-f][1,2,4]triazolo[4,3-a][1,4]diazepin-6-yl)acetamido)prop-1-yn-1-yl)furan-3-yl)methyl dihydrogen phosphate |
| 4-200' | (S)-N-(4-(5-(3-aminoprop-1-yn-1-yl)furan-2-yl)but-3-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-201' | (S)-N-(3-(4-(4-aminobut-1-yn-1-yl)furan-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-202' | (S)-N-(4-(4-(4-aminobut-1-yn-1-yl)furan-2-yl)but-3-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-218' | (S)-N-(3-(4-(3-aminoprop-1-yn-1-yl)-5-methylthiophen-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-219' | (S)-N-(3-(4-(4-aminobut-1-yn-1-yl)-5-methylthiophen-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide |
| 4-223' | (S)-N-(3-(5-(3-(aminooxy)prop-1-yn-1-yl)thiophen-2-yl)prop-2-yn-1-yl)-2-(4-(4-chlorophenyl)-2,3,9-trimethyl-6H-thieno[3,2-f][1,2,4]triazolo[4,3-a][1,4]diazepin-6-yl)acetamide hydrochloride |
| 5-52' | ethyl (S)-2-(3-aminoprop-1-yn-1-yl)-5-(3-(2-(4-(4-chlorophenyl)-2,3,9-trimethyl-6H-thieno[3,2-f][1,2,4]triazolo[4,3-a][1,4]diazepin-6-yl)acetamido)prop-1-yn-1-yl)oxazole-4-carboxylate |
| 5-116' | methyl (S)-2-(3-aminoprop-1-yn-1-yl)-4-(3-(2-(4-(4-chlorophenyl)-2,3,9-trimethyl-6H-thieno[3,2-f][1,2,4]triazolo[4,3-a][1,4]diazepin-6-yl)acetamido)prop-1-yn-1-yl)benzoate hydrochloride |
**[Table 6]**
| No. | Compound |
|---|---|
| 1-1" | N-(4-(3-aminoprop-1-yn-1-yl)phenyl)-4-((9-chloro-7-(2-fluoro-6-methoxyphenyl)-5H-benzo[c]pyrimido[4,5-e]azepin-2-yl)amino)-2-methoxybenzamide trifluoroacetic acid |
| 1-3" | N-(3-(3-aminoprop-1-yn-1-yl)phenyl)-4-((9-chloro-7-(2-fluoro-6-methoxyphenyl)-5H-benzo[c]pyrimido[4,5-e]azepin-2-yl)amino)-2-methoxybenzamide trifluoroacetic acid |
| 3-25" | methyl 2-(3-aminoprop-1-yn-1-yl)-4-(4-((9-chloro-7-(2-fluoro-6-methoxyphenyl)-5H-benzo[c]pyrimido[4,5-e]azepin-2-yl)amino)-2-methoxybenzamido)benzoate trifluoroacetic acid |
| 3-49" | N-(4-(3-amino-4-hydroxybut-1-yn-1-yl)phenyl)-4-((9-chloro-7-(2-fluoro-6-methoxyphenyl)-5H-benzo[c]pyrimido[4,5-e]azepin-2-yl)amino)-2-methoxybenzamide trifluoroacetic acid |
| 4-1" | N-(3-(5-(3-aminoprop-1-yn-1-yl)furan-2-yl)prop-2-yn-1-yl)-4-((9-chloro-7-(2-fluoro-6-methoxyphenyl)-5H-benzo[c]pyrimido[4,5-e]azepin-2-yl)amino)-2-methoxybenzamide trifluoroacetic acid |
| 5-116" | methyl 2-(3-aminoprop-1-yn-1-yl)-4-(3-(4-((9-chloro-7-(2-fluoro-6-methoxyphenyl)-5H-benzo[c]pyrimido[4,5-e]azepin-2-yl)amino)-2-methoxybenzamido)prop-1-yn-1-yl)benzoate trifluoroacetic acid |
**[Table 7]**
| No. | Compound |
|---|---|
| 1-1‴ | 2-(4-(5'-(cyclopropylcarbamoyl)-2'-methyl-[1,1'-biphenyl]-4-carbonyl)phenoxy)ethyl (4-(3-aminoprop-1-yn-1-yl)phenyl)carbamate hydrochloride |
| 3-49‴ | 2-(4-(5'-(cyclopropylcarbamoyl)-2'-methyl-[1,1'-biphenyl]-4-carbonyl)phenoxy)ethyl (4-(3-amino-4-hydroxybut-1-yn-1-yl)phenyl)carbamate hydrochloride |
| 5-116‴ | methyl 2-(3-aminoprop-1-yn-1-yl)-4-(3-(((2-(4-(5'-(cyclopropylcarbamoyl)-2'-methyl-[1,1'-biphenyl]-4-carbonyl)phenoxy)ethoxy)carbonyl)amino)prop-1-yn-1-yl)benzoate hydrochloride |
**[Table 8]**
| No. | Compound |
|---|---|
| 4-6' | (S)-2-(3-aminoprop-1-yn-1-yl)-5-(3-(2-(4-(4-chlorophenyl)-2,3,9-trimethyl-6H-thieno[3,2-f][1,2,4]triazolo[4,3-a][1,4]diazepin-6-yl)acetamido)propanamido)benzoic acid |
| 4-109' | methyl (S)-2-(4-aminobut-1-yn-1-yl)-5-(3-(2-(4-(4-chlorophenyl)-2,3,9-trimethyl-6H-thieno[3,2-f][1,2,4]triazolo[4,3-a][1,4]diazepin-6-yl)acetamido)propanamido)benzoate |
| 4-110' | (S)-2-(4-aminobut-1-yn-1-yl)-5-(3-(2-(4-(4-chlorophenyl)-2,3,9-trimethyl-6H-thieno[3,2-f][1,2,4]triazolo[4,3-a][1,4]diazepin-6-yl)acetamido)propanamido)benzoic acid |
| 4-111' | (S)-N-(4-(4-aminobut-1-yn-1-yl)-3-(hydroxymethyl)phenyl)-3-(2-(4-(4-chlorophenyl)-2,3,9-trimethyl-6H-thieno[3,2-f][1,2,4]triazolo[4,3-a][1,4]diazepin-6-yl)acetamido)propanamide |
| 4-112' | methyl (S)-2-(5-aminopent-1-yn-1-yl)-5-(3-(2-(4-(4-chlorophenyl)-2,3,9-trimethyl-6H-thieno[3,2-f][1,2,4]triazolo[4,3-a][1,4]diazepin-6-yl)acetamido)propanamido)benzoate |
| 4-113' | (S)-2-(5-aminopent-1-yn-1-yl)-5-(3-(2-(4-(4-chlorophenyl)-2,3,9-trimethyl-6H-thieno[3,2-f][1,2,4]triazolo[4,3-a][1,4]diazepin-6-yl)acetamido)propanamido)benzoic acid |
| 4-114' | (S)-N-(4-(5-aminopent-1-yn-1-yl)-3-(hydroxymethyl)phenyl)-3-(2-(4-(4-chlorophenyl)-2,3,9-trimethyl-6H-thieno[3,2-f][1,2,4]triazolo[4,3-a][1,4]diazepin-6-yl)acetamido)propanamide |
| 4-115' | methyl (S)-2-(6-aminohex-1-yn-1-yl)-5-(3-(2-(4-(4-chlorophenyl)-2,3,9-trimethyl-6H-thieno[3,2-f][1,2,4]triazolo[4,3-a][1,4]diazepin-6-yl)acetamido)propanamido)benzoate |
| 4-116' | (S)-2-(6-aminohex-1-yn-1-yl)-5-(3-(2-(4-(4-chlorophenyl)-2,3,9-trimethyl-6H-thieno[3,2-f][1,2,4]triazolo[4,3-a][1,4]diazepin-6-yl)acetamido)propanamido)benzoic acid |
| 4-117' | (S)-N-(4-(6-aminohex-1-yn-1-yl)-3-(hydroxymethyl)phenyl)-3-(2-(4-(4-chlorophenyl)-2,3,9-trimethyl-6H-thieno[3,2-f][1,2,4]triazolo[4,3-a][1,4]diazepin-6-yl)acetamido)propanamide |
| 4-118' | methyl (S)-2-(7-aminohept-1-yn-1-yl)-5-(3-(2-(4-(4-chlorophenyl)-2,3,9-trimethyl-6H-thieno[3,2-f][1,2,4]triazolo[4,3-a][1,4]diazepin-6-yl)acetamido)propanamido)benzoate |
| 4-119' | (S)-2-(7-aminohept-1-yn-1-yl)-5-(3-(2-(4-(4-chlorophenyl)-2,3,9-trimethyl-6H-thieno[3,2-f][1,2,4]triazolo[4,3-a][1,4]diazepin-6-yl)acetamido)propanamido)benzoic acid |
| 4-120' | (S)-N-(4-(7-aminohept-1-yn-1-yl)-3-(hydroxymethyl)phenyl)-3-(2-(4-(4-chlorophenyl)-2,3,9-trimethyl-6H-thieno[3,2-f][1,2,4]triazolo[4,3-a][1,4]diazepin-6-yl)acetamido)propanamide |
| 4-121' | methyl (S)-2-(8-aminooct-1-yn-1-yl)-5-(3-(2-(4-(4-chlorophenyl)-2,3,9-trimethyl-6H-thieno[3,2-f][1,2,4]triazolo[4,3-a][1,4]diazepin-6-yl)acetamido)propanamido)benzoate |
| 4-122' | (S)-2-(8-aminooct-1-yn-1-yl)-5-(3-(2-(4-(4-chlorophenyl)-2,3,9-trimethyl-6H-thieno[3,2-f][1,2,4]triazolo[4,3-a][1,4]diazepin-6-yl)acetamido)propanamido)benzoic acid |
| 4-123' | (S)-N-(4-(8-aminooct-1-yn-1-yl)-3-(hydroxymethyl)phenyl)-3-(2-(4-(4-chlorophenyl)-2,3,9-trimethyl-6H-thieno[3,2-f][1,2,4]triazolo[4,3-a][1,4]diazepin-6-yl)acetamido)propanamide |
| 4-124' | (S)-N-(4-(3-aminoprop-1-yn-1-yl)phenyl)-3-(2-(4-(4-chlorophenyl)-2,3,9-trimethyl-6H-thieno[3,2-f][1,2,4]triazolo[4,3-a][1,4]diazepin-6-yl)acetamido)propanamide hydrochloride |
| 4-125' | (S)-N-(4-(3-aminoprop-1-yn-1-yl)-3-(hydroxymethyl)phenyl)-3-(2-(4-(4-chlorophenyl)-2,3,9-trimethyl-6H-thieno[3,2-f][1,2,4]triazolo[4,3-a][1,4]diazepin-6-yl)acetamido)propanamide hydrochloride |
| 4-126' | methyl (S)-2-(3-aminoprop-1-yn-1-yl)-5-(3-(2-(4-(4-chlorophenyl)-2,3,9-trimethyl-6H-thieno[3,2-f][1,2,4]triazolo[4,3-a][1,4]diazepin-6-yl)acetamido)propanamido)benzoate hydrochloride |
| 4-127' | (S)-N-(4-(3-aminoprop-1-yn-1-yl)-3,5-bis(hydroxymethyl)phenyl)-3-(2-(4-(4-chlorophenyl)-2,3,9-trimethyl-6H-thieno[3,2-f][1,2,4]triazolo[4,3-a][1,4]diazepin-6-yl)acetamido)propanamide |
| 4-148' | (S)-5-(3-aminoprop-1-yn-1-yl)-N-(4-(2-(4-(4-chlorophenyl)-2,3,9-trimethyl-6H-thieno[3,2-f][1,2,4]triazolo[4,3-a][1,4]diazepin-6-yl)acetamido)but-2-yn-1-yl)furan-2-carboxamide hydrochloride |
| 4-149' | ((S)-5-(3-aminoprop-1-yn-1-yl)-N-(4-(2-(4-(4-chlorophenyl)-2,3,9-trimethyl-6H-thieno[3,2-f][1,2,4]triazolo[4,3-a][1,4]diazepin-6-yl)acetamido)butyl)furan-2-carboxamide hydrochloride |
| 4-150' | (S)-5-(4-aminobut-1-yn-1-yl)-N-(4-(2-(4-(4-chlorophenyl)-2,3,9-trimethyl-6H-thieno[3,2-f][1,2,4]triazolo[4,3-a][1,4]diazepin-6-yl)acetamido)but-2-yn-1-yl)furan-2-carboxamide |
| 4-161' | (S)-N-(3-(3-aminoprop-1-yn-1-yl)phenyl)-3-(2-(4-(4-chlorophenyl)-2,3,9-trimethyl-6H-thieno[3,2-f][1,2,4]triazolo[4,3-a][1,4]diazepin-6-yl)acetamido)propanamide hydrochloride |
| 4-162' | (S)-N-(4-(3-aminoprop-1-yn-1-yl)phenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide hydrochloride |
| 4-163' | (S)-N-(4-(3-aminoprop-1-yn-1-yl)phenyl)-5-(2-(4-(4-chlorophenyl)-2,3,9-trimethyl-6H-thieno[3,2-f][1,2,4]triazolo[4,3-a][1,4]diazepin-6-yl)acetamido)pentanamide hydrochloride |
| 4-164' | (S)-N-(3-(3-aminoprop-1-yn-1-yl)phenyl)-5-(2-(4-(4-chlorophenyl)-2,3,9-trimethyl-6H-thieno[3,2-f][1,2,4]triazolo[4,3-a][1,4]diazepin-6-yl)acetamido)pentanamide hydrochloride |
| 4-165' | (S)-N-(4-(3-aminoprop-1-yn-1-yl)phenyl)-6-(2-(4-(4-chlorophenyl)-2,3,9-trimethyl-6H-thieno[3,2-f][1,2,4]triazolo[4,3-a][1,4]diazepin-6-yl)acetamido)hexanamide hydrochloride |
| 4-166' | ((S)-5-(3-aminoprop-1-yn-1-yl)-N-(3-(2-(4-(4-chlorophenyl)-2,3,9-trimethyl-6H-thieno[3,2-f][1,2,4]triazolo[4,3-a][1,4]diazepin-6-yl)acetamido)propyl)furan-2-carboxamide hyd rochloride |
| 4-167' | (S)-5-(3-aminoprop-1-yn-1-yl)-N-(5-(2-(4-(4-chlorophenyl)-2,3,9-trimethyl-6H-thieno[3,2-f][1,2,4]triazolo[4,3-a][1,4]diazepin-6-yl)acetamido)pentyl)furan-2-carboxamide hydrochloride |
| 4-168' | (S)-N-(4-(3-aminoprop-1-yn-1-yl)-3-methoxyphenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide |
| 4-169' | (S)-N-(4-(3-aminoprop-1-yn-1-yl)-3-methoxyphenyl)-5-(2-(4-(4-chlorophenyl)-2,3,9-trimethyl-6H-thieno[3,2-f][1,2,4]triazolo[4,3-a][1,4]diazepin-6-yl)acetamido)pentanamide |
| 4-170' | (S)-N-(3-(3-aminoprop-1-yn-1-yl)-4-methoxyphenyl)-5-(2-(4-(4-chlorophenyl)-2,3,9-trimethyl-6H-thieno[3,2-f][1,2,4]triazolo[4,3-a][1,4]diazepin-6-yl)acetamido)pentanamide |
| 4-171' | (S)-N-(4-(3-aminoprop-1-yn-1-yl)-3-methoxyphenyl)-6-(2-(4-(4-chlorophenyl)-2,3,9-trimethyl-6H-thieno[3,2-f][1,2,4]triazolo[4,3-a][1,4]diazepin-6-yl)acetamido)hexanamide |
| 4-192' | (S)-5-(3-aminoprop-1-yn-1-yl)-N-(4-(2-(4-(4-chlorophenyl)-2,3,9-trimethyl-6H-thieno[3,2-f][1,2,4]triazolo[4,3-a][1,4]diazepin-6-yl)acetamido)butyl)-3-methylfuran-2-carboxamide |
| 4-199' | (S)-N-((5-(3-aminoprop-1-yn-1-yl)furan-2-yl)methyl)-4-(2-(4-(4-chlorophenyl)-2,3,9-trimethyl-6H-thieno[3,2-f][1,2,4]triazolo[4,3-a][1,4]diazepin-6-yl)acetamido)butanamide |
| 4-203' | (S)-4-(3-aminoprop-1-yn-1-yl)-N-(4-(2-(4-(4-chlorophenyl)-2,3,9-trimethyl-6H-thieno[3,2-f][1,2,4]triazolo[4,3-a][1,4]diazepin-6-yl)acetamido)butyl)furan-2-carboxamide |
| 4-204' | (S)-4-(4-aminobut-1-yn-1-yl)-N-(4-(2-(4-(4-chlorophenyl)-2,3,9-trimethyl-6H-thieno[3,2-f][1,2,4]triazolo[4,3-a][1,4]diazepin-6-yl)acetamido)butyl)furan-2-carboxamide |
| 4-205' | (S)-4-(3-aminoprop-1-yn-1-yl)-N-(3-(2-(4-(4-chlorophenyl)-2,3,9-trimethyl-6H-thieno[3,2-f][1,2,4]triazolo[4,3-a][1,4]diazepin-6-yl)acetamido)propyl)furan-2-carboxamide |
| 4-206' | (S)-4-(3-aminoprop-1-yn-1-yl)-N-(5-(2-(4-(4-chlorophenyl)-2,3,9-trimethyl-6H-thieno[3,2-f][1,2,4]triazolo[4,3-a][1,4]diazepin-6-yl)acetamido)pentyl)furan-2-carboxamide |
| 4-209' | (S)-5-(3-aminoprop-1-yn-1-yl)-N-(4-(2-(4-(4-chlorophenyl)-2,3,9-trimethyl-6H-thieno[3,2-f][1,2,4]triazolo[4,3-a][1,4]diazepin-6-yl)acetamido)butyl)benzofuran-2-carboxamide |
| 4-210' | (S)-5-(3-aminoprop-1-yn-1-yl)-N-(3-(2-(4-(4-chlorophenyl)-2,3,9-trimethyl-6H-thieno[3,2-f][1,2,4]triazolo[4,3-a][1,4]diazepin-6-yl)acetamido)propyl)benzofuran-2-carboxamide |
| 4-214' | (S)-5-(3-aminoprop-1-yn-1-yl)-N-(4-(2-(4-(4-chlorophenyl)-2,3,9-trimethyl-6H-thieno[3,2-f][1,2,4]triazolo[4,3-a][1,4]diazepin-6-yl)acetamido)butyl)-7-methoxybenzofuran-2-carboxamide |
| 4-215' | (S)-5-(3-aminoprop-1-yn-1-yl)-N-(4-(2-(4-(4-chlorophenyl)-2,3,9-trimethyl-6H-thieno[3,2-f][1,2,4]triazolo[4,3-a][1,4]diazepin-6-yl)acetamido)butyl)-6-methoxybenzofuran-2-carboxamide |
| 4-216' | (S)-5-(3-aminoprop-1-yn-1-yl)-N-(3-(2-(4-(4-chlorophenyl)-2,3,9-trimethyl-6H-thieno[3,2-f][1,2,4]triazolo[4,3-a][1,4]diazepin-6-yl)acetamido)propyl)-6-methoxybenzofuran-2-carboxamide |
| 4-217' | (S)-5-(3-aminoprop-1-yn-1-yl)-N-(3-(2-(4-(4-chlorophenyl)-2,3,9-trimethyl-6H-thieno[3,2-f][1,2,4]triazolo[4,3-a][1,4]diazepin-6-yl)acetamido)propyl)-7-methoxybenzofuran-2-carboxamide |
| 4-224' | (S)-5-(3-aminoprop-1-yn-1-yl)-N-(7-(2-(4-(4-chlorophenyl)-2,3,9-trimethyl-6H-thieno[3,2-f][1,2,4]triazolo[4,3-a][1,4]diazepin-6-yl)acetamido)heptyl)furan-2-carboxamide |
| 4-225' | (S)-5-(3-aminoprop-1-yn-1-yl)-N-(9-(2-(4-(4-chlorophenyl)-2,3,9-trimethyl-6H-thieno[3,2-f][1,2,4]triazolo[4,3-a][1,4]diazepin-6-yl)acetamido)nonyl)furan-2-carboxamide hydrochloride |
| 4-228' | methyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzoate hydrochloride |
| 4-229' | (S)-4-(3-aminoprop-1-yn-1-yl)-N-(4-(2-(4-(4-chlorophenyl)-2,3,9-trimethyl-6H-thieno[3,2-f][1,2,4]triazolo[4,3-a][1,4]diazepin-6-yl)acetamido)butyl)benzamide |
| 4-231' | (S)-5-(3-aminoprop-1-yn-1-yl)-N-(2-(2-(4-(4-chlorophenyl)-2,3,9-trimethyl-6H-thieno[3,2-f][1,2,4]triazolo[4,3-a][1,4]diazepin-6-yl)acetamido)pyridin-4-yl)furan-2-carboxamide |
| 4-232' | (S)-5-(3-(aminooxy)prop-1-yn-1-yl)-N-(3-(2-(4-(4-chlorophenyl)-2,3,9-trimethyl-6H-thieno[3,2-f][1,2,4]triazolo[4,3-a][1,4]diazepin-6-yl)acetamido)propyl)furan-2-carboxamide |
| 4-233' | methyl 2-amino-4-(5-((3-(2-((S)-4-(4-chlorophenyl)-2,3,9-trimethyl-6H-thieno[3,2-f][1,2,4]triazolo[4,3-a][1,4]diazepin-6-yl)acetamido)propyl)carbamoyl)furan-2-yl)but-3-ynoate |
| 4-234' | methyl (S)-2-(3-(aminooxy)prop-1-yn-1-yl)-5-((3-(2-(4-(4-chlorophenyl)-2,3,9-trimethyl-6H-thieno[3,2-f][1,2,4]triazolo[4,3-a][1,4]diazepin-6-yl)acetamido)propyl)carbamoyl)furan-3-carboxylate |
| 4-235' | ((S)-5-(3-aminoprop-1-yn-1-yl)-N-(8-(2-(4-(4-chlorophenyl)-2,3,9-trimethyl-6H-thieno[3,2-f][1,2,4]triazolo[4,3-a][1,4]diazepin-6-yl)acetamido)octyl)furan-2-carboxamide hydrochloride |
| 5-1' | ethyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzoate hydrochloride |
| 5-2' | isopropyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzoate hydrochloride |
| 5-3' | cyclopropyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzoate |
| 5-4' | methyl (S)-2-(3-aminoprop-1-yn-1-yl)-4-(3-(2-(4-(4-chlorophenyl)-2,3,9-trimethyl-6H-thieno[3,2-f][1,2,4]triazolo[4,3-a][1,4]diazepin-6-yl)acetamido)propanamido)benzoate hydrochloride |
| 5-5' | methyl (S)-2-(3-aminoprop-1-yn-1-yl)-4-(5-(2-(4-(4-chlorophenyl)-2,3,9-trimethyl-6H-thieno[3,2-f][1,2,4]triazolo[4,3-a][1,4]diazepin-6-yl)acetamido)pentanamido)benzoate hydrochloride |
| 5-6' | methyl (S)-2-(3-aminoprop-1-yn-1-yl)-4-(6-(2-(4-(4-chlorophenyl)-2,3,9-trimethyl-6H-thieno[3,2-f][1,2,4]triazolo[4,3-a][1,4]diazepin-6-yl)acetamido)hexanamido)benzoate hydrochloride |
| 5-8' | (S)-N-(4-(3-aminoprop-1-yn-1-yl)-3-(hydroxymethyl)phenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide hydrochloride |
| 5-9' | (S)-N-(4-(3-aminoprop-1-yn-1-yl)-3-(hydroxymethyl)phenyl)-5-(2-(4-(4-chlorophenyl)-2,3,9-trimethyl-6H-thieno[3,2-f][1,2,4]triazolo[4,3-a][1,4]diazepin-6-yl)acetamido)pentanamide hydrochloride |
| 5-10' | methyl (S)-2-(3-aminoprop-1-yn-1-yl)-4-((4-(2-(4-(4-chlorophenyl)-2,3,9-trimethyl-6H-thieno[3,2-f][1,2,4]triazolo[4,3-a][1,4]diazepin-6-yl)acetamido)butyl)amino)benzoate |
| 5-11' | methyl (S)-2-(3-aminoprop-1-yn-1-yl)-4-((3-(2-(4-(4-chlorophenyl)-2,3,9-trimethyl-6H-thieno[3,2-f][1,2,4]triazolo[4,3-a][1,4]diazepin-6-yl)acetamido)propyl)amino)benzoate |
| 5-12' | methyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butoxy)benzoate hydrochloride |
| 5-13' | methyl (S)-2-(3-aminoprop-1-yn-1-yl)-4-(3-(2-(4-(4-chlorophenyl)-2,3,9-trimethyl-6H-thieno[3,2-f][1,2,4]triazolo[4,3-a][1,4]diazepin-6-yl)acetamido)propoxy)benzoate |
| 5-14' | methyl (S)-2-(3-aminoprop-1-yn-1-yl)-4-((3-(2-(4-(4-chlorophenyl)-2,3,9-trimethyl-6H-thieno[3,2-f][1,2,4]triazolo[4,3-a][1,4]diazepin-6-yl)acetamido)propanoyl)oxy)benzoate |
| 5-15' | methyl (S)-2-(3-aminoprop-1-yn-1-yl)-4-((4-(2-(4-(4-chlorophenyl)-2,3,9-trimethyl-6H-thieno[3,2-f][1,2,4]triazolo[4,3-a][1,4]diazepin-6-yl)acetamido)butanoyl)oxy)benzoate |
| 5-16' | methyl (S)-2-(3-aminoprop-1-yn-1-yl)-4-((5-(2-(4-(4-chlorophenyl)-2,3,9-trimethyl-6H-thieno[3,2-f][1,2,4]triazolo[4,3-a][1,4]diazepin-6-yl)acetamido)pentanoyl)oxy)benzoate |
| 5-17' | methyl (S)-2-(3-aminoprop-1-yn-1-yl)-4-((6-(2-(4-(4-chlorophenyl)-2,3,9-trimethyl-6H-thieno[3,2-f][1,2,4]triazolo[4,3-a][1,4]diazepin-6-yl)acetamido)hexanoyl)oxy)benzoate |
| 5-18' | methyl (S)-2-(3-aminoprop-1-yn-1-yl)-4-((3-(2-(4-(4-chlorophenyl)-2,3,9-trimethyl-6H-thieno[3,2-f][1,2,4]triazolo[4,3-a][1,4]diazepin-6-yl)acetamido)propyl)carbamoyl)benzoate hydrochloride |
| 5-19' | methyl (S)-2-(3-aminoprop-1-yn-1-yl)-4-((2-(2-(4-(4-chlorophenyl)-2,3,9-trimethyl-6H-thieno[3,2-f][1,2,4]triazolo[4,3-a][1,4]diazepin-6-yl)acetamido)ethyl)carbamoyl)benzoate |
| 5-20' | methyl (S)-2-(3-aminoprop-1-yn-1-yl)-4-((4-(2-(4-(4-chlorophenyl)-2,3,9-trimethyl-6H-thieno[3,2-f][1,2,4]triazolo[4,3-a][1,4]diazepin-6-yl)acetamido)butyl)carbamoyl)benzoate |
| 5-21' | methyl (S)-2-(3-aminoprop-1-yn-1-yl)-4-((5-(2-(4-(4-chlorophenyl)-2,3,9-trimethyl-6H-thieno[3,2-f][1,2,4]triazolo[4,3-a][1,4]diazepin-6-yl)acetamido)pentyl)carbamoyl)benzoate |
| 5-30' | methyl (S)-2-(3-aminoprop-1-yn-1-yl)-4-((4-(2-(4-(4-chlorophenyl)-2,3,9-trimethyl-6H-thieno[3,2-f][1,2,4]triazolo[4,3-a][1,4]diazepin-6-yl)acetamido)but-2-yn-1-yl)carbamoyl)benzoate |
| 5-31' | methyl (S,Z)-2-(3-aminoprop-1-yn-1-yl)-4-((4-(2-(4-(4-chlorophenyl)-2,3,9-trimethyl-6H-thieno[3,2-f][1,2,4]triazolo[4,3-a][1,4]diazepin-6-yl)acetamido)but-2-en-1-yl)carbamoyl)benzoate |
| 5-34' | methyl 2-(3-aminoprop-1-yn-1-yl)-4-(((1S,4r)-4-(2-((S)-4-(4-chlorophenyl)-2,3,9-trimethyl-6H-thieno[3,2-f][1,2,4]triazolo[4,3-a][1,4]diazepin-6-yl)acetoxy)cyclohexyl)amino)benzoate |
| 5-35' | methyl (S)-2-(3-aminoprop-1-yn-1-yl)-4-((4-(2-(4-(4-chlorophenyl)-2,3,9-trimethyl-6H-thieno[3,2-f][1,2,4]triazolo[4,3-a][1,4]diazepin-6-yl)acetamido)cyclohexyl)amino)benzoate trifluoroacetic acid |
| 5-49' | methyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)bicyclo[2.2.2]octane-1-carboxamido)benzoate |
| 5-50' | methyl (S)-2-(3-aminoprop-1-yn-1-yl)-4-(1-(2-(2-(4-(4-chlorophenyl)-2,3,9-trimethyl-6H-thieno[3,2-f][1,2,4]triazolo[4,3-a][1,4]diazepin-6-yl)acetamido)ethyl)cyclopropane-1-carboxamido)benzoate |
| 5-53' | (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzoic acid hydrochloride |
| 5-54' | difluoromethyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzoate |
| 5-55' | (S)-N-(3-(3-aminoprop-1-yn-1-yl)-4-(1-(trifluoromethyl)cyclopropane-1-carbonyl)phenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide |
| 5-56' | (S)-N-(3-(3-aminoprop-1-yn-1-yl)-4-(1-fluorocyclopropane-1-carbonyl)phenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide |
| 5-57' | (S)-N-(3-(3-aminoprop-1-yn-1-yl)-4-(1-fluorocyclobutane-1-carbonyl)phenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide |
| 5-58' | 1,1-difluoroethyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzoate |
| 5-59' | (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzamide hydrochloride |
| 5-60' | (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)-N-methylbenzamide |
| 5-61' | (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)-N,N-dimethylbenzamide hydrochloride |
| 5-62' | (S)-N-(3-(3-aminoprop-1-yn-1-yl)-4-hydroxyphenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide |
| 5-63' | (S)-N-(3-(3-aminoprop-1-yn-1-yl)-4-methoxyphenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide trifluoroacetic acid |
| 5-64' | (S)-N-(3-(3-aminoprop-1-yn-1-yl)-4-(2-methoxyethoxy)phenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide |
| 5-65' | (S)-N-(3-(3-aminoprop-1-yn-1-yl)-4-methylphenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide hydrochloride |
| 5-66' | (S)-N-(4-acetyl-3-(3-aminoprop-1-yn-1-yl)phenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide |
| 5-67' | (S)-N-(3-(3-aminoprop-1-yn-1-yl)-4-(2-hydroxyethyl)phenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide |
| 5-68' | (S)-N-(4-(aminomethyl)-3-(3-aminoprop-1-yn-1-yl)phenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide |
| 5-69' | (S)-N-(4-amino-3-(3-aminoprop-1-yn-1-yl)phenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide |
| 5-70' | (S)-N-(3-(3-am inoprop-1-yn-1-yl)-4-(5-methyl-1 ,3,4-oxadiazol-2-yl)phenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide |
| 5-71' | (S)-N-(3-(3-am inoprop-1-yn-1-yl)-4-(3-methyl-1 ,2,4-oxadiazol-5-yl)phenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide |
| 5-72' | (S)-N-(3-(3-aminoprop-1-yn-1-yl)-4-(3-(methylamino)oxetan-3-yl)phenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide |
| 5-73' | (S, E)-N-(3-(3-aminoprop-1-yn-1-yl)-4-(2-fluoroprop-1-en-1-yl)phenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide |
| 5-74' | (S)-N-(3-(3-aminoprop-1-yn-1-yl)-4-carbamimidoylphenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide |
| 5-75' | (S)-N-(3-(3-aminoprop-1-yn-1-yl)-4-(trifluoromethyl)phenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide hydrochloride |
| 5-76' | (S)-N-(3-(3-aminoprop-1-yn-1-yl)-4-chlorophenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide |
| 5-77' | (S)-N-(3-(3-aminoprop-1-yn-1-yl)-4-fluorophenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide hydrochloride |
| 5-78' | methyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)-6-methylbenzoate hydrochloride |
| 5-79' | methyl (S)-2-(3-aminoprop-1-yn-1-yl)-4-(5-(2-(4-(4-chlorophenyl)-2,3,9-trimethyl-6H-thieno[3,2-f][1,2,4]triazolo[4,3-a][1,4]diazepin-6-yl)acetamido)pent-1-yn-1-yl)benzoate |
| 5-80' | methyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)but-1-yn-1-yl)benzoate |
| 5-81' | (S)-N-(3-(3-aminoprop-1-yn-1-yl)-4-(2-(trifluoromethyl)pyrimidin-5-yl)phenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide hydrochloride |
| 5-82' | (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzyl dihydrogen phosphate |
| 5-83' | (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzyl di-tert-butyl phosphate |
| 5-87' | methyl 4-(4-(2-((S)-4-(4-chlorophenyl)-2,3,9-trimethyl-6H-thieno[3,2-fl[1,2,4]triazolo[4,3-a][1,4]diazepin-6-yl)acetamido)butanamido)-2-(3,4-diamino-4-oxobut-1-yn-1-yl)benzoate |
| 5-89' | methyl (S)-3-(3-aminoprop-1-yn-1-yl)-5-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzoate hydrochloride |
| 5-90' | methyl (S)-4-(3-aminoprop-1-yn-1-yl)-2-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzoate hydrochloride |
| 5-92' | methyl (S)-2-((1-aminocyclopropyl)ethynyl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzoate |
| 5-93' | 2-amino-4-(5-(4-(2-((S)-4-(4-chlorophenyl)-2,3,9-trimethyl-6H-thieno[3,2-f][1,2,4]triazolo[4,3-a][1,4]diazepin-6-yl)acetamido)butanamido)-2-(methoxycarbonyl)phenyl)but-3-ynoic acid |
| 5-94' | methyl 2-(3-am i no-4-methoxy-4-oxobut-1-yn-1-yl)-4-(4-(2-((S)-4-(4-chlorophenyl)-2,3,9-trimethyl-6H-thieno[3,2-f][1,2,4]triazolo[4,3-a][1,4]diazepin-6-yl)acetamido)butanamido)benzoate |
| 5-95' | methyl 2-(3-amino-4-hydroxybut-1-yn-1-yl)-4-(4-(2-((S)-4-(4-chlorophenyl)-2,3,9-trimethyl-6H-thieno[3,2-f][1,2,4]triazolo[4,3-a][1,4]diazepin-6-yl)acetamido)butanamido)benzoate |
| 5-96' | methyl (S)-3-(3-aminoprop-1-yn-1-yl)-6-(4-(2-(4-(4-chlorophenyl)-2,3,9-trimethyl-6H-thieno[3,2-f][1,2,4]triazolo[4,3-a][1,4]diazepin-6-yl)acetamido)butanamido)picolinate hydrochloride |
| 5-97' | dimethyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)isophthalate |
| 5-98' | methyl (S)-6-(3-aminoprop-1-yn-1-yl)-2-(4-(2-(4-(4-chlorophenyl)-2,3,9-trimethyl-6H-thieno[3,2-f][1,2,4]triazolo[4,3-a][1,4]diazepin-6-yl)acetamido)butanamido)nicotinate |
| 5-99' | (S)-2-(2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)phenyl)acetic acid |
| 5-100' | ethyl (S)-2-(2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)phenyl)acetate |
| 5-101' | acetoxymethyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzoate |
| 5-102' | (methacryloyloxy)methyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzoate |
| 5-103' | (pivaloyloxy)methyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzoate |
| 5-104' | (S)-N-(3-(3-aminoprop-1-yn-1-yl)phenyl)-6-(2-(4-(4-chlorophenyl)-2,3,9-trimethyl-6H-thieno[3,2-f][1,2,4]triazolo[4,3-a][1,4]diazepin-6-yl)acetamido)hexanamide hydrochloride |
| 5-106' | methyl (S)-2-(3-aminoprop-1-yn-1-yl)-4-(5-(2-(4-(4-chlorophenyl)-2,3,9-trimethyl-6H-thieno[3,2-f][1,2,4]triazolo[4,3-a][1,4]diazepin-6-yl)ethoxy)-5-oxopentanamido)benzoate hydrochloride |
| 5-107' | (S)-N-(3-(3-aminoprop-1-yn-1-yl)phenyl)-8-(2-(4-(4-chlorophenyl)-2,3,9-trimethyl-6H-thieno[3,2-f][1,2,4]triazolo[4,3-a][1,4]diazepin-6-yl)acetamido)octanamide hydrochloride |
| 5-108' | (S)-N-(3-(3-aminoprop-1-yn-1-yl)phenyl)-11-(2-(4-(4-chlorophenyl)-2,3,9-trimethyl-6H-thieno[3,2-f][1,2,4]triazolo[4,3-a][1,4]diazepin-6-yl)acetamido)undecanamide hydrochloride |
| 5-109' | (S)-2-(4-(4-chlorophenyl)-2,3,9-trimethyl-6H-thieno[3,2-f][1,2,4]triazolo[4,3-a][1,4]diazepin-6-yl)ethyl 11-((3-(3-aminoprop-1-yn-1-yl)phenyl)amino)-11-oxoundecanoate hydrochloride |
| 5-110' | methyl (S)-2-(3-aminoprop-1-yn-1-yl)-4-(11-(2-(4-(4-chlorophenyl)-2,3,9-trimethyl-6H-thieno[3,2-f][1,2,4]triazolo[4,3-a][1,4]diazepin-6-yl)ethoxy)-11-oxoundecanamido)benzoate hydrochloride |
| 5-113' | methyl (S)-2-(3-aminoprop-1-yn-1-yl)-4-(5-((7-(2-(4-(4-chlorophenyl)-2,3,9-trimethyl-6H-thieno[3,2-f][1,2,4]triazolo[4,3-a][1,4]diazepin-6-yl)acetamido)heptyl)amino)-5-oxopentanamido)benzoate |
| 5-115' | methyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetoxy)butanamido)benzoate hydrochloride |
| 5-121' | (S)-2,4-bis(3-aminoprop-1-yn-1-yl)-N-(7-(2-(4-(4-chlorophenyl)-2,3,9-trimethyl-6H-thieno[3,2-f][1,2,4]triazolo[4,3-a][1,4]diazepin-6-yl)acetamido)heptyl)benzamide |
| 5-122' | methyl (S)-2-(3-aminoprop-1-yn-1-yl)-4-(3-(4-(2-(4-(4-chlorophenyl)-2,3,9-trimethyl-6H-thieno[3,2-f][1,2,4]triazolo[4,3-a][1,4]diazepin-6-yl)acetamido)butanamido)prop-1-yn-1-yl)benzoate |
| 5-123' | (S)-N-(3-(3-(3-aminoprop-1-yn-1-yl)phenyl)prop-2-yn-1-yl)-4-(2-(4-(4-chlorophenyl)-2,3,9-trimethyl-6H-thieno[3,2-f][1,2,4]triazolo[4,3-a][1,4]diazepin-6-yl)acetamido)butanamide |
| 5-126' | (S)-N-(4-(3-aminoprop-1-yn-1-yl)phenyl)-20-(2-(4-(4-chlorophenyl)-2,3,9-trimethyl-6H-thieno[3,2-f][1,2,4]triazolo[4,3-a][1,4]diazepin-6-yl)acetamido)-3,6,9,12,15,18-hexaoxaicosanamide |
| 5-127' | (S)-N-(3-(3-aminoprop-1-yn-1-yl)phenyl)-20-(2-(4-(4-chlorophenyl)-2,3,9-trimethyl-6H-thieno[3,2-f][1,2,4]triazolo[4,3-a][1,4]diazepin-6-yl)acetamido)-3,6,9,12,15,18-hexaoxaicosanamide |
| 5-135' | (S)-N-(4-(3-aminoprop-1-yn-1-yl)-3-(hydroxymethyl)phenyl)-8-(2-(4-(4-chlorophenyl)-2,3,9-trimethyl-6H-thieno[3,2-f][1,2,4]triazolo[4,3-a][1,4]diazepin-6-yl)acetamido)octanamide |
| 5-148' | methyl (S)-2-(3-acetamidoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)butanamido)benzoate |
| 5-149' | methyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)-N-methylacetamido)butanamido)benzoate hydrochloride |
| 5-150' | methyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)-N-methylbutanamido)benzoate hydrochloride |
| 5-169' | (S)-2-(3-aminoprop-1-yn-1-yl)-N-(3-(2-(4-(4-chlorophenyl)-2,3,9-trimethyl-6H-thieno[3,2-f][1,2,4]triazolo[4,3-a][1,4]diazepin-6-yl)acetamido)propyl)oxazole-5-carboxamide |
| 5-170' | (S)-N-(3-(3-aminoprop-1-yn-1-yl)-4-morpholinophenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide |
| 5-171' | (S)-N-(3-(3-aminoprop-1-yn-1-yl)-4-(4-methylpiperazin-1-yl)phenyl)-4-(2-(4-(4-chlorophenyl)-2,3,9-trimethyl-6H-thieno[3,2-f][1,2,4]triazolo[4,3-a][1,4]diazepin-6-yl)acetamido)butanamide |
**[Table 9]**
| No. | Compound |
|---|---|
| 4-165" | N-(6-((4-(3-aminoprop-1-yn-1-yl)phenyl)amino)-6-oxohexyl)-4-((9-chloro-7-(2-fluoro-6-methoxyphenyl)-5H-benzo[c]pyrimido[4,5-e]azepin-2-yl)amino)-2-methoxybenzamide trifluoroacetic acid |
| 4-166" | 5-(3-aminoprop-1-yn-1-yl)-N-(3-(4-((9-chloro-7-(2-fluoro-6-methoxyphenyl)-5H-benzo[c]pyrimido[4,5-e]azepin-2-yl)amino)-2-methoxybenzamido)propyl)furan-2-carboxamide trifluoroacetic acid |
| 4-228" | methyl 2-(3-aminoprop-1-yn-1-yl)-4-(4-(4-((9-chloro-7-(2-fluoro-6-methoxyphenyl)-5H-benzo[c]pyrimido[4,5-e]azepin-2-yl)amino)-2-methoxybenzamido)butanamido)benzoate trifluoroacetic acid |
| 5-8" | N-(4-((4-(3-aminoprop-1-yn-1-yl)-3-(hydroxymethyl)phenyl)amino)-4-oxobutyl)-4-((9-chloro-7-(2-fluoro-6-methoxyphenyl)-5H-benzo[c]pyrimido[4,5-e]azepin-2-yl)amino)-2-methoxybenzamide trifluoroacetic acid |
| 5-18" | methyl 2-(3-aminoprop-1-yn-1-yl)-4-((3-(4-((9-chloro-7-(2-fluoro-6-methoxyphenyl)-5H-benzo[c]pyrimido[4,5-e]azepin-2-yl)amino)-2-methoxybenzamido)propyl)carbamoyl)benzoate hydrochloride |
| 5-35" | methyl 2-(3-aminoprop-1-yn-1-yl)-4-((4-(4-((9-chloro-7-(2-fluoro-6-methoxyphenyl)-5H-benzo[c]pyrimido[4,5-e]azepin-2-yl)amino)-2-methoxybenzamido)cyclohexyl)amino)benzoate trifluoroacetic acid |
| 5-81" | N-(4-((3-(3-aminoprop-1-yn-1-yl)-4-(2-(trifluoromethyl)pyrimidin-5-yl)phenyl)amino)-4-oxobutyl)-4-((9-chloro-7-(2-fluoro-6-methoxyphenyl)-5H-benzo[c]pyrimido[4,5-e]azepin-2-yl)amino)-2-methoxybenzamide trifluoroacetic acid |
| 5-104" | N-(6-((3-(3-aminoprop-1-yn-1-yl)phenyl)amino)-6-oxohexyl)-4-((9-chloro-7-(2-fluoro-6-methoxyphenyl)-5H-benzo[c]pyrimido[4,5-e]azepin-2-yl)amino)-2-methoxybenzamide trifluoroacetic acid |
| 5-150" | methyl 2-(3-aminoprop-1-yn-1-yl)-4-(4-(4-((9-chloro-7-(2-fluoro-6-methoxyphenyl)-5H-benzo[c]pyrimido[4,5-e]azepin-2-yl)amino)-2-methoxybenzamido)-N-methylbutanamido)benzoate trifluoroacetic acid |
**[Table 10]**
| No. | Compound |
|---|---|
| 4-166‴ | 2-(4-(5'-(cyclopropylcarbamoyl)-2'-methyl-[1,1'-biphenyl]-4-carbonyl)phenoxy)ethyl (3-(5-(3-aminoprop-1-yn-1-yl)furan-2-carboxamido)propyl)carbamate trifluoroacetic acid |
| 5-106‴ | methyl 2-(3-aminoprop-1-yn-1-yl)-4-(5-(2-(4-(5'-(cyclopropylcarbamoyl)-2'-methyl-[1,1'-biphenyl]-4-carbonyl)phenoxy)ethoxy)-5-oxopentanamido)benzoate hydrochloride |
**[Table 11]**
| No. | Compound |
|---|---|
| 4-2' | methyl (S)-2-(3-aminoprop-1-yn-1-yl)-5-(4-(2-(4-(4-chlorophenyl)-2,3,9-trimethyl-6H-thieno[3,2-f][1,2,4]triazolo[4,3-a][1,4]diazepin-6-yl)acetyl)piperazin-1-yl)benzoate hydrochloride |
| 4-3' | (S)-2-(3-aminoprop-1-yn-1-yl)-5-(4-(2-(4-(4-chlorophenyl)-2,3,9-trimethyl-6H-thieno[3,2-f][1,2,4]triazolo[4,3-a][1,4]diazepin-6-yl)acetyl)piperazin-1-yl)benzoic acid |
| 4-4' | (S)-1-(4-(4-(3-aminoprop-1-yn-1-yl)-3-(hydroxymethyl)phenyl)piperazin-1-yl)-2-(4-(4-chlorophenyl)-2,3,9-trimethyl-6H-thieno[3,2-f][1,2,4]triazolo[4,3-a][1,4]diazepin-6-yl)ethan-1-one |
| 4-94' | methyl (S)-2-(4-aminobut-1-yn-1-yl)-5-(4-(2-(4-(4-chlorophenyl)-2,3,9-trimethyl-6H-thieno[3,2-f][1,2,4]triazolo[4,3-a][1,4]diazepin-6-yl)acetyl)piperazin-1-yl)benzoate |
| 4-95' | (S)-2-(4-aminobut-1-yn-1-yl)-5-(4-(2-(4-(4-chlorophenyl)-2,3,9-trimethyl-6H-thieno[3,2-f][1,2,4]triazolo[4,3-a][1,4]diazepin-6-yl)acetyl)piperazin-1-yl)benzoic acid |
| 4-96' | (S)-1-(4-(4-(4-aminobut-1-yn-1-yl)-3-(hydroxymethyl)phenyl)piperazin-1-yl)-2-(4-(4-chlorophenyl)-2,3,9-trimethyl-6H-thieno[3,2-f][1,2,4]triazolo[4,3-a][1,4]diazepin-6-yl)ethan-1-one |
| 4-97' | methyl (S)-2-(5-aminopent-1-yn-1-yl)-5-(4-(2-(4-(4-chlorophenyl)-2,3,9-trimethyl-6H-thieno[3,2-f][1,2,4]triazolo[4,3-a][1,4]diazepin-6-yl)acetyl)piperazin-1-yl)benzoate |
| 4-98' | (S)-2-(5-aminopent-1-yn-1-yl)-5-(4-(2-(4-(4-chlorophenyl)-2,3,9-trimethyl-6H-thieno[3,2-f][1,2,4]triazolo[4,3-a][1,4]diazepin-6-yl)acetyl)piperazin-1-yl)benzoic acid |
| 4-99' | (S)-1-(4-(4-(5-aminopent-1-yn-1-yl)-3-(hydroxymethyl)phenyl)piperazin-1-yl)-2-(4-(4-chlorophenyl)-2,3,9-trimethyl-6H-thieno[3,2-f][1,2,4]triazolo[4,3-a][1,4]diazepin-6-yl)ethan-1-one |
| 4-100' | methyl (S)-2-(6-aminohex-1-yn-1-yl)-5-(4-(2-(4-(4-chlorophenyl)-2,3,9-trimethyl-6H-thieno[3,2-f][1,2,4]triazolo[4,3-a][1,4]diazepin-6-yl)acetyl)piperazin-1-yl)benzoate |
| 4-101' | (S)-2-(6-aminohex-1-yn-1-yl)-5-(4-(2-(4-(4-chlorophenyl)-2,3,9-trimethyl-6H-thieno[3,2-f][1,2,4]triazolo[4,3-a][1,4]diazepin-6-yl)acetyl)piperazin-1-yl)benzoic acid |
| 4-102' | (S)-1-(4-(4-(6-aminohex-1-yn-1-yl)-3-(hydroxymethyl)phenyl)piperazin-1-yl)-2-(4-(4-chlorophenyl)-2,3,9-trimethyl-6H-thieno[3,2-f][1,2,4]triazolo[4,3-a][1,4]diazepin-6-yl)ethan-1-one |
| 4-103' | methyl (S)-2-(7-aminohept-1-yn-1-yl)-5-(4-(2-(4-(4-chlorophenyl)-2,3,9-trimethyl-6H-thieno[3,2-f][1,2,4]triazolo[4,3-a][1,4]diazepin-6-yl)acetyl)piperazin-1-yl)benzoate |
| 4-104' | (S)-2-(7-aminohept-1-yn-1-yl)-5-(4-(2-(4-(4-chlorophenyl)-2,3,9-trimethyl-6H-thieno[3,2-f][1,2,4]triazolo[4,3-a][1,4]diazepin-6-yl)acetyl)piperazin-1-yl)benzoic acid |
| 4-105' | (S)-1-(4-(4-(7-aminohept-1-yn-1-yl)-3-(hydroxymethyl)phenyl)piperazin-1-yl)-2-(4-(4-chlorophenyl)-2,3,9-trimethyl-6H-thieno[3,2-f][1,2,4]triazolo[4,3-a][1,4]diazepin-6-yl)ethan-1-one |
| 4-106' | methyl (S)-2-(8-aminooct-1-yn-1-yl)-5-(4-(2-(4-(4-chlorophenyl)-2,3,9-trimethyl-6H-thieno[3,2-f][1,2,4]triazolo[4,3-a][1,4]diazepin-6-yl)acetyl)piperazin-1-yl)benzoate |
| 4-107' | (S)-2-(8-aminooct-1-yn-1-yl)-5-(4-(2-(4-(4-chlorophenyl)-2,3,9-trimethyl-6H-thieno[3,2-f][1,2,4]triazolo[4,3-a][1,4]diazepin-6-yl)acetyl)piperazin-1-yl)benzoic acid |
| 4-108' | (S)-1-(4-(4-(8-aminooct-1-yn-1-yl)-3-(hydroxymethyl)phenyl)piperazin-1-yl)-2-(4-(4-chlorophenyl)-2,3,9-trimethyl-6H-thieno[3,2-f][1,2,4]triazolo[4,3-a][1,4]diazepin-6-yl)ethan-1-one |
| 4-134' | (S)-1-(4-(4-(3-aminoprop-1-yn-1-yl)phenyl)-[1,4'-bipiperidin]-1'-yl)-2-(4-(4-chlorophenyl)-2,3,9-trimethyl-6H-thieno[3,2-f][1,2,4]triazolo[4,3-a][1,4]diazepin-6-yl)ethan-1-one |
| 4-135' | (S)-1-(3-(4-(4-(3-aminoprop-1-yn-1-yl)phenyl)piperidin-1-yl)azetidin-1-yl)-2-(4-(4-chlorophenyl)-2,3,9-trimethyl-6H-thieno[3,2-f][1,2,4]triazolo[4,3-a][1,4]diazepin-6-yl)ethan-1-one |
| 4-136' | (S)-1-(4-(4-(3-aminoprop-1-yn-1-yl)phenyl)piperidin-1-yl)-2-(4-(4-chlorophenyl)-2,3,9-trimethyl-6H-thieno[3,2-f][1,2,4]triazolo[4,3-a][1,4]diazepin-6-yl)ethan-1-one |
| 4-146' | (S)-1-(4-(4-(3-aminoprop-1-yn-1-yl)phenyl)-3,6-dihydropyridin-1(2H)-yl)-2-(4-(4-chlorophenyl)-2,3,9-trimethyl-6H-thieno[3,2-f][1,2,4]triazolo[4,3-a][1,4]diazepin-6-yl)ethan-1-one |
| 4-226' | (S)-1-(4-(4-(3-aminoprop-1-yn-1-yl)phenyl)piperazin-1-yl)-2-(4-(4-chlorophenyl)-2,3,9-trimethyl-6H-thieno[3,2-f][1,2,4]triazolo[4,3-a][1,4]diazepin-6-yl)ethan-1-one hydrochloride |
| 4-227' | (S)-1-(4-(5-(3-aminoprop-1-yn-1-yl)furan-2-carbonyl)piperazin-1-yl)-2-(4-(4-chlorophenyl)-2,3,9-trimethyl-6H-thieno[3,2-f][1,2,4]triazolo[4,3-a][1,4]diazepin-6-yl)ethan-1-one hydrochloride |
| 5-7' | methyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetyl)piperazin-1-yl)benzoate hydrochloride |
| 5-22' | methyl (S)-2-(3-aminoprop-1-yn-1-yl)-4-((1-(2-(4-(4-chlorophenyl)-2,3,9-trimethyl-6H-thieno[3,2-f][1,2,4]triazolo[4,3-a][1,4]diazepin-6-yl)acetyl)piperidin-4-yl)carbamoyl)benzoate hydrochloride |
| 5-23' | methyl (S)-2-(3-aminoprop-1 -yn- 1 -yl)-4-(1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamido)benzoate hydrochloride |
| 5-24' | methyl (S)-2-(3-aminoprop-1-yn-1-yl)-4-(2-(1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamido)acetamido)benzoate |
| 5-25' | methyl (S)-2-(3-aminoprop-1-yn-1-yl)-4-(3-(1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamido)propanamido)benzoate hydrochloride |
| 5-26' | methyl (S)-2-(3-aminoprop-1-yn-1-yl)-4-(4-(1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamido)butanamido)benzoate hydrochloride |
| 5-27' | methyl (S)-2-(3-aminoprop-1-yn-1-yl)-4-((3-(1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamido)propyl)carbamoyl)benzoate hydrochloride |
| 5-28' | methyl (S)-2-(3-aminoprop-1-yn-1-yl)-4-(3-(1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamido)prop-1-yn-1-yl)benzoate hydrochloride |
| 5-29' | methyl (S)-2-(3-aminoprop-1-yn-1-yl)-4-(4-((1-(2-(4-(4-chlorophenyl)-2,3,9-trimethyl-6H-thieno[3,2-f][1,2,4]triazolo[4,3-a][1,4]diazepin-6-yl)acetyl)piperidin-4-yl)methyl)piperazin-1-yl)benzoate hydrochloride |
| 5-36' | methyl (S)-2-(3-aminoprop-1-yn-1-yl)-4-((1-(2-(4-(4-chlorophenyl)-2,3,9-trimethyl-6H-thieno[3,2-f][1,2,4]triazolo[4,3-a][1,4]diazepin-6-yl)acetyl)piperidin-4-yl)oxy)benzoate |
| 5-37' | methyl (S)-2-(3-aminoprop-1-yn-1-yl)-4-(4-(1-(2-(4-(4-chlorophenyl)-2,3,9-trimethyl-6H-thieno[3,2-f][1,2,4]triazolo[4,3-a][1,4]diazepin-6-yl)acetyl)azetidin-3-yl)piperazin-1-yl)benzoate |
| 5-38' | methyl (S)-2-(3-aminoprop-1-yn-1-yl)-4-(2-(2-(4-(4-chlorophenyl)-2,3,9-trimethyl-6H-thieno[3,2-f][1,2,4]triazolo[4,3-a][1,4]diazepin-6-yl)acetyl)-2,7-diazaspiro[3.5]nonan-7-yl)benzoate |
| 5-39' | methyl (S)-2-(3-aminoprop-1-yn-1-yl)-4-(2-(2-(4-(4-chlorophenyl)-2,3,9-trimethyl-6H-thieno[3,2-f][1,2,4]triazolo[4,3-a][1,4]diazepin-6-yl)acetyl)-2,7-diazaspiro[3.5]nonane-7-carbonyl)benzoate trifluoroacetic acid |
| 5-40' | methyl (S)-2-(3-aminoprop-1-yn-1-yl)-4-(6-(2-(4-(4-chlorophenyl)-2,3,9-trimethyl-6H-thieno[3,2-f][1,2,4]triazolo[4,3-a][1,4]diazepin-6-yl)acetyl)-2,6-diazaspiro[3.3]heptan-2-yl)benzoate trifluoroacetic acid |
| 5-41' | methyl (S)-2-(3-aminoprop-1-yn-1-yl)-4-(6-(2-(4-(4-chlorophenyl)-2,3,9-trimethyl-6H-thieno[3,2-f][1,2,4]triazolo[4,3-a][1,4]diazepin-6-yl)acetyl)-2,6-diazaspiro[3.3]heptane-2-carbonyl)benzoate trifluoroacetic acid |
| 5-42' | methyl (S)-2-(3-aminoprop-1-yn-1-yl)-4-((2-(2-(4-(4-chlorophenyl)-2,3,9-trimethyl-6H-thieno[3,2-f][1,2,4]triazolo[4,3-a][1,4]diazepin-6-yl)acetyl)-2-azaspiro[3.3]heptan-6-yl)carbamoyl)benzoate |
| 5-43' | methyl (S)-2-(3-aminoprop-1-yn-1-yl)-4-(((2-(2-(4-(4-chlorophenyl)-2,3,9-trimethyl-6H-thieno[3,2-f][1,2,4]triazolo[4,3-a][1,4]diazepin-6-yl)acetyl)-2-azaspiro[3.3]heptan-6-ylidene)amino)oxy)benzoate |
| 5-44' | methyl 2-(3-aminoprop-1-yn-1-yl)-4-(5-(2-((S)-4-(4-chlorophenyl)-2,3,9-trimethyl-6H-thieno[3,2-f][1,2,4]triazolo[4,3-a][1,4]diazepin-6-yl)acetyl)hexahydropyrrolo[3,4-c]pyrrol-2(1H)-yl)benzoate trifluoroacetic acid |
| 5-45' | methyl 2-(3-aminoprop-1-yn-1-yl)-4-(5-(2-((S)-4-(4-chlorophenyl)-2,3,9-trimethyl-6H-thieno[3,2-f][1,2,4]triazolo[4,3-a][1,4]diazepin-6-yl)acetyl)octahydropyrrolo[3,4-c]pyrrole-2-carbonyl)benzoate |
| 5-46' | methyl (S)-2-(3-aminoprop-1-yn-1-yl)-4-(4-(1-(2-(4-(4-chlorophenyl)-2,3,9-trimethyl-6H-thieno[3,2-f][1,2,4]triazolo[4,3-a][1,4]diazepin-6-yl)acetyl)azetidin-3-yl)piperidin-1-yl)benzoate |
| 5-47' | methyl (S)-2-(3-aminoprop-1-yn-1-yl)-4-(3-(1-(2-(4-(4-chlorophenyl)-2,3,9-trimethyl-6H-thieno[3,2-f][1,2,4]triazolo[4,3-a][1,4]diazepin-6-yl)acetyl)piperidin-4-yl)azetidin-1-yl)benzoate |
| 5-48' | methyl 2-(3-aminoprop-1-yn-1-yl)-4-(6-(2-((S)-4-(4-chlorophenyl)-2,3,9-trimethyl-6H-thieno[3,2-f][1,2,4]triazolo[4,3-a][1,4]diazepin-6-yl)acetyl)-3,6-diazabicyclo[3.1.1]heptan-3-yl)benzoate |
| 5-85' | methyl (S)-2-(3-aminoprop-1-yn-1-yl)-4-(((1-(2-(4-(4-chlorophenyl)-2,3,9-trimethyl-6H-thieno[3,2-f][1,2,4]triazolo[4,3-a][1,4]diazepin-6-yl)acetyl)piperidin-4-yl)methyl)amino)benzoate trifluoroacetic acid |
| 5-86' | methyl (S)-2-(3-aminoprop-1-yn-1-yl)-4-((1-(2-(4-(4-chlorophenyl)-2,3,9-trimethyl-6H-thieno[3,2-f][1,2,4]triazolo[4,3-a][1,4]diazepin-6-yl)acetyl)piperidin-4-yl)amino)benzoate |
| 5-88' | (S)-1-(4-(4-(3-aminoprop-1-yn-1-yl)phenyl)-4-hydroxypiperidin-1-yl)-2-(4-(4-chlorophenyl)-2,3,9-trimethyl-6H-thieno[3,2-f][1,2,4]triazolo[4,3-a][1,4]diazepin-6-yl)ethan-1-one |
| 5-112' | methyl (S)-3-(3-aminoprop-1-yn-1-yl)-6-(1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamido)picolinate |
| 5-118' | methyl 2-(3-aminoprop-1-yn-1-yl)-5-(6-(2-((S)-4-(4-chlorophenyl)-2,3,9-trimethyl-6H-thieno[3,2-f][1,2,4]triazolo[4,3-a][1,4]diazepin-6-yl)acetyl)-3,6-diazabicyclo[3.1.1]heptan-3-yl)benzoate |
| 5-119' | methyl (S)-2-(3-aminoprop-1-yn-1-yl)-5-(4-(2-(4-(4-chlorophenyl)-2,3,9-trimethyl-6H-thieno[3,2-f][1,2,4]triazolo[4,3-a][1,4]diazepin-6-yl)acetyl)-3-oxopiperazin-1-yl)benzoate |
| 5-120' | methyl (S)-2-(3-aminoprop-1-yn-1-yl)-4-(4-(1-(2-(4-(4-chlorophenyl)-2,3,9-trimethyl-6H-thieno[3,2-f][1,2,4]triazolo[4,3-a][1,4]diazepin-6-yl)acetyl)piperidine-4-carbonyl)piperazin-1-yl)benzoate |
| 5-124' | (S)-1-(4-((4-(5-(4-(3-aminoprop-1-yn-1-yl)phenyl)furan-2-carbonyl)piperazin-1-yl)methyl)piperidin-1-yl)-2-(4-(4-chlorophenyl)-2,3,9-trimethyl-6H-thieno[3,2-f][1,2,4]triazolo[4,3-a][1,4]diazepin-6-yl)ethan-1-one |
| 5-125' | (S)-1-(4-(5-(4-(3-aminoprop-1-yn-1-yl)phenyl)furan-2-carbonyl)piperazin-1-yl)-2-(4-(4-chlorophenyl)-2,3,9-trimethyl-6H-thieno[3,2-f][1,2,4]triazolo[4,3-a][1,4]diazepin-6-yl)ethan-1-one |
| 5-129' | (S)-5-(4-(3-aminoprop-1-yn-1-yl)phenyl)-N-(1-(2-(4-(4-chlorophenyl)-2,3,9-trimethyl-6H-thieno[3,2-f][1,2,4]triazolo[4,3-a][1,4]diazepin-6-yl)acetyl)piperidin-4-yl)furan-2-carboxamide |
| 5-131' | (S)-N-(4-(3-aminoprop-1-yn-1-yl)phenyl)-1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamide |
| 5-134' | (S)-N-(4-(3-aminoprop-1-yn-1-yl)-3-(hydroxymethyl)phenyl)-1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamide |
| 5-139' | (S)-2-(3-aminoprop-1-yn-1-yl)-4-(((1-(2-(4-(4-chlorophenyl)-2,3,9-trimethyl-6H-thieno[3,2-f][1,2,4]triazolo[4,3-a][1,4]diazepin-6-yl)acetyl)piperidin-4-yl)methyl)amino)benzoic acid trifluoroacetic acid |
| 5-140' | methyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetyl)piperazine-1-carbonyl)benzoate hydrochloride |
| 5-152' | methyl (S)-2-(3-aminoprop-1-yn-1-yl)-4-(1-(2-(4-(4-chlorophenyl)-2,3,9-trimethyl-6H-thieno[3,2-f][1,2,4]triazolo[4,3-a][1,4]diazepin-6-yl)acetyl)-N-methylpiperidine-4-carboxamido)benzoate trifluoroacetic acid |
| 5-153' | methyl (S)-2-(3-aminoprop-1-yn-1-yl)-4-((1-(2-(4-(4-chlorophenyl)-2,3,9-trimethyl-6H-thieno[3,2-f][1,2,4]triazolo[4,3-a][1,4]diazepin-6-yl)acetyl)piperidin-4-yl)(methyl)carbamoyl)benzoate trifluoroacetic acid |
| 5-154' | methyl (S)-4-(1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamido)-2-(3-(sulfamoylamino)prop-1-yn-1-yl)benzoate trifluoroacetic acid |
| 5-155' | methyl (S)-2-((1-aminocyclopropyl)ethynyl)-4-(1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamido)benzoate |
| 5-156' | methyl (S)-2-(3-(((4-chlorophenyl)(5-fluoro-2-hydroxyphenyl)methylene)amino)prop-1-yn-1-yl)-4-(1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamido)benzoate |
| 5-157' | methyl 4-(1-(2-((S)-4-(4-chlorophenyl)-2,3,9-trimethyl-6H-thieno[3,2-f][1,2,4]triazolo[4,3-a][1,4]diazepin-6-yl)acetyl)piperidine-4-carboxamido)-2-(3-(((1-(isobutyryloxy)ethoxy)carbonyl)amino)prop-1-yn-1-yl)benzoate |
| 5-158' | methyl (S)-4-(1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamido)-2-(3-(sulfamoyloxy)prop-1-yn-1-yl)benzoate trifluoroacetic acid |
| 5-159' | methyl 2-(3-amino-4-mercaptobut-1-yn-1-yl)-4-((1-(2-((S)-4-(4-chlorophenyl)-2,3,9-trimethyl-6H-thieno[3,2-f][1,2,4]triazolo[4,3-a][1,4]diazepin-6-yl)acetyl)piperidin-4-yl)carbamoyl)benzoate |
| 5-160' | (S)-5-(3-(3-aminoprop-1-yn-1-yl)phenyl)-N-(1-(2-(4-(4-chlorophenyl)-2,3,9-trimethyl-6H-thieno[3,2-f][1,2,4]triazolo[4,3-a][1,4]diazepin-6-yl)acetyl)piperidin-4-yl)furan-2-carboxamide |
| 5-162' | methyl 4-(6-(2-((S)-4-(4-chlorophenyl)-2,3,9-trimethyl-6H-thieno[3,2-f][1,2,4]triazolo[4,3-a][1,4]diazepin-6-yl)acetyl)-2,6-diazaspiro[3.3]heptan-2-yl)-2-(3-(((1-(isobutyryloxy)ethoxy)carbonyl)amino)prop-1-yn-1-yl)benzoate |
| 5-167' | methyl (S)-3-(3-aminoprop-1-yn-1-yl)-6-(1-(2-(4-(4-chlorophenyl)-2,3,9-trimethyl-6H-thieno[3,2-f][1,2,4]triazolo[4,3-a][1,4]diazepin-6-yl)acetyl)piperidine-4-carboxamido)picolinate |
| 5-168' | methyl (S)-2-(3-aminoprop-1-yn-1-yl)-4-(7-(2-(4-(4-chlorophenyl)-2,3,9-trimethyl-6H-thieno[3,2-f][1,2,4]triazolo[4,3-a][1,4]diazepin-6-yl)acetyl)-2,7-diazaspiro[3.5]nonan-2-yl)benzoate hydrochloride |
**[Table 12]**
| No. | Compound |
|---|---|
| 5-23" | methyl 2-(3-aminoprop-1-yn-1-yl)-4-(1-(4-((9-chloro-7-(2-fluoro-6-methoxyphenyl)-5H-benzo[c]pyrimido[4,5-e]azepin-2-yl)amino)-2-methoxybenzoyl)piperidine-4-carboxamido)benzoate hydrochloride |
| 5-28" | methyl 2-(3-aminoprop-1-yn-1-yl)-4-(3-(1-(4-((9-chloro-7-(2-fluoro-6-methoxyphenyl)-5H-benzo[c]pyrimido[4,5-e]azepin-2-yl)amino)-2-methoxybenzoyl)piperidine-4-carboxamido)prop-1-yn-1-yl)benzoate trifluoroacetic acid |
| 5-29" | methyl 2-(3-aminoprop-1-yn-1-yl)-4-(4-((1-(4-((9-chloro-7-(2-fluoro-6-methoxyphenyl)-5H-benzo[c]pyrimido[4,5-e]azepin-2-yl)amino)-2-methoxybenzoyl)piperidin-4-yl)methyl)piperazin-1-yl)benzoate trifluoroacetic acid |
| 5-40" | methyl 2-(3-aminoprop-1-yn-1-yl)-4-(6-(4-((9-chloro-7-(2-fluoro-6-methoxyphenyl)-5H-benzo[c]pyrimido[4,5-e]azepin-2-yl)amino)-2-methoxybenzoyl)-2,6-diazaspiro[3.3]heptan-2-yl)benzoate trifluoroacetic acid |
**[Table 13]**
| No. | Compound |
|---|---|
| 5-39‴ | 2-(4-(5'-(cyclopropylcarbamoyl)-2'-methyl-[1,1'-biphenyl]-4-carbonyl)phenoxy)ethyl 7-(3-(3-aminoprop-1-yn-1-yl)-4-(methoxycarbonyl)benzoyl)-2,7-diazaspiro[3.5]nonane-2-carboxylate trifluoroacetic acid |
| 5-140‴ | 2-(4-(5'-(cyclopropylcarbamoyl)-2'-methyl-[1,1'-biphenyl]-4-carbonyl)phenoxy)ethyl 4-(3-(3-aminoprop-1-yn-1-yl)-4-(methoxycarbonyl)benzoyl)piperazine-1-carboxylate trifluoroacetic acid |
**[Table 14]**
| No. | Compound |
|---|---|
| 4-207' | (S)-5-(4-(3-aminoprop-1-yn-1-yl)phenyl)-N-(4-(2-(4-(4-chlorophenyl)-2,3,9-trimethyl-6H-thieno[3,2-f][1,2,4]triazolo[4,3-a][1,4]diazepin-6-yl)acetamido)but-2-yn-1-yl)furan-2-carboxamide |
| 4-208' | (S)-5-(4-(3-aminoprop-1-yn-1-yl)phenyl)-N-(4-(2-(4-(4-chlorophenyl)-2,3,9-trimethyl-6H-thieno[3,2-f][1,2,4]triazolo[4,3-a][1,4]diazepin-6-yl)acetamido)butyl)furan-2-carboxamide |
| 4-211' | (S)-5-(4-(3-aminoprop-1-yn-1-yl)phenyl)-N-(3-(2-(4-(4-chlorophenyl)-2,3,9-trimethyl-6H-thieno[3,2-f][1,2,4]triazolo[4,3-a][1,4]diazepin-6-yl)acetamido)propyl)furan-2-carboxamide |
| 5-32' | methyl (S)-2-(3-aminoprop-1-yn-1-yl)-4-(4-(3-(2-(4-(4-chlorophenyl)-2,3,9-trimethyl-6H-thieno[3,2-f][1,2,4]triazolo[4,3-a][1,4]diazepin-6-yl)acetamido)propanoyl)piperazin-1-yl)benzoate hydrochloride |
| 5-33' | methyl (S)-2-(3-aminoprop-1-yn-1-yl)-5-(4-(3-(2-(4-(4-chlorophenyl)-2,3,9-trimethyl-6H-thieno[3,2-f][1,2,4]triazolo[4,3-a][1,4]diazepin-6-yl)acetamido)propanoyl)piperazin-1-yl)benzoate hydrochloride |
| 5-51' | methyl (S)-2-(3-aminoprop-1-yn-1-yl)-4-(5-(2-(4-(4-chlorophenyl)-2,3,9-trimethyl-6H-thieno[3,2-f][1,2,4]triazolo[4,3-a][1,4]diazepin-6-yl)acetamido)-1,3,4-oxadiazol-2-yl)benzoate |
| 5-111' | (S)-5-(4-(3-aminoprop-1-yn-1-yl)phenyl)-N-(7-(2-(4-(4-chlorophenyl)-2,3,9-trimethyl-6H-thieno[3,2-f][1,2,4]triazolo[4,3-a][1,4]diazepin-6-yl)acetamido)heptyl)furan-2-carboxamide hydrochloride |
| 5-114' | methyl (S)-2-(3-aminoprop-1-yn-1-yl)-4-(4-(6-(2-(4-(4-chlorophenyl)-2,3,9-trimethyl-6H-thieno[3,2-f][1,2,4]triazolo[4,3-a][1,4]diazepin-6-yl)acetamido)hexanoyl)piperazin-1-yl)benzoate |
| 5-117' | methyl (S)-2-(3-aminoprop-1-yn-1-yl)-4-(4-(2-(4-(4-chlorophenyl)-2,3,9-trimethyl-6H-thieno[3,2-f][1,2,4]triazolo[4,3-a][1,4]diazepin-6-yl)acetamido)piperidin-1-yl)benzoate |
| 5-128' | (S)-N-(1-(5-(4-(3-aminoprop-1-yn-1-yl)phenyl)furan-2-carbonyl)piperidin-4-yl)-2-(4-(4-chlorophenyl)-2,3,9-trimethyl-6H-thieno[3,2-f][1,2,4]triazolo[4,3-a][1,4]diazepin-6-yl)acetamide |
| 5-130' | (S)-1-(5-(4-(3-aminoprop-1-yn-1-yl)phenyl)furan-2-carbonyl)-N-(4-(2-(4-(4-chlorophenyl)-2,3,9-trimethyl-6H-thieno[3,2-f][1,2,4]triazolo[4,3-a][1,4]diazepin-6-yl)acetamido)butyl)piperidine-4-carboxamide |
| 5-132' | (S)-N-(4-(3-aminoprop-1-yn-1-yl)phenyl)-1-(4-(2-(4-(4-chlorophenyl)-2,3,9-trimethyl-6H-thieno[3,2-f][1,2,4]triazolo[4,3-a][1,4]diazepin-6-yl)acetamido)butanoyl)piperidine-4-carboxamide |
| 5-133' | (S)-N-(4-(3-aminoprop-1-yn-1-yl)-3-(hydroxymethyl)phenyl)-1-(4-(2-(4-(4-chlorophenyl)-2,3,9-trimethyl-6H-thieno[3,2-f][1,2,4]triazolo[4,3-a][1,4]diazepin-6-yl)acetamido)butanoyl)piperidine-4-carboxamide |
| 5-136' | (S)-5-(4-(3-aminoprop-1-yn-1-yl)-3-(hydroxymethyl)phenyl)-N-(7-(2-(4-(4-chlorophenyl)-2,3,9-trimethyl-6H-thieno[3,2-f][1,2,4]triazolo[4,3-a][1,4]diazepin-6-yl)acetamido)heptyl)furan-2-carboxamide |
| 5-137' | (S)-5-(6-(3-aminoprop-1-yn-1-yl)pyridin-3-yl)-N-(4-(2-(4-(4-chlorophenyl)-2,3,9-trimethyl-6H-thieno[3,2-f][1,2,4]triazolo[4,3-a][1,4]diazepin-6-yl)acetamido)butyl)furan-2-carboxamide |
| 5-138' | (S)-5-(6-(3-aminoprop-1-yn-1-yl)pyridin-3-yl)-N-(7-(2-(4-(4-chlorophenyl)-2,3,9-trimethyl-6H-thieno[3,2-f][1,2,4]triazolo[4,3-a][1,4]diazepin-6-yl)acetamido)heptyl)furan-2-carboxamide |
| 5-141' | methyl (S)-2-(3-aminoprop-1-yn-1-yl)-4-(5-((4-(2-(4-(4-chlorophenyl)-2,3,9-trimethyl-6H-thieno[3,2-f][1,2,4]triazolo[4,3-a][1,4]diazepin-6-yl)acetamido)butyl)carbamoyl)furan-2-yl)benzoate |
| 5-142' | (S)-5-(4-(3-aminoprop-1-yn-1-yl)-3-(hydroxymethyl)phenyl)-N-(4-(2-(4-(4-chlorophenyl)-2,3,9-trimethyl-6H-thieno[3,2-f][1,2,4]triazolo[4,3-a][1,4]diazepin-6-yl)acetamido)butyl)furan-2-carboxamide |
| 5-143' | (S)-5-(3-(3-aminoprop-1-yn-1-yl)phenyl)-N-(4-(2-(4-(4-chlorophenyl)-2,3,9-trimethyl-6H-thieno[3,2-f][1,2,4]triazolo[4,3-a][1,4]diazepin-6-yl)acetamido)butyl)furan-2-carboxamide |
| 5-144' | methyl (S)-2-(3-aminoprop-1-yn-1-yl)-5-(5-((4-(2-(4-(4-chlorophenyl)-2,3,9-trimethyl-6H-thieno[3,2-f][1,2,4]triazolo[4,3-a][1,4]diazepin-6-yl)acetamido)butyl)carbamoyl)furan-2-yl)benzoate |
| 5-145' | methyl (S)-2-(3-aminoprop-1-yn-1-yl)-5-(5-((7-(2-(4-(4-chlorophenyl)-2,3,9-trimethyl-6H-thieno[3,2-f][1,2,4]triazolo[4,3-a][1,4]diazepin-6-yl)acetamido)heptyl)carbamoyl)furan-2-yl)benzoate |
| 5-164' | methyl (S)-2-(3-aminoprop-1-yn-1-yl)-4-(4-(3-(2-(4-(4-chlorophenyl)-2,3,9-trimethyl-6H-thieno[3,2-f][1,2,4]triazolo[4,3-a][1,4]diazepin-6-yl)acetamido)propyl)piperazin-1-yl)benzoate hydrochloride |
| 5-165' | methyl (S)-2-(3-aminoprop-1-yn-1-yl)-5-(4-(3-(2-(4-(4-chlorophenyl)-2,3,9-trimethyl-6H-thieno[3,2-f][1,2,4]triazolo[4,3-a][1,4]diazepin-6-yl)acetamido)propyl)piperazin-1-yl)benzoate hydrochloride |
**[Table 15]**
| No. | Compound |
|---|---|
| 5-32" | methyl 2-(3-aminoprop-1-yn-1-yl)-4-(4-(3-(4-((9-chloro-7-(2-fluoro-6-methoxyphenyl)-5H-benzo[c]pyrimido[4,5-e]azepin-2-yl)amino)-2-methoxybenzamido)propanoyl)piperazin-1-yl)benzoate trifluoroacetic acid |

16. A pharmaceutical composition for preventing or treating diseases through degrading a target protein or polypeptide, comprising, as an active ingredient, the compound according to any one of claims 1 to 8, or a pharmaceutically acceptable salt, isomer, solvate, hydrate, or prodrug thereof.

17. A pharmaceutical composition for preventing or treating bromodomain-containing protein-related diseases, comprising, as an active ingredient, the compound according to any one of claims 6 to 8, or a pharmaceutically acceptable salt, isomer, solvate, hydrate, or prodrug thereof, wherein the TBM in the compound is a BRD4 inhibitor.

18. A pharmaceutical composition for preventing or treating cell proliferative disorders, comprising, as an active ingredient, the compound according to any one of claims 6 to 8, or a pharmaceutically acceptable salt, isomer, solvate, hydrate, or prodrug thereof, wherein the TBM in the compound is an Aurora A inhibitor.

19. A pharmaceutical composition for preventing or treating inflammation and inflammation-related diseases, comprising, as an active ingredient, the compound according to any one of claims 6 to 8, or a pharmaceutically acceptable salt, isomer, solvate, hydrate, or prodrug thereof, wherein the TBM in the compound is a p38 inhibitor.
